# EUROPEAN PATENT SPECIFICATION

(11) **EP 3 773 737 B1**
(45) Date of publication and mention of the grant of the patent: **01.09.2021**
(21) Application number: 19835231.2
(22) Date of filing: 09.12.2019
(51) Int. Cl.: A61K 47/64, A61K 47/68, A61K 47/59, A61P 35/00, C07K 16/32, A61K 39/00

(54) **SAPONIN CONJUGATED TO EPITOPE-BINDING PROTEINS**
SAPONIN KONJUGIERT MIT EPITOPE-BINDENDEN PROTEINEN
SAPONINE CONJUGUÉE À DES PROTÉINES DE LIAISON D'ÉPITOPES

(30) Priority: 21.12.2018 NL 2022283; 10.07.2019 NL 2023468; 25.07.2019 NL 2023568
(43) Date of publication of application: 17.02.2021
(60) Divisional application: 20209450.4
(73) Proprietor: SAPREME TECHNOLOGIES B.V., 3723 MB Bilthoven (NL); Charité Universitätsmedizin Berlin, 10117 Berlin (DE)
(72) Inventor: POSTEL, Ruben, 3584 CM Utrecht (NL); FUCHS, Hendrik, Berlin (DE)
(74) Representative: Nederlandsch Octrooibureau
(86) International application number: PCT/EP2019/000334
(87) International publication number: WO 2020/126064

(56) References cited:
- WO-A2-2006/005581
- US-A- 5 591 829
- US-A1- 2012 183 621
- MAKOTO SAITO ET AL: "3[beta]-Hydroxysterol [Delta]24-Reductase on the Surface of Hepatitis C Virus-Related Hepatocellular Carcinoma Cells Can Be a Target for Molecular Targeting Therapy", PLOS ONE, vol. 10, no. 4, 13 April 2015 (2015-04-13) , page e0124197, XP055677053, DOI: 10.1371/journal.pone.0124197
- DAI XIAOJUN: "Anti-metastatic Efficacy of Traditional Chinese Medicine (TCM) Ginsenoside Conjugated to a VEFGR-3 Antibody on Human Gastric Cancer in an Orthotopic Mouse Model", ANTICANCER RESEARCH - INTERNATIONAL JOURNAL OF CANCER RESEARCH AND TREATMENT, vol. 37, no. 3, 17 March 2017 (2017-03-17) , pages 979-986, XP055677059, GR ISSN: 0250-7005, DOI: 10.21873/anticanres.11407
- ROGER GILABERT-ORIOL ET AL: "Modified Trastuzumab and Cetuximab Mediate Efficient Toxin Delivery While Retaining Antibody-Dependent Cell-Mediated Cytotoxicity in Target Cells", MOLECULAR PHARMACEUTICS, vol. 10, no. 11, 7 October 2013 (2013-10-07), pages 4347-4357, XP055677122, US ISSN: 1543-8384, DOI: 10.1021/mp400444q
- GILABERT-ORIOL ROGER ET AL: "Dianthin-30 or gelonin versus monomethyl auristatin E, each configured with an anti-calcitonin receptor antibody, are differentially potent in vitro in high-grade glioma cell lines derived from glioblastoma", CANCER IMMUNOLOGY, IMMUNOTHERAPY, NIH AUTHOR MANUSCRIPT, SPRINGER, BERLIN/HEIDELBERG, vol. 66, no. 9, 13 May 2017 (2017-05-13), pages 1217-1228, XP036309505, ISSN: 0340-7004, DOI: 10.1007/S00262-017-2013-Z [retrieved on 2017-05-13]
- ROGER GILABERT-ORIOL ET AL: "Combinatorial approach to increase efficacy of Cetuximab, Panitumumab and Trastuzumab by dianthin conjugation and co-application of SO1861", BIOCHEMICAL PHARMACOLOGY, vol. 97, no. 3, 1 October 2015 (2015-10-01), pages 247-255, XP055677136, US ISSN: 0006-2952, DOI: 10.1016/j.bcp.2015.07.040
- THAKUR MAYANK ET AL: "High-speed countercurrent chromatographic recovery and off-line electrospray ionization mass spectrometry profiling of bisdesmodic saponins from Saponaria officinalispossessing synergistic toxicity enhancing properties on targeted antitumor toxins", JOURNAL OF CHROMATOGRAPHY B: BIOMEDICAL SCIENCES & APPLICATIONS, ELSEVIER, AMSTERDAM, NL, vol. 955, 19 February 2014 (2014-02-19), pages 1-9, XP028830726, ISSN: 1570-0232, DOI: 10.1016/J.JCHROMB.2014.02.008
- HENDRIK FUCHS ET AL: "Glycosylated Triterpenoids as Endosomal Escape Enhancers in Targeted Tumor Therapies", BIOMEDICINES, vol. 5, no. 2, 29 March 2017 (2017-03-29), page 14, XP055429235, DOI: 10.3390/biomedicines5020014
- EKLADIOUS IRINY ET AL: "Polymer-drug conjugate therapeutics: advances, insights and prospects", NATURE REVIEWS. DRUG DISCOVERY, NATURE PUBLISHING GROUP, GB, vol. 18, no. 4, 12 December 2018 (2018-12-12), pages 273-294, XP036746043, ISSN: 1474-1776, DOI: 10.1038/S41573-018-0005-0 [retrieved on 2018-12-12]
- BHARGAVA: MOLECULAR ONCOLOGY, vol. 11, 2017, pages 1527-1543,
- PASTAN: CELL, vol. 47, 1986, pages 641-648,

## Description

### TECHNICAL FIELD

The invention relates to a conjugate of a molecule for binding to a cell-surface molecule and a saponin, said conjugate for improving efficacy of e.g. an ADC. The core of the invention is a proteinaceous molecule comprising a saponin linked to a moiety that binds a cell surface molecule. The invention also relates to a composition comprising the conjugate of the invention and an oligonucleotideThe invention also relates to an antibody-drug conjugate or a ligand-drug conjugate comprising the conjugate of the invention and an effector moiety.

### BACKGROUND

Molecules with a therapeutic biological activity are in many occasions in theory suitable for application as an effective therapeutic drug for the treatment of a disease such as a cancer in human patients in need thereof. A typical example are small-molecule biologically active moieties. However, many if not all potential drug-like molecules and therapeutics currently used in the clinic suffer from at least one of a plethora of shortcomings and drawbacks. When administered to a human body, therapeutically active molecules may exert off-target effects, in addition to the biologically activity directed to an aspect underlying a to-be-treated disease or health problem. Such off-target effects are undesired and bear a risk for induction of health- or even life-threatening side effects of the administered molecule. It is the occurrence of such adverse events that cause many drug-like compounds and therapeutic moieties to fail phase III clinical trials or even phase IV clinical trials (post-market entry follow-up). Therefore, there is a strong desire to provide drug molecules such as small-molecule therapeutics, wherein the therapeutic effect of the drug molecule should, e.g., (1) be highly specific for a biological factor or biological process driving the disease, (2) be sufficiently safe, (3) be sufficiently efficacious, (4) be sufficiently directed to the diseased cell with little to no off-target activity on non-diseased cells, (5) have a sufficiently timely mode of action (e.g. the administered drug molecule should reach the targeted site in the human patient within a certain time frame and should remain at the targeted site for a certain time frame), and/or (6) have sufficiently long lasting therapeutic activity in the patient's body, amongst others. Unfortunately, to date, 'ideal' therapeutics with many or even all of the beneficial characteristics here above outlined, are not available to the patients, despite already long-lasting and intensive research and despite the impressive progress made in several areas of the individually addressed encountered difficulties and drawbacks.

Chemotherapy is one of the most important therapeutic options for cancer treatment. However, it is often associated with a low therapeutic window because it has no specificity towards cancer cells over dividing cells in healthy tissue. The invention of monoclonal antibodies offered the possibility of exploiting their specific binding properties as a mechanism for the targeted delivery of cytotoxic agents to cancer cells, while sparing normal cells. This can be achieved by chemical conjugation of cytotoxic effectors (also known as payloads or warheads) to antibodies, to create antibody-drug conjugates (ADCs). Typically, very potent payloads such as emtansine (DM1) are used which have a limited therapeutic index (a ratio that compares toxic dose to efficacious dose) in their unconjugated forms. The conjugation of DM1 to trastuzumab (ado-trastuzumab emtansine), also known as Kadcycla, enhances the tolerable dose of DM1 at least two-fold in monkeys. In the past few decades tremendous efforts and investments have been made to develop therapeutic ADCs. However, it remains challenging to bring ADCs into the clinic, despite promising preclinical data. The first ADC approved for clinical use was gemtuzumab ozogamicin (Mylotarg, CD33 targeted, Pfizer/Wyeth) for relapsed acute myelogenous leukemia (AML) in 2000. Mylotarg was however, withdrawn from the market at the request of the Federal Drug Administration (FDA) due to a number of concerns including its safety profile. Patients treated with Mylotarg were more often found to die than patients treated with conventional chemotherapy. Mylotarg was admitted to the market again in 2017 with a lower recommended dose, a different schedule in combination with chemotherapy or on its own, and a new patient population. To date, only five ADCs have been approved for clinical use, and meanwhile clinical development of approximately fifty-five ADCs has been halted. However, interest remains high and approximately eighty ADCs are still in clinical development in nearly six-hundred clinical trials at present.

Despite the potential to use toxic payloads that are normally not tolerated by patients, a low therapeutic index (a ratio that compares toxic dose to efficacious dose) is a major problem accounting for the discontinuance of many ADCs in clinical development, which can be caused by several mechanisms such as off-target toxicity on normal cells, development of resistance against the cytotoxic agents and premature release of drugs in the circulation. A systematic review by the FDA of ADCs found that the toxicity profiles of most ADCs could be categorized according to the payload used, but not the antibody used, suggesting that toxicity is mostly determined by premature release of the payload. Of the approximately fifty-five ADCs that were discontinued, it is estimated that at least twenty-three were due to a poor therapeutic index. For example, development of a trastuzumab tesirine conjugate (ADCT-502, HER-2 targeted, ADC therapeutics) was recently discontinued due to a narrow therapeutic index, possibly due to an on-target, off-tissue effect in pulmonary tissue which expresses considerable levels of HER2. In addition, several ADCs in phase 3 trials have been discontinued due to missing primary endpoint. For example, phase 3 trials of a depatuxizumab mafodotin conjugate (ABT-414, EGFR targeted, AbbVie) tested in patients with newly diagnosed glioblastoma, and a mirvetuximab soravtansine conjugate (IMGN853, folate receptor alpha (FRα) targeted, ImmunoGen) tested in patients with platinum-resistant ovarian cancer, were recently stopped, showing no survival benefit. It is important to note that the clinically used dose of some ADCs may not be sufficient for its full anticancer activity. For example, ado-trastuzumab emtansine has an MTD of 3.6 mg/kg in humans. In preclinical models of breast cancer, ado-trastuzumab emtansine induced tumor regression at dose levels at or above 3 mg/kg, but more potent efficacy was observed at 15 mg/kg. This suggests that at the clinically administered dose, ado-trastuzumab emtansine may not exert its maximal potential anti-tumor effect.

ADCs are mainly composed of an antibody, a cytotoxic moiety such as a payload, and a linker. Several novel strategies have been proposed and carried out in the design and development of new ADCs to overcome the existing problems, targeting each of the components of ADCs. For example, by identification and validation of adequate antigenic targets for the antibody component, by selecting antigens which have high expression levels in tumor and little or no expression in normal tissues, antigens which are present on the cell surface to be accessible to the circulating ADCs, and antigens which allows internalizing of ADCs into the cell after binding; and alternative mechanisms of activity; design and optimize linkers which enhance the solubility and the drug-to-antibody ratio (DAR) of ADCs and overcome resistance induced by proteins that can transport the chemotherapeutic agent out of the cells; enhance the DAR ratio by inclusion of more payloads, select and optimize antibodies to improve antibody homogeneity and developability. In addition to the technological development of ADCs, new clinical and translational strategies are also being deployed to maximize the therapeutic index, such as, change dosing schedules through fractionated dosing; perform biodistribution studies; include biomarkers to optimize patient selection, to capture response signals early and monitor the duration and depth of response, and to inform combination studies.

An example of ADCs with clinical potential are those ADCs such as brentuximab vedotin, inotuzumab ozogamicin, moxetumomab pasudotox, and polatuzumab vedotin, which are evaluated as a treatment option for lymphoid malignancies and multiple myeloma. Polatuzumab vedotin, binding to CD79b on (malignant) B-cells, and pinatuzumab vedotin, binding to CD22, are tested in clinical trials wherein the ADCs each were combined with co-administered rituximab, a monoclonal antibody binding to CD20 and not provided with a payload [B. Yu and D. Liu, Antibody-drug conjugates in clinical trials for lymphoid malignancies and multiple myeloma; Journal of Hematology & Oncology (2019) 12:94]. Combinations of monoclonal antibodies such as these examples are yet a further approach and attempt to arrive at the 'magic bullet' which combines many or even all of the aforementioned desired characteristics of ADCs.

Meanwhile in the past few decades, nucleic acid-based therapeutics are under development. Therapeutic nucleic acids can be based on deoxyribonucleic acid (DNA) or ribonucleic acid (RNA), Antisense oligonucleotides (ASOs, AONs), and short interfering RNAs (siRNAs), MicroRNAs, and DNA and RNA aptamers, for approaches such as gene therapy, RNA interference (RNAi). Many of them share the same fundamental basis of action by inhibition of either DNA or RNA expression, thereby preventing expression of disease-related abnormal proteins. The largest number of clinical trials is being carried out in the field of gene therapy, with almost 2600 ongoing or completed clinical trials worldwide but with only about 4% entering phase 3. This is followed by clinical trials with ASOs. Similarly to ADCs, despite the large number of techniques being explored, therapeutic nucleic acids share two major issues during clinical development: delivery into cells and off-target effects. For instance, ASOs such as peptide nucleic acid (PNA), phosphoramidate morpholino oligomer (PMO), locked nucleic acid (LNA) and bridged nucleic acid (BNA), are being investigated as an attractive strategy to inhibit specifically target genes and especially those genes that are difficult to target with small molecules inhibitors or neutralizing antibodies. Currently, the efficacy of different ASOs is being studied in many neurodegenerative diseases such as Huntington's disease, Parkinson's disease, Alzheimer's disease, and amyotrophic lateral sclerosis and also in several cancer stages. The application of ASOs as potential therapeutic agents requires safe and effective methods for their delivery to the cytoplasm and/or nucleus of the target cells and tissues. Although the clinical relevance of ASOs has been demonstrated, inefficient cellular uptake, both in vitro and in vivo, limit the efficacy of ASOs and has been a barrier to therapeutic development. Cellular uptake can be < 2% of the dose resulting in too low ASO concentration at the active site for an effective and sustained outcome. This consequently requires an increase of the administered dose which induces off-target effects. Most common side-effects are activation of the complement cascade, the inhibition of the clotting cascade and toll-like receptor mediated stimulation of the immune system.

Chemotherapeutics are most commonly small molecules, however, their efficacy is hampered by the severe off-target side toxicity, as well as their poor solubility, rapid clearance and limited tumor exposure. Scaffold-small-molecule drug conjugates such as polymer-drug conjugates (PDCs) are macromolecular constructs with pharmacologically activity, which comprises one or more molecules of a small-molecule drug bound to a carrier scaffold (e.g. polyethylene glycol (PEG)).

Such conjugate principle has attracted much attention and has been under investigation for several decades. The majority of conjugates of small-molecule drugs under pre-clinical or clinical development are for oncological indications. However, up-to-date only one drug not related to cancer has been approved (Movantik, a PEG oligomer conjugate of opioid antagonist naloxone, AstraZeneca) for opioid-induced constipation in patients with chronic pain in 2014, which is a non-oncology indication. Translating application of drug-scaffold conjugates into treatment of human subjects provides little clinical success so far. For example, PK1 (N-(2-hydroxypropyl)methacrylamide (HPMA) copolymer doxorubicin; development by Pharmacia, Pfizer) showed great anti-cancer activity in both solid tumors and leukemia in murine models, and was under clinical investigation for oncological indications. Despite that it demonstrated significant reduction of nonspecific toxicity and improved pharmacokinetics in man, improvements in anticancer efficacy turned out to be marginal in patients, and as a consequence further development of PK1 was discontinued.

The failure of scaffold-small-molecule drug conjugates is at least partially attributed to its poor accumulation at the tumor site. For example, while in murine models PK1 showed 45-250 times higher accumulation in the tumor than in healthy tissues (liver, kidney, lung, spleen, and heart), accumulation in tumor was only observed in a small subset of patients in the clinical trial.

A potential solution to the aforementioned problems is application of nanoparticle systems for drug delivery such as liposomes. Liposomes are sphere-shaped vesicles consisting of one or more phospholipid bilayers, which are spontaneously formed when phospholipids are dispersed in water. The amphiphilicity characteristics of the phospholipids provide it with the properties of self-assembly, emulsifying and wetting characteristics, and these properties can be employed in the design of new drugs and new drug delivery systems. Drug encapsulated in a liposomal delivery system may convey several advantages over a direct administration of the drug, such as an improvement and control over pharmacokinetics and pharmacodynamics, tissue targeting property, decreased toxicity and enhanced drug activity. An example of such success is liposome-encapsulated form of a small molecule chemotherapy agent doxorubicin (Doxil: a pegylated liposome-encapsulated form of doxorubicin; Myocet: a non-pegylated liposomal doxorubicin), which have been approved for clinical use.

Gilabert-Oriol et al. (2015; Biochem. Pharmacol. 97(3):247-255) describe the improvement of the efficacy of cetuximab, panitumumab and trastuzumab by conjugation of these antibodies with dianthin and by co-application of the said conjugates and saponin SO1861.

Bhargava et al. (2017; Mol. One. 11:1527-1543) describe a fusion protein of EGF and dianthin which is combined with endosomal escape enhancer glycosylated triterpene SO1861, and describe that said combination shows an improved anti-tumor activity in an *in vitro* and an *in vivo* pancreatic carcinoma model.

Therefore, a solution still needs to be found that allows for drug therapies such as anti-tumor therapies, applicable for non-systemic use when desired, wherein the drug has for example an acceptable safety profile, little off-target activity, sufficient efficacy, sufficiently low clearance rate from the patient's body, etc.

### SUMMARY

The present invention concerns itself with improving current drugs that are found to be less efficacious than desired when administered to a patient in need thereof. In particular the following problems are encountered:
- non-specificity, encountered when administering small-molecule therapeutically active compounds to a human patient in need thereof;
- non-optimal specificity for a biological factor or biological process driving a disease;
- insufficient safety characteristics of current drugs, when administered to human patients in need thereof;
- insufficiently direction of drugs to the diseased cell with little to no off-target activity on non-diseased cells, when administered to human patients in need thereof;
- insufficiently timely mode of action (e.g. the administered drug molecule should reach the targeted site in the human patient within a certain time frame and should remain at the targeted site for a certain time frame), when administered to human patients in need thereof;
- insufficient long lasting therapeutic activity in the patient's body, when administered to human patients in need thereof.

The invention, which is defined in the appended claims, provides solutions to one or more of these problems.

In essence, it has been found that the uptake of certain compounds is improved if they are co-administered with, or linked to, a molecule that comprises a saponin linked to a cell-targeting moiety, as defined in the appended claims.

### DEFINITIONS

The term "linker" has its regular scientific meaning, and here refers to a chemical moiety or a linear stretch of amino-acid residues complexed through peptide bonds, which attaches a molecule or an atom to another molecule, e.g. to a ligand or to an effector molecule or to a scaffold. Typically, the linker comprises a chain of atoms linked by chemical bonds. Any linker molecule or linker technology known in the art can be used in the present disclosure. Where indicated, the linker is a linker for covalently binding of molecules through a chemical group on such a molecule suitable for forming a covalent linkage or bond with the linker. The linker may be a non-cleavable linker, e.g., the linker is stable in physiological conditions. The linker may be a cleavable linker, e.g. a linker that is cleavable, in the presence of an enzyme or at a particular pH range or value, or under physiological conditions such as intracellular conditions in the endosomes such as the late endosomes and the lysosomes of mammalian cells such as human cells. Exemplary linkers that can be used in the context of the present disclosure includes, but is not limited to, N-ε-mateimidocaproic acid hydrazide (EMCH), succinimidyl 3-(2-pyridyldithio)propionate or 3-(2-Pyridyldithio)propionic acid N-hydroxysuccinimide ester (SPDP), and 1-[Bis(dimethylamino)methylene]-1H-1,2,3-triazolo[4,5-b]pyridinium 3-oxid hexafluorophosphate (HATU).

The term "tri-functional linker" has its regular scientific meaning, and here refers to a linker which attaches three molecules via a chemical group on each of the three molecules. The skilled person is able to design such tri-functional linkers, based on the present disclosure and the common general knowledge. Such tri-functional linker can exhibit, for instance, a maleimido group that can be used for conjugation to targeting ligands that exhibit thiol groups to perform a thiol-ene reaction. In addition, the tri-functional linker could exhibit a dibenzocyclooctyne (DBCO) group to perform the so-called strain-promoted alkyne-azide cycloaddition (SPAAC, click chemistry) with an azido bearing saponin. Finally, the tri-functional linker could obtain a third functional group such as a trans-cyclooctene (TCO) group to perform the so-called inverse electron demand Diels-Alder (IEDDA) reaction with a tetrazine (Tz) bearing effector molecule. The skilled person will appreciate that the chemical groups of the tri-functional linker can be all three the same, or different, or the linker may comprise two of the same chemical groups for linking a molecule to the tri-functional linker. The formed bonds between the tri-functional linker can be covalent or non-covalent, and covalent bonds are preferred. The formed bonds between the tri-functional linker and the one or two or three bound molecules via respective chemical groups, can be cleavable (labile) bonds, such as cleavable under acidic conditions inside cells such as endosomes and lysosomes of mammalian cells such as human cells, or can be non-cleavable bonds. Of course, the tri-functional linker may encompass one or two chemical groups for forming covalent bonds while the further two or one chemical group(s), respectively, are/is for forming a non-covalent bond. Of course, the tri-functional linker may encompass one or two chemical groups for forming cleavable bonds while the further two or one chemical group(s), respectively, are/is for forming a non-cleavable bond.

The term "cleavable", such as used in the term "cleavable linker" or "cleavable bond" has its regular scientific meaning, and here refers to being subject to cleavage under acidic conditions, reductive conditions, enzymatic conditions or light-induced conditions. For example, a cleavable linker may be subject to cleavage under acidic conditions, preferably said cleavable linker is subject to cleavage in vivo under acidic conditions as present in endosomes and/or lysosomes of mammalian cells, preferably human cells, preferably at pH 4.0 - 6.5, and more preferably at pH ≤ 5.5. As another example, a cleavable linker may be subject to cleavage by an enzyme, e.g. by cathepsin. Furthermore, an example of a covalent bond cleavable under reductive conditions is a disulphide bond.

The terms "oligomer" and "polymer" in the context of an oligomeric or polymeric scaffold has its regular scientific meaning. A polymer here refers to a substance which has a molecular structure built up chiefly or completely from a large number of equal or similar units bonded together; an oligomer here refers to a polymer whose molecules consist of relatively few repeating units. For example, a structure comprising 5-10 or less equal or similar units, may be called an oligomeric structure, whereas a structure comprising 10-50 monomeric units or more may be called a polymeric structure, whereas a structure of 10 monomeric units may be called either oligomeric or polymeric.

The term "binding site" has its regular scientific meaning, and here refers to a region or an epitope on a molecule, e.g. a protein, DNA or RNA, to which another molecule can bind.

The term "scaffold" has its regular scientific meaning, and here refers to an oligomeric or polymeric template or a carrier or a base (base molecule or base structure), to which one or more molecules, e.g. ligand molecule, effector molecule, can be covalently bound, either directly, or via a linker, such as a cleavable linker. A scaffold may have a structurally ordered formation such as a polymer, oligomer, dendrimer, dendronized polymer, or dendronized oligomer or have an assembled polymeric structure such as a hydrogel, microgel, nanogel, stabilized polymeric micelle or liposome, but excludes structures that are composed of non-covalent assemblies of monomers such as cholesterol/phospholipid mixtures. A scaffold may comprise a polymeric or oligomeric structure, such as poly- or oligo(amines), e.g., polyethylenimine and poly(amidoamine); or structures such as polyethylene glycol, poly- or oligo(esters), such as poly(lactids), poly(lactams), polylactide-co-glycolide copolymers; or poly(dextrin), poly- or oligosaccharides, such as cyclodextrin or polydextrose; or structures such as natural and/or artificial poly- or oligoamino acids such as poly-lysine or a peptide or a protein, DNA oligo-or polymers, stabilized RNA polymers or PNA (peptide nucleic acid) polymers. Preferably, the polymeric or oligomeric structures are biocompatible, wherein biocompatible means that the polymeric or oligomeric structure does not show substantial acute or chronic toxicity in organisms and can be either excreted as it is or fully degraded to excretable and/or physiological compounds by the body's metabolism.

The term "ligand" has its regular scientific meaning, and here refers to any molecule or molecules which may selectively bind to a target cell-surface molecule or target cell-surface receptor expressed at target cells, e.g. target cancer cells or target auto-immune cells. The ligand may bind to an epitope comprised by receptors or other antigens on the target cells. Preferably, the cell-binding ligands are antibodies.

The term "antibody" as used herein is used in the broadest sense, which may refer to an immunoglobulin (Ig) defined as a protein belonging to the class IgG, IgM, IgE, IgA, or IgD (or any subclass thereof), or a functional binding fragment or binding domain of an immunoglobulin. In the context of the present invention, a "binding fragment" or a "binding domain" of an immunoglobulin is defined as antigen-binding fragment or -domain or other derivative of a parental immunoglobulin that essentially maintains the antigen binding activity of such parental immunoglobulin. Functional fragments and functional domains are antibodies in the sense of the present invention even if their affinity to the antigen is lower than that of the parental immunoglobulin. "Functional fragments and -domains" in accordance with the invention include, but are not limited to, F(ab')2 fragments, Fab' fragments, Fab fragments, scFv, dsFv, single-domain antibody (sdAb), monovalent IgG, scFv-Fc, reduced IgG (rlgG), minibody, diabodies, triabodies, tetrabodies, Fc fusion proteins, nanobodies, variable V domains such as VHH, Vh, and other types of antigen recognizing immunoglobulin fragments and domains. The fragments and domains may be engineered to minimize or completely remove the intermolecular disulphide interactions that occur between the CH1 and CL domains. Functional fragment and -domains offer the advantage of greater tumor penetration because of their smaller size. In addition, the functional fragment or -domain can be more evenly distributed throughout the tumor mass as compared to whole immunoglobulin.

The antibodies (immunoglobulins) of the present invention may be bi- or multifunctional. For example, a bifunctional antibody has one arm having a specificity for one receptor or antigen, while the other arm recognizes a different receptor or antigen. Alternatively, each arm of the bifunctional antibody may have specificity for a different epitope of the same receptor or antigen of the target cell.

The antibodies (immunoglobulins) of the present invention may be, but are not limited to, polyclonal antibodies, monoclonal antibodies, human antibodies, humanized antibodies, chimeric antibodies, resurfaced antibodies, anti-idiotypic antibodies, mouse antibodies, rat antibodies, rat/mouse hybrid antibodies, llama antibodies, llama heavy-chain only antibodies, heavy-chain only antibodies, and veterinary antibodies. Preferably, the antibody (immunoglobulin) of the present invention is a monoclonal antibody. The resurfaced, chimeric, humanized and fully human antibodies are also more preferred because they are less likely to cause immunogenicity in humans. The antibodies of the ADC of the present invention preferably specifically binds to an antigen expressed on the surface of a cancer cell, an autoimmune cell, a diseased cell, an aberrant cell, while leaving any healthy cell essentially unaltered (e.g. by not binding to such normal cell, or by binding to a lesser extent in number and/or affinity to such healthy cell).

Specific antibodies that can be used for the ADCs of the present invention include, but are not limited to, anti-HER2 monoclonal antibody such as trastuzumab and pertuzumab, anti-CD20 monoclonal antibody such as rituximab, ofatumumab, tositumomab and ibritumomab, anti-CA125 monoclonal antibody such as oregovomab, anti-EpCAM (17-1A) monoclonal antibody such as edrecolomab, anti-EGFR monoclonal antibody such as cetuximab, panitumumab and nimotuzumab, anti-CD30 monoclonal antibody such brentuximab, anti-CD33 monoclonal antibody such as gemtuzumab and huMy9-6, anti-vascular integrin alpha-v beta-3 monoclonal antibody such as etaracizumab, anti-CD52 monoclonal antibody such as alemtuzumab, anti-CD22 monoclonal antibody such as epratuzumab, anti-CEA monoclonal antibody such as labetuzumab, anti-CD44v6 monoclonal antibody such as bivatuzumab, anti-FAP monoclonal antibody such as sibrotuzumab, anti-CD19 monoclonal antibody such as huB4, anti-CanAg monoclonal antibody such as huC242, anti-CD56 monoclonal antibody such huN901, anti-CD38 monoclonal antibody such as daratumumab, anti-CA6 monoclonal antibody such as DS6, anti-IGF-IR monoclonal antibody such as cixutumumab and 3B7, anti-integrin monoclonal antibody such as CNTO 95, and anti-syndecan-1 monoclonal antibody such as B-B4.

Any other molecules than antibodies that bind to a cell receptor or antigen of a target cell can also be used as the cell-binding ligand for the ligand-drug conjugates of the present invention and the ligands provided with covalently bound saponin according to the invention. These ligands include, but are not limited to, proteins, polypeptides, peptides, small molecules. Examples of these non-antibody ligands are interferons (e.g. IFN-α, IFN-β, and IFN-y), transferrins, lectins, epidermal growth factors (EGF) and EGF-like domains, gastrin-releasing peptides (GRP), platelet-derived growth factors (PDGF), transforming growth factors (TGF), vaccinia growth factor (VGF), insulin and insulin-like growth factors (IGF, e.g. IGF-1 and IGF-2), other suitable hormones such as thyrotropin releasing hormones (TRH), melanocyte-stimulating hormones (MSH), steroid hormones (e.g. estrogen and androgen), somatostatin, lymphokines (e.g. IL-2, IL-3, IL-4, and IL-6), colony-stimulating factors (CSF, e.g. G-CSF, M-CSF and GM-CSF), bombesin, gastrin, Arg-Gly-Asp or RGD, aptamers (e.g. AS-1411, GBI-10, RNA aptamers against HIV glycoprotein), small molecules (e.g. folate, anisamide phenylboronic acid), vitamins (e.g., vitamin D), carbohydrates (e.g. hyaluronic acid, galactose).

An "effector molecule" or "effector moiety" or "payload" has its regular scientific meaning and in the context of this invention is any substance that affects the metabolism of a cell by interaction with an intracellular effector molecule target, wherein this effector molecule target is any molecule or structure inside cells excluding the lumen of compartments and vesicles of the endocytic and recycling pathway but including the membranes of these compartments and vesicles. Said structures inside cells thus include the nucleus, mitochondria, chloroplasts, endoplasmic reticulum, Golgi apparatus, other transport vesicles, the inner part of the plasma membrane and the cytosol.

The effector molecule or -moiety is a pharmaceutically active substance, such as a toxin such as a proteinaceous toxin, a drug, a polypeptide or a polynucleotide. A pharmaceutically active substance in this invention is an effector molecule or -moiety that is used to achieve a beneficial outcome in an organism, preferably a vertebrate, more preferably a mammal such as non-human subjects or a human being/subject. Benefits include diagnosis, prognosis, treatment, cure and prevention (prophylaxis) of diseases and/or symptoms and/or health problems. The pharmaceutically active substance may also lead to undesired and sometimes even harmful side effects (adverse events such as observed during clinical trials). In this case, pros and cons must be weighed to decide whether the pharmaceutically active substance is suitable in the particular case. If the effect of the pharmaceutically active substance inside a cell is predominantly beneficial for the organism as a whole, the cell is called a target cell. If the effect inside a cell is predominantly harmful for the organism as a whole, the cell is called an off-target cell. In artificial systems such as cell cultures and bioreactors, target cells and off-target cells depend on the purpose and are defined by the user. Examples of effector molecules and -moieties are a drug, a toxin, a polypeptide (such as an enzyme), a polynucleotide (including polypeptides and polynucleotides that comprise non-natural amino acids or nucleic acids), and any combination thereof.

An effector molecule or effector moiety that is a drug may include, but not limited to, anti-cancer agents, anti-inflammatory agents, and anti-infective (e.g., anti-fungal, antibacterial, anti-parasitic, antiviral) agents. Preferably, the drug molecule of the present invention is an anti-cancer agent or an anti-auto-immune agent. Suitable anti-cancer agents include, but are not limited to, alkylating agents, antimetabolites, spindle poison plant alkaloids, cytotoxic/antitumor antibiotics, topoisomerase inhibitors, photosensitizers, and kinase inhibitors. Also included in the definition of "anti-cancer agent" are: e.g. (i) anti-hormonal agents that act to regulate or inhibit hormone action on tumors such as anti-estrogens and selective estrogen receptor modulators; (ii) aromatase inhibitors that inhibit the enzyme aromatase, which regulates estrogen production in the adrenal glands; (iii) anti-androgens; (iv) protein kinase inhibitors; (v) lipid kinase inhibitors; (vi) antisense oligonucleotides, particularly those which inhibit expression of genes in signaling pathways implicated in aberrant cell proliferation; (vii) ribozymes such as VEGF expression inhibitors and HER2 expression inhibitors; (viii) vaccines such as gene therapy vaccines; topoisomerase 1 inhibitors; (ix) anti-angiogenic agents; and pharmaceutically acceptable salts, acids, solvates and derivatives of any of the above.

An effector molecule or -moiety that is a toxin may include, but is not limited to, proteinaceous toxins (e.g. bacterial-derived toxins, and plant-derived toxins), toxins targeting tubulin filaments, toxins targeting DNA, toxins targeting RNA. Examples of proteinaceous toxins are saporin, dianthin, ricin, modeccin, abrin, volkensin, viscumin, shiga toxin, shiga-like toxin, pseudomonas exotoxin (PE, also known as exotoxin A), diphtheria toxin (DT), and cholera toxin. Examples of tubulin filaments-targeting toxins are maytansinoids (e.g. DM1 and DM4), auristatins (e.g. Monomethyl auristatin E (MMAE) and Monomethyl auristatin F (MMAF)), toxoids, tubulysins, cryptophycins, rhizoxin. Examples of DNA-targeting toxins are calicheamicins: N-Acetyl- γ-calicheamicin, CC-1065 analogs, duocarmycins, doxorubicin, methotrexate, benzodiazepines, camptothecin analogues, and anthracyclines. Examples of DNA-targeting toxins are amanitins, spliceostatins, and thailanstatins. A toxin, as used in this invention, is defined as a pharmaceutically active substance that is able to kill or inactivate a cell. Preferably, a targeted toxin is a toxin that is only, or at least predominantly, toxic for target cells but not for off-target cells. The net effect of the targeted toxin is preferably beneficial for the organism as a whole.

An effector molecule or -moiety that is a polypeptide may be, e.g., a polypeptide that recover a lost function, such as for instance enzyme replacement, gene regulating functions, or a toxin. Examples of polypeptides as effector molecules are, e.g., Cas9; toxins (e.g. saporin, dianthin, gelonin, (de)bouganin, agrostin, ricin (toxin A chain); pokeweed antiviral protein, apoptin, diphtheria toxin, pseudomonas exotoxin) metabolic enzymes (e.g. argininosuccinate lyase, argininosuccinate synthetase), enzymes of the coagulation cascade, repairing enzymes; enzymes for cell signaling; cell cycle regulation factors; gene regulating factors (transcription factors such as NF-κB or gene repressors such as methionine repressor).

An effector molecule or an effector moiety that is a polynucleotide may, e.g., be a polynucleotide that comprises coding information, such as a gene or an open reading frame encoding a protein. It may also comprise regulatory information, e.g. promotor or regulatory element binding regions, or sequences coding for micro RNAs. Such polynucleotide may comprise natural and artificial nucleic acids. Artificial nucleic acids include, e.g. peptide nucleic acid (PNA), Morpholino and locked nucleic acid (LNA), as well as glycol nucleic acid (GNA) and threose nucleic acid (TNA). Each of these is distinguished from naturally occurring DNA or RNA by changes to the backbone of the molecule. Examples of nucleotides as effector molecules are, but not limited to, e.g., DNA: single stranded DNA (e.g. DNA for adenine phosphoribosyltransferase); linear double stranded DNA (e.g. clotting factor IX gene); circular double stranded DNA (e.g. plasmids); RNA: mRNA (e.g. TAL effector molecule nucleases), tRNA, rRNA, siRNA, miRNA, antisense RNA; anti-sense oligonucleotides (ASOs, AONs e.g. PNA, PMO, LNA and BNA).

The term "proteinaceous", used in e.g. "proteinaceous molecule" and "proteinaceous toxin", are molecules and toxins comprising at least a string of amino acid residues that can be obtained as an expression product from a single mRNA. Such a molecule or toxin may further comprise any post-translational modifications, a carbohydrate such as an N- or O-linked carbohydrate, disulphide bonds, phosphorylations, sulphatations, etc., as a result of any post-translational modification, and/or may further comprise any other modification such as those resulting from chemical modifications (e.g., linking of effector moieties, saponin, scaffolds, ligands, etc., either directly to e.g. an amino-acid side chain, or via at least one linker (covalently) bound to the molecule for chemically modifying the proteinaceous molecule, and chemically bound (covalently) to the proteinaceous molecule). The term "proteinaceous" also encompasses and includes assemblies of such molecules, e.g. homodimers, heterotrimers, heterohexamers or complex assemblies such as ribosomes.

The terms "specific" and "specifically", in the context of for example "specific binding" and "receptor or molecular target specifically present or expressed at the surface of a tumor cell" and the like, have their normal scientific meaning known in the art, and here refer to e.g. a binding interaction of a first molecule with a second molecule which occurs with a higher affinity relative to any putative binding of the first molecule to a further molecule different from the second molecule, or e.g. to the expression or expression to a higher extent when e.g. the number of receptors or molecular targets is considered, of a cell-surface receptor or molecular target on the surface of a first type of cell such as a tumor cell, autoimmune cell, diseased cell, aberrant cell, relative to the extent of expression of the same receptor or molecular target at a second type of cell such as a healthy cell, etc., wherein expression at the second type of cell can be fully absent or very low, relative to any extent of expression on the tumor cell, etc. Furthermore, the term "specific", for example in "specific binding", has its normal scientific meaning known in the art, and here has the meaning of indicating a molecule that can have an interaction with another molecule with higher binding affinity than background interactions between molecules. Similarly, the term "specificity" refers to an interaction, for example, between two molecules or between a cell and a molecule, which has higher binding affinity than background interactions between molecules. Binding molecules such as immunoglobulins bind via their binding site such as immunoglobulin variable regions of the immunoglobulin, to binding sites on molecules, such as epitopes, cell-surface receptors, etc., with a higher binding affinity than background interactions between molecules. In the context of the invention, background interactions are typically interactions with an affinity lower than a K_{D} of 10E-4 M. Similarly, "specific binding domains" are domains that preferentially bind to binding sites on molecules, such as epitopes, cell-surface receptors, etc., with a higher binding affinity than background interactions between molecules. In the context of the invention, "background interactions" are typically interactions with an affinity lower than a K_{D} of 10E-4 M. Preferably, specific binding domains bind with an affinity higher than a K_{D} of about 10E-5 M.

The term "binding" is defined as interactions between molecules that can be distinguished from background interactions.

Throughout the specification, the term "fragment" refers to an amino acid sequence which is part of a protein domain or which builds up an intact protein domain. Binding fragments according to the invention must have binding specificity for the respective target such as a cell-surface receptor, e.g. on the surface of a diseased cell such as a tumor cell.

The term "ADC" or "antibody-drug conjugate" has its regular scientific meaning known to the skilled person, and here refers to a class of biopharmaceutical drugs designed as a targeted therapy for treating e.g. cancer. Unlike chemotherapy, ADCs are intended to target and kill tumor cells while sparing healthy cells. ADCs are composed of an antibody linked to a biologically active cytotoxic (anticancer) payload or drug. ADCs combine the targeting capabilities of monoclonal antibodies with the cancer-killing ability of cytotoxic drugs. They are designed with the intention to discriminate between healthy cells and diseased tissue such as tumor cells in a tumor.

The term "Saponinum album" has its normal meaning and here refers to a mixture of saponins produced by Merck KGaA (Darmstadt, Germany) containing saponins from *Gypsophila paniculata* and *Gypsophila arostii,* containing SA1657 and mainly SA1641.

The term "Quillajasaponin" has its normal meaning and here refers to the saponin fraction of Quillaja saponaria and thus the source for all other QS saponins, mainly containing QS-18 and QS-21.

"QS-21" or "QS21" has its regular scientific meaning and here refers to a mixture of QS-21 A-apio (-63%), QS-21 A-xylo (-32%), QS-21 B-apio (-3.3%), and QS-21 B-xylo (-1.7%).

Similarly, "QS-21A" has its regular scientific meaning and here refers to a mixture of QS-21 A-apio (-65%) and QS-21 A-xylo (-35%).

Similarly, "QS-21B" has its regular scientific meaning and here refers to a mixture of QS-21 B-apio (-65%) and QS-21 B-xylo (-35%).

The term "Quil-A" refers to a commercially available semi-purified extract from Quillaja saponaria and contains variable quantities of more than 50 distinct saponins, many of which incorporate the triterpene-trisaccharide substructure Gal-(1→2)-[Xyl-(1→3)]-GlcA- at the C-3beta-OH group found in QS-7, QS-17, QS18, and QS-21. The saponins found in Quil-A are listed in van Setten (1995), Table *2 [*Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)]. Quil-A and also Quillajasaponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et al. (2019) in Table 1, row no. 28 [Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171]*.* The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

The terms first, second, third and the like in the description and in the claims, are used for distinguishing between similar elements and not necessarily for describing a sequential or chronological order. The terms are interchangeable under appropriate circumstances. The embodiments of the invention can operate in other sequences than described or illustrated herein.

Furthermore, the various embodiments, although referred to as "preferred" or "e.g." or "for example" or "in particular" are to be construed as exemplary manners in which the invention may be implemented rather than as limiting the scope of the invention.

The term "comprising", used in the claims, should not be interpreted as being restricted to the elements or steps listed thereafter; it does not exclude other elements or steps. It needs to be interpreted as specifying the presence of the stated features, integers, steps or components as referred to, but does not preclude the presence or addition of one or more other features, integers, steps or components, or groups thereof. Thus, the scope of the expression "a pharmaceutical composition comprising A and B" should not be limited to a pharmaceutical composition consisting only of components A and B, rather with respect to the present invention, the only enumerated components of the pharmaceutical composition are A and B, and further the claim should be interpreted as including equivalents of those components. Similarly, the scope of the expression "a method comprising step A and step B" should not be limited to a method consisting only of steps A and B, rather with respect to the present invention, the only enumerated steps of the method are A and B, and further the claim should be interpreted as including equivalents of those steps.

In addition, reference to a feature by the indefinite article "a" or "an" does not exclude the possibility that more than one of the features such as for example a component, excipient, saponin, etc. are present, unless the context clearly requires that there is one and only one of the features. The indefinite article "a" or "an" thus usually means "at least one".

### BRIEF DESCRIPTION OF THE DRAWINGS

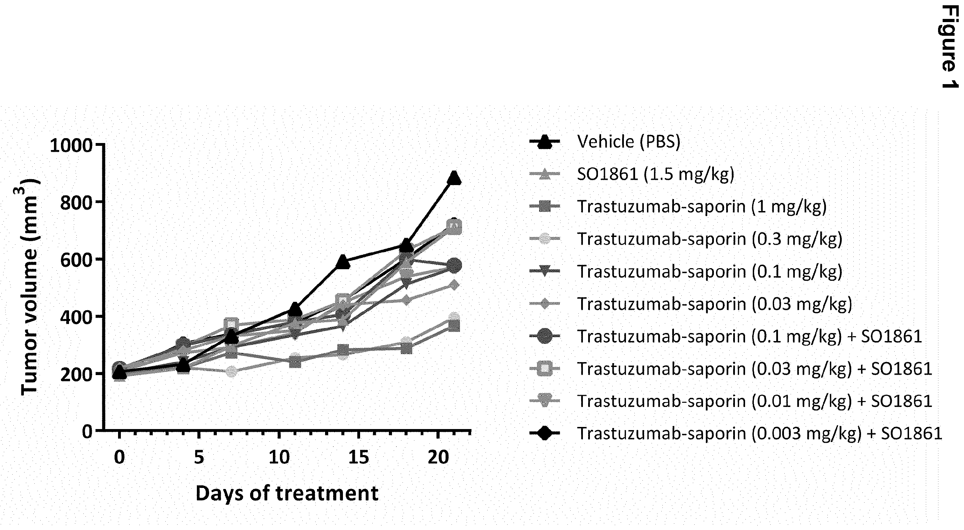

**Figure 1****.** *Antibody-protein toxin* + *unconjugated SO1861 vivo study.* BT474 tumor bearing mice treated with various concentrations of Trastuzumab-saporin (i.v.) + 1.5 mg/kg unconjugated SO1861 (subQ injection 1 hour before trastuzumab-saporin treatment).

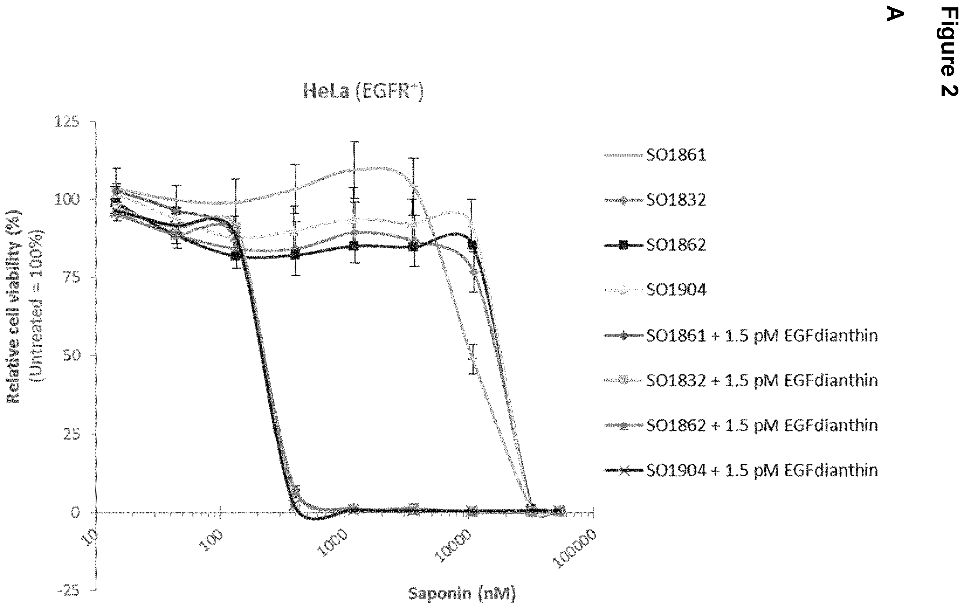

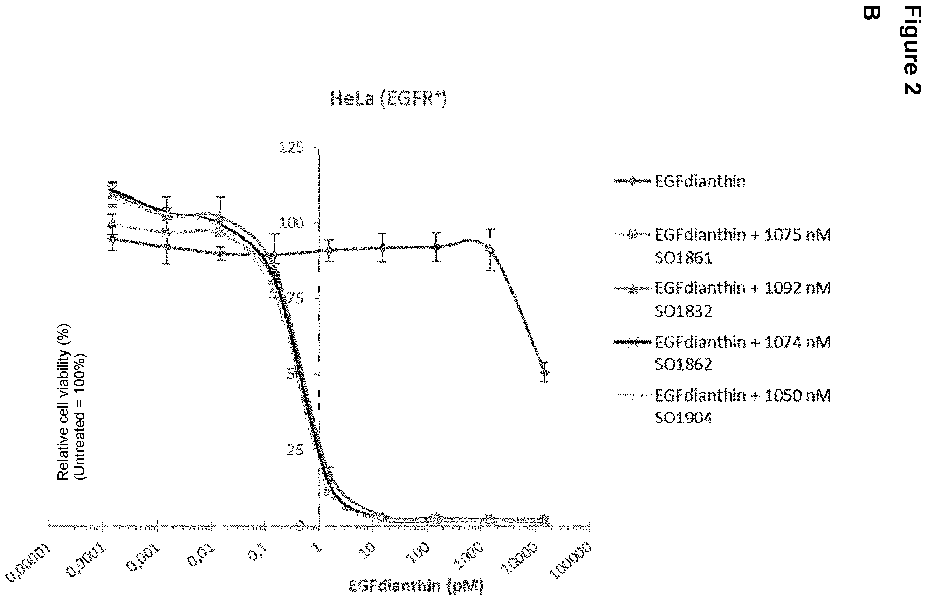

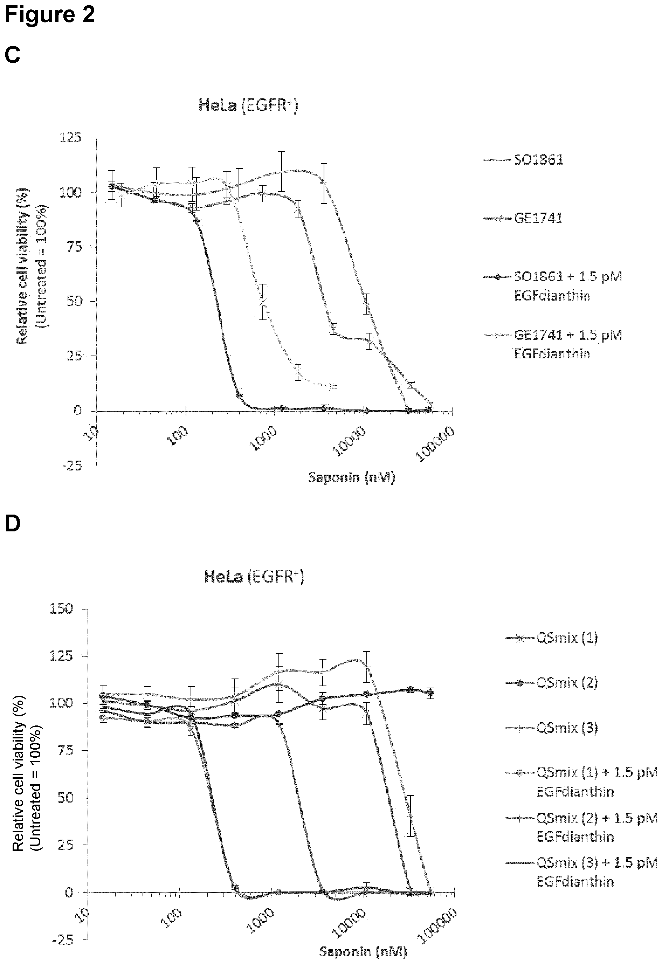

**Figure 2****.** *unconjugated saponin-mediated endosomal escape and target cell killing enhancement.* A) Cell viability analyses of HeLa cells (EGFR⁺) treated with SO1861, SO1832, SO1862 (isomer of SO1861) or SO1904 with or without 1.5 pM EGFdianthin B) Cell viability analyses of HeLa cells (EGFR⁺) treated with EGFdianthin and fixed concentrations of SO1861, SO1832, SO1862 (isomer of SO1861) or SO1904. The axes and legends of A) and B) are the same. C) Cell viability analyses of HeLa cells (EGFR⁺) treated with SO1861 or GE1741 with or without 1.5 pM EGFdianthin. D) Cell viability analyses of HeLa cells (EGFR⁺) treated with various QSmix (saponin mixture from Quillaia Saponaria) with or without 1.5 pM EGFdianthin. The Y-axes of C) and D) are the same.

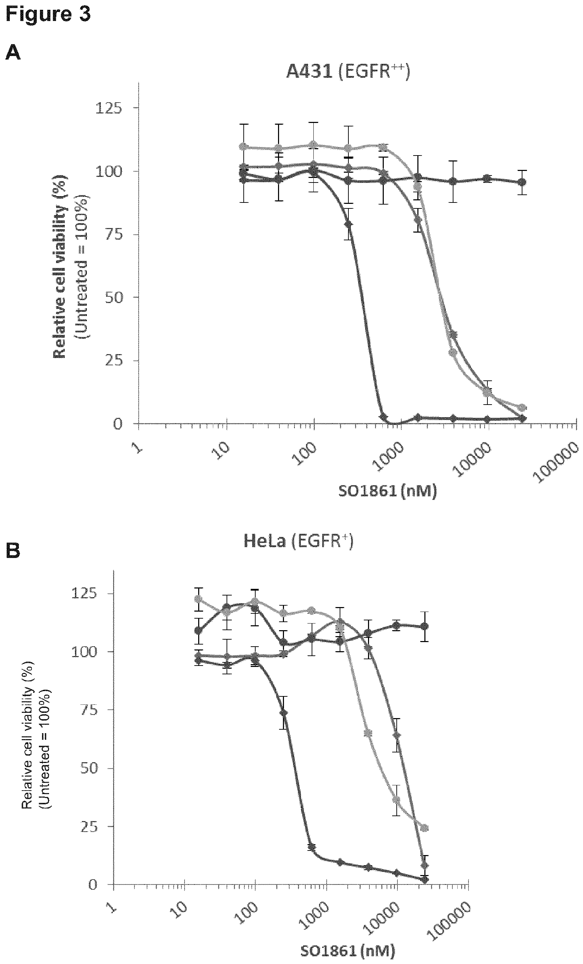

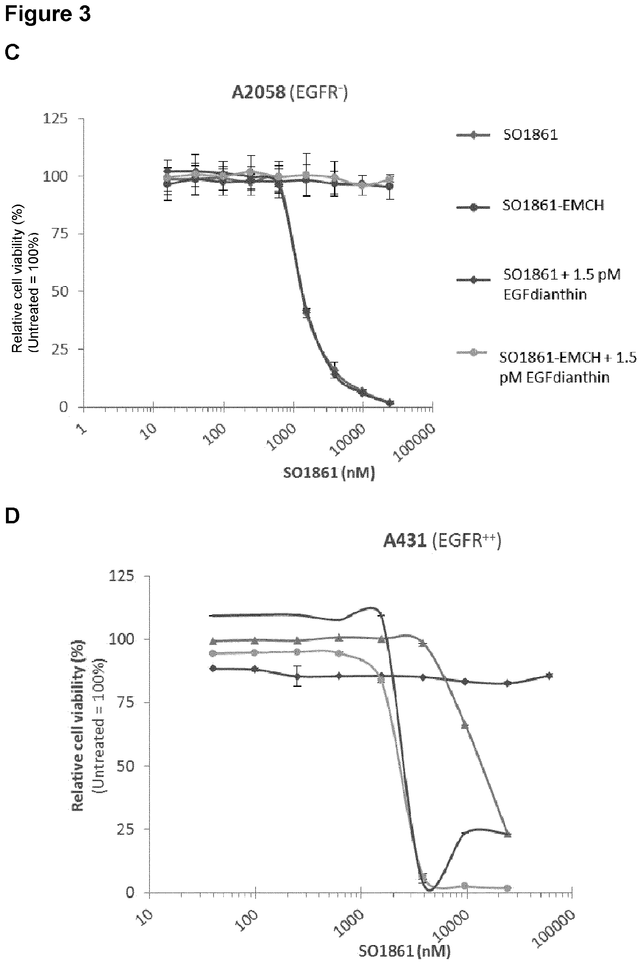

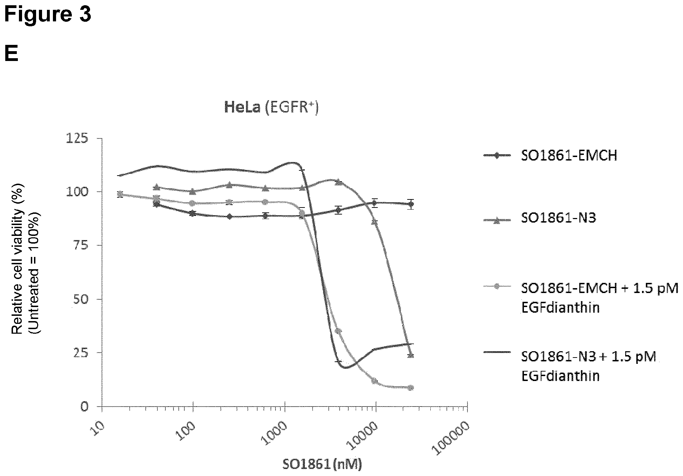

**Figure 3****.** *unconjugated SO1861 versus SO1861-EMCH activity. EGFR targeted antisense BNA oligo delivery and gene silencing in cancer cells, according to the invention.* A, B, C) Cell viability analyses of A431 (EGFR⁺⁺), HeLa (EGFR⁺) or A2058 (EGFR) cells treated with SO1861 or SO1861-EMCH with or without 1.5 pM EG dianthin. D, E) Cell viability analyses of A431 (EGFR⁺⁺) or HeLa (EGFR⁺) cells treated with SO1861 or SO1861-N3 with or without 1.5 pM EGFdianthin. The axes and legends of A), B), C), D) and E) are the same. That is to say, the legend for Figures 3A-C are displayed next to Figure 3C; the legend for Figure 3D and 3E is displayed next to Figure 3E.

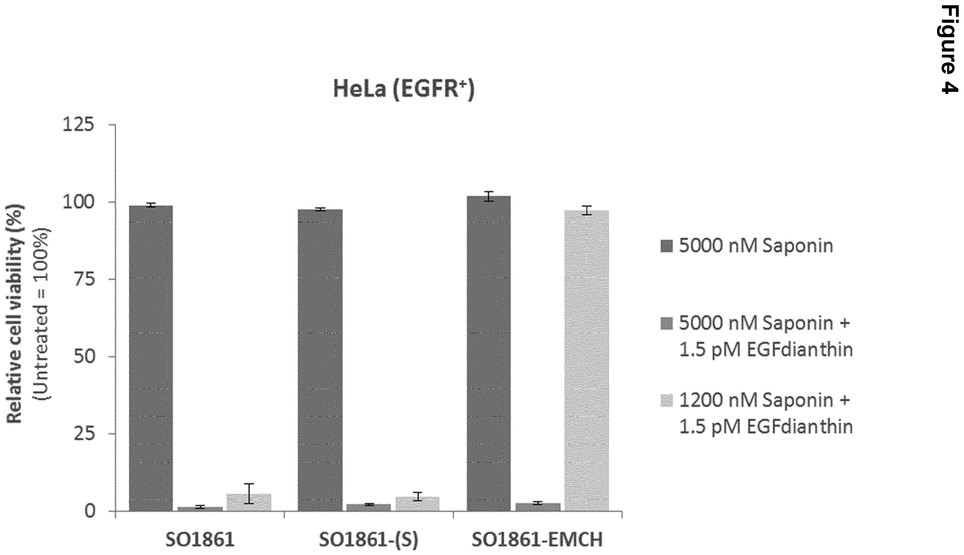

**Figure 4****.** *unconjugated SO1861 versus* SO1861-EMCH *(labile) versus SO1861-S (stable).* Cell viability analyses of HeLa cells (EGFR⁺) treated with SO1861, SO1861-S (S=HATU, stable linker) and SO1861-EMCH (labile linker) with or without EGFdiantin.

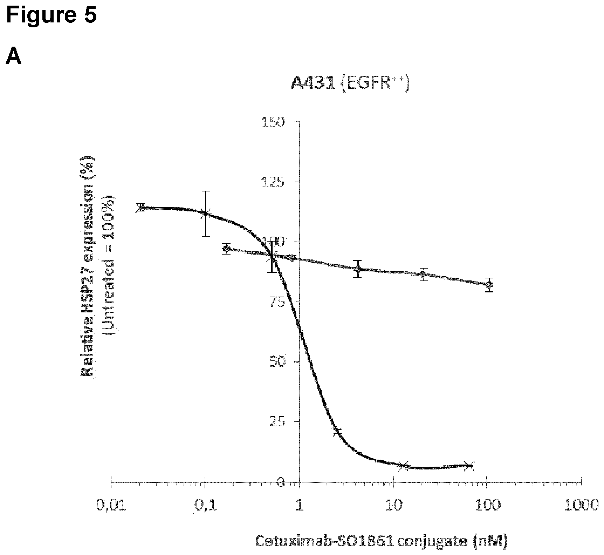

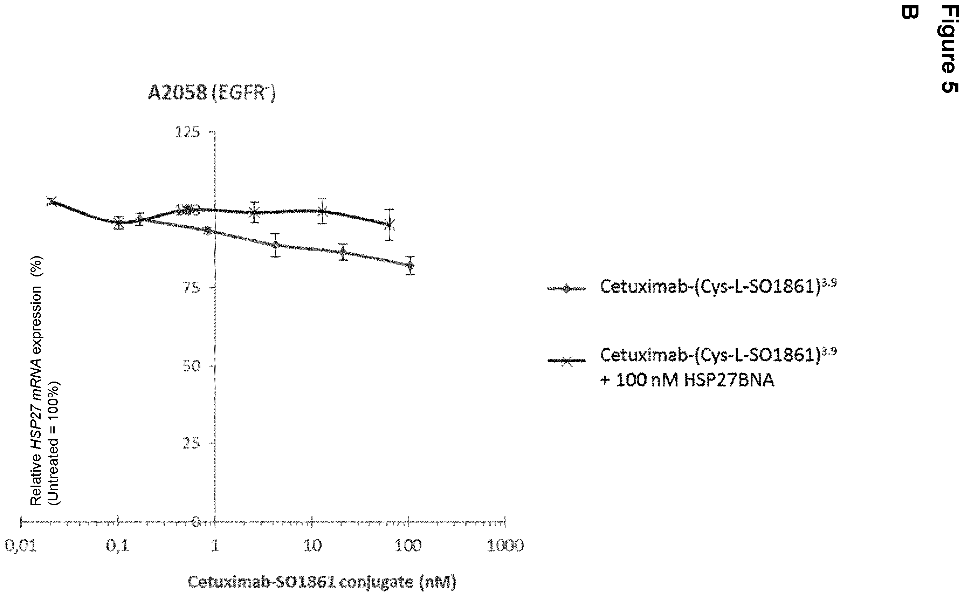

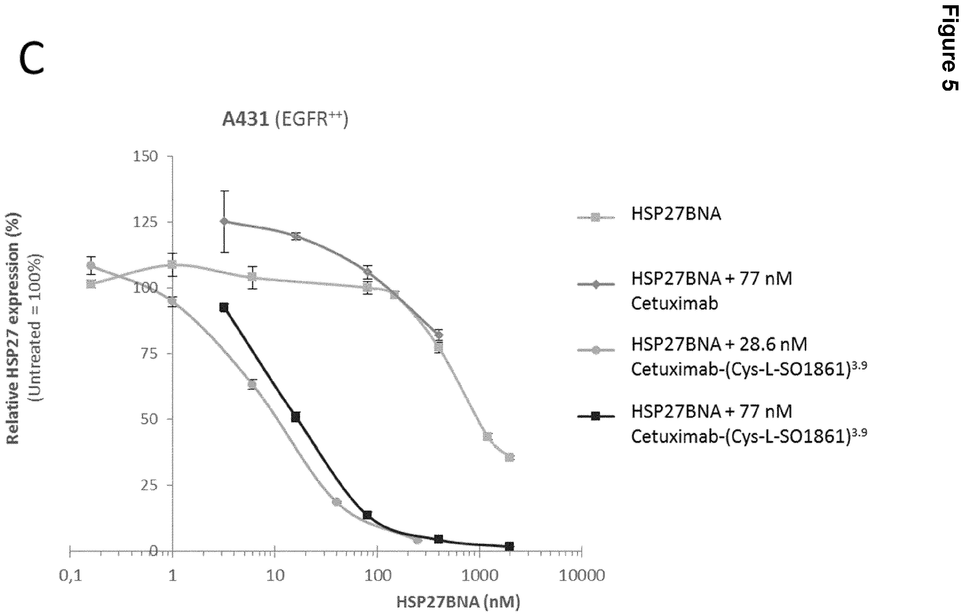

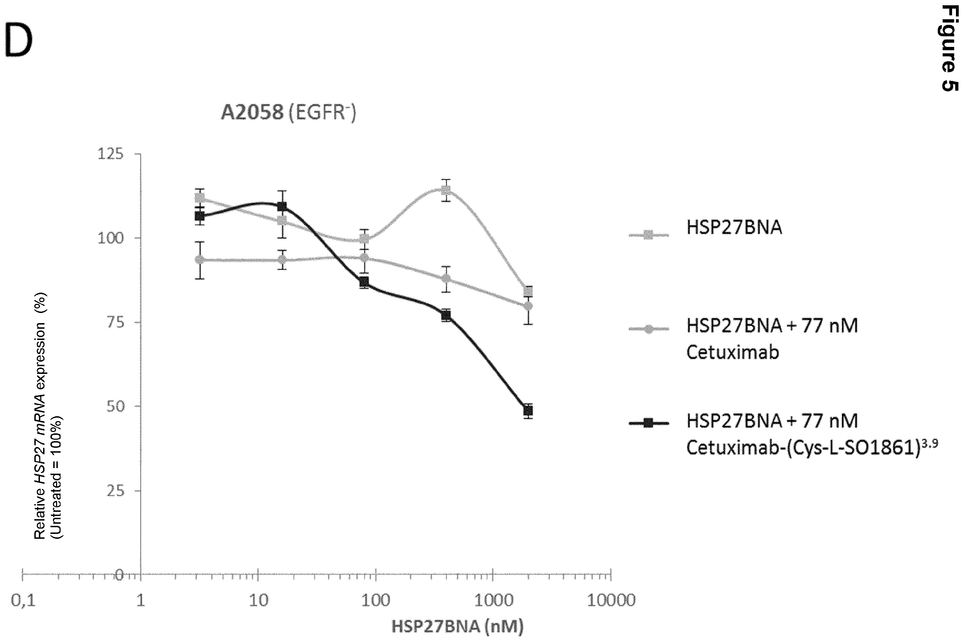

**Figure 5****.** *EGFR targeted antisense BNA oligonucleotide delivery and gene silencing.* HSP27 mRNA expression analyses of A431 (EGFR⁺⁺) and A2058 (EGFR) cells treated with cetuximab-(Cys-L-SO1861)^{3.9} or cetuximab-(Cys-L-SO1861)^{3.9} + 100nM HSP27BNA. The axes and legend of A) and B) are the same, and the legend is displayed next to Figure 5B. The Y-axis of C) and D) is the same.

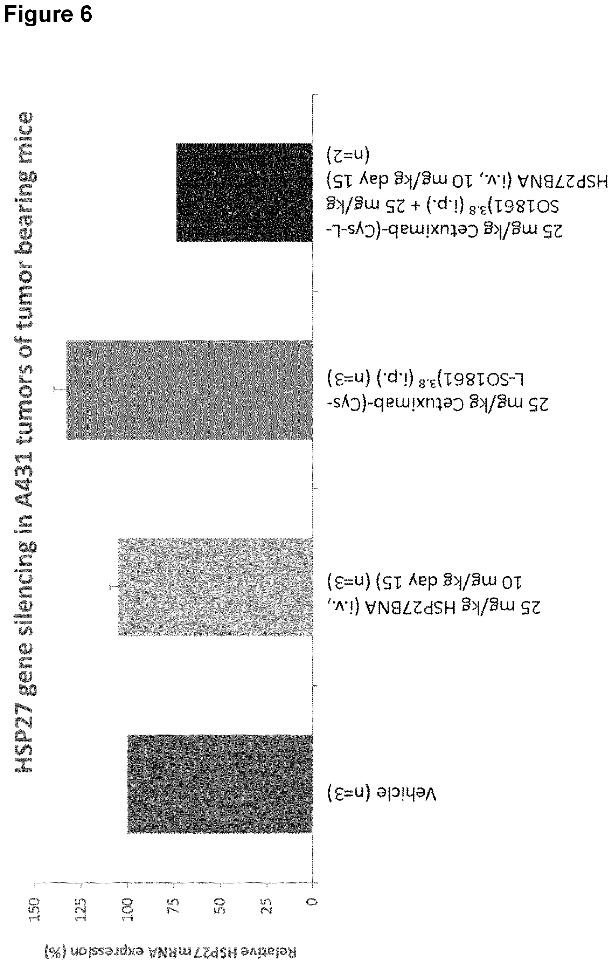

**Figure 6****.** *Tumor targeted antisense BNA oligo nucleotide delivery and gene silencing in tumor bearing mice.* Mice treated with HSP27BNA + cetuximab-(Cys-L-SO1861)^{3,9} in A431 tumor bearing mice reveals efficient tumor targeted gene silencing, compared to the controls.

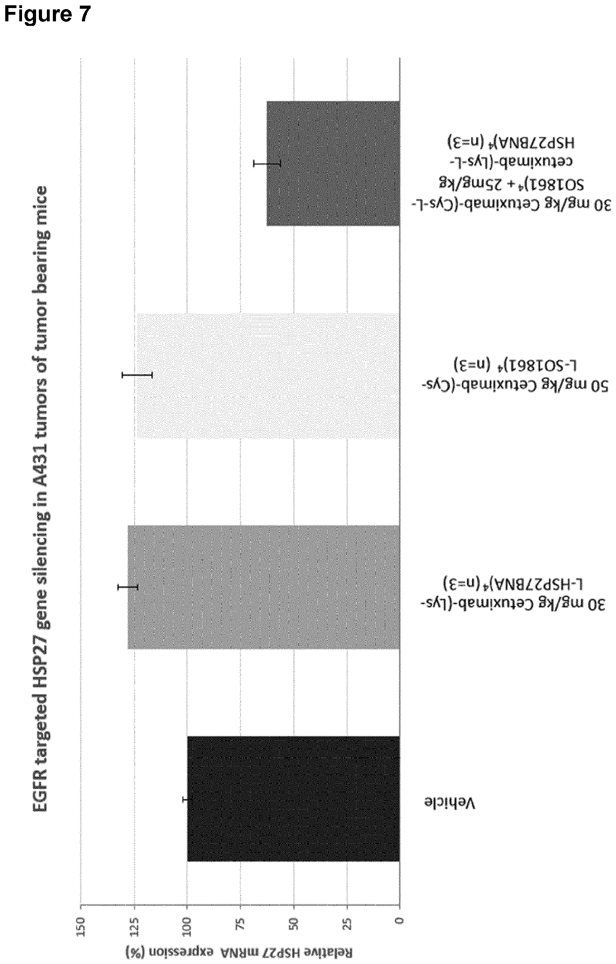

**Figure 7****.** *1T2C in vivo activity.* The 1T2C combination of 50mg/kg cetuximab-(Cys-L-SO1861)⁴ + 25 mg/kg cetuximab-(-L-HSP27BNA)⁴ in A431 tumor bearing mice reveals strong tumor targeted gene silencing, compared to the controls.

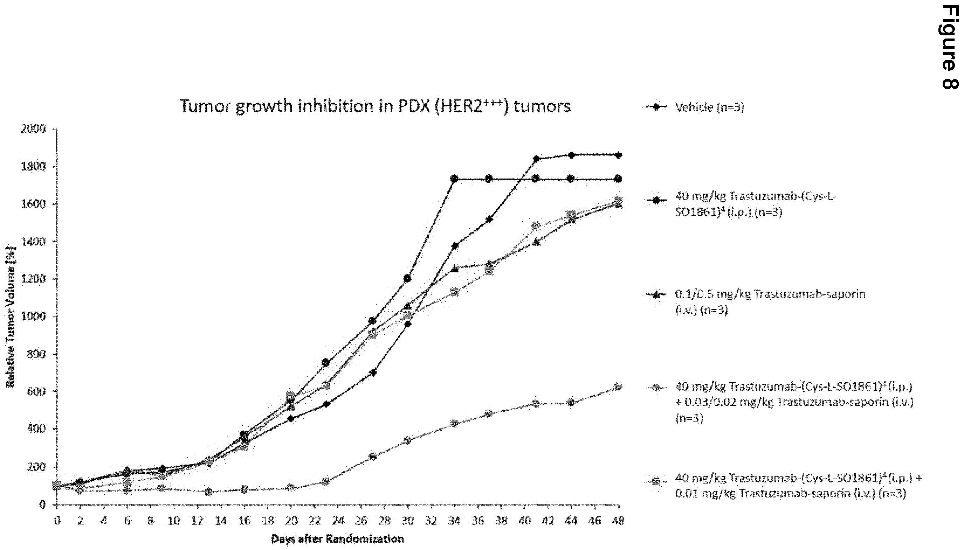

**Figure 8****.** *1T2C in vivo activity.* The 1T2C combination of 40 mg/kg trastuzumab-(Cys-L-SO1861)⁴ + 0.02/0.03 mg/kg trastuzumab-saporin in a PDX tumor mouse model (high HER2 expression) shows effective tumor growth inhibition.

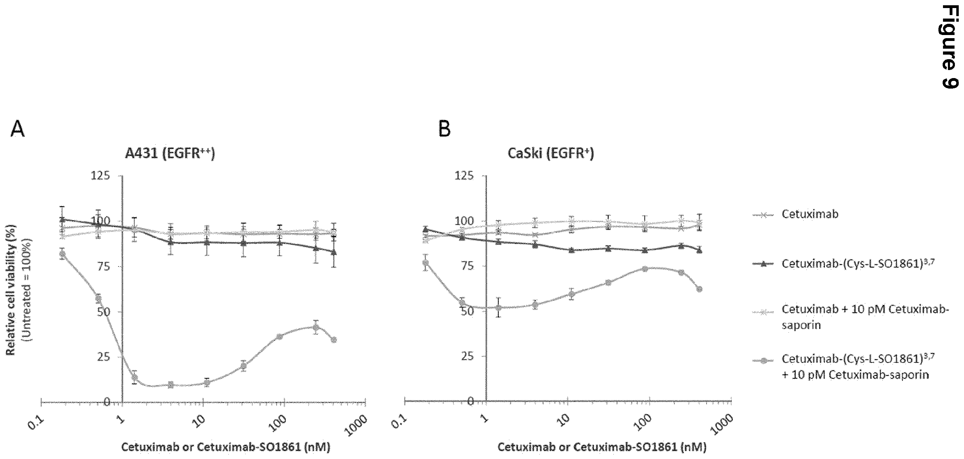

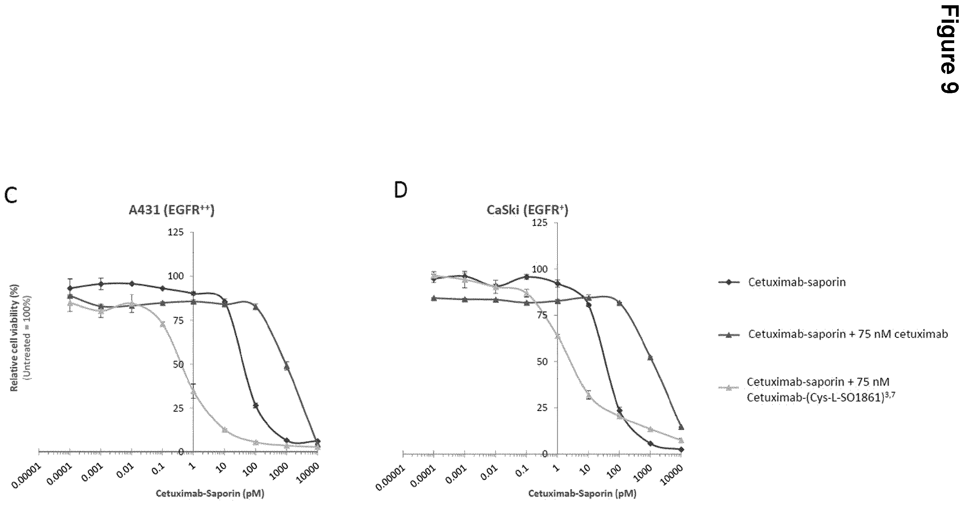

**Figure 9****.** *1-target 2-component.* EGFR targeted cell killing in A431 cells (EGFR⁺⁺) (A, C) and CaSKi cells (EGFR⁺) (B, D) by a therapeutic combination according to the invention. A, B) Cetuximab-(Cys-L-SO1861)^{3,7} titration + fixed concentration 10 pM cetuximab-saporin and controls on A431 (A) and CaSKi (B) cells. C, D) Cetuximab-saporin titration + fixed concentration of 75nM cetuximab-(Cys-L-SO1861)^{3,7} and controls on A431 (C) and CaSKi (D) cells.

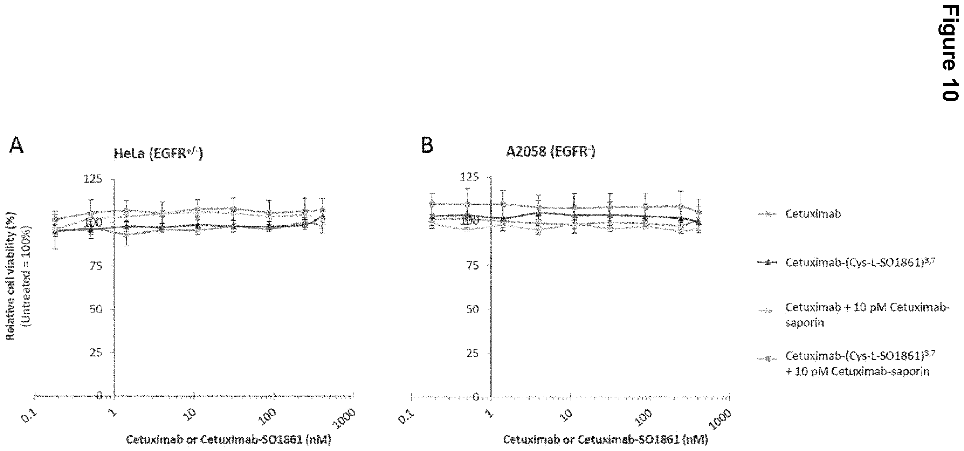

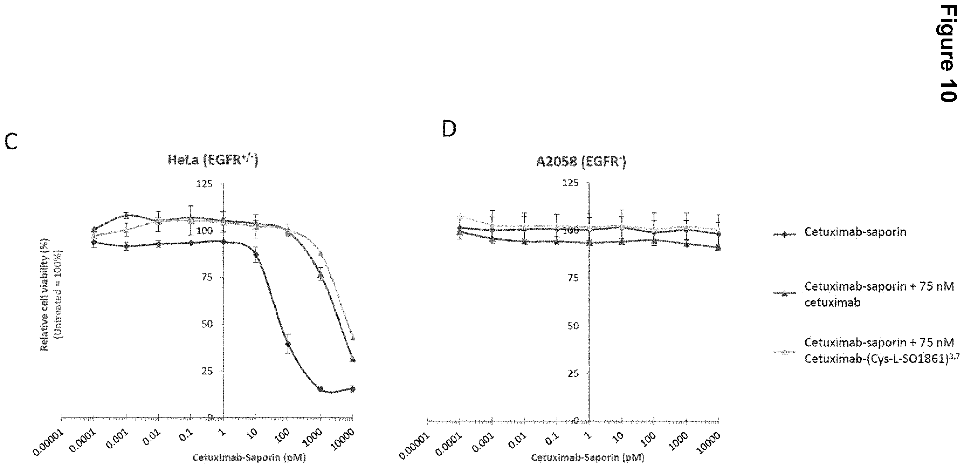

**Figure 10****.** *1-target 2-component.* EGFR targeted cell killing in HeLa cells (EGFR^{+/-}) (A, C) and A2058 cells (EGFR⁻) (B, D) by a therapeutic combination according to the invention. A, B) Cetuximab-(Cys-L-SO1861)^{3,7} titration + fixed concentration 10 pM cetuximab-saporin and controls on HeLa (A) and CaSKi (B) cells. C, D) Cetuximab-saporin titration + fixed concentration of 75nM cetuximab-(Cys-L-SO1861)^{3,7} and controls on Hela (C) and A2058 (D) cells.

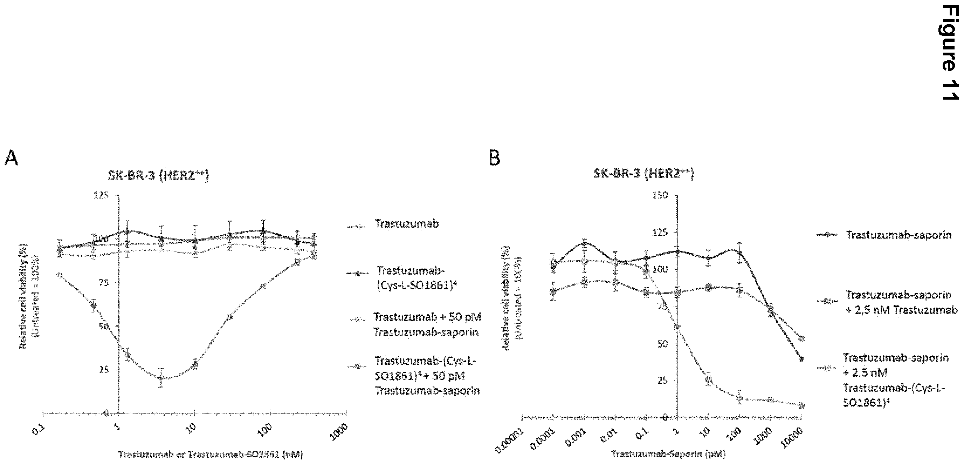

**Figure 11****:** *1-target 2-component.* HER2 targeted cell killing in SKBR3 cells (HER2⁺⁺) (A, B) by a therapeutic combination according to the invention. A) Trastuzumab-(Cys-L-SO1861)⁴ titration + fixed concentration 50 pM trastuzumab-saporin and controls on SKBR3 cells. B) Trastuzumab-saporin titration + fixed concentration of 2.5nM trastuzumab-(Cys-L-SO1861)⁴ and controls on SKBR3 cells.

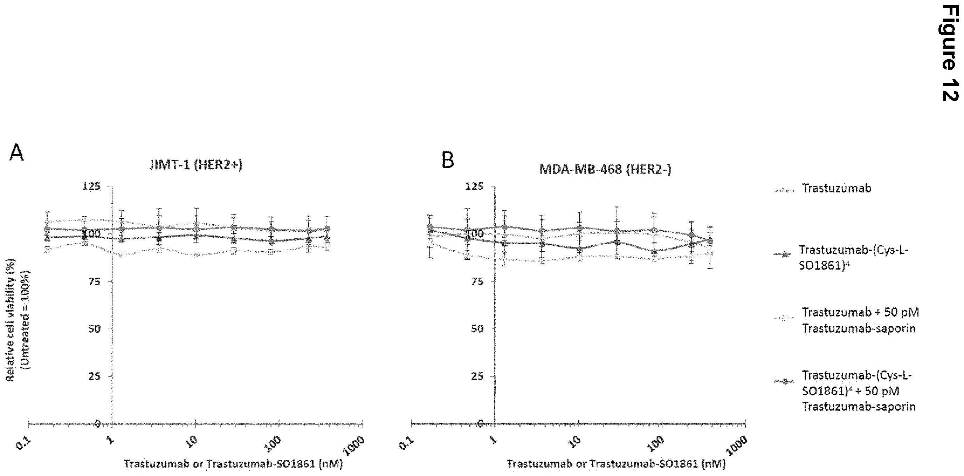

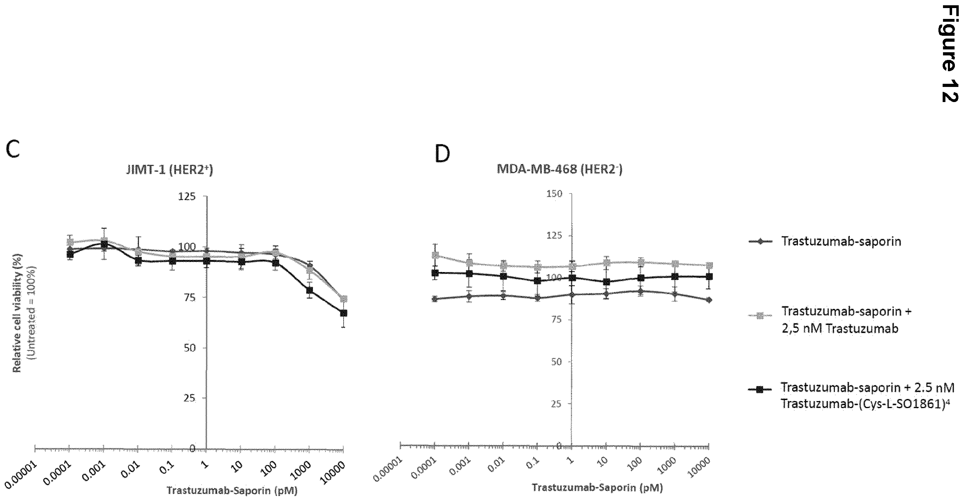

**Figure 12****.** *1-target 2-component.* HER2 targeted cell killing in JIMT-1 cells (HER2^{+/-}) (A, C) and MDA-MB-468 cells (HER2⁻) (B, D) by a therapeutic combination according to the invention. A, B) Trastuzumab-(Cys-L-SO1861)⁴ titration + fixed concentration of 50 pM trastuzumab-saporin and controls on JIMT-1 (A) and MDA-MB-468 (B) cells. C, D) Trastuzumab-saporin titration + fixed concentration of 2.5nM trastuzumab-(Cys-L-SO1861)⁴ and controls on JIMT-1 (C) and MDA-MB-468 (D) cells.

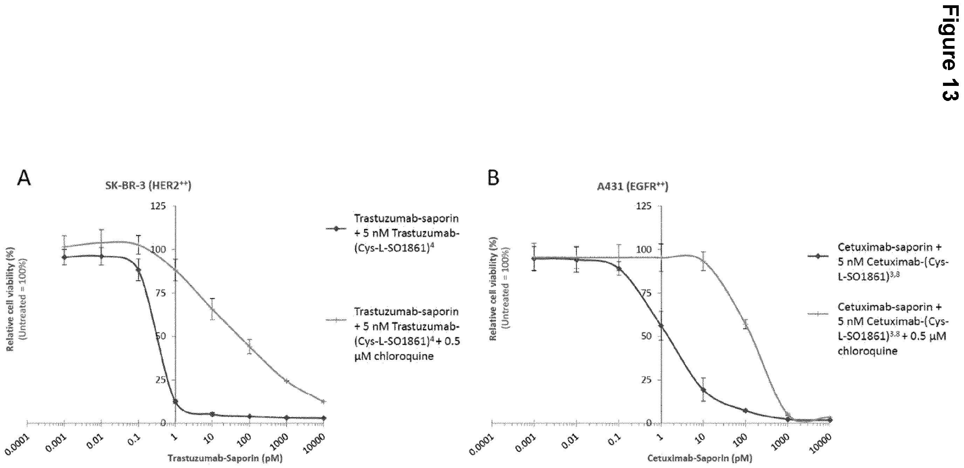

**Figure 13****:** *Chloroquine inhibits the 1-target 2-component.* HER2 and EGFR targeted cell killing in SK-BR-3 (HER2⁺⁺) and A431 cells (EGFR⁺⁺), by a therapeutic combination according to the invention + chloroquine. A) Trastuzumab-saporin titration + fixed concentration of 5nM trastuzumab-(Cys-L-SO1861)⁴ + 0.5 µM chloroquine and control on SK-BR-3 cells. B) Cetuximab-saporin titration + fixed concentration of 5 nM cetuximab-(Cys-L-SO1861)^{3,8} + 0.5 µM chloroquine and control on A431 cells.

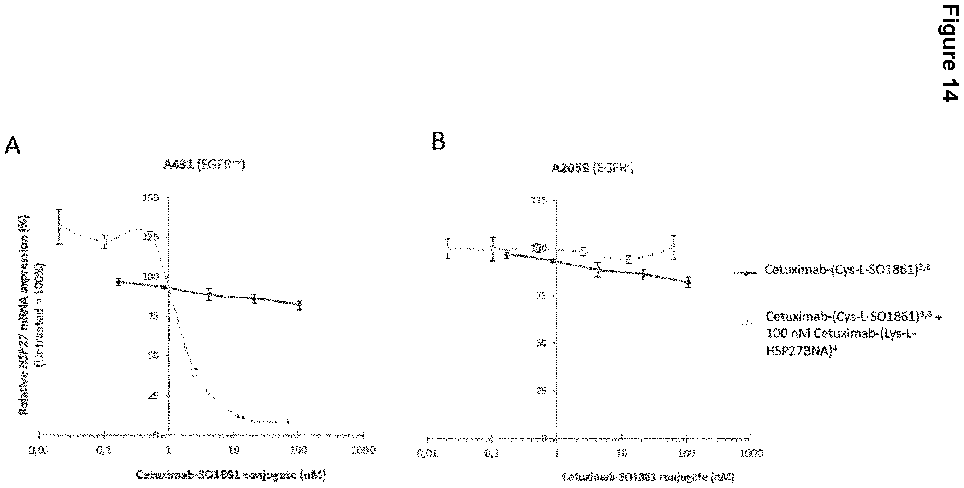

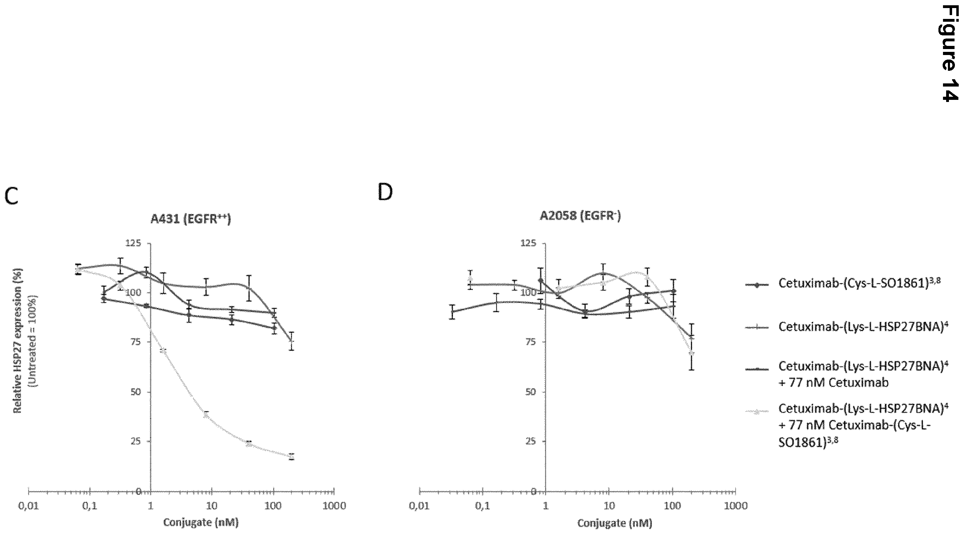

**Figure 14****:** *1-target 2-component.* EGFR targeted gene silencing in A431 cells (EGFR⁺⁺) and A2058 cells (EGFR⁻) by a therapeutic combination according to the invention. A,B) Cetuximab-(Cys-L-SO1861)^{3,8} titration + fixed concentration of 100 nM Cetuximab-(Lys-L-HSP27BNA)⁴ and control on A431 cells (A) and A2058 cells (B). C, D) Cetuximab-(Lys-L-HSP27BNA)⁴ titration + fixed concentration of 77 nM Cetuximab-(Cys-L-SO1861)^{3,8} and control on A431 cells (C) and A2058 cells (D).

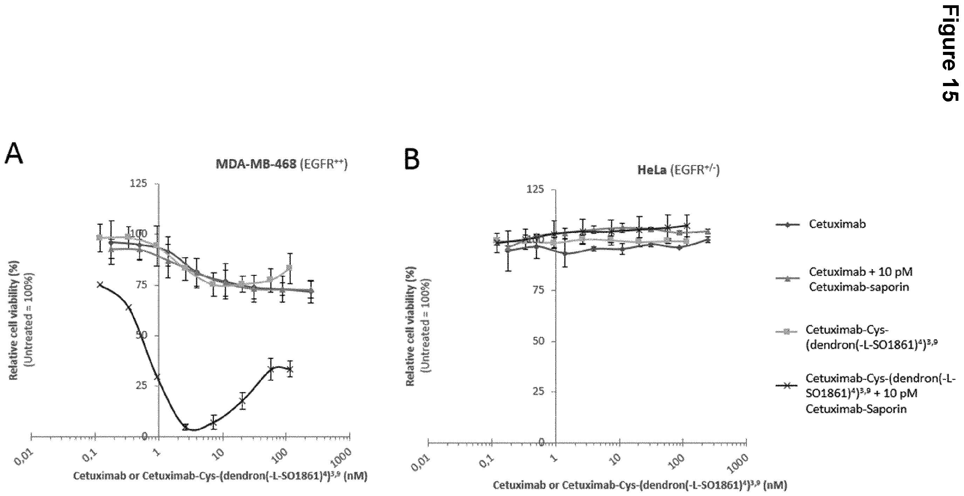

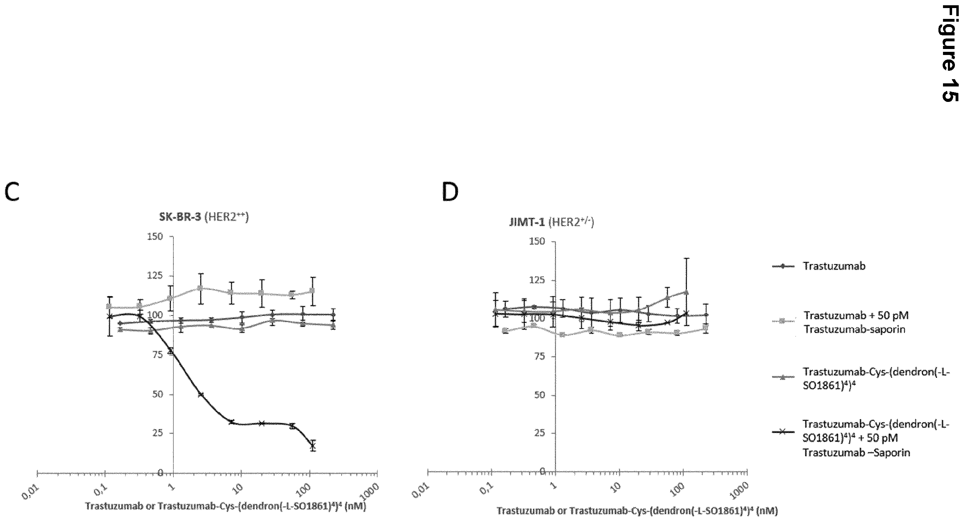

**Figure 15****:** *2-target 2-component.* A) EGFR and HER2 targeted cell killing in MDA-MB-468 cells (EGFR⁺⁺) and HeLa cells (EGFR^{+/-}) and HER2 targeted cell killing in SK-BR-3 cells (HER2⁺⁺) and JIMT-1 cells (HER2^{+/-}) by a therapeutic combination according to the invention. A) Cetuximab-Cys-(dendron(-L-SO1861)⁴)^{3,9} titration + fixed concentration 10 pM cetuximab-saporin and controls on MDA-MB-468 cells (A) and HeLa cells (B). C,D) Trastuzumab-(Cys-(dendron(-L-SO1861)⁴)⁴ titration + fixed concentration 50 pM trastuzumab-saporin and controls on SK-BR-3 cells (C) and JIMT-1 cells (D).

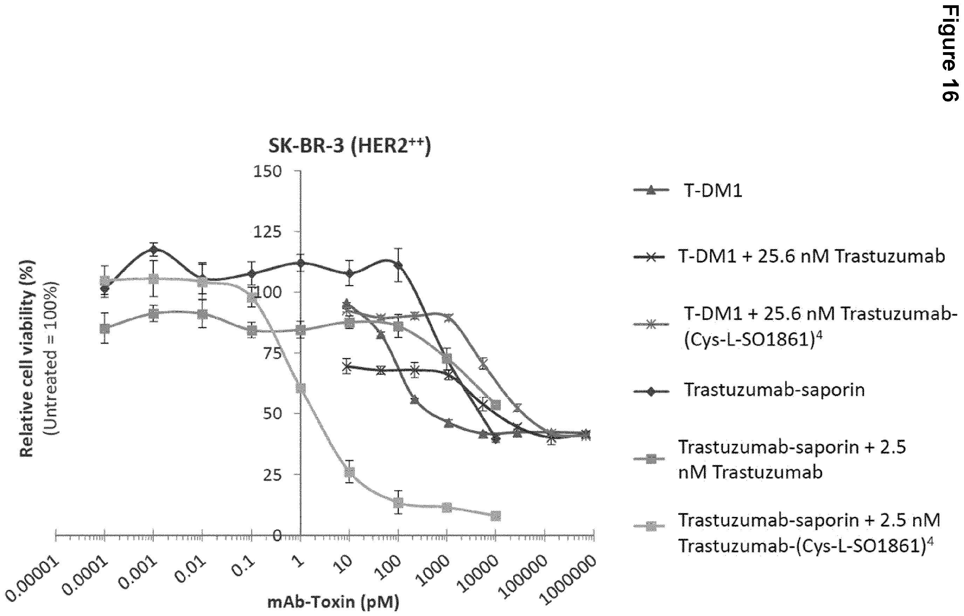

**Figure 16****.** *1-target 2-component.* SK-BR-3 cells (HER2^{+/-}) can efficiently be killed with the therapeutic combination according to the invention, Tratuzumab-saporin + 2.5 nM trastuzumab-(Cys-L-SO1861)⁴, however titration of T-DM1 + 2.5 nM trastuzumab-(Cys-L-SO1861)⁴ is not effective at such low toxin concentrations. T-DM1 is Trastuzumab-emtansine (Kadcyla®), carrying ∼3.5 emtansine (DM1) toxin molecules per antibody (DAR3.5).

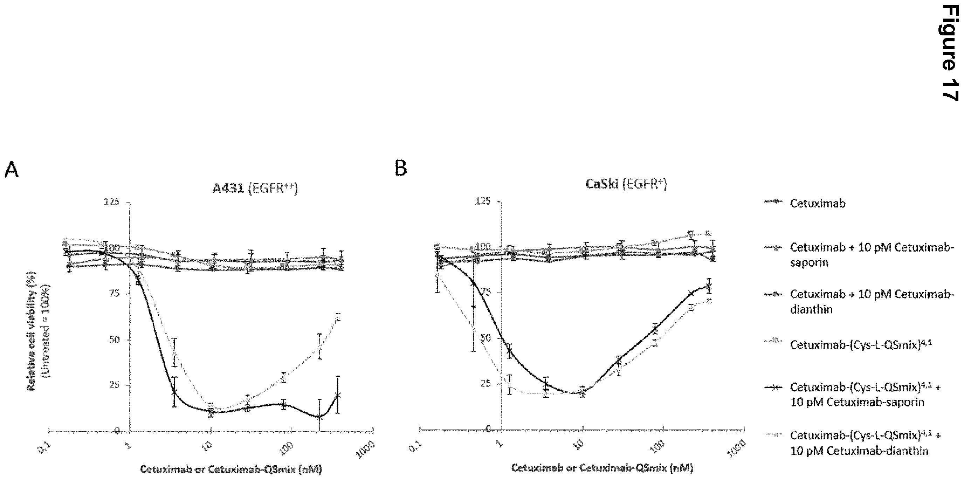

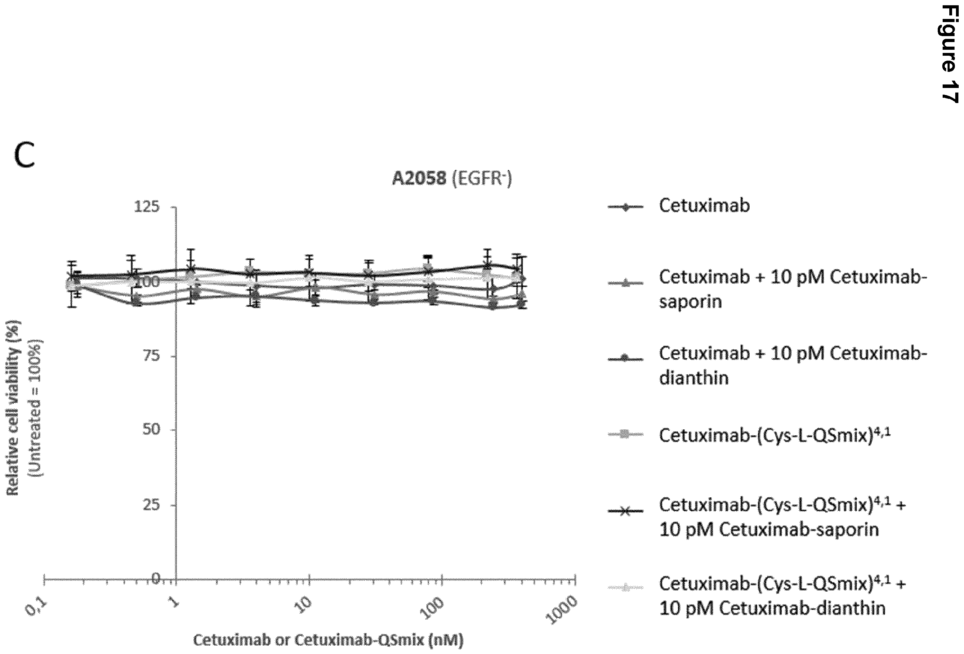

**Figure 17****.** *1-target 2-component.* EGFR targeted cell killing in A431 cells (EGFR⁺⁺) (A) and CaSKi cells (EGFR⁺) (B) and A2058 cells (EGFR⁻) by a therapeutic combination according to the invention. A, B, C) Cetuximab-(Cys-L-QSmix)^{4,1} titration + fixed concentration 10 pM cetuximab-saporin or 10pM cetuximab-dianthin and controls in A431 cells (A), CaSKi cells (B) and A2058 cells (C). QSmix is a mixture of saponins from an extract Quillaja Saponaria.

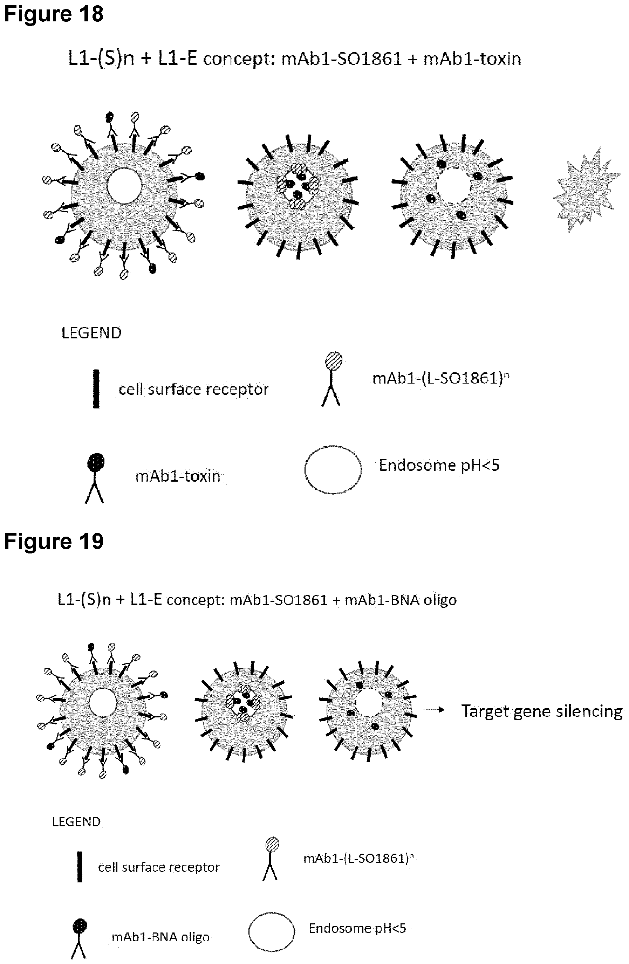

**Figure 18****:** *1-target 2-component concept: mAb1-SO1861* + *mAb1-protein toxin.* SO1861 and toxin (ribosomal inactivating protein) are each, independently, conjugated to an antibody (mAb1) for delivery and internalization into target cells. 1) mAb1-SO1861 and mAb1-protein toxin bind to the cell surface receptor, 2) receptor-mediated endocytosis of both conjugates occurs, 3) at low endolysosomal pH and appropriate concentration, SO1861 becomes active to enable endolysosomal escape, 4) release of toxin into cytoplasm occurs and 5) toxin induces cell death
**Figure 19****:** *1-target 2-component concept: mAb1-SO1861* + *mAb2-BNA oligo.* SO1861 and antisense BNA oligo nucleotide are each, independently, conjugated to an antibody (mAb1) for delivery and internalization into target cells. 1) mAb1-SO1861 and mAb1-BNAoligo bind to the cell surface receptor, 2) receptor-mediated endocytosis of both conjugates occurs, 3) at low endolysosomal pH and appropriate concentration, SO1861 becomes active to enable endolysosomal escape, 4) release of BNA oligo into cytoplasm occurs and 5) target gene silencing.

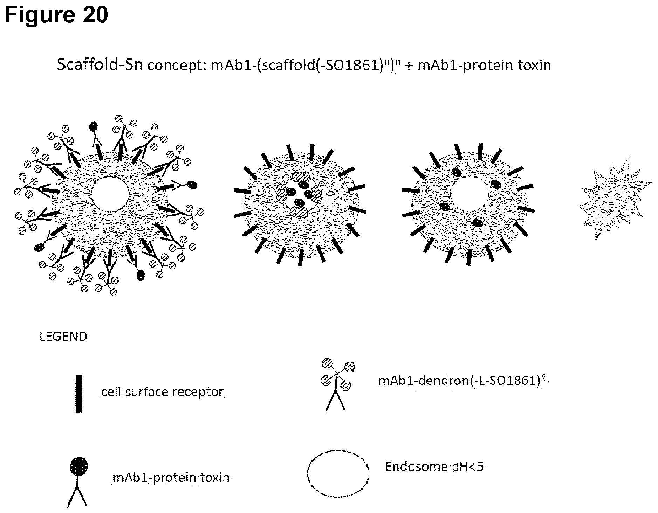

**Figure 20****:** *1-target 2-component concept: mAb1-(scaffold(-SO1861)ⁿ)ⁿ* + *mAb1-protein toxin.* Dendron(-SO1861)ⁿ and protein toxin (ribosomal inactivating protein) are each, independently, conjugated to an antibody (mAb1) for delivery and internalization into target cells. 1) mAb1-dendron(-SO1861)⁴ and mAb1-protein toxin bind to the cell surface receptor, 2) receptor-mediated endocytosis of both conjugates occurs, 3) at low endolysosomal pH and appropriate concentration, SO1861 becomes active to enable endolysosomal escape, 4) release of toxin into cytoplasm occurs and 5) toxin induces cell death

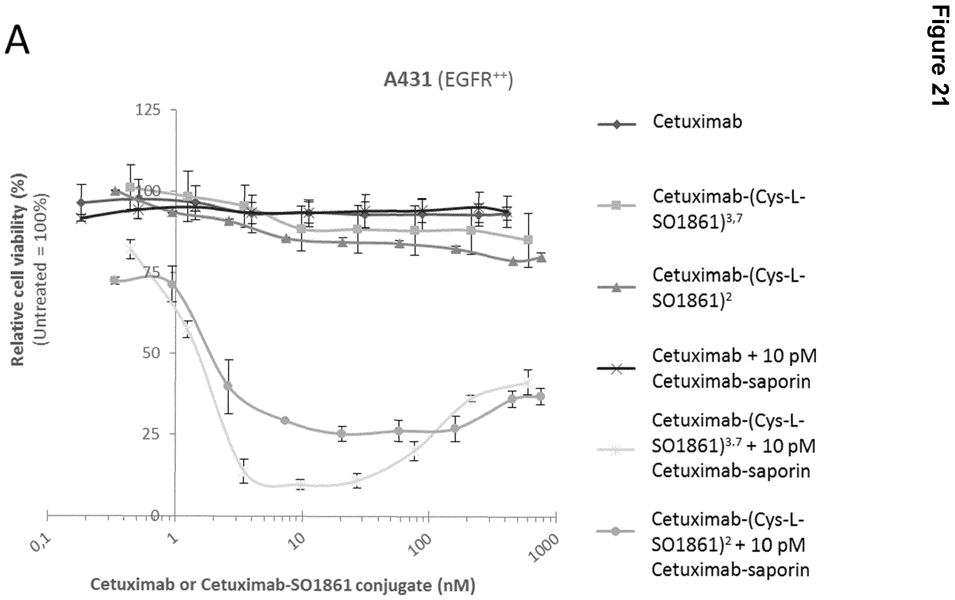

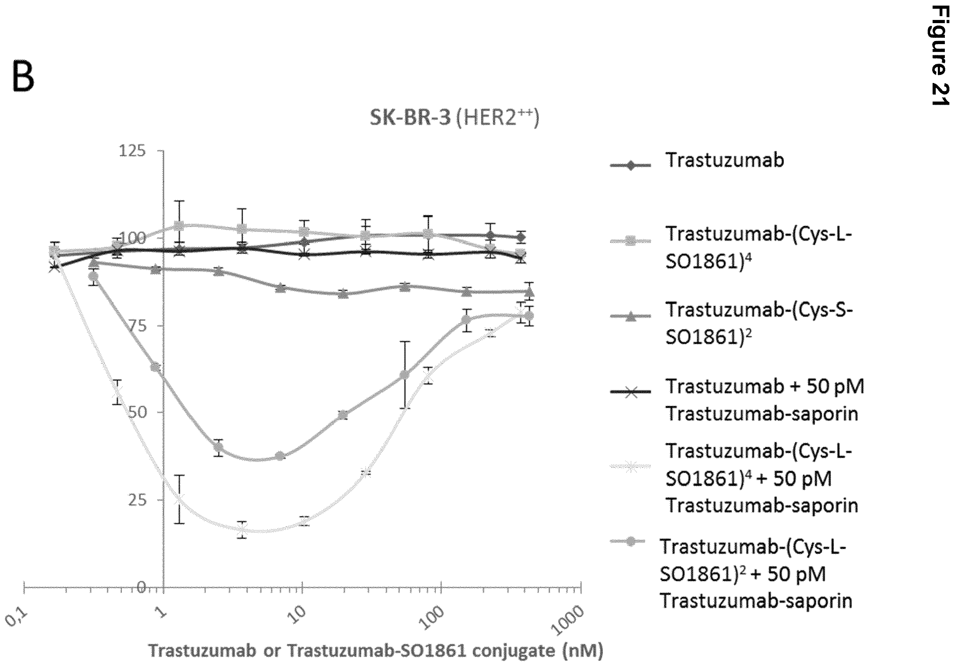

**Figure 21****:** *antibody-(-L-SO1861)⁴ vs antibody-(-L-SO1861)².* HER2 and EGFR targeted cell killing in A431 cells (EGFR⁺⁺) and SK-BR-3 (HER2⁺⁺), by a therapeutic combination according to the invention A) cetuximab-(-L-SO1861)⁴ + 10 pM cetuximab-saporin compared to cetuximab-(-L-SO1861)² + 10 pM cetuximab-saporin in A431 cells. B) Trastuzumab-(-L-SO1861)⁴ + 50 pM trastuzumab-saporin compared to trastuzumab-(-L-SO1861)² + 50 pM trastuzumab-saporin in SK-BR-3 cells.

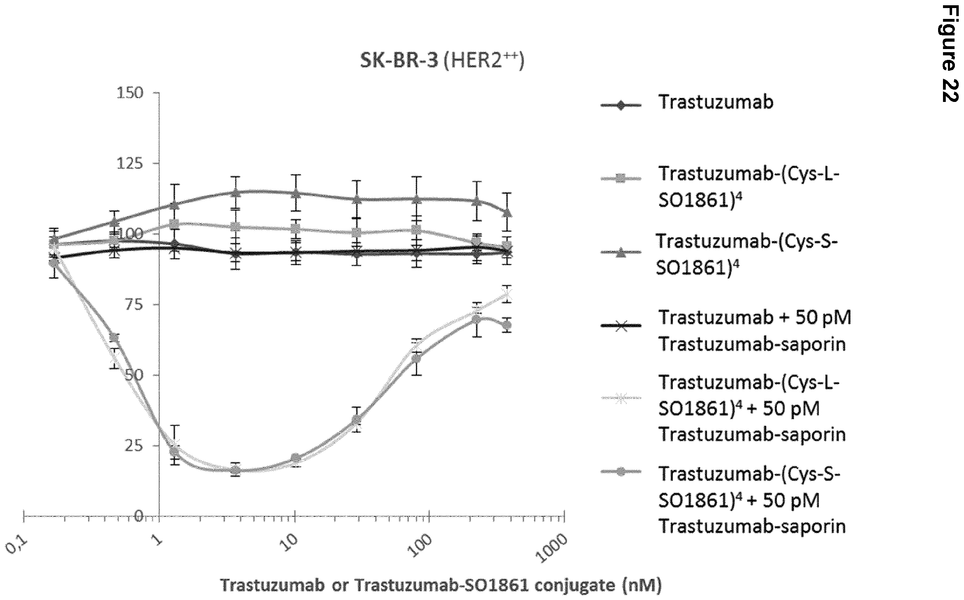

**Figure 22****:** *antibody-(-L-SO1861)⁴ vs antibody-(-S-SO1861)⁴.* HER2 targeted cell killing in SK-BR-3 (HER2⁺⁺), by a therapeutic combination according to the invention. B) Trastuzumab-(-L-SO1861)⁴ + 50 pM trastuzumab-saporin compared to trastuzumab-(-S-SO1861)⁴ + 50 pM trastuzumab-saporin in SK-BR-3 cells.

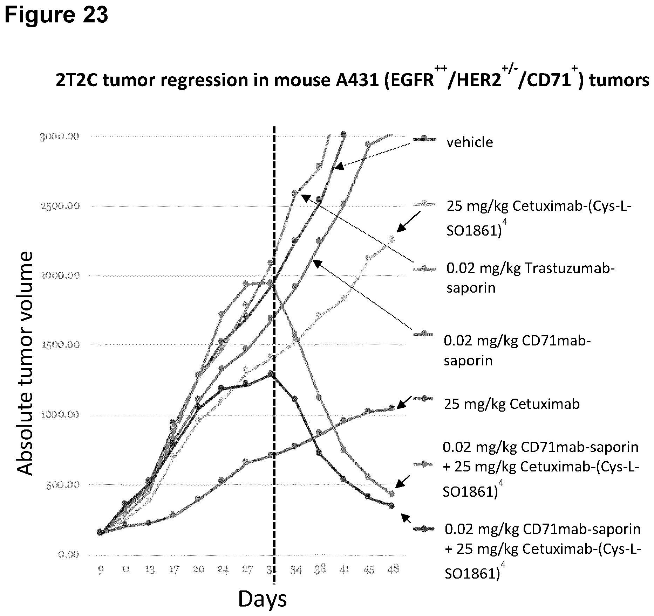

**Figure 23****.** *The 2T2 component system tested in A431 tumor bearing mice model reveals tumor regression.*

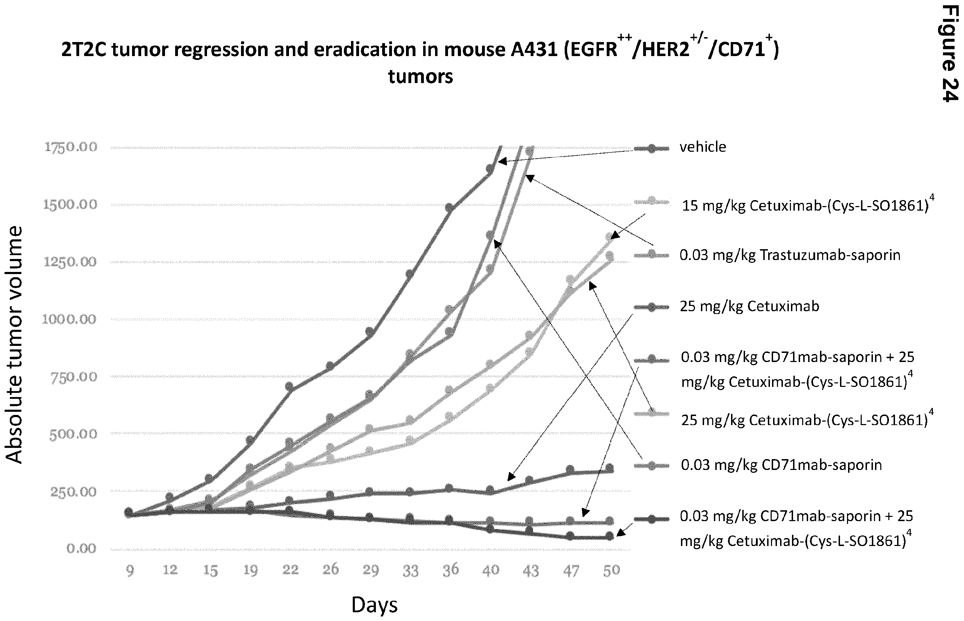

**Figure 24****.** *The 2T2 component system tested in A431 tumor bearing mice model reveals tumor regression and eradication.*

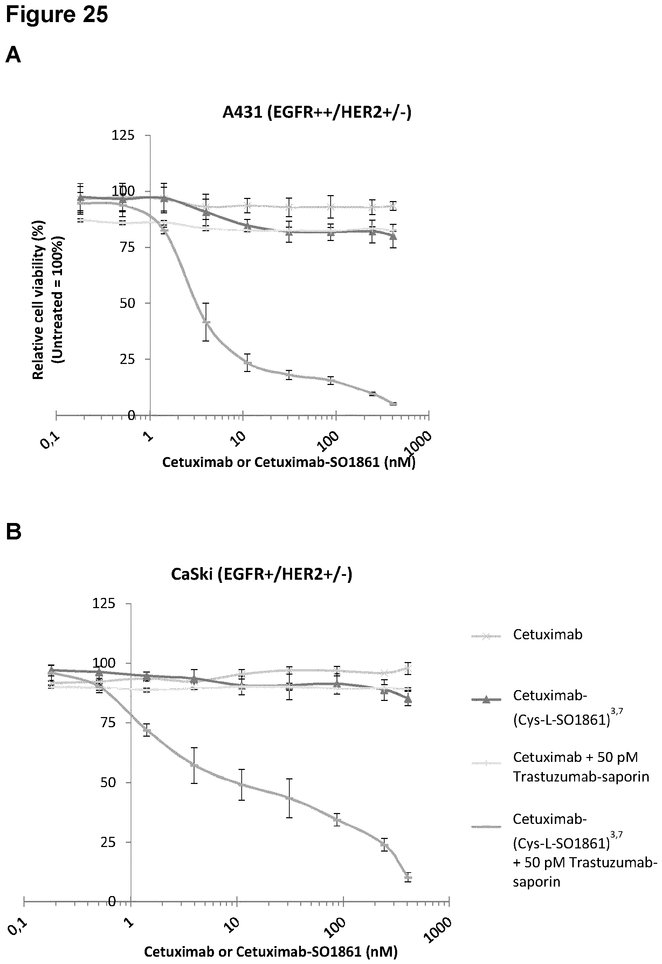

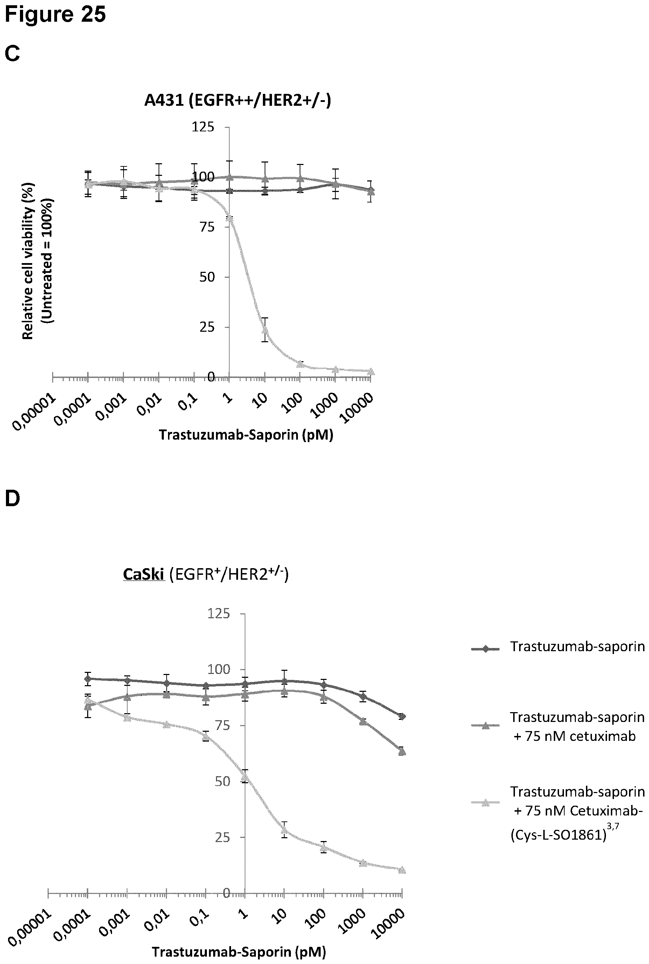

**Figure 25****:** *2-target 2-component.* EGFR/HER2 targeted cell killing in A431 cells (EGFR⁺⁺/HER2^{+/-}) (A, C) and CaSKi cells (EGFR⁺⁺/HER2^{+/-}) (B, D) by a therapeutic combination according to the invention. A, B) Cetuximab-(Cys-L-SO1861)^{3,7} titration + fixed concentration 50 pM trastuzumab-saporin and controls on A431 cells. C, D) Trastuzumab-saporin titration + fixed concentration of 75nM cetuximab-(Cys-L-SO1861)^{3,7} and controls on Caski cells. The axes and legends are the same for A) and B), and C) and D). That is to say, the legend for Figures 25A and B is displayed next to Figure 25B; the legend for Figure 25C and 25D is displayed next to Figure 25D.

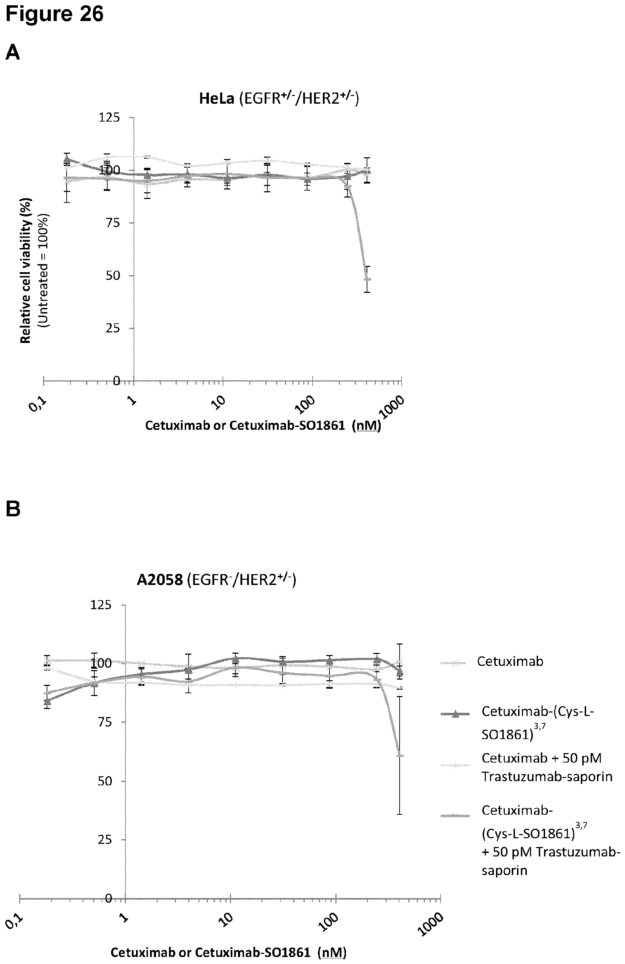

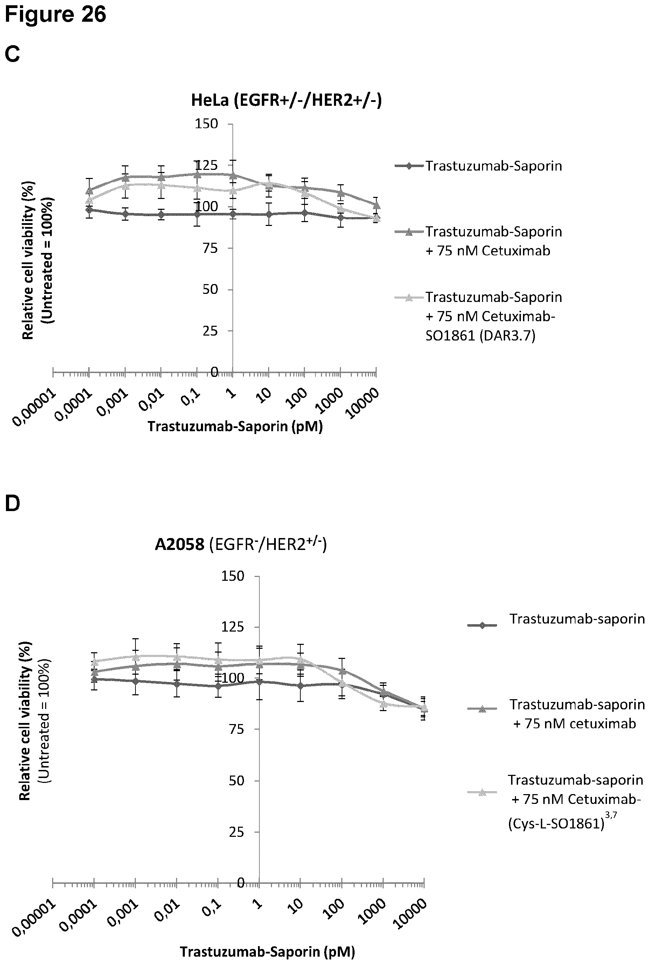

**Figure 26****.** *2-target 2-component.* EGFR/HER2 targeted cell killing in HeLa cells (EGFR^{+/-}/HER2^{+/-}) (A, C) and A2058 cells (EGFR⁻/HER2^{+/-}) (B, D) by a therapeutic combination according to the invention. A, B) Cetuximab-(Cys-L-SO1861)^{3,7} titration + fixed concentration 50 pM trastuzumab-saporin and controls on HeLa cells. C, D) Trastuzumab-saporin titration + fixed concentration of 75nM cetuximab-(Cys-L-SO1861)^{3,7} and controls on A2058 cells. The axes and legends are the same for A) and B). That is to say, the legend for Figures 26A and B is displayed next to Figure 26B.

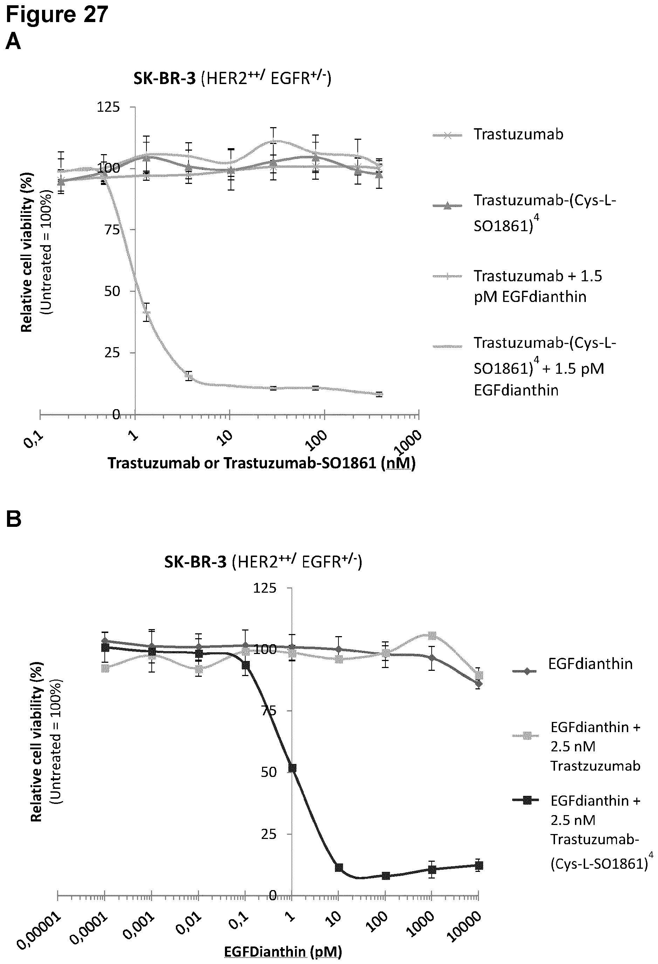

**Figure 27****:** *2-target 2-component.* HER2/EGFR targeted cell killing in SKBR3 cells (HER2⁺⁺/EGFR^{+/-}) (A, B) by a therapeutic combination according to the invention. A Trastuzumab-(Cys-L-SO1861)⁴ titration + fixed concentration 1.5 pM EGFdianthin and controls on SKBR3 cells. B) EGFdianthin titration + fixed concentration of 2.5nM trastuzumab-(Cys-L-SO1861)⁴ and controls on SKBR3 cells.

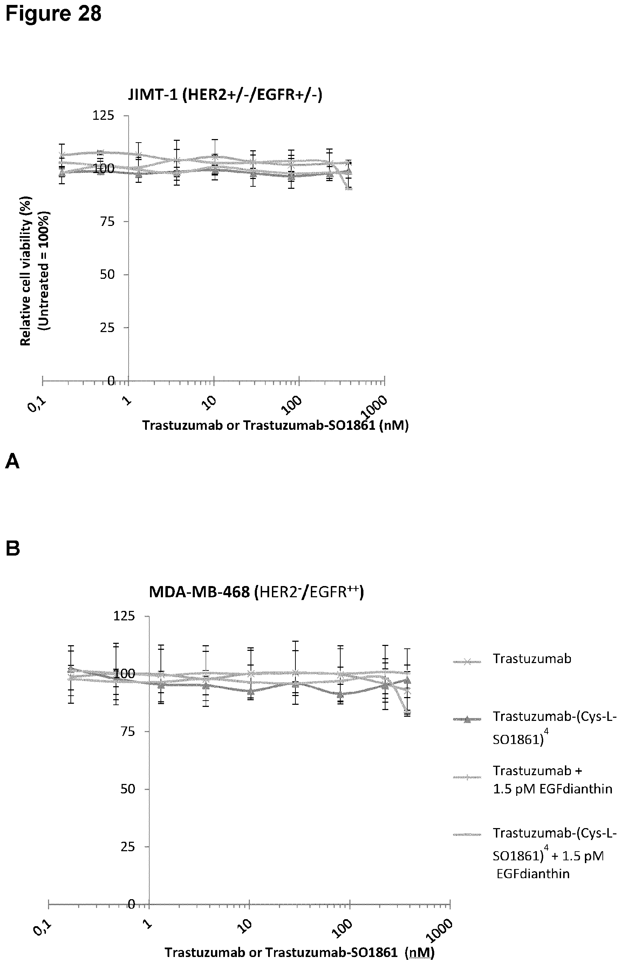

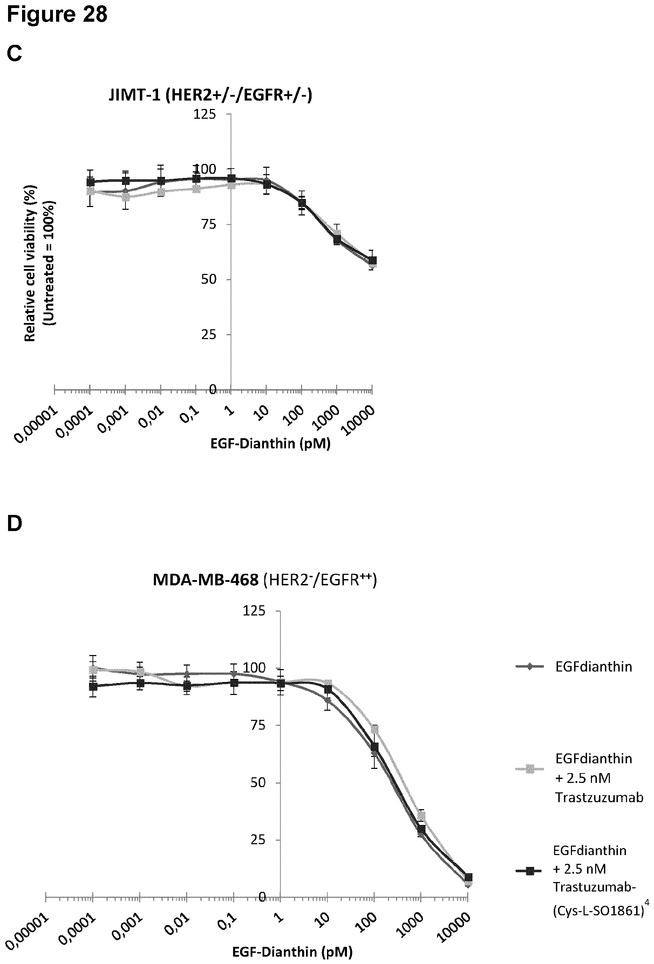

**Figure 28****.** *2-target 2-component.* HER2/EGFR targeted cell killing in JIMT-1 cells (HER2^{+/-}EGFR^{+/-}) (A, C) and MDA-MB-468 cells (HER2⁻/EGFR⁺⁺) (B, D) by a therapeutic combination according to the invention. A, B) Trastuzumab-(Cys-L-SO1861)⁴ titration + fixed concentration 1.5 pM EGFdianthin and controls on JIMT-1 cells. C, D) EGFdianthin titration + fixed concentration of 2.5nM trastuzumab-(Cys-L-SO1861)⁴ and controls on MDA-MB-468 cells. The axes and legends are the same for A) and B), and C) and D). That is to say, the legend for Figures 28A and B is displayed next to Figure 28B; the legend for Figure 28C and 28D is displayed next to Figure 28D.

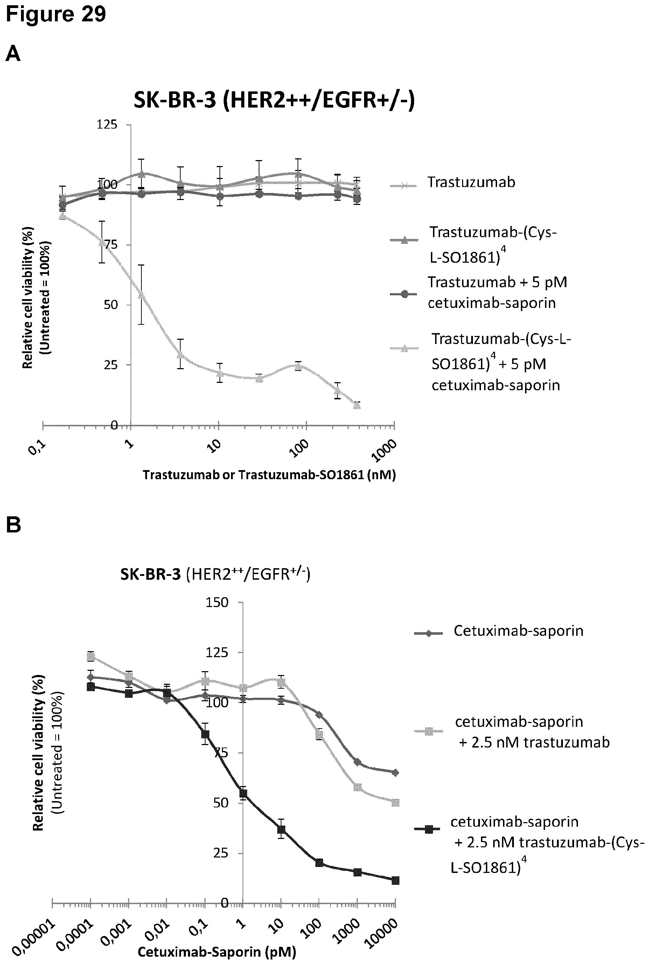

**Figure 29****:** *2-target 2-component.* HER2/EGFR targeted cell killing in SKBR3 cells (HER2⁺⁺/EGFR^{+/-}) (A, B) by a therapeutic combination according to the invention. A) Trastuzumab-(Cys-L-SO1861)⁴ titration + fixed concentration 10 pM cetuximab-saporin and controls on SKBR3 cells. B) Cetuximab-saporin titration + fixed concentration of 2.5nM trastuzumab-(Cys-L-SO1861)⁴ and controls on SKBR3 cells.

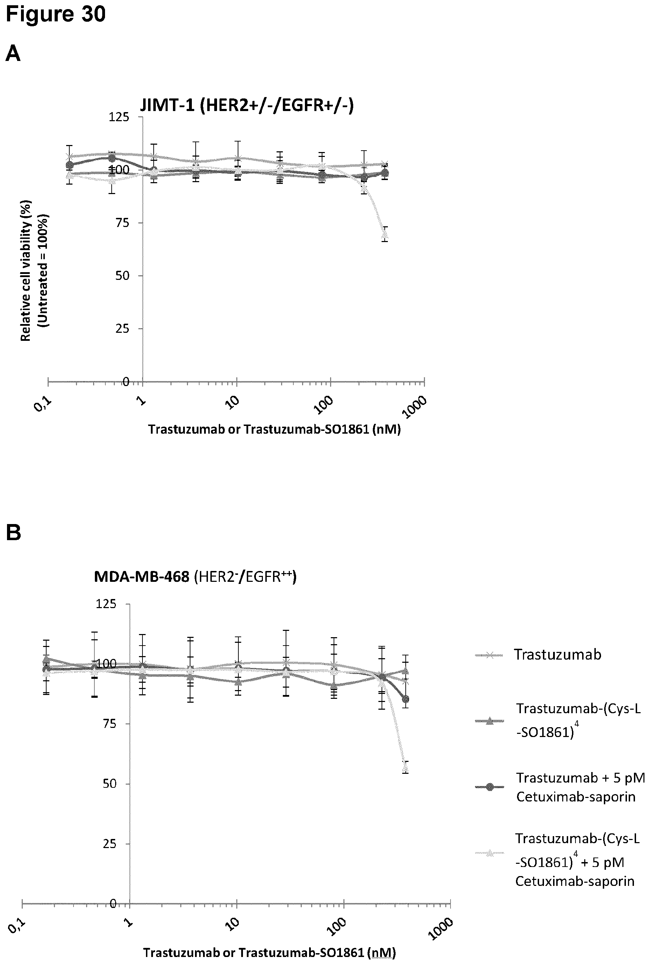

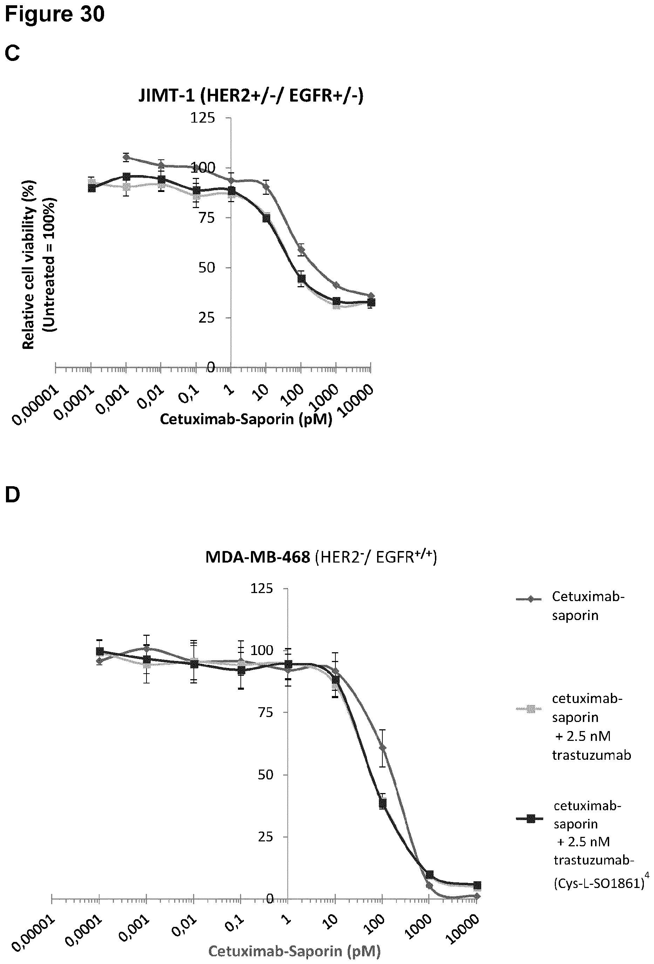

**Figure 30****.** *2-target 2-component.* HER2/EGFR targeted cell killing in JIMT-1 cells (HER2^{+/-}EGFR^{+/-}) (A, C) and MDA-MB-468 cells (HER2⁻/EGFR⁺⁺) (B, D) by a therapeutic combination according to the invention. A, B) Trastuzumab-(Cys-L-SO1861)⁴ titration + fixed concentration 10 pM cetuximab-saporin and controls on JIMT-1 cells. C, D) Cetuximab-saporin titration + fixed concentration of 2.5nM trastuzumab-(Cys-L-SO1861)⁴ and controls on MDA-MB-468 cells. The axes and legends are the same for A) and B), and C) and D). That is to say, the legend for Figures 30A and B is displayed next to Figure 30B; the legend for Figure 30C and 30D is displayed next to Figure 30D.

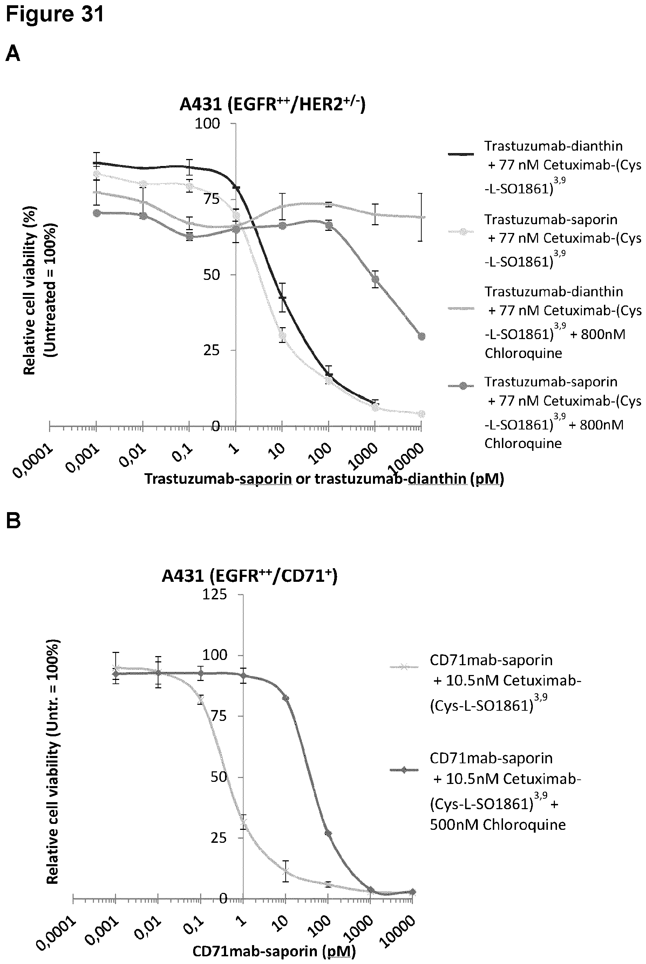

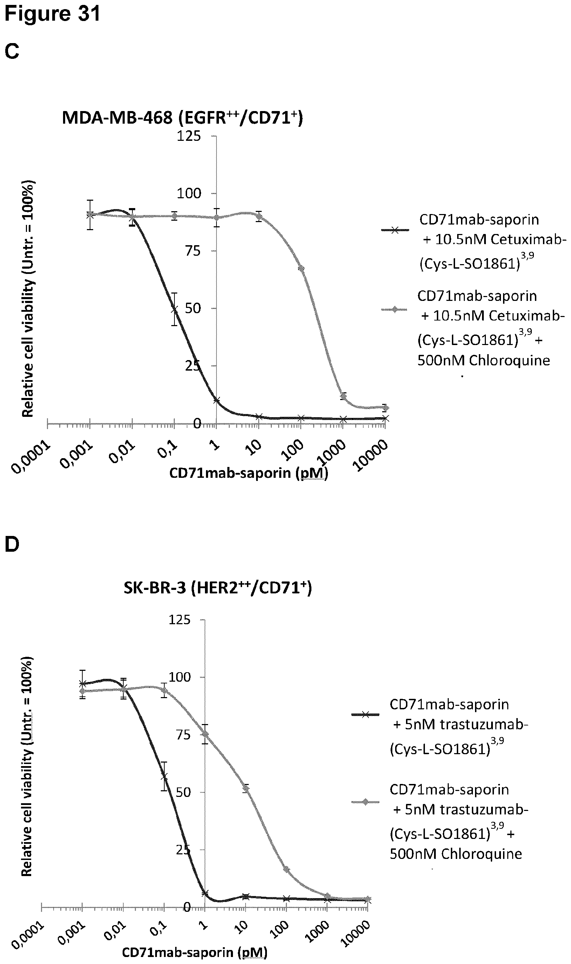

**Figure 31****:** *Chloroquine inhibits the 2-target 2-component.* EGFR/HER2, EGFR/CD71 or HER2/CD71 targeted cell killing in A431 cells (EGFR⁺⁺/HER2^{+/-}/CD71⁺) (A, B), MDA-MB-468 cells (EGFR⁺⁺/HER2⁻ /CD71⁺) (C) or SK-BR-3 (HER2⁺⁺/EGFR^{+/-}/CD71⁺) (D) by a therapeutic combination according to the invention + chloroquine. A) Trastuzumab-dianthin or trastuzumab-saporin titration + fixed concentration of 75nM cetuximab-(Cys-L-SO1861 )^{3,9} + 800 nM chloroquine and controls on A431 cells. B) CD71mab-saporin titration + fixed concentration of 10.5 nM cetuximab-(Cys-L-SO1861)^{3,9} + 500 nM chloroquine and control on A431 cells. C) CD71mab-saporin titration + fixed concentration of 10.5 nM cetuximab-(Cys-L-SO1861)^{3,9} + 500 nM chloroquine and control on MDA-MB-468 cells. D) CD71mab-saporin titration + fixed concentration of 5 nM trastuzumab-(Cys-L-SO1861)^{3,9} + 500 nM chloroquine and control on SK-BR-3 cells.

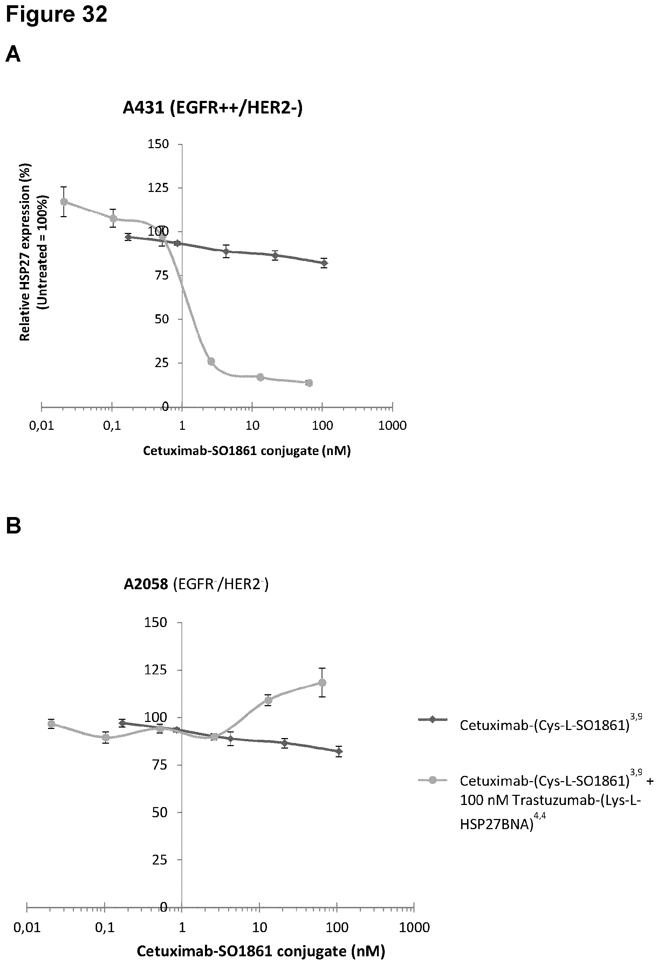

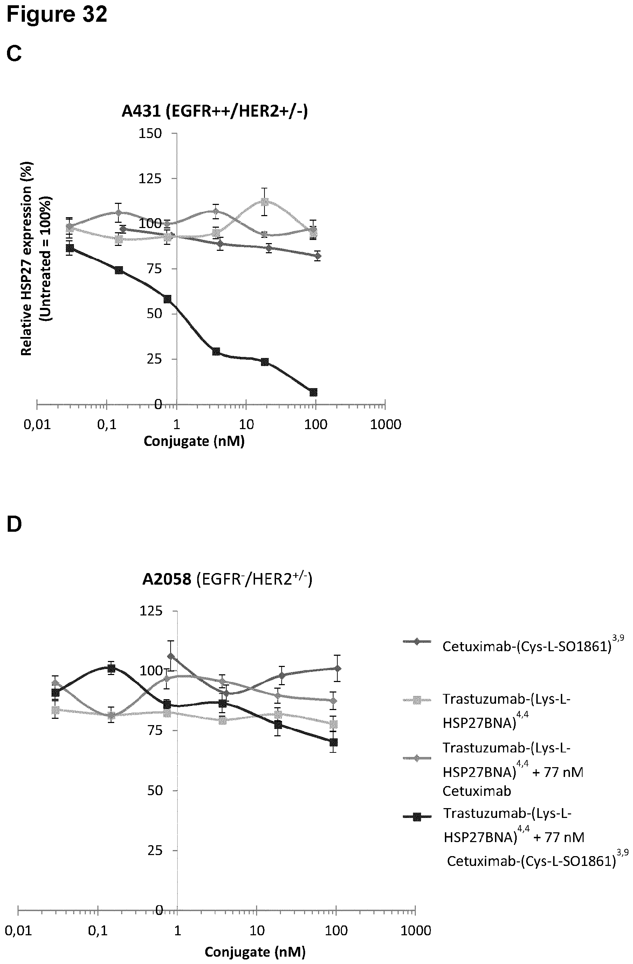

**Figure 32****:** *2-target 2-component.* EGFR/HER2 targeted gene silencing in A431 cells (EGFR⁺⁺/HER2^{+/-} ) (A) and A2058 cells (EGFR⁻/HER2^{+/-}) (B) by a therapeutic combination according to the invention. A) Cetuximab-(Cys-L-SO1861)^{3,9} titration + fixed concentration of 100 nM trastuzumab-(Lys-L-HSP27BNA)^{4,4} and control on A431 cells (A) and A2058 cells (B) . C, D) Trastuzumab-(Lys-L-HSP27BNA)^{4,4} titration + fixed concentration of 77nM cetuximab-(Cys-L-SO1861)^{3,9} and controls on A431 cells (A) and A2058 cells (B). The axes and legends are the same for A) and B), and C) and D). That is to say, the legend for Figures 32A and B is displayed next to Figure 32B; the legend for Figure 32C and 32D is displayed next to Figure 32D.

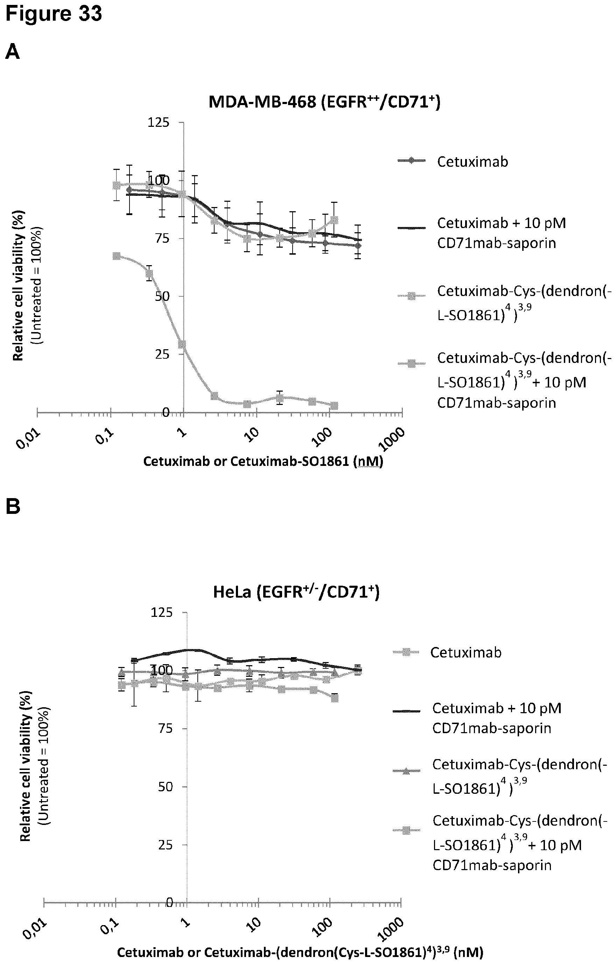

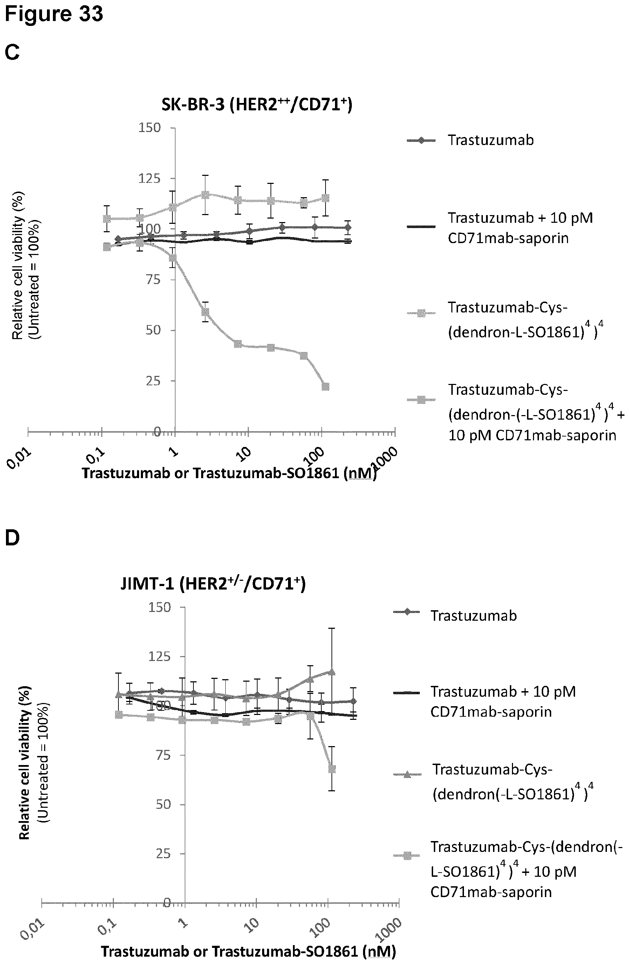

**Figure 33****:** *2-target 2-component.* A) EGFR/CD71 or HER2/CD71 targeted cell killing in MDA-MB-468 cells (EGFR⁺⁺/CD71⁺) (A) HeLa cells (EGFR^{+/-}/CD71⁺), SK-BR-3 cells (HER2⁺⁺/CD71⁺) (B) and JIMT-1 cells (HER2^{+/-}/CD71⁺) by a therapeutic combination according to the invention. A) Cetuximab-Cys-(dendron(-L-SO1861)⁴)^{3,9} titration + fixed concentration 10 pM CD71 mab-saporin and controls on MDA-MB-468 cells. B) A) Cetuximab-Cys-(dendron(-L-SO1861)⁴)^{3,9} titration + fixed concentration 10 pM CD71mab-saporin and controls on HeLa cells. C) Trastuzumab-Cys-(dendron(-L-SO1861)⁴)⁴ titration + fixed concentration 10 pM CD71mab-saporin and controls on SK-BR-3 cells. D) Trastuzumab-Cys-(dendron(-L-SO1861)⁴)⁴ titration + fixed concentration 10 pM CD71mab-saporin and controls on JIMT-1 cells.

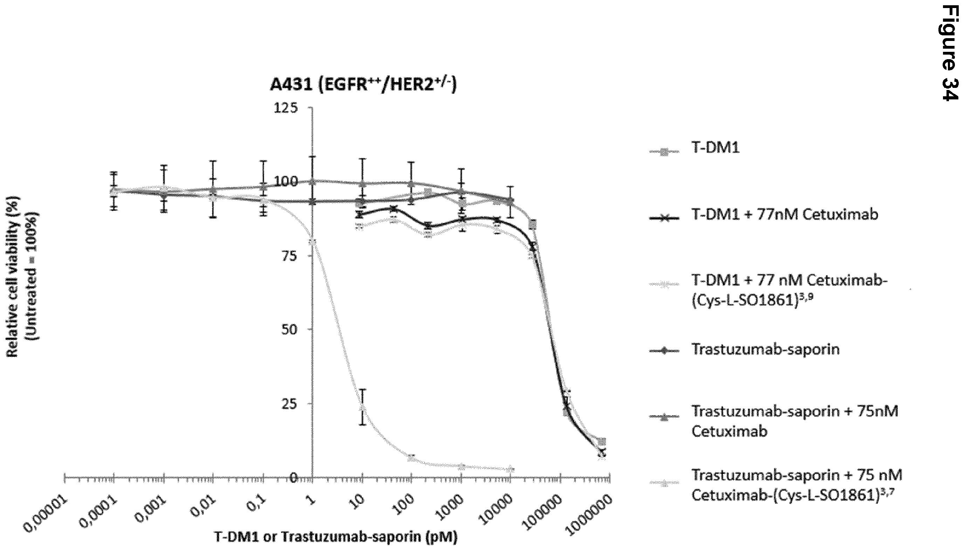

**Figure 34****.** *2-target 2-component versus T-DM1.* A431 cells (EGFR⁺⁺/HER2^{+/-}) can efficiently be killed with the therapeutic combination according to the invention, Tratuzumab-saporin + 75 nM cetuximab-(Cys-L-SO1861)^{3,9} , however titration of T-DM1 + 75 nM cetuximab-(Cys-L-SO1861)^{3,9} is not effective at such low toxin concentrations. T-DM1 is Trastuzumab-emtansine (Kadcyla®), carrying ∼3.5 emtansine (DM1) toxin molecules per antibody.

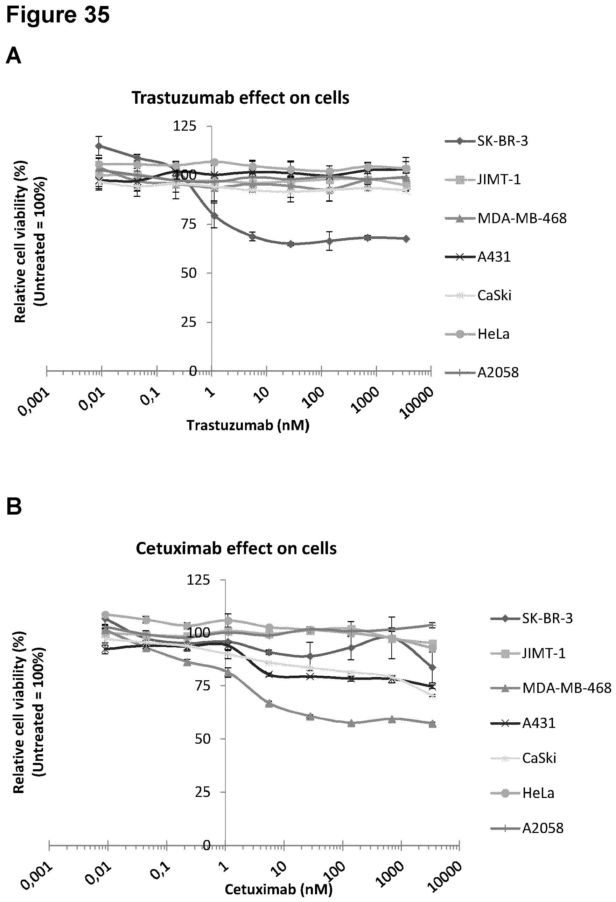

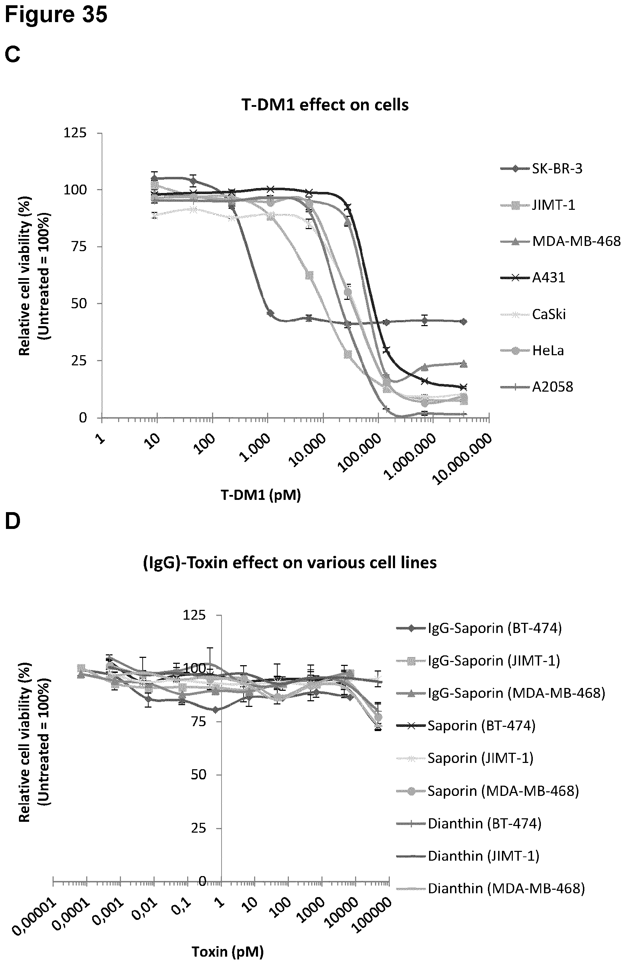

**Figure 35****:** *Control treatments on all cell lines.* A-D) Cell viability when trastuzumab (A), cetuximab (B), T-DM1, (C) free toxins: saporin and dianthin (D) or saporin coupled to a non-cell binding IgG (D) are treated with the indicated cell lines SK-BR-3, JIMT-1, MDA-MB-468, A431, CaSki, HeLa, A2058, BT-474.

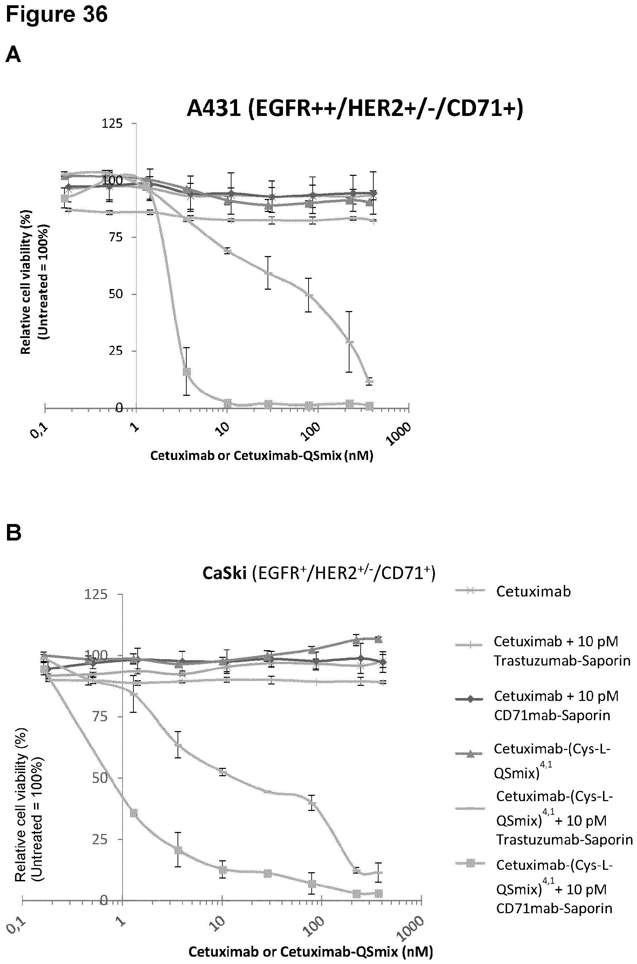

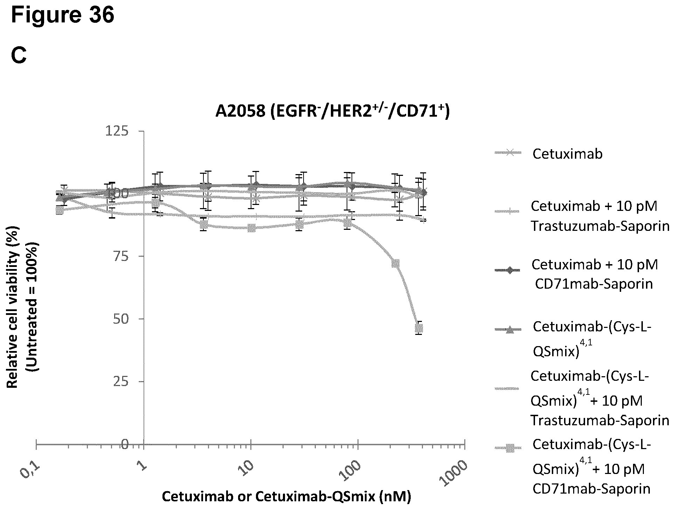

**Figure 36****.** *2-target 2-component.* EGFR/CD71 and EGFR/HER2 targeted cell killing in A431 cells (EGFR⁺⁺⁺/HER2^{+/-}) (A) and CaSKi cells (EGFR⁺⁺/HER2^{+/-}) (B) and A2058 cells (EGFR⁻/HER2^{+/-}) by a therapeutic combination according to the invention. A, B,C) Cetuximab-(Cys-L-QSmix)^{4,1} titration + fixed concentration 10 pM trastuzumab-saporin or 10 pM CD71mab-saporin and controls on A431 cells (A). CaSKi cells (B) and A2058 cells (C). QSmix is a mixture of saponins from an extract Quillaja Saponaria. The legend for Figures 36A and 36B is displayed next to Figure 36B.

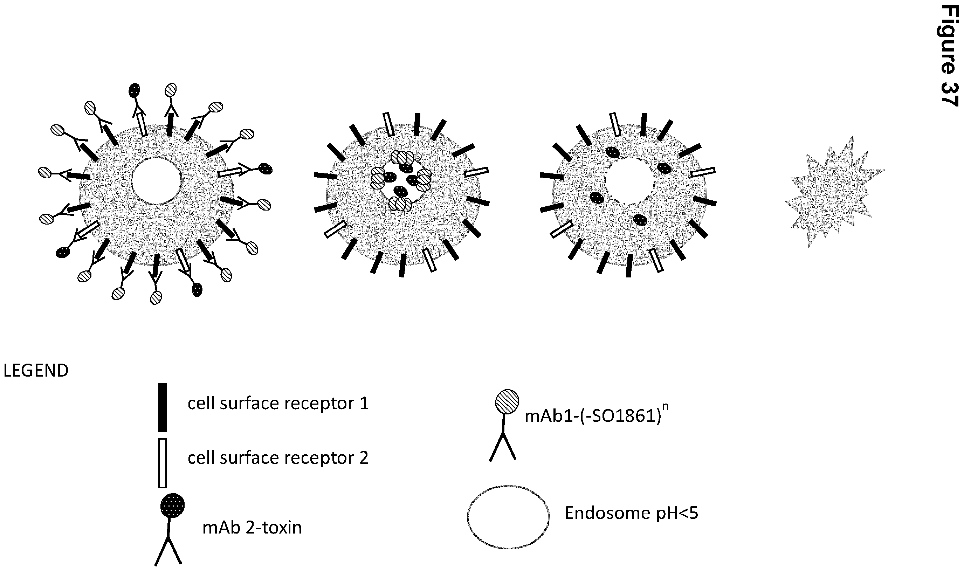

**Figure 37****:** *2-target 2-component concept: mAb1-SO1861* + *mAb2-protein toxin.* SO1861 and toxin (ribosomal inactivating protein) are each, separately, conjugated to an antibody (mAb) for delivery and internalization into target cells. 1) mAb1-SO1861 and mAb2-protein toxin bind to their corresponding cell surface receptor, 2) receptor-mediated endocytosis of both conjugates occurs, 3) at low endolysosomal pH and appropriate concentration, SO1861 becomes active to enable endolysosomal escape, 4) release of toxin into cytoplasm occurs and 5) toxin induces cell death.

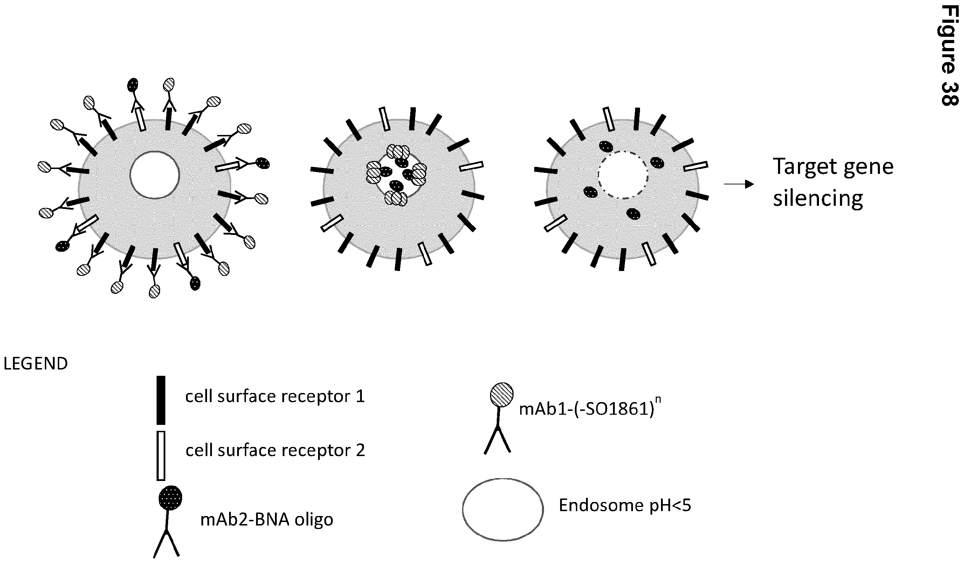

**Figure 38****:** *2-target 2-component concept: mAb1-SO1861* + *mAb2-BNA oligo.* SO1861 and antisense BNA oligo nucleotide are each, separately, conjugated to an antibody (mAb) for delivery and internalization into target cells. 1) mAb1-SO1861 and mAb2-BNAoligo bind to their corresponding cell surface receptor, 2) receptor-mediated endocytosis of both conjugates occurs, 3) at low endolysosomal pH and appropriate concentration, SO1861 becomes active to enable endolysosomal escape, 4) release of BNA oligo into cytoplasm occurs and 5) target gene silencing.

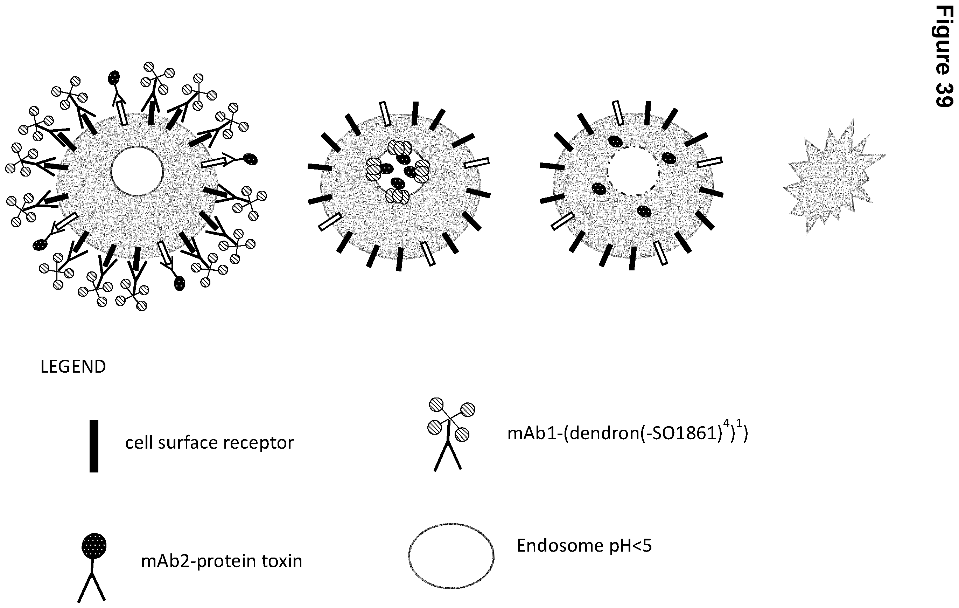

**Figure 39****:** *2-target 2-component concept: mAb1-(scaffold(-SO1861)ⁿ)ⁿ* + *mAb2-protein toxin.* Dendron(-SO1861)ⁿ and protein toxin (ribosomal inactivating protein) are each, separately, conjugated to an antibody (mAb) for delivery and internalization into target cells. 1) mAb1-(dendron(-SO1861)⁴)¹) and mAb2-protein toxin bind to their corresponding cell surface receptor, 2) receptor-mediated endocytosis of both conjugates occurs, 3) at low endolysosomal pH and appropriate concentration, SO1861 becomes active to enable endolysosomal escape, 4) release of toxin into cytoplasm occurs and 5) toxin induces cell death.

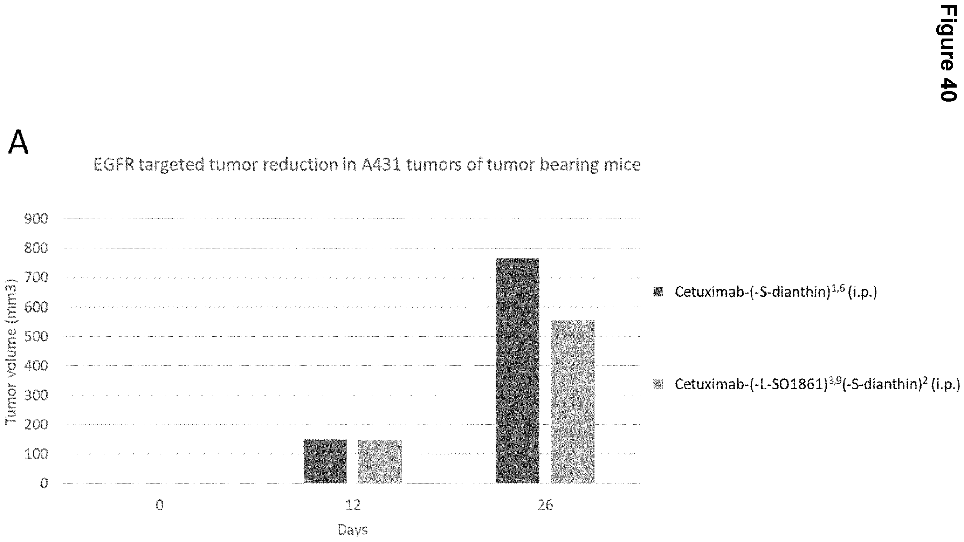

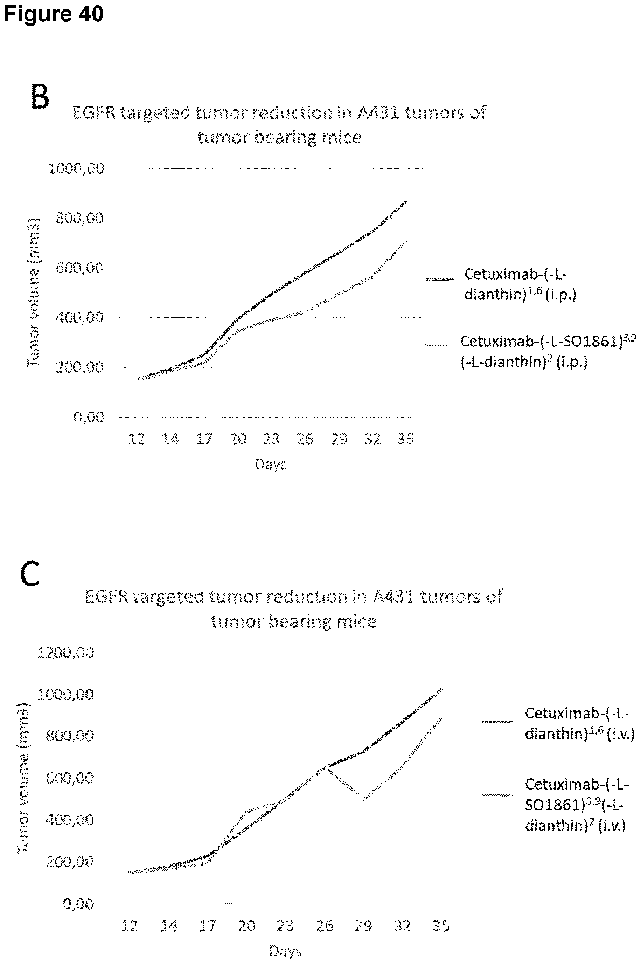

**Figure 40****.** *Tumor targeted protein toxin delivery results in tumor volume reduction and tumor growth inhibion, in tumor bearing mice.* A) Dose escalation (intraperitoneal, i.p.) of cetuximab-(Cys-L-SO1861)^{3,9}(Lys-S-dianthin)² in A431 tumor bearing mice reveals tumor volume reduction, compared to the control. B, C) Dose escalation (intraperitoneal, i.p. (B) or intravenous i.v. (C)) of cetuximab-(Cys-L-SO1861)^{3,9}(Lys-L-dianthin)² in A431 tumor bearing mice reveals tumor growth reduction, compared to the controls.

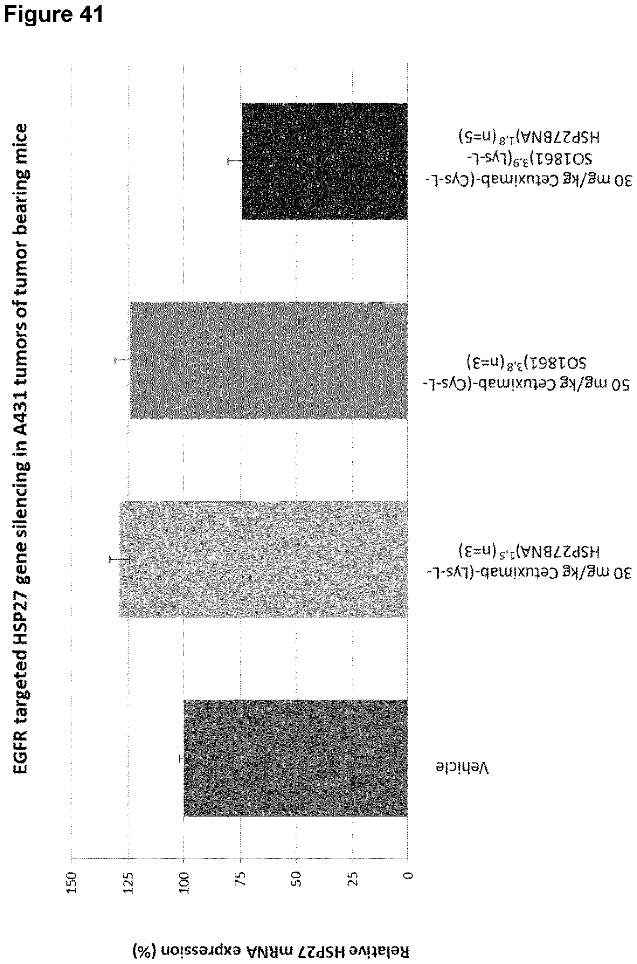

**Figure 41****.** *Tumor targeted antisense BNA oligo nucleotide delivery and gene silencing in tumor bearing mice.* 30 mg/kg cetuximab-(Cys-L-SO1861)^{3,9}(Lys-L-HSP27BNA)^{1,8} in A431 tumor bearing mice reveals induced efficient tumor targeted gene silencing, compared to the controls.

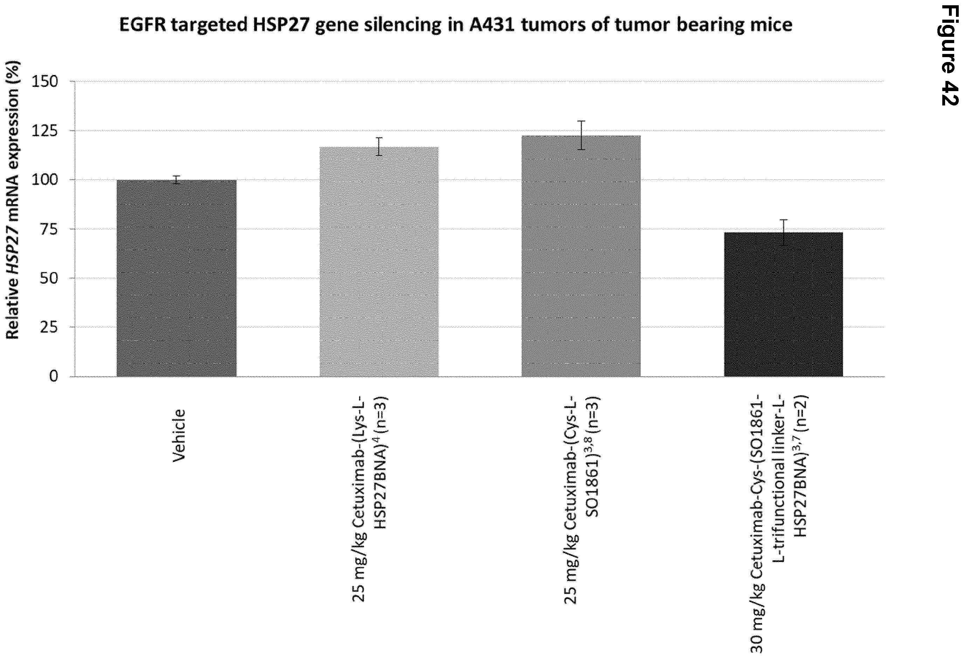

**Figure 42****.** *Tumor targeted antisense BNA oligo nucleotide delivery and gene silencing in tumor bearing mice.* 30 mg/kg cetuximab-Cys-(SO1861-L-trifunctional linker-L-HSP27BNA)^{3,7} in A431 tumor bearing mice reveals induced efficient tumor targeted gene silencing, compared to the controls.

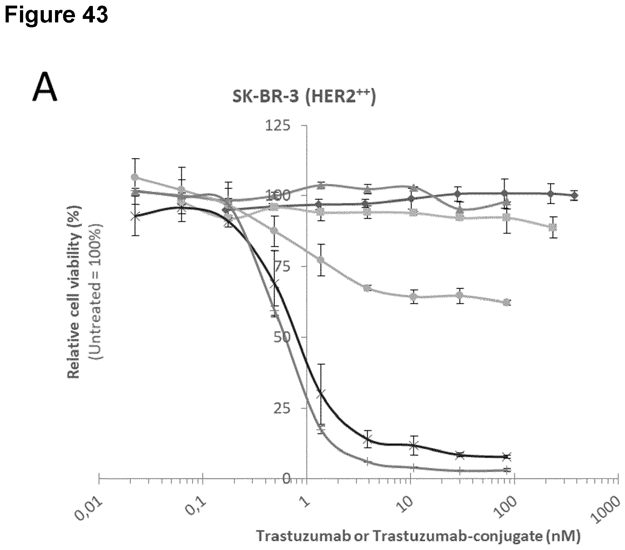

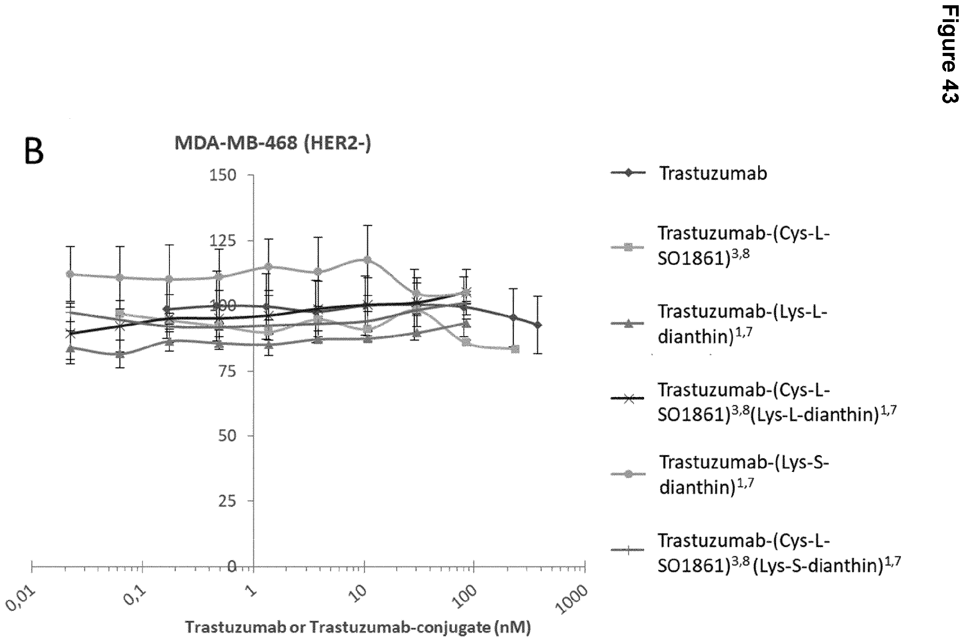

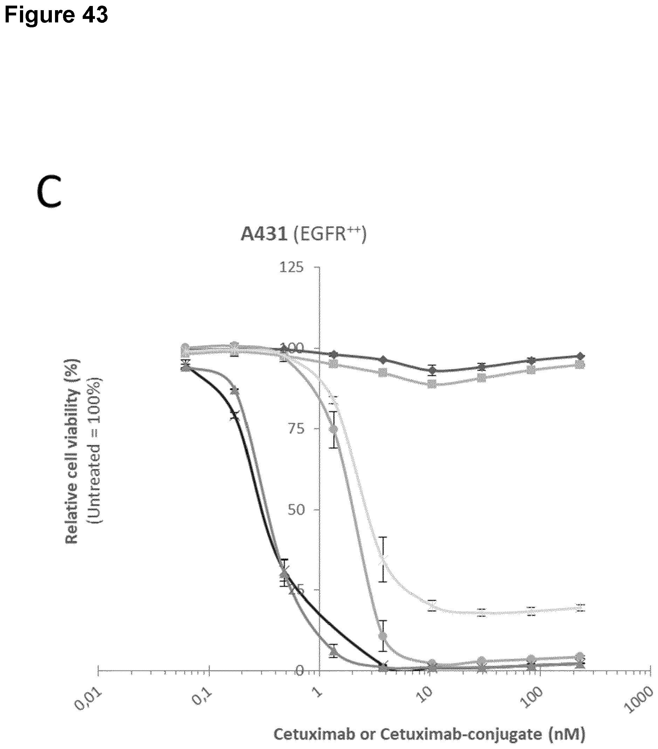

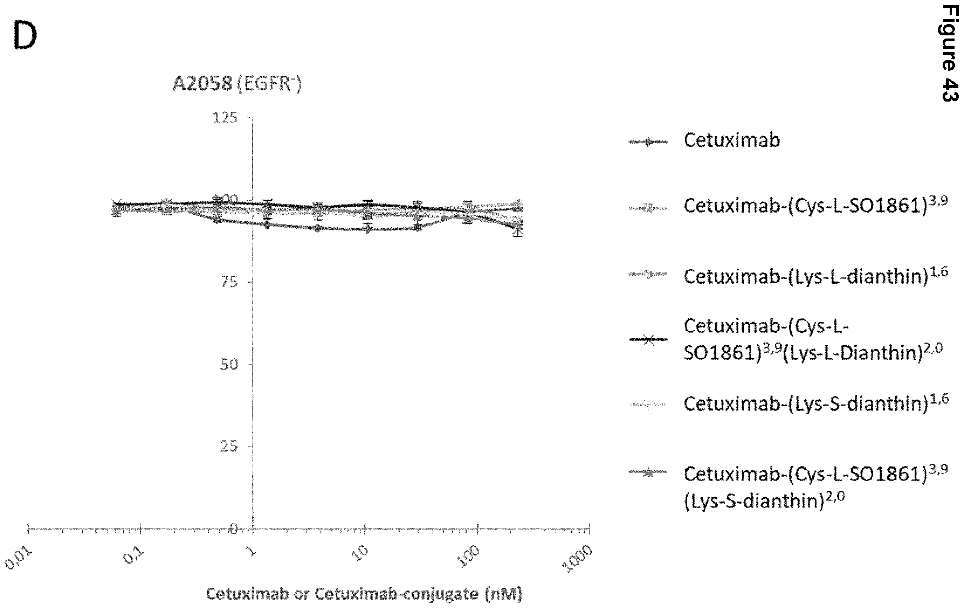

**Figure 43**: *HER2 or EGFR targeted protein toxin delivery and cell killing in cancer cells, according to the invention.* A, B) Trastuzumab-(Cys-L-SO1861)^{3,8}(Lys-L-dianthin)^{1,7} or Trastuzumab-(Cys-L-SO1861)^{3,8}(Lys-S-dianthin)^{1,7} treatment and controls on SK-BR-3 cells (HER2⁺⁺) and MDA-MB-468 cells (HER2⁻). C, D) Cetuximab-(Cys-L-SO1861)^{3,8}(Lys-L-dianthin)^{1,7} or Cetuximab-(Cys-L-SO1861)^{3,8}(Lys-S-dianthin)^{1,7} treatment and controls on A431 cells (EGFR⁺⁺) and A2058 cells (EGFR⁻). Figure 43A and B have the same legend, outlined next to Figure 43B. Figure 43C and D have the same legend, outlined next to Figure 43D.

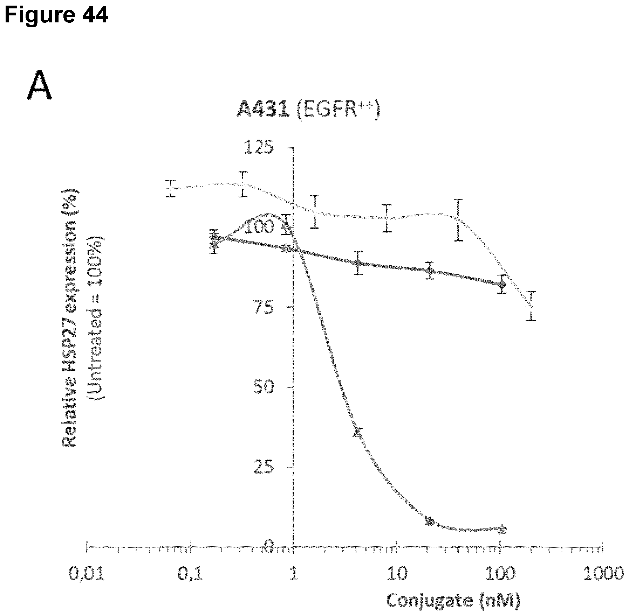

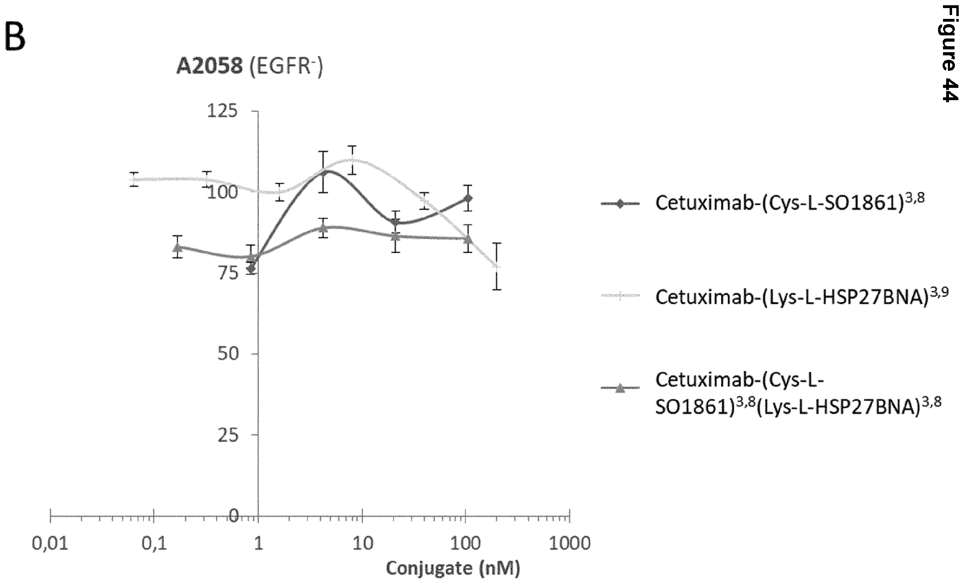

**Figure 44**: *EGFR targeted antisense BNA oligo delivery and gene silencing in cancer cells, according to the invention.* A,B) Cetuximab-(Cys-L-SO1861)^{3,8}(Lys-L-HSP27BNA)^{1,7} treatment and controls on A431 cells (EGFR⁺⁺) and A2058 cells (EGFR⁻). Figure 44A and B have the same legend, outlined next to Figure 44B.

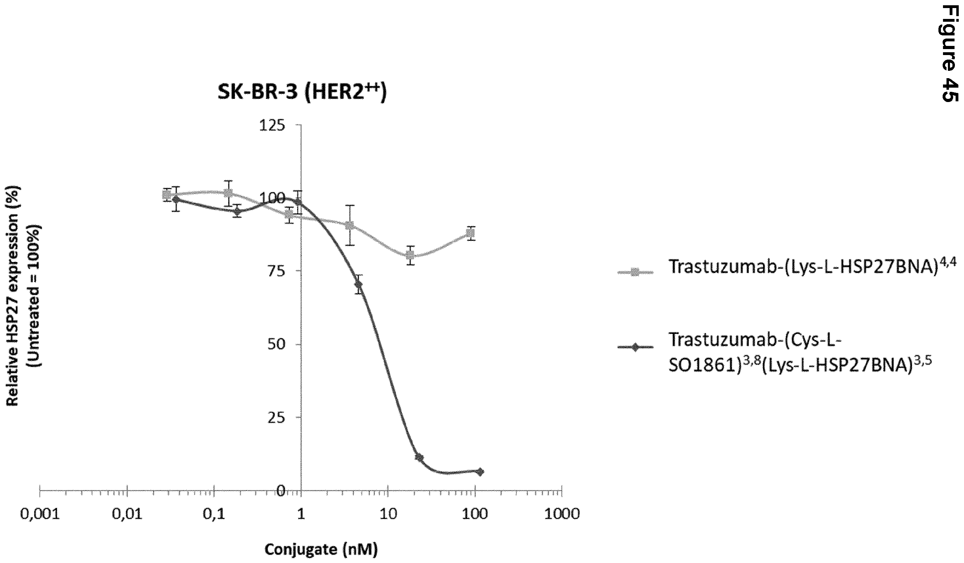

**Figure 45**: *HER2 targeted antisense BNA oligo delivery and gene silencing in cancer cells, according to the invention.* Trastuzumab-(Cys-L-SO1861)^{3,8}(Lys-L-HSP27BNA)^{3,5} treatment and controls on SK-BR-3 cells (HER2⁺⁺).

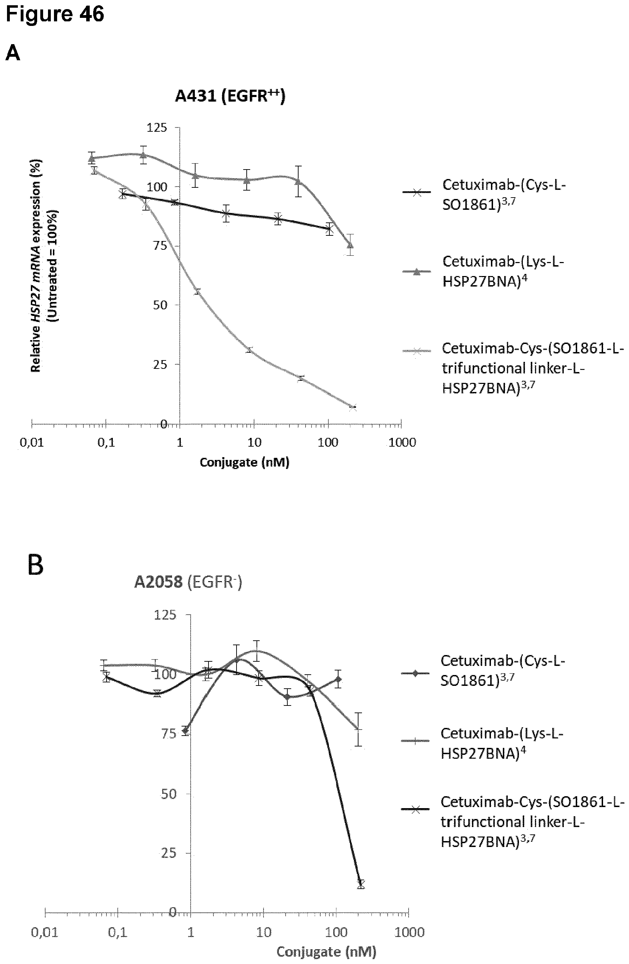

**Figure 46**: *EGFR targeted antisense BNA oligo delivery and gene silencing in cancer cells, according to the invention.* A,B) Cetuximab-Cys-(SO1861-L-trifunctional linker-L-HSP27BNA)^{3,7} treatment and controls on A431 cells (EGFR⁺⁺) and A2058 cells (EGFR⁻).

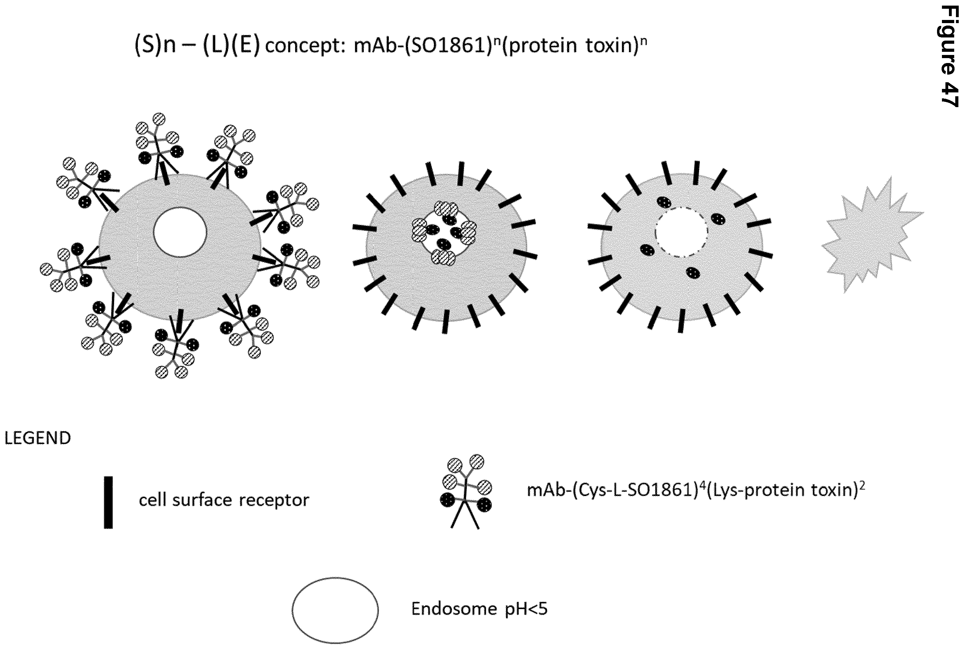

**Figure 47**: **(S)n - (L)(E) concept:** *mAb-(SO1861)ⁿ(protein toxin)ⁿ.* Both, SO1861 at the cysteine residues (Cys) and protein toxin (ribosomal inactivating protein) at the lysine residues are conjugated to the same antibody (mAb) for delivery and internalization into the target cells. 1) mAb-(Cys-L-SO1861)⁴(Lys-protein toxin)² bind to its corresponding cell surface receptor, 2) receptor-mediated endocytosis the conjugate occurs, 3) at low endolysosomal pH and appropriate concentration, SO1861 becomes active to enable endolysosomal escape, 4) release of toxin into cytoplasm occurs and 5) toxin induces cell death

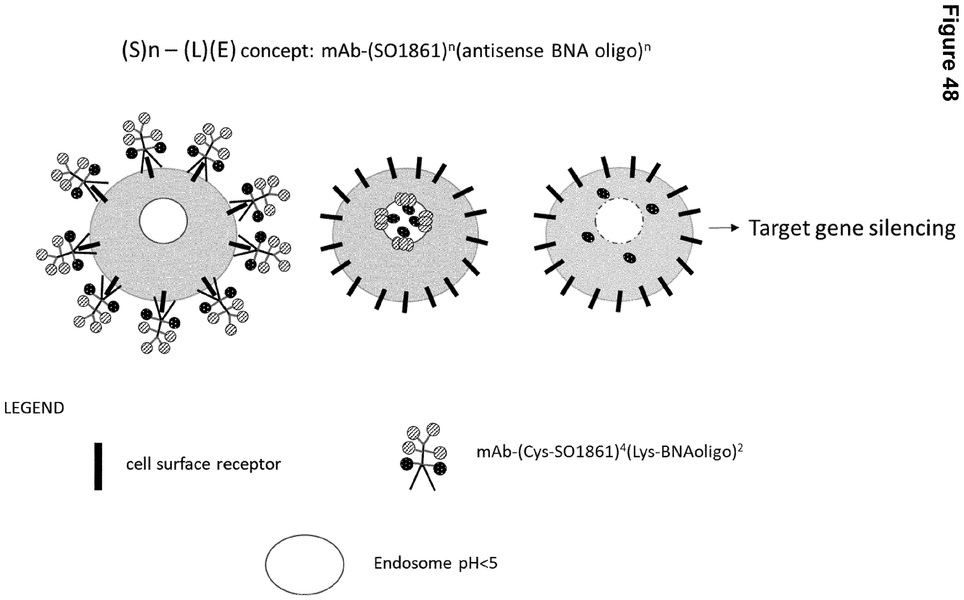

**Figure 48****: (S)n - (L)(E) concept:** *mAb-(SO1861)ⁿ(antisense BNA oligo)ⁿ.* Both, SO1861, at the cysteine residues (Cys) and the antisense BNA oligo nucleotide, at the lysine residues are conjugated to the same antibody (mAb) for delivery and internalization into the target cells. 1) mAb-(Cys-SO1861)⁴(Lys-BNAoligo)² bind to its corresponding cell surface receptor, 2) receptor-mediated endocytosis of both conjugates occurs, 3) at low endolysosomal pH and appropriate concentration, SO1861 becomes active to enable endolysosomal escape, 4) release of BNA oligo into cytoplasm occurs and 5) target gene silencing is induced.

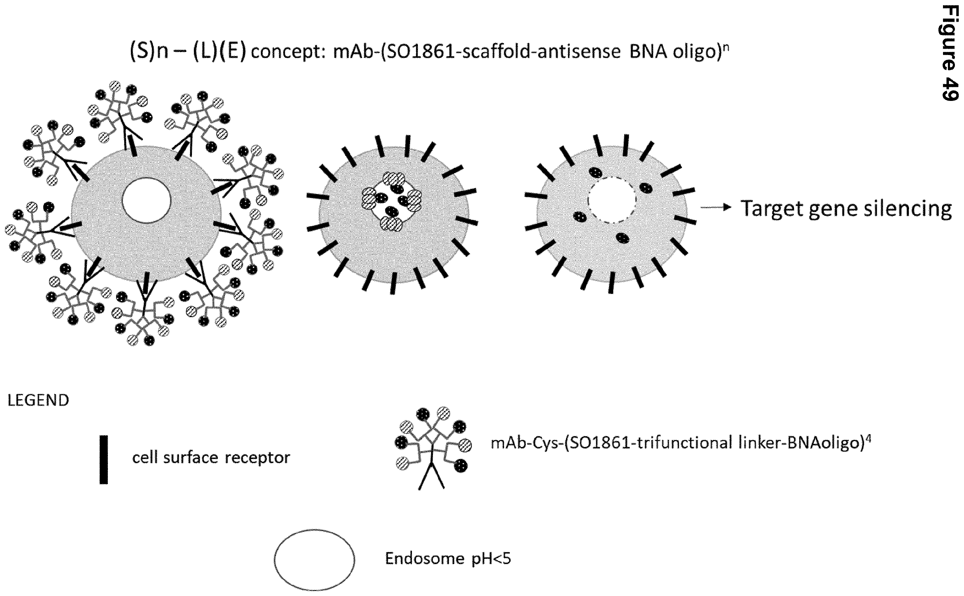

**Figure 49****: (S)n - (L)(E) concept:** *mAb-(SO1861-scaffold-antisense BNA oligo)".* the (SO1861-trifunctional linker-BNAoligo)ⁿ is conjugated to an antibody (mAb) for delivery and internalization into the target cells. 1) mAb-(SO1861-trifunctional linker-BNAoligo)⁴ binds to its corresponding cell surface receptor, 2) receptor-mediated endocytosis of both conjugates occurs, 3) at low endolysosomal pH and appropriate concentration, SO1861 becomes active to enable endolysosomal escape, 4) release of BNA oligo into cytoplasm occurs and 5) target gene silencing is induced.

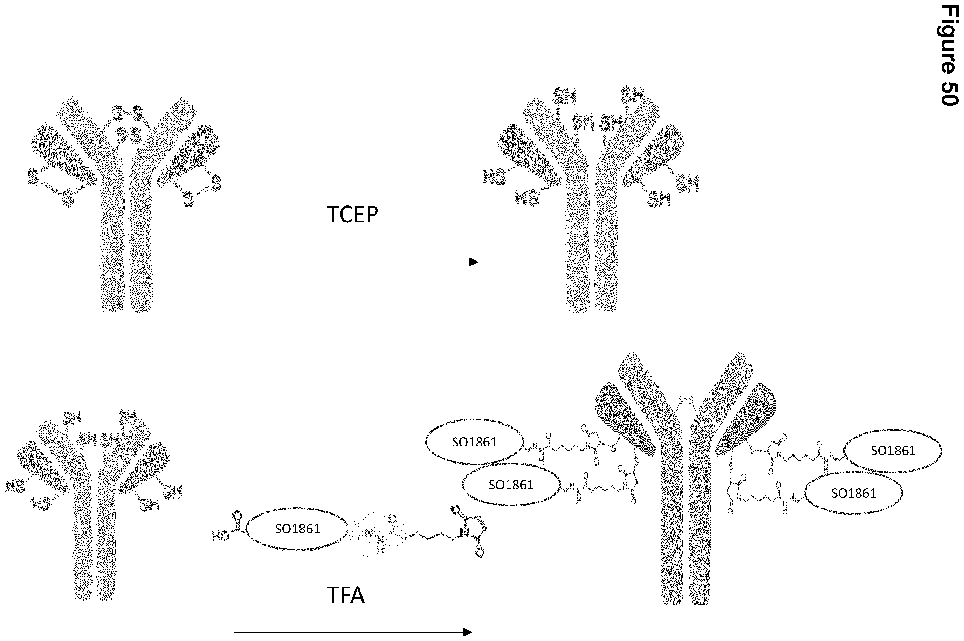

**Figure 50****.** *Antibody-SO1861 conjugation procedure.* Shown is the coupling reaction of the linking of four moieties of a plant-derived saponin SO1861 to the four cysteines in the light chain of an antibody. First, the disulphide bonds in the IgG are disrupted under influence of exposure to TCEP (Tris(2-carboxyethyl)phosphine); second, the saponin SO1861 comprising a chemical linker bound to it, is added together with trifluoro acetic acid, and four saponin moieties are linked to the IgG. For producing cleavable 'ready to conjugate' saponins the aldehyde group of SO1861 was reacted with an EMCH (ε-maleimidocaproic acid hydrazide) linker. The hydrazide group of EMCH forms an acid cleavable hydrazone bond with the aldehyde of SO1861. At the same time the EMCH linker presents a maleimide group that is thiol (sulfhydryl group) reactive and thus can be conjugated to thiols of the IgG, i.e. the ligand moiety. Herewith, an endosomal escape enhancing conjugate of the invention is provided, and/or a first binding molecule of the invention is provided.

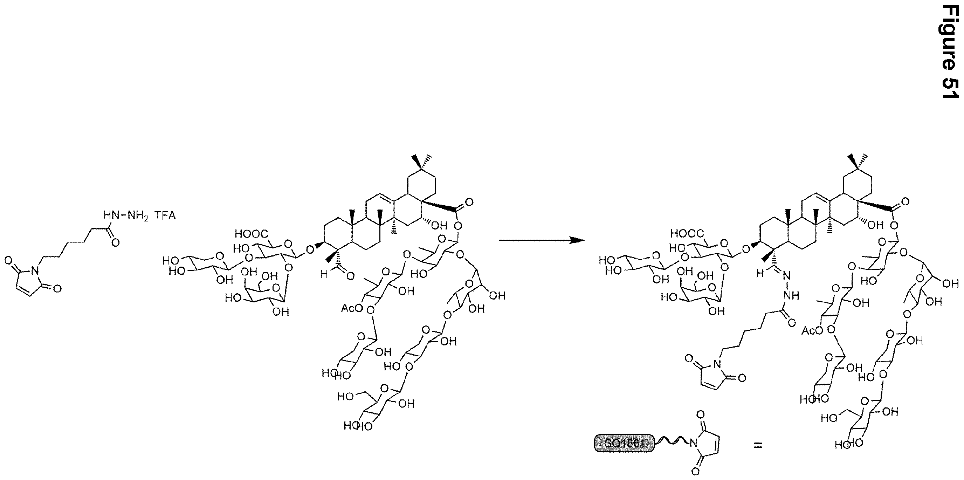

**Figure 51****.** SO1861-EMCH synthesis

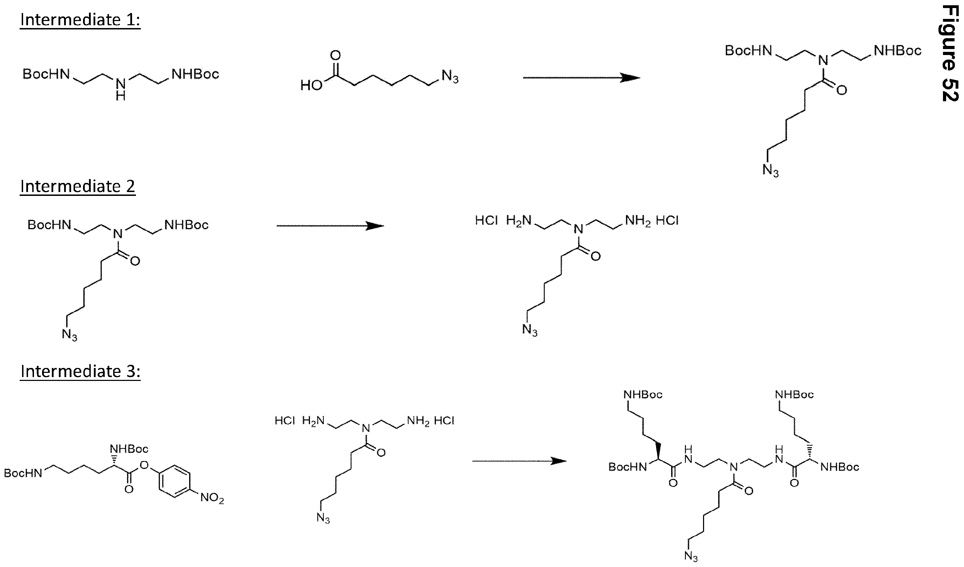

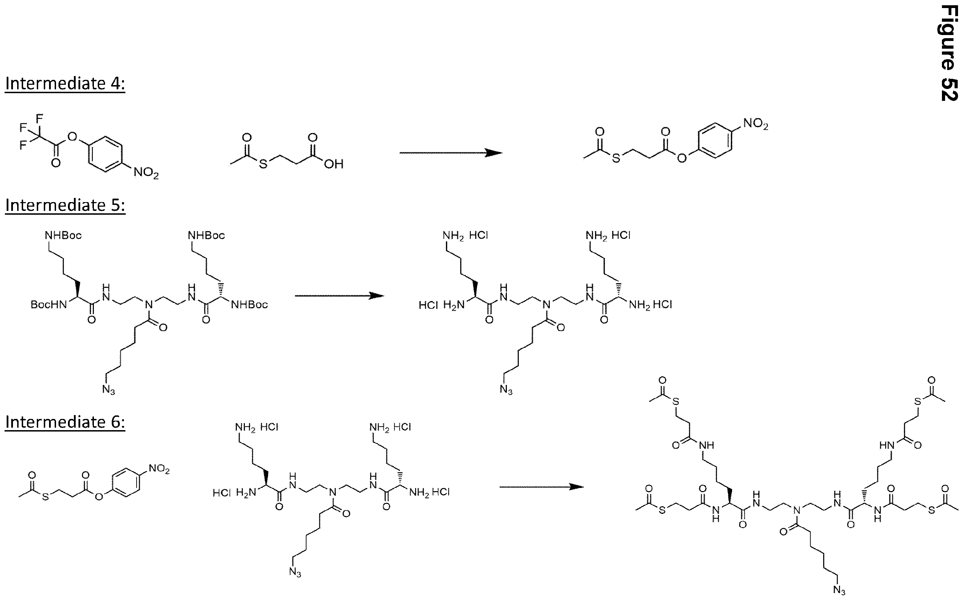

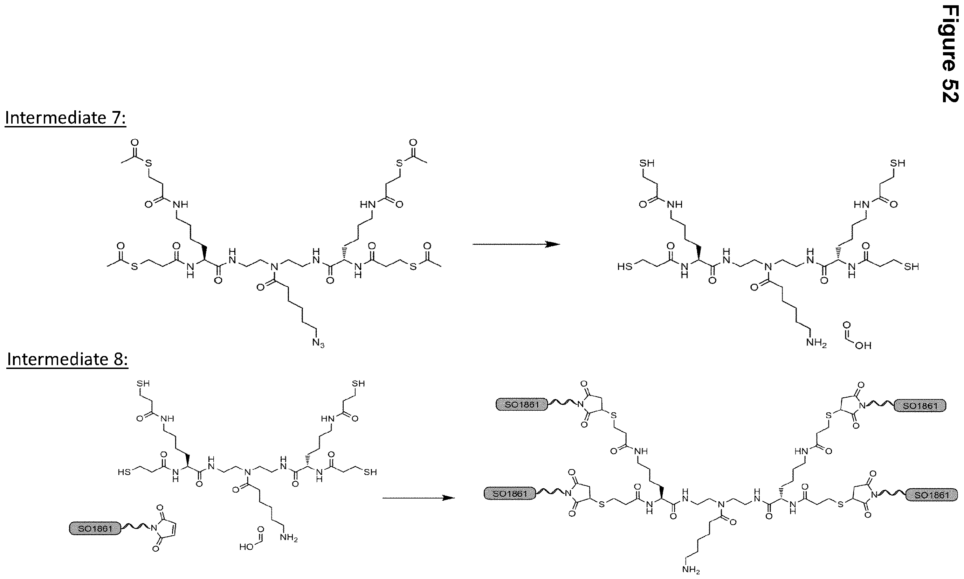

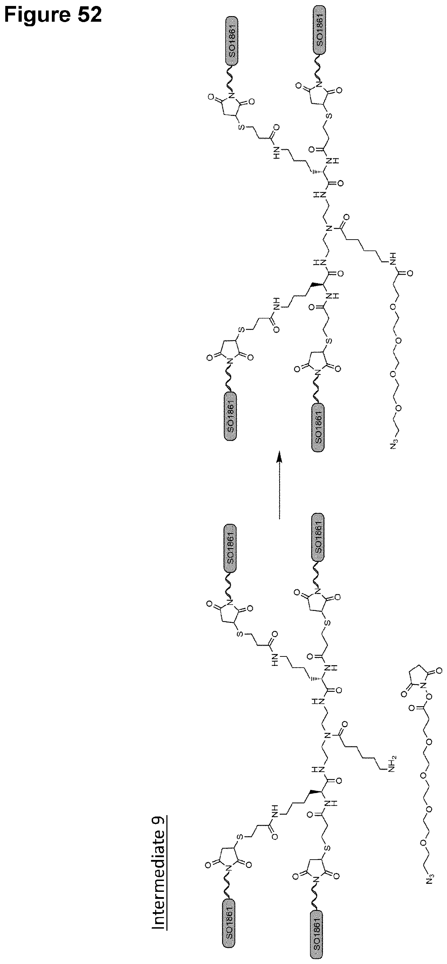

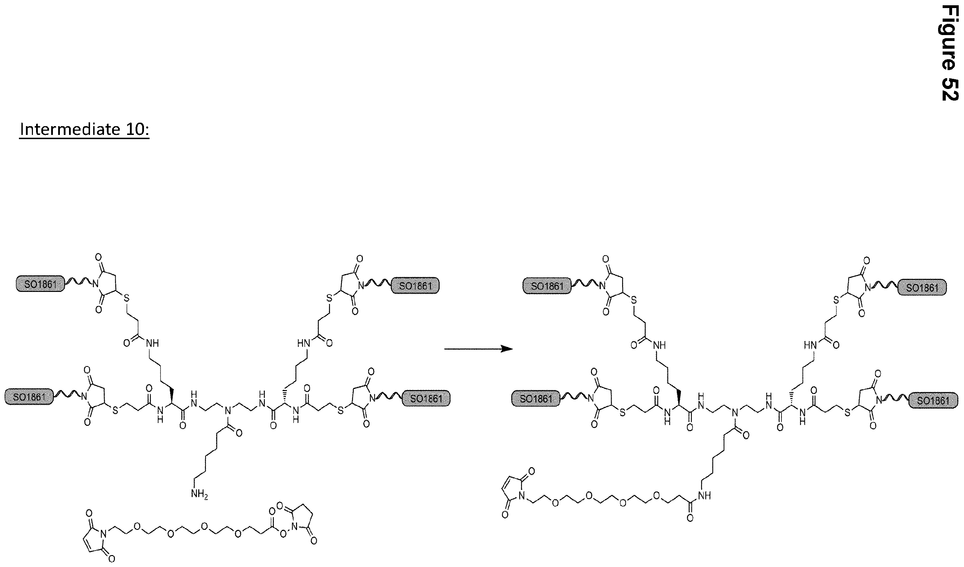

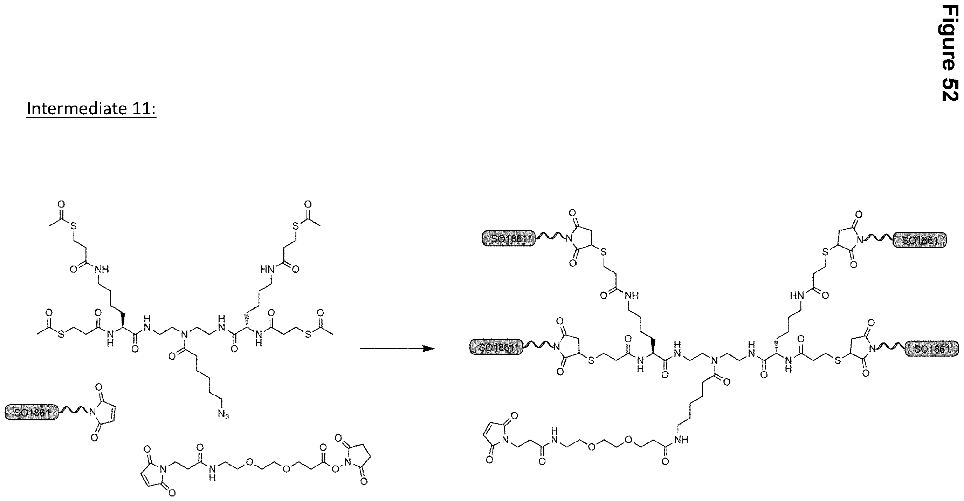

**Figure 52** Dendron-(-L-SO1861)⁴ synthesis

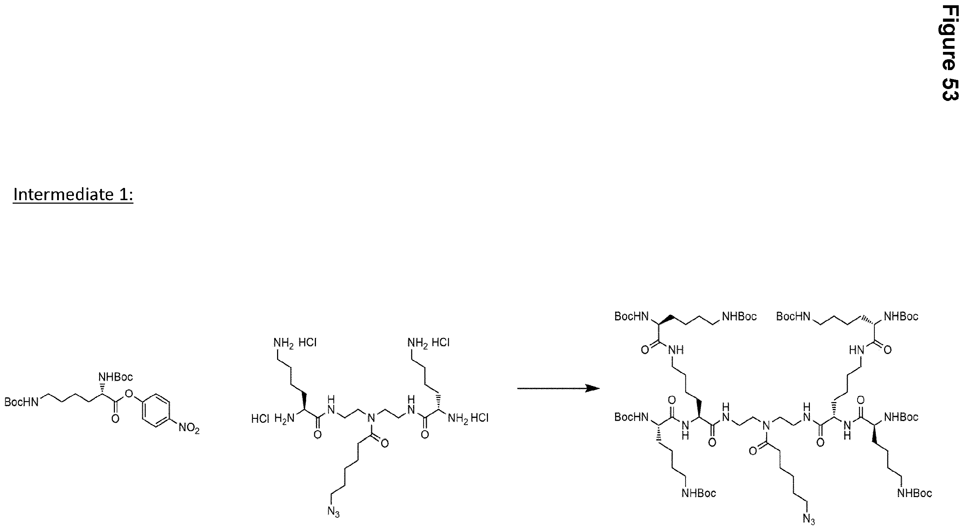

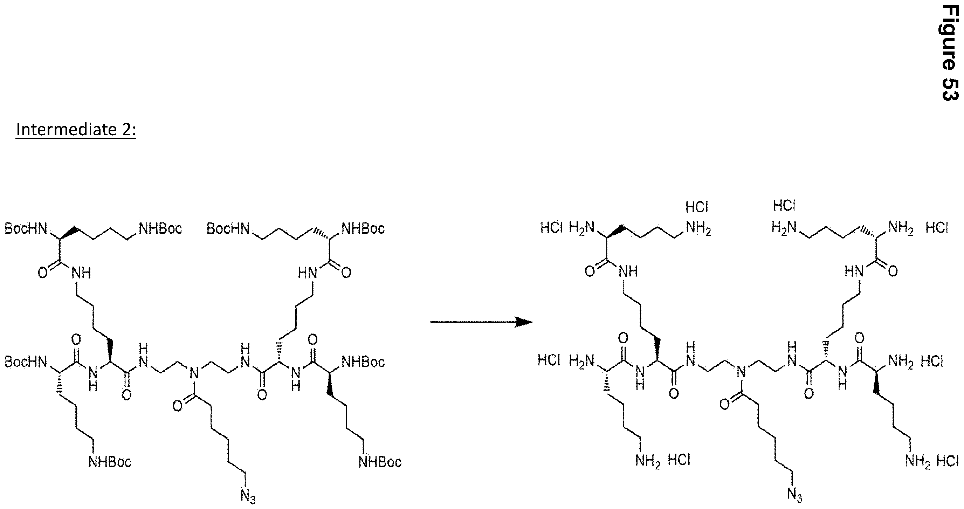

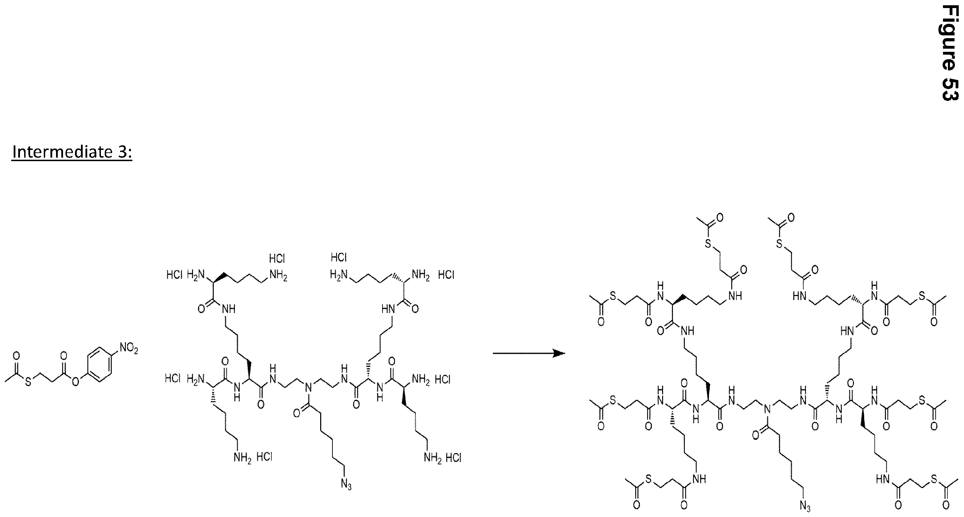

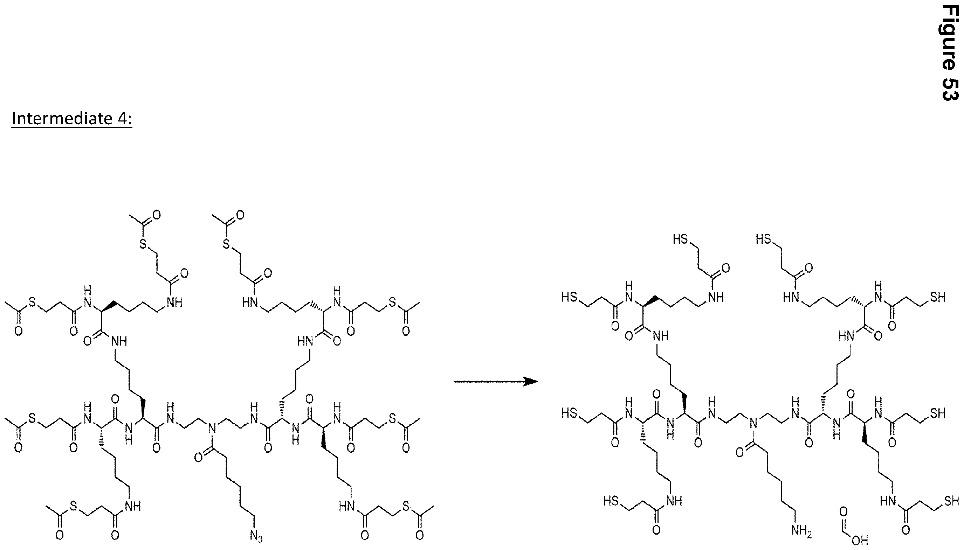

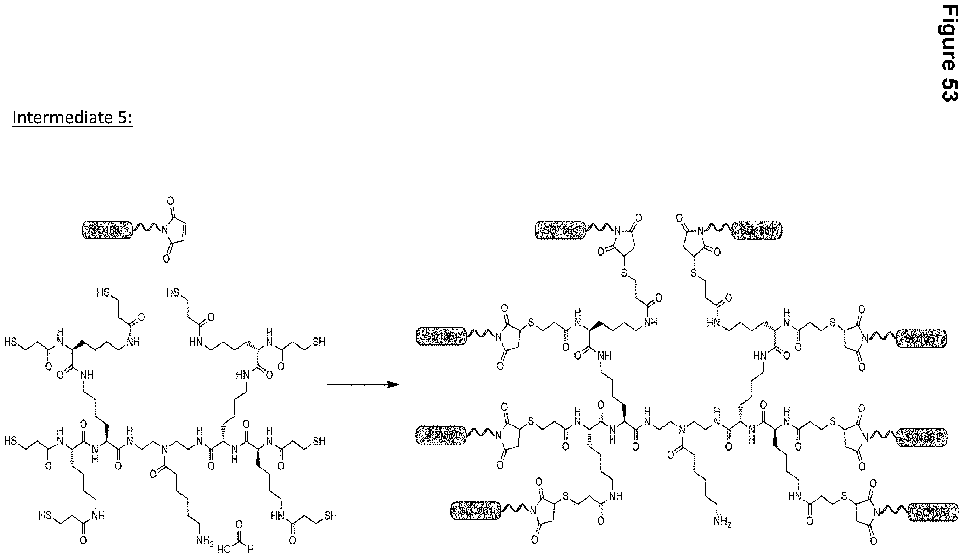

**Figure 53****.** Dendron-(-L-SO1861)⁸ synthesis

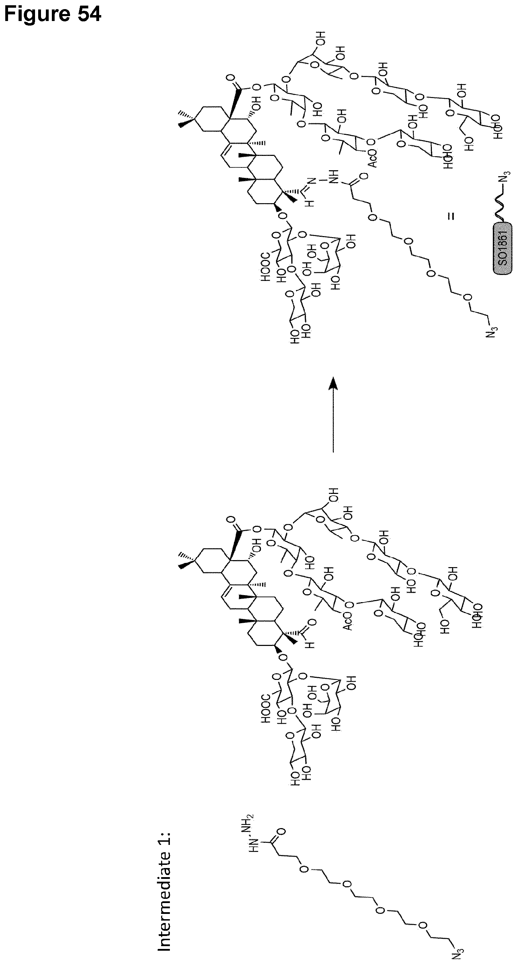

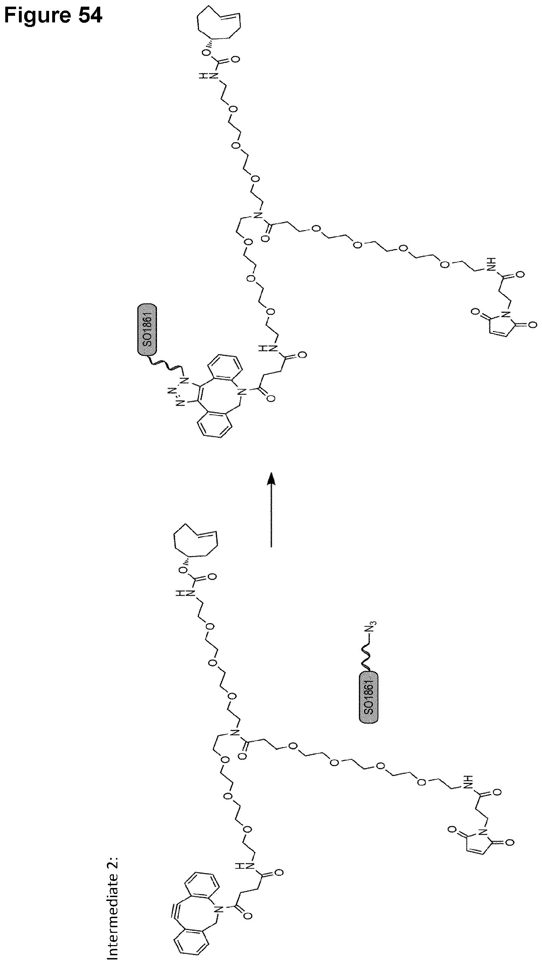

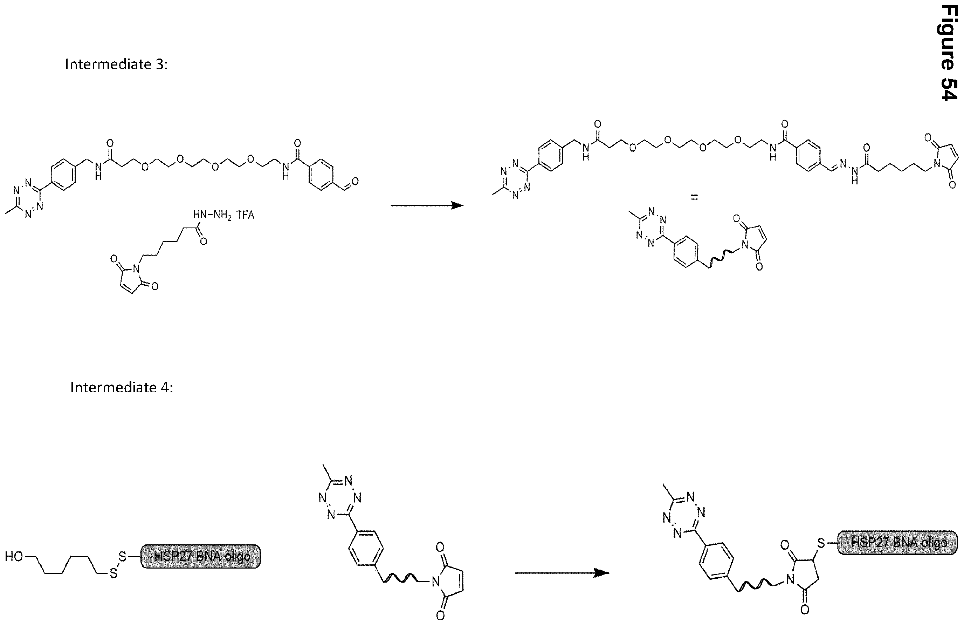

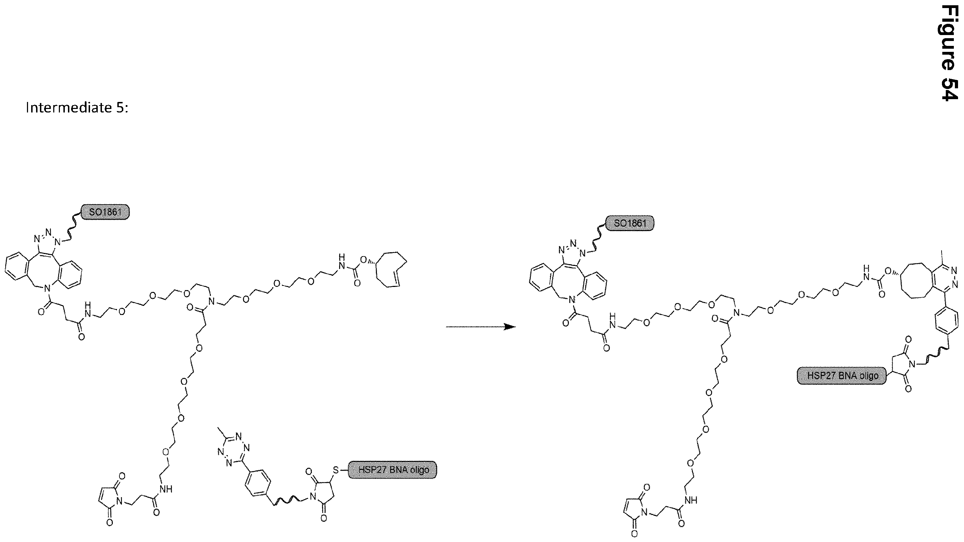

**Figure 54****.** SO181-L-trifunctional linker-L-HSP27BNA synthesis

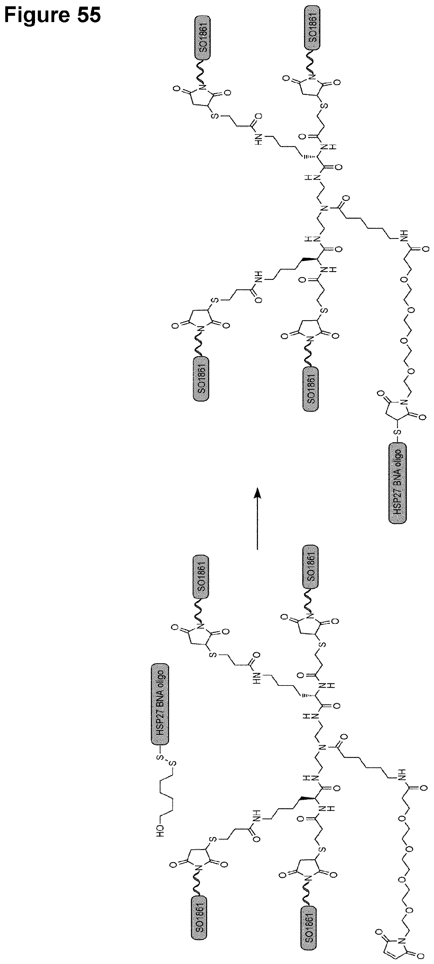

**Figure 55****.** HSP27BNA-dendron-(-L-SO1861)⁴ synthesis

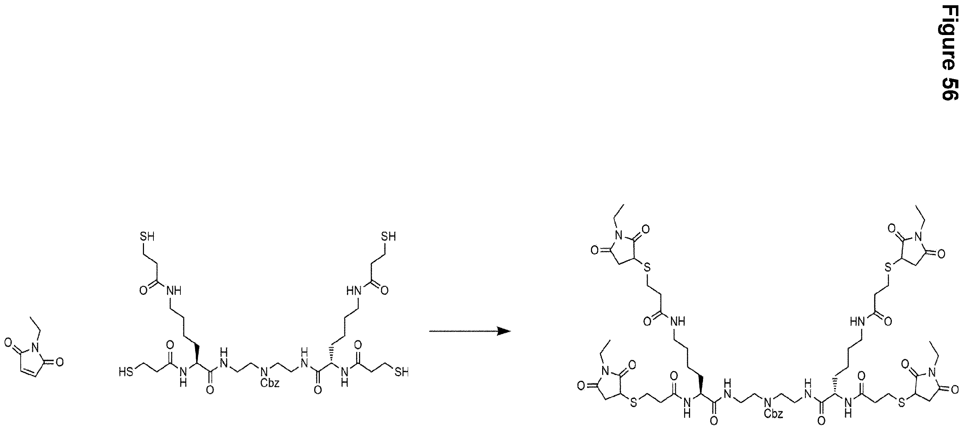

**Figure 56****.** Dendron(NEM)⁴ synthesis synthesis

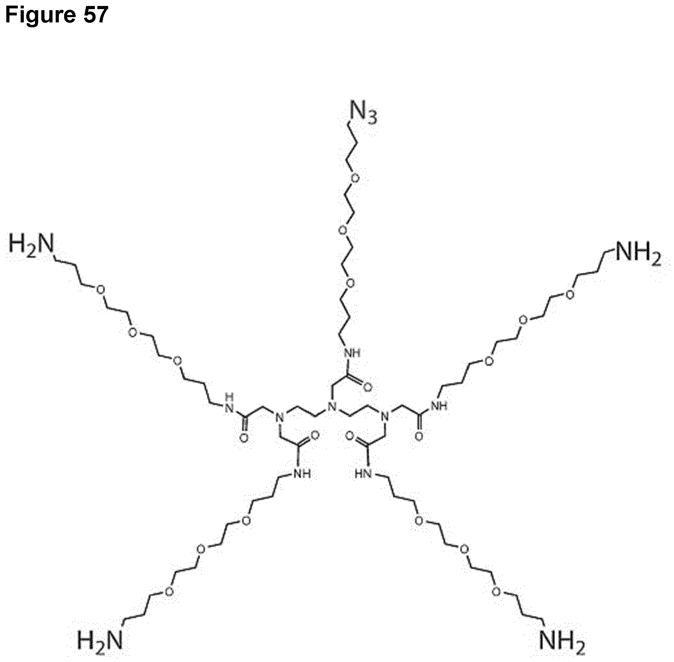

**Figure 57****:** Scaffold precursor with four amino groups for saponin linkage and an azide group for click chemistry.

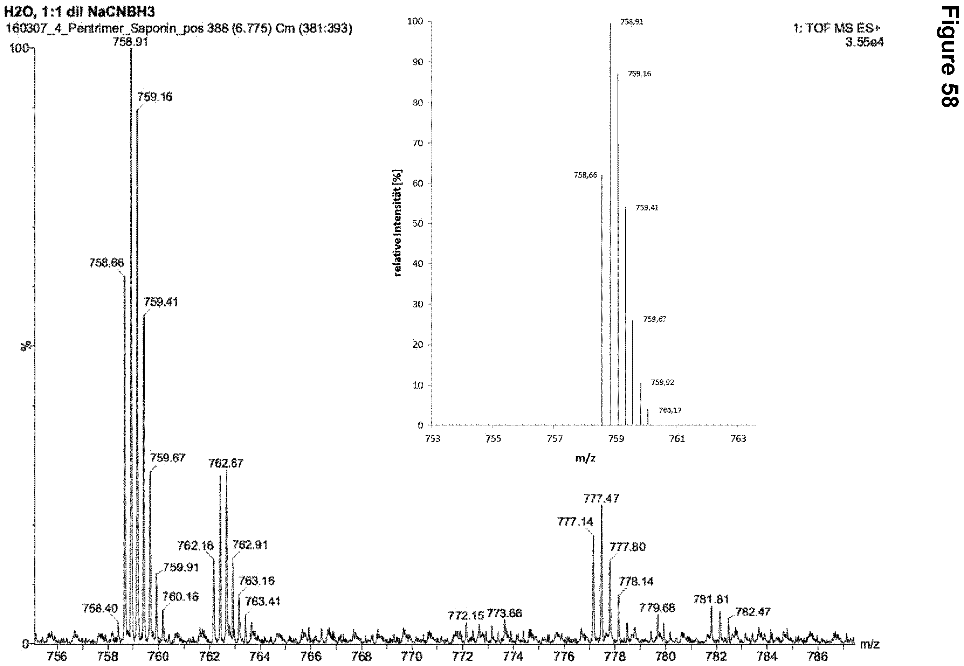

**Figure 58****:** Evidence for the coupling of saponins to the model scaffold. The inset shows the theoretically expected peeks and intensity distribution for coupled saponins. The experimental data obtained by LC-MS/ESI-MS show almost exactly the same peaks at *m*/*z* 758-760 Da proving successful saponin coupling.

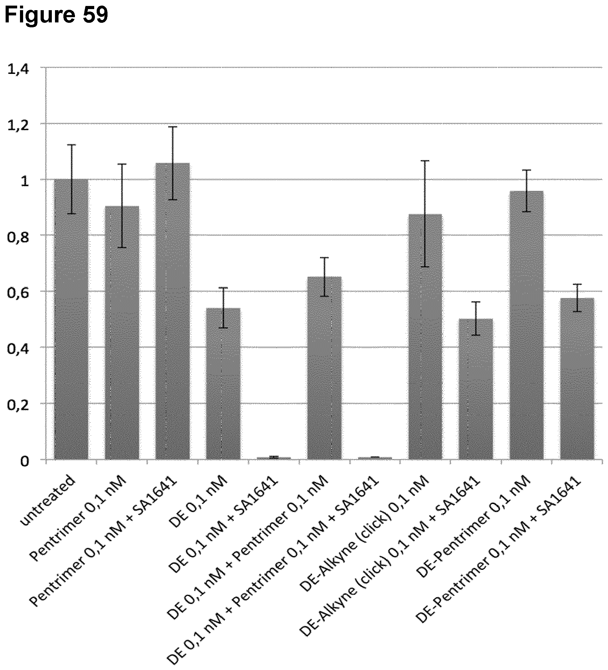

**Figure 59****:** Cytotoxicity assays using the targeted toxin dianthin-Epidermal Growth Factor (dianthin-EGF). Untreated cells were normalized to 1. The polymeric structure (Pentrimer) has no influence on cell viability neither in the presence nor in the absence of Dianthin-EGF and saponin (SA1641) indicating no intrinsic cytotoxicity of the polymeric structure. The clickable targeted toxin (Dianthin-EGF-Alkyne) has a markedly reduced activity, which is a result of the toxin modification but does not have any relation to the scaffold. The functionalized polymeric structure has the same activity as the unclicked targeted toxin, indicating that the functionalization of the scaffold does not impair effector molecule activity. The effect of saponins is identical in the presence and absence of the polymeric structure showing that the polymeric structure does not impair the efficacy of the saponins in the two-component system.

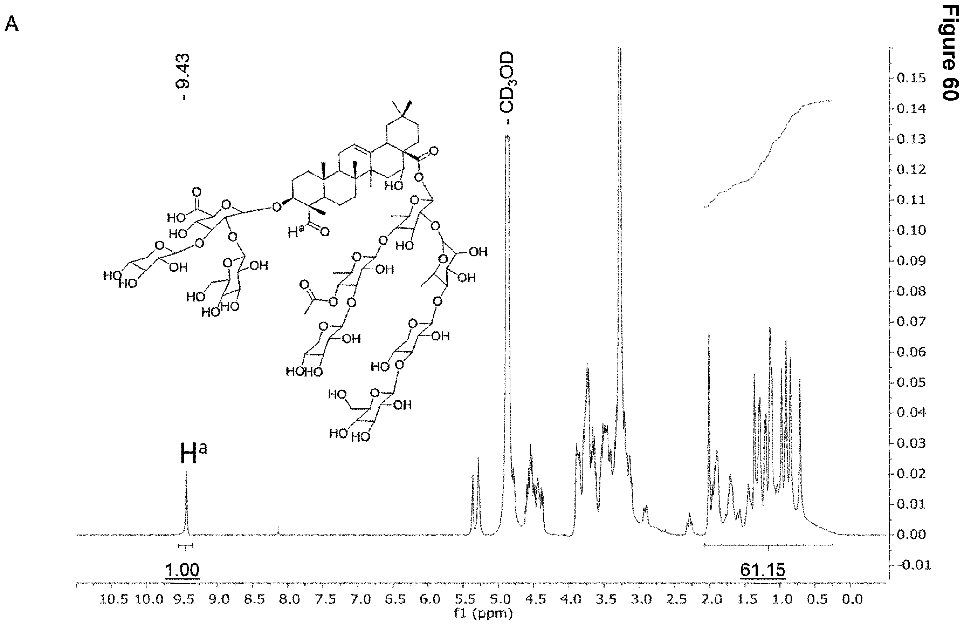

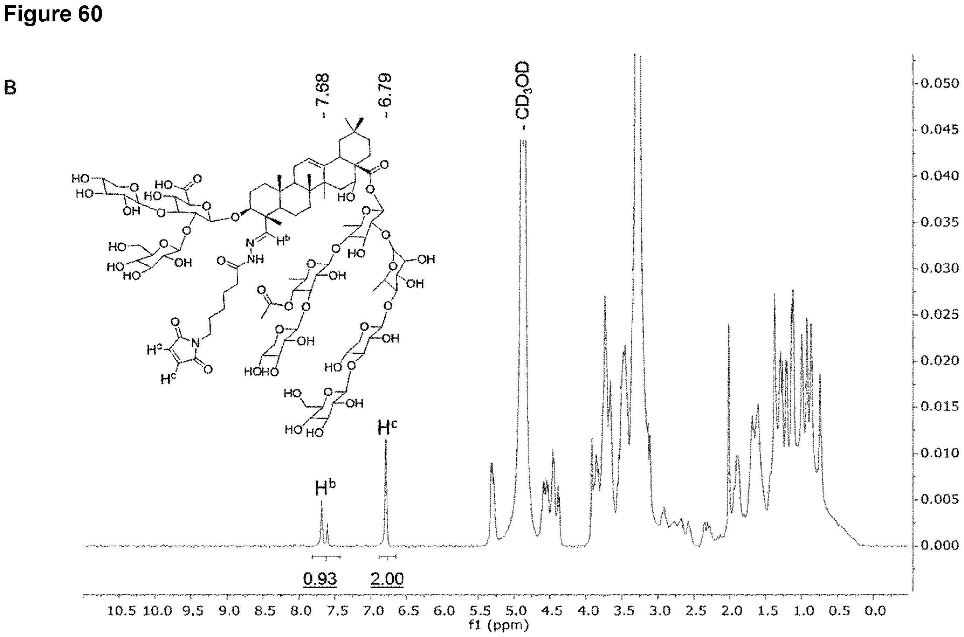

**Figure 60****:** H-NMR spectrum of (A) SO1861 and (B) SO1861-EMCH (EMCH = N-ε-maleimidocaproic acid hydrazide). (A) The peak at 9.43 ppm (H^{a}) corresponds to the aldehyde proton of SO1861.(B) The peak at 6.79 ppm (H^{c}) corresponds to the maleimide protons of SO1861-EMCH, while the peak at 7.68 ppm (H^{b}) corresponds to the hydrazone proton. The absence of the signal at 9.43 ppm indicates a quantitative conversion of the aldehyde group.

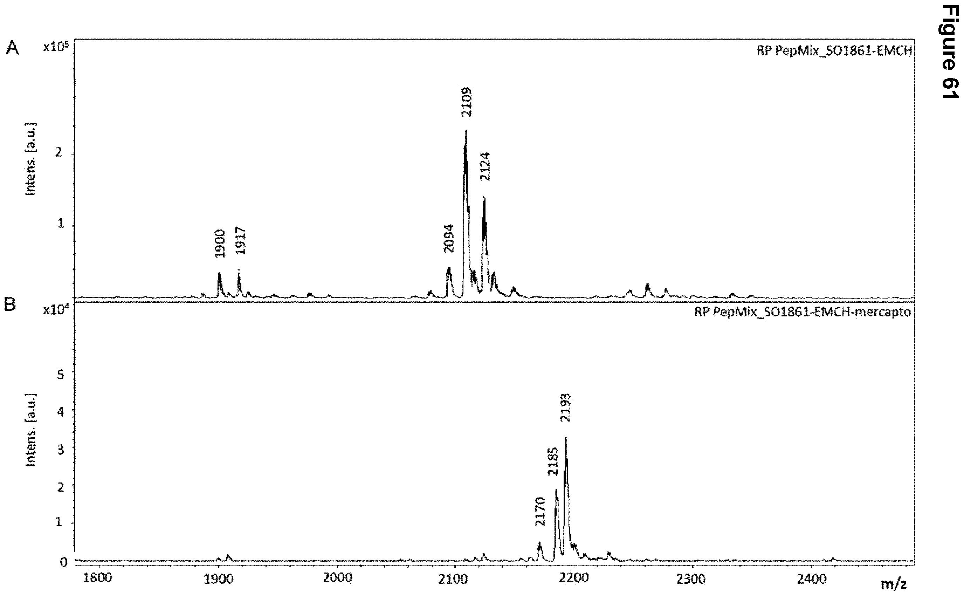

**Figure 61****:** (A) MALDI-TOF-MS spectrum of SO1861-EMCH and (B) SO1861-EMCH-mercaptoethanol. (A) RP mode: *m*/*z* 2124 Da ([M+K]⁺, saponin-EMCH), *m*/*z* 2109 Da ([M+K]⁺, SO1861-EMCH), *m*/*z* 2094 Da ([M+Na]⁺, SO1861-EMCH). (B) RP mode: *m*/*z* 2193 Da ([M+K]⁺, saponin-EMCH-mercaptoethanol), *m*/*z* 2185 Da ([M+K]⁺, SO1861-EMCH-mercaptoethanol), *m*/*z* 2170 Da ([M+Na]⁺, SO1861-EMCH-mercaptoethanol).

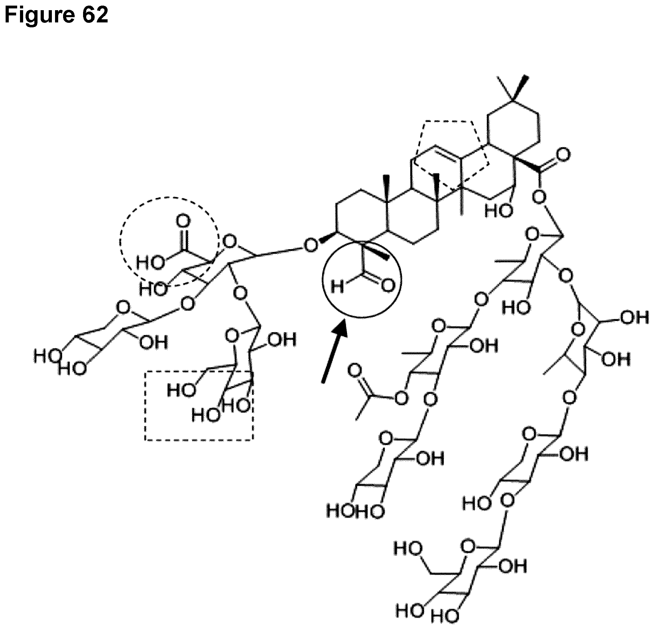

**Figure 62****:** SO1861 structure with highlighted chemical groups for conjugation of endosomal escape enhancing saponins to a polymeric structure. Highlighted groups are aldehyde (black circle), carboxylic acid (dashed circle), alkene (dashed pentagon), and alcohol (dashed box). The aldehyde group (arrow) is most suitable group for chemoselective and reversible conjugation reactions.

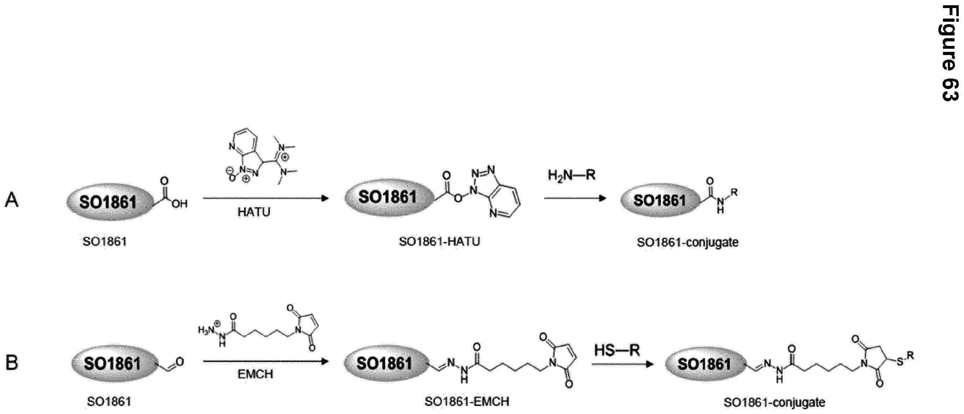

**Figure 63****:** Strategy for producing (A) stable and (B) cleavable 'ready-to conjugate' endosomal escape enhancer saponins.

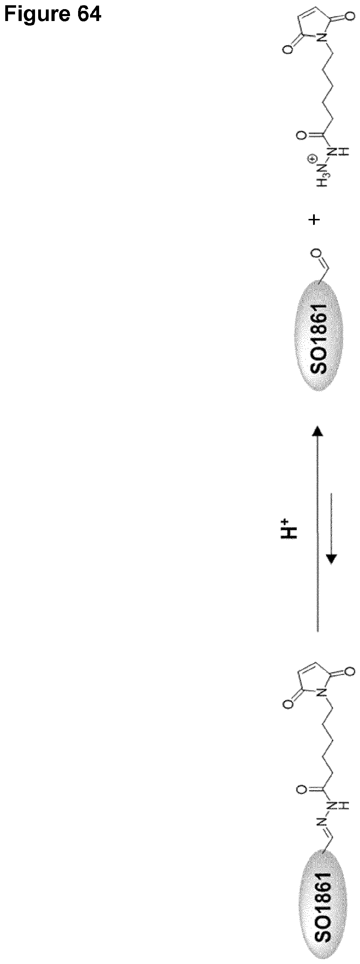

**Figure 64****:** Hydrolysis of the hydrazone bond of SO1861-EMCH under acidic conditions.

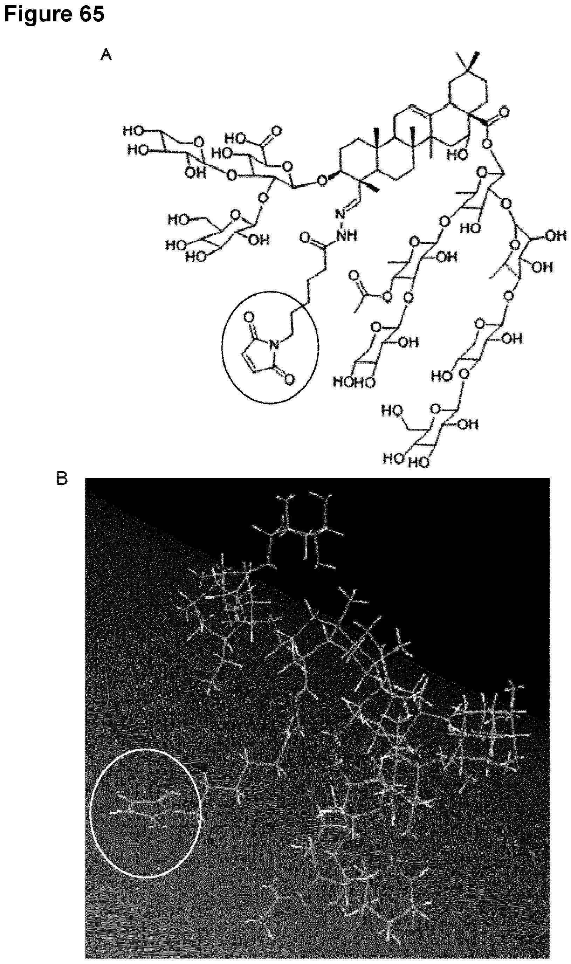

**Figure 65****:** SO1861-EMCH structure. (A) Standard molecular structure, and (B) 3D model. Maleimide group is marked with a circle.

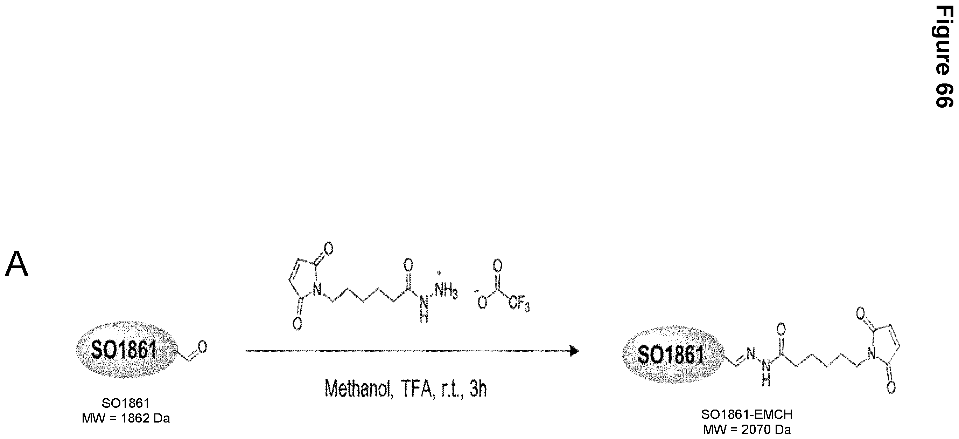

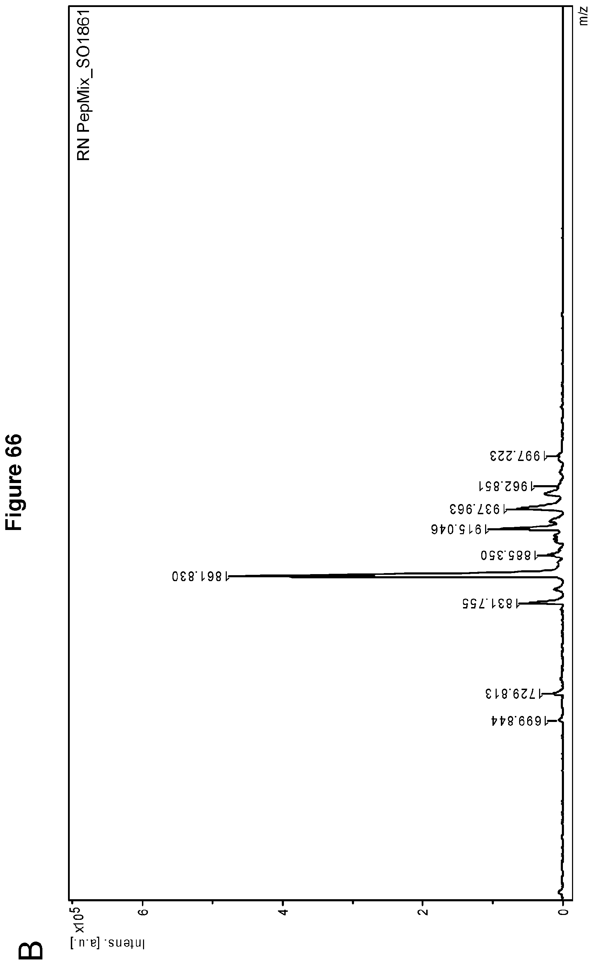

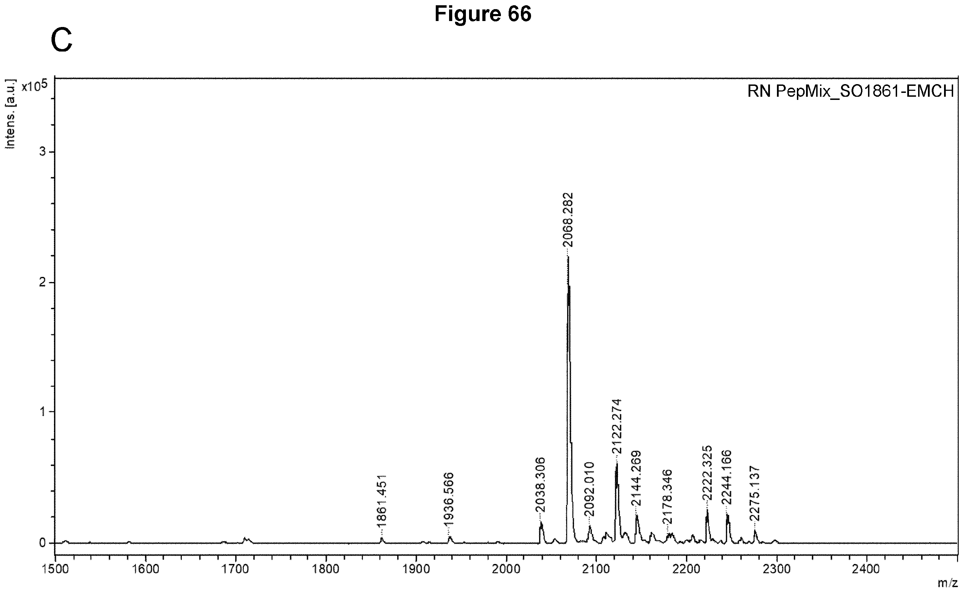

**Figure 66****:** (A) SO1861-EMCH synthesis scheme. (B) MALDI-TOF-MS spectra of SO1861 (*m*/*z* 1861 Da) and (C) SO1861-EMCH (*m*/*z* 2068 Da) in negative reflector mode. TFA: trifluoroacetic acid, r.t: room temperature, h: hours, and MW: molecular weight.

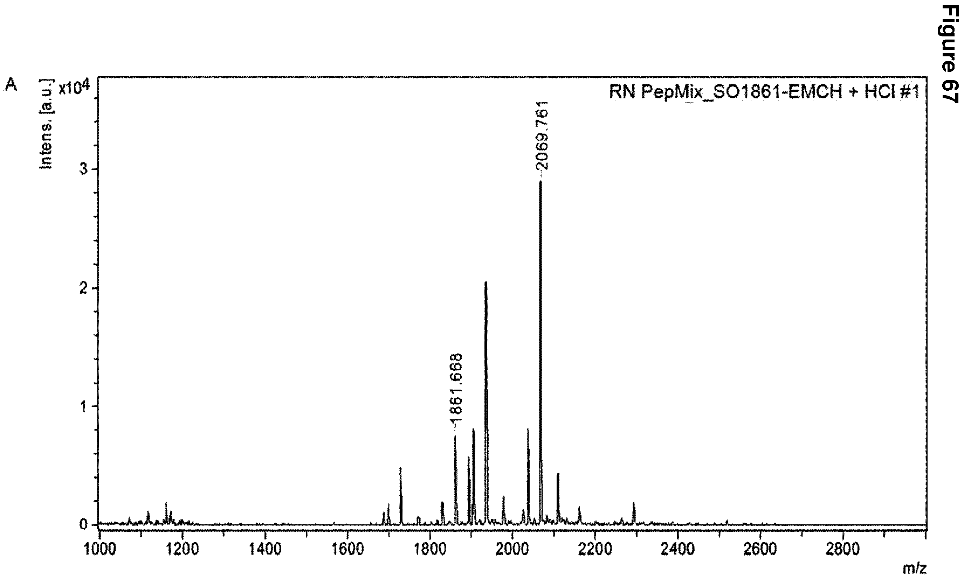

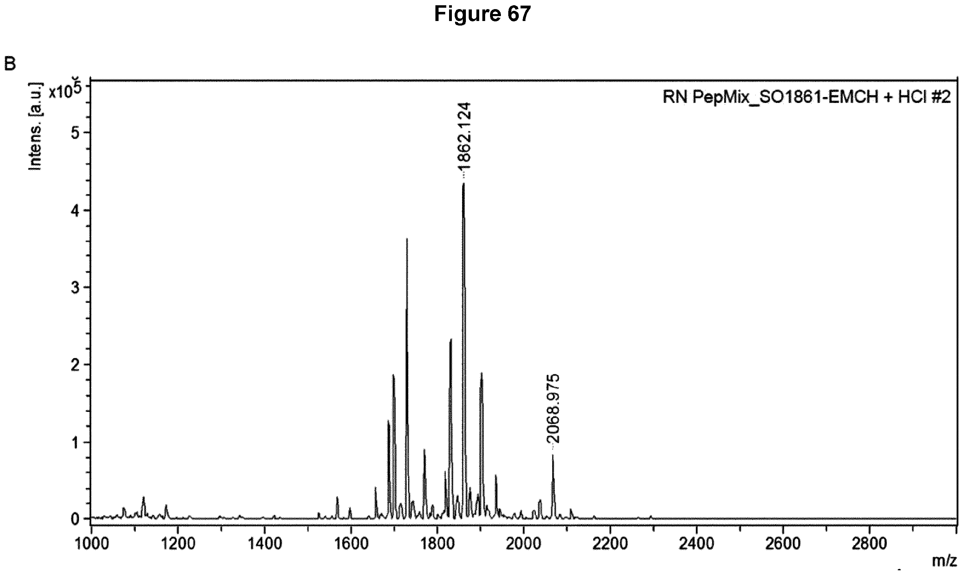

**Figure 67****:** MALDI-TOF-MS spectra of SO1861-EMCH (A) before and (B) after hydrolysis in HCI solution at pH 3.

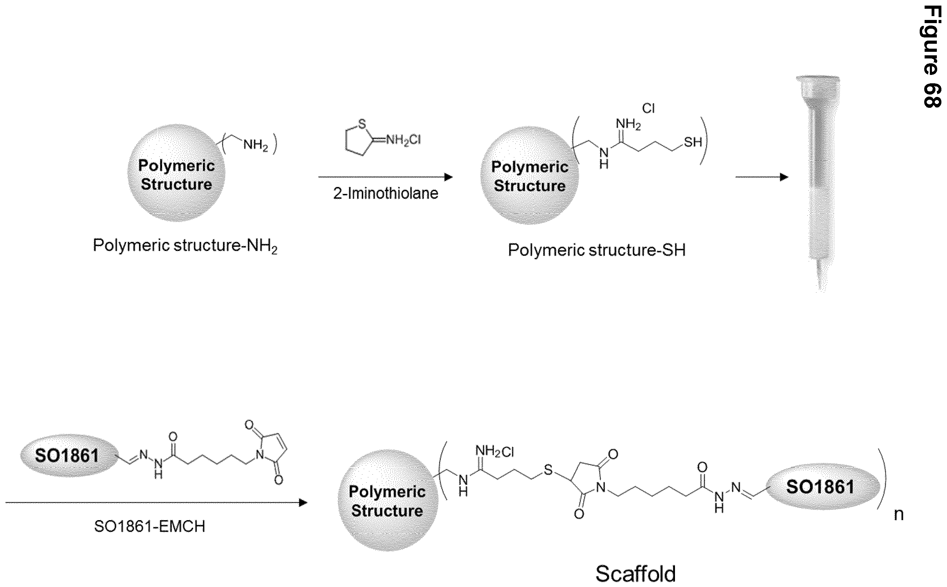

**Figure 68****:** Reaction scheme of SO1861-EMCH conjugation to any amine-bearing polymeric structure.

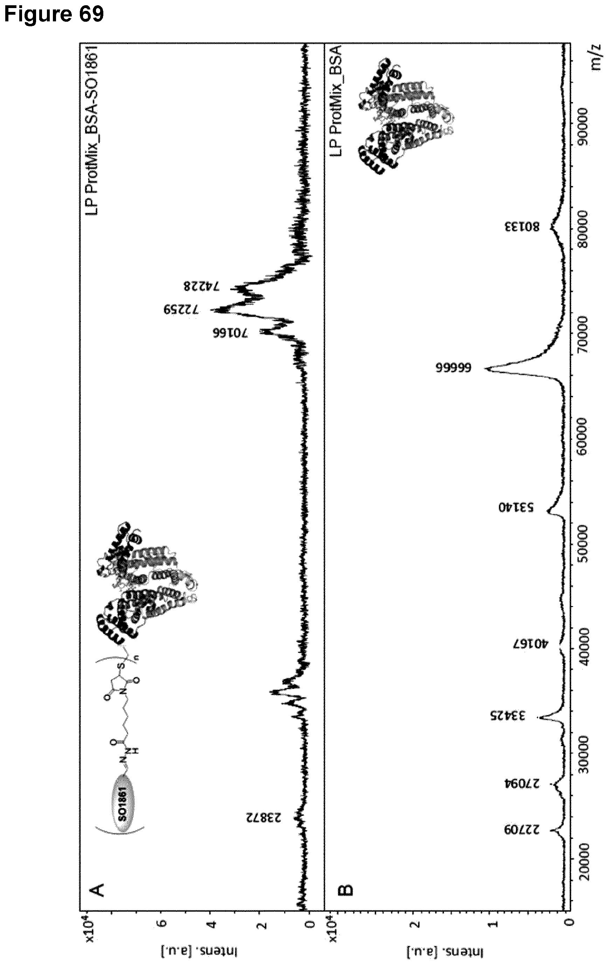

**Figure 69****:** MALDI-TOF-MS spectra of (A) BSA-SO1861 (*m*/*z* 70.0 kDa, 72.1 kDa, 74.2 kDa), and (B) BSA (*m*/*z* 66.6 kDa).

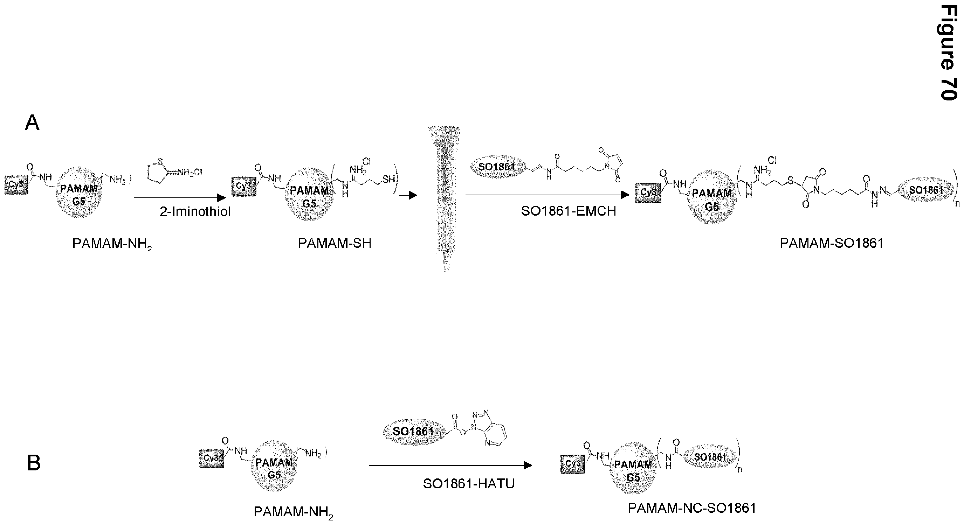

**Figure 70****:** Reaction scheme of (A) SO1861-EMCH and (B) SO1861-HATU (HATU = 1-[Bis(dimethylamino)methylene]-1H-1,2,3-triazolo[4,5-b]pyridinium 3-oxid hexafluorophosphate) conjugation to a cyanine 3 dye labeled polyamidoamine (PAMAM) G5 dendrimer.

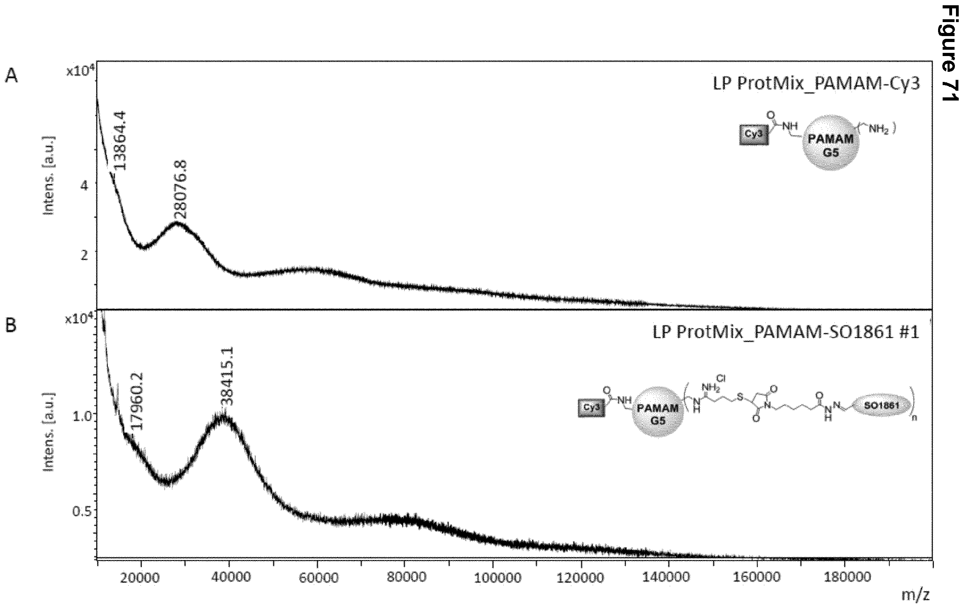

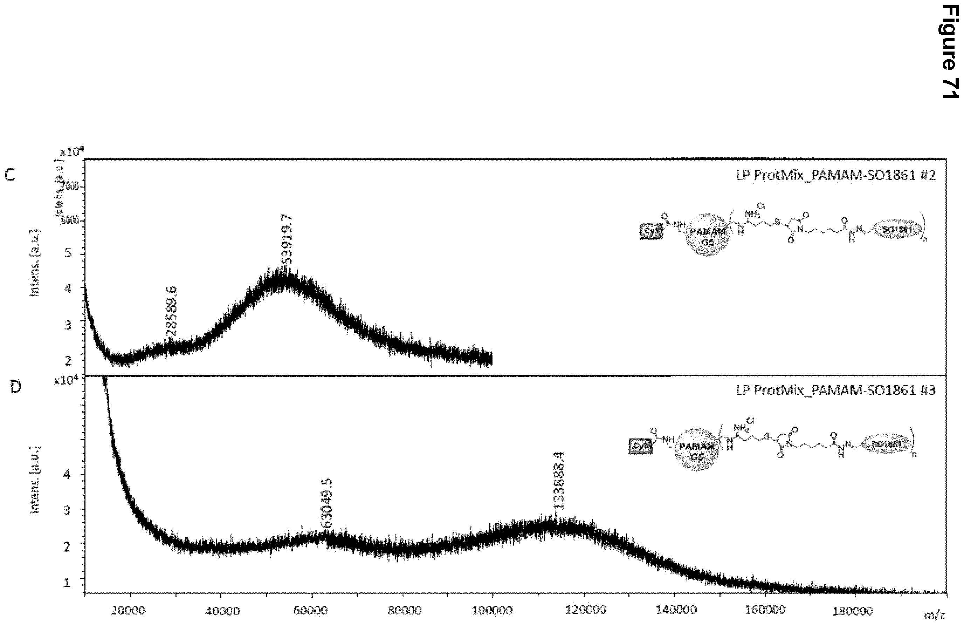

**Figure 71****:** MALDI-TOF-MS spectra of (A) Cy3-PAMAM, (B-D) Cy3-PAMAM-SO1861 with increasing SO1861-EMCH feed equivalents from (B) up to bottom (D). (B) corresponds to Cy3-PAMAM-SO1861 with 5 SO1861 attached per PAMAM, (C) corresponds to Cy3-PAMAM-SO1861 with 13 SO1861 attached per PAMAM, and (D) corresponds to Cy3-PAMAM-SO1861 with 51 SO1861 attached per PAMAM.

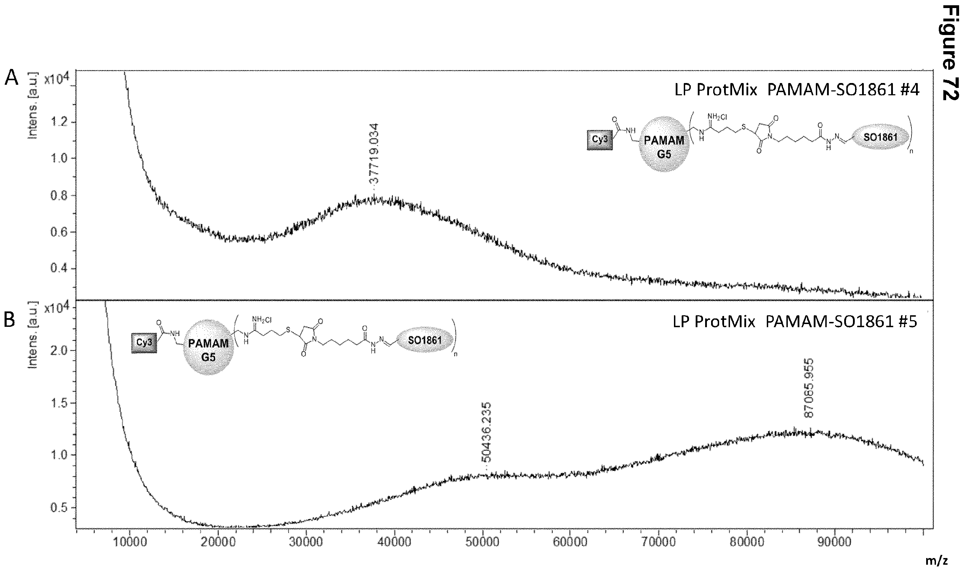

**Figure 72****:** MALDI-TOF-MS spectra of (A) Cy3-PAMAM-SO1861 with 5 equivalents feed SO1861-EMCH and (B) Cy3-PAMAM-SO1861 with 30 equivalents feed SO1861-EMCH.

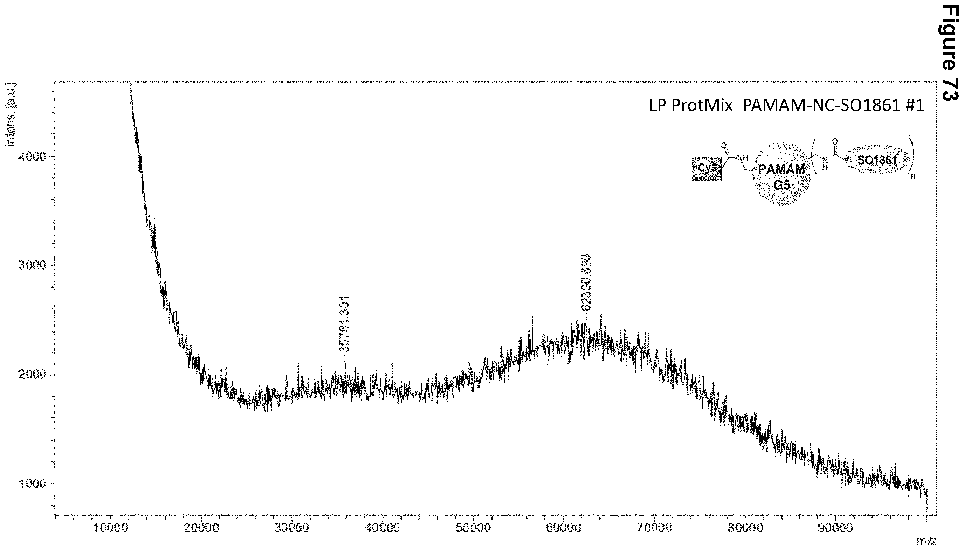

**Figure 73****:** MALDI-TOF-MS spectra of Cy3-PAMAM-NC-SO1861 (NC = stable bond ("non-cleavable").

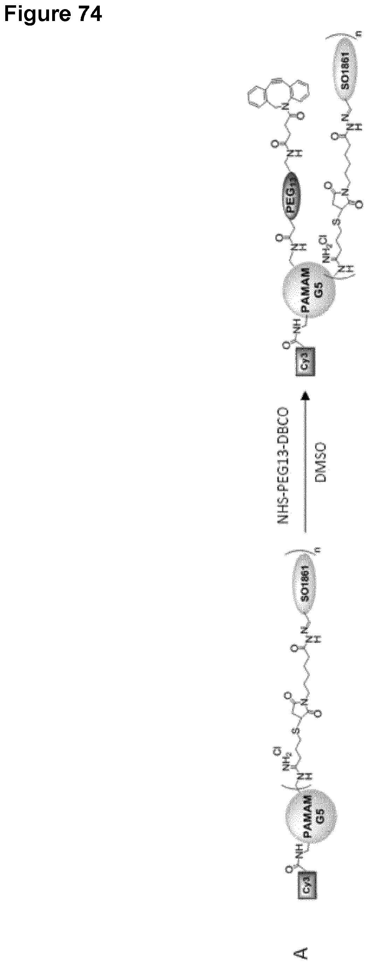

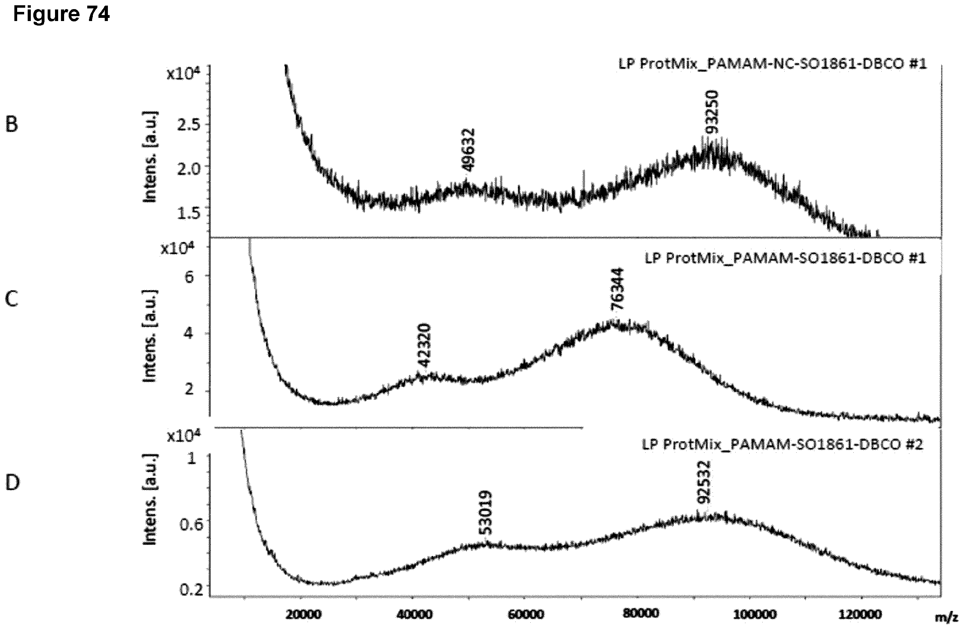

**Figure** 74: (A) Reaction scheme and MALDI-TOF-MS spectra of (B) Cy3-PAMAM-NC-SO1861-Dibenzocyclooctyne (DBCO), (C) Cy3-PAMAM-(SO1861)₅-DBCO, and (D) Cy3-PAMAM-(SO1861)₂₇-DBCO.

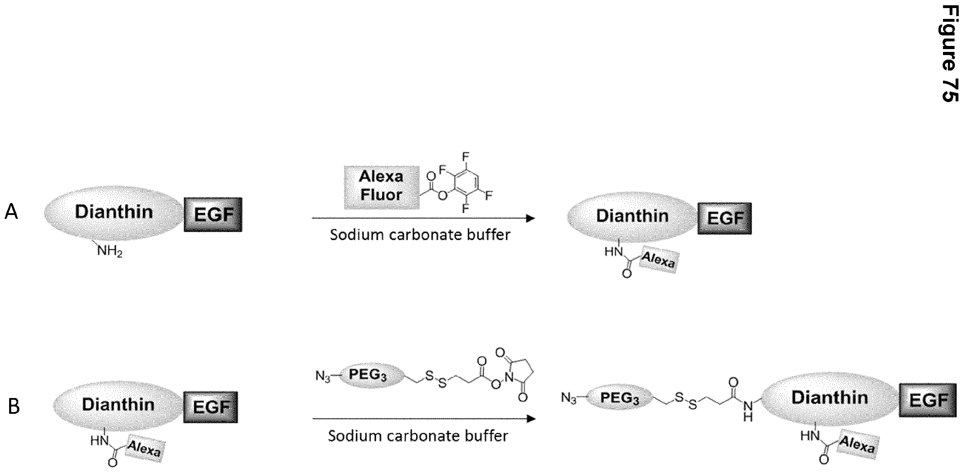

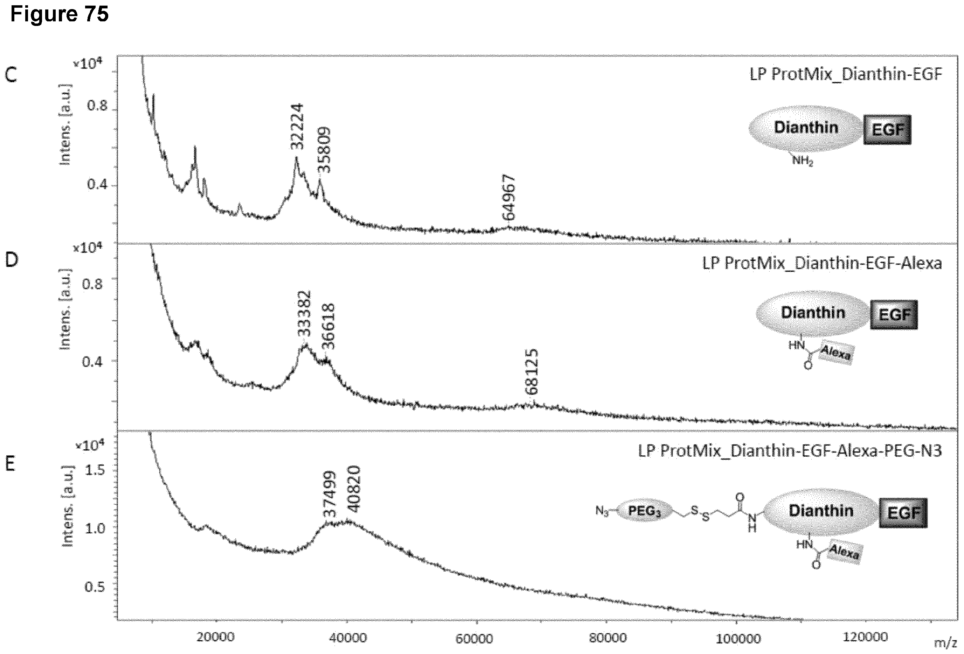

**Figure 75****:** Reaction scheme of (A) dianthin-EGF-Alexa488 and (B) dianthin-EGF-Alexa488-SS-PEG-N₃. MALDI-TOF-MS spectra of (C) dianthin-EGF, (D) dianthin-EGF-Alexa488, and (E) dianthin-EGF-Alexa488-SS-PEG-N₃; Alexa488: Alexa Fluor 488 dye.

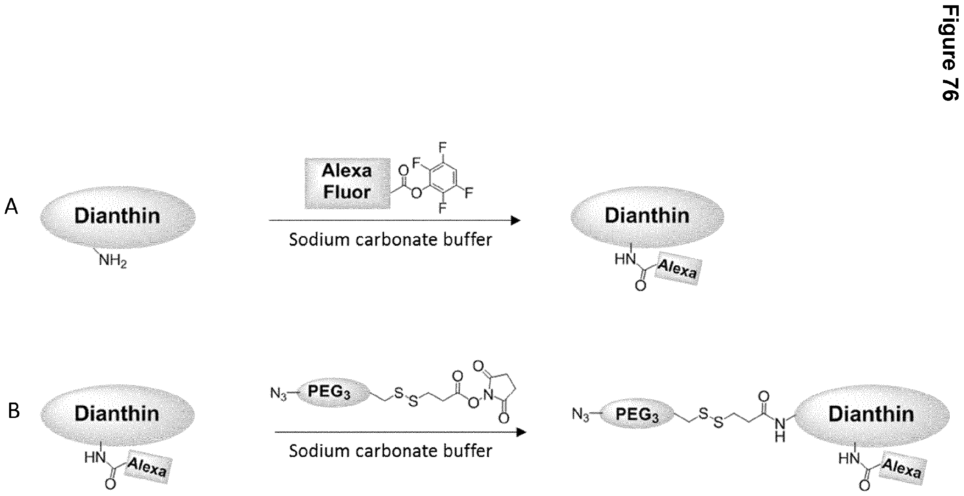

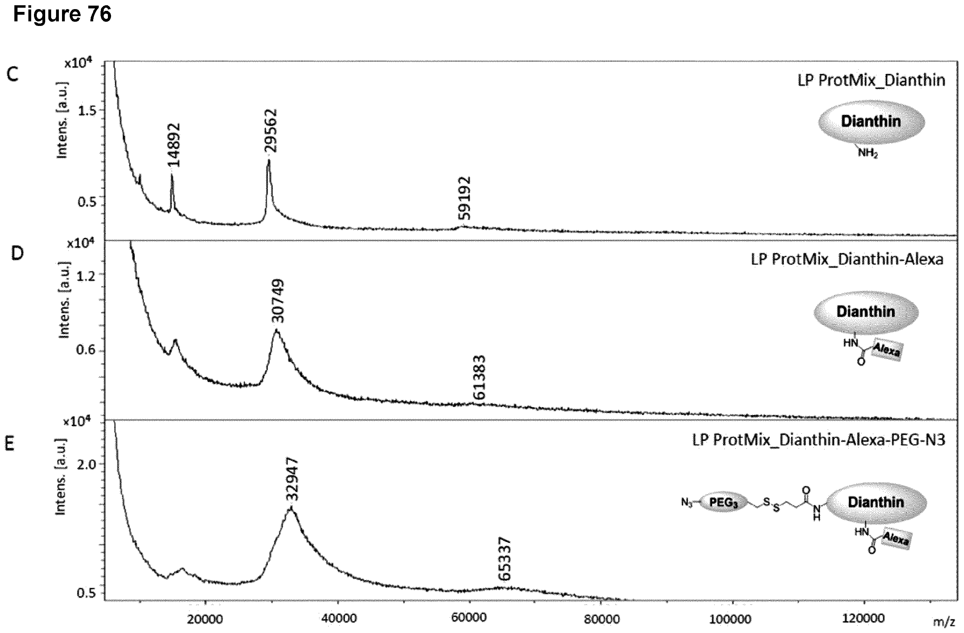

**Figure 76****:** Reaction scheme of (A) dianthin-Alexa488 and (B) dianthin-Alexa488-SS-PEG-N₃. MALDI-TOF-MS spectra of (C) dianthin, (D) dianthin-Alexa488, and (E) dianthin-Alexa488-SS-PEG-N₃; Alexa488: Alexa Fluor 488 dye.

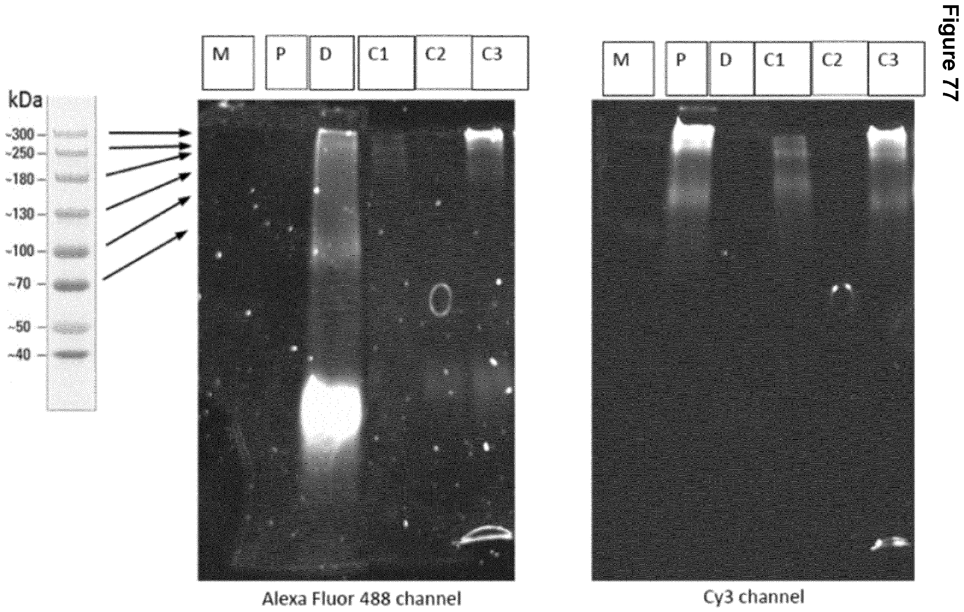

**Figure 77****:** Fluorescence images of SDS-PAGE gel performed on a VersaDoc imaging system. M = marker, P = Cy3-PAMAM-(SO1861)₂₇-DBCO, D = dianthin-EGF-Alexa488-SS-PEG-N₃, C1 = Cy3-PAMAM-(SO1861)₅-Dianthin-EGF-Alexa488, C2 = Cy3-PAMAM-NC-SO1861-Dianthin-EGF-Alexa488, and C3 = Cy3-PAMAM-(SO1861)₂₇-Dianthin-EGF-Alexa488.

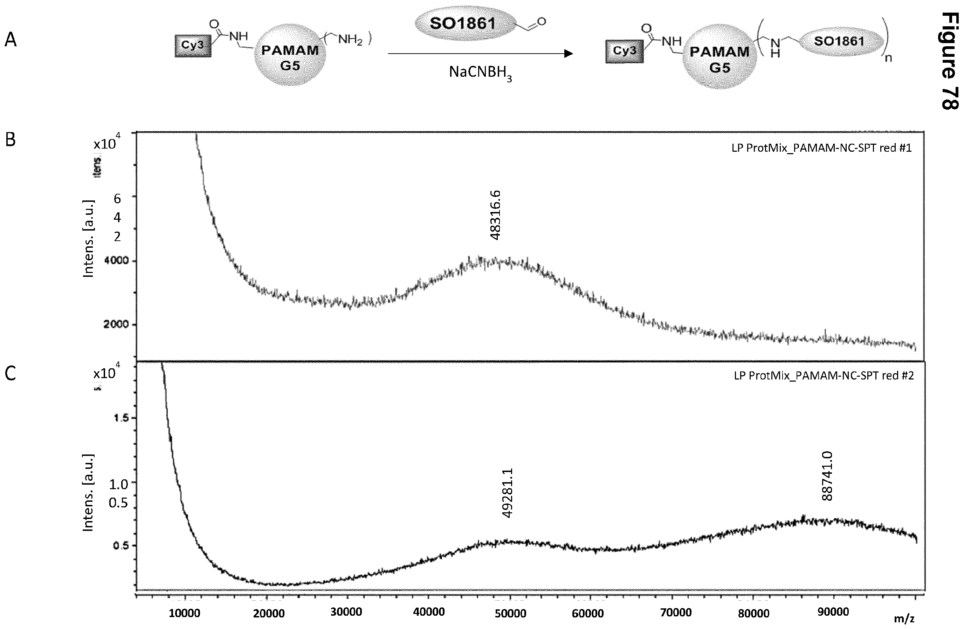

**Figure 78****:** (A) Synthesis scheme of Cy3-PAMAM-NC-SO1861 via reductive amination. (B, and C) Respective MALDI-TOF-MS spectra.

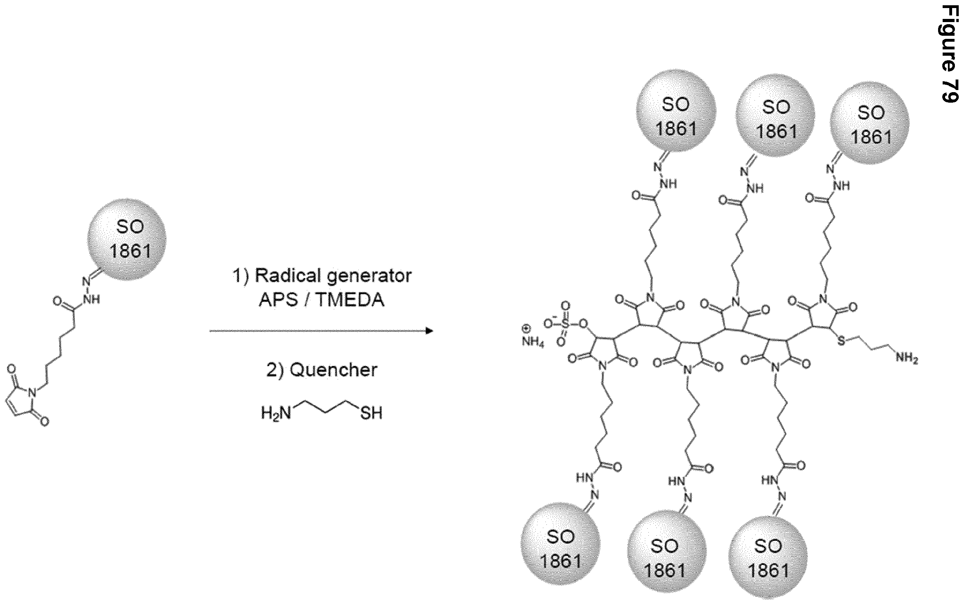

**Figure 79****:** Reaction scheme for the generation of poly(SO1861) using SO1861-EMCH as monomer, the APS / TMEDA system as polymerization initiator, and aminopropanethiol as radical quencher.

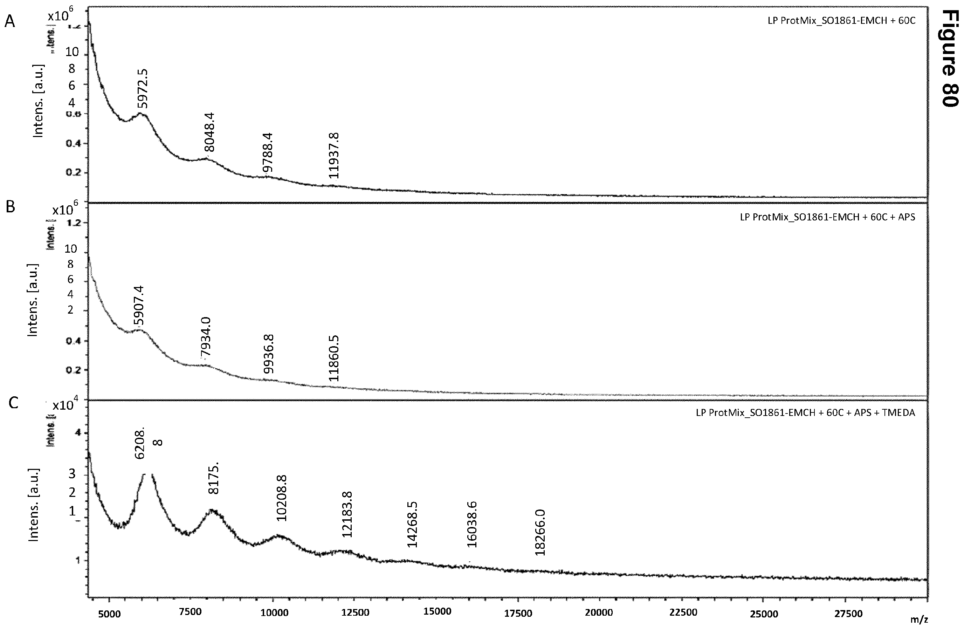

**Figure 80****:** MALDI-TOF-MS spectra of poly(SO1861) reaction batches. (A) SO1861-EMCH at 60 °C, (B) SO1861-EMCH + 11⁻³ equivalents APS at 60 °C, (C) SO1861-EMCH + 11⁻³ equivalents APS/TMEDA at 60 °C.

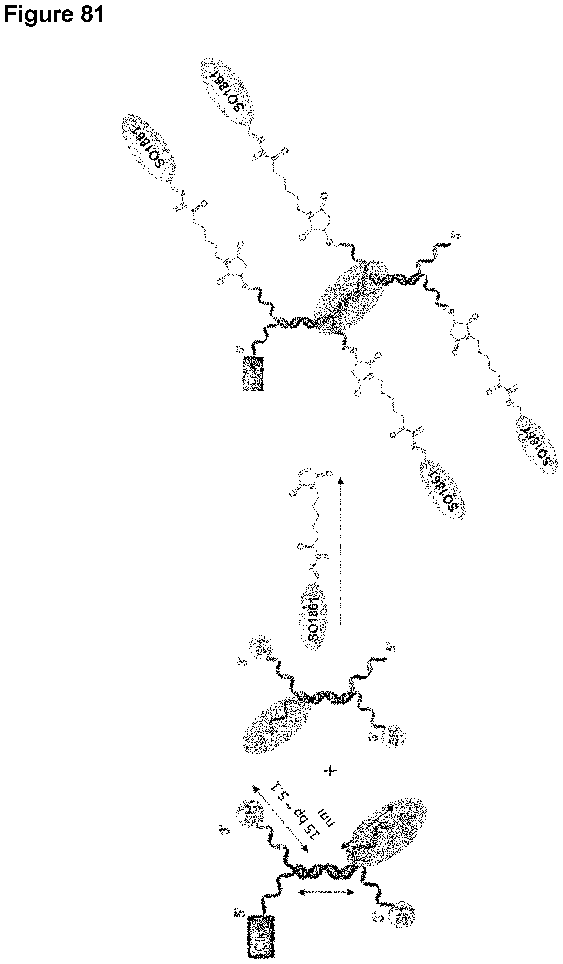

**Figure 81****:** DNA approach. Usage of the principle of DNA-origami to generate a DNA based scaffold that is able to conjugate and release glycoside molecules. In addition, one of the DNA strands obtains a click chemistry moiety that can be used for conjugation to a targeted toxin to form a functionalized scaffold. bp: base pair.

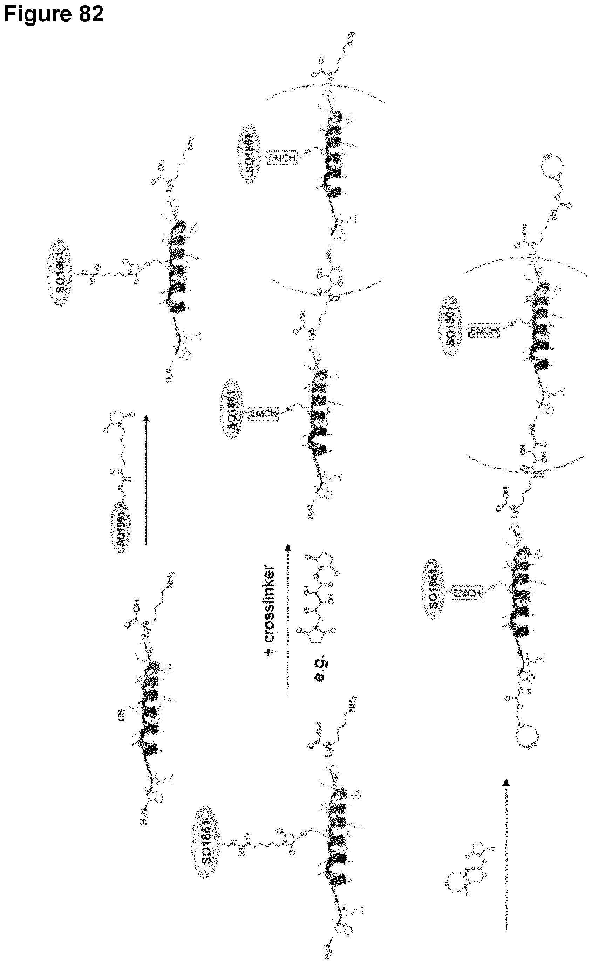

**Figure 82****:** Poly(peptide-SO1861) approach. Usage of a peptide sequence that can conjugate and release glycoside molecules and which can react with itself to form a poly(peptide-SO1861) construct. The poly(peptide) chain endings can be further modified with click chemistry moieties (e.g., BCN-NHS linker) that can be used for conjugation to a toxin.

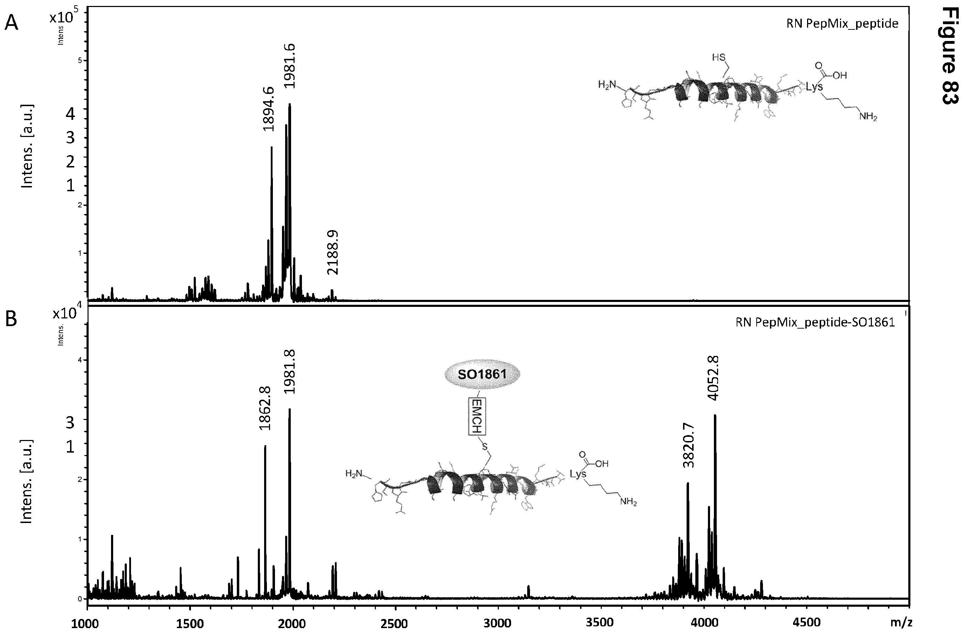

**Figure 83****.** MALDI-TOF-MS spectra of (A) native peptide, (B) peptide-S01861 conjugate.

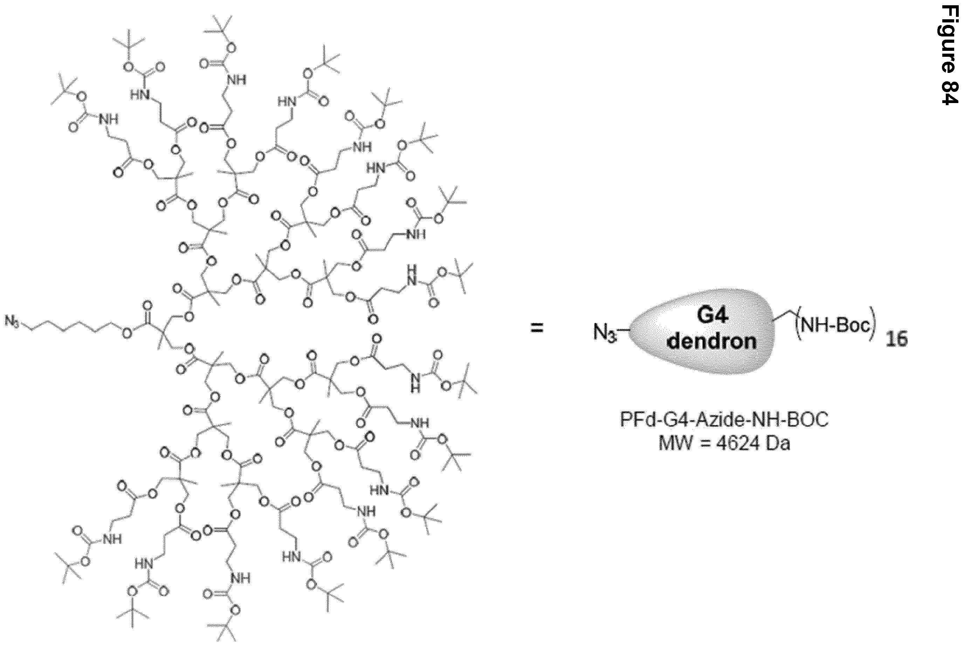

**Figure 84****.** Molecular structure of G4-dendron with protected amino groups.

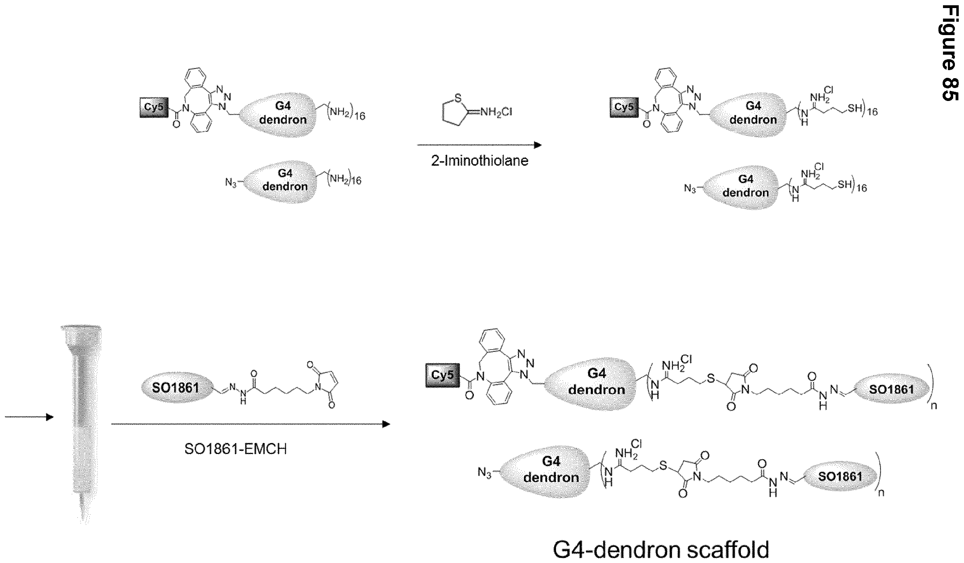

**Figure 85****.** Synthesis scheme for the generation of dendron based scaffolds and functional scaffolds.

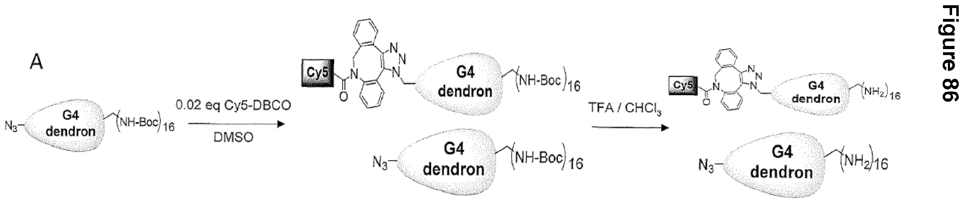

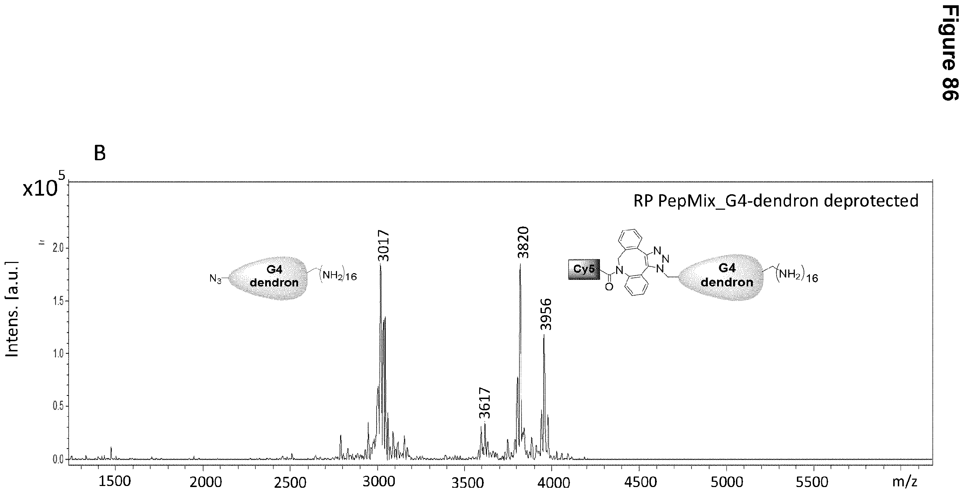

**Figure 86****.** (A) Reaction scheme for partial dye labeling and deprotection of the G4-dendron. (B) MALDI-TOF-MS spectrum of deprotected and partially dye labeled G4-dendron.

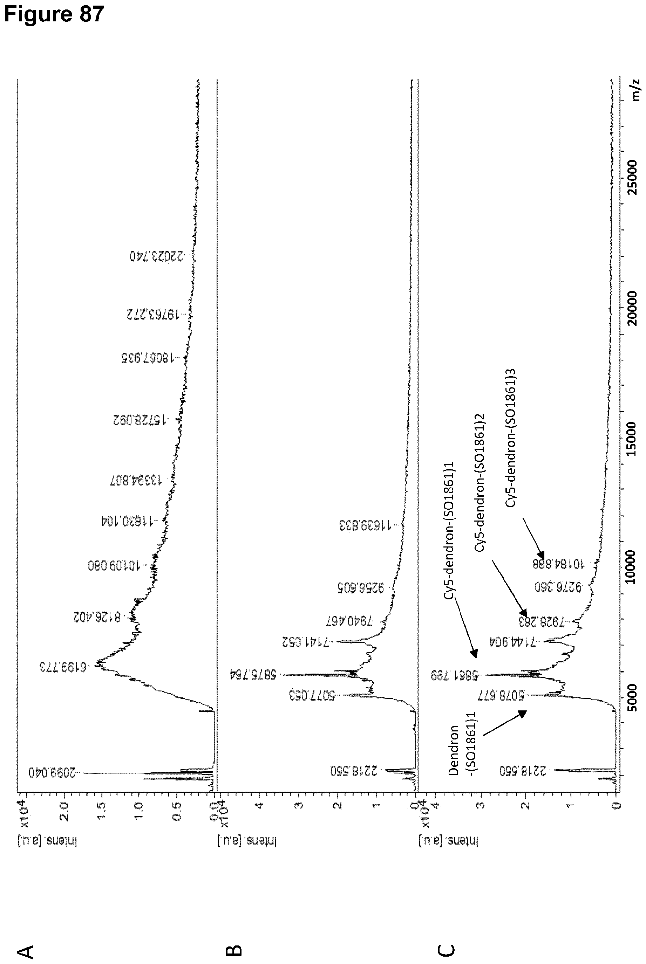

**Figure 87****.** MALDI-TOF-MS spectra of G4-dendron-SO1861 scaffolds with (A) 22 feed equivalents of SO1861-EMCH, (B) 10 feed equivalents of SO1861-EMCH, and (C) 3 feed equivalents of SO1861-EMCH.

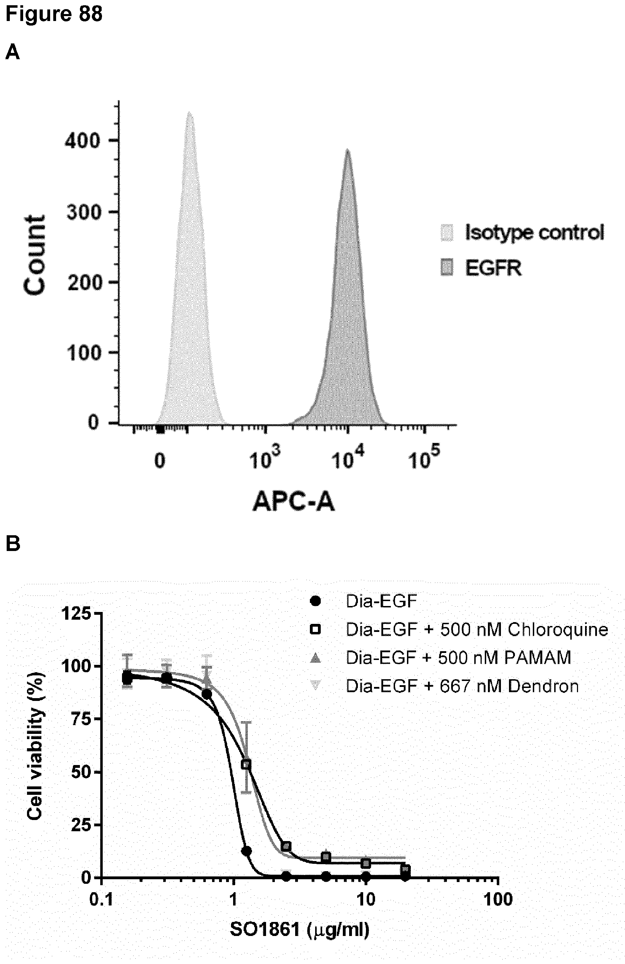

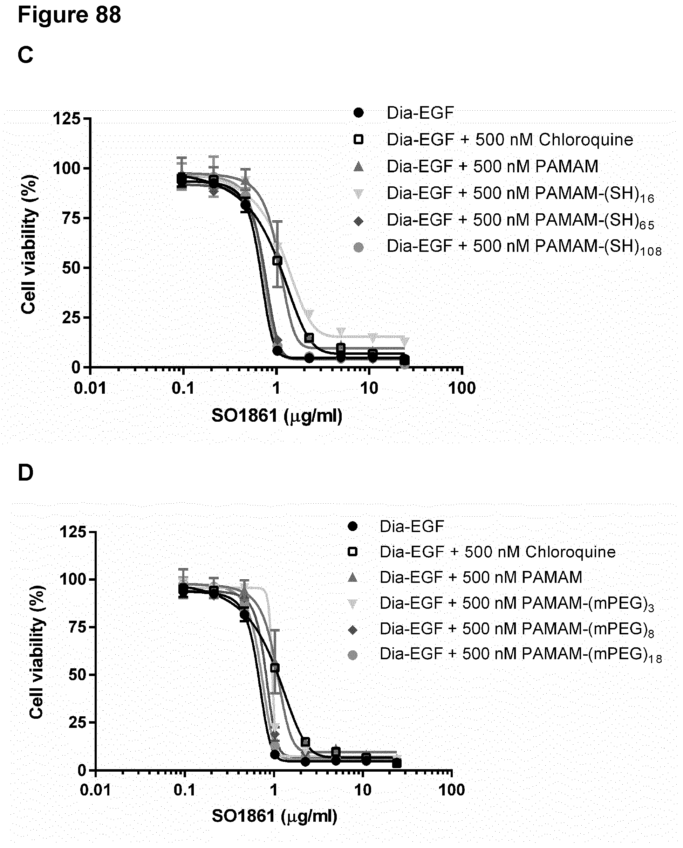

**Figure 88****.** Cell viability curves of HeLa cells treated with (A) EGFR cell surface expression as determined by FACS analyses of HeLa cells (B, see Table 19), cell viability of HeLa cells treated with SO1861 + dianthin-EGF (Dia-EGF), SO1861 + dianthin-EGF + 500 nM chloroquine, SO1861 + dianthin-EGF + 500 nM PAMAM, SO1861 + dianthin-EGF + 667 nM dendron (C) cell viability of HeLa cells treated with SO1861 + dianthin-EGF, SO1861 + dianthin-EGF + 500 nM chloroquine, SO1861 + dianthin-EGF + 500 nM PAMAM, SO1861 + dianthin-EGF + 500 nM PAMAM-(SH)₁₆, SO1861 + dianthin-EGF + 500 nM PAMAM-(SH)₆₅, SO1861 + dianthin-EGF + 500 nM PAMAM-(SH)₁₀₈ (D) cell viability of HeLa cells treated with SO1861 + dianthin-EGF, SO1861 + dianthin-EGF + 500 nM chloroquine, SO1861 + dianthin-EGF + 500 nM PAMAM, SO1861 + dianthin-EGF + 500 nM PAMAM-(mPEG)₃, SO1861 + dianthin-EGF + 500 nM PAMAM-(mPEG)₈, SO1861 + dianthin-EGF + 500 nM PAMAM-(mPEG)₁₈.

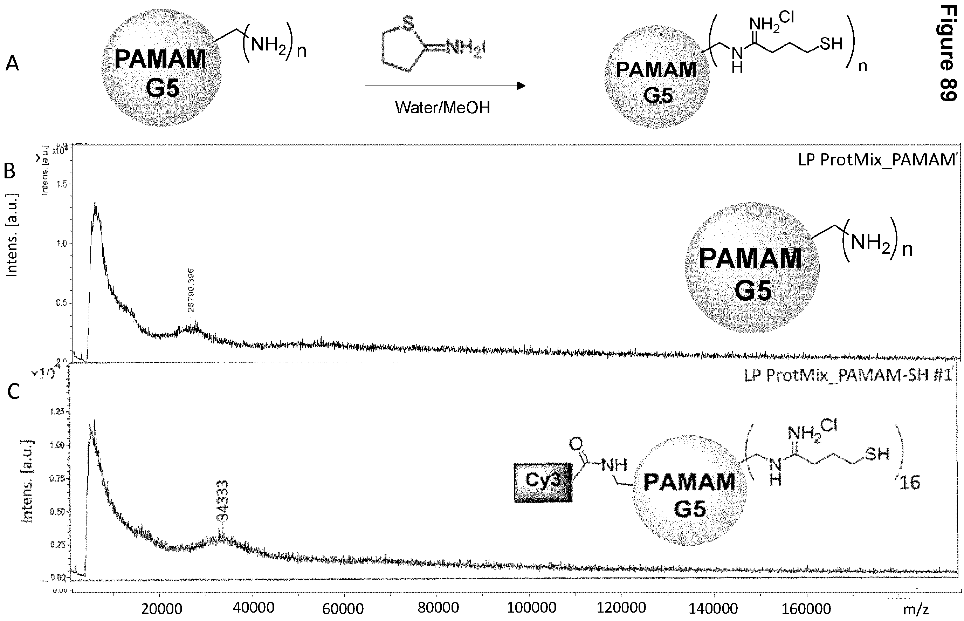

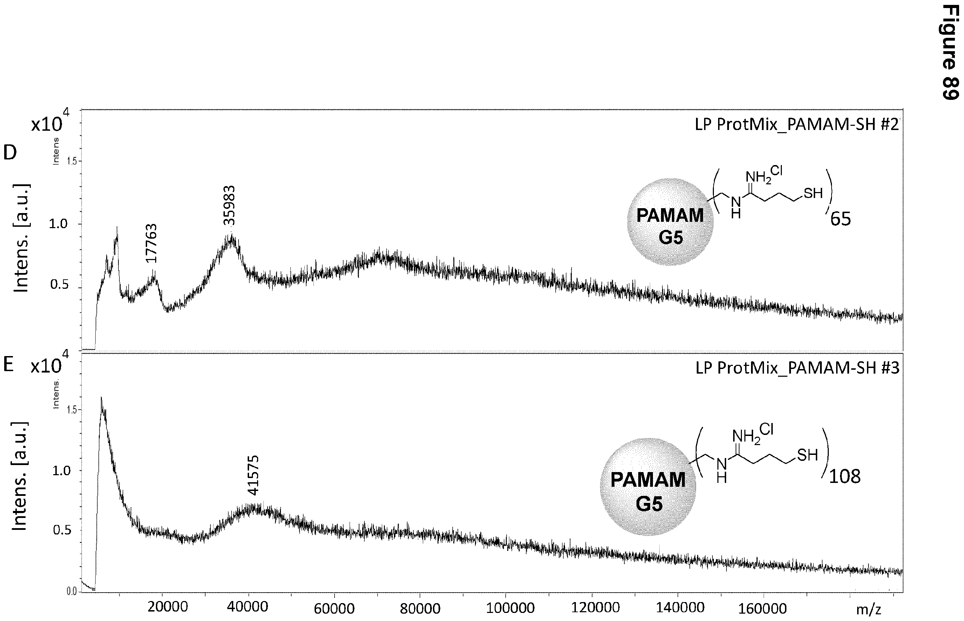

**Figure 89****.** (A) Reaction scheme of the thiolation of PAMAM using the thiolation reagent 2-iminothiolane. MALDI-TOF-MS spectra of (B) native PAMAM, (C) thiolated PAMAM-(SH)₁₆, (D) thiolated PAMAM-(SH)₆₅, and (E) thiolated PAMAM-(SH)₁₀₈.

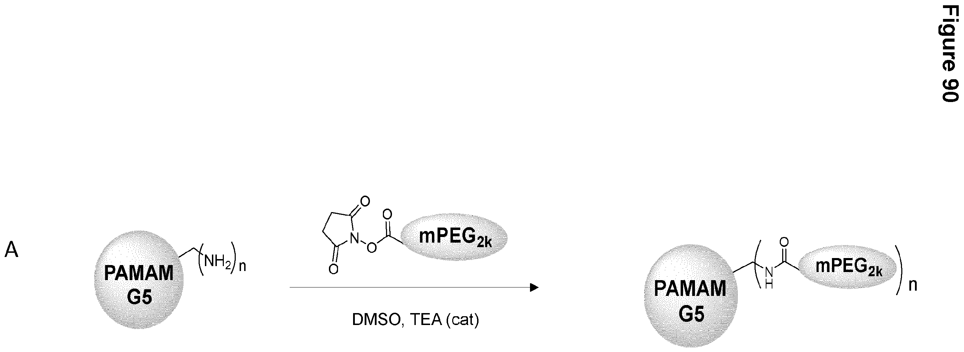

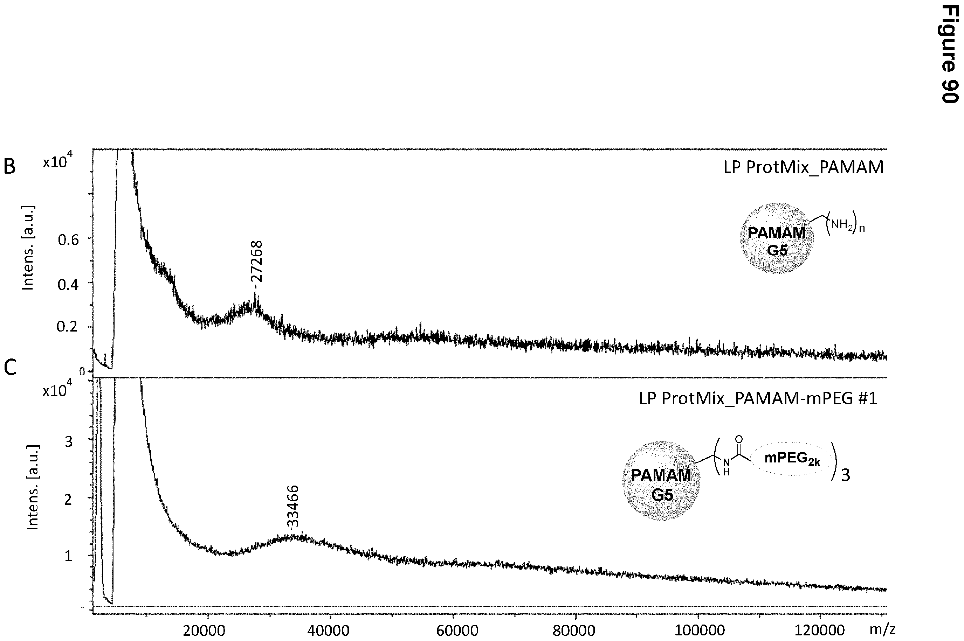

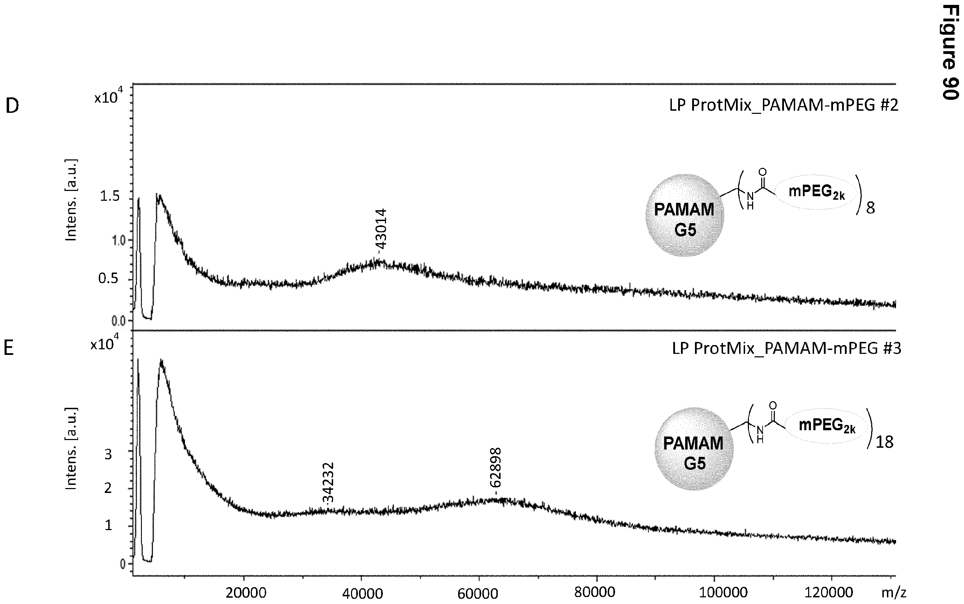

**Figure 90****.** (A) Reaction scheme of the PEGylation of PAMAM using the PEGylating reagent mPEG₂ₖ-NHS. MALDI-TOF-MS spectra of (B) native PAMAM, (C) PEGylated PAMAM-(mPEG₂ₖ)₃, (D) PEGylated PAMAM-(mPEG₂ₖ)₈, and (E) PEGylated PAMAM-(MPEG₂ₖ)₁₈.

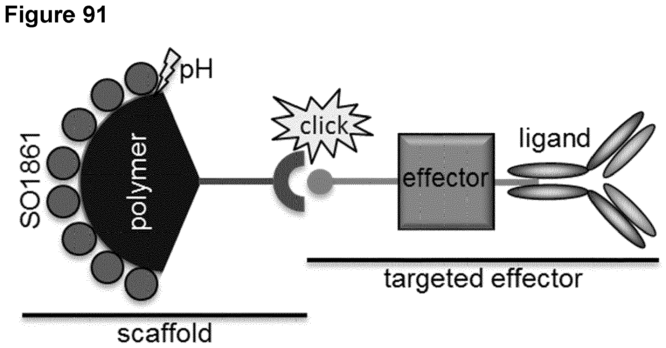

**Figure 91****:** Basic scaffold with click chemistry function to link any desired effector molecule. The user determines the position of the click chemistry position in the effector molecule and all further properties of the effector molecule, e.g. choice and position of an optional ligand.

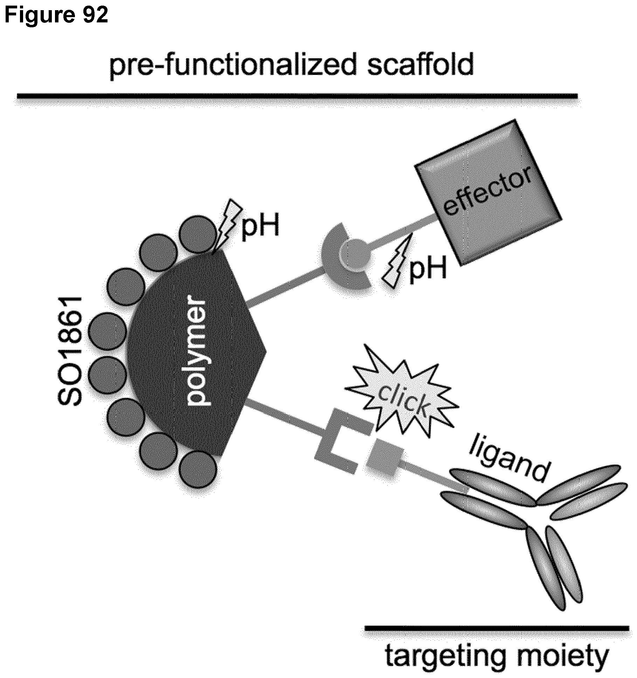

**Figure 92****:** Functionalized scaffold with pre-bound effector molecule and click chemistry function to link any desired ligand. Optionally, a pH-sensitive linkage can be provided to release the effector molecule from the scaffold after reaching the endosomes.

### DETAILED DESCRIPTION

The invention is defined by the claims. Any subject-matter falling outside the scope of the claims is provided for information purposes only.

In order for a bioactive molecule to work, the molecule must be able to engage with its target, e.g. in the blood serum, on the outside of the cell surface or inside a cell or an organelle. The active moiety of almost all protein-based targeted toxins, e.g., must enter the cytosol of the target cell to mediate its target modulatory effect. In many constellations the toxin remains ineffective since (1) the targeting moiety is poorly internalized and remains bound to the outside of the cells, (2) is recycled back to the cell surface after internalization or (3) transported to the endolysosomes where it is degraded. Although these fundamental issues are known for decades and more than 500 targeted toxins have been investigated in the past decades, the problems have not been solved yet and only one antibody-targeted protein toxin, moxetumomab pasudotox-tdfk (LUMOXITI®, AstraZeneca Pharmaceuticals LP), has been approved for relapsed or refractory hairy cell leukemia by the FDA to date.

To overcome these problems, many strategies have been described including approaches to redirect the toxins to endogenous cellular membrane transport complexes of the biosynthetic pathway in the endoplasmic reticulum and techniques to disrupt or weaken the membrane integrity of endosomes, i.e. the compartments of the endocytic pathway in a cell, and thus facilitating the endosomal escape. This comprises the use of lysosomotropic amines, carboxylic ionophores, calcium channel antagonists, various cell-penetrating peptides of viral, bacterial, plant, animal, human and synthetic origin, other organic molecules and light-induced techniques. Although the efficacy of the targeted toxins was typically augmented in cell culture hundred- or thousand-fold, in exceptional cases more than million-fold, the requirement to co-administer endosomal escape enhancers with other substances harbors new problems including additional side effects, loss of target specificity, difficulties to determine the therapeutic window and cell type-dependent variations.

All strategies, including physicochemical techniques, require enhancer molecules that interact more or less directly with membranes and comprise essentially small chemical molecules, secondary metabolites, peptides and proteins. A common feature of all these substances is that they are *per* se not target cell-specific and distribute with other kinetics than the targeted toxins. This is one major drawback of the current approaches.

The present invention will be described with respect to particular embodiments but the invention is not limited thereto but only by the claims. The embodiments of the invention described herein can operate in combination and cooperation, unless specified otherwise.

An aspect of the invention relates to a first proteinaceous molecule comprising a first binding site for binding to a first epitope of a first cell-surface molecule, covalently bound to at least one saponin via at least one linker and/or via an oligomeric or polymeric scaffold to an amino-acid residue of said first proteinaceous molecule, or covalently bound directly to an amino-acid residue of said first proteinaceous molecule, wherein the first binding site comprises or consists of an immunoglobulin, such as an antibody or an IgG, and/or comprises or consists of at least one ligand for binding to a cell-surface molecule such as EGF or a cytokine, and wherein the at least one saponin is selected from the group consisting of: SO1861, SA1641, GE1741, QS-21, QS-21A, QS21-B, Quil-A, SO1832, SO1904, and SO1862. Thus, the invention relates to the provision of a conjugate, the conjugate comprising or consisting of the first proteinaceous molecule comprising a first binding site for binding to a first epitope of a first cell-surface molecule, wherein at least one saponin is covalently bound via at least one linker to the first proteinaceous molecule and/or bound via an oligomeric or polymeric scaffold to an amino-acid residue of said first proteinaceous molecule, or covalently bound directly to an amino-acid residue of said first proteinaceous molecule, wherein the first binding site comprises or consists of an immunoglobulin, such as an antibody or an IgG, and/or comprises or consists of at least one ligand for binding to a cell-surface molecule such as EGF or a cytokine, and wherein the at least one saponin is selected from the group consisting of: SO1861, SA1641, GE1741, QS-21, QS-21A, QS21-B, Quil-A, SO1832, SO1904, and SO1862.

An embodiment is the first proteinaceous molecule of the invention, wherein the first binding site comprises or consists of an immunoglobulin, or at least one binding domain of an immunoglobulin, such as an antibody or an IgG, and/or comprises or consists of at least one ligand for binding to a cell-surface molecule such as EGF or a cytokine.

An embodiment is the first proteinaceous molecule of the invention, wherein the first epitope of the first cell-surface molecule is a tumor-cell specific first epitope of a first tumor-cell surface molecule, more preferably a tumor-cell specific first epitope of a first tumor-cell surface receptor specifically present on a tumor cell.

Provided but not claimed is the first proteinaceous molecule, wherein the at least one saponin is a triterpenoid saponin and/or a bisdesmosidic triterpene saponin belonging to the type of a 12,13-dehydrooleanane with an aldehyde function in position C-23 and optionally comprising a glucuronic acid function in a carbohydrate substituent at the C-3beta-OH group of the saponin, and/or a saponin isolated from *a Gypsophila* species and/or a *Saponaria* species and/or an *Agrostemma* species and/or a *Quillaja* species such as *Quillaja saponaria.*

An embodiment is the first proteinaceous molecule of the invention, wherein the at least one saponin is a single specific saponin or is a mixture of two or more different saponins, such as one or more of the saponins SO1861, GE1741, SA1641, QS-21, QS-21A, QS-21B, Quil-A, SO1832, or any of their stereomers and/or any combinations thereof, preferably the saponin is SO1861 and/or GE1741 and/or SA1641 and/or QS-21, more preferably the saponin is SO1861 and/or QS-21.

An embodiment is the first proteinaceous molecule of the invention, wherein the at least one saponin is QS-21 or any one or more of QS-21A, QS-21B, Quil-A.

An embodiment is the first proteinaceous molecule of the invention, wherein
- the at least one saponin is a bisdesmosidic triterpene saponin belonging to the type of a 12,13-dehydrooleanane with an aldehyde function in position C-23,
- the at least one saponin is covalently coupled to the amino-acid residue of the first proteinaceous molecule via an aldehyde function in the saponin, preferably said aldehyde function in position C-23, preferably via at least one linker, more preferably via at least one cleavable linker, wherein the amino-acid residue preferably is selected from cysteine and lysine.

An embodiment is the first proteinaceous molecule of the invention, wherein
- the at least one saponin is a bisdesmosidic triterpene saponin belonging to the type of a 12,13-dehydrooleanane with an aldehyde function in position C-23 and comprising a glucuronic acid function in a carbohydrate substituent at the C-3beta-OH group of the saponin,
- the at least one saponin is covalently coupled to the amino-acid residue of the first proteinaceous molecule via the glucuronic acid function in the carbohydrate substituent at the C-3beta-OH group of the saponin, preferably via at least one linker, wherein the amino-acid residue preferably is selected from cysteine and lysine.

An embodiment is the first proteinaceous molecule of the invention, wherein the aldehyde function in position C-23 of the at least one saponin is covalently coupled to linker N-ε-maleimidocaproic acid hydrazide, which linker is covalently coupled via a thio-ether bond to a sulfhydryl group in the first proteinaceous molecule, such as a sulfhydryl group of a cysteine.

An embodiment is the first proteinaceous molecule of the invention, wherein the glucuronic acid function in the carbohydrate substituent at the C-3beta-OH group of the at least one saponin is covalently coupled to linker 1-[Bis(dimethylamino)methylene]-1H-1,2,3-triazolo[4,5-b]pyridinium 3-oxid hexafluorophosphate, which linker is covalently coupled via an amide bond to an amine group in the first proteinaceous molecule, such as an amine group of a lysine or an N-terminus of the first proteinaceous molecule.

An embodiment is the first proteinaceous molecule of the invention, wherein the first epitope of the first cell-surface molecule to which the first binding site of the first proteinaceous molecule binds is a tumor-cell specific first epitope of the tumor-cell specific receptor preferably selected from CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, more preferably selected from CD71, EGFR, HER2.

Provided and not claimed is the first proteinaceous molecule of the invention, wherein the tumor cell-specific first epitope, first tumor-cell surface molecule or first tumor-cell specific receptor, are a first epitope or a first molecule or a first receptor that are internalized by the tumor cell after binding of the first proteinaceous molecule of any one of the claims 1-8 to the first epitope or first molecule or first receptor, and wherein preferably the first proteinaceous molecule is subjected to tumor-cell receptor-mediated internalization, e.g. via endocytosis, or tumor-cell surface molecule mediated internalization, e.g. via endocytosis, when bound to the cell-surface molecule comprising the first epitope, the tumor-cell surface molecule or the tumor-cell specific receptor.

An embodiment is the first proteinaceous molecule of the invention, wherein the first binding site of the first proteinaceous molecule comprises or consists of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell receptor binding-fragment or domain thereof, preferably at least one tumor-cell specific receptor binding-fragment thereof and/or at least one tumor-cell specific receptor binding-domain thereof.

An aspect of the invention relates to a therapeutic combination, wherein the therapeutic combination comprises:
(a) a first pharmaceutical composition comprising the first proteinaceous molecule of the invention and optionally a pharmaceutically acceptable excipient; and
   either
(b) a second pharmaceutical composition comprising a second proteinaceous molecule different from the first proteinaceous molecule and optionally further comprising a pharmaceutically acceptable excipient, the second proteinaceous molecule comprising
   - a second binding site for binding to a second epitope of a second cell-surface molecule different from the first cell-surface molecule, and
   - comprising an effector moiety, such as a toxin, an enzyme or an oligonucleotide, wherein the second epitope is different from the first epitope,
   or
(c) a third pharmaceutical composition comprising a third proteinaceous molecule and optionally further comprising a pharmaceutically acceptable excipient, the third proteinaceous molecule comprising
   - a third binding site for binding to the first epitope on the cell-surface molecule of (a) and
   - an effector moiety, such as a toxin, an enzyme or an oligonucleotide;
   wherein the first binding site and the third binding site are the same, and wherein the first cell-surface molecule and the first epitope on the first cell-surface molecule, to which the first proteinaceous molecule can bind, and the first cell-surface molecule and the first epitope on the first cell-surface molecule, to which the third proteinaceous molecule can bind, are the same.An embodiment is the therapeutic combination of the invention, wherein:
   (a) the therapeutic combination comprises the said first pharmaceutical composition and the said second pharmaceutical composition, wherein
      - the first epitope on the first cell-surface molecule is a tumor-cell specific first epitope on a first tumor cell-specific surface molecule, preferably a tumor-cell specific first epitope on a first cell-surface receptor specifically present at a tumor cell; and
      - the second cell-surface molecule is a second tumor cell-specific surface molecule different from the first tumor cell-specific surface molecule, preferably a second cell-surface receptor specifically present at a tumor cell different from the first cell-surface receptor specifically present at said tumor cell, and wherein the second epitope is a tumor-cell specific second epitope;
      or
   (b) the therapeutic combination comprises the said first pharmaceutical composition and the said third pharmaceutical composition,
   wherein the first cell-surface molecule is expressed on a tumor cell surface, and preferably the first cell-surface molecule is a tumor cell-specific surface molecule, and wherein preferably the first epitope is a first tumor-cell specific epitope.

The inventors established that the therapeutic window of an antibody drug conjugate, such as the second and third proteinaceous molecules in the second or third pharmaceutical composition of the invention, respectively, increases when administered to a tumor-bearing mammal (mouse) to whom also the first pharmaceutical composition is administered. The first proteinaceous protein has at least one of SO1861, SA1641, GE1741, QS-21, QS-21A, QS21-B, Quil-A, SO1832, SO1904, and SO1862 bound thereto, preferably covalently, more preferably via a cleavable linker. The saponin augments the therapeutic efficacy of the effector moiety bound to the second and third proteinaceous molecule, likely by enhancing the endosomal escape of the effector moiety into the cytosol where the activity of the effector moiety is desired. This way, already at a lower dose than the conventional dose of the ADC, i.e. the second or third proteinaceous molecule, therapeutic effect is established under influence of the presence of the first proteinaceous molecule comprising the saponin near, at and/or inside the targeted cell. The targeted cell is for example a diseased cell such as a tumor cell or an auto-immune cell or a B-cell disease related B-cell, etc. The effector moiety is for example a toxin as part of an ADC or an oligonucleotide such as a BNA as part of an AOC according to the invention.

An embodiment is the therapeutic combination of the invention, wherein the second binding site of the second proteinaceous molecule and/or the first binding site of the third proteinaceous molecule comprises or consists of an immunoglobulin, at least one binding domain of an immunoglobulin and/or at least one binding fragment of an immunoglobulin, such as an antibody, an IgG, a molecule comprising or consisting of a Vhh domain or Vh domain, a Fab, an scFv, an Fv, a dAb, an F(ab)2, Fcab fragment, and/or comprises or consists of at least one ligand for binding to a cell-surface molecule such as EGF or a cytokine.

An embodiment is the therapeutic combination of the invention comprising the second pharmaceutical composition, wherein the second binding site of the second proteinaceous molecule for binding to the second epitope is a second binding site for a tumor-cell specific second epitope on a second cell-surface receptor specifically present at the tumor cell, wherein the second binding site is different from the first binding site.

By targeting (two) different cell-surface molecules with the first and second proteinaceous molecule, the delivery of the saponin and the effector molecule at and inside the cytosol of the very same targeted cell, exposing both different cell-surface molecule on the cell surface, is improved and more specific, compared to exposure of such cells to only the second proteinaceous molecule such as an ADC or an AOC, without the presence of the cell-targeted saponin (first proteinaceous molecule). An aberrant cell selected for separate targeting by the binding site of the first proteinaceous molecule and by the binding site of the second proteinaceous molecule, wherein the binding sites are different and wherein the epitope to which the first and second proteinacous molecules bind are different and are located in/on a different kind and type of cell-surface molecule such as two different receptors, ideally bears the first epitope and the second epitope on the first cell-surface molecule and the second cell-surface molecule respectively, to a high extent (i.e. relativelty higher expression of the two distinct and different cell-surface molecules on the targeted cell such as for example a tumor cell or an auto-immune cell, than the expression on a non-targeted cell such as for example a healthy cell) and/or expose the first and second cell-surface molecules specifically, when (neighboring) healthy cells in a patient are considered. Preferably, both cell-surface molecules targeted by the first and second binding sites are relatively highly and/or specifically expressed on the targeted (diseased, tumor) cell compared to healthy cells. An embodiment is the pharmaceutical combination, wherein at least one of the first and second binding site and thus at least one of the first and second cell-surface molecule such as a first and second tumor-cell receptor, is expressed specifically or to a relatively higher extent when compared to expression of the first cell-surface molecule and/or the second cell-surface molecule on the surface of a healthy (neighbouring) cell. Thus, the first epitope or the second epitope, preferably the first epitope and the second epitope, on the targeted cell-surface molecule is/are ideally unique to the targeted diseased cells, and is/are at least specifically present and exposed at the surface of the targeted cells. Binding of the first and second proteinaceous molecules to their respective first and second epitope on a targeted cell is followed by endocytosis of the complexes of the first proteinaceous molecule and the first target cell-surface molecule and the second proteinaceous molecule and the second target cell-surface molecule. Since the first and second proteinaceous molecules have to enter the same target cell through binding interaction with two different cell-surface molecules both expressed to a sufficient extent or uniquely on the targeted cell when compared to healthy cells that should not be targeted, accumulation of a therapeutically active amount of first and second proteinaceous molecules inside the target cells is only possible and occurring if expression levels of the two distinct targeted cell-surface molecules is both above a certain minimal expression threshold. At the same time, the fact that the effector moiety bound to the second proteinaceous molecule is only capable of exerting its intracellular (e.g. cytotoxic or gene silencing) activity in the presence of the first proteinaceous molecule bearing the covalently bound saponin, when both the first and second proteinaceous molecules were capable to enter the target cell in sufficient amounts by binding to sufficiently exposed and expressed first and second cell-surface molecules, also provides a safeguard against negative and undesired side effects of the effector moiety towards e.g. healthy cells and healthy tissue not meant to be targeted and affected by the effector moiety, when expression of at least on of the first and second cell-surface molecules is sufficiently low at the healthy cells and preferably when expression of both the first and second targeted cell-surface molecules is sufficiently low at the healthy cells. That is to say, sufficiently low expression or even absence of exposed first and second cell-surface molecules with regard to the first and second cell-surface molecules, and at least either the first cell-surface molecule or the second cell-surface molecule, bound by the first and second binding site of the first and second proteinaceous molecules respectively, does ideally not allow entrance into (non-targeted) healthy cells of both the first and second proteinaceous molecules to amounts that would in concert result in endosomal escape of the effector moiety under influence of the saponin bound to the first proteinaceous molecule. Since the ADC or the AOC can be used at lower dose compared to when the first proteinaceous molecule was not added to the therapeutic regimen, ADC or AOC entrance in healthy cells to low extent already bears a lower risk for occurrence of unwanted side effects when for example the targeting and killing of target diseased cells such as tumor cells and auto-immune cells is considered.

An embodiment is the first proteinaceous molecule of the invention or the therapeutic combination of the invention comprising the second pharmaceutical composition, wherein said first and second proteinaceous molecules comprise the first and second binding site respectively for binding to a first and a second tumor-cell specific epitope on a first and a second tumor-cell specific receptor respectively, the receptors being different and being present at the same tumor cell, wherein the first and second binding site are different and the first and second tumor cell specific epitope are different.

An embodiment is the first proteinaceous molecule of the invention or the therapeutic combination of the invention comprising the third pharmaceutical composition, wherein said first and third proteinaceous molecules comprise the same first binding site for binding to a first tumor-cell specific epitope on a first tumor-cell specific receptor.
An embodiment is the first proteinaceous molecule of the invention or the therapeutic combination of the invention comprising the second pharmaceutical composition, wherein the first receptor and/or the second receptor are selected from CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably selected from CD71, EGFR and HER2; with the provisos that
- when the first and second proteinaceous molecules are present in the therapeutic combination, the first binding site of the first proteinaceous molecule is different from the second binding site of the second proteinaceous molecule,
- when the first and third proteinaceous molecules are present in the therapeutic combination, the first and third binding sites are the same.

Provided and not claimed is the first proteinaceous molecule of the invention or the therapeutic combination of the invention comprising the second pharmaceutical composition, wherein the first and second tumor-cell specific receptors are internalized by the tumor cell after binding to the first proteinaceous molecule of the invention and/or the second proteinaceous molecule of the invention when the therapeutic combination comprises the second pharmaceutical composition, and wherein preferably binding of the first proteinaceous molecule and/or the second proteinaceous molecule to the first and second tumor-cell specific receptors respectively, results in tumor-cell receptor-mediated internalization, e.g. via endocytosis, of a complex of the first proteinaceous molecule and the first tumor-cell specific receptor and of a complex of the second proteinaceous molecule and the second tumor-cell specific receptor.

Provided and not claimed is the therapeutic combination comprising the third pharmaceutical of the invention or the first pharmaceutical composition according to the invention, wherein the first tumor-cell receptor, preferably the first tumor-cell specific receptor, is internalized by the tumor cell after binding to the first proteinaceous molecule of the invention and/or after binding to the third proteinaceous molecule of the invention, and wherein preferably binding of the first proteinaceous molecule and/or the third proteinaceous molecule to the first tumor-cell receptor, such as the first tumor-cell specific receptor, is followed by tumor-cell receptor-mediated internalization, e.g. via endocytosis, of a complex of the first proteinaceous molecule and the first tumor-cell receptor and of a complex of the third proteinaceous molecule and the first tumor-cell receptor.

Synchronization is the missing link between a successful delivery strategy for mice and its application in humans. Indeed, the inventors established in a series of in vivo mouse tumor models that separately administering to the mice a dose of free saponin and a dose of ADC (second or third proteinaceous molecule according to the invention) did not result in any desired anti-tumor activity such as delayed tumor growth, tumor regression, diminished and slower tumor growth, compared to control animals not treated with the ADC and free saponin. The free saponin was administered using various routes of administration and using various time points of administering the free saponin compared to the moment of administering the ADC (administering free saponin before, during and after administering the ADC). The ADC tested in in vivo tumor models was cetuximab-dianthin (with free SO1861), or trastuzumab-saporin (with free SO1861). Varying the dose of free saponin did not provide for an efficacious anti-tumor activity. The ADCs referred to were administered at a dose that in itself did not inflict any beneficial anti-tumor effect on the tumor-bearing animals. Surprisingly, the inventors now established that beneficial anti-tumor activity in various in vitro mammalian cell-based bioassays and/or in various in vivo animal tumor models can be achieved by treating the animals with conjugates according to the invention, optionally comprising a scaffold according to the invention, i.e. combinations of first and second or first and third proteinaceous molecules of the invention. The scaffold for example being a tri-functional linker with a covalently bound saponin (e.g. SO1861, QS-21) via a cleavable or non-cleavable linkage, and/or with a covalently bound effector moiety (e.g. dianthin, silencing BNA (HSP27) via a non-cleavable bond or a cleavable bond, and/or with a covalently bound monoclonal antibody such as cetuximab, trastuzumab, OKT-9, or the scaffold being a dendron, such as a dendron to which for example four moieties can bind such as four saponin molecules, or a dendron for binding for example two saponins and two effector molecules, the dendron comprising a chemical group for (covalent) coupling to a ligand or an antibody or fragment or domain thereof. Reference is made to the Examples section, exemplifying various of these scaffolds according to the invention, showing in vivo and/or in vitro anti-tumor cell activity when cell toxicity exerted by e.g. a proteinaceous toxin is considered or when gene silencing in the tumor cell is considered.

Without wishing to be bound by any theory, in view of the failures observed when treatment of tumor-bearing animals with an ADC together with free saponin is considered, it is preferred to synchronize the presence of both, the at least one saponin, and the effector moiety, preferably a toxin or an oligonucleotide, in compartments or vesicles of the endocytic pathway of the target cell, e.g. a tumor cell or an auto-immune cell. With ADC and free saponin, synchronizing the presence of the molecules in the late endosomes, in order to obtain the synergistic effects *in vivo* was not beneficially obtainable according to attempts of the inventors. In one aspect, the invention preferably solves at least the following problem with respect to combining the effector moiety comprised by the second proteinaceous molecule and the saponins comprised by the first proteinaceous molecule: without wishing to be bound by any theory the only reasonable chemical group within, e.g., the saponins that can be used for (covalent), in particular single and cleavable, retainable coupling is required for the endosomal escape activity. Known restrictions are most likely the reason why saponins have not been used in combination with pharmaceutically active substances in clinical investigations other than the application of saponins in vaccination regimes wherein the use of an immune-potentiating adjuvant substance was implied, although the striking endosomal escape enhancer effect of, e.g., saponins listed in Table A1 and Scheme I is known for more than 10 years. For example providing a first proteinaceous molecule of the invention with a covalently conjugated scaffold solves these difficulties, at least in part. Surprisingly, the saponins previously applied for their immune-potentiating activity in the vaccination context involving saponins as adjuvant component, are now also suitably for (covalent) coupling to the first proteinaceous molecule of the invention, for anti-tumor activity in vitro and in vivo.

An effector moiety useful in the present invention preferably relies on late endosomal escape for exerting its effect. Some effectors, such as, e.g., a pseudomonas exotoxin, are rerouted to other organelles prior to the "late endosomal stage" and, thus, would normally not benefit from coupling to the second proteinaceous molecule according to the present invention. However, such toxin may be adapted for use with the present invention, e.g., by deleting the signal peptide responsible rerouting. In particular toxins that are highly toxic and would require only one molecule to escape the endosomes to kill a cell maybe modified to be less potent. It is preferred to use a toxin that kills a cell if at least 2, more preferably at least 5, more preferably at least 10, more preferably at least 20, more preferably at least 50, most preferably at least 100 toxin molecules escape the endosome. It is further preferred that a second proteinaceous molecule of the invention comprises a covalently conjugated functionalized scaffold, i.e. a scaffold comprising covalently bound effector moietie(s) for targeting the scaffold comprising the bound effector moietie(s) at a target cell such as a tumor cell or an auto-immune cellFurther, in order to reduce off-target toxicity, cell membrane non-permeable small molecule toxins are preferred effector molecules over cell membrane permeable toxins.

The term "ligand" as used in this invention has its ordinary meaning and preferably means a molecule or structure that is able to bind another molecule or structure on the cell surface of a target cell, wherein said molecule or structure on the cell surface can be endocytosed and is preferably absent or less prominent on off-target cells. Preferably, said molecule or structure on the cell surface is constitutively endocytosed. More preferably a ligand in this invention induces endocytosis of said molecule or structure on the cell surface of target cells after binding to said molecule or structure. This is for instance the case for Epidermal Growth Factor Receptor (EGFR), present on the surface of a variety of cancer cells. Examples of molecules or structures on the cell surface of target cells that are constitutively endocytosed, are for instance Claudin-1 or major histocompatibility complex class II glycoproteins. A ligand can, e.g., be an antibody, a growth factor or a cytokine. Combining in a carrier molecule a toxin with a ligand is one possibility to create a targeted toxin. A toxin that is only toxic in a target cell because it interferes with processes that occur in target cells only can also be seen as a targeted toxin (as in off-target cells it cannot exert its toxic action, e.g. apoptin). Preferably, a targeted toxin is a toxin that is combined with a ligand or e.g. a monoclonal antibody in order to be active in target cells and not in off-target cells (as it is only bound to and endocytosed by target cells). In a functionalized scaffold comprising a carrier molecule comprising a ligand and an effector moiety (i.e. a second or third proteinaceous molecule), the ligand or the monoclonal antibody guides the effector moiety and scaffold to the target cells. After internalization, the at least one glycoside, preferably a saponin comprised by the conjugate of the first proteinaceous molecule and the saponin, mediates the endosomal escape of the effector moiety. The saponin is typically a saponin listed in Table A1 and Scheme I, and preferably the saponin is SO1861 and/or QS-21, and/or SA1641 and/or GE1741.

Preferably, the effector moiety bound to the second or third proteinaceous molecule, which effect is enhanced by the saponins bound to the first proteinaceous molecule, detaches from the second or third proteinaceous molecule, e.g. an antibody, when endocytosed. This can be achieved by a cleavable bond that breaks, e.g. under acidic, reductive, enzymatic or light-induced conditions.

An embodiment is the first proteinaceous molecule of the invention, and/or therapeutic combination of the invention comprising the second pharmaceutical composition, wherein the first binding site and/or the second binding site is/are or comprise(s) a monoclonal antibody or at least one cell-surface molecule binding fragment and/or -domain thereof, and preferably comprise or consist of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, and an antibody of Table A4, preferably cetuximab or trastuzumab or OKT-9, or at least one cell-surface molecule binding fragment or -domain thereof, with the proviso that the first binding site of the first proteinaceous molecule is different from the second binding site of the second proteinaceous molecule.

An embodiment is the therapeutic combination comprising the third pharmaceutical composition of the invention or the first pharmaceutical composition according to the invention when comprised by the therapeutic combination comprising the third pharmaceutical composition, wherein the first binding site of the first proteinaceous molecule and the third proteinaceous molecule comprises a monoclonal antibody or at least one of a cell-surface molecule binding domain and/or -fragment thereof, and preferably comprise or consist of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one cell-surface molecule binding fragment and/or -domain thereof, with the proviso that the first binding site of the first proteinaceous molecule is the same as the first binding site of the third proteinaceous molecule.

An embodiment is the therapeutic combination comprising the second or the third pharmaceutical composition of the invention, wherein the second binding site of the second proteinaceous molecule and/or the first binding site of the third proteinaceous molecule is or comprises a monoclonal antibody or at least one cell-surface molecule binding fragment or -domain thereof, and preferably comprises or consists of any one of Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3.

The inventors established that such immunoglobulins, domains thereof, ligands, etc., are particularly suitable for application as the first binding site of the first proteinaceous molecule (and the same binding site of the third proteinaceous molecule) comprising the first binding site. Similarly, the inventors established that such immunoglobulins, domains thereof, ligands, etc., are particularly suitable for application as the second binding site of the second proteinaceous molecule comprising the second binding site. For example, antibodies and binding domains of antibodies are suitable for targeting an epitope on the exposed surface of a selected cell-surface molecule, resulting in targeting the first and third (and separately the second) proteinaceous molecule to target cells expressing the cell-surface molecule targeted by the first and third proteinaceous molecule and/or target also cells expressing the second cell-surface molecule targeted by the second proteinaceous molecule, these cells also expressing the first and third cell-surface molecule (which is the same cell-surface molecule), and having said cell-surface molecules on their cell surface. Similarly, ligands such as EGF, targeting the EGFR on target cells, are suitable for application as the binding site in the first and third proteinaceous molecules, or as the second binding site in the second proteinaceous molecule with the proviso that the second binding site is different from both the first and third binding site which first and third binding site are the same. Preferred are binding sites for the first and third epitope or for the second epitope, which are specific for the binding of the first and third proteinaceous molecules to the first cell-surface molecule and/or for the binding of the second proteinaceous molecule to the second cell-surface molecule, the first and second cell-surface molecules exposed on the very same target cell. Binding sites based on antibodies or domains or binding fragments thereof for example provide for such desired specificity for a selected first, second, third epitope on a selected first or second cell-surface molecule of a selected cell for targeting such as a diseased cell, a tumor cell, an auto-immune cell, etc. Therefore, first, second and third binding sites based on antibodies or binding molecules (fragments, domains) are preferred for the first and second and third proteinaceous molecules.

By targeting the same cell-surface molecule with the first and third proteinaceous molecule, the delivery of the saponin and the effector moiety at and inside the cytosol of the very same targeted cell is improved and more specific. An aberrant cell selected for targeting by the binding site of the first and third proteinaceous molecule ideally bears the cell-surface molecule to a high extent and/or specifically, when (neighboring) healthy cells in a patient are considered. Thus, the epitope on the targeted cell-surface molecule is ideally unique to the targeted diseases cells, and is at least specifically present and exposed at the surface of the targeted cells. Binding of the first and third proteinaceous molecules is followed by endocytosis of the complexes of the first proteinaceous molecule and the target cell-surface molecule and the third proteinaceous molecule and the target cell-surface molecule. Since the first and third proteinaceous molecules have to enter the same target cell through binding interaction with the very same cell-surface molecules, accumulation of a therapeutically active amount of first and third proteinaceous molecules inside the target cells is only possible and occurring if expression levels of the targeted cell-surface molecule is above a certain minimal expression threshold. At the same time, the fact that the effector moiety bound to the third proteinaceous molecule is only capable of exerting its intracellular (e.g. cytotoxic or gene silencing) activity in the presence of the first proteinaceous molecule bearing the covalently bound saponin, when both the first and third proteinaceous molecules were capable to enter the target cell in sufficient amounts by binding to sufficiently exposed and expressed cell-surface molecule, also provides a safeguard against negative and undesired side effects of the effector moiety towards e.g. healthy cells and healthy tissue not meant to be targeted and affected by the effector moiety, when expression of the targeted cell-surface molecule is sufficiently low at the healthy cells. That is to say, low expression of the cell-surface molecule bound by the binding site of the first and third proteinaceous molecules, does not allow entrance of both the first and third proteinaceous molecules to amounts that would in concert result in endosomal escape of the effector moiety under influence of the saponin bound to the first proteinaceous molecule. Since the ADC or AOC can be used at lower dose compared to when the first proteinaceous molecule was not added to the therapeutic regimen, ADC or AOC entrance in healthy cells to low extent already bears a lower risk for occurrence of unwanted side effects when for example the targeting and killing of target diseased cells such as tumor cells and auto-immune cells is considered.

Throughout the description and claims (the whole application), the terms 'first' and 'third' have the same meaning when the first and third epitope, the first and third binding site, the first and third cell-surface molecule are considered. That is to say, for the first and third proteinaceous molecules, the targeted epitope is the same, the binding site is the same, the targeted cell-surface molecule such as a tumor-cell (specific) receptor is the same.

Tables A2, A3 and A4 list preferred examples of the first cell-surface molecule comprising the first epitope for the first binding site of the first and third proteinaceous molecule. In addition, Tables A2, A3 and A4 also list preferred examples of the second cell-surface molecule comprising the second epitope for the second binding site of the second proteinaceous molecule. When the first and/or second cell-surface molecule is specifically expressed on the target cell, preferably both the first and second cell-surface molecules, and when the first and second epitopes on the first and second cell-surface molecules respectively, to which the first binding site and/or the second binding site can bind respectively, is specifically present in the first and/or second cell-surface molecule, specific targeting of the first, third and/or second proteinaceous molecule to the same desired target cell such as a tumor cell exposing the first and second tumor-cell surface molecules, is facilitated, whereas other cells such as healthy cells, which do not express the first and/or second cell-surface molecule or do express the first and/or second cell-surface molecule to a lower extent, preferably which which do not express the first and second cell-surface molecule or do express the first and second cell-surface molecule to a lower extent compared to expression of the cell-surface molecule(s) on the targeted (aberrant) cell, are not targeted by the first, third and second proteinaceous molecule or are targeted to a lower extent.

An embodiment is the therapeutic combination comprising the second or the third pharmaceutical composition of the invention, wherein the effector moiety that is comprised by the second proteinaceous molecule or by the third proteinaceous molecule comprises or consists of any one or more of an oligonucleotide, a nucleic acid, a xeno nucleic acid, preferably selected from any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression.

An embodiment is the therapeutic combination comprising the second or the third pharmaceutical composition of the invention, wherein the effector moiety that is comprised by the second proteinaceous molecule or by the third proteinaceous molecule comprises or consists of at least one proteinaceous molecule, preferably selected from any one or more of a peptide, a protein, an enzyme such as urease and Cre-recombinase, a ribosome-inactivating protein, a proteinaceous toxin, more preferably selected from any one or more of a protein toxin selected from Table A5 and/or a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin.

An embodiment is the therapeutic combination comprising the second or the third pharmaceutical composition of the invention, wherein the effector moiety comprised by the second proteinaceous molecule or by the third proteinaceous molecule comprises or consists of at least one payload, preferably selected from any one or more of a toxin targeting ribosomes, a toxin targeting elongation factors, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-γ-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

A pharmaceutically active substance in this invention is an effector moiety that is used to achieve a beneficial outcome in an organism, preferably a vertebrate, more preferably a human being such as a cancer patient or an auto-immune patient. Benefit includes diagnosis, prognosis, treatment, cure and/or prevention of diseases and/or symptoms. The pharmaceutically active substance may also lead to undesired harmful side effects. In this case, pros and cons must be weighed to decide whether the pharmaceutically active substance is suitable in the particular case. If the effect of the pharmaceutically active substance inside a cell is predominantly beneficial for the whole organism, the cell is called a target cell. If the effect inside a cell is predominantly harmful for the whole organism, the cell is called an off-target cell. In artificial systems such as cell cultures and bioreactors, target cells and off-target cells depend on the purpose and are defined by the user.

An effector moiety that is a polypeptide may be, e.g., a polypeptide that recover a lost function, such as for instance enzyme replacement, gene regulating functions, or a toxin.

An embodiment is the first proteinaceous molecule of the invention, wherein the first proteinaceous molecule comprises more than one saponin, preferably 2, 3, 4, 5, 6, 8, 10, 16, 32, 64 or 1-100 saponins, or any number of saponins therein between, such as 7, 9, 12 saponins, covalently bound directly to an amino-acid residue of the first proteinaceous molecule, preferably to a cysteine and/or to a lysine, and/or covalently bound via at least one linker and/or via at least one cleavable linker and/or via at least one polymeric or oligomeric scaffold, preferably 1-8 of such scaffolds or 2-4 of such scaffolds, wherein the at least one scaffold is optionally based on a dendron, wherein 1-32 saponins such as 2, 3, 4, 5, 6, 8, 10, 16, 32 saponins, or any number of saponins therein between, such as 7, 9, 12 saponins, are covalently bound to the at least one scaffold.

Table A1 and Scheme I and the above embodiments summarize a series of saponins that have been identified for their endosomal escape enhancing activity when contacted to mammalian cells, in particular human tumor cells, in free form together with a second molecule (e.g. an effector moiety or effector molecule, such as a toxin, an oligonucleotide). Indeed, in cell-based bioassays using human tumor cells it was established for the saponins tabulated in Table A1 and those in Scheme I and in the various embodiments of the invention described herein, that under influence of these saponins, when bound to the first proteinaceous molecule, a second molecule (effector moiety) such as a nucleic acid and/or a toxin such as a protein toxin (e.g. one or more of the protein toxins listed in Table A5), bound to the second or third proteinaceous molecule, is delivered into the cytosol with increased efficiency and/or efficacy, presumably through intracellular release from the (late) endosomes and lysosomes. That is to say, endosomal and/or lysosomal escape of such second molecules (effector moieties bound to a second or to a third proteinaceous molecule of the invention), e.g. nucleic acids and/or toxins, is less efficient in the absence of the saponin.

Surprisingly, the inventors now demonstrate that a water-soluble saponin fraction from Quillaja saponaria, comprising QS-21 and its family members QS-21A, QS-21 A-api, QS-21 A-xyl, QS-21B, QS-21 B-api, QS-21 B-xyl, QS-7-xyl, QS-7-api, QS-17-api, QS-17-xyl, QS1861, QS1862, QS-18 and Quil-A, also exhibits the ability to potentiate a biological effect in vitro of e.g. a nucleic acid bound to a monoclonal antibody or a protein toxin bound to a monoclonal antibody (examples of a second and/or third proteinaceous molecule of the invention comprising covalently bound oligonucleotide or payload such as a (protein) toxin), when administered to tumor cells of a mammalian species (human) in the form of a covalent conjugate comprising a monoclonal antibody (first proteinaceous molecule of the invention), together with the second and/or third proteinaceous molecule comprising the effector moiety (the aforementioned second and/or third proteinaceous molecule) and the at least one glycoside such as the QS-21 and its family member saponins encompassed by such QS-21 preparation (e.g. water soluble fraction of Quillaja saponaria), comprised by the first proteinaceous molecule as a covalent conjugate, wherein the effector molecule and the glycoside, e.g. saponin fraction of Quillaja saponaria, QS-21, SO1861, SA1641, GE1741, are covalently bound to for example the proteinaceous molecules directly or via a linker or via a polymeric or oligomeric scaffold, either directly or via at least one linker. Without wishing to be bound by any theory, the observed stimulation or potentiation of for example antisense BNA mediated reduction of tumor-cell HSP27 expression (HSP27 gene silencing) in the presence of saponins derived from Quillaja saponaria in vitro may (also) relate to activation of the inflammasome in the tumor cell by the saponins, for example resulting in tumor cell pyroptosis. The inventors established that second and third proteinaceous molecules conjugated to for example antisense BNA or dianthin or saporin, exerted any anti-tumor cell activity in vitro at all or improved anti-tumor cell activity when contacted with cells in bio-based cell assays, when in the presence of the first proteinaceous molecule of the invention, comprising the saponin, and targeted to the same (tumor) cells as the cell surface molecule targeted by the second and/or third proteinaceous molecule, whereas in the absence of the first proteinaceous molecule and thus in the absence of saponin, no such activity towards the tumor cell was observed.

QS-21, and also the water-soluble saponins fraction comprising QS-21 from Quillaja saponaria is already for a long time known and previously intensively applied for its immune-potentiating abilities, e.g. as an adjuvant in e.g. sub-unit vaccines. For example, QS-21 is applied in two phase III clinical trials with human patients, who were vaccinated with a sub-unit vaccine mixed with an adjuvant comprising QS-21 (Glaxo-Smith-Kline, MAGRIT trial, DERMA study), wherein the sub-unit was MAGE-A3 protein, which is specifically expressed and presented by tumor cells. The anti-tumor vaccinations, potentiated with QS-21, aimed for extension of disease-free survival of the cancer patients (melanoma; non-small cell lung cancer). In addition, QS-21 has been tested as an adjuvant in clinical trials for developing anti-cancer vaccine treatment, for vaccines for HIV-1 infection, in development of a vaccine against hepatitis B, and for anti-malaria vaccine development using QS-21 comprising adjuvants AS01 and AS02 of Glaxo-Smith-Kline. Previous studies revealed an immune response elicited against MAGE-A3 peptides presented at the cancer cell surface, under influence of the QS-21 saponin comprising adjuvant (AS15; GSK). To the surprise of the inventors, the saponin fraction of Quillaja saponaria, and thus likely QS-21 (as part of the water soluble saponin fraction of Quillaja saponaria) potentiates the anti-tumor cell activity of e.g. a payload such as a protein toxin (dianthin), bound to the second proteinaceous molecule (e.g. the ligand EGF).

The inventors show that a tumor-cell targeting monoclonal antibody provided with covalently coupled antisense BNA such as BNA(HSP27), and contacted with the tumor cells together with a first proteinaceous molecule of the invention with covalently coupled saponin (e.g. SO1861, QS-21), both the BNA and the saponin coupled to the respective antibody (e.g. cetuximab) of the first and third proteinaceous molecule via a cleavable bond is capable of silencing HSP27 in vivo in tumors, compared to control and compared to AOC (third proteinaceous molecule) only, without presence of first proteinaceous molecule with coupled saponin. Co-administering an ADC or an antibody-oligonucleotide conjugate (AOC), such as an antibody-BNA conjugate, with a first proteinaceous molecule with a saponin thus endows the ADC or AOC with anti-tumor cell activity not seen with only the ADC or only the AOC at the same dose. Noteworthy, the AOC (the second or third proteinaceous molecule) and the monoclonal antibody with covalently coupled saponin (first proteinaceous molecule) increase HSP27 expression in tumor cells, when administered to tumor-bearing mice separately in separate groups of mice, compared to a control group (vehicle administered, only). Only co-administration of the AOC comprising the effector moiety of the invention (second or third proteinaceous molecule) and the first proteinaceous molecule with covalently coupled saponin, displays reduced HSP27 expression when compared to controls. The antisense BNA (HSP27) was BNA with oligo nucleic acid sequence 5'-GGCacagccagtgGCG-3' according to Zhang et al. (2011) [Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333], Noteworthy, to the best of the knowledge of the inventors, BNA is designed for application as a free nucleic acid. The inventors are now the first to demonstrate that the antisense BNA can be covalently coupled through a (non-)cleavable linker with a ligand or an antibody, in a way that gene-silencing activity is retained in vitro and more importantly in vivo in the tumor cells of a tumor-bearing animal. This approach of providing BNA based AOCs opens new ways to administer targeted BNA to human (cancer) patients in need thereof.

The inventors disclose here that covalently coupling saponins such as saponins in the water-soluble fraction of Quillaja saponaria, QS-21, SA1641, SO1861, Table A1, Scheme I, to a first proteinaceous molecule, such as via a tri-functional linker, e.g. the tri-functional linker of Scheme II and Structure B, or via an oligomeric or polymeric structure of a scaffold comprising covalently bound saponins, results in improved cell toxicity exerted by the effector moiety such as a toxin, comprised by the second and/or third proteinaceous molecule, under influence of the covalently coupled saponin in the first proteinaceous molecule.

An embodiment is the first proteinaceous molecule of the invention comprising a saponin comprising one or several or all of the indicated structural features of the saponin of Structure A in Scheme I, the saponin of structure A referred to as a saponin with an 'ideal' structure when endosomal escape enhancing activity towards an effector moiety present in the endosome of a cell contacted with first proteinaceous molecule, and/or a saponin selected from any one or more of the further saponins in Scheme I: 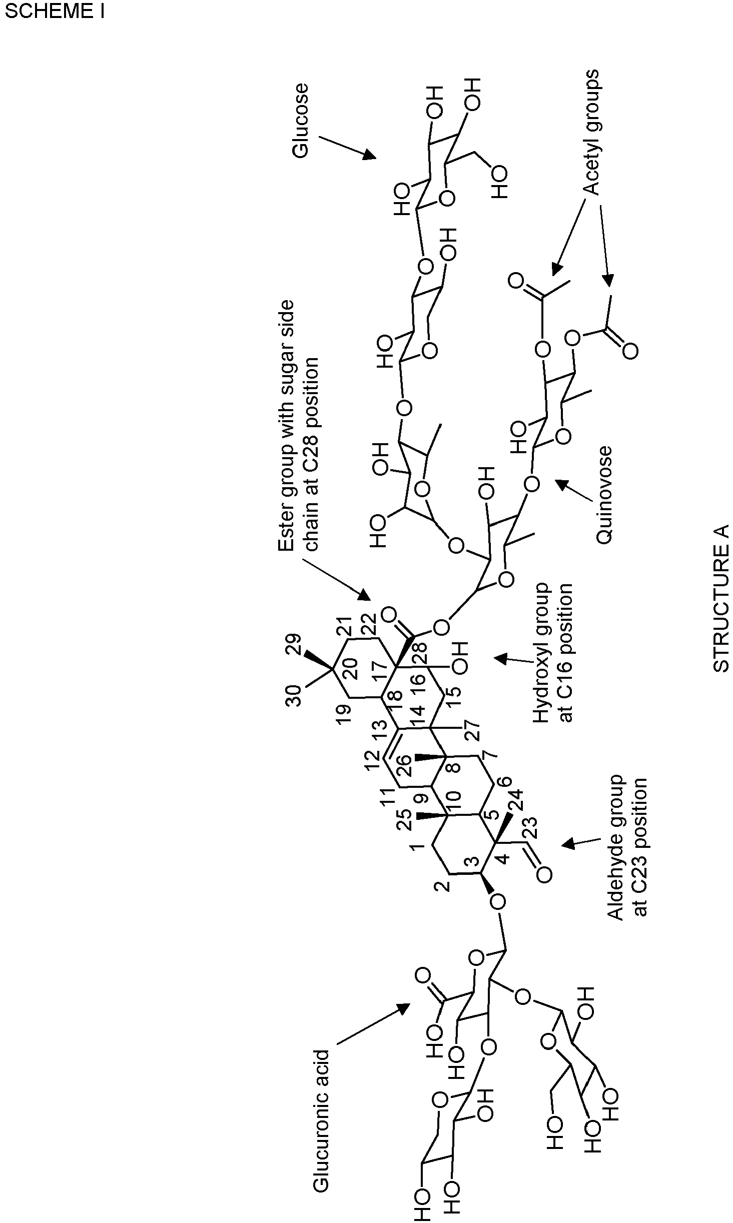 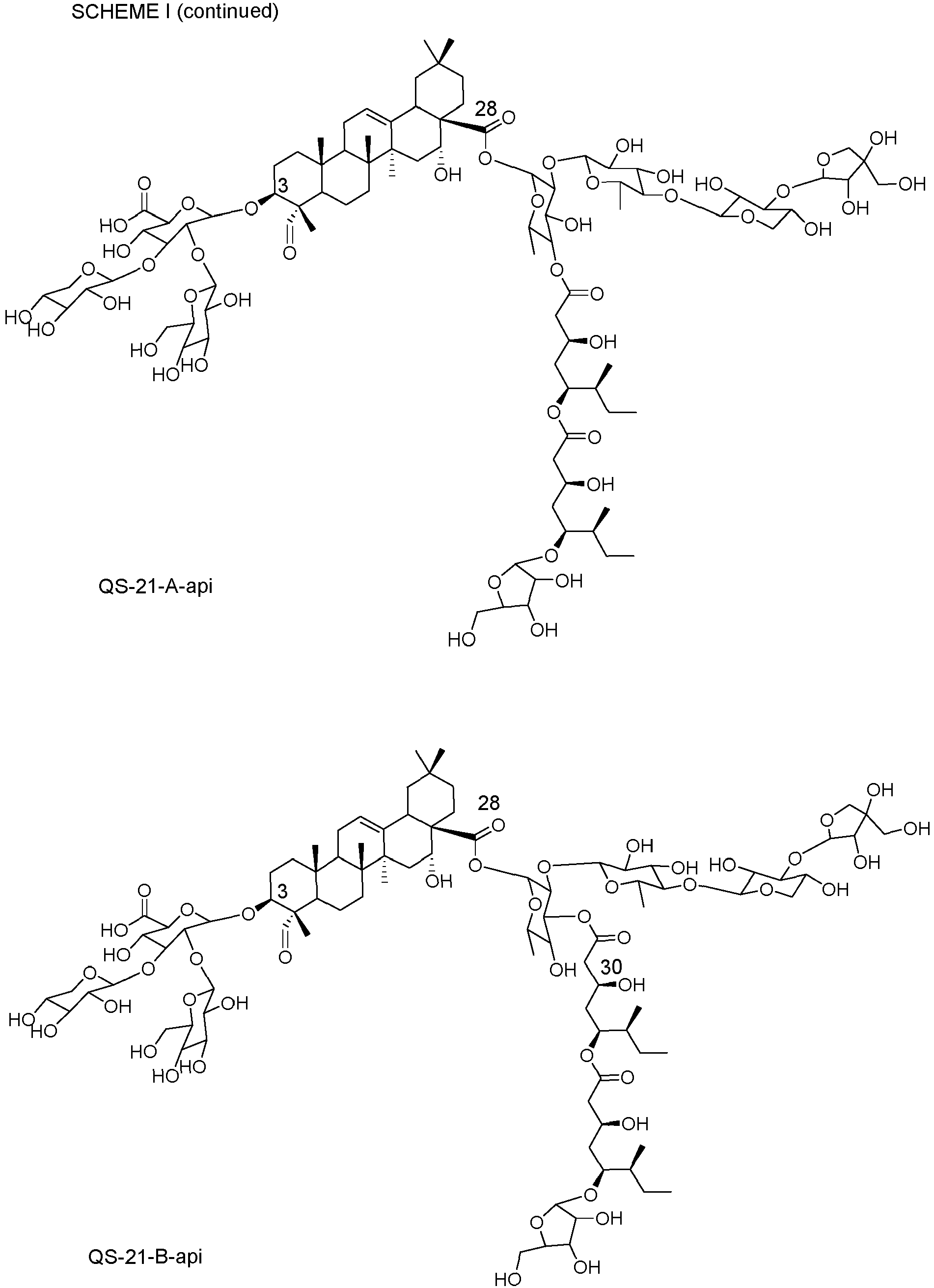 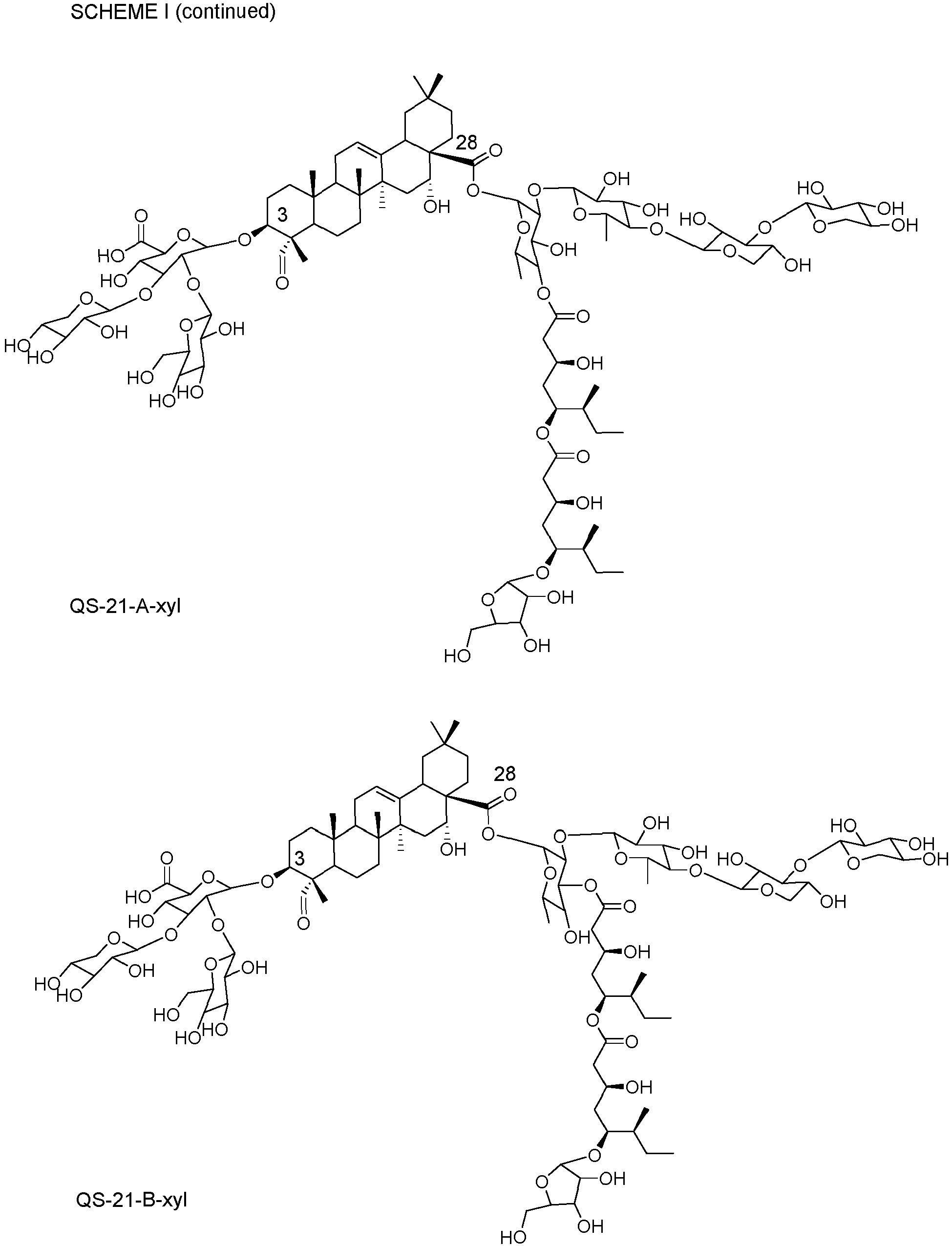 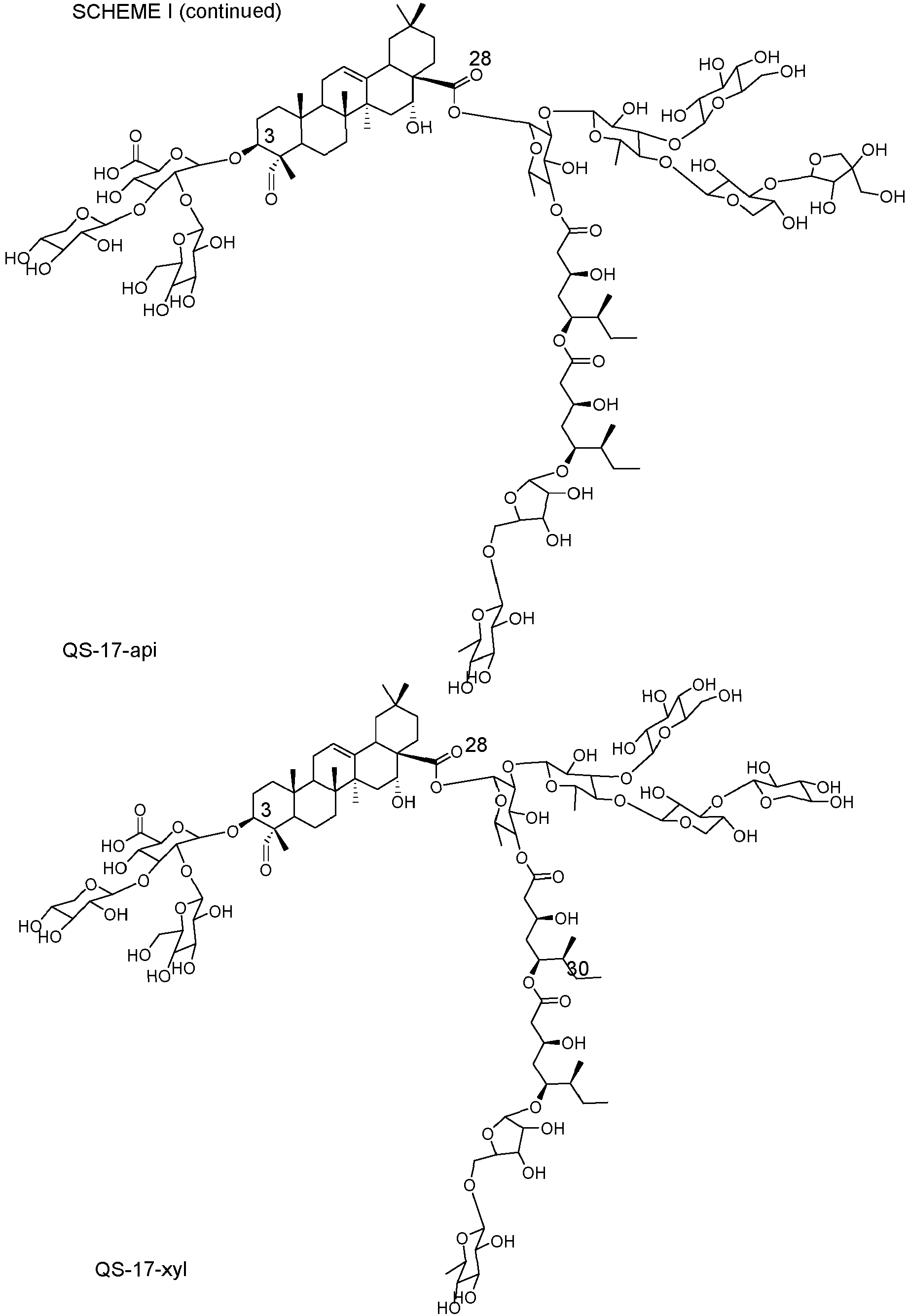 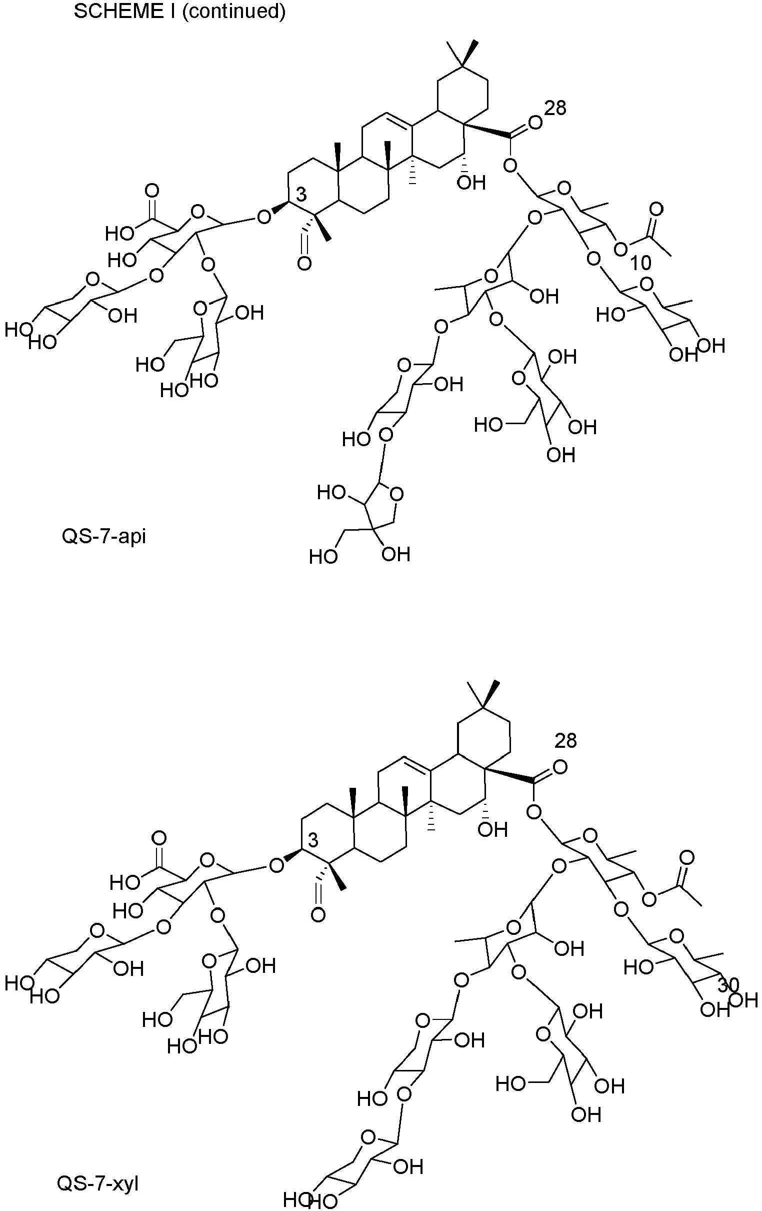 According to the invention, a glycoside, such as a saponin selected from the group consisting of: SO1861, SA1641, GE1741, QS-21, QS-21A, QS21-B, Quil-A, SO1832, SO1904, and SO1862 according to the invention, bound to the first proteinaceous molecule of the invention, which has the 'ideal' structure for the purpose of enhancing endosomal escape of an effector molecule bound to the second or third proteinaceous molecule of the invention is a bisdesmosidic saponin according to Structure A of Scheme I, having a molecular mass of at least 1.500 Dalton and comprising an oleanan-type triterpene containing an aldehyde group at the C-23 position and optionally a hydroxyl group at the C-16 position, with a first branched carbohydrate side chain at the C-3 position which first branched carbohydrate side chain optionally contains glucuronic acid, wherein the saponin contains an ester group with a second branched carbohydrate side chain at the C-28 position which second branched carbohydrate chain preferably comprises at least four carbohydrate units, optionally containing at least one acetyl residue such as two acetyl residues and/or optionally comprising deoxy carbohydrates and/or optionally comprising quinovose and/or optionally comprising glucose and/or optionally comprising 4-methoxycinnamic acid and/or optionally comprising 5-O-[5-O-Ara/Api-3,5-dihydroxy-6-methyl-octanoyl]-3,5-dihydroxy-6-methyl-octanoic acid and/or optionally comprising 5-O-[5-O-Rha-(142)-Ara/Api-3,5-dihydroxy-6-methyl-octanoyl]-3,5-dihydroxy-6-methyl-octanoic acid bound to a carbohydrate via an ester bond.

SO1861 is different from the *"ideal structure"* displayed in Scheme I, Structure A, only in having only one acetyl residue at the quinovose and having an additional xylose. The "ideal structure" of a saponin for enhancing endosomal escape of an effector molecule or effector moiety, is a saponin which preferably has the Structure A of Scheme I, and saponins which display the endosomal escape enhancing activity have one or more of the structural features displayed in Structure A of Scheme I. Without wishing to be bound by any theory, the inventors belief that the Structure A of Scheme I represents an "ideal saponin" (and not a minimum requirement saponin) for endosomal escape enhancing activity, which means that not all of the structures (chemical groups) can or must be present in each saponin with at least sufficient endosomal escape enhancing activity to promote accumulation of the effector moiety in the cytosol, and which means that some saponins might have other structure elements such as acyl chains, and/or for yet other saponins that display endosomal escape enhancing activity, the sugars can be different than the sugars displayed in Scheme I. For example, the QS-21 saponin and some of the saponins in the water soluble fraction of Quillaja saponaria (Quillaja saponins; Quil-A) differ in the carbohydrate modification at C-28 when the ideal structure of Structure A in Scheme I is considered: presence of an acyl chain in QS-21 for example. In the water soluble fraction of Quillaja saponaria, saponins such as QS-7, QS1862, are similar to the ideal Structure A, and are similar to SO1861.

An embodiment is the first proteinaceous molecule of the invention, wherein the at least one linker is a non-cleavable linker or a cleavable linker, wherein the cleavable linker is for example subject to cleavage under acidic conditions, reductive conditions, enzymatic conditions or light-induced conditions, and preferably the cleavable linker comprises a hydrazone bond or a hydrazide bond subject to cleavage under acidic conditions when bound to saponin, and/or comprises a bond susceptible to proteolysis, for example proteolysis by Cathepsin B, and/or is a bond susceptible for cleavage under reductive conditions such as a disulphide bond, when bound to saponin.

An embodiment is the first proteinaceous molecule of the invention, wherein the cleavable linker is subject to cleavage *in vivo* under acidic conditions as present in endosomes and/or lysosomes of mammalian cells, preferably human cells, preferably at pH 4.0 - 6.5, and more preferably at pH ≤ 5.5, when the cleavable linker is bound to a saponin.

An embodiment is the first proteinaceous molecule of the invention, wherein the oligomeric or polymeric scaffold comprises a polymeric or oligomeric structure and comprises a chemical group, the chemical group for covalently coupling of the scaffold to the amino-acid residue of said first proteinaceous molecule.

According to the invention, typically the saponin is a saponin listed in Table A1, Scheme I. It has been proven beneficial for the activity of the saponin, e.g. the endosomal escape enhancing activity inside cells when the entry into the cell and the accumulation inside the cytosol of an effector moiety covalently coupled to the second or third proteinaceous molecule, is considered, when the saponin is covalently coupled to the first proteinaceous molecule involving a hydrazone bond, and/or a hydrazide bond, and/or a disulphide bond. Such bond types readily cleave under the acidic conditions inside (late) endosomes and lysosomes of mammalian cells, e.g. human cells, and/or under the reductive conditions. Alternatively, the inventors also demonstrate that covalent coupling of saponin to the first proteinaceous molecule via a bond that is not readily cleavable under the physiological conditions inside cells, e.g. (late) endosomes, lysosomes, cytosol, is also beneficial to the potentiating activity of the saponin on the biological effect of e.g. an effector moiety such as a nucleic acid (e.g. BNA silencing HSP27) and a proteinaceous toxin such as saporin. Throughout the application, including the claims, the term 'cleavable linker', 'cleavable bond', etc., is also referred to as 'labile linker' ('L') and 'labile bond', for example in the context of cleavage of such a bond or linker in the (late) endosome and/or lysosome when a conjugate of the invention, e.g. a first proteinaceous molecule optionally comprising a scaffold with saponins coupled to the first proteinaceous molecule through a linker and/or via the scaffold via hydrazone bonds or disulphide bonds, is referred to. For example, Figure 6 and 7 shows the in vivo HSP27 gene silencing in human tumors in mice. The tumor-bearing mice were treated with a first proteinaceous molecule consisting of monoclonal antibody with saponin bound thereto via a labile linker (hydrazone bond) according to the invention, whereas the third proteinaceous molecule comprised bound antisense BNA for silencing the HSP27 gene in the tumor cells, covalently coupled to the monoclonal antibody (same type as the first monoclonal antibody) via a a disulphide bond. That is to say, without wishing to be bound by any theory, the hydrazone bond and the disulphide bond are cleaved in the (late) endosomes and/or lysosomes of the targeted tumor cells that express the epitope on the targeted cell-surface molecule, here the EGFR, at the cell surface, once the therapeutic combination of the invention is internalized by e.g. endocytosis. Cleavage of the bonds likely contributes to the endosomal escape enhancing activity of the saponin when the entry of the BNA from the endosome and/or lysosome into the cytosol is considered, although such cleavage is not a necessity for observing the gene silencing effect of the combination of the cetuximab-SO1861 conjugate and the cetuximab-BNA conjugate of the invention.

The skilled person will appreciate that a tri-functional linker is a scaffold of the invention suitable for covalently coupling one, two or three saponin moieties. For the tri-functional linker covalent coupling of one or two saponin moieties is preferred. The second and/or third binding site is for example suitable for covalent coupling a proteinaceous ligand such as the first proteinaceous molecule. Typical proteinaceous ligands are EGF for targeting (tumor) cells expressing EGFR at the cell surface, and cytokines for targeting tumor cells or autoimmune cells. Moreover, the second or third binding site of the tri-functional linker is suitable for covalent coupling of an immunoglobulin such as a monoclonal antibody, i.e. the first proteinaceous molecule for binding to a cell surface molecule such as a tumor cell surface molecule, preferably a tumor-cell specific molecule, more preferably a tumor cell receptor that is specifically (over-)expressed at the surface of the tumor cell. Similarly, the immunoglobulin, or any fragment(s) and/or domain(s) thereof which encompass the binding specificity of the immunoglobulin, is suitable for binding to a cell surface molecule such as a receptor, expressed at the surface of an autoimmune cell. Thus, in an embodiment, the first proteinaceous molecule comprises the tri-functional linker, said linker comprises or consists of a covalently bound saponin, e.g. QS-21, SO1861, and the covalently bound binding site such as a cell targeting moiety such as a ligand or an antibody for (specific) binding to a tumor cell, an auto-immune cell, a diseased cell, an aberrant cell, a non-healthy cell, a B-cell disease.

Provided and not claimed is the first proteinaceous molecule of the invention, comprising the oligomeric tri-functional linker as the scaffold core structure, according to Scheme II: 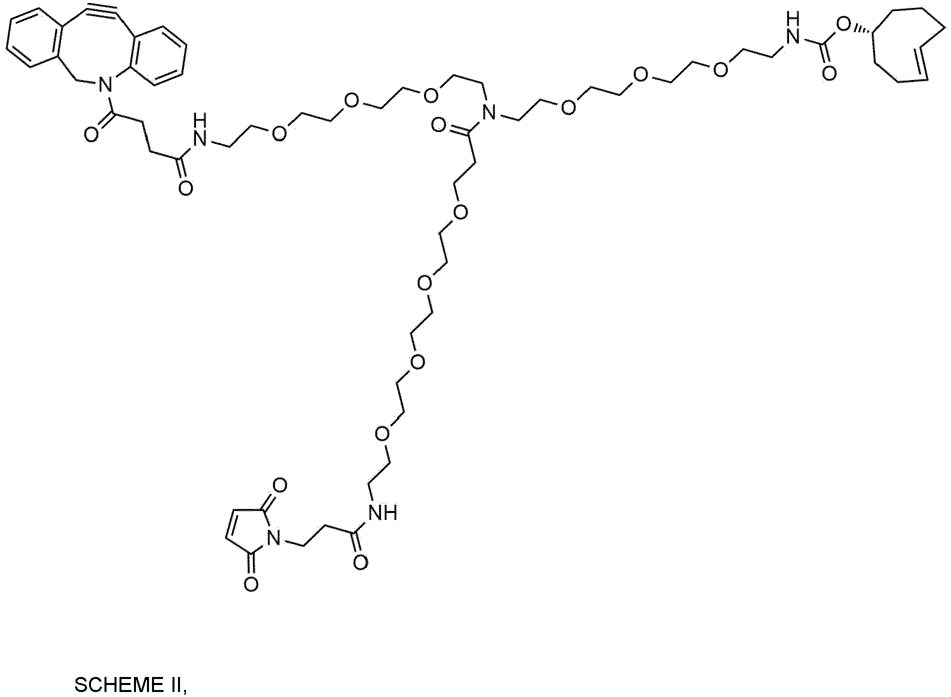 wherein the saponins are covalently bound to the tri-functional linker scaffold via labile, cleavable hydrazone linkers (acid sensitive) and/or via a maleimide comprising bond, whereas the binding of the scaffold to the binding site such as an antibody is established via labile, cleavable hydrazone linkers (acid sensitive) and/or via a maleimide comprising bond with cysteines in the binding site such as 1, 2, 3 or 4 cysteines, therewith forming Structure B: 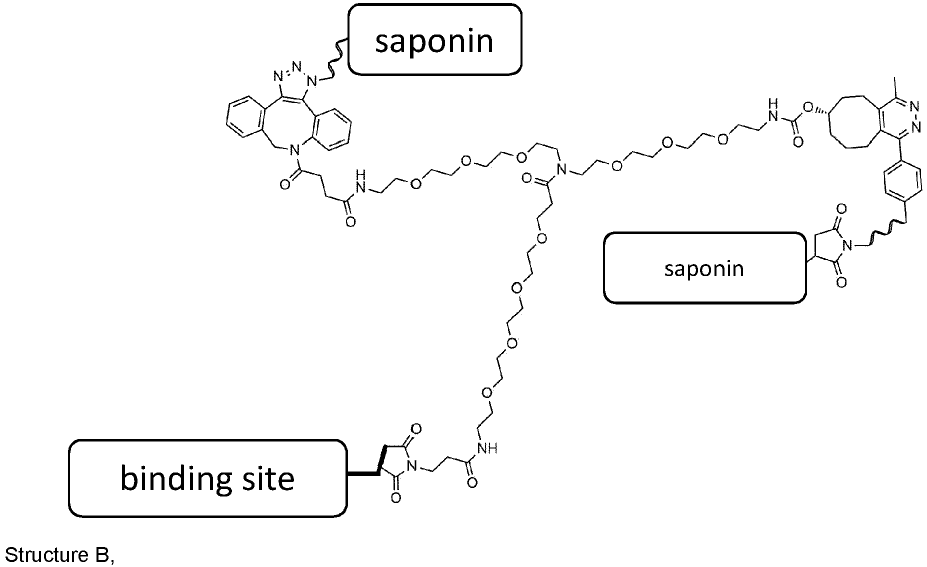 such that 1-4 scaffolds are covalently bound to a single e.g. antibody such as a monoclonal antibody.

An embodiment is the first proteinaceous molecule of the invention wherein the glycoside molecule is a saponin and the linkage between saponin and the first proteinaceous molecule preferably occurs via an acid-labile bond that is stable at pH 7.4 and, preferably releases the saponin below pH 6.5, more preferably between pH 6.5 and 5.0. This is, e.g., realized via an imine formed by an amino group of a linker linking the saponin and the first proteinaceous molecule and the aldehyde group of the saponin. Other chemical bonds that fulfill the pH-condition can also be used for aldehyde coupling, e.g. particular hydrazones or acetals, requiring hydrazides and hydroxyl groups as the functional group of the linker, respectively. If the bond is a cleavable bond, a saponin is preferably attached to the polymeric or oligomeric structure of a scaffold via an aldehyde function or via one of the carboxyl groups in saponin, more preferably through the aldehyde function, preferably an aldehyde function in position 23. Alternatively, a saponin is preferably attached to the first proteinaceous molecule via the polymeric or oligomeric structure of the scaffold via a linker that connects the polymeric or oligomeric structure of the scaffold either via the aldehyde function or via the carboxylic acid function of the glycoside molecule, i.e. the saponin.

Provided but not claimed is the first proteinaceous molecule of the invention, wherein the at least one saponin is bound to the first proteinaceous molecule via a stable bond. Provided but not claimed, is when the stable bond between saponin first proteinaceous molecule preferably occurs via an amide coupling or amine formation. This is, e.g., realized via carbodiimide mediated amide bond formation by an amino group of a polymeric or oligomeric scaffold structure linking the saponin and the first proteinaceous molecule together, and the activated glucuronic acid group of the saponin. Chemical bonds that fulfill the stable bond definition can also be used for aldehyde coupling, e.g. particular amines derived after reductive amination, requiring primary amino groups as the functional group of a polymeric or oligomeric structure of a scaffold or a linker. If the bond is a stable bond, the saponin is preferably attached to a linker or a scaffold via one of the carboxyl groups of the saponin, the linker or scaffold further linked to the first proteinaceous molecule.

An embodiment is the first proteinaceous molecule of the invention wherein the saponin is coupled to the binding site via a scaffold according to the invention, wherein the chemical group for covalently coupling of the scaffold to the binding site is a click chemistry group.

An embodiment is the first proteinaceous molecule of the invention wherein the saponin is coupled to the binding site via a scaffold according to the invention,, wherein the click chemistry group is a tetrazine, an azide, an alkene or an alkyne, or a cyclic derivative of any of these groups, preferably an azide. A click chemistry group is a functional chemical group suitable for click chemistry, which is defined as a reaction that is modular, wide in scope, gives very high yields, generates only inoffensive byproducts, offers high selectivity, and high tolerance over different functional groups, and is stereospecific. The required process characteristics include simple reaction conditions, readily available starting materials and reagents, the use of no solvent or a solvent that is benign (such as water) or easily removed, and simple product isolation. The click chemistry group for coupling the saponin to the binding site in the first proteinaceous molecule optionally via a scaffold or a linker, is preferably a tetrazine, azide, alkene, or alkyne, or reactive derivates of them such as methyl-tetrazine or maleimide (alkene), more preferably an alkyne, or a cyclic derivative of these groups, such as cyclooctyne (e.g. azadibenzocyclooctyne, difluorocyclooctyne, bicyclo[6.1.0]non-4-yne, dibenzocyclooctyne).

A first proteinaceous molecule according to the invention thus comprises at least one saponin selected from the group consisting of: SO1861, SA1641, GE1741, QS-21, QS-21A, QS21-B, Quil-A, SO1832, SO1904, and SO1862. With "at least one" in this context is meant that the first proteinaceous molecule comprises one saponin molecule but may also comprise a couple (e.g. two, three or four) of saponins or a multitude (e.g. 10, 20 or 100) of saponins. Depending on the application, the first proteinaceous molecule may comprise a covalently bound scaffold with covalently bound saponins, wherein the scaffold may be designed such that it comprises a defined number of saponins. Preferably, a first proteinaceous molecule according to the invention comprises a defined number or range of saponins, rather than a random number. This is especially advantageous for drug development in relation to marketing authorization. A defined number in this respect means that a first proteinaceous molecule preferably comprises a previously defined number of saponins. This is, e.g., achieved by designing a scaffold comprising a polymeric structure with a certain number of possible moieties for the saponin(s) to attach. Under ideal circumstances, all of these moieties are coupled to a saponin and the scaffold than comprises the prior defined number of saponins. It is envisaged to offer a standard set of scaffolds, comprising, e.g., two, four, eight, sixteen, thirty-two, sixty-four, etc., saponins so that the optimal number can be easily tested by the user according to his needs. An embodiment is the first proteinaceous molecule of the invention comprising the scaffold of the invention, wherein the saponin is present in a defined range as, e.g., under non-ideal circumstances, not all moieties present in a polymeric structure bind a saponin. Such ranges may for instance be 2 - 4 saponin molecules per scaffold, 3 - 6 saponin molecules per scaffold, 4 - 8 saponin molecules per scaffold, 6 - 8 saponin molecules per scaffold, 6 - 12 saponin molecules per scaffold and so on. In such case, a first proteinaceous molecule comprising a scaffold according to the invention thus comprises 2, 3 or 4 saponins if the range is defined as 2 - 4.

The scaffold is fundamentally independent of the type of saponin covalently bound to the scaffold, the scaffold subsequently (in sequential order) covalently coupled to the first proteinaceous molecule. Thus, first proteinaceous molecule comprising the scaffold is the basis product for a new platform technology. Since the at least one covalently bound saponin mediates intracellular delivery of the effector moiety bound to the second proteinaceous molecule, the scaffold technology according to the invention is the first system known that mediates controlled intracellular effector moiety delivery by saponins. The scaffold provides an optimized and functionally active unit that can be linked to the saponin(s) and to the binding site comprised by the first proteinaceous molecule, e.g. a ligand, an antibody, etc., at a single and defined position.

Provided but not claimed is the first proteinaceous molecule comprising a scaffold according to the invention, wherein the number of monomers of the polymeric or oligomeric structure is an exactly defined number or range. Preferably, the polymeric or oligomeric structure comprises structures such as poly(amines), e.g., polyethylenimine and poly(amidoamine), or structures such as polyethylene glycol, poly(esters), such as poly(lactides), poly(lactams), polylactide-co-glycolide copolymers, poly(dextrin), or a peptide or a protein, or structures such as natural and/or artificial polyamino acids, e.g. poly-lysine, DNA polymers, stabilized RNA polymers or PNA (peptide nucleic acid) polymers, either appearing as linear, branched or cyclic polymer, oligomer, dendrimer, dendron, dendronized polymer, dendronized oligomer or assemblies of these structures, either sheer or mixed. Preferably, the polymeric or oligomeric structures are biocompatible, wherein biocompatible means that the polymeric or oligomeric structure does not show substantial acute or chronic toxicity in organisms and can be either excreted as it is or fully degraded to excretable and/or physiological compounds by the body's metabolism. Assemblies can be built up by covalent cross-linking or non-covalent bonds and/or attraction. They can therefore also form nanogels, microgels, or hydrogels, or they can be attached to carriers such as inorganic nanoparticles, colloids, liposomes, micelles or particle-like structures comprising cholesterol and/or phospholipids. Said polymeric or oligomeric structures preferably bear an exactly defined number or range of coupling moieties for the coupling of glycoside molecules (and/or effector molecules and/or carrier molecules such as a ligand, monoclonal antibody or a fragment thereof). Preferably at least 50%, more preferably at least 75%, more preferably at least 85%, more preferably at least 90%, more preferably at least 95%, more preferably at least 98%, more preferably at least 99%, most preferably 100% of the exactly defined number or range of coupling moieties in the polymeric or oligomeric structure is occupied by a glycoside molecule in a scaffold according to the invention.

Preferably, a dendron is a branched, clearly defined tree-like polymer with a single chemically addressable group at the origin of the tree, called the focal point. A dendrimer is a connection of two or more dendrons at their focal point. A dendronized polymer is a connection of the focal point of one or more dendrons to a polymer. In a preferred embodiment, a scaffold according to the invention is provided, wherein the polymeric or oligomeric structure comprises a linear, branched or cyclic polymer, oligomer, dendrimer, dendron, dendronized polymer, dendronized oligomer or assemblies of these structures, either sheer or mixed, wherein assemblies can be built up by covalent cross-linking or non-covalent attraction and can form nanogels, microgels, or hydrogels, and wherein, preferably, the polymer is a derivative of a poly(amine), e.g., polyethylenimine and poly(amidoamine), and structures such as polyethylene glycol, poly(esters), such as poly(lactids), poly(lactams), polylactide-co-glycolide copolymers, and poly(dextrin), and structures such as natural and/or artificial polyamino acids such as poly-lysine, or a peptide or a protein or DNA polymers, stabilized RNA polymers or PNA (peptide nucleic acid) polymers. Preferably, the polymeric or oligomeric structures are biocompatible.

An embodiment is the therapeutic combination of the invention or the therapeutic combination for use according to the invention, wherein the first proteinaceous molecule comprises more than one covalently bound saponin, preferably 2, 3, 4, 5, 6, 8, 10, 16, 32, 64, 128 or 1-100 saponins, or any number of saponins therein between, such as 7, 9, 12 saponins.

An embodiment is the first proteinaceous molecule of the invention, wherein the at least one saponin is covalently bound to the polymeric or oligomeric structure of the oligomeric or polymeric scaffold via at least one cleavable linker according to the invention.

An embodiment is the first proteinaceous molecule of the invention, wherein the chemical group of the oligomeric or polymeric scaffold, for covalently coupling of the oligomeric or polymeric scaffold to the amino-acid residue of said first proteinaceous molecule, is a click chemistry group, preferably selected from a tetrazine, an azide, an alkene or an alkyne, or a cyclic derivative of these groups, more preferably said chemical group is an azide.

An embodiment is the first proteinaceous molecule of the invention, wherein the polymeric or oligomeric structure of the oligomeric or polymeric scaffold comprises a linear, branched and/or cyclic polymer, oligomer, dendrimer, dendron, dendronized polymer, dendronized oligomer, a DNA, a polypeptide, poly-lysine, a poly-ethylene glycol, or an assembly of these polymeric or oligomeric structures which assembly is preferably built up by covalent cross-linking.

The inventors established that covalent coupling, preferably via cleavable bonds or linkers, of the saponin to the first proteinaceous molecule, according to any of the embodiments here above, provides efficient and cell-targeted potentiation of the activity of an effector moiety bound to the second and to the third proteinaceous molecule, wherein the first and third proteinaceous molecules comprise the same first binding site and wherein the first and second proteinaceous molecules comprise a first and second binding site which are different. Coupling saponin to a cysteine side chain or a lysine side chain of the first proteinaceous molecule such as a monoclonal antibody, directly or via a linker, proved to be a beneficial way of specific and efficient delivery of effector-moiety potentiating activity inside the target cell, when also the effector moiety is delivered in the same target cell by using the second and/or third proteinaceous molecule comprising the same first binding site as the first proteinaceous molecule when the third proteinaceous molecule is considered and comprising different first and second binding sites respectively when the first and second proteinaceous molecules are considered.

To explain the invention in more detail, the process of cellular uptake of substances (although the inventors do not wish to be bound by any theory) and the used terminology in this invention is described. The uptake of extracellular substances into a cell by vesicle budding is called endocytosis. Said vesicle budding can be characterized by (1) receptor-dependent ligand uptake mediated by the cytosolic protein clathrin, (2) lipid-raft uptake mediated by the cholesterol-binding protein caveolin, (3) unspecific fluid uptake (pinocytosis), or (4) unspecific particle uptake (phagocytosis). All types of endocytosis run into the following cellular processes of vesicle transport and substance sorting called the endocytic pathways. The endocytic pathways are complex and not fully understood. Without wishing to be bound by any theory, organelles may be formed *de novo* and mature into the next organelle along the endocytic pathway. It is however, now hypothesized that the endocytic pathways involve stable compartments that are connected by vesicular traffic. A compartment is a complex, multifunctional membrane organelle that is specialized for a particular set of essential functions for the cell. Vesicles are considered to be transient organelles, simpler in composition, and are defined as membrane-enclosed containers that form *de novo* by budding from a preexisting compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles. The endocytic vesicle, which arises at the plasma membrane, most prominently from clathrin-coated pits, first fuses with the early endosome, which is a major sorting compartment of approximately pH 6.5. A large part of the cargo and membranes internalized are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of an endocytosed molecule occurs inside an endolysosome or lysosome. Endosomal escape is the active or passive release of a substance from the inner lumen of any kind of compartment or vesicle from the endocytic pathway, preferably from clathrin-mediated endocytosis, or recycling pathway into the cytosol. Endosomal escape thus includes but is not limited to release from endosomes, endolysosomes or lysosomes, including their intermediate and hybrid organelles.

Unless specifically indicated otherwise and in particular when relating to the endosomal escape mechanism of the glycoside molecule such as the saponin of the invention, whenever the word "endosome" or "endosomal escape" is used herein, it also includes the endolysosome and lysosome, and escape from the endolysosome and lysosome, respectively. After entering the cytosol, said substance might move to other cell units such as the nucleus.

In formal terms, a glycoside is any molecule in which a sugar group is bound through its anomeric carbon to another group via a glycosidic bond. Glycoside molecules, such as saponins, in the context of the invention are such molecules that are further able to enhance the effect of an effector moiety, without wishing to be bound by any theory, in particular by facilitating the endosomal escape of the effector moiety. Without wishing to be bound by any theory, the glycoside molecules (saponins, such as those listed in Table A1) interact with the membranes of compartments and vesicles of the endocytic and recycling pathway and make them leaky for said effector moieties resulting in augmented endosomal escape. With the term "the scaffold is able to augment endosomal escape of the effector moiety" is meant that the at least one saponin (glycoside molecule), which is coupled to the polymeric or oligomeric structure of the scaffold, is able to enhance endosomal escape of an effector moiety when both molecules are within an endosome, e.g. a late endosome, optionally and preferably after the at least one glycoside such as a saponin is released from the first proteinaceous molecule such as from a linker or polymeric or oligomeric structure comprised by said first proteinaceous molecule, e.g., by cleavage of a cleavable bond between the at least one glycoside (saponin) and the the first proteinaceous molecule (for example via a polymeric or oligomeric structure of a scaffold and/or via a linker). Even though a bond between the at least one glycoside such as a saponin according to the invention and the first proteinaceous molecule, optionally via a linker or a scaffold, may be a "stable bond", that does not mean that such bond cannot be cleaved in the endosomes by, e.g., enzymes. For instance, the glycoside or saponin, optionally together with a linker or a part of the oligomeric or polymeric structure of a scaffold, may be cleaved off from the remaining linker fragment or oligomeric or polymeric structure. It could, for instance be that a protease cuts a (proteinaceous) linker or proteinaceous polymeric structure, e.g., albumin, thereby releasing the at least one glycoside, saponin. It is, however, preferred that the glycoside molecule (preferably saponin) is released in an active form, preferably in the original form that it had before it was (prepared to be) coupled to the first proteinaceous molecule optionally via a linker and/or an oligomeric or polymeric scaffold; thus the glycoside (saponin) has its natural structure after such cleavage or the glycoside (saponin) has (part of) a chemical group or linker bound thereto, after such cleavage, while glycoside biological activity (saponin biological activity), e.g. endosomal/lysosomal escape enhancing activity towards an effector moiety present in the same endosome or lysosome, is maintained or restored upon said cleavage of the bond between the glycoside (saponin) and the carrier molecule, i.e. the first proteinaceous molecule optionally comprising a linker and/or a scaffold of the invention. With regard to the present invention the term "stable" with respect to bonds between e.g. saponins and amino-acid residues of the first proteinaceous molecule, a linker, a polymeric or oligomeric structures (of the scaffold), ligands, (monoclonal) immunoglobulins or binding domains or-fragments thereof, and/or effectors (effector moieties, effector molecules), is meant that the bond is not readily broken or at least not designed to be readily broken by, e.g., pH differences, salt concentrations, or UV-light, reductive conditions. With regard to the present invention the term "cleavable" with respect to bonds between e.g. saponins and the first proteinaceous molecule, linkers, amino-acid residues, polymeric or oligomeric structures of the scaffold, ligands, antibodies and/or effectors, is meant that the bond is designed to be readily broken by, e.g., pH differences, salt concentrations, under reductive conditions, and the like. The skilled person is well aware of such cleavable bonds and how to prepare them.

Before the present invention one of the major hurdles of introducing ADCs and AOCs on the market was the small therapeutic window: a therapeutically effective dose of an ADC or an AOC is accompanied with (unacceptable) side effects, hampering development and implication in treatment of patients with the ADCs. By the application of the first proteinaceous molecule of the invention it has now become possible to guide one or multiple glycoside molecules (saponin) to a (target) cell, together with the ADC carrying a payload or together with a (monoclonal) antibody conjugated with an oligonucleotide such as a BNA according to the invention (i.e. a particular second or third proteinaceous molecule of the invention). In particular, it was previously not possible to specifically guide an effector moiety of a second or third proteinaceous molecule and a (predefined, controllable) particular number or range of glycoside molecules (saponins) per effector moiety at the same time to the cytosol of cells, such as via the endocytic pathway of a cell.

A solution provided for by the invention comprises the covalent binding of at least one saponin selected from the group consisting of: SO1861, SA1641, GE1741, QS-21, QS-21A, QS21-B, Quil-A, SO1832, SO1904, and SO1862 to the first proteinaceous molecule. A further solution provided for by the invention comprises (first) polymerizing the glycoside molecules (saponins) using an oligomeric or polymeric scaffold, and providing the first proteinaceous molecule with a cluster of covalently bound saponins, enabling re-monomerization of the one or more saponins at the intracellular site where the mode of action of the saponin is desired, e.g. after endocytosis. "Polymerizes" in this context means the reversible and/or irreversible multiple conjugation of saponin molecules to the first proteinaceous molecule, either via linker, or directly or via a polymeric or oligomeric structure to form a scaffold or the reversible and/or irreversible multiple conjugation of (modified) saponins thereby forming a polymeric or oligomeric structure to form a scaffold. "Re-monomerization" in this context means the cleavage of the saponins from the first proteinaceous molecule, from the linker linking the saponin(s) to the first proteinaceous molecule or from the scaffold, for example after endocytosis, and regaining the (native) chemical state of the unbound saponins, which unbound saponins may or may not comprise additional chemical groups such as a chemical group for linking the saponin to a linker, an amino-acid residue of the first proteinaceous molecule or to the scaffold, and/or a (chemical) linker bound to a chemical group of the saponin such as an aldehyde group or carboxylic acid group. Due to the complex chemistry of the saponins for example the 'polymerization' of saponins at a scaffold or other linking linker and their 're-monomerization' at a desired location such as intracellularly e.g. after endocytosis, was a challenging task. In particular, the chemical reactions used for providing the linkers and the scaffold comprising covalently linked glycosides for covalent binding to the first proteinaceous molecule, e.g. triterpenoid saponins (polymerization of the glycosides), normally occur in water-free organic solvents, but saponins and for example biocompatible polymers applied as a scaffold for bearing bound saponins, are water-soluble molecules. The chemical properties of the unmodified saponin further prohibited polymerization by itself and, one other possible solution, to bind multiple saponins (directly) to the effector molecule was estimated not to be very promising, as an effector molecule (drug, toxin, polypeptide or polynucleotide) does typically not provide sufficient binding sites and because the coupling product would become quite heterogeneous and/or coupling biologically active molecules such as a saponin and e.g. a peptide, a toxin, a nucleic acid together bears the risk for influencing and hampering the activity of one or even both molecules bound together in such saponin-comprising conjugate. Further, there was a considerable risk that the effector moiety comprised by the second or third proteinaceous molecule loses its function after coupling of a saponin to the e.g. ADC or antibody-oligonucleotide conjugate (AOC). Embodiments of the present invention solves at least one of these drawbacks.

An aspect of the invention relates to a composition comprising the first proteinaceous molecule of the invention and the second proteinaceous molecule of the invention.

An aspect of the invention relates to a composition comprising the first proteinaceous molecule of the invention and the third proteinaceous molecule of the invention.

An embodiment is the composition comprising the first proteinaceous molecule of the invention and the second proteinaceous molecule of the invention, or is the composition comprising the first proteinaceous molecule of the invention and the third proteinaceous molecule of the invention, wherein the effector moiety that is comprised by the second proteinaceous molecule or by the third proteinaceous molecule is any one of the effector moieties according to the invention, preferably a BNA.

An aspect of the invention relates to a composition comprising the first proteinaceous molecule of the invention and any one or more of an oligonucleotide, a nucleic acid and a xeno nucleic acid, preferably selected from at least one of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression (antisense BNA(HSP27)).

An effector molecule, or effector moiety, in the context of this invention is any substance that affects the metabolism of a cell by interaction with an intracellular effector molecule target, wherein this effector molecule target is any molecule or structure inside cells excluding the lumen of compartments and vesicles of the endocytic and recycling pathway but including the membranes of these compartments and vesicles. Said structures inside cells thus include the nucleus, mitochondria, chloroplasts, endoplasmic reticulum, Golgi apparatus, other transport vesicles, the inner part of the plasma membrane and the cytosol. Cytosolic delivery of an effector moiety in the context of the invention preferably means that the effector moiety is able to escape the endosome (and/or lysosome), which, as defined previously, also includes escaping the endolysosome and the lysosome, and is preferably able to reach the effector moiety target as described herein. The invention also encompasses a new type of molecule, referred to as scaffold that serves to bring both an effector moiety and at least one glycoside molecule such as a saponin of the invention in an endosome at the same time in a pre-defined ratio, when the effector moiety is comprised by the second or third proteinaceous molecule of the invention and the saponin is comprised by the first proteinaceous molecule. Within the context of the present invention, the polymeric or oligomeric structure of the scaffold is a structurally ordered formation such as a polymer, oligomer, dendrimer, dendronized polymer, or dendronized oligomer or it is an assembled polymeric structure such as a hydrogel, microgel, nanogel, stabilized polymeric micelle or liposome, but excludes structures that are composed of non-covalent assemblies of monomers such as cholesterol/phospholipid mixtures. The terms "polymer, oligomer, dendrimer, dendronized polymer, or dendronized oligomer" have their ordinary meaning. In particular a polymer is a substance which has a molecular structure built up chiefly or completely from a large number of equal or similar units bonded together and an oligomer is a polymer whose molecules consist of relatively few repeating units. There is no consensus about one specific cut-off for "many" and "a few" as used in the above definition of polymer and oligomer, respectively. However, as the scaffold may comprise a polymeric or an oligomeric structure, or both, the full range of numbers of similar units bonded together applies to such structure. i.e. from 2 monomeric units to 100 monomeric units, 1000 monomeric units, and more. A structure comprising 5 or less, for instance maybe called an oligomeric structure, whereas a structure comprising 50 monomeric units maybe called a polymeric structure. A structure of 10 monomeric units maybe called either oligomeric or polymeric. A scaffold as defined herein, further comprises at least one glycoside molecule such as a saponin of the invention. A scaffold preferably includes a polymeric or oligomeric structure such as poly- or oligo(amines), e.g., polyethylenimine and poly(amidoamine), and biocompatible structures such as polyethylene glycol, poly- or oligo(esters), such as poly(lactids), poly(lactams), polylactide-co-glycolide copolymers, and poly(dextrin), poly- or oligosaccharides, such as cyclodextrin or polydextrose, and poly- or oligoamino acids, such as poly-lysine or a peptide or a protein, or DNA oligo- or polymers. An assembled polymeric structure as defined herein comprises at least one scaffold and, optionally, other individual polymeric or oligomeric structures. Other individual polymeric or oligomeric structures of said assembly may be (a) scaffolds (thus comprising at least one glycoside molecule such as a saponin of the invention), (b) functionalized scaffolds (thus comprising at least one glycoside molecule such as a saponin, and a ligand, antibody, etc. as the first proteinaceous molecule, (c) polymeric or oligomeric structures without a glycoside molecule such as a saponin of the invention (See Table A1 for example), without a ligand, antibody, etc., as the first proteinaceous molecule. A functionalized assembled polymeric structure is an assembled polymeric structure that contains (a) at least one functionalized scaffold or (b) at least one scaffold and at least one polymeric structure comprising at least one ligand, antibody, etc. as the first proteinaceous molecule. Polymeric or oligomeric structures within an assembled polymeric structure that do not comprise any of the above mentioned molecules (i.e. no glycosides such as saponins, no first proteinaceous molecule such as ligands, antibodies) are in particular added as structural components of the assembled structures, which help to build up or to stabilize the assembled structure ("glue-like").Without wishing to be bound by any theory, the acidic environment seems to be a prerequisite for the synergistic action between glycoside (saponin) and effector moiety.

Whether or not a first proteinaceous molecule comprising saponins, either or not further comprising one or more (cleavable) linkers and/or optionally a scaffold, is able to disturb the acidic environment and inhibit the endosomal escape function of the at least one glycoside (saponin) can be easily determined with an assay as described in Example 3 and as known in the art. The inhibition is described as "fold amount increases of glycoside necessary to induced 50% cell killing". It is preferred that the scaffold does not lead to an increase that is at least the increase in glycoside molecules (saponins) necessary to obtain 50% cell killing observed when using Chloroquine as a positive control. Alternatively, and preferably, the first proteinaceous molecule comprising saponins, either or not further comprising one or more (cleavable) linkers and/or optionally a scaffold does not lead to an at least 4-fold increase of glycoside molecules to induce 50% cell killing, more preferably does not lead to an at least 2-fold increase. The fold increase is to be measured in assay, essentially as described in Example 4, wherein Chloroquine, as a positive control, induces a 2-fold increase in glycoside amount, preferably saponin amount wherein the saponin is any one or more of the saponins of the invention (see Table A1, Scheme I, previous embodiments) to observe 50% cell killing.

With the term "improving or enhancing an effect of an effector moiety" is meant that the glycoside molecule, preferably a saponin of the invention, increases the functional efficacy of that effector moiety (e.g. the therapeutic index of a toxin or a drug or an oligonucleotide such as a BNA; the metabolic efficacy of a modifier in biotechnological processes; the transfection efficacy of genes in cell culture research experiments), preferably by enabling or improving its target engagement. Acceleration, prolongation, or enhancement of antigen-specific immune responses are preferably not included. Therapeutic efficacy includes but is not limited to a stronger therapeutic effect, preferably with lower dosing and/or with less side effects. "Improving an effect of an effector moiety" can also mean that an effector moiety, which could not be used because of lack of effect (and was e.g. not known as being an effector moiety), becomes effective when used in combination with the present invention. Any other effect, which is beneficial or desired and can be attributed to the combination of effector moiety and the second or third proteinaceous molecule, as provided by the invention is considered to be "an improved effect". In an embodiment, the scaffold comprising bound saponin(s) and comprised by the first proteinaceous molecule enhances an effect of the effector moiety comprised by the second proteinaceous molecule which effect is intended and/or desired. In case of a first proteinaceous molecule comprising saponin bound to a proteinaceous scaffold, the proteinaceous polymeric structure of the scaffold as such may have, for instance, an effect on colloid osmotic pressure in the blood stream. If such effect is not the intended or desired effect of such a functionalized scaffold comprised by the first proteinaceous molecule, the proteinaceous structure of the scaffold is not an effector moiety as defined in the invention. Or, for instance in case of a DNA- or RNA-based scaffold carrying bound saponins and comprised by the first proteinaceous molecule, parts of that DNA or RNA may have an (unintended) function, e.g., by interfering with expression. If such interference is not the intended or desired effect of the ultimate functionalized scaffold, the DNA- or RNA polymeric structure of the scaffold is not the effector moiety as defined in the invention.

A number of preferred features can be formulated for endosomal escape enhancers comprised by the first proteinaceous molecule, i.e. a glycoside or saponin, preferably a saponin according to the invention: (1) they are preferably not toxic and do not invoke an immune response, (2) they preferably do not mediate the cytosolic uptake of the effector moiety into off-target cells, (3) their presence at the site of action is preferably synchronized with the presence of the effector moiety, (4) they are preferably biodegradable or excretable, and (5) they preferably do not substantially interfere with biological processes of the organism unrelated to the biological activity of the effector molecule with which the endosomal escape enhancer is combined with, e.g. interact with hormones. Examples of glycoside molecules such as saponins selected from the group consisting of: SO1861, SA1641, GE1741, QS-21, QS-21A, QS21-B, Quil-A, SO1832, SO1904, and SO1862 of the invention that fulfill the before mentioned criteria, at least to some extent, are bisdesmosidic triterpenes, preferably bisdesmosidic triterpene saponins, such as SO1861, SA1641, QS-21, GE1741, and the saponins in Table A1, Scheme I.

An aspect of the invention relates to an antibody-drug conjugate or an antibody-oligonucleotide conjugate or a ligand-drug conjugate comprising the first proteinaceous molecule of the invention and an effector moiety.

An embodiment is the antibody-drug conjugate or ligand-drug conjugate of the invention, wherein the antibody can bind to any one of CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably CD71, HER2, EGFR, and/or is or comprises any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell receptor binding-fragment thereof and/or at least one tumor-cell receptor binding-domain thereof, and/or wherein the antibody-drug conjugate comprises any one of Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3. Provided and not claimed is the ligand-drug conjugate which comprises at least one ligand for binding to a cell-surface molecule such as EGF or a cytokine.

An embodiment is the antibody-drug conjugate or antibody-oligonucleotide conjugate or ligand-drug conjugate of the invention, wherein the effector moiety is any one or more of the effector moieties according to the invention.

An aspect of the invention relates to a pharmaceutical composition comprising the composition comprising the first proteinaceous molecule of the invention and the second proteinaceous molecule of the invention, or comprising the first proteinaceous molecule of the invention and the third proteinaceous molecule of the invention, or comprising the antibody-drug conjugate of the invention or comprising the antibody-oligonucleotide conjugate of the invention or comprising the ligand-drug conjugate of the invention, and optionally further comprising a pharmaceutically acceptable excipient.

An aspect of the invention relates to the the therapeutic combination of the invention, either comprosing the second pharmaceutical composition or comprising the third pharmaceutical composition, or comprising the composition comprising the first proteinaceous molecule of the invention and the second proteinaceous molecule of the invention, or comprising the first proteinaceous molecule of the invention and the third proteinaceous molecule of the invention, or comprising the antibody-drug conjugate or antibody-oligonucleotide conjugate or ligand-drug conjugate of the invention or the pharmaceutical composition of the invention, for use as a medicament.

An aspect of the invention relates to the the therapeutic combination of the invention, either comprosing the second pharmaceutical composition or comprising the third pharmaceutical composition, or comprising the composition comprising the first proteinaceous molecule of the invention and the second proteinaceous molecule of the invention, or comprising the first proteinaceous molecule of the invention and the third proteinaceous molecule of the invention, or comprising the antibody-drug conjugate or antibody-oligonucleotide conjugate or ligand-drug conjugate of the invention or the pharmaceutical composition of the invention, for use in the treatment or prevention of a cancer or an autoimmune disease.

As said before, the at least one saponin that is comprised by the first proteinaceous molecule according to the invention increases the efficacy of at least current and new effector moieties as defined in this invention. Potential side-effects will be decreased due to lowering of dosing of the effector moiety comprised by the second or third proteinaceous molecule, without lowering the efficacy. Therefore, the invention provides a first proteinaceous molecule according to the invention for use in medicine. Provided is the use of a first proteinaceous molecule according to the invention for manufacturing a medicament. Especially cancer medicines, and in particular the classical chemotherapy medicaments, are notorious for their side effects. Because of targeting and synchronization in time and place of both the pharmaceutically active substance comprised by the second or third proteinaceous molecule and the saponin comprised by the first proteinaceous molecule, since the first and third proteinaceous molecule bear the same binding site for the same epitope on the same cell-surface molecule, or since the first and second proteinaceous molecule bear different binding sites for different first and second epitopes on the first and second cell-surface molecules respectively, a therapeutic combination according to the invention is especially valuable for use as a medicament, in particular for use in a method of treating cancer. The invention thus provides a therapeutic combination according to the invention or a first proteinaceous molecule of the invention for use in a method of treating cancer. The invention also provides a therapeutic combination according to the invention or a first proteinaceous molecule of the invention for use in a method of treating acquired or hereditary disorders, in particular monogenic deficiency disorders. The therapeutic combination thus comprises the first and second proeteinaceous molecule and/or comprises the first and third proteinaceous molecule. Thus, an aspect of the invention relates to a therapeutic combination according to the invention, wherein the second or third proteinaceous molecule comprises a covalently bound effector moiety, for use in a method for the treatment of a cancer or an auto-immune disease.

A further application of the first, second and third proteinaceous molecules of the invention in medicine is the substitution of intracellular enzymes in target cells that produce these enzymes in insufficient amount or insufficient functionality. The resulting disease might be hereditary or acquired. In most cases, only symptomatic treatment is possible and for a number of rare diseases, insufficient treatment options lead to a shortened life span of concerned patients. An example for such a disease is phenylketonuria, which is an inborn error of metabolism that results in decreased metabolism of the amino acid phenylalanine. The disease is characterized by mutations in the gene for the hepatic enzyme phenylalanine hydroxylase. Phenylketonuria is not curable to date. The incidence is approximately 1:10,000 with the highest known incidence in Turkey with 1:2,600. A second or third proteinaceous molecule, preferably an antibody, with bound phenylalanine hydroxylase or with a bound polynucleotide that encodes phenylalanine hydroxylase can be used to target liver cells by use of a suitable specific antibody, and to substitute the defect enzyme in hepatocytes. This is one example of use of the therapeutic combination of the invention comprising a first proteinaceous molecule with a saponin bound thereto and a second or third proteinaceous molecule with the enzyme or the oligonucleotide bound thereto according to the invention for substitution or gene therapy. In a preferred embodiment, a therapeutic combination according to the invention for use in a method of gene therapy or substitution therapy is provided.

The present invention also provides a method of treating cancer, the method comprising administering a medicament comprising a therapeutic combination according to the invention to a patient in need thereof, preferably administering an effective dose of said medicament to a patient in need thereof, preferably a human cancer patient.

Considerations concerning forms suitable for administration are known in the art and include toxic effects, solubility, route of administration, and maintaining activity. For example, pharmacological compositions injected into the bloodstream should be soluble.

Suitable dosage forms, in part depend upon the use or the route of entry, for example transdermal or by injection. Such dosage forms should allow the compound to reach a target cell whether the target cell is present in a multicellular host. Other factors are known in the art, and include considerations such as toxicity and dosage form which retard the compound or composition from exerting its effect.

An embodiment is the combination of an endosomal escape enhancing conjugate according to the invention, comprising the first proteinaceous molecule comprising at least one covalently bound saponin, and a binding moiety, wherein the binding moiety comprises at least one effector moiety, the binding moiety being the second or third proteinaceous molecule comprising the bound effector moiety, wherein the endosomal escape enhancing conjugate and the binding moiety are, independently from one another, able to specifically bind to a target cell-specific surface molecule or structure, thereby inducing receptor-mediated endocytosis of a complex of the endosomal escape enhancing conjugate and the target cell-specific surface molecule, and of the complex of the binding moiety and the target cell-specific surface molecule, wherein the endosomal escape enhancing conjugate and the binding moiety can bind to the same target cell-specific surface molecule via their same binding site, or wherein the endosomal escape enhancing conjugate and the binding moiety can bind to the different target cell-specific surface molecules via their different binding sites. An embodiment is the combination according to the invention, wherein the endosomal escape enhancing conjugate is able to compete with the binding moiety for binding to the target cell-specific surface molecule or structure. An embodiment is the combination according to the invention, wherein the endosomal escape enhancing conjugate and the binding moiety are, independently from one another, able to specifically bind to the same epitope, or to a different epitope. An embodiment is the combination for use in a method for the treatment of an aberrancy such as a cancer according to the invention, wherein said endosomal escape enhancing conjugate and said binding moiety are to be administered concomitant or sequentially, preferably concomitant.

Provided but not claimed is a kit comprising a first container containing an endosomal escape enhancing conjugate according to the invention (i.e. the first proteinaceous molecule) and a second container containing a binding moiety according to the invention (i.e. the second and/or third proteinaceous molecule), the kit further comprising instructions for using the binding molecules (i.e. the therapeutic combination comprising the first and second or the first and third pharmaceutical compositions).

| TABLE A1. Saponins displaying (late) endosomal/lysosomal escape enhancing activity, and saponins comprising a structure reminiscent to such saponins displaying (late) endosomal/lysosomal escape enhancing activity | | | |
|---|---|---|---|
| **Saponin Name** | **Aglycon core** | **Carbohydrate substituent at the C-3beta-OH group** | **Carbohydrate substituent at the C-28-OH group** |
| NP-005236 | 2alpha-Hydroxyoleanolic acid | GlcA- | Glc/Gal- |
| AMA-1 | 16alpha-Hydroxyoleanolic acid | Glc- | Rha-(1→2)-[Xyl-(1→4)]-Rha- |
| AMR | 16alpha-Hydroxyoleanolic acid | Glc- | Rha-(1→2)-[Ara-(1→3)-Xyl-(1→4)]-Rha- |
| alpha-Hederin | Hederagenin (23-Hydroxyoleanolic acid) | Rha-(1→2)-Ara- | - |
| NP-012672 | 16alpha,23-Dihydroxyoleanolic acid | Ara/Xyl-(1→4)-Rha/Fuc-(1→2)-Glc/Gal-(1→2)-Rha/Fuc-(1→2)-GlcA- | Ara/Xyl- |
| NP-017777 | Gypsogenin | Gal-(1→2)-[Xyl-(1→3)]-GlcA- | Xyl-(1→4)-Rha-(1→2)-[R-(→4)]-Fuc-(R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha-(1→2)-4-OAc-Fuc- |
| NP-018110^{c}, NP-017772^{d} | Gypsogenin | Gal-(1→2)-[Xyl-(1→3)]-GlcA- | Xyl-(1→4)-[Glc-(1→3)]-Rha-(1→2)-3,4-di-OAc-Fuc- |
| NP-018109 | Gypsogenin | Gal-(1→2)-[Xyl-(1→3)]-GlcA- | Xyl-(1→4)-[Glc-(1→3)]-Rha-(1→2)-[R-(→4)]-3-OAc-Fuc- (R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/Xyl-(1→4)-Rha/Fuc-(1→2)-[4-OAc-Rha/Fuc-(1→4)]-Rha/Fuc- |
| SA1641^{a}, AE X55^{b} | Gypsogenin | Gal-(1→2)-[Xyl-(1→3)]-GlcA- | Xyl-(1→3)-Xyl-(1→4)-Rha-(1→2)-[Qui-(1→4)]-Fuc- |
| NP-017674 | Quillaic acid | Gal-(1→2)-[Xyl-(1→3)]-GlcA- | Api-(1→3)-Xyl-(1→4)-[Glc-(1→3)]-Rha-(1→2)-Fuc- |
| NP-017810 | Quillaic acid | Gal-(1→2)-[Xyl-(1→3)]-GlcA- | Xyl-(1→4)-[Gal-(1→3)]-Rha-(1→2)-Fuc- |
| AG1 | Quillaic acid | Gal-(1→2)-[Xyl-(1→3)]-GlcA- | Xyl-(1→4)-[Glc-(1→3)]-Rha-(1→2)-Fuc- |
| NP-003881 | Quillaic acid | Gal-(1→2)-[Xyl-(1→3)]-GlcA- | Ara/Xyl-(1→4)-Rha/Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc- |
| | | | (R = 5-O-[5-O-Ara/Api-3,5-dihydroxy-6-methyl-octanoyl]-3,5-dihydroxy-6-methyl-octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]-Fuc- |
| | | | (R = 5-O-[5-O-Ara/Api-3,5-dihydroxy-6-methyl-octanoyl]-3,5-dihydroxy-6-methyl-octanoic acid) |
| NP-017706 | Quillaic acid | Gal-(1→2)-[Xyl-(1→3)]-GlcA- | Api-(1→3)-Xyl-(1→4)-Rha-(1→2)-[Rha-(1→3)]-4-OAc-Fuc- |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc-Rha-(1→3)]-Fuc- |
| SA1657 | Quillaic acid | Gal-(1→2)-[Xyl-(1→3)]-GlcA- | Xyl-(1→3)-Xyl-(1→4)-Rha-(1→2)-[Qui-(1→4)]-Fuc- |
| AG2 | Quillaic acid | Gal-(1→2)-[Xyl-(1→3)]-GlcA- | Glc-(1→3)-[Xyl-(1→4)]-Rha-(1→2)-[Qui-(1→4)]-Fuc- |
| SO1861 | Quillaic acid | Gal-(1→2)-[Xyl-(1→3)]-GlcA- | Glc-(1→3)-Xyl-(1→4)-Rha-(1→2)-[Xyl-(1→3)-4-OAc-Qui-(1→4)]-Fuc- |
| GE1741 | Quillaic acid | Gal-(1→2)-[Xyl-(1→3)]-GlcA- | Xyl-(1→3)-Xyl-(1→4)-Rha-(1→2)-[3,4-di-OAc-Qui-(1→4)]-Fuc- |
| S01542 | Quillaic acid | Gal-(1→2)-[Xyl-(1→3)]-GlcA- | Glc-(1→3)-[Xyl-(1→4)]-Rha-(1→2)-Fuc- |
| SO1584 | Quillaic acid | Gal-(1→2)-[Xyl-(1→3)]-GlcA- | 6-OAc-Glc-(1→3)-[Xyl-(1→4)]-Rha-(1→2)-Fuc- |
| SO1658 | Gypsogenin | Gal-(1→2)-[Xyl-(1→3)]-GlcA- | Glc-(1→3)-[Xyl-(1→3)-Xyl-(1→4)]-Rha-(1→2)-Fuc- |
| SO1674 | Quillaic acid | Gal-(1→2)-[Xyl-(1→3)]-GlcA- | Glc-(1→3)-[Xyl-(1→3)-Xyl-(1→4)]-Rha-(1→2)-Fuc- |
| SO1832 | Quillaic acid | Gal-(1→2)-[Xyl-(1→3)]-GlcA- | Xyl-(1→3)-Xyl-(1→4)-Rha-(1→2)-[Xyl-(1→3)-4-OAc-Qui-(1→4 )]-Fuc- |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc- |
| | | | (R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc- |
| | | | (R = 5-O-[5-O-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc- |
| | | | (R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc- |
| | | | (R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→3)-Xyl-(1→4)-Rha-(1→2)-[R-(→3)]-Fuc- |
| | | | (R = 5-O-[5-O-Ara/Api-3,5-dihydroxy-6-methyl-octanoyl]-3,5-dihydroxy-6-methyl-octanoic acid) |
| QS-21 B-xylo | Quillaic acid | Gal-(1→2)-[Xyl-(1→3)]-GlcA- | Xyl-(1→3)-Xyl-(1→4)-Rha-(1→2)-[R-(→3)]-Fuc- |
| | | | (R = 5-O-[5-O-Ara/Api-3,5-dihydroxy-6-methyl-octanoyl]-3,5-dihydroxy-6-methyl-octanoic acid) |
| beta-Aescin (described: Aescin la) | Protoaescigenin-21(2-methylbut-2-enoate)-22-acetat | Glc-(1→2)-[Glc-(1→4)]-GlcA- | - |
| Teaseed saponin I | 23-Oxobarringtogenol C - 21,22-bis(2-methylbut-2-enoate) | Glc-(1→2)-Ara-(1→3)-[Gal-(1→2)]-GlcA- | - |
| Teaseedsaponin J | 23-Oxobarringtogenol C - 21,22-bis(2-methylbut-2-enoate) | Xyl-(1→2)-Ara-(1→3)-[Gal-(1→2)]-GlcA- | - |
| Assamsaponin F | 23-Oxobarringtogenol C - 21(2-methylbut-2-enoate)-16,22-diacetat | Glc-(1→2)-Ara-(1→3)-[Gal-(1→2)]-GlcA- | - |
| Digitonin | Digitogenin | Glc-(1→3)-Gal-(1→2)-[Xyl-(1→3)]-Glc-(1→4)-Gal- | - |
| Primula acid 1 | 3,16,28-Trihydroxyoleanan-12-en | Rha-(1→2)-Gal-(1→3)-[Glc-(1→2)]-GlcA- | - |
| AS64R | Gypsogenic acid | - | Glc-(1→3)-[Glc-(1→6)]-Gal- |

| | | **Carbohydrate substituent at the C-23-OH group** | |
|---|---|---|---|
| AS6.2 | Gypsogenic acid | Gal- | Glc-(1→3)-[Glc-(1→6)]-Gal- |
| a, b: Different names refer to different isolates of the same structure | | | |
| c, d: Different names refer to different isolates of the same structure | | | |

**TABLE A2 - ADCs which were previously investigated in the human clinical setting, and subsequently retracted from further clinical investigation**

| **Drug Name** | **Indication** | **Target** | **Last Development Stage** |
|---|---|---|---|
| Monoclonal Antibody Conjugate to Target EGFR for Oncology | Oncology | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Discovery |
| Affilutin | Multiple Myeloma (Kahler Disease) | | Discovery |
| IMGN-779 | Myelodys-plastic Syndrome | Cells Expressing Myeloid Cell Surface Antigen CD33 (Sialic Acid Binding Ig Like Lectin 3 or gp67 or CD33) | IND/CTA Filed |
| Neuradiab | Non-Hodgkin Lymphoma | Cells Expressing Tenascin (Cytotactin or GMEM or GP 150-225 or Glioma Associated Extracellular Matrix Antigen or Hexabrachion or JI or Myotendinous Antigen or Neuronectin or Tenascin C or TNC) | Phase I |
| IMGN-779 | Refractory Acute Myeloid Leukemia; Relapsed Acute Myeloid Leukemia | Cells Expressing Myeloid Cell Surface Antigen CD33 (Sialic Acid Binding Ig Like Lectin 3 or gp67 or CD33) | Phase I |
| AGS-67E | Acute Myelocytic Leukemia (AML, Acute Myeloblas-tic Leukemia) | Cells Expressing Leukocyte Antigen CD37 (Tetraspanin 26 or CD37) | Phase I |
| AGS-67E | Hairy Cell Leukemia; Non-Hodgkin Lymphoma; Refractory Chronic Lymphocy-tic Leukemia (CLL); Relapsed Chronic Lymphocy-tic Leukemia (CLL); T-Cell Leukemia | Cells Expressing Leukocyte Antigen CD37 (Tetraspanin 26 or CD37) | Phase I |
| ASG-15ME | Metastatic Transitional (Urothelial) Tract Cancer | Cells Expressing SLIT And NTRK Like Protein 6 (SLITRK6) | Phase I |
| vandortuzumab vedotin | Metastatic Hormone Refractory (Castration Resistant, Androgen-Indepen-dent) Prostate Cancer | Cells Expressing Metalloreductase STEAP1 (Six Transmembrane Epithelial Antigen Of The Prostate 1 or STEAP1 or EC 1.16.1.) | Phase I |
| CDX-014 | Ovarian Cancer | Cells Expressing Hepatitis A Virus Cellular Receptor 1 (Kidney Injury Molecule 1 or T Cell Immunoglobulin And Mucin Domain Containing Protein 1 or T-Cell Immunoglobulin Mucin Receptor 1 or T Cell Membrane Protein 1 or CD365 or HAVCR1) | Phase I |
| AGS-16M18 | Liver Cancer; Renal Cell Carcinoma | | Phase I |
| vorsetuzumab mafodotin | Non-Hodgkin Lymphoma; Renal Cell Carcinoma | Cells Expressing CD70 Antigen (CD27 Ligand or Tumor Necrosis Factor Ligand Superfamily Member 7 or CD70) | Phase I |
| denintuzumab mafodotin | Acute Lymphocy-tic Leukemia (ALL, Acute Lympho-blastic Leukemia); B-Cell Non-Hodgkin Lymphoma; Burkitt Lymphoma; Lympho-blastic Lymphoma; Mantle Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I |
| SGN-CD70A | Diffuse Large B-Cell Lymphoma; Follicular Lymphoma; Mantle Cell Lymphoma; Metastatic Renal Cell Carcinoma; Non-Hodgkin Lymphoma | Cells Expressing CD70 Antigen (CD27 Ligand or Tumor Necrosis Factor Ligand Superfamily Member 7 or CD70) | Phase I |
| RG-7636 | Metastatic Melanoma | Endothelin B Receptor (Endothelin Receptor Non Selective Type or EDNRB) | Phase I |
| SC-006 | Metastatic Colorectal Cancer | | Phase I |
| MM-310 | Breast Cancer; Endome-trial Cancer; Esophageal Cancer; Gastric Cancer; Gastroeso-phageal (GE) Junction Carcino-mas; Head And Neck Cancer Squamous Cell Carcinoma; Non-Small Cell Lung Cancer; Ovarian Cancer; Pancreatic Ductal Adenocar-cinoma; Prostate Cancer; Small-Cell Lung Cancer; Soft Tissue Sarcoma; Solid Tumor; Transitional Cell Carcinoma (Urothelial Cell Carcinoma) | Ephrin Type A Receptor 2 (Epithelial Cell Kinase or Tyrosine Protein Kinase Receptor ECK or EPHA2 or EC 2.7.10.1) | Phase I |
| PF-06647263 | Metastatic Breast Cancer; Ovarian Cancer | Cells Expressing Ephrin A4 (EPH Related Receptor Tyrosine Kinase Ligand 4 or EFNA4) | Phase I |
| PF-06263507 | Solid Tumor | Cells Expressing Trophoblast Glycoprotein (M6P1 or 5T4 Oncofetal Antigen or 5T4 Oncofetal Trophoblast Glycoprotein or Wnt Activated Inhibitory Factor 1 or TPBG) | Phase I |
| PF-06650808 | Metastatic Breast Cancer; Non-Small Cell Lung Cancer; Ovarian Cancer | Cells Expressing Neurogenic Locus Notch Homolog Protein 3 (NOTCH3) | Phase I |
| XMT-1522 | Breast Cancer; Gastric Cancer; Non-Small Cell Lung Cancer | Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1); Tubulin | Phase I |
| AMG-595 | Anaplastic Astrocyto-ma; Recurrent Glioblasto-ma Multiforme (GBM) | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I |
| pinatuzumab vedotin | Chronic Lymphocytic Leukemia (CLL) | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase I |
| cantuzumab ravtansine | Colorectal Cancer; Non-Small Cell Lung Cancer; Pancreatic Cancer; Solid Tumor | | Phase I |
| AVE-9633 | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase I |
| **BIWI-1⁽¹⁾** | Breast Cancer; Carcino-mas; Esophageal Cancer; Head And Neck Cancer Squamous Cell Carcinoma | Cells Expressing CD44 Antigen (CDw44 or Epican or Extracellular Matrix Receptor III or GP90 Lymphocyte Homing/Adhesion Receptor or HUTCH I or Heparan Sulfate Proteoglycan or Hermes Antigen or Hyaluronate Receptor or Phagocytic Glycoprotein 1 or CD44) | Phase I |
| RG-7882 | Epithelial Ovarian Cancer; Fallopian Tube Cancer; Pancreatic Cancer; Peritoneal Cancer | Cells Expressing Mucin 16 (Ovarian Cancer Related Tumor Marker CA125 or Ovarian Carcinoma Antigen CA125 or MUC16) | Phase I |
| ASG-5ME | Adenocar-cinoma; Hormone Refractory (Castration Resistant, Androgen-Indepen-dent) Prostate Cancer; Metastatic Adenocar-cinoma of The Pancreas | Cells Expressing Choline Transporter Like Protein 4 (Solute Carrier Family 44 Member 4 or SLC44A4) | Phase I |
| DCDS-0780A | B-Cell Non-Hodgkin Lymphoma | | Phase I |
| SC-004 | Endome-trial Cancer; Epithelial Ovarian Cancer; Fallopian Tube Cancer; Peritoneal Cancer | | Phase I |
| RG-7600 | Ovarian Cancer; Pancreatic Ductal Adenocar-cinoma | | Phase I |
| sofituzumab vedotin | Epithelial Ovarian Cancer; Fallopian Tube Cancer; Ovarian Cancer; Pancreatic Cancer; Peritoneal Cancer | Cells Expressing Mucin 16 (Ovarian Cancer Related Tumor Marker CA125 or Ovarian Carcinoma Antigen CA125 or MUC16) | Phase I |
| IMGN-289 | Breast Cancer; Esophageal Cancer; Gastric Cancer; Head And Neck Cancer Squamous Cell Carcinoma; Non-Small Cell Lung Cancer; Solid Tumor | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I |
| SAR-428926 | Breast Cancer; Colorectal Cancer; Gastric Cancer; Non-Small Cell Lung Cancer; Ovarian Cancer; Prostate Cancer; Solid Tumor | Cells Expressing Lysosome Associated Membrane Glycoprotein 1 (CD107 Antigen Like Family Member A or CD107a or LAMP1) | Phase I |
| SGNCD-19B | B-Cell Non-Hodgkin Lymphoma; Diffuse Large B-Cell Lymphoma; Follicular Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I |
| SGNCD-123A | Refractory Acute Myeloid Leukemia; Relapsed Acute Myeloid Leukemia | Cells Expressing Interleukin 3 Receptor Subunit Alpha (CD123 or IL3RA) | Phase I |
| SGNCD-352A | Refractory Multiple Myeloma; Relapsed Multiple Myeloma | Cells Expressing SLAM Family Member 6 (Activating NK Receptor or NK T B Antigen or CD352 or SLAMF6) | Phase I |
| RG-7841 | Breast Cancer; Non-Small Cell Lung Cancer; Solid Tumor | Cells Expressing Lymphocyte Antigen 6E (Retinoic Acid Induced Gene E Protein or Stem Cell Antigen 2 or Thymic Shared Antigen 1 or LY6E) | Phase I |
| IMGN-388 | Solid Tumor | Cells Expressing Integrin Alpha V (Vitronectin Receptor Subunit Alpha or CD51 or ITGAV) | Phase I |
| lorvotuzumab mertansine | Refractory Multiple Myeloma; Relapsed Multiple Myeloma | Cells Expressing Neural Cell Adhesion Molecule 1 (Antigen Recognized By Monoclonal Antibody 5.1H11 or CD56 or NCAM1) | Phase I |
| lorvotuzumab mertansine | Neuroendo-crine Carcinoma; Neuroendo-crine Tumors; Non-Small Cell Lung Cancer; Ovarian Cancer; Skin Cancer | Cells Expressing Neural Cell Adhesion Molecule 1 (Antigen Recognized By Monoclonal Antibody 5.1H11 or CD56 or NCAM1) | Phase I |
| BAY-794620 | Lung Cancer; Solid Tumor | Cells Expressing Carbonic Anhydrase 9 (Carbonate Dehydratase IX or pMW1 or Membrane Antigen MN or P54/58N or Renal Cell Carcinoma Associated Antigen G250 or CA9 or EC 4.2.1.1) | Phase I |
| RG-7598 | Refractory Multiple Myeloma; Relapsed Multiple Myeloma | | Phase I |
| Oncolysin B | B-Cell Leukemia; Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I |
| **ADCT-502⁽¹⁾** | Bladder Cancer; Breast Cancer; Esophageal Cancer; Gastric Cancer; Non-Small Cell Lung Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Phase I |
| AMG-172 | Renal Cell Carcinoma | Cells Expressing CD70 Antigen (CD27 Ligand or Tumor Necrosis Factor Ligand Superfamily Member 7 or CD70) | Phase I |
| ImmuRAIT-LL2 | B-Cell Non-Hodgkin Lymphoma | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase I/II |
| indusatumab vedotin | Adenocar-cinoma Of The Gastroe-sophageal Junction; Gastric Cancer | Cells Expressing Heat Stable Enterotoxin Receptor (Guanylyl Cyclase C or or Intestinal Guanylate Cyclase or GUCY2C or EC 4.6.1.2) | Phase I/II |
| clivatuzumab tetraxetan | Pancreatic Cancer | Cells Expressing Mucin 1 (Breast Carcinoma Associated Antigen DF3 or Episialin or H23AG or Krebs Von Den Lungen 6 or PEMT or Peanut Reactive Urinary Mucin or Polymorphic Epithelial Mucin or Tumor Associated Epithelial Membrane Antigen or Tumor Associated Mucin or CD227 or MUC1) | Phase I/II |
| **depatuxizumab mafodotin⁽²⁾** | Recurrent Malignant Glioma | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase I/II |
| CDX-014 | Metastatic Renal Cell Carcinoma; Papillary Renal Cell Carcinoma | Cells Expressing Hepatitis A Virus Cellular Receptor 1 (Kidney Injury Molecule 1 or T Cell Immunoglobulin And Mucin Domain Containing Protein 1 or T-Cell Immunoglobulin Mucin Receptor 1 or T Cell Membrane Protein 1 or CD365 or HAVCR1) | Phase I/II |
| **vadastuximab talirine⁽¹⁾** | Refractory Acute Myeloid Leukemia; Relapsed Acute Myeloid Leukemia | Cells Expressing Myeloid Cell Surface Antigen CD33 (Sialic Acid Binding Ig Like Lectin 3 or gp67 or CD33) | Phase I/II |
| vadastuximab talirine | Myelodys-plastic Syndrome | Cells Expressing Myeloid Cell Surface Antigen CD33 (Sialic Acid Binding Ig Like Lectin 3 or gp67 or CD33) | Phase I/II |
| MLN-2704 | Metastatic Hormone Refractory (Castration Resistant, Androgen-Indepen-dent) Prostate Cancer | Cells Expressing Glutamate Carboxypeptidase 2 (Folate Hydrolase 1 or Prostate Specific Membrane Antigen or PSMA or Pteroylpoly Gamma Glutamate Carboxypeptidase or Cell Growth Inhibiting Gene 27 Protein or FOLH1 or EC 3.4.17.21) | Phase I/II |
| Oncolysin B | AIDS - Related Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase I/II |
| coltuximab ravtansine | Diffuse Large B-Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II |
| coltuximab ravtansine | Acute Lymphocy-tic Leukemia (ALL, Acute Lympho-blastic Leukemia) | Cells Expressing B Lymphocyte Antigen CD19 (B Lymphocyte Surface Antigen B4 or Differentiation Antigen CD19 or T Cell Surface Antigen Leu 12 or CD19) | Phase II |
| coltuximab ravtansine | Diffuse Large B-Cell Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II |
| **indusatumab vedotin⁽²⁾** | Adenocar-cinoma Of The Gastroe-sophageal Junction; Gastric Cancer; Metastatic Adenocar-cinoma of The Pancreas | Cells Expressing Heat Stable Enterotoxin Receptor (Guanylyl Cyclase C or or Intestinal Guanylate Cyclase or GUCY2C or EC 4.6.1.2) | Phase II |
| depatuxizumab mafodotin | Squamous Non-Small Cell Lung Cancer | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase II |
| **depatuxizumab mafodotin⁽²⁾** | Anaplastic Astrocyto-ma; Anaplastic Oligoastro-cytoma; Gliosar-coma; High-Grade Glioma; Oligodendroglioma; Pediatric Diffuse Intrinsic Pontine Glioma; Recurrent Glioblastoma Multiforme (GBM) | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase II |
| lifastuzumab vedotin | Non-Small Cell Lung Cancer | Sodium Dependent Phosphate Transport Protein 2B (Sodium Phosphate Transport Protein 2B or NaPi3b or Sodium/Phosphate Cotransporter 2B or NaPi 2b or Solute Carrier Family 34 Member 2 or SLC34A2) | Phase II |
| lifastuzumab vedotin | Ovarian Cancer | Sodium Dependent Phosphate Transport Protein 2B (Sodium Phosphate Transport Protein 2B or NaPi3b or Sodium/Phosphate Cotransporter 2B or NaPi 2b or Solute Carrier Family 34 Member 2 or SLC34A2) | Phase II |
| Bismab-A | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase II |
| denintuzumab mafodotin | Diffuse Large B-Cell Lymphoma; Follicular Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase II |
| **Avicidin⁽¹⁾** | Colorectal Cancer; Prostate Cancer | Cells Expressing Epithelial Cell Adhesion Molecule (Adenocarcinoma Associated Antigen or Cell Surface Glycoprotein Trop 1 or Epithelial Cell Surface Antigen or Epithelial Glycoprotein 314 or KS 1/4 Antigen or KSA or Tumor Associated Calcium Signal Transducer 1 or CD326 or EPCAM) | Phase II |
| pinatuzumab vedotin | Diffuse Large B-Cell Lymphoma; Follicular Lymphoma | Cells Expressing B Cell Receptor CD22 (B Lymphocyte Cell Adhesion Molecule or Sialic Acid Binding Ig Like Lectin 2 or T Cell Surface Antigen Leu 14 or CD22) | Phase II |
| SGN-15 | Metastatic Breast Cancer; Non-Small Cell Lung Cancer; Ovarian Cancer; Prostate Cancer | Cells Expressing Lewis Y Antigen (CD174) | Phase II |
| cantuzumab ravtansine | Gastric Cancer; Gastroe-sophageal (GE) Junction Carcino-mas | | Phase II |
| ASP-6183 | Ovarian Cancer | | Phase II |
| SAR-566658 | Metastatic Breast Cancer | Cells Expressing Sialoglycotope CA6 Antigen | Phase II |
| Oncolysin S | Small-Cell Lung Cancer | Cells Expressing Neural Cell Adhesion Molecule 1 (Antigen Recognized By Monoclonal Antibody 5.1H11 or CD56 or NCAM1) | Phase II |
| lorvotuzumab mertansine | Small-Cell Lung Cancer | Cells Expressing Neural Cell Adhesion Molecule 1 (Antigen Recognized By Monoclonal Antibody 5.1H11 or CD56 or NCAM1) | Phase II |
| glembatumumab vedotin | Metastatic Melanoma; Metastatic Uveal Melanoma; Osteosar-coma; Squamous Non-Small Cell Lung Cancer | Cells Expressing Transmembrane Glycoprotein NMB (Transmembrane Glycoprotein HGFIN or GPNMB) | Phase II |
| MM-302 | Metastatic Breast Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Phase II/III |
| Neuradiab | Brain Cancer; Glioblasto-ma Multiforme (GBM) | Cells Expressing Tenascin (Cytotactin or GMEM or GP 150-225 or Glioma Associated Extracellular Matrix Antigen or Hexabrachion or JI or Myotendinous Antigen or Neuronectin or Tenascin C or TNC) | Phase III |
| clivatuzumab tetraxetan | Metastatic Adenocar-cinoma of The Pancreas | Cells Expressing Mucin 1 (Breast Carcinoma Associated Antigen DF3 or Episialin or H23AG or Krebs Von Den Lungen 6 or PEMT or Peanut Reactive Urinary Mucin or Polymorphic Epithelial Mucin or Tumor Associated Epithelial Membrane Antigen or Tumor Associated Mucin or CD227 or MUC1) | Phase III |
| **depatuxizumab mafodotin⁽²⁾** | Glioblasto-ma Multiforme (GBM) | Epidermal Growth Factor Receptor (Proto Oncogene c ErbB 1 or Receptor Tyrosine Protein Kinase erbB 1 or HER1 or ERBB1 or EGFR or EC 2.7.10.1) | Phase III |
| **vadastuximab talirine⁽¹⁾** | Acute Myelocytic Leukemia (AML, Acute Myeloblas-tic Leukemia) | Cells Expressing Myeloid Cell Surface Antigen CD33 (Sialic Acid Binding Ig Like Lectin 3 or gp67 or CD33) | Phase III |
| **glembatumuma b vedotin⁽²⁾** | Metastatic Breast Cancer | Cells Expressing Transmembrane Glycoprotein NMB (Transmembrane Glycoprotein HGFIN or GPNMB) | Phase III |
| Oncolysin B | B-Cell Leukemia; Lymphoma | Cells Expressing B Lymphocyte Antigen CD19 (B Lymphocyte Surface Antigen B4 or Differentiation Antigen CD19 or T Cell Surface Antigen Leu 12 or CD19) | Phase III |
| ImmuRAIT-LL2 | B-Cell Leukemia | Cells Expressing B Cell Receptor CD22 (B Lymphocyte Cell Adhesion Molecule or Sialic Acid Binding Ig Like Lectin 2 or T Cell Surface Antigen Leu 14 or CD22) | Preclinical |
| indusatumab vedotin | Metastatic Colorectal Cancer | Cells Expressing Heat Stable Enterotoxin Receptor (Guanylyl Cyclase C or or Intestinal Guanylate Cyclase or GUCY2C or EC 4.6.1.2) | Preclinical |
| ASG-15ME | Lung Cancer | Cells Expressing SLIT And NTRK Like Protein 6 (SLITRK6) | Preclinical |
| HTI-1511 | Bile Duct Cancer (Cholangiocarcinoma) ; Breast Cancer; Colorectal Cancer; Non-Small Cell Lung Cancer | Cells Expressing Epidermal Growth Factor Receptor (Proto Oncogene c ErbB 1 or Receptor Tyrosine Protein Kinase erbB 1 or HER1 or ERBB1 or EGFR or EC 2.7.10.1) | Preclinical |
| ZW-33 | Gastric Cancer; Metastatic Breast Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Preclinical |
| ZW-33 | Ovarian Cancer | Cells Expressing Receptor Tyrosine Protein Kinase ERBB 2 (Metastatic Lymph Node Gene 19 Protein or Proto Oncogene Neu or Proto Oncogene C ErbB 2 or Tyrosine Kinase Type Cell Surface Receptor HER2 or p185erbB2 or HER2 or CD340 or ERBB2 or EC 2.7.10.1) | Preclinical |
| SGNCD-352A | Non-Hodgkin Lymphoma | Cells Expressing SLAM Family Member 6 (Activating NK Receptor or NK T B Antigen or CD352 or SLAMF6) | Preclinical |
| HuMax-CD74-ADC | Oncology | Cells Expressing HLA Class II Histocompatibility Antigen Gamma Chain (HLA DR Antigens Associated Invariant Chain or Ia Antigen Associated Invariant Chain or p33 or CD74) | Preclinical |
| sacituzumab govitecan | Pancreatic Ductal Adenocar-cinoma | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Adenocar-cinoma; Cervical Cancer; Colorectal Cancer; Endome-trial Cancer; Epithelial Ovarian Cancer; Esophageal Cancer; Follicular Thyroid Cancer; Gastric Cancer; Glioblasto-ma Multiforme (GBM); Head And Neck Cancer Squamous Cell Carcinoma; Hepato-cellular Carcinoma; Kidney Cancer (Renal Cell Cancer); Metastatic Hormone Refractory (Castration Resistant, Androgen-Indepen-dent) Prostate Cancer; Metastatic Transitional (Urothelial) Tract Cancer; Transitional Cell Cancer (Urothelial Cell Cancer) | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Hepato-cellular Carcinoma | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Metastatic Breast Cancer; Transitional Cell Cancer (Urothelial Cell Cancer) | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Non-Small Cell Lung Cancer; Small-Cell Lung Cancer | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| sacituzumab govitecan | Metastatic Breast Cancer | Cells Expressing Tumor Associated Calcium Signal Transducer 2 (Cell Surface Glycoprotein Trop 2 or Membrane Component Chromosome 1 Surface Marker 1 or Pancreatic Carcinoma Marker Protein GA733-1 or TACSTD2) | |
| (1) Discontinued due to adverse events | | | |
| (2) Discontinued due to lack of efficacy | | | |

**TABLE A3 - ADCs that reached phase III clinical development**

| **Drug Name** | **Indication** | **Development Stage** | **Last Development Stage** | **Reason for Discontinuation** |
|---|---|---|---|---|
| trastuzumab emtansine | Gastric Cancer | Marketed | Phase II/III | Unspecified |
| MM-302 | Metastatic Breast Cancer | Discontinued | Phase II/III | Business/Strategic Decision |
| trastuzumab emtansine | Metastatic Breast Cancer | Marketed | Phase III | Unspecified |
| trastuzumab emtansine | Gastric Cancer | Marketed | Phase III | Unspecified |
| ibritumomab tiuxetan | Diffuse Large B-Cell Lymphoma | Marketed | Phase III | |
| inotuzumab ozogamicin | Follicular Lymphoma | Marketed | Phase III | |
| inotuzumab ozogamicin | Diffuse Large B-Cell Lymphoma; Non-Hodgkin Lymphoma | Marketed | Phase III | Lack of Efficacy |
| rovalpituzumab tesirine | Small-Cell Lung Cancer | Phase III | Phase III | |
| rovalpituzumab tesirine | Small-Cell Lung Cancer | Phase III | Phase III | |
| Neuradiab | Brain Cancer; Glioblastoma Multiforme (GBM) | Inactive | Phase III | Unspecified |
| clivatuzumab tetraxetan | Metastatic Adenocarcinoma of The Pancreas | Inactive | Phase III | Unspecified |
| depatuxizumab mafodotin | Glioblastoma Multiforme (GBM) | Inactive | Phase III | Lack of Efficacy |
| vadastuximab talirine | Acute Myelocytic Leukemia (AML, Acute Myeloblastic Leukemia) | Discontinued | Phase III | Adverse Events |
| glembatumumab vedotin | Metastatic Breast Cancer | Discontinued | Phase III | Lack of Efficacy |
| Oncolysin B | B-Cell Leukemia; Lymphoma | Discontinued | Phase III | Business/Strategic Decision |

| **TABLE A4.** Tumor-specific cell-surface receptor targets which can be targeted by immunoglobulins according to the invention, and antibodies that can be used for the ADCs and the antibodies provided with a saponin selected from the group consisting of: SO1861, SA1641, GE1741, QS-21, QS-21A, QS21-B, Quil-A, SO1832, SO1904, and SO1862, and the ADCs provided with a saponin selected from the group consisting of: SO1861, SA1641, GE1741, QS-21, QS-21A, QS21-B, Quil-A, SO1832, SO1904, and SO1862, of the present invention (not presented as a limitation; further immunoglobulins are equally suitable for the invention) | |
|---|---|
| **Target cell-surface receptor** | **Example monoclonal antibodies** |
| HER2 | anti-HER2 monoclonal antibody such as trastuzumab and pertuzumab |
| CD20 | anti-CD20 monoclonal antibody such as rituximab, ofatumumab, tositumomab and ibritumomab |
| CA125 | anti-CA125 monoclonal antibody such as oregovomab |
| EpCAM (17-1A) | anti-EpCAM (17-1A) monoclonal antibody such as edrecolomab |
| EGFR | anti-EGFR monoclonal antibody such as cetuximab, panitumumab and nimotuzumab |
| CD30 | anti-CD30 monoclonal antibody such brentuximab |
| CD33 | anti-CD33 monoclonal antibody such as gemtuzumab and huMy9-6 |
| vascular integrin alpha-v beta-3 | anti-vascular integrin alpha-v beta-3 monoclonal antibody such as etaracizumab |
| CD52 | anti-CD52 monoclonal antibody such as alemtuzumab |
| CD22 | anti-CD22 monoclonal antibody such as epratuzumab |
| CEA | anti-CEA monoclonal antibody such as labetuzumab |
| CD44v6 | anti-CD44v6 monoclonal antibody such as bivatuzumab |
| FAP | anti- FAP monoclonal antibody such as sibrotuzumab |
| CD19 | anti-CD19 monoclonal antibody such as huB4 |
| CanAg | anti-CanAg monoclonal antibody such as huC242 |
| CD56 | anti-CD56 monoclonal antibody such huN901 |
| CD38 | anti-CD38 monoclonal antibody such as daratumumab |
| CA6 | anti-CA6 monoclonal antibody such as DS6 |
| IGF-IR | anti-IGF-IR monoclonal antibody such as cixutumumab and 3B7 |
| integrin | anti-integrin monoclonal antibody such as CNTO 95 |
| syndecan-1 | anti-syndecan-1 monoclonal antibody such as B-B4 |

**Table A5: RIPs from plants***

| **Plant Family** | **Plant Species** | **Proteins** | **Classification** |
|---|---|---|---|
| Adoxaceae | Sambucus ebulus L. | Ebulitin α, Ebulitin β, Ebulitin γ | RIP 1 |
| | | Ebulin f, Ebulin I, Ebulin r1, Ebulin r2, SEA | RIP 2 |
| | | SEAII, SELfd, SELId, SELIm | lectin |
| | Sambucus nigra L. | α-Nigritin, β-Nigritin, γ-Nigritin, Nigritin f1, Nigritin f2 | RIP 1 |
| | | basic Nigrin b, Nigrin b = SNA-V, Nigrin f = SNA-Vf, Nigrin I1, Nigrin I2, Nigrin s, SNA-I, SNA-I', SNA-If, SNAflu-I, SNLRP1, SNLRP2 | RIP 2 |
| | | SNA-Id, SNA-Im, SNA-II, SNA-III, SNA-IV = SNA-IVf, SNA-IVI, SNApol-I, SNApol-II, TrSNA-I, TrSNA-If | lectin |
| | Sambucus racemosa L. | basic racemosin b, SRA | RIP 2 |
| | | SRLbm = SRAbm | lectin |
| | Sambucus sieboldiana (Miq.) Blume ex Graebn. | SSA = SSA-b-1, Sieboldin-b = SSA-b-2 | RIP 2 |
| | | SSA-b-3, SSA-b-4 | lectin |
| Aizoaceae | Mesembryanthe-mum crystallinum L. | RIP1 | RIP 1 |
| Amaranthaceae | Amaranthus caudatus L. | Amaranthin = ACA | lectin |
| | Amaranthus cruentus L. | ACL | lectin |
| | Amaranthus hypochondriacus L. [Syn.: Amaranthus leucocarpus S. Watson] | A. leucocarpus lectin | lectin |
| | Amaranthus mangostanus L. | Amaramangin | RIP 1 |
| | Amaranthus tricolor L. | AAP-27 | RIP 1 |
| | Amaranthus viridis L. | Amaranthin | RIP 1 |
| | Beta vulgaris L. | Beetin-27 = BE27, Beetin-29 = BE29, Betavulgin | RIP 1 |
| | Celosia argentea L. [Syn.: Celosia cristata L.] | CCP-25, CCP-27 | RIP 1 |
| | Chenopodium album L. | CAP30 | RIP 1 |
| | Spinacia oleracea L. | SoRIP1 = BP31 | RIP 1 |
| | | SoRIP2 | RIP 1 candidate |
| Araliaceae | Aralia elata (Miq.) Seem. | Aralin | RIP 2 |
| | Panax ginseng C.A.Mey | Panaxagin | peculiar RIP 1 candidate/RNase |
| | Panax quinquefolius L. | Quinqueginsin | peculiar RIP 1 candidate/RNase |
| Asparagaceae | Asparagus officinalis L. | Asparin 1, Asparin 2 | RIP 1 |
| | Drimia maritima (L.) Stearn [Syn.: Charybdis maritima (L.) Speta] | Charybdin | RIP 1 |
| | Muscari armeniacum Leichtlin ex Baker | Musarmin 1, Musarmin 2, Musarmin 3, Musarmin 4 | RIP 1 |
| | Polygonatum multiflorum (L.) All. | PMRIPm, PMRIPt | RIP 2 |
| | Yucca gloriosa var. tristis Carrière [Syn.: Yucca recurvifolia Salisb.] | Yucca leaf protein = YLP | RIP 1 |
| Basellaceae | Basella rubra L. | Basella RIP 2a, Basella RIP 2b, Basella RIP 3 | RIP 1 |
| Caryophyllaceae | Agrostemma githago L. | Agrostin 2, Agrostin 5, Agrostin 6, Agrostin | RIP 1 |
| | Dianthus barbatus L. | Dianthin 29 | RIP 1 |
| | Dianthus caryophyllus L. | Dianthin 30, Dianthin 32 | RIP 1 |
| | Dianthus chinensis L. [Syn.: Dianthus sinensis Link] | D. sinensis RIP | RIP 1 |
| | Gypsophila elegans M.Bieb. | Gypsophilin | RIP 1 |
| | Silene chalcedonica (L.) E.H.L.Krause [Syn.: Lychnis chalcedonica L.] | Lychnin | RIP 1 |
| | Silene glaucifolia Lag. [Syn.: Petrocoptis glaucifolia (Lag.) Boiss.] | Petroglaucin 1, Petroglaucin 2 | RIP 1 |
| | Silene laxipruinosa Mayol & Rosselló [Syn.: Petrocoptis grandiflora Rothm.] | Petrograndin | RIP 1 |
| | Saponaria ocymoides L. | Ocymoidin | RIP 1 |
| | Saponaria officinalis L. | Saporin-L1 = SO-L1, Saporin-L2 = SO-L2, Saporin-L3 = SO-L3, Saporin-I = SO-I = SO-4, Saporin-R1 = SO-R1, Saporin-R2 = SO-R2, Saporin-R3 = SO-R3, SO3a, SO3b, Saporin-S5 = Saporin 5 = SO-S5, Saporin-S6 = Saporin 6 = SO-6 = SO-S6, Saporin-S8 = SO-S8, Saporin-S9 = Saporin 9 = SO-S9, SAP-C, SAP-S | RIP 1 |
| | Myosoton aquaticum (L.) Moench [Syn.: Stellaria aquatica (L.) Scop.] | Stellarin | RIP 1 |
| | Stellaria media (L.) Vill. | RIP Q3 | RIP 1 |
| | Vaccaria hispanica (Mill.) Rauschert [Syn.: Vaccaria pyramidata Medik.] | Pyramidatin | RIP 1 |
| Cucurbitaceae | Benincasa hispida (Thunb.) Cogn. | Hispin | RIP 1 |
| | | α-benincasin, β-benincasin | sRIP 1 |
| | | Bryodin 1 = BD1, Bryodin 2, Bryodin-L, Bryodin-R | RIP 1 |
| | Bryonia cretica subsp. dioica (Jacq.) Tutin. [Syn.: Bryonia dioica L.] | BDA | lectin/ RIP 2 like |
| | Citrullus colocynthis (L.) Schrad. | Colocin 1, Colocin 2 | RIP 1 |
| | Cucurbita foetidissima Kunth | Foetidissimin | peculiar RIP 2 |
| | | Foetidissimin II | RIP 2 |
| | Cucumis ficifolius A.Rich. [Syn.: Cucumis figarei Delile ex Naudin] | Cucumis figarei RIP = CF-RIP | RIP 1 candidate |
| | Cucurbita maxima Duchesne | Cucurmoschin | sRIP 1 candidate |
| | Cucurbita moschata Duchesne [Syn.: Cucurbita moschata (Duchesne ex Lam.) Duchesne ex Poir.] | Cucurmosin, Cucurmosin 2, C. moschata RIP, Moschatin, PRIP 1, PRIP 2 | RIP 1 |
| | | α-moschin, β-moschin | sRIP 1 candidate |
| | Cucurbita pepo L. | Pepocin | RIP 1 |
| | Cucurbita pepo var. texana (Scheele) D.S.Decker [Syn.: Cucurbita texana (Scheele) A. Gray] | Texanin | RIP 1 |
| | Gynostemma pentaphyllum (Thunb.) Makino | Gynostemmin | RIP 1 |
| | Lagenaria siceraria (Molina) Standl. | Lagenin | RIP 1 candidate |
| | Luffa acutangula (L.) Roxb. | Luffaculin-1, Luffaculin-2 | RIP 1 |
| | | Luffangulin | sRIP 1 |
| | | Luffa acutangula fruit lectin | lectin |
| | Luffa cylindrica (L.) M.Roem [Syn.: Luffa aegyptiaca Mill.] | Luffin, Luffin-a, Luffin-b, α-luffin, β-luffin, LRIP | RIP 1 |
| | | Luffacylin, Luffin P1 | sRIP 1 |
| | | Luffin-S, LuffinS(1), LuffinS(2) = luffin S2, LuffinS(3) | sRIP 1 candidate |
| | Marah oreganus (Torr. & A. Gray) Howell | MOR-I, MOR-II | RIP 1 |
| | Momordica balsamina L. | Balsamin, MbRIP-1, Momordin II | RIP 1 |
| | Momordica charantia L. | MAP 30, α-momorcharin = α-MC = α-MMC, β-momorcharin = β-MC = β-MMC, δ-momorcharin = δ-MMC, Momordin, Momordin = Momordica charantia inhibitor, Momordin II, Momordin-a, Momordin-b | RIP 1 |
| | | γ-momorcharin = γ-MMC, Charantin | sRIP 1 |
| | | RIP 1 candidate | RIP 1 candidate |
| | | MCL = M. charantia lectin, anti-H Lectin, Momordica agglutinin, Momordin, protein fraction 1, protein fraction 2 | lectin |
| | | MCL = Momordica charantia seed lectin = Momordica charantia lectin, MCL1 | RIP 2 |
| | Momordica cochinchinensis Spreng. | Cochinin B, Momorcochin, Momorcochin-S | RIP 1 |
| | Siraitia grosvenorii (Swingle) C.Jeffrey ex A.M.Lu & Zhi Y.Zhang [Syn.: Momordica grosvenorii Swingle] | Momorgrosvin | RIP 1 |
| | Sechium edule (Jacq.) Sw. | Sechiumin | RIP 1 |
| | | Sechium edule fruit lectin | lectin |
| | Trichosanthes anguina L. | Trichoanguin | RIP 1 |
| | | SGSL | lectin/ RIP 2 like |
| | Trichosanthes cordata Roxb. | TCA-I, TCA-II | lectin |
| | Trichosanthes cucumerina L. | TCSL | lectin/ RIP 2 candidate |
| | Trichosanthes cucumeroides (Ser.) Maxim. | β-trichosanthin = β-TCS | RIP 1 |
| | Trichosanthes kirilowii Maxim. | α-kirilowin, β-kirilowin, TAP 29, TK-35, Trichobitacin, Trichokirin, Trichomislin = TCM, Trichosanthin = Trichosanthes antiviral protein = TAP = TCS = α-trichosanthin = α-TCS = GLQ223, Trichosanthin, β-trichosanthin = β-TCS, γ-trichosanthin = γ-TCS | RIP 1 |
| | | Trichokirin S1, S-Trichokirin, Trichosanthrip | sRIP 1 |
| | | TKL-1 = Trichosanthes kirilowii lectin-1 | lectin/ RIP 2 candidate |
| | | TK-I, TK-II, TK-III, Trichosanthes kirilowii lectin | lectin |
| | Trichosanthes kirilowii Maximovicz var. japonica (Miquel) Kitamura | Karasurin-A, Karasurin-B, Karasurin-C | RIP 1 |
| | Trichosanthes lepiniate | Trichomaglin | RIP 1 |
| | Trichosanthes dioica Roxb. | TDSL | lectin/ RIP 2 candidate |
| | Trichosanthes sp. Bac Kan 8-98 | Trichobakin | RIP 1 |
| Cupressaceae | Thuja occidentalis L. | Arborvitae RIP | RIP candidate |
| Euphorbiaceae | Croton tiglium L. | Crotin I | RIP 1 candidate |
| | | Crotin 2 | RIP 1 |
| | Euphorbia characias L. | E. characias lectin | lectin |
| | Suregada multiflora (A.Juss.) Baill. [Syn.: Gelonium multiflorum A.Juss.] | Gelonin = GAP 31 | RIP 1 |
| | Hura Crepitans L. | Hura crepitans RIP, Hura crepitans RIP-5 | RIP 1 |
| | | Hura crepitans latex lectin | RIP 2 |
| | | Crepitin, Hurin, Hura crepitans seed lectin | lectin |
| | Jatropha curcas L. | Curcin, Curcin 2, Curcin-L, Jc-SCRIP | RIP 1 |
| | Manihot palmata Müll. Arg. | Mapalmin | RIP 1 |
| | Manihot esculenta Crantz. [Syn.: Manihot utilissima Pohl] | Manutin 1, Manutin 2 | RIP 1 |
| | Ricinus communis L. | Ricin = crystalline Ricin = Ricin D, Ricin E, RCA = Ricinus communis agglutinin = RCAI = RCA120 = R. communis hemagglutinin = RCB-PHA I, RCAII = RCA60 = RCB-PHA II | RIP 2 |
| | Ricinus communis, USA | Ricin 1, Ricin 2, Ricin 3 | RIP 2 |
| | Ricinus communis, India | Ricin I, Ricin II, Ricin III | RIP 2 |
| | Ricinus sanguienus, France | Ricin₁₁, Ricin₁₂, Ricin₂ | RIP 2 |
| Fabaceae | Abrus precatorius L. | Abrin, Abrin-a = Abrin C = Abrin-III, Abrin-b, Abrin-c = Abrin A = Abrin-I, Abrin-d, Abrin-II, APA = Abrus precatorius agglutinin = Abrus lectin = AAG, APA-I, APA-II | RIP 2 |
| | Abrus pulchellus Thwaites | Pulchellin, Pulchellin PI, Pulchellin PII, Pulchellin PIII | RIP 2 |
| | Pisum sativum subsp. sativum L. [Syn.: Pisum sativum var. arvense (L.) Poir.] | α-pisavin, (β-pisavin | RIP 1 |
| | Pisum sativum var. macrocarpon | Sativin | RIP 1 candidate |
| Iridaceae | Iris hollandica var. Professor Blaauw | IrisRIP = IRIP, IrisRIP.A1, IrisRIP.A2, IrisRIP.A3 | RIP 1 |
| | | IRA, IRAb, IRAr | RIP 2 |
| Lamiaceae | Clerodendrum aculeatum (L.) Schltdl. | CA-SRI | RIP 1 candidate |
| | Clerodendrum inerme (L.) Gaertn. | CIP-29 | RIP 1 |
| | | CIP-34 | RIP 1 candidate |
| | Leonurus japonicus Houtt. | Leonurin | RIP candidate |
| Lauraceae | Cinnamomum bodinieri H. Lév | Bodinierin | RIP 2 |
| | Cinnamomum camphora (L.) J.Presl | Camphorin | RIP 1 |
| | | Cinnamomin, Cinnamomin 1, Cinnamomin 2, Cinnamomin 3 | RIP 2 |
| | | Cinphorin | sRIP 2 |
| | Cinnamomum parthenoxylon (Jack) Meisn. [Syn.: Cinnamomum porrectum (Roxb.) Kosterm.] | Porrectin | RIP 2 |
| Malvaceae | Abelmoschus esculentus (L.) Moench | Abelesculin | RIP 1 |
| Nyctaginaceae | Boerhaavia diffusa L. | Boerhaavia inhibitor | RIP 1 candidate |
| | Bougainvillea spectabilis Willd. | BAP I, Bouganin = Bougainvillea RIP I | RIP 1 |
| | Bougainvillea × buttiana cv. Enid Lancester | BBP-24, BBP-28 | RIP 1 |
| | Bougainvillea × buttiana cv. Mahara | BBAP1 | RIP 1 |
| | Mirabilis expansa (Ruiz & Pav.) Standl. | ME1, ME2 | RIP 1 |
| | Mirabilis jalapa L. | MAP, MAP-2, MAP-3, MAP-4, MAP-S | RIP 1 |
| Olacaceae | Malania oleifera Chun & S. K. Lee | Malanin | lectin/RIP 2 candidate |
| | Ximenia americana L. | Riproximin = Rpx, Rpx-I, Rpx-II | RIP 2 |
| Passifloraceae | Adenia digitata (Harv.) Engl. | Modeccin = Modeccin 4B, Modeccin 6B | RIP 2 |
| | Adenia ellenbeckii Harms | A. ellenbeckii lectin | RIP 2 candidate |
| | Adenia fruticosa Burtt Davy | A. fruticosa lectin | lectin |
| | Adenia glauca Schinz | A. glauca lectin | RIP 2 candidate |
| | Adenia goetzei Harms (unresolved name) | A. goetzei lectin | RIP 2 |
| | Adenia keramanthus Harms | A. keramanthus lectin | RIP 2 candidate |
| | Adenia lanceolata Engl. | Lanceolin | RIP 2 |
| | Adenia racemosa W. J. de Wilde | A. racemosa lectin | lectin |
| | Adenia spinosa Burtt Davy | A. spinosa lectin | RIP 2 candidate |
| | Adenia stenodactyla Harms | Stenodactylin | RIP 2 |
| | Adenia venenata Forssk. | A. venenata lectin | RIP 2 candidate |
| | Adenia volkensii Harms | Volkensin | RIP 2 |
| Phytolaccaceae | Phytolacca americana L. | α-PAP, PAP = Phytolacca americana protein = pokeweed antiviral protein, PAP-I, PAP-II, PAP-III, PAP-C, PAP-H, PAP-R, PAP-S, PAP-S1, PAP-S2 | RIP 1 |
| | Phytolacca dioica L. | Diocin 1, Diocin 2, PD-L1, PD-L2, PD-L3, PD-L4, PD-S1, PD-S2, PD-S3 | RIP 1 |
| | Phytolacca dodecandra L'Hér. | Dodecandrin, Dodecandrin C | RIP 1 |
| | Phytolacca heterotepala H. Walter | Heterotepalin 4, Heterotepalin 5b | RIP 1 |
| | Phytolacca insularis Nakai | Insularin = PIP = Phytolacca insularis antiviral protein, PIP2 = P. insularis antiviral protein 2 | RIP 1 |
| Poaceae | Hordeum vulgare L. | Barley toxin = Barley translation inhibitor = Barley Protein Synthesis Inhibitor= BPSI = RIP 30, Barley toxin I = Barley translation inhibitor I, Barley toxin II = Barley translation inhibitor II = Barley Protein Synthesis Inhibitor II = BPSI II, Barley toxin III = Barley translation inhibitor III, JIP60 | RIP 1 |
| | Oryza sativa L. | Oryza sativa RIP | RIP 1 |
| | Secale cereale L. | RPSI | RIP 1 |
| | Triticum aestivum L. | Tritin, Tritin 1, Tritin 2, Tritin 3, Tritin-S, Tritin-L | RIP 1 |
| | Zea mays L. | b-32 = maize RIP = maize proRIP1, Maize proRIP2 | RIP 3/peculiar RIP 1 |
| Ranunculaceae | Eranthis hyemalis (L.) Salisb. | EHL | RIP 2 |
| Santalaceae | Phoradendron californicum Nutt. | PCL | RIP 2 |
| | Viscum album L. (Himalayan mistletoe) | HmRip, HmRip 1, HmRip 2, HmRip 3, HmRip 4 | RIP 2 |
| | Viscum album L. (European mistletoe) | ML-I = Mistletoe lectin I = Viscumin = Eu-ML = EML-1 = VAA-I, ML-II = Mistletoe lectin II = VAA-II, ML-III = Mistletoe lectin III = VAA-III | RIP 2 |
| | Viscum articulatum Burm. f. | Articulatin-D | RIP 2 |
| | Viscum coloratum (Kom.) Nakai [Syn.: Viscum album subsp. coloratum Kom.] | KML, KML-C, KML-IIL, KML-IIU, VCA | RIP 2 |
| Solanaceae | Nicotiana tabacum L. | CIP31 | RIP-like protein |
| | | TRIP | RIP 1 candidate |
| Thymelaeaceae | Phaleria macrocarpa (Scheff.) Boerl. | P. macrocarpa RIP | RIP candidate |
| * Schrot J, Weng A, Melzig MF, et al. Ribosome-inactivating and related proteins. Toxins (Basel). 2015 May 8;7(5):1556-615. | | | |

It is part of the invention that the therapeutic combination, the first pharmaceutical composition, the first proteinaceous molecule, the second or third pharmaceutical composition or the second or third proteinaceous molecule of the invention is further combined with a covalent conjugate (complex) of a binding molecule or a binding moiety and a saponin, or is further combined with a pharmaceutical compound, an antibody, etc., therewith providing a composition comprising three or more enhancers, pharmaceutically active ingredients, etc., e.g. a conjugate of the invention (e.g. a first proteinaceous molecule and/or a second or third proteinaceous molecule) combined with a binding moiety complexed with an effector molecule, further combined with a pharmaceutical, which is either or not linked to a saponin, and which is either or not coupled to a ligand such as a targeting immunoglobulin, a domain or a fragment thereof. Furthermore, an embodiment is the therapeutic combination, the first pharmaceutical composition, the first proteinaceous molecule, the second or third pharmaceutical composition or the secondor third proteinaceous molecule of the invention, wherein the second or third proteinaceous molecule is provided with two or more effector moieties such as a toxin or immunotoxin, wherein the two or more effector moieties are the same or different.

### Exemplary embodiments

Provided and not claimed is the endosomal escape enhancing conjugate, wherein the saponin is a bisdesmosidic triterpene saponin belonging to the type of a 12,13-dehydrooleanane with an aldehyde function, in position 23, and wherein the saponin is preferably a saponin that can be isolated from *Gypsophila* or *Saponaria* species, more preferably the saponin is the saponin SO1861 or any of its diastereomers.

An embodiment is the endosomal escape enhancing conjugate of the invention, wherein the binding site is at least a ligand, such as an immunoglobulin, with at least an effector moiety bound thereto.

An embodiment is the endosomal escape enhancing conjugate of the invention, wherein the binding site is an immunoglobulin or at least a binding domain thereof (not claimed) for binding to a cell surface molecule, wherein preferably the cell surface molecule is selected from any of HER2, EGFR, CD20, CD22, Folate receptor 1, CD146, CD56, CD19, CD138, CD27L, PSMA, CanAg, integrin-alphaV, CA6, CD33, mesothelin, Cripto, CD3, CD30, CD33, CD239, CD70, CD123, CD352, DLL3, CD25, ephrinA4, MUC1, Trop2, CEACAM5, HER3, CD74, PTK7, Notch3, FGF2, C4.4A, FLT3, CD71.

An embodiment is the endosomal escape enhancing conjugate of the invention, wherein a linker is coupled to the glycoside via a cleavable bond, and wherein the ligand is an immunoglobulin, wherein preferably said cleavable bond is subject to cleavage under acidic, reductive, enzymatic or light-induced conditions, and preferably the cleavable bond is a covalent bond, preferably an imine bond, a hydrazone bond, an oxime bond, a 1,3-dioxolane bond or an ester bond, wherein preferably the cleavable bond is a disulfide bond or a peptide bond.

An embodiment is the endosomal escape enhancing conjugate of the invention, wherein the saponin moiety is a terminal saponin, preferably the saponin SO1861, the linker is a chemical linker covalently linking the saponin to the binding site of the first proteinaceous molecule, and the same first binding site of the third and first proteinaceous molecule is an immunoglobulin such as trastuzumab or cetuximab, the linker preferably providing a cleavable bond between the terminal saponin moiety and the first binding site comprised by the first and third proteinaceous molecule.

An embodiment is the combination of an endosomal escape enhancing conjugate (i.e. the first proteinaceous molecule) according to the invention and a binding moiety (i.e. the second or third proteinaceous molecule), wherein the binding moiety comprises at least one effector moiety, wherein the endosomal escape enhancing conjugate and the binding moiety are, independently from one another, able to specifically bind to a target cell-specific surface molecule or structure, thereby inducing receptor-mediated endocytosis of a complex of the endosomal escape enhancing conjugate and the target cell-specific surface molecule, and of the complex of the binding moiety and the target cell-specific surface molecule.

An embodiment is the combination according to the invention, wherein the endosomal escape enhancing conjugate and the binding moiety are able to specifically bind to the same target cell-specific surface molecule or structure, when the binding moiety is the third proteinaceous molecule.

An embodiment is the combination according to the invention, wherein the endosomal escape enhancing conjugate is able to compete with the binding moiety for binding to the target cell-specific surface molecule or structure, when the binding moiety is the third proteinaceous molecule.

An embodiment is the combination according to the invention, wherein the endosomal escape enhancing conjugate and the binding moiety are, independently from one another, able to specifically bind to the same epitope.

An embodiment is the combination according to the invention, wherein the endosomal escape enhancing conjugate is able to specifically bind to a first epitope, which is the same as the first epitope to which the binding moiety is able to specifically bind, when the binding moiety is the third proteinaceous molecule.

An embodiment is the combination according to the invention, wherein the endosomal escape enhancing conjugate and the binding moiety are able to specifically bind to different target cell-specific surface molecules or structures, when the binding moiety is the second proteinaceous molecule.

An embodiment is the combination according to the invention, wherein the target cell-specific surface molecule or structure is selected from HER2, EGFR, CD20, CD22, Folate receptor 1, CD146, CD56, CD19, CD138, CD27L, PSMA, CanAg, integrin-alphaV, CA6, CD33, mesothelin, Cripto, CD3, CD30, CD33, CD239, CD70, CD123, CD352, DLL3, CD25, ephrinA4, MUC1, Trop2, CEACAM5, HER3, CD74, PTK7, Notch3, FGF2, C4.4A, FLT3, CD71.

Provided but not claimed is the combination, wherein the glycoside molecule is a bisdesmosidic triterpene, preferably a saponin.
Provided but not claimed is the combination, wherein the glycoside molecule is a bisdesmosidic triterpene saponin.
Provided but not claimed is the combination, wherein the saponin is a bisdesmosidic triterpene saponin belonging to the type of a 12,13-dehydrooleanane with an aldehyde function in position 23.
Provided but not claimed is the combination, wherein the saponin is a saponin that can be isolated from *Gypsophila* or *Saponaria* species.
An embodiment is the combination according to the invention, wherein the saponin is a SO1861 or any of its diastereomers.

An embodiment is the combination according to the invention, wherein the at least one glycoside selected from the group consisting of: SO1861, SA1641, GE1741, QS-21, QS-21A, QS21-B, Quil-A, SO1832, SO1904, and SO1862 is bound to the ligand (binding site for the epitope on the cell-surface molecule) via a cleavable bond, wherein preferably said cleavable bond is subject to cleavage under acidic, reductive, enzymatic or light-induced conditions, and wherein the cleavable bond preferably is a disulfide bond or a peptide bond.

An embodiment is the combination according to the invention, wherein the cleavable bond is a covalent bond, preferably an imine bond, a hydrazone bond, an oxime bond, a 1,3-dioxolane bond or an ester bond.

An embodiment is the combination according to the invention, wherein the endosomal escape enhancing conjugate comprises a defined number of glycosides or a defined range.

An embodiment is the combination according to the invention, wherein the defined range is between 1 - 30 glycoside(s), preferably between 1 - 20, more preferably between 1 - 10, more preferably between 1 - 6, more preferably between 2-6, more preferably between 2-5, more preferably between 3-5, more preferably between 3-4 glycosides.

An embodiment is the combination according to the invention, wherein the effector moiety is a pharmaceutically active substance, such as a toxin such as a proteinaceous toxin, a drug, a polypeptide or a polynucleotide.

An embodiment is the combination according to the invention, wherein the target cell is a diseased cell or a disease-related cell, preferably a tumor cell or a tumor-associated cell (e.g. tumor vascular cell), or an immune cell (e.g. a T regulatory cell), or an autoimmune cell.

Provided and not claimed is the combination according to the invention, wherein the at least one effector moiety is bound to the binding moiety (second or third proteinaceous molecule) via a cleavable bond, wherein preferably said cleavable bond is subject to cleavage under acidic, reductive, enzymatic or light-induced conditions, and/or wherein the cleavable bond is a disulfide bond or a peptide bond.

Provided and not claimed is the combination according to the invention, wherein the glycoside (saponin) is capable of augmenting endosomal escape of the effector molecule.

An embodiment is the combination according to the invention, for use as a medicament.

An embodiment is the pharmaceutical composition comprising a combination according to the invention (previous embodiments) and a pharmaceutically acceptable excipient.

An embodiment is the pharmaceutical composition according to the invention, further comprising at least one further active pharmaceutically ingredient, such as a further immunoglobulin.

An embodiment is the combination for use according to the invention, or pharmaceutical composition according to the invention, for use in a method of treating cancer or an autoimmune disease.

Provided and not claimed is the combination for use according to the invention, wherein the endosomal escape enhancing conjugate (first proteinaceous molecule) and the binding moiety (second or third proteinaceous molecule) are to be administered concomitant or sequentially, preferably concomitant.

Provided and not claimed is a method of treating cancer, the method comprising administering a combination according to the invention to a patient in need thereof.

Provided and not claimed is the method of treating cancer, the method comprising administering a pharmaceutical composition according to the invention, to a patient in need thereof.

Provided but not claimed is a kit comprising a first container containing an endosomal escape enhancing conjugate according to the invention and a second container containing a binding moiety according to the invention, the kit further comprising instructions for using the binding molecules.

The first proteinaceous molecule is suitable for use as a semi-finished product for the manufacture of a functionalized ADC or a functionalized AOC wherein the functionalized ADC or the functionalized OAC comprises at least one covalently coupled saponin of the invention and at least one effector moiety of the invention. An embodiment is the first proteinaceous molecule of the invention further comprising a payload or effector moitety of the invention such as a toxin or an oligonucleotide covalently bound to the first proteinaceous molecule of the invention, either directly or via a linker of the invention, preferably a cleavable linker of the invention, and/or via an oligomeric or polymeric scaffold according to the invention. For example, such a functionalized ADC or OAC comprises 2-4 saponins covalently coupled to e.g. a cysteine side chain in the first proteinaceous molecule such as a ligand or an antibody (fragment), either directly or via a (cleavable) linker, or comprises for example a dendron comprising 1-16 covalently coupled saponins bound thereto, the dendron covalently coupled to e.g. a cysteine side chain and/or a lysine side chain of the first proteinaceous molecule according to the invention.
At least one of the objectives of embodiments of the invention, as listed here above in the *Summary of the invention,* is achieved by providing a first proteinaceous molecule of the invention, comprising a cell-targeting moiety and at least one saponin, the first proteinaceous molecule also suitable for use as a medicament or suitable for implication in a pharmaceutical combination according to the invention, and suitable for use as a semi-finished product in the manufacture of an ADC or an antibody-oligonucleotide conjugate (AOC) of the invention, according to the invention. The therapeutic combination comprises the first proteinaceous molecule comprising covalently bound saponin and comprises a second proteinaceous molecule comprising an effector molecule, also referred to as an effector moiety, wherein the first and second proteinaceous molecule comprise a different binding site for a different epitope exposed on a different cell-surface molecule of a targeted cell, wherein the different cell-surface molecules are expressed by the same target cell and exposed on the surface of the same target cell.

According to the invention, the first proteinaceous molecule is a finished product for application in *e.g.* a therapeutic combination comprising a first pharmaceutical composition comprising the first proteinaceous molecule with saponin covalently coupled to it (first conjugate comprising the first proteinaceous molecule with covalently coupled saponin(s)). Secondly, the first proteinaceous molecule with covalently coupled saponin is also a semi-finished product. The first proteinaceous molecule can be linked to e.g. at least one effector moiety such as an enzyme, toxin such as a protein toxin, oligonucleotide such as a BNA, therwith providing an ADC or an AOC according to the invention, the ADC or AOC provided with one or more covalently linked saponins, optionally via a linker and/or an oligomeric or polymeric scaffold. Thus, an aspect of the invention relates to a conjugate comprising of or consisting of the first proteinaceous molecule comprising a first binding site for binding to a first epitope of a first cell-surface molecule, with at least one saponin covalently bound via at least one linker to the first proteinaceous molecule and/or with at least one saponin covalently bound via an oligomeric or polymeric scaffold to an amino-acid residue of said first proteinaceous molecule, or covalently bound directly to an amino-acid residue of said first proteinaceous molecule, wherein the first binding site comprises or consists of an immunoglobulin, such as an antibody or an IgG, and/or comprises or consists of at least one ligand for binding to a cell-surface molecule such as EGF or a cytokine, and wherein the at least one saponin is selected from the group consisting of: SO1861, SA1641, GE1741, QS-21, QS-21A, QS21-B, Quil-A, SO1832, SO1904, and SO1862.

An embodiment is the first proteinaceous molecule of the invention, wherein the tumor cell-specific first epitope, first tumor-cell surface molecule or first tumor-cell specific receptor, are a first epitope or a first molecule or a first receptor that are internalized by the tumor cell after binding of the first proteinaceous molecule of the invention to the first epitope or first molecule or first receptor, and wherein preferably the first proteinaceous molecule is subjected to tumor-cell receptor-mediated internalization, e.g. via endocytosis, or tumor-cell surface molecule mediated internalization, e.g. via endocytosis, when bound to the cell-surface molecule comprising the first epitope, the tumor-cell surface molecule or the tumor-cell specific receptor.

An embodiment is the first proteinaceous molecule and/or the second proteinaceous molecule of the invention, which is a semi-finished product for the manufacture of an ADC conjugated to at least one saponin, or which is a semi-finished product for the manufacture of an AOC conjugated to at least one saponin, the at least one saponin coupled to the ADC or the AOC via covalent bonds, preferably via at least one linker, and preferably via an oligomeric or polymeric scaffold to which the at least one saponin is covalently coupled, preferably via a linker (Figure 91, 92).

An aspect of the invention relates to any of the following ADCs and AOCs, and their semi-finished conjugates, comprising the first proteinaceous molecule of the invention and/or the second proteinaceous molecule of the invention and/or the third proteinaceous molecule of the invention and either comprising at least one saponin of the invention, or comprising both said saponin and at least one effector molecule of the invention:
Anti-EGFR antibody - SO1861;
Anti-EGFR antibody - GE1741;
Anti-EGFR antibody - SA1641;
Anti-EGFR antibody - Quil-A;
Anti-EGFR antibody - QS-21;
Anti-EGFR antibody - saponins in water soluble saponin fraction of Quillaja saponaria;
Cetuximab - SO1861;
Cetuximab - GE1741;
Cetuximab - SA1641;
Cetuximab - Quil-A;
Cetuximab - QS-21;
Cetuximab - saponins in water soluble saponin fraction of Quillaja saponaria;
Anti-HER2 antibody - SO1861;
Anti-HER2 antibody - GE1741;
Anti-HER2 antibody - SA1641;
Anti-HER2 antibody - Quil-A;
Anti-HER2 antibody - QS-21;
Anti-HER2 antibody - saponins in water soluble saponin fraction of Quillaja saponaria;
Trastuzumab - SO1861;
Trastuzumab - GE1741;
Trastuzumab - SA1641;
Trastuzumab - Quil-A;
Trastuzumab - QS-21;
Trastuzumab - saponins in water soluble saponin fraction of Quillaja saponaria;
Anti-CD71 antibody - SO1861;
Anti-CD71 antibody - GE1741;
Anti-CD71 antibody - SA1641;
Anti-CD71 antibody - Quil-A;
Anti-CD71 antibody - QS-21;
Anti-CD71 antibody - saponins in water soluble saponin fraction of Quillaja saponaria;
OKT-9 - SO1861;
OKT-9 - GE1741;
OKT-9 - SA1641;
OKT-9 - Quil-A;
OKT-9 - QS-21;
OKT-9 - saponins in water soluble saponin fraction of Quillaja saponaria;
Anti-EGFR antibody - oligonucleotide;
Anti-EGFR antibody - antisense oligonucleotide;
Anti-EGFR antibody - siRNA;
Anti-EGFR antibody - antisense BNA;
Anti-EGFR antibody - antisense BNA(HSP27);
Anti-EGFR antibody - proteinaceous toxin;
Anti-EGFR antibody - ribosome inactivating protein;
Anti-EGFR antibody - dianthin;
Anti-EGFR antibody - saporin;
Cetuximab - oligonucleotide;
Cetuximab - antisense oligonucleotide;
Cetuximab - siRNA;
Cetuximab - antisense BNA;
Cetuximab - antisense BNA(HSP27);
Cetuximab - proteinaceous toxin;
Cetuximab - ribosome inactivating protein;
Cetuximab - dianthin;
Cetuximab - saporin;
Anti-HER2 antibody - oligonucleotide;
Anti-HER2 antibody - antisense oligonucleotide;
Anti-HER2 antibody - siRNA;
Anti-HER2 antibody - antisense BNA;
Anti-HER2 antibody - antisense BNA(HSP27);
Anti-HER2 antibody - proteinaceous toxin;
Anti-HER2 antibody - ribosome inactivating protein;
Anti-HER2 antibody - dianthin;
Anti-HER2 antibody - saporin;
Trastuzumab - oligonucleotide;
Trastuzumab - antisense oligonucleotide;
Trastuzumab - siRNA;
Trastuzumab - antisense BNA;
Trastuzumab - antisense BNA(HSP27);
Trastuzumab - proteinaceous toxin;
Trastuzumab - ribosome inactivating protein;
Trastuzumab - dianthin;
Trastuzumab - saporin;
Anti-CD71 antibody - oligonucleotide;
Anti-CD71 antibody - antisense oligonucleotide;
Anti-CD71 antibody - siRNA;
Anti-CD71 antibody - antisense BNA;
Anti-CD71 antibody - antisense BNA(HSP27);
Anti-CD71 antibody - proteinaceous toxin;
Anti-CD71 antibody - ribosome inactivating protein;
Anti-CD71 antibody - dianthin;
Anti-CD71 antibody - saporin;
OKT-9 - oligonucleotide;
OKT-9 - antisense oligonucleotide;
OKT-9 - siRNA;
OKT-9 - antisense BNA;
OKT-9 - antisense BNA(HSP27);
OKT-9 - proteinaceous toxin;
OKT-9 - ribosome inactivating protein;
OKT-9 - dianthin;
OKT-9 - saporin;
Anti-EGFR antibody (- oligonucleotide)(- saponin), wherein the oligonucleotide is any one or more of antisense oligonucleotide, siRNA, antisense BNA, and antisense BNA(HSP27), and wherein the saponin is any one or more of SO1861, GE1741, SA1641, Quil-A, QS-21, and saponins in water soluble saponin fraction of Quillaja saponaria, wherein the anti-EGFR antibody preferably is cetuximab;
Anti-EGFR antibody (- proteinaceous toxin)(- saponin), wherein the proteinaceous toxin is any one or more of a ribosome inactivating protein, dianthin and saporin, and wherein the saponin is any one or more of SO1861, GE1741, SA1641, Quil-A, QS-21, and saponins in water soluble saponin fraction of Quillaja saponaria, wherein the anti-EGFR antibody preferably is cetuximab;
Anti-HER2 antibody (- oligonucleotide)(- saponin), wherein the oligonucleotide is any one or more of antisense oligonucleotide, siRNA, antisense BNA, and antisense BNA(HSP27), and wherein the saponin is any one or more of SO1861, GE1741, SA1641, Quil-A, QS-21, and saponins in water soluble saponin fraction of Quillaja saponaria, wherein the anti-HER2 antibody preferably is trastuzumab;
Anti-HER2 antibody (- proteinaceous toxin)(- saponin), wherein the proteinaceous toxin is any one or more of a ribosome inactivating protein, dianthin and saporin, and wherein the saponin is any one or more of SO1861, GE1741, SA1641, Quil-A, QS-21, and saponins in water soluble saponin fraction of Quillaja saponaria, wherein the anti-HER2 antibody preferably is trastuzumab;
Anti-CD71 antibody (- oligonucleotide)(- saponin), wherein the oligonucleotide is any one or more of antisense oligonucleotide, siRNA, antisense BNA, and antisense BNA(HSP27), and wherein the saponin is any one or more of SO1861, GE1741, SA1641, Quil-A, QS-21, and saponins in water soluble saponin fraction of Quillaja saponaria, wherein the anti-CD71 antibody preferably is OKT-9; and
Anti-CD71 antibody (- proteinaceous toxin)(- saponin), wherein the proteinaceous toxin is any one or more of a ribosome inactivating protein, dianthin and saporin, and wherein the saponin is any one or more of SO1861, GE1741, SA1641, Quil-A, QS-21, and saponins in water soluble saponin fraction of Quillaja saponaria, wherein the anti-CD71 antibody preferably is OKT-9.

An embodiment is the first proteinaceous molecule of the invention, the semi-finished conjugate of the invention or the conjugate of the invention, wherein the first binding site is selected from cetuximab, trastuzumab, OKT-9, and/or wherein the effector molecule is selected from dianthin, saporin and antisense BNA(HSP27), and/or wherein the saponin is selected from SO1861, GE1741, SA1641, Quil-A, QS-21, and saponins in water soluble saponin fraction of Quillaja saponaria.

An embodiment is the conjugate according to the invention, wherein the first proteinaceous molecule is selected from cetuximab, trastuzumab, OKT-9, and/or wherein the effector molecule is selected from dianthin, saporin and antisense BNA(HSP27), and/or wherein the saponin is selected from SO1861, GE1741, SA1641, Quil-A, QS-21, and saponins in water soluble saponin fraction of Quillaja saponaria.

An aspect of the invention relates to an ADC or an AOCs or a semi-finished ADC conjugate or a semi-finished AOC conjugate comprising the first proteinaceous molecule of the invention and comprising at least one effector molecule of the invention and/or comprising at least one saponin of the invention, of Structure C:

A (- S)b (- E)c Structure C,

wherein A is the first binding site;
S is the saponin;
E is the effector molecule;
b = 0 - 64, preferably 0, 1, 2, 3, 4, 8, 16, 32, 64 or any whole number or fraction therein between;
c = 0 - 8, preferably 0, 1, 2, 3, 4, 6, 8 or any whole number or fraction therein between,
wherein S is coupled to A and/or E, E is coupled to A and/or S, preferably S is coupled to A and E is coupled to A, wherein A is an anti-EGFR antibody such as cetuximab, an anti-HER2 antibody such as trastuzumab, an anti-CD71 antibody such as OKT-9, and wherein S is any one or more of SO1861, GE1741, SA1641, Quil-A, QS-21, and saponins in water soluble saponin fraction of Quillaja saponaria, and/or wherein E is any one or more of an oligonucleotide, an antisense oligonucleotide, an siRNA, an antisense BNA, and an antisense BNA(HSP27), and/or any one or more of a proteinaceous toxin, a ribosome inactivating protein, dianthin and saporin.

An aspect of the invention relates to the use of any of the aforementioned conjugates of the invention or the semi-finished conjugates of the invention or the first proteinaceous molecule of the invention, as a medicament.

An aspect of the invention relates to the use of any of the conjugates of the invention or the semi-finished conjugate of the invention or the first proteinaceous molecule of the invention, for use in the treatment or prophylaxis of a cancer or an auto-immune disease.

Figure 91 and Figure 92 show examples of ADCs of the invention with covalently coupled saponin(s) and OACs of the invention with covalently coupled saponin(s).

The invention is further illustrated by the following examples, which should not be interpreted as limiting the present invention in any way: the invention is limited only by the appended claims.

### EXAMPLES

### EXAMPLE A - TREATING A MAMMALIAN TUMOR-BEARING ANIMAL WITH A CONJUGATE OF THE INVENTION IN COMBINATION WITH AN ADC RESULTS IN SURVIVAL AND TUMOR REGRESSION

Female Balb/c nude mice were injected subcutaneously with a suspension of human A431 tumor cells. Under the skin of the mice, a human epidermal carcinoma developed in the xenograft animal tumor model. After injection of the tumor cells, the xenograft tumor was allowed to develop to a size of approximately 170-180 mm³. The A431 tumor cells have the following characteristics: high EGFR expressors, medium CD71 expressors, low HER2 expressors.

In Table A, the results of the treatment of control mice and tumor-bearing mice are presented. Tumor-bearing mice were treated with the indicated antibodies directed to either human Her2/neu, human EGFR, or human CD71, which are cell-surface receptors on the xenograft tumor. Cetuximab was covalently conjugated with saponin SO1861. The SO1861 was first provided with the linker EMCH (N-ε-mateimidocaproic acid hydrazide), which EMCH is a maleimide-and-hydrazide crosslinker for covalently conjugating sulfhydryls (reduced cysteines of the antibody)) to carbonyls (aldehyde or ketones; here the carbonyl of the aldehyde at position C-23 of the saponin). The saponin-EMCH was covalently coupled to reduced cysteines of the Cetuximab, forming a covalent thio-ether bond between the EMCH and the cysteine side chain. The ADCs trastuzumab-saporin (covalent conjugate) and anti-CD71 mAb (OKT-9, IgG) - saporin (covalent conjugate) were tested for their tumor-attacking efficacy in the mice, measured as tumor volume in time after start of the treatment with the ADCs. The dose of the ADCs was sub-optimal in the tumor model. That is to say, from previous experiments, it was established at which sub-optimal dose of the ADCs no tumor-regression or arrest of tumor growth would be observable.

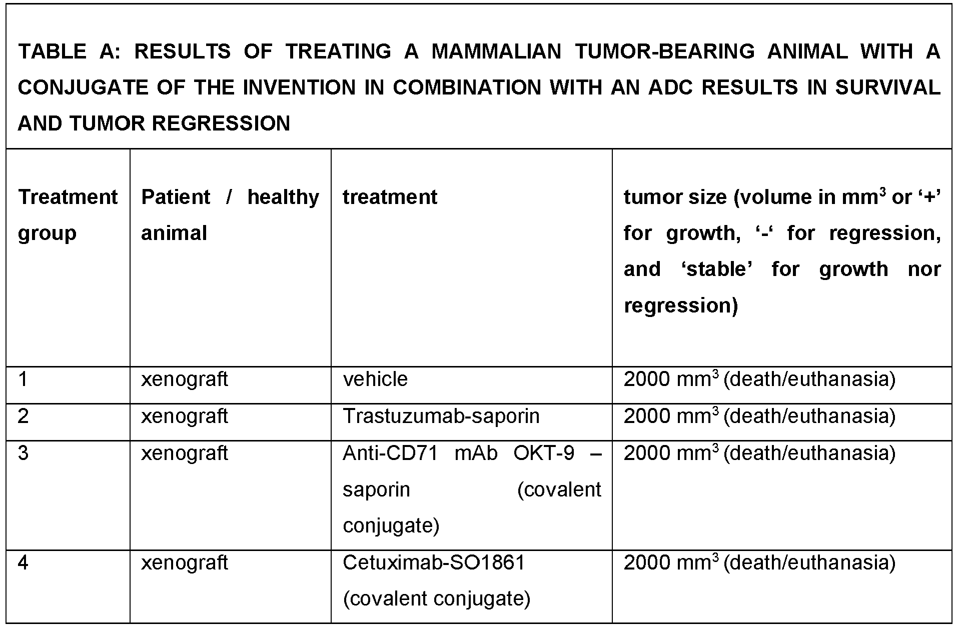

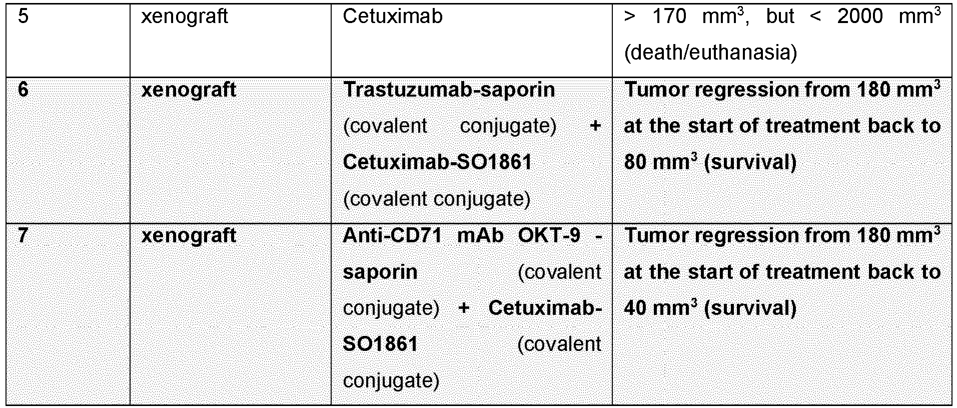

These results demonstrate that the combination therapy of an ADC at a dose which is ineffective when treatment of tumor-bearing mice with the ADC alone is considered (tumor growths, death of the mice is not prevented (euthanasia)), with a conjugate of the invention consisting of a tumor-cell specific receptor targeting antibody covalently bound to a saponin, i.e. SO1861, the covalent conjugate administered to the mice suffering from cancer, at a non-effective dose when administered alone (tumor growths, death of the mice is not prevented (euthanasia)), provides an efficient and efficacious treatment regimen, expressed as tumors in regression and prolonged survival of the treated animals (beyond the duration of the experiment). The sub-optimal dose of ADC combined with a covalently bound saponin-comprising conjugate of the invention which has no anti-tumor activity when administered alone, thus provide for an effective treatment option for cancer patients, wherein a relative low dose of the ADC is efficacious. A lower dose of ADC bears the promise of less risk for adverse events, or even no side effects at all. In addition, the stimulatory effect of the saponin-bearing conjugate of the invention when the efficacy of the ADC is considered, shows that ADCs which previously have proven to lack efficacy when tumor patient treatment is concerned, may gain renewed attention and value, since ADC efficacy is improved in combination therapy setting, as the current example demonstrated. Reference is made to Table A2 and Table A3, summarizing ADCs which were previously investigated in the human clinical setting, but then were for some ADCs retracted from further clinical investigation. Especially the ADCs for which clinical development was terminated due to observed lack of efficacy and/or due to occurrence of unacceptable adverse event are ADCs which may gain renewed value for cancer patients when combined with a covalently bound saponin-comprising conjugate of the invention, such as the cetuximab-saponin tested.

### EXAMPLE B - saponins mixture of Quillaja saponaria comprising QS-21, with endosomal/lysosomal escape enhancing activity

Scheme I displays the common molecular structure of a series of QS-21 saponins (in part adapted from: Conrado Pedebos, Laércio Pol-Fachin, Ramon Pons, Cilaine V. Teixeira Hugo Verli, Atomic Model and Micelle Dynamics of QS-21 Saponin, Molecules 2014, 19, 3744-3760). A mixture of water-soluble saponins obtained from Quillaja saponaria (Sigma-Aldrich, product No. S4521; Roth, Item No. 6857; InvivoGen, product 'Quil-A') may be applied in the endosomal/lysosomal escape enhancing conjugate, composition, combination of the invention, based on endosomal/lysosomal escape enhancing properties of at least one individual saponin present in the mixture, e.g. QS-21, or based on a combination of two or more of the saponins comprised by the mixture, such as QS-21 and QS-7.

The inventors demonstrated that the mixture of saponins from Quillaja saponaria at 2,5 microgram/ml dose was capable of enhancing endosomal escape of dianthin, as tested with mammalian tumor cells in a cell-based bioassay. The effector moiety exposed to the cells was dianthin covalently coupled to the ligand EGF: EGF-dianthin. Cells tested were tumor cell lines HeLa for free saponins, and A431, MDA-MB-468, CaSki and A2058 for testing the saponins when covalently coupled to cetuximab.

### Example 1

Various concentrations of trastuzumab-saporin (HER2 targeted protein-toxin conjugate; intravenous) were tested in combination with 1.5 mg/kg SO1861 (1 hour before antibody-toxin injection; subcutaneous) for enhanced efficacy in a BT474 (HER2++) xenograph mouse model. Dosing started at day 13 when tumors reached ∼150mm³ in size and tumor volume was determined after every treatment. Although tumor growth inhibition was observed in the mice treated with 1 mg/kg and 0.3 mg/kg trastuzumab-saporin, there was no enhanced tumor growth inhibition observed in the mice treated with the combination of trastuzumab-saporin + SO1861. This shows that unconjugated SO1861 is not able to enhance antibody-protein toxins within the current settings and mouse model.

### Example 2

### Materials:

QSmix (1): S4521 (Sigma Aldrich); QSmix (2) : 6857.1 (Carl Roth) QSmix (3): Quil-A® Adjuvant: vacquil (InvivoGen/Brenntag).
Previously, the efficacy of various saponins (SO1861, SO1642) were co administrated as 'free' unconjughated molecules to cells in combination with a ligand toxin fusion (e.g. EGFdianthin) or an antibody-protein toxin conjugate, resulting in enhanced cell killing activity of target expressing cells. Here, three different saponin molecules (SO1861, SO1862 (isomer of SO1861), SO1832 and SO1904) isolated from a root extract of *Saponaria officinalis* were titrated in the presence and absence of a non-effective fixed concentration of 1.5 pM EGFdianthin on HeLa (EGFR⁺) cells. This revealed a strong enhancement of cell killing activity for all tested saponin variants (IC50= 300 nM; Figure 2A) compared to the treatments without EGFdianthin. Next, EGFdianthin was titrated with a fixed concentration of saponin (∼1000nM) and this revealed strong targeted cell killing enhancement at low pM concentrations of EGFdianthin (IC50= 0.4 pM; Figure 2B), observed for all used saponins SO1861, SO1862 (isomer of SO1861), SO1832 and SO1904. EGF-dianthin alone could only induce cell killing at very high concnetrations (IC50=10.000 pM). This shows that these specific types of saponins, all have the intrinsic capacity to efficiently induce endosomal escape with only a very low amount of targeted toxin available.

To extend this test, saponins from other sources were analyzed. A saponin purified from a root extract of *Gypsophila elegans* M.Bieb. (GE1741) was titrated on HeLa cells in the presence and absence of 1.5 pM EGFdianthin and compared with purified SO1861. GE1741 also enhances the EGFdianthin induced HeLa cell killing, but shows slightly less efficacy compared to SO1861. (GE1741 IC50= 800 nM; Figure 2C) and also displays a higher general toxicity (IC50= 5.000 nM in absence of EGFdianthin; Figure 1C). A similar test in which different partially purified mixtures of *Quillaja saponaria* saponins (QSmix 1-3) were co-administrated with 1.5 pM EGFdianthin on HeLa cells and this revealed for 2 out of 3 (QSmix 1 and QSmix 3) similar activity as SO1861 (IC50 QSmix/QSmix3=300nM; Figure 1D). QSmix (2) is less efficient in enhancing 1.5 pM EGFdianthin induced cell killing (IC50= 2000 nM; Figure 2D), however, no general toxicity is observed. This shows that also in QS extracts, specific type of saponins are available that efficiently induce endosomal escape of the targeting ligand toxin EGFdianthin.

### Example 3

In order to conjugate SO1861 molecules to antibodies, according to the invention, labile/acid sensitive linkers (-EMCH or -N3), was conjugated to SO1861 via the aldehyde group, producing SO1861-EMCH or SO1861-N3 (Figure 60-66. To verify the activity of SO1861-EMCH the molecule was titrated in the presence and absence of a fixed non-effective (1.5 pM) EGFdianthin concentration on EGFR expressing (A431, HeLa) and non-expressing cells (A2058). In all three cell lines SO1861 alone showed a strong cell viability reduction, whereas SO1861-EMCH as single compound showed no toxicity up to 25.000 nM (Figure 3A-C). When SO1861-EMCH was combined with 1.5 pM EGFdianthin a strong target specific cell viability reduction is observed in the EGFR⁺ A431 and HeLa cells (IC50= 3.000 nM; Figure 2A, B), while the EGFR⁻ A2058 cells are not affected at all (Figure 3C). Similar results were obtained for SO1861-N3. SO1861-N3 co-administrated with 1.5 pM EGFdianthin also shows efficient cell killing on A431 and HeLa cells (IC50= 3.000 nM), but without EGFdianthin a general toxicity is observed at above 10.000 nM (Figure 3D, 2E).

For the stable conjugation of SO1861 to antibodies, according to the invention, a stable linker (HATU, Figure 70) was conjugated to SO1861 via the carboxylic acid group of SO1861 producing, SO1861-(S). To determine the activity different concentrations of SO1861-(S) were co-administrated with 1.5 pM EGFdianthin and tested for cell killing activity in EGFR expressing HeLa cells. SO1861-(S) showed a similar activity as SO1861, indicating that conjugation to the carboxylic acid does not affect the endosomal escape enhancing potency of the molecule as is observed with SO1861-EMCH (Figure 4).

The 1 target 2-components system (1T2C) is the combination treatment of mAb1-protein toxin and mAb1-SO1861, as illustrated in Figure 19. SO1861-EMCH was conjugated via cysteine residues (Cys) and HSP27BNA oligo was conjugated via lysine residues to cetuximab (monoclonal antibody recognizing and binding human EGFR), both with a DAR 4 resulting in the production of 2 conjugates: cetuximab-(Cys-L-SO1861)⁴ and cetuximab-(Lys-L-HSP27BNA)⁴. The combination of cetuximab-(Cys-L-SO1861)⁴ (intraperitoneal administration, (i.p.)) and cetuximab-(Lys-L-HSP27BNA)⁴ (intravenous administration, (i.v.)) was tested in a A431 xenograph 'mouse tumor model for EGFR tumor targeted gene silencing activity. Dosings started at day 12 when tumors reached ∼150mm³ in size and tumor samples were collected at 72h after the first dosing and analysed for HSP27 gene expression compared to control gene mRNA expression levels (reference genes). This revealed that 1 dosing of 50 mg/kg cetuximab-(Cys-L-SO1861)⁴+ 25 mg/kg cetuximab-(Lys-L-HSP27BNA)⁴ resulted in a 50% reduction in HSP27 gene expression in the A431 tumors compared to single dosing of cetuximab-(Cys-L-SO1861)⁴ or cetuximab-(Lys-L-HSP27BNA)⁴ mono therapies (Figure 7). Compared to the vehicle control tumors, a reduction of 40% HSP27 gene silencing was observed. This shows and enables that the combination of cetuximab-conjugated SO1861 + cetuximab-conjugated HSP27BNA oligo, according to the 1T2C invention, induces efficient targeted delivery of a therapeutic antisense oligo nucleotide in the cytoplasm of solid tumor cells, thereby inducing tumor targeted gene silencing, *in vivo.*

Next, SO1861-EMCH was conjugated via cysteine residues (Cys) to trastuzumab (monoclonal antibody recognizing and binding human HER2), with a DAR 4 resulting in the production of trastuzumab-(Cys-L-SO1861)⁴. The combination of trastuzumab-(Cys-L-SO1861)⁴ and trastuzumab-saporin (trastuzumab protein toxin conjugate) was tested in a mouse tumor model (patient derived xenograph tumor model, PDX) with high HER2 expression levels and resistant for trastuzumab mono therapy. The combination, according to the 1T2C invention of 40 mg/kg trastuzumab-(Cys-L-SO1861)⁴ (intraperitoneal administration, (i.p.)) + 0.03 (Day1, 8)/ 0.02 (Day 15, 22, 30, 36,43) mg/kg trastuzumab-saporin (intravenous administration, (i.v.)) revealed strong tumor growth inhibition compared to the vehicle control and the 40 mg/kg trastuzumab-(Cys-L-SO1861)⁴ or 0.03/0.02 mg/kg trastuzumab-saporin mono therapies (Figure 8). Besides, in tumor bearing mice that were treated with a lower dosing combination (40 mg/kg trastuzumab-(Cys-L-SO1861)⁴+ 0.01 mg/kg trastuzumab-saporin) no tumor growth inhibiting activity was observed (Figure 8). This shows and enables that the 1T2C combination of trastuzumab conjugated SO1861 + trastuzumab conjugated protein toxin induces efficient targeted delivery of a therapeutic protein toxin in the cytoplasm of solid tumor cells, thereby inducing tumor cell death and tumor growth inhibition, *in vivo.*

The 1 target 2-components system (1T2C) is the combination treatment of mAb1-SO1861 and mAb1-protein toxin (Figure 19)

SO1861-EMCH was conjugated via cysteine residues (Cys) to cetuximab (monoclonal antibody recognizing and binding human EGFR), with a DAR 3,7 (cetuximab-(Cys-L-SO1861)^{3,7}). Cetuximab-(Cys-L-SO1861)^{3,7} was titrated on a fixed concentration of 10 pM cetuximab-saporin (cetuximab, conjugated to the protein toxin, saporin) and targeted protein toxin mediated cell killing on EGFR expressing cells (A431, EGFR⁺⁺; CaSKi, EGFR⁺) was determined. This revealed strong cell killing at low concentrations of cetuximab-(Cys-L-SO1861)^{3,7} (A431: IC50= 0,6 nM and Caski IC50= 1 nM; Figure 9A, 9B) whereas cetuximab, cetuximab-(Cys-L-SO1861)^{3,7} or cetuximab + 10 pM cetuximab-saporin could not induce any cell killing activity in EGFR expressing cells. This shows that cetuximab conjugated SO1861 efficiently enhances endosomal escape of the cetuximab conjugated protein toxin (at non-effective concentrations), thereby inducing cell killing of EGFR expressing cells. The cell killing activity in A431 is more effective compared to CaSki correlating with EGFR expression levels in these cell lines. EGFR receptor binding competition between both conjugates within the 1T2C is also observed when cetuximab-(Cys-L-SO1861)^{3,7} concentrations increase, cell killing activity declines due to outcompeting receptor binding and internalization of cetuximab-saporin (Figure 9A, 9B).

Next, cetuximab-saporin was titrated on a fixed concentration of 75 nM cetuximab-(Cys-L-SO1861)^{3,7} and targeted protein toxin mediated cell killing on EGFR expressing cells was determined. This revealed that 75nM cetuximab-(Cys-L-SO1861)^{3,7} in combination with low concentrations cetuximab-saporin induced already efficient cell killing in EGFR expressing cells (A431: IC50= 0.4 pM; and CaSKi: (IC50= 2 pM; Figure 9C and 9D), whereas cetuximab-saporin alone or cetuximab-saporin + 75 nM cetuximab showed cell killing only at high concentrations cetuximab-saporin (IC50= 40 pM, IC50= 1000 pM, resp.) in both cell lines (Figure 9C, 9D). All this shows that relatively low concentrations of cetuximab-saporin can be effective and induce cell killing only in combination with low cetuximab-SO1861 concentrations in high EGFR expressing cells. The receptor competition between both conjugates within the 1T2C system is also observed in the cetuximab-toxin titration treatments when the cell killing activity of cetuximab-saporin with and without 75 nM cetuximab was compared (Figure 9C, 9D).

Next, cetuximab-(Cys-L-SO1861)^{3,7} was titrated on a fixed concentration of 10 pM cetuximab-saporin and targeted protein toxin mediated cell killing on low EGFR expressing cells or cells without EGFR expression (HeLa, EGFR^{+/-}; A2058, EGFR⁻) was determined. Cells with low (HeLa) or no (A2058) EGFR expression were not sensitive at all for any combination of cetuximab-(Cys-L-SO1861)^{3,7} + 10 pM cetuximab-saporin (HeLa: IC50> 1000 nM; A2058: IC50> 1000 nM; Figure 10A, 10B). This shows that in the absence of sufficient EGFR receptor expression, effective intracellular delivered SO1861 concentrations are not optimal (threshold) to induce endosomal protein toxin escape and toxin-mediated cell killing. Next, cetuximab-saporin was titrated on a fixed concentration of 75 nM cetuximab-(Cys-L-SO1861)^{3,7} and targeted protein toxin mediated cell killing on low (HeLa) or no (A2058) EGFR expressing cells was determined. Low EGFR expressing cells (HeLa) showed cell killing only at high cetuximab-saporin concentrations in combination with 75 nM cetuximab-(Cys-L-SO1861)^{3,7} (HeLa: IC50= 60 pM), Figure 10C), whereas A2058 cells (EGFR) are not sensitive at any of the tested concentrations (A2058: IC50> 10.000 pM; Figure 10D). All this shows that cells with low or no EGFR receptor expression are not susceptible for the combination of cetuximab-(Cys-L-SO1861)^{3,7} + cetuximab-saporin, due to a lack of sufficient EGFR receptor that facilitates the antibody-mediated delivery of sufficient SO1861 within the endolysosomal compartments, to facilitate the escape of the protein toxin.

Next, SO1861-EMCH was conjugated via cysteine residues (Cys) to trastuzumab (monoclonal antibody recognizing and binding human HER2, with a DAR 4, (trastuzumab-(Cys-L-SO1861)⁴). Trastuzumab-(Cys-L-SO1861)⁴ was titrated on a fixed concentration of 50 pM trastuzumab-saporin (trastuzumab, conjugated to the protein toxin, saporin) and targeted protein toxin mediated cell killing on HER2 expressing cells (SK-BR-3, HER2⁺⁺) was determined. This revealed strong cell killing at low concentrations of trastuzumab-(Cys-L-SO1861)⁴ (SK-BR-3: IC50= 0,8 nM; Figure 11A) whereas equivalent concentrations trastuzumab, trastuzumab-(Cys-L-SO1861)⁴ or trastuzumab + 50 pM trastuzumab-saporin could not induce any cell killing activity in HER2 expressing cells. This shows that trastuzumab conjugated SO1861 efficiently enhances endosomal escape of the trastuzumab conjugated protein toxin (at non-effective concentrations), thereby inducing cell killing of HER2 expressing cells. The receptor competition between both conjugates within the 1T2C is also observed when trastuzumab-(Cys-L-SO1861)⁴ concentrations increase, cell killing activity declines due to outcompeting receptor binding and internalization of trastuzumab-saporin (Figure 11A).

Next, trastuzumab-saporin was titrated on a fixed concentration of 2,5 nM trastuzumab-(Cys-L-SO1861)⁴, according to the invention and targeted protein toxin mediated cell killing on HER2 expressing cells was determined. This revealed that 2,5 nM trastuzumab-(Cys-L-SO1861)⁴ in combination with low concentrations trastuzumab-saporin induced already efficient cell killing in HER2 expressing cells (SK-BR-3: IC50= 2 pM; Figure 11B), whereas trastuzumab-saporin alone or trastuzumab-saporin + 2,5 nM trastuzumab showed cell killing only at high concentrations trastuzumab-saporin (Figure 11B). All this shows that relatively low concentrations of trastuzumab-saporin can be effective and induce cell killing only in combination with low trastuzumab-(Cys-L-SO1861)⁴ concentrations in high HER2 expressing cells.

Next, trastuzumab-(Cys-L-SO1861)⁴ was titrated on a fixed concentration of 50 pM trastuzumab-saporin, according to the invention and targeted protein toxin mediated cell killing on low HER2 expressing cells (A431 HER2^{+/-}) or cells without HER2 expression (JIMT-1: HER2⁺; MDA-MB-468: HER2⁻) was determined. Cells with low or no HER2 expression were not sensitive at all for any combination of trastuzumab-(Cys-L-SO1861)⁴+ 50 pM trastuzumab-saporin (JIMT-1: IC50> 1000 nM; MDA-MB-468: IC50> 1000 nM; Figure 13A, 13B). This shows that in the absence of sufficient HER2 receptor expression, effective intracellular delivered SO1861 concentrations are not optimal (threshold) to induce endosomal protein toxin escape and toxin-mediated cell killing. Next, trastuzumab-saporin was titrated on a fixed concentration of 2,5 nM trastuzumab-(Cys-L-SO1861)⁴ and targeted protein toxin mediated cell killing on low or no HER2 expressing cells was determined. Low HER2 expressing cells (JIMT-1) showed cell killing only at high trastuzumab-saporin concentrations in combination with 2,5 nM trastuzumab-(Cys-L-SO1861)⁴ (JIMT-1: IC50> 10.000 pM; Figure 13C), whereas MDA-MB-468 cells (HER2⁻) are not sensitive at any of the tested concentrations (MDA-MB-468: IC50> 10.000 pM; Figure 13D).

All this shows that cells with low or no HER2 receptor expression are not susceptible for the combination of trastuzumab-(Cys-L-SO1861)^{3,7}+ trastuzumab-saporin, due to a lack of sufficient HER2 receptor that facilitates the antibody-mediated delivery of sufficient SO1861 within the endolysosomal compartments, to facilitate the escape of the protein toxin.

In order to show that the activity of the 1T2C system is driven by the acidification of the endolysosomal compartments, the 1T2C system , according to the invention was tested in combination with an endosomal acidification inhibitor, chloroquine. Trastuzumab-saporin was titrated in combination with 5 nM trastuzumab-(Cys-L-SO1861)⁴ in combination with or without chloroquine. Trastuzumab-saporin + 5 nM trastuzumab-(Cys-L-SO1861)⁴ showed a strong cell killing activity in high HER2 expressing cells (SK-BR-3, HER2⁺⁺; IC50= 0.2 pM;), however, trastuzumab-saporin + 5 nM trastuzumab-(Cys-L-SO1861)⁴+ 0.5 µM chloroquine resulted in strong inhibition of the 1T2C cell killing activity in SK-BR-3 (HER2⁺⁺) cells (IC50= 40pM). This shows that activity of the antibody conjugated SO1861 is reduced/blocked when acidification of endoslysomes is prohibited (Figure 14A). Same results were derived with the 1T2C combination, according to the invention of cetuximab-saporin + 5 nM cetuximab-(Cys-L-SO1861)^{3,8} (IC50= 1 pM) compared with cetuximab-saporin + 5 nM cetuximab-(Cys-L-SO1861)^{3,8} + 0.5 µM chloroquine (IC50= 200 pM) in EGFR expressing cells (A431, EGFR⁺⁺; Figure 14B).

The 1 target 2-components system (1T2C) can also be the combination treatment of mAb1-SO1861 and mAb1-antisense BNA oligo nucleotide as illustrated in Figure 20. For this we used an antisense BNA oligonucleotide against the mRNA of a cancer specific target gene (upregulated in cancer cells), heat shock protein 27 (HSP27). Upon release into the cytoplasm the antisense BNA recognizes and binds the mRNA encoding for HSP27, targeting the mRNA for destruction thereby depleting the HSP27 mRNA expression within the cancer cell. HSP27BNA was conjugated to cetuximab with a DAR4 (Cetuximab-(Lys-L-HSP27BNA)⁴) and tested in combination with cetuximab-(Cys-L-SO1861)^{3,8} for enhanced HSP27 gene silencing activity in EGFR expressing cells (A431, EGFR⁺⁺) and non-expressing cells (A2058, EGFR), according to the invention (Figure 20). Cetuximab-(Cys-L-SO1861)^{3,8}+ 100 nM cetuximab-(Lys-L-HSP27BNA)⁴ showed strong HSP27 gene silencing in EGFR expressing cells (A431: IC50= nM, Figure 15A), whereas cetuximab-(Cys-L-SO1861)^{3,8} alone did not show any gene silencing activity. In A2058 cells (EGFR) no gene silencing activity was observed in the 1T2C combination (Figure 15B). Next, cetuximab-(Lys-L-HSP27BNA)⁴ + 76.9 nM Cetuximab-(Cys-L-SO1861)^{3,8} show strong HSP27 gene silencing activity in EGFR expressing cells (A431: IC50= 4 nM, Figure 15C), whereas cetuximab-(Lys-L-HSP27BNA)⁴ or cetuximab-(Cys-L-SO1861)^{3,8} or the combination of Cetuximab-(Lys-L-HSP27BNA)⁴ + 77 nM cetuximab did not reveal any significant gene silencing activity (IC50> 100nM). When the experiment was performed in EGFR non-expressing cells (A2058) no gene silencing activity was observed in the 1T2C combination (IC50>100 nM; Figure 15D). All this shows that the 1T2C system efficiently delivers an antisense BNA oligo to the cytoplasm of high EGFR expressing cells, thereby inducing mRNA degradation of the BNA target mRNA resulting in target gene silencing.

The 1 target 2-components system (1T2C) can also be the combination treatment of mAb1-(scaffold(-SO1861)ⁿ)ⁿ and mAb1-protein toxin as illustrated in Figure 21. Dendron(-L-SO1861)⁴ was conjugated to cetuximab via cysteine residues (Cys) conjugation with a DAR3,9 and cetuximab-Cys-(dendron(-L-SO1861)⁴)^{3,9} was tested for enhanced cell killing activity in combination with an anti-EGFR antibody-protein toxin conjugate (cetuximab-saporin) in EGFR expressing cells (MDA-MB-468). Cetuximab-Cys-(dendron(-L-SO1861)⁴)^{3,9} + 10 pM cetuximab-saporin efficiently induces toxin-mediated cell killing in high EGFR expressing cells (IC50= 0.4 nM; Figure 16A), whereas this was not induced by cetuximab-Cys-(dendron(-L-SO1861)⁴)^{3,9} or cetuximab + 10 pM cetuximab-saporin or cetuximab (Figure 16A). This shows that according to the 1T2C invention, cetuximab conjugated dendron(-L-SO1861)⁴ efficiently enhances endosomal escape of the cetuximab conjugated protein toxin (at non-effective concentrations), thereby inducing cell killing of high HER2 expressing cells.Similar 1T2C experiments were performed in cells that express low levels of EGFR (HeLa, EGFR^{+/-}) and this revealed no cell killing activity when the 1T2C combination, according ot the invention was used (IC50> 100pM; Figure 16B) indicating that in the absence of sufficient EGFR receptor expression, effective intracellular SO1861 concentrations are not optimal (threshold) to induce cytoplasmic delivery of the protein toxin that results in toxin-mediated cell killing.

Next, dendron(-L-SO1861)⁴ was conjugated to the anti-HER2 antibody, trastuzumab via cysteine conjugation (Cys) with a DAR4, trastuzumab-Cys-(dendron(-L-SO1861)⁴)⁴ and tested for enhanced cell killing activity in combination with an anti-HER2 antibody-protein toxin conjugate (trastuzumab-saporin) in HER2 expressing cells (SK-BR-3, HER2⁺⁺). Trastuzumab-Cys-(dendron(-L-SO1861)⁴)⁴ + 50 pM trastuzumab-saporin efficiently induces toxin-mediated cell killing (IC50= 2 nM, Figure 16C), whereas this was not induced by trastuzumab-Cys-(dendron(-L-SO1861)⁴)⁴ ortrastuzumab + 50nM trastuzumab-saporin or trastuzumab (Figure 16C). This shows that trastuzumab conjugated dendron(-L-SO1861)⁴ efficiently enhances endosomal escape of the trastuzumab conjugated protein toxin (at non-effective concentrations), thereby inducing cell killing of high HER2 expressing cells. Similar experiments in cells that express low levels of HER2 (JIMT-1, HER2^{+/-}) revealed no activity of Trastuzumab-Cys-(dendron(-L-SO1861)⁴)⁴ + 50 pM trastuzumab-saporin (IC50> 200 nM; Figure 16D) indicating that in the absence of sufficient HER2 receptor expression, effective intracellular SO1861 concentrations are not optimal (threshold) to induce endosomal protein toxin escape and toxin-mediated cell killing.

The clinical approved ADC, trastuzuzmab-emtansine (T-DM1) is a conjugate of the anti-Her2 antibody, trastuzumab and the small molecule toxin emtansine (DAR3.5). T-DM1 was titrated in combination with trastuzumab-(Cys-L-SO1861)⁴ and compared with the antibody protein toxin conjugate, trastuzumab-saporin + Trastuzumab-(Cys-L-SO1861)⁴, according to the invention. Whereas trastuzumab-saporin + 2.5 nM trastuzumab-(Cys-L-SO1861)⁴ showed enhanced activity compared to Trastuzumab-saporin + 2.5 nM trastuzumab or trastuzumab-saporin alone (IC50= 2 pM, Figure 17), T-DM1 + 25.6 nM trastuzumab-(Cys-L-SO1861)⁴ showed no enhanced cell killing activity (IC50> 100 pM; Figure 17). This shows that the 1T2C system, according to the invention, cannot enhance the delivery of an antibody small molecule conjugate, since small molecules can already passively cross (endolysosomal) membranes.

The 1 target 2-components system (1T2C) can also be the combination treatment of mAb1-QSmix (mixture of saponins from Quillaja Saponaria) and mAb1-protein toxin.

QSmix-EMCH was conjugated via cysteine residues (Cys) to cetuximab (monoclonal antibody recognizing and binding human EGFR), with a DAR 4.1 (cetuximab-(Cys-L-QSmix)^{4,1}). Cetuximab-(Cys-L-QSmix)^{4,1} was titrated on a fixed concentration of 10 pM cetuximab-saporin or 10pM cetuximab-dianthin and targeted protein toxin mediated cell killing on A431 (EGFR⁺⁺), CaSKi (EGFR⁺) and A2058 (EGFR) cells was determined. This revealed strong cell killing at low concentrations of cetuximab-(Cys-L-QSmix)^{4.1} + 10 pM cetuximab-saporin or 10pM cetuximab-dianthin in A431 (EGFR⁺⁺) and CaSKi (EGFR⁺) cells (A431: IC50= 3 nM, Figure 18A; CaSKi: IC50= 1nM, Figure 18B) whereas all control treatments could not induce any cell killing in EGFR expressing cells. In cells that do not express EGFR (A2058; EGFR⁻) no HSP27 gene silencing is observed with the combination, according to the invention (IC50> 1000 nM; Figure 18C). This shows that cetuximab conjugated QS21mix efficiently enhances endosomal escape of the cetuximab conjugated protein toxin (at non-effective concentrations), thereby inducing cell killing only in EGFR expressing cells.

### Example 4

Labile SO1861 was conjugated via cysteine residues (Cys) to the anti-EGFR antibody cetuximab (monoclonal antibody recognizing and binding human EGFR), with DAR3.9 (cetuximab-(Cys-L-SO1861)^{3.9}) and tested for its enhanced delivery of antisense BNA oligo nucleotides resulting in enhanced target gene silencing. In this study we used an antisense BNA oligonucleotide against the mRNA of a cancer specific target gene, heat shock protein 27 (HSP27). Within the cytoplasm of the cell HSP27BNA bind the mRNA encoding for HSP27, target the mRNA for destruction, thereby reducing the HSP27 expression within the cancer cell. Cetuximab-(Cys-L-SO1861)^{3.9} was titrated on fixed concentration of 100 nM HSP27BNA on EGFR⁺⁺ (A431) and EGFR (A2058) cells. The combination according ot the invention showed efficient HSP27 silencing on A431 (IC50= 2 nM; Figure 5A), while no silencing was observed for cetuximab-(Cys-L-SO1861)^{3.9} alone. Cetuximab-(Cys-L-SO1861)^{3.9} + 100nM HSP27BNA showed no gene silencing activity in EGFR⁻ cells (A2058) (Figure 5B). This shows that low concentrations of antibody-conjugated SO1861 efficiently can enhance cytoplasmic delivery and endolysosomal escape of an antisense BNA oligo nucleotide, thereby inducing efficient gene silencing in target expressing cells.

Next the HSP27BNA was titrated on EGFR⁺⁺ (A431) and EGFR⁻ (A2058) cells combined with fixed concentration of cetuximab-(Cys-L-SO1861)^{3.9} . This shows that HSP27BNA in combination with 28.6 nM cetuximab-(Cys-L-SO1861)^{3.9} or 77 nM cetuximab-(Cys-L-SO1861)^{3.9} very efficiently enhances HSP27 gene silencing in A431 cells (IC50= 10 nM; Figure 5C). HSP27BNA alone or combined with a fixed equivalent of 77 nM cetuximab are less efficient (IC50= 1.000 nM; Figure 12C). The combination treatment of HSP27BNA + 77 nM cetuximab-(Cys-L-SO1861)^{3.9} was also tested on EGFR- cells (A2058) and this revealed no HSP27 gene silencing enhancement (IC50=1.000 nM; Figure 5D). This shows that cells with low or no EGFR receptor expression are not susceptible for the combination of cetuximab-(Cys-L-SO1861)^{3,9} + HSP27BNA, while cetuximab targeted SO1861 can enhance HSP27 gene silencing efficiently at low concentrations of non-targeted HS27BNA in high EGFR cells.

Next, SO1861-EMCH was conjugated via cysteine residues (Cys) to cetuximab (monoclonal antibody recognizing and binding human EGFR), with a DAR 3,8. The combination according to the invention, cetuximab-(Cys-L-SO1861)^{3,8} + HSP27BNA (antisense HSP27BNA oligo nucleotide targeting and inducing degradation of the onco-target *hsp27* mRNA (gene silencing) in cancer cells) was tested in a A431 xenograph 'nude' mouse tumor model for EGFR-mediated tumor targeted HSP27 gene silencing. Dosing started at day 12 when tumors reached ∼150mm³ in size and HSP27 mRNA expression was determined. For this, tumor samples were collected at 72h after the first dosing and analysed for HSP27 gene expression levels compared to cellular control mRNA expression levels (reference genes). Tumor bearing mice (n=3) were treated (intraperitoneal; i.p.) at day 12: 25 mg/kg cetuximab-(Cys-L-SO1861)^{3,8} + 25 mg HSP27BNA and at day 15: 25 mg/kg cetuximab-(Cys-L-SO1861)^{3,8} + 10 mg HSP27BNA and this revealed a 25% reduction in HSP27 mRNA expression in the tumors compared to vehicle control or single dosing of 25 mg/kg HSP27BNA (Figure 6). This shows and enables that conjugation of SO1861 to a targeting antibody, according to the invention, efficiently induces SO1861-mediated enhanced cytoplasmic delivery of a therapeutic antisense oligo nucleotide in solid tumors of tumor bearing mice, inducing tumor targeted gene silencing, *in vivo.*

### DAR2 vs DAR4

SO1861-EMCH (labile linker, L) was conjugated via cysteine residues (Cys) to cetuximab, with DAR3.7 (cetuximab-(Cys-L-SO1861)^{3.7}) or DAR2 (cetuximab-(Cys-L-SO1861)²). These cetuximab-SO1861 conjugates were tested for enhanced cell killing activity in combination with 10pM cetuximab-saporin on high EGFR expressing cells (A431, EGFR⁺⁺). This revealed the same cell killing potency for cetuximab conjugated SO1861, DAR2 and DAR4 (Figure 22A). Same results were obtained when trastuzumab-(Cys-L-SO1861)⁴ and trastuzumab-(Cys-L-SO1861)^{2.1} were compared in combination with 50pM trastuzumab-saporin on high HER2 expressing cells (SK-BR-3, HER2⁺⁺) (Figure 22B), showing that lowering the amount of conjugated SO1861, according to the invention, reduces the potency for effective endosomal escape and toxin mediated cell killing.

### Stable vs Labile

The SO1861-HATU (stable linker, S) was conjugated via cysteine residues (Cys) to the anti-EGFR antibody cetuximab with DAR3.7 (cetuximab-(Cys-S-SO1861)^{3.7}). This cetuximab-SO1861 conjugate was combined with a fixed concentration of 10 pM of the anti-EGFR antibody-protein toxin conjugate (cetuximab-saporin) and tested for enhanced cell killing activity on high EGFR expressing cells (MDA-MB-468, EGFR⁺⁺), in comparison to labile conjugated (cetuximab-(Cys-L-SO1861)^{3.7}. This revealed that stable or labile antibody-conjugated SO1861, according to the invention, show the same enhanced cell killing activity when combined with non-effective concentrations of an antibody-protein toxin conjugate (Figure 23A). The same data were revealed when trastuzumab-(Cys-L-SO1861)⁴ was compared with trastuzumab-(Cys-S-SO1861)⁴ in combination with 50 pM trastuzumab-saporin in SK-BR-3 cells (HER2⁺⁺)(Figure 23B). All this indicates that stable or labile conjugated SO1861, according to the invention, effectively functions, inducing endosomal escape of the antibody-conjugated toxin.

### 2 target 2-component system (in vivo)

The 2 target 2-components system (2T2C) is the combination treatment of mAb1-SO1861 and mAb2-protein toxin, (Figure 37). SO1861-EMCH was conjugated via cysteine residues (Cys) to cetuximab (monoclonal antibody recognizing and binding human EGFR), with a DAR 4 resulting in the production of: cetuximab-(Cys-L-SO1861)⁴. The combination of cetuximab-(Cys-L-SO1861)⁴ and trastuzumab-saporin or CD71mab-saporin was tested in a A431 (EGFR⁺⁺/HER2^{+/-}/CD71⁺) xenograph 'nude' mouse tumor model for EGFR tumor targeted cell killing as illustrated in Figure 37. Dose escalation was performed to determine the therapeutic efficacy *(Day 9: 0.3 mg*/*kg trastuzumab-saporin or 0.1 mg*/*kg CD71mab-saporin* + *5 mg*/*kg cetuximab-(Cys-L-SO1861)⁴; Day 14, 18: 0.1 mg*/*kg trastuzumab-saporin or 0.05 mg*/*kg CD71mab-saporin* + *5 mg*/*kg cetuximab-(Cys-L-SO1861)⁴; Day 21: 0.05 mg*/*kg trastuzumab-saporin or 0.05 mg*/*kg CD71mab-saporin* + *15 mg*/*kg cetuximab-(Cys-L-SO1861)⁴; Day 28: 0.02 mg*/*kg trastuzumab-saporin or 0.02 mg*/*kg CD71mab-saporin* + *15 mg*/*kg cetuximab-(Cys-L-SO1861)⁴ trastuzumab-saporin*/*cetuximab-SO1861. Controls were on the same dosing scheme respectively, only cetuximab (i.v.) was given 25 mg*/*kg every treatment day).* At day 32 (dashed line), 35 and 39 we started the combination, according to the 2T2C invention of 25 mg/kg cetuximab-(Cys-L-SO1861)⁴ (intraperitoneal injection (i.p.) + 0.02 mg/kg trastuzumab-saporin or 0.02 CD71mab-saporin (intravenous administration, (i.v.)) and this revealed strong tumor regression for both 2T2C combination groups compared to the vehicle control, 25 mg/kg cetuximab-(Cys-L-SO1861)⁴ or 0.02 mg/kg trastuzumab-saporin/CD71mab-saporin mono therapies (Figure 24). The 2T2C system even outcompetes cetuximab, the clinically used monoclonal antibody against EGFR. Next we performed the same experiment but then we started with 25 mg/kg cetuximab-(Cys-L-SO1861)⁴ (intraperitoneal injection (i.p.) + 0.03 mg/kg trastuzumab-saporin or 0.03 CD71mab-saporin (intravenous administration, (i.v.)) treatment with a dosing at day 9 and 14 and thereafter 1 dosing per week. The 2T2C system according to the invention showed tumor regression in all mice and even in 1 mice in both 2T2C groups, complete tumor eradication (tumor volume= 0 mm³) (Figure 25). Also here the controls showed a strong increased in tumor volume whereas the positive control for this A431 mice model, cetuximab showed only tumor growth inhibition, but no regression (Figure 25). This shows and enables the 2T2C system approach, according to the invention, of cetuximab conjugated SO1861 + trastuzumab conjugated protein toxin or CD71mab conjugated protein toxin inducing highly efficient targeted delivery of a therapeutic protein toxin in the cytoplasm of solid tumors of tumor bearing mice, *in vivo,* thereby inducing even full tumor eradication in some mice and strong tumor regression in others even in large size tumors (2000 mm³).

### 2 target 2-component system (in vitro)

### Results

The 2 target 2-components system (2T2C) is the combination treatment of mAb1-SO1861 and mAb2-protein toxin, (Figure 37). SO1861-EMCH was conjugated via cysteine residues (Cys) to cetuximab (monoclonal antibody recognizing and binding human EGFR), with a DAR 3,7 (cetuximab-(Cys-L-SO1861)^{3,7}). Cetuximab-(Cys-L-SO1861)^{3,7} was titrated on a fixed concentration of 50 pM trastuzumab-saporin (trastuzumab, conjugated to the protein toxin, saporin) and targeted protein toxin mediated cell killing on EGFR/HER2 expressing cells (A431, EGFR⁺⁺/HER2^{+/-}; CaSKi, EGFR⁺/HER2^{+/-}) was determined as illustrated in Figure 37. This revealed strong cell killing at low concentrations of cetuximab-(Cys-L-SO1861)^{3,7} (A431: IC50= 3 nM and CaSKi IC50= 10 nM; Figure 26A, 26B) whereas equivalent concentrations cetuximab, cetuximab-(Cys-L-SO1861)^{3,7} or cetuximab + 50 pM trastuzumab -saporin could not induce any cell killing activity in EGFR/HER2 expressing cells. This shows that relatively low concentrations of cetuximab-SO1861 conjugate efficiently enhances endosomal escape of the trastuzumab conjugated protein toxin (at non-effective concentrations), thereby inducing efficient cell killing of high EGFR/low HER2 expressing cells.

Next, trastuzumab-saporin was titrated on a fixed concentration of 75 nM cetuximab-(Cys-L-SO1861)^{3,7} and targeted protein toxin mediated cell killing on EGFR/HER2 expressing cells was determined. This revealed that 75 nM cetuximab-(Cys-L-SO1861)^{3,7} in combination with low concentrations trastuzumab-saporin induced already efficient cell killing in EGFR/HER2 expressing cells (A431: IC50= 5 pM; and CaSKi: IC50= 1 pM; Figure 26C and 26D), whereas trastuzumab-saporin alone or trastuzumab-saporin + 75 nM cetuximab did not show significant cell killing activity (IC50> 10.000 pM) in both cell lines (Figure 26C, 26D). All this shows that relatively low concentrations of trastuzumab-saporin can be effective and induce cell killing in combination with low cetuximab-SO1861 conjugate concentrations in high EGFR/low HER2 expressing cells.

Next, cetuximab-(Cys-L-SO1861)^{3,7} was titrated on a fixed concentration of 50 pM trastuzumab-saporin and targeted protein toxin-mediated cell killing on HeLa (EGFR^{+/-}/HER2^{+/-}) or A2058 (EGFR⁻/HER2^{+/-}) was determined as illustrated in Figure 37. Both HeLa (EGFR^{+/-}/HER2^{+/-}) and A2058 (EGFR⁻/HER2^{+/-}) cells do not show cell killing at low concentrations of cetuximab-(Cys-L-SO1861)^{3,7} + 50 pM trastuzumab-saporin (HeLa: IC50= 400 nM; A2058: IC50> 400 nM; Figure 27A, 27B). This shows that in the absence of sufficient receptor expression, effective intracellular delivered SO1861 concentrations are not reached (threshold) to induce endosomal escape and cytoplasmic delivery of the protein toxin. Next, trastuzumab-saporin was titrated on a fixed concentration of 75 nM cetuximab-(Cys-L-SO1861)^{3,7} and targeted protein toxin mediated cell killing on HeLa or A2058 (EGFR⁻/HER2^{+/-}) was determined. Both HeLa (EGFR^{+/-}/HER2^{+/-}) and A2058 (EGFR⁻/HER2^{+/-}) cells showed no cell killing activity (HeLa: IC50> 10.000 pM; A2058: IC50> 10.000 pM; Figure 27C, 27D). All this shows that cells with low or no EGFR receptor expression are not susceptible for the combination of cetuximab-(Cys-L-SO1861)^{3,7}+ trastuzumab-saporin, due to a lack of sufficient EGFR receptorthat facilitates the antibody-mediated delivery of sufficient SO1861 (threshold) to ensure endosomal escape of the toxin within the cytoplasm of the cell.

Next, SO1861-EMCH was conjugated via cysteine residues (Cys) to trastuzumab (monoclonal antibody recognizing and binding human HER2), with a DAR 4 (trastuzumab-(Cys-L-SO1861)⁴). Trastuzumab-(Cys-L-SO1861)⁴ was titrated on a fixed concentration of 1.5 pM EGFdianthin (EGFR targeted ligand toxin fusion protein) and targeted protein toxin mediated cell killing on HER2/EGFR expressing cells (SK-BR-3: HER2⁺⁺/EGFR^{+/-}) was determined. This revealed strong cell killing at low concentrations of trastuzumab-(Cys-L-SO1861)⁴ + 1.5 pM EGFdianthin (SK-BR-3: IC50= 1 nM; Figure 28A) whereas equivalent concentrations trastuzumab, trastuzumab-(Cys-L-SO1861)⁴ or trastuzumab + 1.5 pM EGFdianthin could not induce any cell killing activity in HER2⁺⁺/EGFR^{+/-} expressing cells. This shows that trastuzumab conjugated SO1861 efficiently enhances endosomal escape of the EGF fusion protein toxin (at non-effective concentrations), thereby inducing cell killing of high HER2/low EGFR expressing cells.

Next, EGFdianthin was titrated on a fixed concentration of 2.5 nM trastuzumab-(Cys-L-SO1861)⁴ and targeted protein toxin mediated cell killing on SK-BR-3 (HER2⁺⁺/EGFR^{+/-}) expressing cells was determined. This revealed that 2.5 nM trastuzumab-(Cys-L-SO1861)⁴ in combination with low concentrations EGFdianthin induced already efficient cell killing in HER2/EGFR expressing cells (SK-BR-3: IC50= 1 pM) (Figure 28B), whereas EGFdianthin alone or EGFdianthin + 2.5 nM trastuzumab showed no cell killing activity (IC50>10.000 pM) (Figure 28B). All this shows that relatively low concentrations of EGFdianthin can be effective and induce cell killing only in combination with low trastuzumab-(Cys-L-SO1861)⁴ concentrations in high HER2/low EGFR expressing cells.

Next, trastuzumab-(Cys-L-SO1861)⁴ was titrated on a fixed concentration of 1.5 pM EGFdianthin and targeted protein toxin mediated cell killing on JIMT-1 (HER2^{+/-}/EGFR^{+/-}) or MDA-MB-468: HER2⁻ /EGFR⁺⁺) was determined. Both cell lines were not sensitive for any combination of trastuzumab-(Cys-L-SO1861)⁴+ 1.5 pM EGFdianthin (JIMT-1: IC50> 1000 nM; MDA-MB-468: IC50> 1000 nM; Figure 29A, 29B). This shows that in the absence of sufficient HER2 receptor expression, effective intracellular delivered SO1861 concentrations are not reached (threshold) to induce endosomal escape and cytoplasmic delivery of the protein toxin.

Next, EGFdianthin was titrated on a fixed concentration of 2.5 nM trastuzumab-(Cys-L-SO1861)⁴ and targeted protein toxin mediated cell killing on JIMT-1 (HER2^{+/-}/EGFR^{+/-}) or MDA-MB-468 (HER2⁻ /EGFR⁺⁺) was determined. Both cell lines showed cell killing at high EGFdianthin concentrations with or without 2,5 nM trastuzumab-(Cys-L-SO1861)⁴ (JIMT-1: IC50= 10.000 pM; MDA-MB-468: IC50= 200 pM Figure 29C, 29D).

All this shows that cells with low or no HER2 receptor expression are not susceptible for the combination of trastuzumab-(Cys-L-SO1861)^{3,7}+ 1.5 pM EGFdianthin, due to a lack of sufficient HER2 receptor that facilitates the antibody-mediated delivery of sufficient SO1861 (threshold) to ensure endosomal escape of the toxin within the cytoplasm of the cell.

Next, SO1861-EMCH was conjugated via cysteine residues (Cys) to trastuzumab (monoclonal antibody recognizing and binding human HER2), with a DAR 4, (trastuzumab-(Cys-L-SO1861)⁴). Trastuzumab-(Cys-L-SO1861)⁴ was titrated on a fixed concentration of 5 pM cetuximab-saporin (EGFR targeting antibody-protein toxin conjugate) and targeted protein toxin mediated cell killing on HER2/EGFR expressing cells (SK-BR-3: HER2⁺⁺/EGFR^{+/-}) was determined as illustrated in Figure 37. This revealed strong cell killing at low concentrations oftrastuzumab-(Cys-L-SO1861)⁴ + 5 pM cetuximab-saporin (SK-BR-3: IC50= 1 nM; Figure 30A) whereas equivalent concentrations trastuzumab, trastuzumab-(Cys-L-SO1861)⁴ or trastuzumab + 5 pM cetuximab-saporin could not induce any cell killing activity in HER2⁺⁺/EGFR^{+/-} expressing cells. This shows that trastuzumab conjugated SO1861 efficiently enhances endosomal escape of the cetuximab conjugated protein toxin (at non-effective concentrations), thereby inducing cell killing of HER2⁺⁺/EGFR^{+/-} expressing cells.

Next, cetuximab-saporin was titrated on a fixed concentration of 2.5 nM trastuzumab-(Cys-L-SO1861)⁴ and 75 nM trastuzumab-(Cys-L-SO1861)⁴ and targeted protein toxin mediated cell killing on HER2/EGFR expressing cells (SK-BR-3: HER2⁺⁺/EGFR^{+/-}) was determined. This revealed that 2.5 nM trastuzumab-(Cys-L-SO1861)⁴ in combination with low concentrations cetuximab-saporin induced already efficient cell killing in SK-BR-3 cells (SK-BR-3: IC50= 1 pM; Figure 30B), whereas cetuximab-saporin alone or cetuximab-saporin + 2.5 nM trastuzumab showed cell killing only at high concentrations trastuzumab-saporin (SK-BR-3: IC50> 4000 pM; Figure 30B). All this shows that relatively low concentrations of cetuximab-saporin can be effective and induce cell killing only in combination with low trastuzumab-(Cys-L-SO1861)⁴ concentrations in HER2⁺⁺/EGFR^{+/-} expressing cells.

Next, trastuzumab-(Cys-L-SO1861)⁴ was titrated on a fixed concentration of 5 pM cetuximab-saporin and targeted protein toxin mediated cell killing on JIMT-1 (HER2^{+/-}/EGFR^{+/-}) and MDA-MB-468 (HER2⁻ /EGFR⁺⁺) cells was determined. Both cell lines were not sensitive for the combination of trastuzumab-(Cys-L-SO1861)⁴+ 5 pM cetuximab-saporin (JIMT-1: IC50> 1000 nM; MDA-MB-468: IC50> 1000 nM; Figure 31A, 31B). This shows that in the absence of sufficient HER2 receptor expression, effective intracellular delivered SO1861 concentrations are not reached (threshold) to induce endosomal escape and cytoplasmic delivery of the protein toxin.

Next, cetuximab-saporin was titrated on a fixed concentration of 2.5 nM trastuzumab-(Cys-L-SO1861)⁴ and targeted protein toxin mediated cell killing on JIMT-1 (HER2^{+/-}/EGFR^{+/-}) and MDA-MB-468 (HER2⁻ /EGFR⁺⁺) cells was determined. Both cell lines showed cell killing at similar cetuximab-saporin concentrations with or without 2.5 nM trastuzumab-(Cys-L-SO1861)⁴ (JIMT-1: IC50= 80pM; MDA-MB-468: IC50= 100 pM; Figure 31C, 31D).

All this shows that cells with low or no HER2 receptor expression are not susceptible for the combination of trastuzumab-(Cys-L-SO1861)⁴ + cetuximab-saporin, due to a lack of sufficient HER2 receptor that facilitates the antibody-mediated delivery of sufficient SO1861 (threshold) to ensure endosomal escape of the toxin within the cytoplasm of the cell.

In orderto show that the activity of the conjugated SO1861 is driven by the acidification of the endosomal compartments, the 2T2 components system, according to the invention was tested in combination with an endosomal acidification inhibitor, chloroquine. Trastuzumab-saporin + 77 nM cetuximab-(Cys-L-SO1861)^{3,9} or trastuzumab-dianthin + 77 nM cetuximab-(Cys-L-SO1861)^{3,9} showed strong cell killing activity in A431 (EGFR⁺⁺/HER2^{+/-}) cells, whereas this 2T2C activity, according to the invention, was inhibited when 800 nM chloroquine was co-administrated to both combinations (Figure 32A). Same results were observed when CD71mab-saporin + 10.5 nM cetuximab-(Cys-L-SO1861)^{3,9} + 500 nM chloroquine was tested in A431 (EGFR⁺⁺/CD71⁺) and MDA-MB-468 (EGFR⁺⁺/CD71⁺) cells (Figure 32B, 9C) or when CD71mab-saporin + 5 nM trastuzumab-(Cys-L-SO1861)⁴ + 500 nM chloroquine was tested in SK-BR-3 (HER2⁺⁺/CD71⁺) cells (Figure 32D). This shows that the intracellular activity of conjugated SO1861 within the 2T2C system can be inhibited when acidification of endosomes is blocked.

The 2 target 2-components system (2T2C) is also the combination treatment of mAb1-SO1861 and mAb2-antisense BNA oligo nucleotide, (Figure 38). Therefore, the 2T2C system was also tested in combination with an antisense BNA oligonucleotide against the mRNA of a cancer specific target gene, heat shock protein 27 (HSP27). Upon release into the cytoplasm the antisense BNA recognizes and binds the mRNA encoding for HSP27, targeting the mRNA for destruction thereby depleting the HSP27 expression within the cancer cell. HSP27BNA was conjugated to trastuzumab with a DAR4.4 (trastuzumab-(Lys-L-HSP27BNA)^{4,4}) and tested in combination with Cetuximab-(Cys-L-SO1861)^{3,9} for enhanced HSP27 gene silencing activity in A431 (EGFR⁺⁺/HER2^{+/-}) cells and A2058 (EGFR⁻/HER2^{+/-}) cells as illustrated in Figure 38. Cetuximab-(Cys-L-SO1861)^{3,9} was titrated on a fixed concentration of 100 nM Trastuzumab-(Lys-L-HSP27BNA)^{4,4} and targeted HSP27BNA-mediated gene silencing activity was determined. Cetuximab-(Cys-L-SO1861)^{3,9} + 100 nM Trastuzumab-(Lys-L-HSP27BNA)^{4,4} show strong gene silencing activity in A431 cells (EGFR⁺⁺/HER2^{+/-}) (A431: IC50= 1 nM; Figure 33A), compared to Cetuximab-(Cys-L-SO1861)^{3,9} alone. In A2058 cells (EGFR⁻/HER2^{+/-}), the combination according to the invention showed no HSP27 gene silencing (A2058: IC50> 100nM; Figure 33B). This shows that cetuximab conjugated SO1861 efficiently enhances endosomal escape of the trastuzumab conjugated BNA oligo nucleotide (at non-effective concentrations), thereby inducing target gene silencing in EGFR⁺⁺/HER2^{+/-} expressing cells.

Next, Trastuzumab-(Lys-L-HSP27BNA)^{4,4} was titrated on a fixed concentration of Cetuximab-(Cys-L-SO1861)^{3,9} and targeted HSP27BNA-mediated gene silencing activity was determined in A431 (EGFR⁺⁺/HER2^{+/-}) cells and A2058 (EGFR⁻/HER2^{+/-}) cells as illustrated in Figure 38. Trastuzumab-(Lys-L-HSP27BNA)^{4,4} + 77 nM Cetuximab-(Cys-L-SO1861)^{3,9} show strong gene silencing activity in A431 cells (EGFR⁺⁺/HER2^{+/-}) (A431: IC50= 1 nM; Figure 33C), whereas trastuzumab-(Lys-L-HSP27BNA)^{4,4} alone or Cetuximab-(Cys-L-SO1861)^{3,9} alone or trastuzumab-(Lys-L-HSP27BNA)^{4,4} + 77 nM cetuximab did not reveal any significant gene silencing activity (IC50>100nM). A2058 (EGFR⁻/HER2^{+/-}) cells did not show any gene silencing activity in the combination according to the invention (A2058: IC50> 100nM; Figure 33D). All this shows that relatively low concentrations of trastuzumab-HSP27BNA can be effective and induce cell killing only in combination with low concentrations of cetuximab-(-L-SO1861) concentrations in HER2⁺⁺/EGFR^{+/-} expressing cells.

The 2 target 2-components system (2T2C) can also be the combination treatment of mAb1-(dendron(-SO1861)ⁿ)ⁿ and mAb2-protein toxin (Figure 39). Dendron(-L-SO1861)⁴ was conjugated to the anti-EGFR antibody, cetuximab via cysteine residues (Cys) with a DAR3,9, (cetuximab-Cys-(dendron(-L-SO1861)⁴)^{3,9}) and tested for enhanced cell killing activity in combination with an anti-CD71 antibody protein toxin conjugate (CD71mab-saporin) in MDA-MB-468 (EGFR⁺⁺/CD71⁺) expressing cells as illustrated in Figure 39. Cetuximab-Cys-(dendron(-L-SO1861)⁴)^{3,9} + 10 pM CD71mab-saporin efficiently induces toxin-mediated cell killing in MDA-MB-468 (EGFR⁺⁺/CD71⁺) expressing cells (IC50= 0.4 nM, Figure 34A), whereas this could not be induced by Cetuximab-Cys-(dendron(-L-SO1861)⁴)^{3,9}) or cetuximab + 10 pM CD71 mab-saporin or cetuximab (Figure 34A). This shows that cetuximab conjugated dendron(-L-SO1861)⁴ efficiently enhances endosomal escape of the CD71mab-protein toxin (at non-effective concentrations), thereby inducing cell killing of EGFR⁺⁺/CD71⁺ expressing cells. Similar experiments were performed in HeLa cells (HER2^{+/-}/CD71⁺) cells and this revealed no activity of cetuximab-Cys-(dendron(-L-SO1861)⁴)^{3,9}) + 10 pM CD71mab-saporin (IC50> 100 nM Figure 34B) indicating that in the absence of sufficient EGFR receptor expression, effective intracellular SO1861 concentrations are not reached (threshold) to induce endosomal escape and cytoplasmic delivery of the protein toxin.

Next, dendron(-L-SO1861)⁴ was conjugated to the anti-HER2 antibody, trastuzumab via cysteine conjugation (Cys) with a DAR4, trastuzumab-Cys-(dendron(-L-SO1861)⁴)⁴ and tested for enhanced cell killing activity in combination with an anti-CD71 antibody protein toxin conjugate (CD71mab-saporin) in SK-BR-3 cells (HER2⁺⁺/CD71⁺) expressing cells. Trastuzumab-Cys-(dendron(-L-SO1861)⁴)⁴ + 10 pM CD71mab-saporin efficiently induces toxin-mediated cell killing in SK-BR3 cells (IC50= 3 nM, Figure 34C), whereas this was not induced by trastuzumab-Cys-(dendron(-L-SO1861)⁴)⁴ or trastuzumab (equivalent) + 10 pM CD71mab-saporin or trastuzumab (Figure 34C). This shows that trastuzumab conjugated dendron(-L-SO1861)⁴, according to the invention efficiently enhances endosomal escape of the CD71mab-protein toxin (at non-effective concentrations), thereby inducing cell killing of HER2⁺⁺/CD71⁺ expressing cells. Similar experiments were performed in JIMT-1 cells (HER2^{+/-}/CD71⁺) and this revealed no activity of trastuzumab-Cys-(dendron(-L-SO1861)⁴)⁴ + 10 pM CD71mab-saporin (IC50> 100 nM Figure 34C) indicating that in the absence of sufficient HER2 receptor expression, effective intracellular SO1861 concentrations are not reached (threshold) to induce endosomal escape and cytoplasmic delivery of the protein toxin.

The clinical approved ADC, trastuzumab-emtansine (T-DM1) is a conjugate of the anti-Her2 antibody, trastuzumab and the small molecule toxin emtansine (DAR3-4). T-DM1 was tested within the 2T2C system, according to the invention in combination with cetuximab-(Cys-L-SO1861)⁴. T-DM1 + 77 nM cetuximab-(Cys-L-SO1861)^{3,9} showed no enhanced cell killing activity compared to T-DM1 alone or T-DM1 + 77 nM cetuximab (IC50= 80.000 pM, Figure 35), whereas trastuzumab-saporin + 75 nM cetuximab-(Cys-L-SO1861)^{3,7}, according to the invention showed enhanced cell killing activity compared to trastuzumab-saporin + 75 nM cetuximab or trastuzumab-saporin alone (IC50=3 pM, Figure 35). All this shows that the 2T2C system does not enhance the delivery of antibody conjugated small molecules, that are already able to passively cross cellular (endosomal) membranes.

### Results

The 2 target 2-components system (1T2C) can also be the combination treatment of mAb1-QSmix (mixture of saponins from Quillaja Saponaria) and mAb2-protein toxin.

QSmix-EMCH was conjugated via cysteine residues (Cys) to cetuximab (monoclonal antibody recognizing and binding human EGFR), with a DAR 4,1 (cetuximab-(Cys-L-QSmix)^{4,1}). Cetuximab-(Cys-L-QSmix)^{4,1} was titrated on a fixed concentration of 10 pM trastuzumab-saporin or CD71mab-saporin and targeted protein toxin mediated cell killing on A431 (EGFR⁺⁺/HER2^{+/-}/CD71⁺) and CaSKi (EGFR⁺/HER2^{+/-}/CD71⁺) was determined. This revealed strong cell killing at low concentrations of Cetuximab-(Cys-L-QSmix)^{4,1} + 10 pM trastuzumab-saporin (A431: IC50= 100 nM; Figure 36A CaSKi: IC50= 10 nM Figure 36B) as well as with Cetuximab-(Cys-L-QSmix)^{4,1} + 10 pM CD71mab-saporin (A431: IC50= 3 nM, Figure 36A; CaSKi: IC50= 0.8 nM, Figure 36B) whereas cetuximab, cetuximab-(Cys-L-QSmix)^{4,1} or cetuximab + 10 pM trastuzumab-saporin or cetuximab + 10 pM CD71mab-saporin could not induce any cell killing in EGFR⁺⁺/HER2^{+/-}/CD71⁺ or EGFR⁺/HER2^{+/-}/CD71⁺ expressing cells. This shows that relatively low concentrations of cetuximab-QSmix conjugate efficiently enhances endosomal escape of the trastuzumab conjugated protein toxin as well as the CD71mab conjugated protein toxin (at non-effective concentrations), thereby inducing efficient cell killing of EGFR⁺⁺/HER2^{+/-} /CD71⁺ or EGFR⁺/HER2^{+/-}/CD71⁺ expressing cells. When the same experiment was performed on A2058 cells (EGFR⁻/HER2^{+/-}/CD71⁺) no activity of cetuximab-(Cys-L-QSmix)^{4,1} + 10 pM trastuzumab-saporin or cetuximab-(Cys-L-QSmix)^{4,1} + 10 pM CD71mab-saporin could be observed (Figure 36C), indicating that in the absence of sufficient EGFR receptor expression, effective intracellular delivered QS saponin concentrations are not reached (threshold) to induce endosomal escape and cytoplasmic delivery of the protein toxin.

### Example 5

SO1861 was conjugated (labile) via cysteine residues (Cys) and dianthin (protein toxin) was conjugated (stable) via lysine residues (Lys) to cetuximab (monoclonal antibody recognizing and binding human EGFR), resulting in the production of: Cetuximab-(Cys-L-SO1861)^{3,9}(Lys-S-dianthin)². The conjugate was tested in a A431 (EGFR⁺⁺) xenograph mouse tumor model for EGFR tumor targeted cell killing as illustrated in Figure 47. Dosings started at day 12 when tumors reached ∼150mm³ in size and tumor volume was determined after every dosing. Mice (n=3) were treated (intraperitoneal; i.p.; dose escalation) at day 12: 0.5 mg/kg; day15: 1 mg/kg and day24: 1.5 mg/kg with cetuximab-(Cys-L-SO1861)^{3,9}(Lys-S-dianthin)² or cetuximab-(Lys-S-dianthin)^{1,6}. At day 26, compared to the control group, tumor volume reduction could be observed in the tumor bearing mice treated with cetuximab-(Cys-L-SO1861)^{3,9}(Lys-S-dianthin)² (Figure 40A). This shows that labile conjugation of SO1861 to an antibody-protein toxin (stable) conjugate can enhance the targeted therapeutic efficacy of the tumor targeted antibody-protein toxin, thereby inducing a more effective tumor targeted therapy.

Next, SO1861 was conjugated (labile) via cysteine residues (Cys) and dianthin (protein toxin) was conjugated (labile) via lysine residues (Lys) to cetuximab (monoclonal antibody recognizing and binding human EGFR), resulting in the production of: Cetuximab-(Cys-L-SO1861)^{3,9}(Lys-L-dianthin)². The conjugate was tested in a A431 (EGFR⁺⁺) xenograph mouse tumor model for EGFR tumor targeted cell killing as illustrated in Figure 47. Dosings started at day 12 when tumors reached ∼150mm³ in size and tumor volume was determined after every dosing. Mice (n=3) were treated (intraperitoneal; i.p.; dose escalation) at day 12: 0.5 mg/kg; day15: 1 mg/kg, day24: 1.5 mg/kg with cetuximab-(Cys-L-SO1861)^{3,9}(Lys-L-dianthin)² or cetuximab-(Lys-L-dianthin)^{1,6}. This revealed that after 35 days compared to the control, tumor bearing mice treated with cetuximab-(Cys-L-SO1861)^{3,9}(Lys-L-dianthin)² showed tumor growth inhibition (Figure 40B). When mice (n=3; were treated (intravenous, i.v.; dose escalation) day 12: 0.5 mg/kg; day15: 1 mg/kg, day18: 2 mg/kg, day24: 2.5 mg/kg with the cetuximab-(Cys-L-SO1861)^{3,9}(Lys-L-dianthin)² according to the invention also tumor growth inhibition could be observed compared to the control (data represents 1 mice, since 2 mice died during the treatments). This shows that labile conjugation of SO1861 to an antibody-protein toxin (labile) conjugate can enhance the targeted therapeutic efficacy of the tumor targeted antibody-protein toxin, thereby inducing a more effective tumor targeted therapy.

Next, SO1861-EMCH was conjugated via cysteine residues (Cys) to cetuximab (monoclonal antibody recognizing and binding human EGFR), with a DAR 3,9 and the antisense HSP27BNA oligo nucleotide (targeting and inducing degradation of the onco-target *hsp27* mRNA (gene silencing) in cancer cells) via a labile (L) linker to the lysine residues (Lys) of the antibody, with a DAR 1,8 resulting in the production of cetuximab-(Cys-L-SO1861)^{3,9}(Lys-L-HSP27BNA)^{1,8}. Cetuximab-(Cys-L-SO1861)^{3,9}(Lys-L-HSP27BNA)^{1,8} was tested in a A431 xenograph 'nude' mouse tumor model for EGFR-mediated tumor targeted HSP27 gene silencing, according to the invention as illustrated in Figure 48. Dosing started at day 12 when tumors reached ∼150mm³ in size and HSP27 mRNA expression was determined. For this, tumor samples were collected at 72h after the first dosing and analysed for HSP27 gene expression levels compared to cellular control mRNA expression levels (reference genes). Tumor bearing mice (n=3) treated (intraperitoneal; i.p.) with 30 mg/kg cetuximab-(Cys-L-SO1861)^{3,9}(Lys-L-HSP27BNA)^{1,8} showed after 1 dosing 40% reduction in HSP27 mRNA expression in the tumors compared to single dosing of cetuximab-(Cys-L-SO1861)^{3,8} or cetuximab-(Lys-L-HSP27BNA)^{1,5} (Figure 41). Compared to the tumor of the vehicle control a reduction of 25% HSP27 gene expression was observed. This shows and enables that conjugation of SO1861 and HSP27BNA to the same targeting antibody, according to the invention, efficiently induces SO1861-mediated enhanced cytoplasmic delivery of a therapeutic antisense oligo nucleotide in solid tumors of tumor bearing mice, inducing tumortargeted gene silencing. In another example, a trifunctional linker scaffold was designed and produced with 3 specific chemical end groups for conjugation with SO1861 on one arm and the HSP27BNA on the other arm to produce SO1861-L-trifunctional linker-L-HSP27BNA. Next, SO1861-L-trifunctional linker-L-HSP27BNA was conjugated with its third arm to cysteine residues (Cys) of the anti-EGFR antibody, cetuximab (cetuximab-Cys-(SO1861-L-trifunctional linker-L-HSP27BNA)^{3,7}) and tested in a A431 xenograph 'nude' mouse tumor model for EGFR-mediated tumor targeted gene silencing activity, according to the invention as illustrated in Figure 49. Dosings started at day 12 when tumors reached ∼150mm³ in size and HSP27 mRNA expression was determined. For this, tumor samples were collected at 72h after the first dosing and analysed for HSP27 gene expression levels compared to cellular control mRNA expression levels (reference genes). This revealed that 1 dosing of 30 mg/kg cetuximab-Cys-(SO1861-L-trifunctional linker-L-HSP27BNA)^{3,7} resulted in a 40% reduction in HSP27 gene expression in the tumors compared to single dosing of 25 mg/kg cetuximab-(Cys-L-SO1861)^{3,8} or 25 mg/kg cetuximab-(Lys-L-HSP27BNA)⁴ mono therapies (Figure 42). Compared to the vehicle control tumors, a reduction of 25% HSP27 gene expression was observed in tumor bearing mice treated with 1 dosing of cetuximab-Cys-(SO1861-L-trifunctional linker-L-HSP27BNA)^{3,7}. This shows and enables that cetuximab-Cys-(SO1861-L-trifunctional linker-L-HSP27BNA)^{3,7} efficiently induces SO1861-mediated enhanced cytoplasmic delivery of a therapeutic antisense oligo nucleotide in a solid tumor of tumor bearing mice, inducing targeted gene silencing, *in vivo.*

In another example according to the invention, SO1861 (labile) and the protein toxin, dianthin (labile or stable) were conjugated to the HER2 targeting antibody, trastuzumab. Trastuzumab-(Cys-L-S0O1861)^{3,8}(Lys-L-dianthin)^{1,7} or trastuzumab-(Cys-L-SO1861)^{3,8}(Lys-S-dianthin)^{1,7}, were produced and tested for enhanced cell killing in SK-BR-3 (HER2⁺⁺) and MDA-MB-468 (HER2⁻) cells as illustrated in Figure 47. Both, trastuzumab-(Cys-L-SO1861)^{3,8}(Lys-L-dianthin)^{1,7} (IC50= 0,8 nM) and trastuzumab-(Cys-L-SO1861)^{3,8}(Lys-S-dianthin)^{1,7} (IC50= 0,8 nM) efficiently induces cell killing of SK-BR-3 cells (HER2⁺⁺) (Figure 43A). This was not observed in SK-BR-3 cells treated with trastuzumab, trastuzumab-(Lys-L-dianthin)^{1,7}, trastuzumab-(Lys-S-dianthin)^{1,7} or trastuzumab-(L-SO1861)^{3,8} alone (Figure 43A). In MDA-MB-468 cells (HER2⁻) no cell killing activity can be observed for any of the conjugates, according to the invention (Figure 43B). This shows that conjugation of SO1861 to an HER targeting antibody-protein toxin conjugate, efficiently induces SO1861-mediated enhanced cytoplasmic delivery of the protein toxin in the target cell resulting in target cell death.

In another example according to the invention, SO1861 (labile) and the protein toxin, dianthin (labile or stable) were conjugated to the EGFR targeting antibody, cetuximab. Cetuximab-(Cys-L-SO1861)^{3,9}(Lys-L-dianthin)² or cetuximab-(Cys-L-SO1861)^{3,9}(Lys-S-dianthin)², was tested for enhanced cell killing in A431 cells (EGFR⁺⁺) and A2058 cells (EGFR) as illustrated in Figure 47. Both, cetuximab-(Cys-L-SO1861)^{3,9}(Lys-L-dianthin)² (IC50= 0,3 nM) and cetuximab-(Cys-L-SO1861)^{3,8}(Lys-S-dianthin)^{1,7}(IC50= 0,3 nM) showed enhanced cell killing in A431 cells (EGFR⁺⁺) compared to cetuximab-(Lys-L-dianthin)^{1,6} (IC50= 2pM), cetuximab-(Lys-S-dianthin)^{1,6} (IC5= 2pM) alone (Figure 43C). In A2058 cells (EGFR⁻) the combination according to the invention did not show any cell killing activity (IC50> 200nM; Figure 43D). This shows that conjugation of SO1861 to an EGFR targeting antibody-protein toxin conjugate, efficiently enhances SO1861-mediated cytoplasmic delivery of the protein toxin in the target cell resulting in enhanced target cell death.

In another example according to the invention, SO1861 (labile) and the HSP27BNA oligo (labile) were conjugated to the EGFR targeting antibody, cetuximab. Cetuximab-(Cys-L-SO1861)^{3,8}(Lys-L-HSP27BNA)^{3,8} was tested for enhanced HSP27 gene silencing in A431 cells (EGFR⁺⁺) and A2058 (EGFR) cells, according to the invention as illustrated in Figure 48. Cetuximab-(Cys-L-SO1861)^{3,8}(Lys-L-HSP27BNA)^{3,8} efficiently induces HSP27 gene silencing in A431 cells (IC50= 3nM) compared to cetuximab, cetuximab-(Lys-L-HSP27BNA)^{3,9} or cetuximab-(Cys-L-SO1861)^{3,8} alone (Figure 44A). In A2058 cells (EGFR) no gene silencing activity can be observed with cetuximab-(Cys-L-SO1861)^{3,8}(Lys-L-HSP27BNA)^{3,8} (IC50> 100nM; Figure 44B). This shows and enables that conjugation of SO1861 and HSP27BNA to the same targeting antibody, according to the invention, efficiently induces SO1861-mediated enhanced cytoplasmic delivery of a therapeutic antisense oligo nucleotide in the target cells, inducing targeted gene silencing.

In another example according to the invention, SO1861 (labile) and the HSP27BNA oligo (labile) were conjugated to the HER2 targeting antibody, trastuzumab. Trastuzumab-(Cys-L-SO1861)^{3,8}(Lys-L-HSP27BNA)^{3,5} was tested for enhanced HSP27 gene silencing in SK-BR-3 cells (HER2⁺⁺) cells, according to the invention as illustrated in Figure 48. Trastuzumab-(Cys-L-SO1861)^{3,8}(Lys-L-HSP27BNA)^{3,5} efficiently induces HSP27 gene silencing in SK-BR-3 cells (IC50= 9 nM) compared to trastuzumab-(Lys-L-HSP27BNA)^{4,4} alone (Figure 45). This shows and enables that conjugation of SO1861 and HSP27BNA to an HER2 targeting antibody, according to the invention, efficiently induces SO1861-mediated enhanced cytoplasmic delivery of a therapeutic antisense oligo nucleotide in the target cells, inducing targeted gene silencing.

In another example, cetuximab-Cys-(SO1861-L-trifunctional linker-L-HSP27BNA)^{3,7} was tested for enhanced HSP27 gene silencing in A431 (EGFR⁺⁺) and A2058 (EGFR⁻) cells according to the invention as illustrated in Figure 49. Cetuximab-Cys-(SO1861-L-trifunctional linker-L-HSP27BNA)^{3,7} efficiently induces HSP27 gene silencing in A431 cells (IC50= 2nM) compared to Cetuximab-(Lys-L-HSP27BNA)⁴ or Cetuximab-(Cys-L-SO1861)^{3,7} alone (Figure 46A). In A2058 cells (EGFR⁻) gene silencing activity was only observed at high (> 80nM) concentrations of Cetuximab-Cys-(SO1861-L-trifunctional linker-L-HSP27BNA)^{3,7} (IC50=100nM; Figure 46B). This shows and enables that in high EGFR expressing cells cetuximab-Cys-(SO1861-L-trifunctional linker-L-HSP27BNA)^{3,7} efficiently induces SO1861-mediated enhanced cytoplasmic delivery of a therapeutic antisense oligo nucleotide in the target cells, inducing targeted gene silencing.

### Example 6

Figure 50A-D displays the relative cell viability when trastuzumab (Figure 50A), cetuximab (Figure 50B) or T-DM1 (Figure 50C), unconjugated protein toxins, saporin, dianthin and saporin conjugated to a (non-cell binding) IgG antibody (Figure 50D) are administrated to various cancer cell lines SK-BR-3, JIMT-1, MDA-MB-468, A431, CaSki, HeLa, A2058.

Trastuzumab and cetuximab do not or hardly influence cell viability when exposed to most of the cell lines, with some effect on cell growth inhibition via blocking the function of the HER2 growth factor receptor when trastuzumab is exposed to SK-BR-3 cells at relatively high dose and with some effect on cell growth inhibition via blocking the function of the EGFR growth factor receptor when cetuximab is exposed to MDA-MB-468 cells at relatively high dose.

TDM-1, or ado-trastuzumab emtansine, is a targeted therapy approved by the U.S. Food and Drug Administration to treat: HER2-positive metastatic breast cancer that has previously been treated with Herceptin (chemical name: trastuzumab) and taxane chemotherapy; early-stage HER2-positive breast cancer after surgery if residual disease was found after neoadjuvant (before surgery) treatment with Herceptin and taxane chemotherapy. The TDM-1 is a combination of Herceptin (Trastuzumab) and the chemotherapy medicine emtansine. Figure 8C shows that the TDM-1 results in decreased cell viability for all cell lines tested at >1000 pM concentrations

The free toxins saporin and dianthin and the toxin saporin coupled to a control IgG with no affinity for any of the cell surface molecules on the cell lines tested, do not or hardly have any influence on cell viability over a wide range of concentrations toxin tested, up to 100.000 pM (Figure 50D).

### Example 7

### dendron(-L-SO1861)ⁿ synthesis (Figure 52, 53, 54)

### materials and methods

### Abbreviations

- DCM: dichloromethane
- DIPEA: *N,N*-diisopropylethylamine
- DMF: *N,N*-dimethylformamide
- EDCl.HCl: 3-((Ethylimino)methyleneamino)-N,N-dimethylpropan-1-aminium chloride
- EMCH.TFA: N-(ε-maleimidocaproic acid) hydrazide, trifluoroacetic acid salt
- min: minutes
- r.t.: retention time
- TCEP: tris(2-carboxyethyl)phosphine hydrochloride
- Temp: temperature
- TFA: trifluoroacetic acid
- THF: tetrahydrofuran

### Analytical methods

### LC-MS method 1, ¹

Apparatus: Agilent 1200 Bin. Pump: G1312A, degasser; autosampler, ColCom, DAD: Agilent G1316A, 210, 220 and 220-320 nm, PDA: 210-320 nm, MSD: Agilent LC/MSD G6130B ESI, pos/neg 100-1000; ELSD Alltech 3300 gas flow 1.5 ml/min, gas temp: 40°C; column: Waters XSelect™ CSH C18, 30×2.1mm, 3.5µm, Temp: 35 °C, Flow: 1 mL/min, Gradient: to = 5% A, t_{1.6min} = 98% A, t₃ₘᵢₙ = 98% A, Posttime: 1.3 min, Eluent A: 0.1% formic acid in acetonitrile, Eluent B: 0.1% formic acid in water.

### LC-MS method 2, ²

Apparatus: Agilent 1260 Bin. Pump: G7112B, Multisampler, Column Comp, DAD: Agilent G7115A, 210, 220 and 220-320 nm, PDA: 210-320 nm, MSD: Agilent LC/MSD G6130B ESI, mass ranges depending on the molecular weight of the product:
^{A}pos/neg 100-1000
^{B}pos/neg 100-1400
; ELSD Alltech 3300 gas flow 1.5 ml/min, gas temp: 40°C; column: Waters XSelect™ C18, 30×2.1mm, 3.5µm, Temp: 40 °C, Flow: 1 mL/min, Gradient: to = 5% A, t_{1.6min} = 98% A, t₃ₘᵢₙ = 98% A, Posttime: 1.3 min, Eluent A: 0.1% formic acid in acetonitrile, Eluent B: 0.1% formic acid in water.

### LC-MS method 3, ³

Apparatus: Waters IClass; Bin. Pump: UPIBSM, SM: UPISMFTN with SO; UPCMA, PDA: UPPDATC, 210-320 nm, SQD: ACQ-SQD2 ESI, pos/neg 800-1500; ELSD: gaspressure 40 psi, drift tube temp: 50°C; column: Waters XSelect™ CSH C18, 50×2.1mm, 25µm Temp: 25°C, Flow: 0.6 mL/min, Gradient: to = 5% A, t_{2.0min} = 98% A, t_{2.7min} = 98% A, Posttime: 0.3 min, Eluent A: acetonitrile, Eluent B: 10 mM ammonium bicarbonate in water (pH=9.5).

### LC-MS method 4, ⁴

Apparatus: Waters IClass; Bin. Pump: UPIBSM, SM: UPISMFTN with SO; UPCMA, PDA: UPPDATC, 210-320 nm, SQD: ACQ-SQD2 ESI, pos/neg 1500-2500; ELSD: gas pressure 40 psi, drift tube temp: 50°C; column: Waters XSelect™ CSH C18, 50×2.1mm, 25µm Temp: 25°C, Flow: 0.6 mL/min, Gradient: to = 15% A, t_{2.0min} = 60% A, t_{2.7min} = 98% A, Posttime: 0.3 min, Eluent A: acetonitrile, Eluent B: 10 mM ammonium bicarbonate in water (pH=9.5).

### LC-MS method 5, ⁵

Apparatus: Waters IClass; Bin. Pump: UPIBSM, SM: UPISMFTN with SO; UPCMA, PDA: UPPDATC, 210-320 nm, SQD: ACQ-SQD2 ESI, mass ranges depending on the molecular weight of the product:
^{A}pos/neg 1500-2500
^{B}neg 2000-3000
; ELSD: gaspressure 40 psi, drift tube temp: 50°C; column: Acquity C18, 50×2.1mm, 1.7pm Temp: 60°C, Flow: 0.6 mL/min, Gradient: to = 2% A, t_{5.0min} = 50% A, t_{6.0min} = 98% A, Posttime: 1.0 min, Eluent A: acetonitrile, Eluent B: 10 mM ammonium bicarbonate in water (pH=9.5).

### Preparative methods

### Preparative MP-LC method 1, ¹

Instrument type: Reveleris™ prep MPLC; column: Waters XSelect™ CSH C18 (145x25 mm, 10µ); Flow: 40 mL/min; Column temp: room temperature; Eluent A: 10 mM ammoniumbicarbonate in water pH = 9.0); Eluent B: 99% acetonitrile + 1% 10 mM ammoniumbicarbonate in water; Gradient: t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B; Detection UV: 210, 225, 285 nm.

### Preparative MP-LC method 2, ²

Instrument type: Reveleris™ prep MPLC; Column: Phenomenex LUNA C18(3) (150x25 mm, 10µ); Flow: 40 mL/min; Column temp: room temperature; Eluent A: 0.1% (v/v) Formic acid in water, Eluent B: 0.1% (v/v) Formic acid in acetonitrile; Gradient: t₀ₘᵢₙ = 5% B, t₁ₘᵢₙ = 5% B, t₂ₘᵢₙ = 10% B, t₁₇ₘᵢₙ = 50% B, t₁₈ₘᵢₙ = 100% B, t₂₃ₘᵢₙ = 100% B; Detection UV : 210, 225, 285 nm.

### Preparative LC-MS method 1, ³

MS instrument type: Agilent Technologies G6130B Quadrupole; HPLC instrument type: Agilent Technologies 1290 preparative LC; Column: Waters XSelect™ CSH (C18, 100×30mm, 10µ); Flow: 25 ml/min; Column temp: room temperature; Eluent A: 100% acetonitrile; Eluent B: 10 mM ammonium bicarbonate in water pH=9.0; lin. gradient depending on the polarity of the product:
^{A}t₀ = 20% A, t₂ₘᵢₙ = 20% A, t_{8.5min} = 60% A, t₁₀ₘᵢₙ = 100% A, t₁₃ₘᵢₙ = 100% A
^{B}t₀ = 5% A, t₂ₘᵢₙ = 5% A, t_{8.5min} = 40% A, t₁₀ₘᵢₙ = 100% A, t₁₃ₘᵢₙ = 100% A
^{C}t₀ = 10% A, t₂ₘᵢₙ = 10% A, t_{8.5min} = 50% A, t₁₀ₘᵢₙ = 100% A, t₁₃ₘᵢₙ = 100% A
; Detection: DAD (220-320 nm); Detection: MSD (ESI pos/neg) mass range: 100 - 800; Fraction collection based on DAD.

### Preparative LC-MS method 2, ⁴

MS instrument type: Agilent Technologies G6130B Quadrupole; HPLC instrument type: Agilent Technologies 1290 preparative LC; column: Waters XBridge Shield (C18, 150×19mm, 5µ); Flow: 25 ml/min; Column temp: room temperature; Eluent A: 100% acetonitrile; Eluent B: 10 mM ammonium bicarbonate in water pH=9.0; lin. gradient: t₀ = 5% A, t_{2.5min} = 5% A, t₁₁ₘᵢₙ = 40% A, t₁₃ₘᵢₙ = 100% A, t₁₇ₘᵢₙ = 100% A; Detection: DAD (220-320 nm); Detection: MSD (ESI pos/neg) mass range: 100 - 800; Fraction collection based DAD

### Flash chromatography

Grace Reveleris X2® C-815 Flash; Solvent delivery system: 3-piston pump with auto-priming, 4 independent channels with up to 4 solvents in a single run, auto-switches lines when solvent depletes; maximum pump flow rate 250 mL/min; maximum pressure 50bar (725psi); Detection: UV 200-400nm, combination of up to 4 UV signals and scan of entire UV range, ELSD; Column sizes: 4-330g on instrument, luer type, 750g up to 3000g with optional holder.

### S01861-EMCH synthesis (Figure 52)

To SO1861 (121 mg, 0.065 mmol) and EMCH.TFA (110 mg, 0.325 mmol) was added methanol (extra dry, 3.00 mL) and TFA (0.020 mL, 0.260 mmol). The reaction mixture stirred at room temperature. After 1.5 hours the reaction mixture was subjected to preparative MP-LC.¹ Fractions corresponding to the product were immediately pooled together, frozen and lyophilized overnight to give the title compound (120 mg, 90%) as a white fluffy solid. Purity based on LC-MS 96%.
LRMS (m/z): 2069 [M-1]¹⁻
LC-MS r.t. (min): 1.08⁴

### Dendron(-L-SO1861)⁴ synthesis (Figure 53)

### Intermediate 1:

### di-tert-butyl (((6-azidohexanoyl)azanediyl)bis(ethane-2,1-diyl))dicarbamate

6-azidohexanoic acid (0.943 g, 6.00 mmol), EDCI.HCl (1.21 g, 6.30 mmol) and Oxyma Pure (0.938 g, 6.60 mmol) were dissolved in DMF (10.0 mL) and the mixture was stirred for 5 min. Next a solution of di-tert-butyl (azanediylbis(ethane-2,1-diyl))dicarbamate (1.82 g, 6.00 mmol) in DMF (5.00 mL) was added and the reaction mixture was stirred at room temperature. After 5 hours the reaction mixture was evaporated *in vacuo* and the residue was dissolved in ethyl acetate (50 mL). The resulting solution was washed with 1N potassium bisulphate solution (50 mL), saturated sodium bicarbonate solution (2 × 50 mL) and brine (50 mL), dried over Na₂SO₄, filtered and evaporated *in vacuo.* The residue was purified by flash chromatography (ethyl acetate - heptane gradient, 10:90 rising to 100:0) to give the title compound (2.67 g, 100%) as a white solid. Purity based on LC-MS 98%.
LRMS (m/z): 287/343/465 [M-155/M-99/M+23]¹⁺
LC-MS r.t. (min): 2.02^{2A}

### Intermediate 2:

### N,N-bis(2-aminoethyl)-6-azidohexanamide dihydrochloride

To di-tert-butyl (((6-azidohexanoyl)azanediyl)bis(ethane-2,1-diyl))dicarbamate (2.66 g, 6.00 mmol) was added HCI in isopropanol (5-6 N, 20.0 mL, 110 mmol) and the reaction mixture was stirred at room temperature. After 4 hours, the reaction mixture was evaporated *in vacuo* and the resulting crude product was co-evaporated with DCM (3 × 20 mL) to give the crude title product (1.49 g, 79%) as a white solid.

LRMS (m/z): 243 [M+1]¹⁺

### Intermediate 3:

### tetra-tert-butyl ((5S,5'S)-((((6-azidohexanoyl)azanediyl)bis(ethane-2,1-diyl))bis(azanediyl))bis(6-oxohexane-6,1,5-triyl))tetracarbamate

To a solution of N,N-bis(2-aminoethyl)-6-azidohexanamide dihydrochloride (1.19 g, 3.76 mmol) in DMF (30.0 mL) and DIPEA (2.62 mL, 15.1 mmol) was added Boc-Lys(Boc)-ONp (3.69 g, 7.90 mmol) and the mixture was stirred at room temperature overnight. The reaction mixture was evaporated *in vacuo* and the residue was dissolved in ethyl acetate (100 mL). The resulting solution was washed with 1N potassium bisulphate solution (100 mL) and saturated sodium bicarbonate solution (5 × 100 mL), dried over Na₂SO₄, filtered and evaporated *in vacuo.* The residue was purified by flash chromatography (DCM - methanol/DCM (1/9, v/v) gradient 100:0 rising to 0:100) to give the give the title product (3.07 g, 91%) as a slightly yellowish solid. Purity based on LC-MS 94%.
LRMS (m/z): 800/900/922 [M-99/M+1/M+23]¹⁺
LC-MS r.t. (min): 2.17^{2A}

### Intermediate 4:

### 4-nitrophenyl 3-(acetylthio)propanoate

4-Nitrophenyl trifluoroacetate (5.17 g, 22.0 mmol) and 3-(Acetylthio)propionic Acid (2.96 g, 20.0 mmol) were dissolved in DCM (50.0 mL). Next, DIPEA (6.97 mL, 40.0 mmol) was added and the reaction mixture was stirred at room temperature overnight. The reaction mixture was evaporated *in vacuo* and the residue was dissolved in ethyl acetate (50 mL). The resulting solution was washed with 1N potassium bisulphate solution (50 mL), saturated sodium bicarbonate solution (5 × 50 mL) and brine (50 mL), dried over Na₂SO₄, filtered and evaporated *in vacuo.* The residue was purified by flash chromatography (DCM - methanol/DCM (1/9, v/v) gradient 100:0 rising to 0:100) to give the give the title product (4.90 g, 91%) as a slightly yellowish solid. Purity based on LC-MS 99%.
LRMS (m/z): 292 [M+23]¹⁺
LC-MS r.t. (min): 1.94^{2A}

### Intermediate 5:

### (S)-2,6-diamino-N-(2-(6-azido-N-(2-((S)-2,6-diaminohexanamido)ethyl)hexanamido)ethyl)hexanamide tetrahydrochloride

tetra-tert-butyl ((5S,5'S)-((((6-azidohexanoyl)azanediyl)bis(ethane-2,1-diyl))bis(azanediyl))bis(6-oxohexane-6,1,5-triyl))tetracarbamate (1.80 g, 2.00 mmol) was dissolved in HCI in isopropanol (5-6N, 50.0 ml, 275 mmol) and the reaction mixture was stirred at room temperature overnight. The reaction mixture was evaporated *in vacuo* and the resulting crude product was co-evaporated with DCM (3 × 20 mL) to give the crude title product as a white solid.
LRMS (m/z): 250 [M+2]²⁺, 500 [M+1]¹⁺

### Intermediate 6:

### (2S)-2,6-bis[3-(acetylsulfanyl)propanamido]-N-[2-(6-azido-N-{2-[(2S)-2,6-bis[3-(acetylsulfanyl)propanamido]hexanamido]ethyl}hexanamido)ethyl]hexanamide

To a solution of (S)-2,6-diamino-N-(2-(6-azido-N-(2-((S)-2,6- diaminohexanamido)ethyl)hexanamido) ethyl)hexanamide tetrahydrochloride (1.29 g, 2.00 mmol) in DMF (30 mL) and DIPEA (3.48 mL, 20.0 mmol) was added 4-nitrophenyl 3-(acetylthio)propanoate (2.26 g, 8.40 mmol) and the reaction mixture was stirred at room temperature over the weekend. The reaction mixture was evaporated *in vacuo* and the residue was dissolved in DCM/methanol (95:5, v/v, 100 mL). The resulting solution was washed with 1N potassium bisulphate solution (100 mL), 1 N sodium hydroxide solution (3 × 100 mL) and brine (100 mL), dried over Na₂SO₄, filtered and evaporated *in vacuo.* The residue was purified by flash chromatography (DCM - methanol/DCM (1/9, v/v) gradient 100:0 rising to 0:100) to give the title product (1.33 g, 65%) as a white solid. On LC-MS an impurity (15%) was found with m/z values corresponding to the product with one deprotected thioacetate group. The impurity was formed during or after work-up. Purity based on LC-MS 85%.
LRMS (m/z): 510 [M+2]²⁺, 1019/1041 [M+1/M+23]¹⁺
LC-MS r.t. (min): 1.86^{2B}

### Intermediate 7:

### N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate

Scaffold 2 (102 mg, 0.100 mmol) was dissolved in methanol (1.00 ml). Next, a freshly prepared 1 N Sodium hydroxide solution (0.440 ml, 0.440 mmol) was added and the reaction mixture was stirred at room temperature. After 30 min a 1.0 M trimethylphosphine solution in THF (0.500 ml, 0.500 mmol) was added and the resulting mixture was stirred at room temperature. After 30 min the reaction mixture was evaporated *in vacuo* and co-evaporated with methanol (2 × 10 mL). The residue was dissolved in a mixture of methanol/water (9:1, v/v, 1.00 mL) and the resulting solution was subjected to preparative MP-LC.² Fractions corresponding to the product were immediately pooled together, frozen and lyophilized overnight to give the title compound (75.6 mg, 87%) as a colorless sticky oil. Purity based on LC-MS 96%.
LRMS (m/z): 513 [M+2]²⁺, 825 [M+1]¹⁺
LC-MS r.t. (min): 1.42^{2A}

### Intermediate 8:

### dendron(-LSO1861)⁴-amine

N,N'-((9S,19S)-14-(6-aminohexanoyl)-1-mercapto-9-(3-mercaptopropanamido)-3,10,18-trioxo-4,11,14,17-tetraazatricosane-19,23-diyl)bis(3-mercaptopropanamide) formate (2.73 mg, 3.13 µmol) was dissolved in a mixture of 20 mM NH₄HCO₃ with 0.5 mM TCEP/acetonitrile (3:1, v/v, 3.00 mL). Next, SO1861-EMCH (29.2 mg, 0.014 mmol) was added and the reaction mixture was stirred at room temperature. After 1.5 hours the reaction mixture was subjected to preparative LC-MS.^{3B} Fractions corresponding to the product were immediately pooled together, frozen and lyophilized overnight to give the title compound (12.3 mg, 43%) as a white fluffy solid. Purity based on LC-MS 97%.
LRMS (m/z): 1517 [M-6]⁶⁻, 1821 [M-5]⁵⁻, 2276 [M-4]⁴⁻
LC-MS r.t. (min): 4.39^{5A}

### Intermediate 9:

### dendron(-L-SO1861)⁴-azide

Dendron(SO1861)₄-amine (6.81 mg, 0.748 µmol) and 2,5-dioxopyrrolidin-1-yl 1-azido-3,6,9,12-tetraoxapentadecan-15-oate (2.90 mg, 7.48 µmol) were dissolved in DMF(1.00 mL). Next, DIPEA (1.302 µL, 7.48 µmol) was added and the mixture was shaken for 1 min and left standing at room temperature. After 2 hours the reaction mixture was subjected to preparative LC-MS.^{3C} Fractions corresponding to the product were immediately pooled together, frozen and lyophilized overnight to give the title compound (5.86 mg, 84%) as a white fluffy solid. Purity based on LC-MS 90%.
LRMS (m/z): 2344 [M-4]⁴⁻
LC-MS r.t. (min): 4.78^{5B}

### Intermediate 10:

### dendron(-LSO1861)⁴-maleimide1

Dendron(SO1861)₄-amine (8.12 mg, 0.891 µmol) and 2,5-dioxopyrrolidin-1-yl 1-(2,5-dioxo-2,5-dihydro-1H-pyrrol-1-yl)-3,6,9,12-tetraoxapentadecan-15-oate (3.94 mg, 8.91 µmol) were dissolved in DMF(1.00 mL). Next, DIPEA (1.55 µL, 8.91 µmol) was added and the mixture was shaken for 1 min and left standing at room temperature. After 3 hours the reaction mixture was subjected to preparative LC-MS.^{3C} Fractions corresponding to the product were immediately pooled together, frozen and lyophilized overnight to give the title compound (6.76 mg, 80%) as a white fluffy solid. Purity based on LC-MS 66%.
LRMS (m/z): 2358 [M-4]⁴⁻
LC-MS r.t. (min): 2.13^{6C}

### Intermediate 11:

### dendron(-LSO1861)⁴-maleimide2

Scaffold 2 (5.10 mg, 5.00 µmol) was dissolved in methanol (100 µL). Next, a freshly prepared 1 N Sodium hydroxide solution (22.0 µL, 22.0 µmol) was added and the mixture was shaken for 1 min and left standing at room temperature. After 30 min a 1.0 M trimethylphosphine solution in THF (25.0 µL, 25.0 µmol) was added and the resulting mixture was shaken for 1 min and left standing at room temperature. After 30 min the reaction mixture was evaporated *in vacuo* and co-evaporated with methanol (2 × 5 mL). The resulting residue was dissolved in a mixture of 20 mM NH₄HCO₃ with 0.5 mM TCEP/acetonitrile (3:1, v/v, 3.242 mL). From this solution, directly, 1000 µL was added to SO1861-EMCH (14.4 mg, 6.94 µmol, 4.5 equiv. compared to the scaffold) and the mixture was shaken for 1 min and left standing at room temperature. After 10 min the reaction mixture was lyophilized overnight. To the resulting residue 2,5-Dioxopyrrolidin-1-yl 3-(2-(2-(3-(2,5-dioxo-2h-pyrrol-1(5h)-yl)propanamido)ethoxy)ethoxy)propanoate (5.84 mg, 0.014 mmol) and DMF (1.00 mL) were added. Next, DIPEA (2.39 µL, 0.014 mmol) was added and the suspension was shaken for 1 min and left standing at room temperature. After 2 hours the reaction mixture was subjected to preparative LC-MS.^{3C} Fractions corresponding to the product were immediately pooled together, frozen and lyophilized overnight to give the title compound (10.9 mg, 85%) as a white fluffy solid. Purity based on LC-MS 80%.
LRMS (m/z): 2354 [M-4]⁴⁻
LC-MS r.t. (min): 4.16^{5B}

### Dendron(-L-SO1861)⁸ synthesis (Figure 54)

### Intermediate 1:

### tert-butyl N-[(1S)-1-{[(1S)-1-{[2-(6-azido-N-{2-[(2S)-2,6-bis[(2S)-2,6-bis({[(tert-butoxy)carbonyl]amino})hexanamido]hexanamido]ethyl}hexanamido)ethyl]carbamoyl}-5-[(2S)-2,6-bis({[(tert-butoxy)carbonyl]amino})hexanamido]pentyl]carbamoyl}-5-{[(tert-butoxy)carbonyl]amino}pentyl]carbamate

(S)-2,6-diamino-N-(2-(6-azido-N-(2-((S)-2,6-diaminohexanamido)ethyl)hexanamido)ethyl)hexanamide tetrahydrochloride (964 mg, 1.50 mmol) was dissolved in DMF (25.0 mL) and triethylamine (2.08 mL, 15.0 mmol). Next, Boc-Lys(Boc)-ONp (3.36 g, 7.18 mmol) was added and the reaction mixture was stirred at room temperature overnight. The reaction mixture was evaporated *in vacuo* and the residue was purified by flash chromatography (DCM - methanol/DCM (1/9, v/v) gradient 100:0 rising to 0:100) to give the title product (2.71 g, 100%) as a white solid. Purity based on LC-MS 97%.
LRMS (m/z): 807 [M-198]²⁺
LC-MS r.t. (min): 2.35^{2B}

### Intermediate 2:

### (2S,2'S)-N,N'-((5S,15S,22S)-22,26-diamino-10-(6-azidohexanoyl)-15-((S)-2,6-diaminohexanamido)-6,14,21-trioxo-7,10,13,20-tetraazahexacosane-1,5-diyl)bis(2,6-diaminohexanamide) octahydrochloride

Intermediate 1 (2.71 g, 1.50 mmol) was dissolved in HCI in isopropanol (5-6N, 25.0 ml, 138 mmol) and the reaction mixture was stirred at room temperature overnight. Next, the reaction mixture was evaporated *in vacuo* and the resulting crude product was co-evaporated with DCM (3 × 20 mL) to give the crude title product as a white solid.
LRMS (m/z): 203/254 [M-200/M+4]⁴⁺, 338 [M+3]³⁺, 507 [M+2]²⁺, 1012 [M+1]¹⁺

### Intermediate 3:

### (2S)-2,6-bis[3-(acetylsulfanyl)propanamido]-N-[(1S)-1-{[2-(6-azido-N-{2-[(2S)-2,6-bis[(2S)-2,6-bis[3-(acetylsulfanyl)propanamido]hexanamido]hexanamido]ethyl}hexanamido)ethyl]carbamoyl}-5-[(2S)-2,6-bis[3-(acetylsulfanyl)propanamido]hexanamido]pentyl]hexanamide

To (2S,2'S)-N,N'-((5S,15S,22S)-22,26-diamino-10-(6-azidohexanoyl)-15-((S)-2,6-diaminohexanamido)-6,14,21-trioxo-7,10,13,20-tetraazahexacosane-1,5-diyl)bis(2,6-diaminohexanamide) octahydrochloride (300 mg, 0.230 mmol) was added DMF (20.0 mL), triethylamine (320 µl, 2.30 mmol) and 4-nitrophenyl 3-(acetylthio)propanoate (595 mg, 2.21 mmol). The resulting suspension was sonicated at 60 °C for 30 min and left stirring at room temperature overnight. The reaction mixture was evaporated *in vacuo* and the residue was purified by first performing flash chromatography (DCM - methanol/DCM (1/9, v/v) gradient 100:0 rising to 0:100), followed by preparative MP-LC² to give the title product (70 mg, 15%) as a white solid. Purity based on LC-MS 100%.
LRMS (m/z): 685 [M+3]³⁺
LC-MS r.t. (min): 1.91^{2A}

### Intermediate 4:

### (2S)-N-[(1S)-1-{[2-(6-amino-N-{2-[(2S)-2,6-bis[(2S)-2,6-bis(3-sulfanylpropanamido)hexanamido]hexanamido]ethyl}hexanamido)ethyl]carbamoyl}-5-[(2S)-2,6-bis(3-sulfanylpropanamido)hexanamido]pentyl]-2,6-bis(3-sulfanylpropanamido)hexanamide formate

Scaffold 4 (10.0 mg, 4.87 µmol) was dissolved methanol (200 µL). Next, a freshly prepared 1 N Sodium hydroxide solution (42.9 µL, 0.043 mmol) was added and the resulting mixture was shaken for 1 min and left standing at room temperature. After 30 min a 1.0 M trimethylphosphine solution in THF (24.4 µL, 0.024 mmol) was added and the resulting mixture was shaken for 1 min and left standing at room temperature. After 30 min the reaction mixture was diluted with water (1 mL) and directly subjected to preparative MP-LC.² Fractions corresponding to the product were immediately pooled together, frozen and lyophilized overnight to give the title compound (4.02 mg, 48%) as a white fluffy solid.
LRMS (m/z): 564 [M+3]³⁺, 846 [M+2]²⁺ LC-MS r.t. (min): 1.54^{2C}

### Intermediate 5:

### dendron(-LSO1861)⁸-amine

Scaffold 5 (0.52 mg, 0.299 µmol) and SO1861-EMCH (29.2 mg, 0.014 mmol) were dissolved in a mixture of 20 mM NH₄HCO₃ with 0.5 mM TCEP/acetonitrile (3:1, v/v, 1.00 mL) and the resulting mixture was shaken for 1 min and left standing at room temperature. After 30 min TCEP (0.30 mg, 1.05 µmol) was added and the reaction mixture was shaken for 1 min. Next, the mixture was directly subjected to preparative LC-MS.^{3B} Fractions corresponding to the product were immediately pooled together, frozen and lyophilized overnight to give the title compound (2.17 mg, 40%) as a white fluffy solid. Purity based on LC-MS 97%.
LRMS (m/z): 2282 [M-8]⁸⁻, 2607 [M-7]⁷⁻
LC-MS r.t. (min): 4.41^{5A}

### Example 8

### S01861-trifunctional linker-BNAoligo synthesis (Figure 55)

### Materials and methods

### Trifunctional linker

Trifunctional linker (DBCO, TCO, maleimide) was ordered at Bio-Synthesis Inc. (Lewisville, Texas).

### HSP27BNA oligo

HSP27BNA(-thiol) oligos (sequence 5'-GGCacagccagtgGCG-3') (Zhang et al., 2011) were purchased at Bio-synthesis Inc. (Lewisville, Texas)

### Intermediate 1:

### S01861-azide

To SO1861 60 mg, 0.032 mmol)) and 1-azido-3,6,9,12-tetraoxapentadecane-15-hydrazide (39.3 mg, 0.129 mmol) was added methanol (extra dry, 1.00 mL) and TFA (9.86 µl, 0.129 mmol) and the reaction mixture was shaken for 1 min and left standing at room temperature. After 2 hours the reaction mixture was subjected to preparative MP-LC.¹ Fractions corresponding to the product were immediately pooled together, frozen and lyophilized overnight to give the title compound (58.4 mg, 84%) as a white fluffy solid. Purity based on LC-MS 100%.
LRMS (m/z): 2150 [M-1]¹⁻
LC-MS r.t. (min): 1.10^{3B}

### Intermediate 2:

### S01861-trifunctional linker

SO1861-azide (45 mg, 0.021 mmol) and trifunctional linker (26.5 mg, 0.022 mmol) were dissolved in DMF (2.50 mL) and the resulting mixture was shaken for 1 min and left standing at room temperature. After 30 min the reaction mixture was subjected to preparative LC-MS.^{3C} Fractions corresponding to the product were immediately pooled together, frozen and lyophilized overnight to give the title compound (58.4 mg, 84%) as a white fluffy solid. Purity based on LC-MS 89%.
LRMS (m/z): 1677 [M-2]²⁻
LC-MS r.t. (min): 2.54^{6A}

### Intermediate 3:

### (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide

To 1-(4-formylbenzamido)-N-(4-(6-methyl-1,2,4,5-tetrazin-3-yl)benzyl)-3,6,9,12-tetraoxapentadecan-15-amide (28.0 mg, 0.048 mmol) and EMCH.TFA (24.5 mg, 0.072 mmol) was added methanol (extra dry, 2.00 mL) and TFA (11.1 µL, 0.145 mmol) and the reaction mixture stirred at 50 °C. After 30 min the reaction mixture was evaporated *in vacuo* and the resulting residue was purified by MP-LC.¹ Fractions corresponding to the product were immediately pooled together, frozen and lyophilized overnight to give the title compound (33.4 mg, 88%) as a bright purple fluffy solid. Purity based on LC-MS 92%.
LRMS (m/z): 394 [M+2]²⁺, 789 [M+1]¹⁺
LC-MS r.t. (min): 1.28^{7A}

### Intermediate 4:

### methyltetrazine-BNA oligo

To HSP27 BNA oligo disulfide (70.0 mg, 0.012 mmol) was dissolved in 20 mM NH₄HCO₃ (20.0 mL). Next, TCEP (14.3 mg, 0.050 mmol) was added and the reaction mixture was shaken for 1 min and left standing at room temperature. The reaction mixture was filtered by using a centrifugal filter with a molecular weight cut-off of 3000 Da (5000 × g for 30 min). Next, a solution of 20 mM NH₄HCO₃ with 2.5 mM TCEP (20.0 mL) was added to the residue solution and the resulting mixture was filtered again under the same conditions described above. The residue solution was diluted with 20 mM NH₄HCO₃ (30.0 mL) and the resulting mixture was added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (14.8 mg, 18.8 µmol) in acetonitrile (10.0 mL). The reaction mixture was shaken for 1 min and left standing at room temperature. After 30 min the reaction mixture was frozen and lyophilized over the weekend to yield the crude title product as a pink fluffy solid. To the crude product was added a solution of 20 mM NH₄HCO₃ (20.0 mL) and the resulting suspension was filtered over a 0.45 µm syringe filter. The filtrate was filtered using a centrifugal filter with the same conditions as described above. Next, again a solution of 20 mM NH₄HCO₃ (20.0 mL) was added to the residue solution and the resulting mixture was again filtered by using a centrifugal filter with the same conditions described above. The residue solution was diluted with 20 mM NH₄HCO₃ (20.0 mL) and the resulting mixture was lyophilized overnight to yield the title product (90.0 mg, 115%) as a pink fluffy solid. Purity based on LC-MS 91%.
LRMS (m/z): 1631 [M-4]⁴⁻, 2174 [M-3]³⁻
LC-MS r.t. (min): 0.73^{7B}

### Intermediate 5:

### S01861-trifunctional linker-BNA oligo

Methyltetrazine-BNA oligo (90.0 mg, 0.014 mmol) and SO1861-trifunctional linker (48.6 mg, 0.014 mmol) were dissolved in a mixture of water/acetonitrile (4:1, v/v, 12.0 mL). The reaction mixture was shaken for 1 min and left standing at room temperature. After 15 min the mixture was subjected to preparative LC-MS.^{4A} Fractions corresponding to the product were immediately pooled together, frozen and lyophilized overnight to give the title compound (82.0 mg, 60%) as a white fluffy solid. Purity based on LC-MS 92% (2 peaks with both m/z values corresponding to the title compound).
LRMS (m/z): 1641 [M-6]⁶⁻, 1970 [M-5]⁵⁻
LC-MS r.t. (min): 3.24 and 3.40^{6B}

### Intermediate 1:

### S01861-azide

To SO1861 60 mg, 0.032 mmol)) and 1-azido-3,6,9,12-tetraoxapentadecane-15-hydrazide (39.3 mg, 0.129 mmol) was added methanol (extra dry, 1.00 mL) and TFA (9.86 µl, 0.129 mmol) and the reaction mixture was shaken for 1 min and left standing at room temperature. After 2 hours the reaction mixture was subjected to preparative MP-LC.¹ Fractions corresponding to the product were immediately pooled together, frozen and lyophilized overnight to give the title compound (58.4 mg, 84%) as a white fluffy solid. Purity based on LC-MS 100%.
LRMS (m/z): 2150 [M-1]¹⁻
LC-MS r.t. (min): 1.10^{3B}

### Intermediate 2:

### S01861-trifunctional linker

SO1861-azide (45 mg, 0.021 mmol) and trifunctional linker (26.5 mg, 0.022 mmol) were dissolved in DMF (2.50 mL) and the resulting mixture was shaken for 1 min and left standing at room temperature. After 30 min the reaction mixture was subjected to preparative LC-MS.^{3C} Fractions corresponding to the product were immediately pooled together, frozen and lyophilized overnight to give the title compound (58.4 mg, 84%) as a white fluffy solid. Purity based on LC-MS 89%.
LRMS (m/z): 1677 [M-2]²⁻
LC-MS r.t. (min): 2.54^{6A}

### Intermediate 3:

### (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide

To 1-(4-formylbenzamido)-N-(4-(6-methyl-1,2,4,5-tetrazin-3-yl)benzyl)-3,6,9,12-tetraoxapentadecan-15-amide (28.0 mg, 0.048 mmol) and EMCH.TFA (24.5 mg, 0.072 mmol) was added methanol (extra dry, 2.00 mL) and TFA (11.1 µL, 0.145 mmol) and the reaction mixture stirred at 50 °C. After 30 min the reaction mixture was evaporated *in vacuo* and the resulting residue was purified by MP-LC.¹ Fractions corresponding to the product were immediately pooled together, frozen and lyophilized overnight to give the title compound (33.4 mg, 88%) as a bright purple fluffy solid. Purity based on LC-MS 92%.
LRMS (m/z): 394 [M+2]²⁺, 789 [M+1]¹⁺
LC-MS r.t. (min): 1.28^{7A}

### Intermediate 4:

### methyltetrazine-BNA oligo

To HSP27 BNA oligo disulfide (70.0 mg, 0.012 mmol) was dissolved in 20 mM NHaHCO₃(20.0 mL). Next, TCEP (14.3 mg, 0.050 mmol) was added and the reaction mixture was shaken for 1 min and left standing at room temperature. The reaction mixture was filtered by using a centrifugal filter with a molecular weight cut-off of 3000 Da (5000 × g for 30 min). Next, a solution of 20 mM NH₄HCO₃ with 2.5 mM TCEP (20.0 mL) was added to the residue solution and the resulting mixture was filtered again under the same conditions described above. The residue solution was diluted with 20 mM NH₄HCO₃ (30.0 mL) and the resulting mixture was added to a solution of (E)-1-(4-((2-(6-(2,5-dioxo-2,5-dihydro-1H-pyrrol-1-yl)hexanoyl)hydrazineylidene)methyl)benzamido)-N-(4-(6-methyl-1,2,4,5-tetrazin-3-yl)benzyl)-3,6,9,12-tetraoxapentadecan-15-amide (14.8 mg, 18.8 µmol) in acetonitrile (10.0 mL). The reaction mixture was shaken for 1 min and left standing at room temperature. After 30 min the reaction mixture was frozen and lyophilized over the weekend to yield the crude title product as a pink fluffy solid. To the crude product was added a solution of 20 mM NH₄HCO₃ (20.0 mL) and the resulting suspension was filtered over a 0.45 µm syringe filter. The filtrate was filtered using a centrifugal filter with the same conditions as described above. Next, again a solution of 20 mM NH₄HCO₃ (20.0 mL) was added to the residue solution and the resulting mixture was again filtered by using a centrifugal filter with the same conditions described above. The residue solution was diluted with 20 mM NH₄HCO₃ (20.0 mL) and the resulting mixture was lyophilized overnight to yield the title product (90.0 mg, 115%) as a pink fluffy solid. Purity based on LC-MS 91%.
LRMS (m/z): 1631 [M-4]⁴⁻, 2174 [M-3]³⁻
LC-MS r.t. (min): 0.73^{7B}

### Intermediate 5:

### S01861-trifunctional linker-BNA oligo

Methyltetrazine-BNA oligo (90.0 mg, 0.014 mmol) and SO1861-trifunctional linker (48.6 mg, 0.014 mmol) were dissolved in a mixture of water/acetonitrile (4:1, v/v, 12.0 mL). The reaction mixture was shaken for 1 min and left standing at room temperature. After 15 min the mixture was subjected to preparative LC-MS.^{4A} Fractions corresponding to the product were immediately pooled together, frozen and lyophilized overnight to give the title compound (82.0 mg, 60%) as a white fluffy solid. Purity based on LC-MS 92% (2 peaks with both m/z values corresponding to the title compound).
LRMS (m/z): 1641 [M-6]⁶⁻, 1970 [M-5]⁵⁻
LC-MS r.t. (min): 3.24 and 3.40^{6B}

### Example 9

### S01861-BNA oligo conjugation

HSP27 BNA oligo disulfide (1.10 mg, 0.187 µmol) was dissolved in 20 mM NH₄HCO₃ with 1.0 mM TCEP (500 µL) and the mixture was shaken for 1 min and left standing at room temperature. After 1 hour the reaction mixture was filtered by using a centrifugal filter with a molecular weight cut-off of 3000 Da (14000 × g for 30 min). The residue solution was diluted with 20 mM NH₄HCO₃with 1.0 mM TCEP (500 µL) and the resulting mixture was filtered again under the same conditions described above. The residue solution was diluted with 20 mM Nh₄HCO₃/acetonitrile (3:1, v/v, 1.00 mL) and the resulting mixture was added to SO1861-EMCH (3.54 mg, 0.375 µmol). The reaction mixture was shaken for 1 min and left standing at room temperature. After 10 min the reaction mixture was subjected to preparative LC-MS.^{4A} Fractions corresponding to the product were immediately pooled together, frozen and lyophilized overnight to give the title compound (1.25 mg, 85%) as a white fluffy solid. Purity based on LC-MS 100%.
LRMS (m/z): 1561 [M-5]⁵⁻, 1951 [M-4]⁴⁻
LC-MS r.t. (min): 2.466B

### Dendron(SO1861)⁴-BNA oligo conjugation (Figure 56)

HSP27 BNA oligo disulfide (1.1 mg, 0.187 µmol) was dissolved in 20 mM NH₄HCO₃ with 1.0 mM TCEP (500 µL) and the mixture was shaken for 1 min and left standing at room temperature. After 1 hour the reaction mixture was filtered by using a centrifugal filter with a molecular weight cut-off of 3000 Da (14000 × g for 30 min). The residue solution was diluted with 20 mM NH₄HCO₃ with 1.0 mM TCEP (500 µL) and the resulting mixture was filtered again under the same conditions described above. The residue solution was diluted with 20 mM NH₄HCO₃/acetonitrile (3:1, v/v, 1.0 mL) and the resulting mixture was added to dendron(SO1861)4-maleimide1 (3.54 mg, 0.375 µmol). The reaction mixture was shaken for 1 min and left standing at room temperature. After 10 min the reaction mixture was subjected to preparative LC-MS.^{4A} Fractions corresponding to the product were immediately pooled together, frozen and lyophilized overnight to give the title compound (1.25 mg, 85%) as a white fluffy solid. Purity based on LC-MS 94%
LRMS (m/z): 1896 [M-8]⁸⁻, 2167 [M-7]⁷⁻
LC-MS r.t. (min): 3.77^{6B}

### Dendron(NEM)⁴ synthesis (Figure 57)

To benzyl bis(2-((S)-2,6-bis(3-mercaptopropanamido)hexanamido)ethyl)carbamate (1.69 mg, 2.00 µmol) and N-Ethylmaleimide (1.05 mg, 8.40 µmol) was added a mixture of 20 mM NH₄HCO₃/acetonitrile (3:1, v/v, 2.00 mL) and the reaction mixture was stirred at room temperature. After 2 hours, the reaction mixture was lyophilized overnight. The resulting residue was purified by using preparative LC-MS^{3A} to give the title compound (1.53 mg, 57%) as a white fluffy solid. Purity based on LC-MS 98%.
LRMS (m/z): 1346 [M+1]¹⁺
LC-MS r.t. (min): 1.43^{3A}

### Example 10

### A431 mouse tumor mouse model and vitro and vivo gene silencing studies

### Materials and methods

HSP27BNA with linkers oligos (sequence 5'-GGCacagccagtgGCG-3') (Zhang et al., 2011) were purchased at Bio-Synthesis Inc. (Lewisville, Texas)

### Vitro RNA isolation and gene expression analyses

RNA from cells was isolated and analysed according to standard protocols (Biorad)

### Vivo mouse tumor model

The mouse study was performed at CrownBio (China) according to standard protocols and procedures. Model: Subcutaneous A431 Human Epidermoid Carcinoma Xenograft Model in female BALB/c nude Mice. Tumor volume was monitored and tumor samples were collected for gene silencing analysis (see below)

### RNA isolation and gene expression analyses of tumor samples from mice

Total RNA was isolated from tumors using TriZol (Thermo Fisher) according to the manufacturer's instructions. Isolated RNA was resuspended in nuclease-free water (NFW). RNA samples were checked for their RNA integrity on the gel. To prepare cDNA, first 100 ng total RNA was mixed with Random Hexamers (Qiagen; final concentration 2 µM) in a final volume of 12.5 µl in NFW, denatured for 5min at 70°C and immediately cooled on ice. Next, 7.5 µl of a cDNA synthesis mix was added, consisting of 4 µl 5xRT Buffer (Promega), 0.4 µl 25mM dNTPs (Promega), 1 µl 200 U/µL MMLV RT-enzyme (Promega), 0.5 µL 40 U/µL RNAse Inhibitor (Promega) and 1.6 µL NFW. The following cDNA synthesis protocol was used: 1) 10 minutes 25°C 2) 60 minutes 37°C 3) 5 minutes 85°C 4) ∞ 4°C

For a single qPCR reaction the following mix was prepared: 1 µL cDNA, 0.05 µL forward primer (250 µM), 0.05 µL reverse primer (250 µM), 8.9 µl LNFW, 10 µl SYBR Green (Bio-Rad). The following qPCR protocol was used: 1 cycle: 5 minutes 95°C, 40 cycles: 15s 95°C + 30s 60°C.

HSP27 gene expression was calculated using 2^{-(Ct}_{HSP27}^{- GEOMEAN(Ct}_{refl} ^{;Ct}_{ref2}^{;Ct}_{ref3}^{;Ct}_{ref4})), where ref1, ref2, ref3 and ref4 are the reference genes IMMT, EIF2S2, GUSB and UBC for the analysis of the tumors. Four reference genes were chosen based on the performance of a GeNORM analysis among nine reference genes tested to choose the most ideal and stable reference gene for this tumor samples. To do so, qPCR results were imported in Qbase+ software program by which two quality measures are calculated: the coefficient of variation of the normalized reference gene expression levels (V); and the geNorm stability M-value (M)1. A reference gene with an M<0.2 and a V<0.15 is considered very stable. Based on this analysis IMMT and EIF2S2 were chosen as the most stable reference genes. However, UBC and GUSB were also added to the group of reference genes to further enhance the accuracy of the normalization. Each sample was analyzed as technical triplicate on a CFX96 Real-Time qPCR machine (Bio-Rad).

**Table 1. Primers used in qPCR are shown below:**

| Gene | Primer | Sequence (5'-3') |
|---|---|---|
| UBC | Forward | CAGCCGGGATTTGGGTCG |
| | Reverse | CACGAAGATCTGCATTGTCAAGT |
| GUSB | Forward | TGCGTAGGGACAAGAACCAC |
| | Reverse | GGGAGGGGTCCAAGGATTTG |
| IMMT | Forward | AACCCACACCTGCACTTTCA |
| | Reverse | TTTTCCTGTTGTGCAAGGCG |
| EIF2S2 | Forward | CCACAAGTCGTCCGAGTAGG |
| | Reverse | GGAGATGTTTGGGCTGACGA |
| HSP27 | Forward | GCAGTCCAACGAGATCACCA |
| | Reverse | TAAGGCTTTACTTGGCGGCA |

### Example 11

### Conjugate synthesis

### Monoclonal antibodies, SO1861, QS saponins

Trastuzumab (Herceptin®), cetuximab (Erbitux®) and T-DM1 (Kadcyla®) were purchased from the pharmacy (Charite, Berlin). CD71 monoclonal antibody was purchased from BioCell (Okt9, #BE0023). SO1861 was isolated and purified by Analyticon Discovery GmbH from raw plant extract obtained from *Saponaria officinalis L. Quillaja Saponaria* saponin extract (QSmix) was purchased (Sigma Aldrich, #S4521)

### materials

Trastuzumab (Tras, Herceptin®, Roche), Cetuximab (Cet, Erbitux®, Merck KGaA), Tris(2-carboxyethyl)phosphine hydrochloride (TCEP, 98 %, Sigma-Aldrich), 5,5-Dithiobis(2-nitrobenzoic acid) (DTNB, Ellman's reagent, 99%, Sigma-Aldrich), Zeba™ Spin Desalting Columns (2 mL, Thermo-Fisher), NuPAGE™ 4-12% Bis-Tris Protein Gels (Thermo-Fisher), NuPAGE™ MES SDS Running Buffer (Thermo-Fisher), Novex™ Sharp Pre-stained Protein Standard (Thermo-Fisher), PageBlue™ Protein Staining Solution (Thermo-Fischer), Pierce™ BCA Protein Assay Kit (Thermo-Fisher), N-Ethylmaleimide (NEM, 98 %, Sigma-Aldrich), 1,4-Dithiothreitol (DTT, 98 %, Sigma-Aldrich), Sephadex G25 (GE Healthcare), Sephadex G50 M (GE Healthcare), Superdex 200P (GE Healthcare), Isopropyl alcohol (IPA, 99.6 %, VWR), Tris(hydroxymethyl)aminomethane (Tris, 99%, Sigma-Aldrich), Tris(hydroxymethyl)aminomethane hydrochloride (Tris.HCL, Sigma-Aldrich), L-Histidine (99%, Sigma-Aldrich), D-(+)-Trehalose dehydrate (99%, Sigma-Aldrich), Polyethylene glycol sorbitan monolaurate (TWEEN 20, Sigma-Aldrich), Dulbecco's Phosphate-Buffered Saline (DPBS, Thermo-Fisher), Guanidine hydrochloride (99%, Sigma-Aldrich), Ethylenediaminetetraacetic acid disodium salt dihydrate (EDTA-Na₂, 99 %, Sigma-Aldrich), sterile filters 0.2 µm and 0.45 µm (Sartorius), Succinimidyl 4-(N-maleimidomethyl)cyclohexane-1-carboxylate (SMCC, Thermo-Fisher), Dianthin-Cys (Dia-Cys, Dianthin mutant with a single C-terminal cysteine function, Proteogenix), Vivaspin T4 and T15 concentrator (Sartorius), Superdex 200PG (GE Healthcare), Tetra(ethylene glycol) succinimidyl 3-(2-pyridyldithio)propionate (PEG₄-SPDP, Thermo-Fisher), HSP27 BNA disulfide oligonucleotide (Biosynthesis), [O-(7-Azabenzotriazol-1-yl)-N,N,N,N-tetramethyluronium-hexafluorphosphat] (HATU, 97%, Sigma-Aldrich), Dimethyl sulfoxide (DMSO, 99%, Sigma-Aldrich), N-(2-Aminoethyl)maleimide trifluoroacetate salt (AEM, 98 %, Sigma-Aldrich), L-Cysteine (98.5 %, Sigma-Aldrich), deionized water (DI) was freshly taken from Ultrapure Lab Water Systems (MilliQ, Merck), Nickel-nitrilotriacetic acid agarose (Ni-NTA agarose, Protino), Glycine (99.5 %, VWR), 5,5-Dithiobis(2-nitrobenzoic acid (Ellman's reagent, DTNB, 98 %, Sigma-Aldrich), S-Acetylmercaptosuccinic anhydride Fluorescein (SAMSA reagent, Invitrogen) Sodium bicarbonate (99.7 %, Sigma-Aldrich), Sodium carbonate (99.9 %, Sigma-Aldrich), PD MiniTrap desalting columns with Sephadex G-25 resin (GE Healthcare), PD10 G25 desalting column (GE Healthcare), Zeba Spin Desalting Columns in 0.5, 2, 5, and 10 mL (Thermo-Fisher), Vivaspin Centrifugal Filters T4 10 kDa MWCO, T4 100 kDa MWCO, and T15 (Sartorius), Biosep s3000 aSEC column (Phenomenex), Vivacell Ultrafiltration Units 10 and 30 kDa MWCO (Sartorius), Nalgene Rapid-Flow filter (Thermo-Fisher),

### Methods

### MALDI-TOF-MS

Matrix-assisted laser desorption/ionization time of flight (MALDI-TOF) spectra were recorded on a MALDI-Mass Spectrometer (Bruker Ultrafex III). Typically, the sample dissolved in MilliQ water in nanomole to micromole range was spotted on the target (MTP 384 target plate polished steel T F, Bruker Daltons) using either super-DHB (99%, Fluka) or sinapinic acid (SA, 99%, Sigma-Aldrich) as the matrix that was dissolved in acetonitrile (MADLI-TOF-MS tested, Sigma) / 0.1 % TFA (7:3 v/v) via the dried-droplet-method. PepMix (Peptide Calibration Standard, Bruker Daltons) or ProteoMass (Protein Calibration Standard, Sigma-Aldrich) served as calibration standards. RP mode refers to reflector positive mode. RN mode refers to reflector negative mode. LP mode refers to linear positive mode.

### Dialysis

Regenerated cellulose membranes: MWCO = 1 and 2 kDa (Spectra/Por), and MWCO = 12-14 kDa (Carl Roth) were used to perform dialysis. Typically, dialysis was carried out for 24 h with 1 L of solvent that was exchanged after first 6 h of the process.

### Lyophilization

Freeze-drying was performed on an Alpha 1-2 LD plus (Martin Christ Gefriertrocknungsanlagen GmbH). Typically, samples were frozen with liquid nitrogen and placed into the freeze-dryer at high vacuum.

### UV-Vis

Absorbance measurements were performed on a Perkin Elmer Lambda 25 UV-Vis or on a Thermo NanoDrop ND-2000 spectrophotometer in the spectral range of 200-750 nm.

Concentrations were determined using a Thermo Nanodrop 2000 or Lambda 25 spectrometer using the following parameters:
Trastuzumab OD₂₈₀ = 1.5 (mg/ml)⁻¹ cm⁻¹
Cetuximab OD₂₈₀ = 1.4 (mg/ml)⁻¹ cm⁻¹
HSP27 Oligonucleotide OD₂₆₀ = 153,000 M⁻¹ cm⁻¹; Rz_{260:280}= 1.819
Dia-Cys OD₂₈₀ = 0.57 (mg/ml)¹ cm⁻¹
PEG₄-SPDP (PDT) OD₃₄₃ = 8,080 M⁻¹ cm⁻¹
SAMSA-Fluorescein OD₄₉₅ = 64,500 M⁻¹ cm⁻¹; Rz_{280:495} = 0.232
Ellmans (TNB) OD₄₁₂ = 14,150 M⁻¹ cm⁻¹

### Immobilized metal ion affinity chromatography (IMCA)

Nickel-nitrilotriacetic acid (Ni-NTA) chromatography was performed to purify histidine-tagged protein and protein-conjugates. Briefly, Ni-NTA agarose (10 mL) was pipetted into a gravity flow column for 5 mL bed volume. The resin was washed with 20 mL deionized water and recharged with 5 mL of 100 mM NiSO4 solution. The resin was washed again five times with 5 mL deionized water and equilibrated five times with DPBS. The protein solution was incubated with the washed Ni-NTA agarose rotating at 4 °C for 30 min. Afterwards, the Ni-NTA protein solution was pipetted back into the gravity flow column. The flow through was collected and the resin was washed three times with 5 mL DPBS. The immobilized sample was then eluted by increasing the imidazole concentration from 50 mL of 125 mM, pH 8 to 50 mL of 250 mM, pH 8. Elution fractions were dialyzed against PBS pH 7.4 to obtain the purified sample.

### Size Exclusion Chromatography

Size exclusion chromatography (SEC) was carried out on an AKTA purifier. Samples were analyzed by SEC using either a Biosep SEC-S3000 column or an Sephadex G50M column (10 x 40 cm) eluting with TBS/ isopropyl alcohol solution (85:15 v/v). Sample purities were determined by integration of the antibody sample peak with respect to the trace aggregate peaks.

### Ellman's assay

Ellman's test (or Ellman's assay) was carried out to quantitatively determine thiol concentration in a sample via spectrophotometry. Presence of thiols was indicated via the stoichiometric release of the 2-nitro-5-thiobenzoate (TNB) from Ellman's reagent in the presence of thiols. TNB obtains an absorption maximum at 412 nm and an extinction coefficient of OD₄₁₂ = 14,150 M⁻¹ cm⁻¹ in buffered systems. Briefly, 2 µL of a 0.5 mg / mL solution of the Ellman's reagent (5,5-Dithiobis(2-nitrobenzoic acid), DTNB) in phosphate buffer (0.1 M, 1 mM EDTA, pH 7.4) was mixed with 20 µL of a thiol containing sample in buffer. The mixture was vortexed for 5 sec. Then, UV-Vis absorbance at 412 nm was measured on a Thermo Nanodrop 2000 to determine TNB concentration and thus thiol content of the sample.

### Dianthin production

Dianthin was expressed in a bacterium culture and the protein was purified following conventional cell culturing and protein purification steps known in the art.

### Production of Saporin conjugates

Custom trastuzumab-saporin cetuximab-saporin, CD71mab-saporin conjugates were produced and purchased from Advanced Targeting Systems (San Diego, CA). IgG-saporin and saporin was purchased from Advanced Targeting Systems

### Antibody-(Cys-dendron-(L-S01861)ⁿ)ⁿ synthesis

Trastuzumab and Cetuximab are referred hereafter as "Ab". Ab was conjugated to dendritic saponin [dendron-(L-SO1861)₄-maleimide] via a tetra(ethylene glycol) succinimidyl 3-(2-pyridyldithio)propionate (PEG₄-SPDP) linker conducting a thiole-ene Michael-type reaction between Ab and dendritic saponin. The procedure is exemplary described for Cetuximab-(S-dendron-(L-SO1861)₄)₄:
Cetuximab was desalted into DPBS pH 7.5 buffer and then normalized to 2.50 mg/ml. To an aliquot of Ab (9.19 mg, 61 nmol) was added an aliquot of freshly prepared PEG₄-SPDP solution (5.0 mg/ml, 6.70 mole equivalents, 411 nmol), the mixture vortexed briefly then incubated for 60 minutes at 20 °C with roller-mixing. After incubation, the reaction was quenched with the addition of glycine (20 mg/ml, 7.7 µl), then the SPDP moiety reduced *in situ* by the addition of TCEP (5.0 mg/ml, 4.0 mole equivalents per SPDP, 1.64 µmol). This mixture was roller-mixed for 15 minutes at 20 °C with roller-mixing. The resulting Ab-SH was purified by gel filtration using a zeba spin desalting column into TBS pH 7.5. The Ab-SH was characterized by UV-vis analysis and Ellman's assay (thiol to Ab ratio = 5.4). To the bulk Ab-SH (7.41 mg, 1.93 mg/ml, 49 nmol) was added an aliquot of freshly prepared dendron-(L-SO1861)₄-maleimide solution in DMSO (10 mg/ml, 8.0 mole equivalents per Ab, 0.4 µmol, 3.16 mg, 0.32 ml), the mixture vortexed briefly then incubated overnight at 20 °C. Besides the conjugation reaction, two aliquots of the desalted Ab-SH (0.25 mg, 1.67 nmol) were removed prior to conjugation, and were reacted with NEM (8.0 mole equivalents per Ab, 13.3 nmol, 6.7 µl of a 0.25 mg/ml solution) or TBS pH 7.5 buffer (6.7 µl) overnight at 20 °C, as positive and negative controls, respectively. After incubation for 18 hours (prior to addition of NEM), the crude conjugate mixture was centrifuged briefly and 100 µl aliquot removed for analysis by UV-vis and alongside positive and negative controls were characterized by Ellman's assay to obtain dendron-(L-SO1861)₄ incorporation. To the bulk Ab-dendron-(L-SO1861)₄mixture was added an aliquot of freshly prepared NEM solution (2.5 mg/ml, 5.0 mole equivalents, 0.25 µmol) and the mixture purified by 1.6 × 30 cm Sephadex G50 column eluting with DPBS pH 7.5 to give purified Cetuximab-(S-dendron-(L-SO1861)₄)₄ conjugate. The product was filtered to 0.2 µm to clarify and then concentrated carefully to *ca.* 3 mg/ml using a vivaspin T15 concentrator (3,000 g, 5 minute intervals, 5 °C) to give the final Cetuximab-(S-dendron(L-SO1861)₄)₄ conjugate. Yield: 4.41 mg, 48%. Dendron-(L-SO1861)₄ to Ab ratio = 4.4.

**Table 2. Summarized reaction outcomes**

| **Batch** | **Ab feed (mg)** | **PEG₄**-**SPDP mol equivalents** | **Dendron(SO1861)⁴**-**maleimide mol equivalents** | **Obtained DAR** | **Purity by analytical SEC** | **Yield** |
|---|---|---|---|---|---|---|
| **Trastuzumab-(Cys-dendron-(L-SO1861** )⁴)⁴ | 9.0 | 6.81 | 8 | 4.7 | 99.2 % | 2.34 mg (26%) |
| **Cetuximab-(Cys-dendron-(L-SO1861)⁴)⁴** | 9.2 | 6.7 | 8 | 4.4 | 96.7 % | 4.41 mg (48%) |

### Antibody-(L-SO1861)ⁿ (as illustrated in figure 51)

Trastuzumab, Cetuximab, are referred hereafter as "Ab". Ab was conjugated to the saponin SO18161-EMCH via Michael-type thiol-ene conjugation reaction at DARs of 1, 2, 3, 4, 5, and 6. The SO1861-EMCH molecule obtains a labil (L) hydrazone bond between its structure and its maleimide function generating a labil bond between the saponin and Ab. The procedure is exemplary described for Trastuzumab-(L-SO1861)₄:
To a solution of Cetuximab (40 mg, 8.0 ml) was added 10 µl/ml each of Tris concentrate (127 mg/ml, 1.05M), Tris.HCl concentrate (623 mg/ml, 3.95M) and EDTA-Na₂ concentrate (95 mg/ml, 0.26M) to give a 50mM TBS, 2.5mM EDTA buffer pH 7.5.

To Cetuximab divided into four portions (each of 9.73 mg, 4.864 mg/ml, 65 nmol) was added an aliquot of freshly prepared TCEP solution (0.5 -2.0 mg/ml, 1.15 - 7.02 mole equivalents, 75 - 455 nmol), the mixtures vortexed briefly then incubated for 300 minutes at 20 °C with roller-mixing. After incubation (prior to addition of SO1861-EMCH), a *ca.* 1 mg (0.210 ml) aliquot of Ab-SH was removed from each mixture and purified by gel filtration using a zeba spin desalting column into TBS pH 7.5. These aliquots were characterized by UV-vis analysis and Ellman's assay (thiol to Ab ratio = 2.0, 4.2, 5.9 and 6.8 respectively). To each of the bulk Ab-SH was added an aliquot of freshly prepared SO1861-EMCH solution (2 mg/ml, 1.3 mole equivalents per 'thiol', 0.15 - 0.61 µmol, 0.16 - 0.63 ml), the mixtures vortexed briefly then incubated for 120 minutes at 20 °C. Besides each conjugation reaction, two aliquots of desalted Ab-SH (0.25 mg, 1.67 nmol) were reacted with NEM (1.3 mole equivalents per 'thiol', 4.3 - 17.4 nmol, 2.2 - 8.7 µl of a 0.25 mg/ml solution) or TBS pH 7.5 buffer (2.2 - 8.7 µl) for 120 minutes at 20 °C, as positive and negative controls, respectively. After incubation (prior to addition of NEM), a 0.200 ml aliquot of Ab - SO1861-EMCH mixture was removed and purified by gel filtration using zeba spin desalting column into TBS pH 7.5. This aliquot was characterized by UV-vis and alongside positive and negative controls were characterized by Ellman's assay to obtain SO1861-EMCH incorporations. To the bulk Ab - SO1861-EMCH mixture was added an aliquot of freshly prepared NEM solution (2.5 mg/ml, 2.5 - 10 mole equivalents, 0.15 - 0.58 µmol) and the mixtures purified by zeba spin desalting columns eluting with DPBS pH 7.5 to give purified Cetuximab - (L-SO1861) conjugates. The products were normalized to 2.5 mg/ml and filtered to 0.2µm prior to dispensing for biological evaluation.

**Table 3. Summarized reaction conditions and results for Trastuzumab-L-SO1861 conjugates**

| **Batch** | **Ab feed** | **TCEP feed mole equivalents** | **SO1861-EMCH feed** | **Obtained DAR** | **Purity by analytical SEC (%)** | **Yield (%)** |
|---|---|---|---|---|---|---|
| **Tras-(L-SO1861**)₂ | 9.91 mg | 1.10 | 0.15 µmol | 1.6 | 99.2 | 79 |
| | 66 nmol | | | | | |
| **Tras-(L-SO1861**)₃ | 9.91 mg | 2.35 | 0.31 µmol | 3.0 | 99.0 | 81 |
| | 66 nmol | | | | | |
| **Tras-(L-SO1861**)₄ | 9.91 mg | 3.83 | 0.46 µmol | 4.0 | 98.4 | 81 |
| | 66 nmol | | | | | |
| **Tras-(L-SO1861)₅** | 9.91 mg | 5.77 | 0.62 µmol | 5.3 | 98.5 | 79 |
| | 66 nmol | | | | | |

**Table 4. Summarized reaction conditions and results for Cetuximab-L-SO1861 conjugates**

| **Batch** | **Ab feed** | **TCEP feed mole equivalents** | **SO1861-EMCH feed** | **Obtained DAR** | **Purity by analytical SEC (%)** | **Yield (%)** |
|---|---|---|---|---|---|---|
| **Cet-(L-SO1861)₁** | 9.73 mg | 1.15 | 0.15 µmol | 1.4 | 99.7 | 74 |
| | 65 nmol | | | | | |
| **Cet-(L-SO1861)₃** | 9.73 mg | 2.49 | 0.31 µmol | 2.8 | 99.6 | 80 |
| | 65 nmol | | | | | |
| **Cet-(L-SO1861**)₄ | 9.73 mg | 4.19 | 0.46 µmol | 4.1 | 99.0 | 77 |
| | 65 nmol | | | | | |
| **Cet-(L-SO1861**)₆ | 9.73 mg | 7.02 | 0.61 µmol | 5.6 | 98.3 | 80 |
| | 65 nmol | | | | | |

### Antibody-(S-SO1861)ⁿ synthesis

### SO1861-S-Mal synthesis

SO1861 from Saponaria officinalis L (15.4 mg, 8.28 µmol) and HATU (140 mg, 368 µmol, 44.5 mole equivalents) were placed as solid into a 20 mL glas vial with magnetic stirrer and 5 mL DMSO was added to dissolve the materials. The dissolved mixture was stirred for 30 min at room temperature. After 30 min, 1 mL of freshly prepared AEM solution (65 mg, 256 µmol, 31 mole equivalents) in DMSO was added to the stirring SO1861-HATU mixture. The reaction mixture was stirred for 17 hours at room temperature. After stirring for 17 hours, the mixture was diluted with deionized water and dialyzed extensively for 24 h against deionized water using regenerated cellulose membrane tubes (Spectra/Por 7) with a MWCO of 1 kDa. After dialysis, the solution was lyophilized to obtain a white powder.

Yield: 7.22 mg (44 %).

Dried aliquots were further used for characterization via MALDI-TOF-MS.

MALDI-TOF-MS (RN mode): *m*/*z* 1983 Da ([M - H]⁻, SO1861-S-Mal conjugate), 2136 Da ([M - H]⁻, saponin-S-Mal conjugate).

MALDI-TOF-MS (RP mode): *m*/*z* 2007 Da ([M + Na]⁺, SO1861-S-Mal conjugate), 2107 Da ([M - Na]⁺, saponin-S-Mal conjugate).

Maleimide functionality was verfied by mixing the purified SO1861-S-Mal with a freshly prepared L-cysteine solution (1000 mole excess) and observing the expected mass shift in MALDI-TOF-MS yielding a cysteine-conjugate at m/z 2103 Da.

### Antibody conjugation

Trastuzumab and Cetuximab are referred hereafter as "Ab". Ab was conjugated to the saponin SO18161-S-Mal via Michael-type thiol-ene conjugation reaction. The saponin obtains a stable (S) amide bond between its structure and its maleimide function generating a stable bond between the saponin and Ab.

5 mg of Ab dissolved in phosphate buffer saline (PBS) was buffer exchanged into tris(hydroxymethyl)-aminomethan buffer saline (TBS) pH 7.5 via zeba spin desalting column and normalized to a concentration of 3 mg/mL. To Ab was added an aliquot of freshly prepared tris(2-carboxyethyl)phosphine (TCEP) solution (0.25 mg/mL, 2.92 mole equivalents), the mixture vortexed briefly then incubated for 90 min at 20 °C. After, the resulting Ab-SH was purified by gel filtration using zeba spin desalting column into TBS pH 7.5. An aliquot was analyzed by Ellman's assay to ascertain sulfhydryl content. The Ab-SH obtained was split into a single portion for conjugation and two aliquots of 0.25 mg for controls. To the main portion of AB-SH was added an aliquot of freshly prepared SO1861-S-Mal solution (2 mg/mL, 2 mol equivalents with respect to sulhydryl content), to the second control aliquot was added buffer TBS pH 7.5. Each mixture was vortexed briefly then incubated for 2 hours at 20 °C. After, an aliquot from each was analyzed by Ellman's assay to ascertain sulfhydryl content remaining in the conjugate with respect to controls. After purification by gel filtration via zeba spin desalting column into Dulbecco's PBS pH 7.1, Ab-S-SO1861 was obtained. The conjugate was further analyzed by SEC to ascertain % purity. For the Trastuzumab-(S-SO1861)₄ sample a purity of 99 % was determined. For the Cetuximab-(S-SO1861)₄ sample a purity of 98.3 % was determined. Retention volumes for Trastuzumab-(S-SO1861)₄ (9.1 mL) and Cetuximab-(S-SO1861)₄ (8.8 mL) were noted to be similar and are consistent with a non-modified Ab (e.g. IgG).

### Antibody-(L-HSP27 BNA)ⁿ

### Trastuzumab-(L-HSP27)⁴,, Cetuximab-(L-HSP27)⁴, synthesis via PEG₄-SPDP with a DAR4 and Cetuximab-(L-HSP27)² synthesis via PEG₄-SPDP with a DAR2

Trastuzumab, Cetuximab, are referred hereafter as "Ab". Ab was conjugated to HSP27 BNA disulfide via a tetra(ethylene glycol) succinimidyl 3-(2-pyridyldithio)propionate (PEG₄-SPDP) linker forming a labil (L) disulfide bond between Ab and HSP27 BNA . The procedure is exemplary described for Trastuzumab-(L-HSP27 BNA)₄:
HSP27 BNA disulfide oligo (2.7mg, 470 nmol, 6.10 mg/ml) was reacted with TCEP (10 mole equivalents, 4.7 µmol, 1.34 mg, 50 mg/ml) for 30 minutes at 20 °C with roller mixing. After, the oligo-SH was purified by PD10 G25 desalting column eluting into TBS pH 7.5 and used promptly. Oligo-SH was obtained (2.48 mg, 90%, 1.24 mg/ml, SH to oligo ratio = 0.8)

Trastuzumab (1.5 mg, 10.3 nmol, 2.50 mg/ml) was reacted with an aliquot of freshly prepared PEG₄-SPDP solution (6.81 mole equivalents, 70.1 nmol, 39 µg) in DMSO (1 mg/ml) for 60 minutes at 20 °C with roller mixing. After, the reaction was quenched with glycine (15.1 µl of 2 mg/ml freshly prepared solution in TBS pH 7.5) and then desalted via zeba desalting column eluting with TBS pH 7.5. An aliquot of the resulting Tras-S-PEG₄-SPDP was taken out and tested by UV-Vis analysis. SPDP incorporation was determined using TCEP to liberate pyridiyl-2-thione (PDT) and by UV-vis analysis at 343 nm (SPDP to Ab ratio: 4). The remaining Tras-(S-PEG₄-SPDP)₄ was reacted with an aliquot of freshly prepared HSP27 oligonucleotide (oligo-SH) (8 mole equivalents, 82.4 nmol, 1.24 mg/ml) and incubated overnight at 20 °C with roller mixing. After 17 hours, the conjugate was analysed by UV-vis analysis to ascertain incorporation of HSP27 by displacement of pyridiyl-2-thione (PDT) at 343 nm. The crude conjugate was purified using a 1.6 × 33 cm Sephadex G50 column eluting with DPBS pH 7.5. The resulting Trastuzumab-(L-HSP27)₄ was obtained as a single fraction. Yield: n.d.. Purity: 96% , HSP27 BNA to Ab ratio = 4.4

**Table 5. Summarized reaction conditions and results**

| **Batch** | **PEG₄**-**SPDP mol equivalents** | **HSP27 (oligo-SH) mol equivalents** | **Obtained DAR** | **Purity by analytical SEC (%)** | **Yield (%)** |
|---|---|---|---|---|---|
| **Tras-(L-HSP27 BNA)⁴** | 6.81 | 8 | 4.4 | 96.0 | n.d. |
| **Cet- (L-HSP27 BNA)⁴** | 6.70 | 8 | 3.9 | 93.9 | n.d. |
| **Cet-(L-HSP27)²** | 2.3 | 3.6 | 1.5 | 94.9 | 87 |

| | | | | | |
|---|---|---|---|---|---|
| n.d. = not determined | | | | | |

### Antibody-(L-HSP27 BNA-L-blocked)"

### Trastuzumab-(L-HSP27-L-blocked)⁴,, Cetuximab-(L-HSP27-L-blocked)

Trastuzumab and Cetuximab are referred hereafter as "Ab". Ab was conjugated a maleimido (Mal) bearing HSP27 derivate which is referred hereafter as "HSP27-Mal". Ab was conjugated to the HSP27-Mal via Michael-type thiol-ene conjugation reaction. The HSP17-Mal obtains a labile (L) hydrazone bond between its structure and its maleimide function generating a labile bond between the HSP27 BNA and Ab. The procedure is exemplary described for Trastuzumab-(L-HSP27 BNA-L-blocked)ⁿ:
Trastuzumab was reconstituted to 21 mg/ml with deionized water (DI), then diluted to 5 mg/ml using histidine buffer pH 6. To a 20 mg (4.0 ml) aliquot was added 10 µl/ml each of Tris concentrate (127 mg/ml, 1.05M), Tris.HCl concentrate (623 mg/ml, 3.95M) and EDTA-Na₂ concentrate (95 mg/ml, 0.26M) to give a 50mM TBS, 2.5mM EDTA buffer pH 7.5. To Trastuzumab (20.30 mg, 4.920 mg/ml, 0.14 µmol) was added an aliquot of freshly prepared TCEP solution (1.00 mg/ml, 2.35 mole equivalents, 0.32 µmol), the mixture vortexed briefly then incubated for 90 minutes at 20 °C with roller-mixing. After incubation (prior to addition of HSP27-Mal), a *ca.* 2 mg (0.439 ml) aliquot of Ab-SH was removed from each mixture and purified by gel filtration using a zeba spin desalting column into TBS pH 7.5. This aliquot was characterized by UV-vis analysis and Ellman's assay (thiol to ab ratio = 4.0). To the bulk Ab-SH (5.1 mg, 35 nmol) was added an aliquot of the HSP27-Mal derivative (freshly prepared in TBS pH 7.5, 2 mg/ml, 1.3 mole equivalents per 'thiol', 182 nmol), the mixture vortexed briefly then incubated for 120 minutes at 20 °C. Besides the Trasuzumab - HSP27 BNA derivative conjugation reaction, two aliquots of desalted Ab-SH (0.5 mg, 3.3 nmol) were reacted with NEM (1.3 mole equivalents per 'thiol', 17.3 nmol, 6.7 µl of a 0.25 mg/ml solution) or TBS pH 7.5 buffer (6.7 µl) for 120 minutes at 20 °C, as positive and negative controls, respectively. After incubation (prior to addition of NEM), a 0.100 ml aliquot of Ab - HSP27 BNA mixture was removed and purified by gel filtration using zeba spin desalting column into TBS pH 7.5. This aliquot was characterized by UV-vis and alongside positive and negative controls was characterized by Ellman's assay to obtain HSP27 incorporation. To the bulk Ab - HSP27 mixture was added an aliquot of freshly prepared NEM solution (0.25 mg/ml, 2.5 mole equivalents, 89 nmol) and the mixture purified by gel filtration using a 1.6 x 30 cm Sephadex G50M eluting with DPBS pH 7.5 followed by repeated centrifugal filtration and washing using a 100 KDa MWCO concentrator to give purified Trastuzumab-(L-HSP27-L-blocked)₄ conjugate. The products were filtered to 0.2µm prior to dispensing for biological evaluation.

**Table 6. Summarized reaction conditions and results**

| **Batch** | **Ab-SH feed** | **Thiol to Ab ratio** | **HSP27-Mal feed** | **Obtained DAR** | **Purity by analytical SEC (%)** | **Yield (%)** |
|---|---|---|---|---|---|---|
| **Tras-(L-HSP27 BNA-L-blocked)⁴** | 5.1 mg | 3.98 | 1.36 mg | 3.6 | 99.4 | 40 |
| | 35 nmol | | | | | |
| **Cet-(L-HSP27 BNA-L-blocked)⁴** | 5.1 mg | 4.16 | 1.36 mg | 3.6 | 97.9 | 50 |
| | 35 nmol | | | | | |

### Antibody-(Cys-Trifunctional linker-(L-SO1861)-(L-HSP27 BNA))ⁿ

### Trastuzumab-[S-Tri-(L-SO1861)-(L-HSP27)]⁴, Trastuzumab-[S-Tri-(blocked)-(L-HSP27)]⁴, Cetuximab-[S-Tri-(L-SO1861)-(L-HSP27)]⁴, Cetuximab-[S-Tri-(blocked)-(L-HSP27)]⁴

Trastuzumab and Cetuximab are referred hereafter as "Ab". Ab was conjugated via Michael-type thiol-ene reaction to two different maleimide (Mal) bearing HSP27 BNA derivatives which are referred hereafter as "HSP27-Mal". These HSP27-Mal derivatives were namely: 1) Mal-Trifunctional linker-(L-SO1861)-(L-HSP27), 2) Mal-Trifunctional linker-(blocked)-(L-HSP27). The procedure is exemplary described for Trastuzumab-[S-Trifunctional linker-(L-SO1861)-(L-HSP27 BNA)]⁴:
Trastuzumab was reconstituted to 21 mg/ml with deionized water (DI), then diluted to 5 mg/ml using histidine buffer pH 6. To a 20 mg (4.0 ml) aliquot was added 10 µl/ml each of Tris concentrate (127 mg/ml, 1.05M), Tris.HCl concentrate (623 mg/ml, 3.95M) and EDTA-Na₂ concentrate (95 mg/ml, 0.26M) to give a 50mM TBS, 2.5mM EDTA buffer pH 7.5.

To Trastuzumab (20.30 mg, 4.920 mg/ml, 0.14 µmol) was added an aliquot of freshly prepared TCEP solution (1.00 mg/ml, 2.35 mole equivalents, 0.32 µmol), the mixture vortexed briefly then incubated for 90 minutes at 20 °C with roller-mixing. After incubation (prior to addition of construct), a *ca.* 2 mg (0.439 ml) aliquot of Ab-SH was removed from each mixture and purified by gel filtration using a zeba spin desalting column into TBS pH 7.5. These aliquots were characterized by UV-vis analysis and Ellman's assay (thiol to ab ratio = 4.0). The bulk Ab-SH was split into two aliquots (1.1 mg, 7.6 nmol and 1.2 mg, 8.3 nmol), and to each aliquot was added an aliquot of each of the HSP27 BNA-Mal derivatives **1 - 2** (freshly prepared in TBS pH 7.5, 2 mg/ml, 1.3 mole equivalents per 'thiol', 40 nmol and 43 nmol), the mixtures vortexed briefly then incubated for 120 minutes at 20 °C. Besides the Trastuzumab - HSP27 BNA derivatives 2 conjugation reaction, two aliquots of desalted Ab-SH (0.5 mg, 3.3 nmol) were reacted with NEM (1.3 mole equivalents per 'thiol', 17.3 nmol, 6.7 µl of a 0.25 mg/ml solution) or TBS pH 7.5 buffer (6.7 µl) for 120 minutes at 20 °C, as positive and negative controls, respectively. After incubation (prior to addition of NEM), a 0.100 ml aliquot of Ab - construct **2** mixture was removed and purified by gel filtration using zeba spin desalting column into TBS pH 7.5. This aliquot was characterized by UV-vis and alongside positive and negative controls was characterized by Ellman's assay to obtain HSP27 BNA derivatives **2** incorporation. To each bulk Ab - construct mixture was added an aliquot of freshly prepared NEM solution (0.25 mg/ml, 2.5 mole equivalents, 19 and 21 nmol) and the mixtures purified by gel filtration using a 1.6 × 30 cm Sephadex G50M eluting with DPBS pH 7.5 followed by repeated centrifugal filtration and washing using a 100 KDa MWCO concentrator to give purified Trastuzumab - construct **1 - 2** conjugates. The products were filtered to 0.2µm prior to dispensing for biological evaluation.

**Table 7. Summarized reaction conditions and results**

| **Batch** | **Ab-SH feed** | **TCEP equivalents** | **Thiol to ab ratio** | **Purity by analytical SEC (%)** | **Yield (%)** |
|---|---|---|---|---|---|
| **Tras-[S-Trifunctional linker-(L-SO1861)-(L-HSP27 BNA)]⁴** | 1.1 mg | 2.35 | 3.98 | 97.0 | 57 |
| | 7.6 nmol | | | | |
| **Tras-[S-Trifunctional linker-(blocked)-(L-HSP27 BNA)]⁴** | 1.2 mg | 2.35 | 3.98 | 96.6 | 33 |
| | 8.3 nmol | | | | |
| **Cet-[S-Trifunctional linker-(L-SO1861)-(L-HSP27 BNA)]⁴** | 1.1 mg | 2.72 | 4.16 | 98.9 | 47 |
| | 7.6 nmol | | | | |
| **Cet-[S-Trifunctional linker-(blocked)-(L-HSP27 BNA)]⁴** | 1.2 mg | 2.72 | 4.16 | 99.1 | 50 |
| | 8.3 nmol | | | | |

### Antibody-(L-SO1861)ⁿ-(L-HSP27 BNA)ⁿ

### Trastuzumab-(L-SO1861)⁴-(L-HSP27)⁴, Cetuximab-(L-SO1861)⁴-(L-HSP27)⁴ synthesis via PEG₄-SPDP with a DAR4 Cetuximab-(L-SO1861)⁴-(L-HSP27)² (FBR703 STB17/7-8) synthesis via PEG₄-SPDP with a DAR2

Trastuzumab-(L-SO1861)₄, Cetuximab-(L-SO1861)₄ are referred hereafter as "Ab". Ab was conjugated to HSP27 BNA disulfide via a tetra(ethylene glycol) succinimidyl 3-(2 pyridyldithio)propionate (PEG₄-SPDP) linker forming a labil (L) disulfide bond between Ab and HSP27 BNA. The procedure is exemplary described for Trastuzumab-(L-SO1861)₄-(L-HSP27)₄:
HSP27 BNA disulfide oligo (2.7mg, 470 nmol, 6.10 mg/ml) was reacted with TCEP (10 mole equivalents, 4.7 µmol, 1.34 mg, 50 mg/ml) for 30 minutes at 20 °C with roller mixing. After, the oligo-SH was purified by PD10 G25 desalting column eluting into TBS pH 7.5 and used promptly. Oligo-SH was obtained (2.48 mg, 90%, 1.24 mg/ml, SH to oligo ratio = 0.8)
Trastuzumab-(L-SO1861)₄ (1.3 mg, 8.7 nmol, 2.50 mg/ml) was reacted with an aliquot of freshly prepared PEG₄-SPDP solution (9.26 mole equivalents, 80.3 nmol, 45 µg) in DMSO (1 mg/ml) for 60 minutes at 20 °C with roller mixing. After, the reaction was quenched with glycine (15.1 µl of 2 mg/ml freshly prepared solution in TBS pH 7.5) and then desalted via zeba desalting column eluting with TBS pH 7.5. An aliquot of the resulting Tras-(L-SO1861)-(L-PEG₄-SPDP) was taken out and tested by UV-Vis analysis. SPDP incorporation
was determined using TCEP to liberate pyridiyl-2-thione (PDT) and by UV-vis analysis at 343 nm (SPDP to Ab ratio = 4). The remaining Tras-(L-SO1861)-(L-PEG₄-SPDP) was reacted with an aliquot of freshly prepared HSP27 oligonucleotide (oligo-SH) (8 mole equivalents, 54.8 nmol, 0.32 mg, 1.24 mg/ml) and incubated overnight at 20 °C with roller mixing. After 17 hours, the conjugate was analyzed by UV-vis analysis to ascertain incorporation of HSP27 by displacement of pyridiyl-2-thione (PDT) at 343 nm. The crude conjugate was purified using a 1.6 × 33 cm Sephadex G50 column eluting with DPBS pH 7.5. The resulting Trastuzumab-(L-SO1861)₄-(L-HSP27 BNA)₄ was obtained as a single fraction. Yield: 0.47 mg, 45% (0.49 mg/ml), HSP27 to Ab ratio = 3.5

**Table 8. Summarized reaction conditions and results**

| **Batch** | **PEG₄**-**SPDP mol equivalents** | **HSP27 (oligo-SH) mol equivalents** | **Obtained DAR** | **Purity by analytical SEC (%)** | **Yield (%)** |
|---|---|---|---|---|---|
| **Tras-(L-SO1861)⁴**-**(L-HSP27 BNA)⁴** | 9.26 | 8 | 3.5 | 85.1 | 45 |
| **Cet-(L-SO1861)₄-( L-HSP27 BNA)⁴** | 7.21 | 8 | 3.8 | 80.8 | n.d. |
| **Cet-(L-SO1861)₄-( L-HSP27 BNA)²** | 3.34 | 3.6 | 1.8 | 76.2 | 81 |

| | | | | | |
|---|---|---|---|---|---|
| n.d. = not determined | | | | | |

### Antibody-(L-QS Mix)"

Trastuzumab, Cetuximab, are referred hereafter as "Ab". Ab was conjugated to the saponin QS Mix-EMCH via Michael-type thiol-ene conjugation reaction. The procedure is exemplary described for Trastuzumab-L-QS Mix:
Trastuzumab ("Ab", 600 mg) was reconstituted to 21 mg/mL with deionized water (DI), then diluted to 5 mg/mL using freshly prepared histidine buffer pH 6 (5mM histidine pH 6, 2% trehalose, 0.01% Tween 20). 10 µL/mL each of Tris concentrate (127 mg/mL, 1.05M), Tris.HCL concentrate (623 mg/mL, 3.95M) and EDTA-Na₂ concentrate (95 mg/ml, 0.26M) was added to give a 50mM TBS, 2.5mM EDTA buffer pH 7.5. To Trastuzumab (603.8 mg, 4.887 mg/mL, 4.0 µmol) was added an aliquot of freshly prepared TCEP solution (1 mg/mL, 2.35 mole equivalents, 9.5 µmol, 2.72 mg), the mixture swirled by hand to mix then incubated for 90 minutes at 20 °C with roller-mixing. After incubation (prior to addition of QS Mix-EMCH), a 2 mg (0.409 mL) aliquot of Ab-SH was removed and purified by gel filtration using zeba spin desalting column into TBS pH 7.5. This aliquot was characterized by UV-vis analysis and Ellman's assay. To the bulk Ab-SH was added an aliquot of freshly prepared QS Mix-EMCH solution (2 mg/mL, 5.2 mole equivalents, 21 µmol, 21.6 mL), the mixtures vortexed briefly then incubated for 120 minutes at 20 °C. Besides the conjugation reaction, two aliquots of desalted Ab-SH (0.5 mg, 0.134 mL, 3.33 nmol) were reacted with NEM (8.00 equivalents, 26.6 nmol, 3.3 µg , 13.3 µL of a 0.25 mg/mL solution) or TBS pH 7.5 buffer (13.3 µL) for 120 minutes at 20 °C, as positive and negative controls, respectively. After incubation (prior to addition of NEM), a ca. 2 mg (0.481 mL) aliquot of Ab - QS Mix- EMCH mixture was removed and purified by gelfiltration using zeba spin desalting column into TBS pH 7.5. This aliquot was characterized by UV-vis and alongside positive and negative controls were characterized by Ellman's assay to obtain QS Mix-EMCH incorporations. To the bulk Ab - QS Mix-EMCH mixture was added an aliquot of freshly prepared NEM solution (2.5 mg/mL, 5 mole equivalents, 20 µmoL, 2.51 mg) and the mixture stored at 2-8 °C overnight. The conjugate was purified by 10 × 40 cm Sephadex G50M column eluting with DPBS pH 7.5 to give purified Trastuzumab - (L-QS Mix) conjugate. The product as a whole was concentrated then normalized to 5 mg/mL using a vivacell 100 concentrator (2,000 g, 4 °C, 200 minutes). The product were filtered to 0.2µm and dispensed for biological evaluation. Yield: n.d. QS Mix to Ab ratio = 4.1.

**Table 9. Summarized reaction outcomes**

| **Method** | **Measure** | **Result** |
|---|---|---|
| Analytical SEC | Purity | n.d. |
| Yield | Mass (percent) | n.d. |
| Ellman's assay | QS Mix-EMCH incorpoation | 4.1 |

### Antibody-(L-HSP27BNA-L-S01861)ⁿ

*Trastuzumab-(L-HSP27BNA-L-SO1861*)*⁴, Cetiximab-*(*L-HSP27BNA-L-SO1861*)*⁴ with a DAR4* Trastuzumab and Cetuximab are referred hereafter as "Ab". Ab was conjugated a saponin SO1861 and maleimido (Mal) bearing HSP27 derivate which is referred hereafter as "HSP27-Mal". Ab was conjugated to the HSP27-Mal via Michael-type thiol-ene conjugation reaction. The HSP17-Mal obtains a labile (L) hydrazone bond between its structure and its maleimide function generating a labile bond between the HSP27 BNA and Ab. The procedure is exemplary described for Trastuzumab-(L-HSP27 BNA-L-SO1861)₄:
Trastuzumab was reconstituted to 21 mg/ml with deionized water (DI), then diluted to 5 mg/ml using histidine buffer pH 6. To a 20 mg (4.0 ml) aliquot was added 10 µl/ml each of Tris concentrate (127 mg/ml, 1.05M), Tris.HCl concentrate (623 mg/ml, 3.95M) and EDTA-Na₂ concentrate (95 mg/ml, 0.26M) to give a 50mM TBS, 2.5mM EDTA buffer pH 7.5. To Trastuzumab (20.30 mg, 4.920 mg/ml, 0.14 µmol) was added an aliquot of freshly prepared TCEP solution (1.00 mg/ml, 2.35 mole equivalents, 0.32 µmol), the mixture vortexed briefly then incubated for 90 minutes at 20 °C with roller-mixing. After incubation (prior to addition of HSP27-Mal), a *ca.* 2 mg (0.439 ml) aliquot of Ab-SH was removed from each mixture and purified by gel filtration using a zeba spin desalting column into TBS pH 7.5. This aliquot was characterized by UV-vis analysis and Ellman's assay (thiol to ab ratio = 4.0). To the bulk Ab-SH (4.7 mg, 32 nmol) was added an aliquot of the HSP27-Mal derivative (freshly prepared in TBS pH 7.5, 2 mg/ml, 1.3 mole equivalents per 'thiol', 166 nmol), the mixture vortexed briefly then incubated for 120 minutes at 20 °C. Besides the Trasuzumab - HSP27 BNA derivative conjugation reaction, two aliquots of desalted Ab-SH (0.5 mg, 3.3 nmol) were reacted with NEM (1.3 mole equivalents per 'thiol', 17.3 nmol, 6.7 µl of a 0.25 mg/ml solution) or TBS pH 7.5 buffer (6.7 µl) for 120 minutes at 20 °C, as positive and negative controls, respectively. After incubation (prior to addition of NEM), a 0.100 ml aliquot of Ab - HSP27 BNA mixture was removed and purified by gel filtration using zeba spin desalting column into TBS pH 7.5. This aliquot was characterized by UV-vis and alongside positive and negative controls was characterized by Ellman's assay to obtain HSP27 incorporation. To the bulk Ab - HSP27 mixture was added an aliquot of freshly prepared NEM solution (0.25 mg/ml, 2.5 mole equivalents, 80 nmol) and the mixture purified by gel filtration using a 1.6 × 30 cm Sephadex G50M eluting with DPBS pH 7.5 followed by repeated centrifugal filtration and washing using a 100 KDa MWCO concentrator to give purified Trastuzumab-(L-HSP27 BNA-L-SO1861)₄ conjugate. The products were filtered to 0.2µm prior to dispensing for biological evaluation.

**Table 10. Summarized reaction conditions and results**

| **Batch** | **Ab-SH feed** | **Thiol to Ab ratio** | **HSP27-Mal feed** | **Purity by analytical SEC (%)** | **Yield (%)** |
|---|---|---|---|---|---|
| **Tras-(L-HSP27 BNA-L-SO1861**)⁴ | 4.7 mg | 3.98 | 1.57 mg | 99.4 | 40 |
| | 32 nmol | | | | |
| **Cet-(L-HSP27 BNA-L-SO1861**)⁴ | 4.7 mg | 4.16 | 1.57 mg | 97.9 | 50 |
| | 32 nmol | | | | |

### Antibody-(L-SO1861)"-(S-Dianthin)"

### Trastuzumab-(L-SO1861)₄-(S-Dianthin)₂ and Cetuximab-(L-SO1861)₄-(S-Dainthin)₂ synthesis via SMCC with a DAR2

Trastuzumab-L-SO1861 and Cetuximab-L-SO1861 are referred hereafter as "Ab". Ab was conjugated to Dianthin-Cys via a succinimidyl 4-(N-maleimidomethyl)cyclohexane-1-carboxylate (SMCC) linker providing a stable (S) amide bond between Ab and Dianthin. The procedure is exemplary described for Trastuzumab-(L-SO1861)₄-(S-Dianthin)₂:
Dianthin-Cys (7.8 mg, 261 nmol, 0.78 mg/ml) was reacted with TCEP (5 mole equivalents, 1.31 µmol, 0.37 mg, 1 mg/ml) for 30 minutes at 20 °C with roller mixing. After, the protein-SH was purified by zeba desalting column eluting into TBS pH 7.5 and used promptly. Protein-SH was obtained (5.2 mg, 67%, 0.52 mg/ml, SH to protein ratio = 1 ± 0.1).

Trastuzumab-(L-SO1861)₄ (1.5 mg, 10 nmol, 2.50 mg/ml) was reacted with an aliquot of freshly prepared SMCC solution (4.83 mole equivalents, 48.3 nmol, 16 µg) in DMSO (0.5 mg/ml) for 60 minutes at 20 °C with roller mixing. After, the reaction was quenched with glycine (18.1 µl of 1 mg/ml freshly prepared solution in TBS pH 7.5) and then desalted via zeba desalting column eluting with TBS pH 7.5. The resulting Tras-(L-SO1861)-(S-SMCC) (1.22 mg, 8.1 nmol, 2.03 mg/ml) was reacted with an aliquot of freshly reduced Dianthin-Cys (3.2 mole equivalents, 32.5 nmol, 0.97 mg, 0.52 mg/ml) and incubated overnight at 20 °C with roller mixing. After 17 hours, the conjugate was concentrated to ≤1 ml using a vivaspin T4 concentrator and purified using a 1.6 × 37 cm Superdex 200PG column eluting with DPBS pH 7.5. The resulting Tras-(L-SO1861)₄-(S-Dianthin)₂ conjugate was obtained as High-Molecular-Weight (HMW) (0.36 mg, 30%, 0.34 mg/ml, Dianthin to Ab ratio = not determined) and Low-Molecular-Weight (LMW) (0.49 mg, 40%, 0.30 mg/ml, Dianthin to Ab ratio = not determined) fractions. Yield: n.d. Purity: 79.3 %.

**Table 11. Summarized reaction conditions and results**

| **Batch** | **SMCC mol equivalents** | **Dianthin-Cys mol equivalents** | **Obtained DAR** | **Purity by analytical SEC (%)** | **Yield (%)** |
|---|---|---|---|---|---|
| **Tras-(L-SO1861**)₄-**(S-Dianthin)ₓ** | 4.82 | 3.2 | n.d. | 79.3 | n.d. |
| **Cet-(L-SO1861**)₄-**(S-Dianthin)₂** | 4.46 | 3.2 | 2.0 | 87.6 | 92 |

| | | | | | |
|---|---|---|---|---|---|
| n.d. = not determined | | | | | |

### 12. Antibody-(S-Dianthin)"

### Trastuzumab-(S-Dianthin)², Cetuximab-(S-Dianthin)², synthesis via SMCC with a DAR2

Trastuzumab and Cetuximab are referred hereafter as "Ab". Ab was conjugated to Dianthin-Cys via a succinimidyl 4-(N-maleimidomethyl)cyclohexane-1-carboxylate (SMCC) linker providing a stable (S) amide bond between Ab and Dianthin. The procedure is exemplary described for Trastuzumab-(S-Dianthin)₂:
Dianthin-Cys (7.8 mg, 261 nmol, 0.78 mg/ml) was reacted with TCEP (5 mole equivalents, 1.31 µmol, 0.37 mg, 1 mg/ml) for 30 minutes at 20 °C with roller mixing. After, the protein-SH was purified by zeba desalting column eluting into TBS pH 7.5 and used promptly. Protein-SH was obtained (5.2 mg, 67%, 0.52 mg/ml, SH to protein ratio = 1 ± 0.1).

Trastuzumab (1.5 mg, 10 nmol, 2.50 mg/ml) was reacted with an aliquot of freshly prepared SMCC solution (3.16 mole equivalents, 31.6 nmol) in DMSO (0.5 mg/ml) for 60 minutes at 20 °C with roller mixing. After, the reaction was quenched with glycine (18.1 µl of 1 mg/ml freshly prepared solution in TBS pH 7.5) and then desalted via zeba desalting column eluting with TBS pH 7.5. The resulting Tras - (S-SMCC) (1.22 mg, 8.1 nmol, 2.03 mg/ml) was reacted with an aliquot of freshly reduced Dianthin-Cys (3.2 mole equivalents, 32.5 nmol, 0.97 mg, 0.52 mg/ml) and incubated overnight at 20 °C with roller mixing. After 17 hours, the conjugate was concentrated to ≤1 ml using a vivaspin T4 concentrator and purified using a 1.6 × 37 cm Superdex 200PG column eluting with DPBS pH 7.5.

Yield: n.d. Purity: 99 %.

**Table 12. Summarized reaction conditions and results**

| **Batch** | **SMCC mol equivalents** | **Dianthin-Cys mol equivalents** | **Obtained DAR** | **Purity by analytical SEC (%)** | **Yield (%)** |
|---|---|---|---|---|---|
| **Tras-(S-Dianthin)ₓ** | 3.16 | 3.2 | n.d. | 99.0 | n.d. |
| **Cet-(S-Dianthin)₂** | 3.70 | 3.2 | 1.6 | 97.2 | 93 |

| | | | | | |
|---|---|---|---|---|---|
| n.d. = not determined | | | | | |

### Antibody-(L-SO1861)ⁿ-(L-Dianthin)ⁿ

### Trastuzumab-(L-SO1861)₄-(L-Dainthin)₂, and Cetuximab-(L-SO1861)₄-(L-Dainthin)₂ synthesis via PEG₄-SPDP with a DAR2

Trastuzumab-L-SO1861, and Cetuximab-L-SO1861 are referred hereafter as "Ab". Ab was conjugated to Dianthin-Cys via a tetra(ethylene glycol) succinimidyl 3-(2-pyridyldithio)propionate (PEG₄-SPDP) linker forming a labil (L) disulfide bond between Ab and Dianthin. The procedure is exemplary described for Trastuzumab-L-SO1861:
Dianthin-Cys (7.8 mg, 261 nmol, 0.78 mg/ml) was reacted with TCEP (5 mole equivalents, 1.31 µmol, 0.37 mg, 1 mg/ml) for 30 minutes at 20 °C with roller mixing. After, the protein-SH was purified by zeba desalting column eluting into TBS pH 7.5 and used promptly. Protein-SH was obtained (5.2 mg, 67%, 0.52 mg/ml, SH to protein ratio = 1 ± 0.1).

Trastuzumab-(L-SO1861)₄ (0.75 mg, 5 nmol, 2.50 mg/ml) was reacted with an aliquot of freshly prepared PEG₄-SPDP solution (4.95 mole equivalents, 24.75 nmol, 14 µg) in DMSO (1 mg/ml) for 60 minutes at 20 °C with roller mixing. After, the reaction was quenched with glycine (18.1 µl of 1 mg/ml freshly prepared solution in TBS pH 7.5) and then desalted via zeba desalting column eluting with TBS pH 7.5. An aliquot of the resulting Tras-(L-SO1861)-(S-PEG₄-SPDP) was taken out and tested by UV-Vis analysis. SPDP incorporation was determined using TCEP to liberate pyridiyl-2-thione (PDT) and by UV-vis analysis at 343 nm (SPDP to Ab ratio = 2.4). The remaining Tras-(L-SO1861)-(S-PEG₄-SPDP) was reacted with an aliquot of freshly prepared Dianthin-Cys (protein-SH) (4 mole equivalents, 20 nmol, 0.6 mg, 0.52 mg/ml) and incubated overnight at 20 °C with roller mixing. After 17 hours, an aliquot of the conjugate was analyzed by UV-vis analysis to ascertain incorporation of Dianthin-Cys by displacement of PDT. After, the conjugate was concentrated to ≤1 ml using a vivaspin T4 concentrator and purified using a 1.6 × 37 cm Superdex 200PG column eluting with DPBS pH 7.5. Dianthin to Ab ratio = 2). Yield: n.d. Purity: 60.5 %.

**Table 13. Summarized reaction conditions and results**

| **Batch** | **PEG₄-SPDP mol equivalents** | **Dianthin-Cys mol equivalents** | **Obtained DAR** | **Purity by analytical SEC (%)** | **Yield (%)** |
|---|---|---|---|---|---|
| **Tras-(L-S01861)₄-(L-Dianthin)₂** | 4.95 | 4 | 1.7 | 60.5 | n.d. |
| **Cet-(L-SO1861**)₄-**(L-Dianthin)₂** | 4.95 | 4 | 2.1 | 85.2 | 89 |

| | | | | | |
|---|---|---|---|---|---|
| n.d. = not determined | | | | | |

### Antibody-(L-Dianthin)"

### Trastuzumab-(L-Dianthin)₂, Cetuximab-(L-Dianthin)₂ and synthesis via PEG₄-SPDP with a DAR2

Trastuzumab and Cetuximab are referred hereafter as "Ab". Ab was conjugated to Dianthin-Cys via a tetra(ethylene glycol) succinimidyl 3-(2-pyridyldithio)propionate (PEG₄-SPDP) linker forming a labil (L) disulfide bond between Ab and Dianthin. The procedure is exemplary described for Trastuzumab-L-SO1861:
Dianthin-Cys (7.8 mg, 261 nmol, 0.78 mg/ml) was reacted with TCEP (5 mole equivalents, 1.31 µmol, 0.37 mg, 1 mg/ml) for 30 minutes at 20 °C with roller mixing. After, the protein-SH was purified by zeba desalting column eluting into TBS pH 7.5 and used promptly. Protein-SH was obtained (5.2 mg, 67%, 0.52 mg/ml, SH to protein ratio = 1 ± 0.1).

Trastuzumab (0.75 mg, 5 nmol, 2.50 mg/ml) was reacted with an aliquot of freshly prepared PEG₄-SPDP solution (3.35 mole equivalents, 16.75 nmol) in DMSO (1 mg/ml) for 60 minutes at 20 °C with roller mixing. After, the reaction was quenched with glycine (18.1 µl of 1 mg/ml freshly prepared solution in TBS pH 7.5) and then desalted via zeba desalting column eluting with TBS pH 7.5. An aliquot of the resulting Tras-(S-PEG₄-SPDP) was taken out and tested by UV-Vis analysis. SPDP incorporation was determined using TCEP to liberate pyridiyl-2-thione (PDT) and by UV-vis analysis at 343 nm. The remaining Tras-(S-PEG₄-SPDP) was reacted with an aliquot of freshly prepared Dianthin-Cys (protein-SH) (4 mole equivalents, 20 nmol, 0.6 mg, 0.52 mg/ml) and incubated overnight at 20 °C with roller mixing. After 17 hours, an aliquot of the conjugate was analyzed by UV-vis analysis to ascertain incorporation of Dianthin-Cys by displacement of PDT. After, the conjugate was concentrated to ≤1 ml using a vivaspin T4 concentrator and purified using a 1.6 × 37 cm Superdex 200PG column eluting with DPBS pH 7.5. Dianthin to Ab ratio = 2). Yield: n.d. Purity: 67.7 %.

**Table 14. Summarized reaction conditions and results**

| **Batch** | **PEG₄-SPDP mol equivalents** | **Dianthin-Cys mol equivalents** | **Obtained DAR** | **Purity by analytical SEC (%)** | **Yield (%)** |
|---|---|---|---|---|---|
| **Tras-(L-Dianthin)₂** | 3.35 | 4 | 1.6 | 67.7 | n.d. |
| **Cet-(L-Dianthin)₂** | 3.35 | 4 | 1.6 | 95.2 | 88 |

| | | | | | |
|---|---|---|---|---|---|
| n.d. = not determined | | | | | |

### Example 12

### Materials and methods

In our current work, we investigated a model scaffold consisting of four molecular arms for saponin binding via a Schiff base (imine) and one arm for click chemistry. The polymeric structure (Figure 19) is a pentavalent polyethylene glycol-based dendrimer of the first generation (i.e. number of repeated branching cycles) that was purchased from Iris Biotech GmbH (Marktredwitz, Germany). The saponin (in this example SA1641) was purified from a saponin composite raw extract from Gypsophila species called Saponinum album obtained from Merck (Darmstadt, Germany). The powdered raw extract (2.5 g) was hydrolyzed in water (100 mL) with sodium hydroxide (0.2 g). The solution was stirred for 20 h at 40 °C and then supplemented with glacial acetic acid until pH 5.0 was reached. To remove tannins, the solution was shaken in a separatory funnel with 30 mL butanol. The aqueous phase was recaptured and butanol extraction repeated two times. The butanol phases were supplemented with anhydrous sodium sulfate, filtered and pooled. Butanol was evaporated and the remaining saponin powder resolved in 20% methanol to a final concentration of 30 mg/mL. After short sonication, different saponins were separated by high performance liquid chromatography (HPLC). Tubes (excluding column) were rinsed with warm water (40 °C) at a flow of 1.5 mL/min and then including Eurospher RP-C18-column (5 µm, 250 × 8 mm) with isopropanol (100%). Saponins were applied to the column and eluted with a methanol gradient (20% methanol to 70% methanol within 30 min at 1.5 mL/min in water supplemented with 0.01% trifluoroacetic acid followed by 70% methanol for further 60 min) (Sama et al, 2018). Aliquots of the fractions were analyzed for their SA1641 content by electrospray ionization mass spectrometry (ESI-MS). Fractions containing pure SA1641 were pooled and methanol evaporated. The aqueous solution was frozen as a thin film in a rotating round-bottom flask by use of dry ice. After storage for 16 h at - 80 °C, the sample was lyophilized. To produce the scaffold as defined in the invention, the polymeric structure (0.2 mM) and SA1641 (3.2 mM) were solved in water (approx. pH 8) and equal volumes mixed and shaken for 24 h at 26 °C. Then sodium cyanoborohydride (NaCNBH₃; 0,1 M) was added in 4-fold molar excess referred to SA1641 and the sample incubated for further 24 h. The structure was then verified by ultra performance liquid chromatography (UPLC)/ESI-MS. The samples were applied to a RP-C4-column and eluted with a methanol gradient (25% methanol to 80% methanol within 15 min in water supplemented with 0.01% trifluoroacetic acid followed by 80% methanol for further 10 min). The fractions were analyzed by use of LockSpray™ that is an ion source designed specifically for exact mass measurement with electrospray ionization using LC-time-of-flight (LC-TOF) mass spectrometers from Waters Corporation.

### Results

The inset of Figure 58 shows the theoretically expected mass spectrum obtained from a calculation with the isotope pattern calculator enviPat Web 2.0. The pattern considers the charge of the molecule and the natural occurrence of isotopes, which is the reason that more than one peek is expected for a single substance. The experimental data (Figure 58) obtained by UPLC/ESI-MS show almost exactly the same peaks at *m*/*z* 758-760 with same intensity as predicted, thus proving successful SA1641 coupling to the polymeric structure.

### Example 13

### Materials and methods

As an example for a pharmaceutical active substance, we used the targeted toxin dianthin-Epidermal Growth Factor (dianthin-EGF). The plasmid His-dianthin-EGF-pET11d (Weng et al, 2009) (100 ng) was added to 20 µL Escherichia coli Rosetta™ 2 (DE3) pLysS Competent Cells (Novagen, San Diego, CA, USA). Cells were transformed by a heat-shock (30 min on ice, 90 s at 42 °C and 1 min on ice). Thereafter, 300 µL lysogeny broth (LB) was added and the suspension incubated for 1 h at 37 °C while shaking at 200 rpm. A preheated lysogeny broth agar plate with 50 µg/mL ampicillin was inoculated with 100 µl bacteria suspension and the plate incubated overnight at 37 °C. Lysogeny broth (3 mL) with 50 µg/mL ampicillin was inoculated with a colony from the plate and the bacteria were incubated for 8 h at 37 °C and 200 rpm. The suspension (50 µL) was added to 500 mL of lysogeny broth with 50 µg/mL ampicillin and incubated overnight at 37 °C and 200 rpm. Subsequently, the volume was scaled-up to 2.0 L and bacteria grew under the same conditions until an optical density at wavelength 600 nm of 0.9 was reached. Thereafter, protein expression was induced by the addition of isopropyl β-D-1-thiogalactopyranoside (IPTG) at a final concentration of 1 mM. Protein expression lasted for 3 h at 37 °C and 200 rpm. Finally, the bacterial suspension was centrifuged at 5,000 × g and 4 °C for 5 min, resuspended in 20 mL PBS (137 mM NaCl, 2.7 mM KCI, 8.1 mM Na₂HPO₄, 1.47 mM KH₂PO₄) and stored at -20 °C until use. For purification, bacterial suspensions were thawed and lysed by sonication. Lysates were centrifuged (15,800 × *g*, 4 °C, 30 min) and imidazole added to a final concentration of 20 mM. The supernatant was incubated with 2 mL of Ni-nitrilotriacetic acid agarose under continuous shaking for 30 min at 4 °C in the presence of 20 mM imidazole. Subsequently, the material was poured into a 20-mL-column and washed three times with 10 mL wash buffer (50 mM NaH₂PO₄, 300 mM NaCl, 20 mM imidazole) and dianthin-EGF eluted by 10-mL-portions of increasing concentrations of imidazole (31, 65, 125 and 250 mM) in wash buffer. Eluate fractions (2 mL) were dialyzed overnight at 4 °C against 2.0 L PBS. Desalted dianthin-EGF was concentrated by an Amicon® Ultra-15 (10 kDa) and the protein concentration quantified.

To introduce a suitable click chemistry group into dianthin-EGF, alkyne-PEGs-N-hydroxysuccinimidyl ester in 8-fold molar excess referred to dianthin-EGF was solved in dimethyl sulfoxide and added to 9 volumes of dianthin-EGF (1 mg in 0.2 M NaH₂PO₄/Na₂HPO₄, pH 8). After incubation at room temperature for 4 h, non-bound alkyne was separated by use of a PD10 column (GE-Healthcare, Freiburg, Germany). Click chemistry with the polymeric structure was conducted by copper(I)-catalyzed alkyne-azide cycloaddition. Alkyne-dianthin-EGF (0.02 mM), dendrimer (0.05 mM), CuSO₄ (0.1 mM), tris(3-hydroxypropyltriazolylmethyl)amine (0.5 mM) and sodium ascorbate (5 mM) were incubated under gentle agitation for 1 h at room temperature in 0.1 M NaH₂PO₄/Na₂HPO₄, pH 8. Low molecular mass substances were then separated using a PD10 column.

To test the efficacy of the invention, we conducted a viability assay with HER14 cells. These cells are fibroblasts stably transfected with the human epidermal growth factor receptor and therefore target cells for the targeted toxin dianthin-EGF. HER14 cells (2,000 cells/100 µL/well) were seeded into wells of 96-well-cell culture plates and incubated for 24 h in DMEM medium supplemented with 10% fetal calf serum and 1% penicillin/streptomycin at 37 °C, 5% CO₂ and 98% humidity. The different test substances (see results and Figure 59) were then added in triplicates in a volume of 25 µL and supplemented with further 25 µL of medium. After an incubation of 72 h, 30 µL 3-(4,5-dimethylthiazol-2-yl)-2,5-diphenyltetrazolium bromide (0.5 mg/mL in water) was added per well and incubated for 2 h. Thereafter, the medium was carefully removed and replaced by an aqueous solution containing 10% (v/v) isopropanol, 5% (w/v) sodium dodecyl sulfate and 400 mM HCI, and incubated for 5 min. Solubilized formazan was photometrically quantitated at 570 nM in a microplate reader (Spectra MAX 340 PC, Molecular Devices, Sunnyvale, CA, USA). Untreated cells were normalized to 1 and all samples referred to the untreated control. Significance was determined by unpaired two-sample t-tests.

### Results

The polymeric structure, in the example a pentameric dendrimer (pentrimer), does not have any cytotoxic effect on the target cells, neither in absence nor in presence of SA1641 (Figure 59, column 2 and 3). In the absence of the scaffold, the targeted toxin (dianthin-EGF) shows half maximal toxicity at a concentration of 0.1 nM (column 4). In the presence of SA1641 the same concentration results in death of all cells indicating the general ability of SA1641 to act as an enhancer of the endosomal escape (column 5). The presence of the polymeric structure does not affect the toxicity of dianthin-EGF neither in the presence nor in the absence of SA1641 (columns 6 and 7), indicating that the scaffold does not affect the toxicity of dianthin-EGF. To couple the model polymeric structure via click chemistry to the example pharmaceutically active substance of dianthin-EGF, the substance had to be coupled with an alkyne group before. In consequence of this modification, dianthin-EGF lost some activity (compare columns 8 and 9 with 6 and 7, respectively), however, the undirected alkyne modification does not affect the idea of the invention and is also not required in future applications. We had to introduce the alkyne in an undirected way for test purposes only with the risk to impede the pharmaceutically active center of the toxin. A manufacturer of a pharmaceutically active substance can introduce the click position during synthesis directly into the substance at a position of his choice where the activity of the substance remains unaffected. There was no additional loss of activity when clicking the alkyne-modified pharmaceutically active substance to the polymeric structure indicating that the polymeric structure itself was not toxic (column 10 and 11).

### Example 14

### Materials

The following chemicals were used as purchased: methanol (MeOH, LiChrosolv, Merck), N-ε-maleimidocaproic acid hydrazide (EMCH, 95%, TCI Chemicals), trifluoroacetic acid (TFA, 99.8%, Carl Roth), 2-mercaptoethanol (98%, Sigma-Aldrich), poly(amidoamine) (PAMAM dendrimer, ethylenediamine core, generation 5.0 solution, Sigma-Aldrich), cyanine 3 carboxylic acid (Cy3-COOH, 95%, Lumiprobe), 1-[Bis(dimethylamino)methylene]-1H-1,2,3-triazolo[4,5-b]pyridinium 3-oxid hexafluorophosphate, N-[(Dimethylamino)-1H-1,2,3-triazolo-[4,5-b]pyridin-1-ylmethylene]-N-methylmethanaminium hexafluorophosphate N-oxide (HATU, 97%, Sigma-Aldrich), bovine serum albumin fraction V (BSA, Carl Roth), dimethylsulfoxide (DMSO, 99%, Carl Roth), 2-Iminothiolane hydrochloride (98%, Sigma-Aldrich), rhodamine b (RhodB, 95%, Merck), Dulbecco's phosphate buffered saline (PBS, Gibco), hydrochloric acid (HCl, 37%, Merck), NHS-PEG₁₃-DBCO (Click Chemistry Tools), Alexa Fluor™ 488 5-TFP (Thermo-Fischer), azido-PEG3-SS-NHS (Conju-Probe), sodium cyanoborohydride (NaCNBH₃, 95 %, Sigma-Aldrich), ammonium persulfate (APS, 98%, Sigma-Aldrich), N,N,N',N'-tetramethylethylenediamine (TMEDA, 99 %, Sigma-Aldrich), customized peptide SESDDAMFCDAMDESDSK (95%, PeptideSynthetics), azido-dPEG₁₂-NHS (95%, Quanta Biodesign), PFd-G4-Azide-NH-BOC Dendron (G4-dendron, 95%, Polymer Factory), Cyanin5-DBCO (Cy5-DBCO, 95%, Lumiprobe), Chloroform (CHCl₃, 99.5 %, Sigma), Amicon Ultra 0.5 mL centrifugal filters (3 kDa MWCO, Sigma), mPEG-SCM (mPEG₂ₖ-NHS, 95.6%, Creative PEG Works), Amicon Ultra 15 mL centrifugal filters (10 kDa MWCO, Sigma).

### Methods

### MALDI-TOF-MS

MALDI-TOF spectra were recorded on a MALDI-Mass Spectrometer (Bruker Ultrafex III). Typically, the sample dissolved in MilliQ water in nanomolar to micromolar range was spotted on the target (MTP 384 target plate polished steel T F, Bruker Daltons) using either super-DHB (99%, Fluka) or sinapinic acid (SA, 99%, Sigma-Aldrich) as the matrix dissolved in acetonitrile (MADLI-TOF-MS tested, Sigma) / 0.1% TFA (7:3 v/v) via the dried-droplet-method. PepMix (Peptide Calibration Standard, Bruker Daltons) or ProteMass (Protein Calibration Standard, Sigma-Aldrich) served as calibration standards. RP mode refers to reflector positive mode. RN mode refers to reflector negative mode. LP mode refers to linear positive mode.

### H-NMR

¹H NMR analysis was performed using a Bruker 400 MHz NMR spectrometer. The sample preparation, in which 2 mg of sample had been dissolved in 0.8 mL of methanol-*D₄* (99%, Deutero), was performed 24 h prior to the measurement.

### UV-Vis

UV-Vis measurements were performed on a NanoDrop ND-1000 spectrophotometer in the spectral range of 200-750 nm.

### Size Exclusion Chromatography

Size exclusion chromatography (SEC) was performed with Sephadex G 25 Superfine from GE Healthcare and on prepacked PD10 columns (GE Healthcare, Sephadex G 25 M). The material was activated by swelling in the respective eluent prior to performing chromatography.

### Dialysis

Regenerated cellulose membranes: MWCO = 1 and 2 kDa (Spectra/Por), and MWCO = 12-14 kDa (Carl Roth) were used to perform dialysis. Typically, dialysis was carried out for 24 h with 1 L of solvent that was exchanged after first 6 h of the process.

### Lyophilization

Freeze-drying was performed on an Alpha 1-2 LD plus (Martin Christ Gefriertrocknungsanlagen GmbH). Typically, samples were frozen with liquid nitrogen and placed into the freeze-dryer at high vacuum.

### S01861-EMCH synthesis

SO1861 from Saponaria officinalis L (59 mg, 31.7 µmol) and EMCH (301 mg, 888 µmol) were placed in a round flask with stirrer and dissolved in 13 mL methanol. TFA (400 µL, cat.) was added to the solution and the reaction mixture was stirred for 3 h at 800 rpm and room temperature on a RCT B magnetic stirrer (IKA Labortechnik). After stirring for 3 h, the mix was diluted either with MilliQ water or PBS and dialyzed extensively for 24 h against either with MilliQ water or PBS using regenerated cellulose membrane tubes (Spectra/Por 7) with a MWCO of 1 kDa. After dialysis, the solution was lyophilized to obtain a white powder. Yield 62.4 mg (95 %). Dried aliquots were further used for characterization via ¹H NMR and MALDI-TOF-MS.

¹H NMR (400 MHz, methanol-*D₄*) (Figure 60 A, SO1861): *δ* = 0.50-5.50 (m, saponin triterpenoid and sugar backbone protons), 9.43 (1H, s, aldehyde proton of saponin, H^{a}).

¹H NMR (400 MHz, methanol-*D₄*) (Figure 60 B. SO1861-EMCH, PBS workup): *δ* = 0.50-5.50 (m, saponin triterpenoid and sugar backbone protons), 6.79 (2 H, s, maleimide protons, H^{c}), 7.62-7.68 (1 H, m, hydrazone proton, H^{b}).

MALDI-TOF-MS (RP mode) (Figure 61 A): *m*/*z* 2124 Da ([M+K]⁺, saponin-EMCH), *m*/*z* 2109 Da ([M+K]+, SO1861-EMCH), *m*/*z* 2094 Da ([M+Na]⁺, SO1861-EMCH)

MALDI-TOF-MS (RN mode): m/z2275 Da ([M-H]⁻, saponin-EMCH conjugate), 2244 Da ([M-H]⁻, saponin-EMCH conjugate), 2222 Da ([M-H]⁻, saponin-EMCH conjugate), 2178 Da ([M-H]⁻, saponin-EMCH conjugate), 2144 Da ([M-H]⁻, saponin-EMCH conjugate), 2122 Da ([M-H]⁻, saponin-EMCH conjugate), 2092 Da ([M-H]⁻, saponin-EMCH conjugate), 2070 Da ([M-H]⁻, SO1861-EMCH), 2038 Da ([M-H]⁻, SO1832-EMCH), 1936 Da ([M-H]⁻, SO1730-EMCH), 1861 Da ([M-H]⁻, SO1861).

### SO1861-EMCH-mercaptoethanol

To SO1861-EMCH (0.1 mg, 48 nmol) 200 µL mercaptoethanol (18 mg, 230 µmol) was added and the solution was shaken for 1 h at 800 rpm and room temperature on a ThermoMixer C (Eppendorf). After shaking for 1 h, the solution was diluted with methanol and dialyzed extensively for 4 h against methanol using regenerated cellulose membrane tubes (Spectra/Por 7) with a MWCO of 1 kDa. After dialysis, an aliquot was taken out and analyzed via MALDI-TOF-MS.

MALDI-TOF-MS (Figure 61B) (RP mode): *mlz* 2193 Da ([M+K]⁺, SO1861-EMCH-mercaptoethanol), *m*/*z* 2185 Da ([M+K]⁺, SO1861-EMCH-mercaptoethanol), *m*/*z* 2170 Da ([M+Na]⁺, SO1861-EMCH-mercaptoethanol).

### BSA-SO1861 synthesis

2-iminothiolane (231 µg, 1.1 µmol) dissolved in 47 µL PBS was added to a BSA-RhodB solution (10 mg, 0.15 µmol) in 200 µL PBS and the mix was shaken for 40 min at 800 rpm and room temperature on a ThermoMixer C (Eppendorf). After shaking for 40 min, the reaction mix was immediately run through a Sephadex G25 superfine size exclusion column (16 mL column volume) and SO1861-EMCH (1 mg, 0.5 µmol) dissolved in 100 µL PBS was added to the collected BSA-SH fraction. The reaction mixture was shaken for 12 h at 800 rpm and room temperature on a ThermoMixer C (Eppendorf). After shaking for 12 h the BSA-SO1861 concentrated using centrifugal filtration at 4,000 rpm (15 °C) via Amicon Ultra 15 filters with a MWCO of 3 kDa. The conjugate was stored as solution in the fridge and aliquots were taken for analysis. Yield: not determined.

MALDI-TOF-MS (Figure 69 A) (LP mode): *m*/*z* 74.2 kDa ([M+H]⁺, BSA-SO1861 with 4 SO1861 attached), 72.2 kDa ([M+H]⁺, BSA-SO1861 with 3 SO1861 attached), 70.2 kDa ([M+H]⁺, BSA-SO1861 with 2 SO1861 attached), 37.0 kDa ([M+H]²⁺, BSA-SO1861 with 4 SO1861 attached), 35.9 kDa ([M+H]²⁺, BSA-SO1861 with 3 SO1861 attached), 34.7 kDa ([M+H]²⁺, BSA-SO1861 with 2 SO1861 attached).

### Cy3-PAMAM

720 µL PAMAM dissolved in methanol (30 mg, 1.04 µmol) was placed into a 250 mL round flask and methanol was removed via a rotary evaporator (20 mbar, 60 °C). Remaining PAMAM was dissolved in 9 mL DMSO. HATU (7.6 mg, 20 µmol) dissolved in 0.5 mL DMSO was added to a Cy3-COOH (0.6 mg, 1.2 µmol) solution in DMSO and the mix was shaken for 1 h at 800 rpm at room temperature on a ThermoMixer C (Eppendorf). After shaking for 1 h, the HATU-Cy3 solution was added to the stirring PAMAM solution and the reaction mix was stirred for 12 h at room temperature. After stirring for 12 h, the reaction mix was diluted with MilliQ water and dialyzed extensively for 24 h against MilliQ water using regenerated cellulose membrane tubes (Spectra/Por 6) with a MWCO of 2 kDa. After dialysis, the volume of the conjugate solution was reduced via a rotary evaporator (20 mbar, 60 °C) and the concentrated conjugate solution was run through a Sephadex G25 superfine size exclusion column (16 mL column volume). The first fraction was collected and lyophilized to obtain the viscous pink PAMAM-Cy3 conjugate. PAMAM-Cy3 conjugate formation was confirmed by chromatography on thin layer chromatography (methanol/water, v/v 1:1), and the appearance of a faster band on a Sephadex G 25 superfine column. Yield 21.3 mg (63 %). The dye per PAMAM molar ratio determined by UV-Vis spectrophotometry was 0.43.

MALDI-TOF-MS (Figure 71 A) (LP mode): *m*/*z* 28.0 kDa ([M+H]⁺, Cy3-PAMAM).

### Cy3-PAMAM-SO1861 synthesis

Procedure is described exemplary for Cy3-PAMAM-(SO1861)₅. 2-iminothiolane (1 mg, 6.7 µmol) dissolved in 250 µL MilliQ water was added to a PAMAM-Cy3 solution (0.5 mg, 17 nmol) in 125 µL MilliQ water and the mix was shaken for 40 min at 800 rpm and room temperature on a ThermoMixer C (Eppendorf). After shaking for 40 min, the reaction mix was immediately run through a Sephadex G25 superfine size exclusion column (16 mL column volume) and SO1861-EMCH (176 µg, 85 nmol) dissolved in 40 µL MilliQ water was added to the collected Cy3-PAMAM-SH fraction. The reaction mixture was shaken for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12 - 14 kDa. After dialysis, the Cy3-PAMAM-SO1861 solution was concentrated using centrifugal filtration at 4000 rpm (15 °C) via Amicon Ultra 15 filters with a MWCO of 3 kDa. The conjugate was stored as solution in the fridge and aliquots were taken for analysis. Yield: 0.5 mg (75%).

MALDI-TOF-MS spectra are illustrated in Figures 71 B-D, and Figure 72. MALDI-TOF-MS of Cy3-PAMAM-(SO1861)₆ (Figure 71 B) (LP mode): *m*/*z* 38.4 kDa ([M+H]⁺, Cy3-PAMAM-SO1861), 17.9 kDa ([M+H]²⁺, Cy3-PAMAM-SO1861).

The synthesis of Cy3-PAMAM-(SO1861)₅, Cy3-PAMAM-(SO1861)₁₃, Cy3-PAMAM-(SO1861)₅₁, and Cy3-PAMAM-(SO1861)₂₇, has been performed via the above described methodology but differ in the feed equivalents of the starting materials 2-iminothiolane and SO1861-EMCH. The respective feed equivalents of the starting materials and the respective mass of the conjugates are highlighted in Table 15.

**Table 15. Reaction parameter for Cy3-PAMAM-SO1861 synthesis.**

| **2-lminothiolane feed equivalents to Cy3-PAMAM** | **S01861-EMCH feed equivalents to Cy3-PAMAM** | **Mass of conjugate via MALDI-TOF-MS** | **SO1861 molecules attached per PAMAM** | **Resulting conjugate** |
|---|---|---|---|---|
| 384 | 6 | 38.7 kDa | ∼5 | Cy3-PAMAM-(SO1861)_{6,} Figure 71 B |
| 384 | 20 | 53.9 kDa | -13 | Cy3-PAMAM-(SO1861)₁₃, Figure 71 C |
| 384 | 57 | 133.9 kDa | ∼51 | Cy3-PAMAM-(SO1861)₅₁, Figure 71 D |
| 8 | 5 | 37.7 kDa | ∼5 | Cy3-PAMAM-(SO1861)₅, Figure 72 A |
| 32 | 30 | 87.0 kDa | -27 | Cy3-PAMAM-(SO1861)₂₇, Figure 72 B |

### Cy3-PAMAM-NC-SO1861 synthesis

Cy3-PAMAM (0.5 mg, 18 nmol), SO1861 (2.3 mg, 1.24 µmol), and HATU (64.6 mg, 170 µmol) were dissolved separately in 200 µL DMSO. SO1861 and HATU solutions were mixed and shaken for 20 min at 800 rpm and room temperature on a ThermoMixer C (Eppendorf). After shaking for 20 min, Cy3-PAMAM solution was added to the shaking SO1861-HATU solution and the reaction mixture was allowed to shake for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-NC-SO1861 solution was concentrated using centrifugal filtration at 4,000 rpm (15 °C) via Amicon Ultra 15 filters with a MWCO of 3 kDa. The Cy3-PAMAM-NC-(SO1861)₁₇ conjugate was stored as solution in the fridge and aliquots were taken for analysis. Yield: 0.77 mg (69%).

MALDI-TOF-MS (Figure 73) (LP mode): *m*/*z 62.3* kDa ([M+H]⁺, Cy3-PAMAM-NC-SO1861), 35.7 kDa ([M+H]²⁺, Cy3-PAMAM-NC-SO1861).

### G4-dendron dye labeling and deprotection

PFd-G4-Azide-NH-BOC (G4-dendron) (9.75 mg, 2.11 µmol) was placed into a 2 mL reaction tube (Eppendorf) and dissolved in 200 µL DMSO. 100 µL of a Cy5-DBCO solution in DMSO (1.72 µmol * mL⁻¹, 170 nmol) was added to the G4-dendron solution and the mix was shaken for 12 hours at room temperature and 800 rpm on a ThermoMixer C (Eppendorf). After shaking for 12 h, the reaction mix was diluted with MilliQ water and dialyzed extensively for 24 h against MilliQ water using regenerated cellulose membrane tubes (Spectra/Por 7) with a MWCO of 1 kDa. After dialysis, the solution was lyophilized to obtain a blue powder. The crude product was used as obtained from lyophilization for the deprotection step.

Partially Cy5 labeled lyophilized G4-dendron was dissolved in 12 mL CHCl₃ in 50 mL round flask with stirrer. 12 mL TFA was added and the reaction mix was stirred for 3 h at 800 rpm and room temperature on a RCT B magnetic stirrer (IKA Labortechnik). After stirring for 3 h, the solvent was removed under reduced pressure (50 °C, 30 mbar) on a rotary evaporator (Heidolph WB 2000). After evaporation, the batch was dissolved in MilliQ water and run through a PD10 size exclusion column. G4-dendron conjugate formation was confirmed by chromatography on thin layer chromatography (methanol/water, v/v 1:1), and the appearance of a faster band on a PD10 column. Obtained fraction of size exclusion chromatography was lyophilized to obtain a blue powder.

Yield 5.7 mg (93 %). The dye per G4-dendron molar ratio determined by UV-Vis spectrophotometry was 0.012.

MALDI-TOF-MS (RP mode): *m*/*z* 3956 Da ([M+Na]⁺, Cy5-G4-dendron + PF₆⁻ counterion), 3820 Da ([M+Na]⁺, Cy5-G4-dendron - PF₆⁻ counterion), 3617 Da ([M+H]⁺, G4-dendron impurity), 3017 ([M+H]⁺, G4-dendron).

### G4-dendron-SO1861 synthesis

Procedure is described exemplary for the lowest G4-dendron to SO1861-EMCH ratio. 2-iminothiolane (2.65 mg, 19.2 µmol) dissolved in 300 µL MilliQ water was added to a partially Cy5 labeled G4-dendron solution (0.577 mg, 192 nmol) in 252 µL MilliQ water and the mix was shaken for 40 min at 800 rpm and room temperature on a ThermoMixer C (Eppendorf). After shaking for 40 min, the reaction mix was immediately run through a PD10 size exclusion column and SO1861-EMCH (1.19 mg, 575 nmol) dissolved in 100 µL MilliQ water was added to the collected G4-dendron-SH fraction. The reaction mixture was shaken for 12 h at 800 rpm and room temperature on a ThermoMixer C (Eppendorf). After shaking for 12 h, the reaction mix was concentrated via centrifugal filtration using Amicon Ultra centrifugal filters (3 kDa MWCO). The conjugate was stored as solution in the fridge and aliquots were taken for analysis. Yield: 90 nmol (47%).

MALDI-TOF-MS spectra are illustrated in Figure 87. MALDI-TOF-MS of G4-dendron-SO1861 (Figure 87 C) (LP mode): *m*/*z* 10.19 kDa ([M+H]⁺, Cy5-G4-dendron-[SO1861]₃), 9.27 kDa ([M+H]⁺, G4-dendron-[SO1861]₃), 7.92 kDa ([M+H]⁺, Cy5-G4-dendron-[SO1861]₂), 7.14 kDa ([M+H]⁺, G4-dendron-[SO1861]₂), 5.86 kDa ([M+H]⁺, Cy5-G4-dendron-[SO1861]₁), 5.07 kDa ([M+H]⁺, G4-dendron-[SO1861]₁).

The synthesis of other G4-dendron-(SO1861)ₙ conjugates has been performed via the above described methodology but differs in the feed equivalents of the starting material SO1861-EMCH. The respective feed equivalents of the starting materials and the respective mass of the conjugates are highlighted in Table 16.

**Table 16. Reaction parameter for G4-dendron-SO1861 synthesis.**

| **2-lminothiolane feed equivalents to G4-dendron** | **SO1861-EMCH feed equivalents to G4-dendron** | **Mass of conjugates via MALDI-TOF-MS** | **SO1861 molecules attached per G4-dendron** | **Resulting MS spectrum** |
|---|---|---|---|---|
| 100 | 3 | 5.07 - 10.18 kDa | ∼1 - 3 | Figure 80 C |
| 100 | 10 | 5.07 - 11.64 kDa | ∼1 - 4 | Figure 80 B |
| 100 | 22 | 6.20 - 22.02 kDa | ~1 - 9 | Figure 80 A |

### PAMAM thiolation

Procedure is described exemplary for the highest PAMAM to 2-iminothiolane ratio. To a PAMAM (333 µg, 12.8 nmol) solution dissolved in 30 µL methanol 2-iminothiolane (0.53 mg, 3.84 µmol) dissolved in 128 µL MilliQ water was added. The reaction mixture was shaken for 12 h at 800 rpm and room temperature on a ThermoMixer C (Eppendorf). After shaking for 12 h, the reaction mix was washed 4 times with MilliQ water via centrifugal filtration using Amicon Ultra centrifugal filters (3 kDa MWCO) at 15 °C and 13500 rpm. After washing the sample was lyophilized to obtain a white solid. Yield was not determined.

MALDI-TOF-MS spectra are illustrated in Figure 89. MALDI-TOF-MS of PAMAM-(SH)₁₀₈ (Figure 89 E) (LP mode): *m*/*z* 41.5 kDa ([M+H]⁺, PAMAM-[SH]₁₀₈).

The synthesis of other PAMAM-iminothiolane conjugates has been performed via the above described methodology but differs in the feed equivalents of the starting material 2-iminothiolane. For the lowest 2-iminothiolane feed reaction Cy3-PAMAM has been used.

The respective feed equivalents of the starting materials and the respective mass of the conjugates are highlighted in Table 17.

**Table 17. Reaction parameter for PAMAM-SH synthesis.**

| **2-Iminothiolane feed equivalents to PAMAM** | **Mass of conjugates via MALDI-TOF-MS** | **Iminothiolane molecules attached per PAMAM** | **Resulting MS spectrum** |
|---|---|---|---|
| 50 | 34.4 kDa | ∼ 16 | Fig. 89 C |
| 100 | 35.9 kDa | ∼ 65 | Fig. 89 D |
| 300 | 41.5 kDa | ∼108 | Fig. 89 E |

### PAMAM PEGylation

Procedure is described exemplary for the lowest PAMAM to mPEG₂ₖ ratio. To a PAMAM (333 µg, 12.8 nmol) solution dissolved in 10 µL DMSO mPEG₂ₖ-NHS (0.268 mg, 128 nmol) dissolved in 13 µL DMSO was added. The reaction mixture was shaken for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (Spectra/Por 6) with a MWCO of 2 kDa. After dialysis, the batch was concentrated via centrifugal filtration using Amicon Ultra 15 mL centrifugal filters (10 kDa MWCO). The concentrated batch was run through a PD10 size exclusion column followed by lyophilization to obtain a white fluffy powder. Yield was not determined.

MALDI-TOF-MS spectra are illustrated in Figure 90. MALDI-TOF-MS of PAMAM-(mPEG₂ₖ)₃ (Figure 90 C) (LP mode): *m*/*z* 33.46 kDa ([M+H]⁺, PAMAM-[mPEG₂ₖ]₃).

The synthesis of other PAMAM-mPEG₂ₖ conjugates has been performed via the above described methodology but differs in the feed equivalents of the starting material mPEG₂ₖ-NHS. The respective feed equivalents of the starting materials and the respective mass of the conjugates are highlighted in Table 18.

**Table 18. Reaction parameter for PAMAM-mPEG₂ₖ synthesis.**

| **mPEG₂ₖ-NHS feed equivalents to PAMAM** | **Mass of conjugates via MALDI-TOF-MS** | **mPEG₂ₖ molecules attached per PAMAM** | **Resulting MS spectrum** |
|---|---|---|---|
| 10 | 28.5 kDa | ∼ 3 | Figure 90 C |
| 20 | 43.0 kDa | ∼ 8 | Figure 90 D |
| 100 | 62.8 kDa | ∼ 18 | Figure 90 E |

### Cy3-PAMAM-SO1861-DBCO synthesis

Procedure is described exemplary for Cy3-PAMAM-(SO1861)₂₇-(DBCO)₁₀. Cy3-PAMAM-(SO1861)₂₇ (0.41 mg, 4.71 nmol) was freeze-fried and dissolved in 100 µL DMSO. DBCO-PEG₁₃-NHS ester (0.197 mg, 188 nmol) dissolved in DMSO was added to the Cy3-PAMAM-SO1861 solution and the mixture was shaken at 800 rpm and room temperature on a ThermoMixer C (Eppendorf). After shaking for 3 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-SO1861-DBCO solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: 0.1 mg (22%).

MALDI-TOF-MS (Figure 74 D) (LP mode): *m*/*z* 92.5 kDa ([M+H]⁺, Cy3-PAMAM-SO1861-DBCO), 53.0 kDa ([M+H]²⁺, Cy3-PAMAM-S01861-DBCO).

The synthesis of Cy3-PAMAM-(SO1861)₅-(DBCO)₃₈, and Cy3-PAMAM-(SO1861)₂₇-(DBCO)₁₀, have been performed via the above described methodology. The respective feed equivalents of the starting material and the respective mass of the conjugates are highlighted in Table 19.

**Table 19. Reaction parameter for Cy3-PAMAM-S01861-DBCO synthesis.**

| **Used Cy3-PAMAM-saponin batch** | **DBCO-PEG13-NHS feed equivalents** | **Mass via MALDI-TOF-MS** | **DBCO molecules attached per PAMAM** | **Resulting conjugate** |
|---|---|---|---|---|
| Cy3-PAMAM-(SO1861)₅ | 40 | 76.3 kDa | ∼38 | Cy3-PAMAM-(SO1861)₅-(DBCO)₃₈, Figure 74 C |
| Cy3-PAMAM-(301861)₂₇ | 40 | 92.5 kDa | ∼10 | Cy3-PAMAM-(SO1861)₂₇-(DBCO)₁₀, Figure 74 D |

### Cy3-PAMAM-NC-SO1861 -DBCO synthesis

Cy3-PAMAM-NC-(SO1861)₁₇ (0.3 mg, 4.8 nmol) was freeze-fried and dissolved in 100 µL DMSO. DBCO-PEG₁₃-NHS ester (0.202 mg, 194 nmol) dissolved in DMSO was added to the Cy3-PAMAM-NC-SO1861 solution and the mixture was shaken at 800 rpm and room temperature on a ThermoMixer C (Eppendorf). After shaking for 3 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-S01861-DBCO solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: 0.1 mg (22%). Mass spectrometry indicates the conjugation of 30 DBCO moieties per PAMAM molecule. MALDI-TOF-MS (Figure 74 B) (LP mode): *m*/*z* 93.2 kDa ([M+H]⁺, Cy3-PAMAM-NC-SO1861-DBCO), 49.6 kDa ([M+H]²⁺, Cy3-PAMAM-NC-SO1861-DBCO).

### EGFDianthin and dianthin expression

Plasmid-DNA (His-dianthin-EGF-pET11d or His-dianthin-pET11d) [20] was transformed into chemically competent Escherichia coli NiCo21 (DE3) (New England Biolabs®, Inc.) and grown in 3 mL lysogeny broth supplemented with 50 µg/mL ampicillin at 37 °C for 5 h at 200 rpm. These bacteria were used to inoculate 500 mL lysogeny broth supplemented with 50 µg/ml ampicillin for overnight culture at 37 °C. Subsequently, the culture volume was scaled up to 2 L and bacteria were grown until an optical density (A600) of 0.9. Protein expression was induced by the addition of isopropyl β-D-1-thiogalactopyranoside (IPTG) at a final concentration of 1 mM. Cells were further grown for 3 h at 37 °C and 200 rpm. After centrifugation (5 min, 5,000 g, 4 °C) cell pellets were resuspended in 20 mL phosphate buffered saline (Dulbecco's phosphate-buffered saline (PBS) with Ca²⁺ and Mg²⁺, pH 7.4) and stored at -20 °C. After thawing, proteins were released by ultrasound device (Branson Sonifier 250, G. Heinemann). The solution was centrifuged (15,800 × g, 30 min, 4 °C) and adjusted to 20 mM imidazole concentration. The construct contained an N-terminal His-tag and was purified by nickel nitrilotriacetic acid chromatography (Ni-NTA Agarose, Qiagen, Hilden, Germany). After elution with imidazole (20-250 mM) the eluates were analyzed by sodium dodecyl sulfate polyacrylamide gel electrophoresis (SDS-PAGE) (12%). Fractions containing dianthin-EGF or dianthin were dialyzed against 2 L chitin binding domain buffer (20 mM tris(hydroxymethyl)-aminomethane/HCI, 500 mM NaCl, 1 mM EDTA, 0.1% Tween-20, pH 8.0) at 4 °C. Further purification by chitin column affinity chromatography served to remove bacterial proteins with binding activity for Ni-NTA agarose. After elution with chitin binding domain buffer, the fractions were analyzed by SDS-PAGE (12%). Fractions containing dianthin-EGF or dianthin were dialyzed against 5 L PBS at 4 °C. Purified proteins were concentrated by Amicon centrifugal filter devices (10 kDa, Millipore, Eschborn, Germany). The protein concentration was determined by a bicinchoninic acid assay (Pierce, Rockford, USA).

### Dianthin-EGF-Alexa488 synthesis

Dianthin-EGF (240 µg, 6.7 nmol) solution in PBS was placed into an Amicon Ultra 15 filter with a MWCO of 3 kDa and centrifuged at 4,000 rpm and 4 °C for 30 min three times. After each cycle, the Amicon filter was refilled with 0.1 M sodium carbonate buffer at pH 9. After the third centrifugation cycle, the volume was reduced to 0.5 mL via centrifugation. The dianthin-EGF sodium carbonate solution was placed into a 2 mL reaction tube and Alexa Fluor™ 488 5-TFP (50 µg, 56 nmol) dissolved in 10 µL DMSO was added to the protein solution. The mix was shaken at 800 rpm and room temperature on a ThermoMixer C (Eppendorf) for 80 min. After shaking, the mix was run through a Sephadex G25 M size exclusion column (GE Healthcare, PD10 column). The dianthin-EGF-Alexa488 conjugate was stored in solution in 0.1 M sodium carbonate buffer at pH 9 in the fridge and aliquots were taken for analysis. Yield: 210 µg (85%).

MALDI-TOF-MS (Figure 75 D) (LP mode): *m*/*z* 36.8 kDa ([M+H]⁺, dianthin-EGF-Alexa488), *m*/*z* 33.6 kDa ([M+H]⁺, dianthin-EGF-Alexa488), 18.8 kDa ([M+H]²⁺, dianthin-EGF-Alexa488), 16.6 kDa ([M+H]²⁺, dianthin-EGF-Alexa488).

### Dianthin-Alexa488 synthesis

Dianthin (184 µg, 6.2 nmol) solution in PBS was placed into an Amicon Ultra 15 filter with a MWCO of 3 kDa and centrifuged at 4,000 rpm and 4 °C for 30 min three times. After each cycle, the Amicon filter was refilled with 0.1 M sodium carbonate buffer at pH 9. After the third centrifugation cycle, the volume was reduced to 0.5 mL via centrifugation. The dianthin sodium carbonate solution was placed into a 2 mL reaction tube and Alexa Fluor™ 488 5-TFP (16.7 µg, 19 nmol) dissolved in 3.5 µL DMSO was added to the protein solution. The mix was shaken at 800 rpm and room temperature on a ThermoMixer C (Eppendorf) for 80 min. After shaking, the mix was run through a Sephadex G25 M size exclusion column (GE Healthcare, PD 10 column). The dianthin-Alexa488 conjugate was stored in solution in 0.1 M sodium carbonate buffer at pH 9 in the fridge and aliquots were taken for analysis. Yield: not determined MALDI-TOF-MS (Figure 76 D) (LP mode): *m*/*z* 30.7 kDa ([M+H]⁺, dianthin-Alexa488).

### Dianthin-EGF-Alexa488-S-S-PEG-N₃, and Dianthin-EGF-Alexa488-PEG₁₂-N₃ synthesis

Procedure is described exemplary for dianthin-EGF-Alexa488-S-S-PEG-N₃. Dianthin-EGF-Alexa488 (70 µg, 1.9 nmol) sodium carbonate solution was placed into a 2 mL reaction tube and azido-PEG₃-S-S-NHS (120 µg, 272 nmol) dissolved in 9 µL DMSO was added to the protein solution. The mix was shaken at 800 rpm and 15 °C on a ThermoMixer C (Eppendorf) for 12 h. After shaking, the reaction mix was diluted with PBS and was washed with PBS via centrifugal filtration at 4,000 rpm and 4 °C using Amicon Ultra 15 filter with a MWCO of 3 kDa.

Yield: 54 µg (70%).

MALDI-TOF-MS (Figure 75 E) (LP mode): *m*/*z* 40.8 kDa ([M+H]⁺, dianthin-EGF-Alexa488-S-S-PEG-N₃), *m*/*z 37.5* kDa ([M+H]⁺, dianthin-EGF-Alexa488-S-S-PEG-N₃).

The synthesis of dianthin-EGF-Alexa488-S-S-PEG-N₃, and dianthin-EGF-Alexa488-PEG₁₂-N₃ have been performed via the above described methodology but differed in the used azido-PEG linker. The respective azido-PEG linker, their feed equivalents, and the respective mass of the conjugates are highlighted in Table 20.

**Table 20. Reaction parameter for dianthin-EGF-Alexa488-PEG-N₃ synthesis**

| **Used toxin batch** | **Azido-PEG linker used** | **Azido-PEG linker feed equivalents** | **Mass of conjugate via MALDI-TOF-MS** | **Resulting conjugate** |
|---|---|---|---|---|
| Dianth in-EGF-Alexa488 | Azido-PEG₃-S-S-NHS | 135 | 40.8 kDa | Dianth in-EGF-Alexa488-S-S-PEG₃-N₃ |
| Dianth in-EGF-Alexa488 | Azido-PEG₁₂-NHS | 135 | 43.3 kDa | Dianth in-EGF-Alexa488-PEG₁₂-N3 |

### Dianthin-Alexa488-S-S-PEG-N₃

Dianthin-Alexa488 (24.5 µg, 0.8 nmol) sodium carbonate solution was placed into a 2 mL reaction tube and azido-PEG₃-S-S-NHS (34 µg, 78 nmol) dissolved in 9 µL DMSO was added to the protein solution. The mix was shaken at 800 rpm and 15 °C on a ThermoMixer C (Eppendorf) for 12 h. After shaking, the reaction mix was diluted with PBS and was washed with PBS via centrifugal filtration at 4,000 rpm and 4 °C using Amicon Ultra 15 filter with a MWCO of 3 kDa.

Yield: 10.3 µg (39%).

MALDI-TOF-MS (Figure 76 E) (LP mode): *m*/*z* 32.9 kDa ([M+H]⁺, dianthin-Alexa488-S-S-PEG-N₃).

### Cy3-PAMAM-Saponin-Toxin conjugate synthesis

Procedure is described exemplary for Cy3-PAMAM-(SO1861)₂₇-DBCO. Cy3-PAMAM-(S01861)₂₇-DBCO (17 µg, 0.184 nmol) solution in MilliQ water was mixed with a dianthin-EGF-Alexa488-SS-PEG3-N₃ (3.6 µg, 0.089 nmol) solution in PBS in a 1.5 mL reaction tube and the reaction mix was shaken at 800 rpm and 15 °C on a ThermoMixer C (Eppendorf) for 2 h. After shaking, small aliquots were taken out for analysis via SDS-PAGE and fluorescence imaging on a Molecular Imager® VersaDoc™ MP 4000 imaging system (Bio-Rad) (Figure 77).

The synthesis of Cy3-PAMAM-(SO1861)₅-S-S-Dianthin-EGF-Alexa488, Cy3-PAMAM-(SO1861)₂₇-S-S-Dianthin-EGF-Alexa488, Cy3-PAMAM-NC-(SO1861)₁₇-S-S-Dianthin-EGF- Alexa488, Cy3-PAMAM-NC-(SO1861)₁₇-S-S-Dianthin-Alexa488, and Cy3-PAMAM-NC-(SO1861)₁₇-Dianthin-EGF-Alexa488, have been performed via the above described methodology but differ in the used PAMAM-saponin-DBCO batch, the used azido-toxin batch, and their feed equivalents. The respective feed equivalents of the starting materials are highlighted in Table 21.

**Table 21. Reaction parameter for Cy3-PAMAM-saponin-toxin synthesis.**

| **PAMAM-saponin-DBCO batch used** | **PAMAM-saponin-DBCO feed equivalents** | **Azido-toxin batch used** | **Azido-toxin feed equivalents** | **Resulting conjugate** |
|---|---|---|---|---|
| Cy3-PAMAM-(SO1861)₅-(DBCO)₃₈ | 3 | Dianth in-EGF-Alexa488-S-S-PEG₃-N₃ | 1 | Cy3-PAMAM-(SO1861)₅-S-S-Dianthin-EGF-Alexa488 |
| Cy3-PAMAM-(SO1861)₂₇-(DBCO)₁₀ | 2.1 | Dianth in-EGF-Alexa488-S-S-PEG₃-N₃ | 1 | Cy3-PAMAM-(SO1861)₂₇-S-S-Dianthin-EGF-Alexa488 |
| Cy3-PAMAM-NC-(SO1861)₁₇-(DBCO)₃₀ | 2.3 | Dianth in-EGF-Alexa488-S-S-PEG₃-N₃ | 1 | Cy3-PAMAM-NC-(SO1861)₁₇-S-S-Dianthin-EGF-Alexa488 |
| Cy3-PAMAM-NC-(SO1861)₁₇-(DBCO)₃₀ | 7.1 | Dianthin-Alexa488-S-S-PEG₃-N₃ | 1 | Cy3-PAMAM-NC-(SO1861)₁₇-S-S-Dianthin-Alexa488 |
| Cy3-PAMAM-NC-(SO1861)₁₇-(DBCO)₃₀ | 2.3 | Dianth in-EGF-Alexa488-PEG₁₂-N3 | 1 | Cy3-PAMAM-NC-(SO1861)₁₇-Dianth in-EGF-Alexa488 |

### Cy3-PAMAM-NC-S01861 synthesis via reductive amination

Cy3-PAMAM (0.19 mg, 13 nmol) and SO1861 (0.73 mg, 0.39 µmol) were dissolved separately in 200 µL 0.1 M acetate buffer at pH 5. SO1861 and Cy3-PAMAM solutions were mixed and shaken for 20 min at 800 rpm and room temperature on a ThermoMixer C (Eppendorf). After shaking for 20 min, NaCNBH₃ (5 mg, 81 µmol) was added to the shaking reaction solution and the reaction mixture was allowed to shake for 12 h at 800 rpm and room temperature on a ThermoMixer C (Eppendorf). After shaking for 12 h, the reaction mix was diluted with MilliQ water and dialyzed extensively for 24 h against MilliQ water using regenerated cellulose membrane tubes (ZelluTrans, Carl Roth) with a MWCO of 12-14 kDa. After dialysis, the Cy3-PAMAM-NC-SO1861 solution was concentrated using centrifugal filtration at 4,000 rpm (15 °C) via Amicon Ultra 15 filters with a MWCO of 3 kDa. The conjugate was stored as solution in the fridge and aliquots were taken for analysis. Yield: not determined

MALDI-TOF-MS (Figure 78 B, C) (LP mode): *m*/*z* 88.7 kDa ([M+H]⁺, Cy3-PAMAM-NC-SO1861), 49.2 kDa ([M+H]²⁺, Cy3-PAMAM-NC-SO1861).

The synthesis of Cy3-PAMAM-NC-(SO1861)₃₀, and Cy3-PAMAM-NC-(SO1861)₁₀, have been performed via the above described methodology but differed in the time after which the reducing agent NaCNBH₃ was added to the reaction batch. The respective time of the NaCNBH₃ addition and the respective mass of the conjugates are highlighted in Table 22.

**Table 22. Reaction parameter Cy3-PAMAM-NC-SO1861 synthesis via reductive amination.**

| **Time of shaking reaction mix before NaCNBH₃ addition** | **Mass via MALDI-TOF-MS** | **SO1861 molecules attached per PAMAM** | **Resulting conjugate** |
|---|---|---|---|
| 20 min | 88.8 kDa | ∼30 | Cy3-PAMAM-NC-(SO1861)₃₀, Figure 78 C |
| 12 h | 48.0 kDa | ∼10 | Cy3-PAMAM-NC-(SO1861)₁₀, Figure 78 B |

### Poly(SO1861)synthesis

SO1861-EMCH (0.13 mg, 63 nmol) was dissolved in 30 µL degased MilliQ water. APS (0.2 µg, 0.8 nmol) dissolved in 4 µL degased MilliQ water was added to the SO1861-EMCH solution and the solution was placed into a ThermoMixer C (Eppendorf) at 60 °C. Then, TMEDA (cat., 0.5 µL) was added to the mix and the mix was shaken at 800 rpm and 60 °C on a ThermoMixer C (Eppendorf) for 2 h. After 2 h, a small aliquot was taken out for analysis via mass spectrometry.

MALDI-TOF-MS (Figure 80 C) (LP mode): *m*/*z* 18.2 kDa ([M+H]⁺, poly(SO1861)₉), 16.0 kDa ([M+H]⁺, poly(SO1861)₈), 14.2 kDa ([M+H]⁺, poly(SO1861)₇), 12.2 kDa ([M+H]⁺, poly(SO1861)₆), 10.2 kDa ([M+H]⁺, poly(SO1861)₅), 8.2 kDa ([M+H]+, poly(SO1861)₄), 6.2 kDa ([M+H]⁺, poly(SO1861)₃).

### SO1861-EMCH peptide coupling

Customized peptide with the sequence SESDDAMFCDAMDESDSK (0.6 mg, 0.3 pmol) and SO1861-EMCH (0.8 mg, 0.39 µmol) were dissolved separately in 200 µL PBS. SO1861-EMCH and peptide solutions were mixed and shaken for 12 h at 800 rpm and room temperature on a ThermoMixer C (Eppendorf). After shaking small aliquots were taken out for analysis. Yield: not determined MALDI-TOF-MS (Figure 83 B) (RN mode): *m*/*z 4.05* kDa ([M+H]⁻, peptide-SO1861), 3.92 kDa ([M+H]⁻, peptide-SO1730), 1.98 kDa ([M+H]⁻, peptide), 1.86 kDa ([M+H]⁻, SO1861).

### Cell viability assay

After treatment the cells were incubated for 72 hr at 37°C before the cell viability was determined by a MTS-assay, performed according to the manufacturer's instruction (CellTiter 96® AQueous One Solution Cell Proliferation Assay, Promega). Briefly, the MTS solution was diluted 20x in DMEM without phenol red (PAN-Biotech GmbH) supplemented with 10% FBS (PAN-Biotech GmbH). The cells were washed once with 200 µL PBS per well, after which 100 µL diluted MTS solution was added per well. The plate was incubated for approximately 20-30 minutes at 37°C. Subsequently, the optical density at 492 nm was measured on a Thermo Scientific Multiskan FC plate reader (Thermo Scientific). For quantification the background signal of 'medium only' wells was subtracted from all other wells, before the ratio of untreated/treated cells was calculated, by dividing the background corrected signal of untreated wells over the background corrected signal of the treated wells.

### FACS analysis

HeLa cells were seeded in DMEM (PAN-Biotech GmbH) supplemented with 10% fetal calf serum (PAN-Biotech GmbH) and 1% penicillin/streptomycin (PAN-Biotech GmbH), at 500,000 c/plate in 10 cm dishes and incubated for 48 hrs (5% CO₂, 37 °C), until a confluency of 90% was reached. Next, the cells were trypsinized (TryplE Express, Gibco Thermo Scientific) to single cells. 0.75 × 10⁶ Cells were transferred to a 15 mL falcon tube and centrifuged (1,400 rpm, 3 min). The supernatant was discarded while leaving the cell pellet submerged. The pellet was dissociated by gentle tapping the falcon tube on a vortex shaker and the cells were washed with 4 mL cold PBS (Mg²⁺ and Ca²⁺ free, 2% FBS). After washing the cells were resuspended in 3 mL cold PBS (Mg²⁺ and Ca²⁺ free, 2% FBS) and divided equally over 3 round bottom FACS tubes (1 mL/tube). The cells were centrifuged again and resuspended in 200 µL cold PBS (Mg²⁺ and Ca²⁺ free, 2% FBS) or 200 µL antibody solution; containing 5 µL antibody in 195 µL cold PBS (Mg²⁺ and Ca²⁺ free, 2% FBS). APC Mouse IgG1, K Isotype Ctrl FC (#400122, Biolegend) was used as isotype control, and APC anti-human EGFR (#352906, Biolegend) was used to stain the EGFR receptor, HER2: APC anti-human CD340 (erbB2/HER-2) (324408, Biolegend). CD71: APC anti-human CD71 #334108, Biolegend. Samples were incubated for 30 min at 4 °C on a tube roller mixer. Afterwards, the cells were washed 3x with cold PBS (Mg²⁺ and Ca²⁺ free, 2% FBS) and fixated for 20 min at room temperature using a 2% PFA solution in PBS. Cells were washed 2x with cold PBS, and resuspended in 250-350 µL cold PBS for FACS analysis. Samples were analyzed with a BD FACSCanto Il flow cytometry system (BD Biosciences) and FlowJo software.

**Table 23. Expression levels of EGFR, HER2 and CD71 of various cells**

| **Cell line** | **EGFR expression level (MFI)** | **HER2 expression level (MFI)** | **CD71 expression level (MFI)** |
|---|---|---|---|
| **MDA-MB-468** | 1656 | 1 | 186 |
| **A431** | 1593 | 10 | 322 |
| **CaSki** | 481 | 12 | 189 |
| **SK-BR-3** | 28 | 1162 | 331 |
| **JIMT-1** | 58 | 74 | 107 |
| **HeLa** | 91 | 7 | 312 |
| **A2058** | 1 | 5 | 59 |

### Results

Considering available chemical groups for conjugation reactions to the SO1861 molecule, four chemical groups have been identified. The alcohols and diols of the sugar residues, the aldehyde group on the triterpenoid backbone, the carboxylic acid on one of the sugar residues (glucuronic acid), and the alkene group on the triterpenoid backbone as highlighted in Figure 62.

In view of the pros and cons of each identified chemical group (Table 24), the aldehyde and alcohol groups are best suitable for reversible conjugation reactions, while the alkene and the carboxylic acid (glucuronic acid) are the groups best suitable for irreversible / stable conjugation reactions. The aldehyde group within the molecule structure of SO1861, however, is the most suitable for reversible conjugation reactions over the alcohols. On the one hand, because there is only one aldehyde present in the structure that allows chemoselective reactions. On the other hand, because the aldehyde can perform reversible conjugation reactions with a variety of chemical groups such as amines, hydrazides, and hydroxylamines forming acid-cleavable moieties like imines, hydrazones, and oximes. This factor enables a freedom of choice over the chemical group for the desired reversible conjugation reaction. Contrary, the alcohols are good candidates for reversible conjugation reaction via the formation of acetals and ketals as well, but lack in chemoselectivity since they are present in a large quantity on the glycosidic structure.

For the formation of an irreversible and stable bond the carboxylic acid is the most suitable since it can form amides and esters with the common tools used in peptide chemistry (e.g. reaction with amines via carbodiimide mediated amide formation).

**Table 24. Functional groups that are available for saponin conjugation reactions**

| **Functional Group** | **Pros** | **Cons** |
|---|---|---|
| **Alcohol (Diols)** | - Suitable for reversible acetal/ketal formation | - Acetal/ketal formation without chemoselectivity |
| | - Suitable for ester formations with activated carboxylic acids | - Ester formation without chemoselectivity |
| **Aldehyde** | - Suitable for chemoselective reversible hydrazone formation with hydrazides | - Not suitable for acetal formation in the presence of unprotected saponin sugar diols |
| | - Suitable for chemoselective reversible imine formation with amines | |
| | - Suitable for chemoselective reversible oxime formation with hydroxylamines | |
| **Alkene** | - Suitable for chemoselective irreversible radical reactions | - Not suitable for reversible conjugation reactions |
| | | - Not suitable for reactions involving a hydrogenation step |
| **Carboxylic acid** | - Suitable for chemoselective amide / ester formation with amines and alcohols after activation | - Not suitable for reversible conjugation reactions under mild conditions |

Thus, for the development of an endosomal escape enhancing saponin (such as SO1861) a methodology has been established that allows the generation of a non-cleavable and cleavable 'ready to conjugate' saponins (Figure 63) using the most suitable chemical groups present on SO1861.

For producing non-cleavable 'ready to conjugate' saponins the carboxylic group of SO1861 is activated via a reagent used in peptide coupling chemistry to generate an active ester (e.g. 1-[Bis(dimethylamino)methylene]-1H-1,2,3-triazolo[4,5-b]pyridinium 3-oxide hexafluorophosphate, HATU). The resulting active ester of SO1861 is able to react with amines forming stable amide bonded conjugates (Figure 63 A).

For producing cleavable 'ready to conjugate' saponins the aldehyde group of SO1861 is reacted with an EMCH (ε-maleimidocaproic acid hydrazide) linker. The hydrazide group of EMCH forms an acid cleavable hydrazone bond with the aldehyde of SO1861. At the same time the EMCH linker presents a maleimide group that is thiol (sulfhydryl group) reactive and thus can be conjugated to thiols (Figure 63 B).

The maleimide group of SO1861-EMCH performs a rapid and specific Michael addition reaction with thiols and thiol bearing polymeric structures when carried out in a pH range of 6.5-7.5 (Figure 63 B). In addition, the acid sensitive hydrazone linkage between the SO1861 and EMCH can be utilized to perform saponin release from a scaffold in acidic environment (Figure 64). Thus, the EMCH linker fulfills both the need for a pH cleavable strategy and a conjugation strategy to a polymeric structure. Regarding an ideal EMCH spacer length for conjugation to a polymeric structure, computer simulation (PerkinElmer, ChemBio3D, Ver. 13.0.0.3015) shows that the maleimide group on SO1861-EMCH is located at the periphery of the molecule and thus should be accessible for thiol bearing polymeric structures (Figure 65).

To synthesize the SO1861-EMCH, a strategy has been developed that allows the conversion of the aldehyde group on the SO1861 to EMCH (Figure 66 A). The SO1861-EMCH conjugate was isolated and successfully characterized via nuclear magnetic resonance spectroscopy (see materials and methods section, Figure 60B) and matrix-assisted laser desorption/ionization time-of-flight mass spectrometry (MALDI-TOF-MS) as shown in Figure 66 B and 66 C, and Figure 61 A.

For testing the pH dependent hydrolysis of the hydrazone bond, SO1861-EMCH was dissolved in an HCI solution at pH 3 and MALDI-TOF-MS spectra were recorded at two different points in time (Figure 67). As shown on Figure 67 A and 67 B, a clear decreasing tendency of the peak at *m*/*z* 2070 Da that corresponds to SO1861-EMCH is visible in Figure 67 B. Since SO1861 is generated during hydrolysis, an increase of the peak at *m*/*z* 1861 Da was recorded that accompanied the decreasing tendency at *m*/*z* 2070 Da. These results show that the hydrazone bond is responsive towards hydrolysis and gets cleaved even if it is attached on SO1861.

In order to conjugate SO1861-EMCH to a polymeric structure, accessible amines of the polymeric structure are converted into thiols with the aid of the agent 2-iminothiolane. The generated free thiols on the polymeric structure act then as the nucleophile for the thiol-ene Michael-type addition to the maleimide group of SO1861-EMCH (Figure 68). This developed methodology is suitable for the conjugation of SO1861-EMCH to any available polymeric structure that obtains accessible amine groups and allows furthermore a control over the number of conjugated SO1861 molecules depending on the polymeric structure, respectively.

First proof of concept for conjugation of ready-to conjugate saponins' to a polymeric structure was obtained by use of the amine of a protein (poly amino acid scaffold example), bovine serum albumin (BSA). After conjugation, mass spectrometry obtained the corresponding peaks of BSA-SO1861 at *m*/*z* ∼ 70, ∼ 72, and ∼ 74 kDa (Figure 69 A). In comparison with the detected mass of BSA with *m*/*z* 66 kDa (Figure 69 B), the obtained masses of BSA-SO1861 correspond to a mixture of BSA-SO1861 conjugates consisting of 2, 3, and 4 SO1861 molecules per BSA.

Next proof of concept for conjugation of 'ready-to conjugate saponins' to a polymeric structure was obtained by the use of the amine bearing generation 5 (G5) dendrimer poly(amidoamine) (PAMAM with covalently coupled red-fluorescent dye (Cy3)). PAMAM-Cy3 was utilized as the polymeric structure for the conjugation to both SO1861-EMCH and SO1861-HATU and served as a model for conjugation of S01861 to a polymeric structure (Figure 70).

All accessible amine groups of Cy3-PAMAM were converted into thiols using a 3 fold excess of 2-iminothiolane per Cy3-PAMAM amines followed by the reaction with SO1861-EMCH. Three different feed equivalents (5, 20 and 57) of SO1861-EMCH were used for the three reaction batches. After reaction, the recorded masses of the Cy3-PAMAM-SO1861 conjugates at MALDI-TOF-MS show an increment of the corresponding masses with increasing the SO1861-EMCH feed (Figure 71). The three different feeds corresponded to an obtained mass *of m*/*z* 38.4 kDa, *m*/*z* 53.9 kDa and *m*/*z* 133.8 kDa for the Cy3-PAMAM-SO1861 conjugates that correspond to 6, 13 and 51 SO1861 molecules attached per PAMAM dendrimer as shown on Figure 71 B-D.

In another reaction, only a certain number of PAMAM amines were converted into thiols prior to reaction with SO1861-EMCH. Here, two different feed equivalents of 2-Iminothiolane (8 and 32) and two different feed equivalents of SO1861-EMCH (5 and 30) were used, respectively. After reaction, the respective spectra of the Cy3-PAMAM-SO1861 conjugates at MALDI-TOF-MS show peaks at *m*/*z* 37.7 kDa (5 feed equivalents of SO1861-EMCH) and at *m*/*z* 87.0 kDa (30 feed equivalents of SO1861-EMCH) as shown in Figure 72. The obtained masses at *m*/*z* 37.7 kDa and *m*/*z* 87.0 kDa correspond to Cy3-PAMAM-SO1861 conjugates with 5 and 30 SO1861 molecules attached and demonstrate that with this method almost all feed of SO1861-EMCH were conjugated.

For the generation of a non-pH-cleavable saponin conjugate the carboxylic acid of SO1861 was activated with HATU and then reacted with the amines of Cy3-PAMAM forming a pH stable amide bound between Cy3-PAMAM and SO1861. The resulting mass of the conjugate was detected via MALDI-TOF-MS with a mass of *m*/*z* 62.3 kDa that corresponds to Cy3-PAMAM-NC-S01861 (NC = non-cleavable) conjugate with 17.5 SO1861 molecules attached per PAMAM (Figure 70 B, Figure 73).

Next, the saponin conjugated scaffolds were conjugated to linking points for a possible conjugation to targeted therapeutics (e.g. targeted toxins) via the so-called strain-promoted alkyne-azide cycloaddition (SPAAC, click chemistry) to obtain a functionalized scaffold. For this reaction, Cy3-PAMAM-SO1861 (Figure 74 C, D) and Cy3-PAMAM-NC-S01861 (Figure 74 B) were conjugated to a heterobifunctional NHS-PEG₁₃-DBCO linker that generated a strained alkyne on the conjugates' surface (Figure 74 A). The NHS (N Hydroxysuccinimide) moiety of the linker reacted with remaining amines of the PAMAM-saponin conjugates forming an amide bond between the scaffold and the linker. The resulting DBCO (dibenzocyclooctyne) moiety on the conjugates is able to perform SPAAC with corresponding azides on the targeted therapeutics.

Dianthin-EGF served as a model targeted toxin and dianthin served as a non-targeted toxin. Both toxins were labeled with Alexa Fluor™ 488 using the tetrafluorophenyl ester (TFP) derivative of the dye. The dye labeled proteins were then conjugated to a heterobifunctional NHS-SS-PEG₃-azide linker to obtain the corresponding chemical moiety for the SPAAC to the PAMAM-saponin conjugates. Maldi-TOF-MS measurements showed that one Alexa Fluor™ 488 dye and 9 NHS-SS-PEG3-azide molecules were attached per dianthin-EGF molecule (Figure 75, Figure 76). Furthermore, Alexa Fluor™ 488 labeled dianthin-EGF was conjugated to a heterobifunctional NHS-PEG₁₂-azide linker that lacked the disulfide bond and would thus generate a non-toxin-cleavable construct.

The Cy3-PAMAM-NC-SO1861-DBCO and Cy3-PAMAM-S01861-DBCO conjugates were reacted with Alexa Fluor™ 488 labeled azido-toxins to perform a strain-promoted alkyne-azide cycloaddition. The conjugation between the reacting agents was indicated via gel electrophoresis and the co-localization of the fluorescent signals of Cy3 that is only attached on the PAMAM polymer and Alexa Fluor™ 488 that is only attached on the toxins on a polyacrylamide gel after gel electrophoresis (Figure 77).

As an alternative polymeric structure to the PAMAM dendrimer, a G4-dendron (PFd-G4-Azide-NH-BOC, Polymer Factory) with 16 functional amino end groups and an azido group at the focal point was utilized for the conjugation to SO1861 (Figure 84). The advantage of using a dendron over a dendrimer is the focal point that the dendron structure is exhibiting. This focal point allows the direct conjugation to a targeted toxin without the need of its post-modification with orthogonal click functions (Figure 85). As shown in Figure 85, the dendron underwent the same methodology as described for the PAMAM dendrimer. Briefly, after partial dye labeling and deprotection (Figure 86), the amino groups of the dendron were converted into thiols using the thiolating reagent 2-iminothiolane followed by conjugation to SO1861-EMCH. For the conjugation to SO1861-EMCH three different feed equivalents of SO1861-EMCH were used. The dendron-SO1861 conjugates were analyzed via MALDI-TOF-MS. As expected, the conjugate species of 1 and 2 SO1861 molecules per dendron molecule were obtained when low SO1861-EMCH feed equivalents of 3 and 10 were used (Figure 87 B, C). Higher dendron-SO1861 conjugate species of up to 9 SO1861 molecules per dendron were obtained (Figure 87 A) when using a feed equivalent of 22 SO1861-EMCH molecules per dendron molecule. In further experiments, the saponin functionalized dendron will be conjugated to targeted toxins over its focal point to yield a functionalized scaffold and will be evaluated biologically.

The previous examples demonstrate that a methodology has been developed that allows the conjugation of a determined amount of SO1861 molecules or other endosomal escape enhancer molecules to a polymeric structure for enhanced cytoplasmic delivery of therapeutic substances such as targeted toxins.

To further test other conjugation methodologies of SO1861 to a polymeric structure, the reductive amination pathway was used. For this, the aldehyde group of SO1861 was directly conjugated to PAMAM amines forming an imine bound. The imine bond formation was followed a reductive amination step through the addition of the reductive agent sodium cyanoborohydride forming a pH-stable amine bond between SO1861 and PAMAM (Figure 78 A). Similar to the EMCH and HATU approach, this methodology allows a control over the number of conjugated saponins per polymer as shown on Figure 78 B, C. Here, PAMAM-saponin conjugates were produced which obtained a number of 10 (Figure 78 B) and 30 (Figure 78 C) SO1861 molecules per PAMAM.

Another approach for the development of a SO1861 scaffold among the discussed polymer, and protein approach is the poly(SO1861) approach. The idea of this approach is to generate a polymerthat consists of SO1861 molecules only, with pH sensitive cleavable bonds that release the SO1861. In addition, the poly(SO1861) should be able to perform conjugation reactions to toxins and biopolymers. The main goal with this approach is to keep it as simple and cost effective as possible. Since a protocol for the generation of acid cleavable SO1861 has been developed already (SO1861-EMCH approach) it would be interesting to see if it is possible to polymerize the SO1861-EMCH through simple addition of a polymerization initiator without further modifying the SO1861 or identifying other conjugation sites on the S01861 molecule. In the past, several papers have discussed the polymerization of maleimide groups by using radical initiators which attack the double bond of the maleimide group and thus initiate a radical polymerization along the double bonds of the maleimides (29-31). Since SO1861-EMCH reveals a maleimide group in its structure this group could potentially be explored for radical polymerization reactions to yield a poly(SO1861) with acid cleavable function. If the polymerization reaction has a reasonable reaction time the generated SO1861 polymers could be quenched with a radical quencher that not only quenches the reaction but also generates a functional group for toxin or biopolymer conjugation. Such a reaction scheme is illustrated in Figure 79. Here, the system of ammonium persulfate (APS) and tetramethylethylenediamine (TMEDA) is shown in an exemplary way as radical generator and aminopropanethiol serves as a model radical quencher. Using aminopropanethiol as a quencher exemplary, the generated amine group could be specifically further modified to a click-able group or being used to directly conjugate the poly(SO1861) to a toxin.

In free radical polymerization the reaction conditions have a huge influence on the polymer properties and the reaction outcome. For instance, temperature, monomer concentration, and initiator concentration play a major role for forming the polymer and have to be fine-tuned according to the desired polymer properties. As radical polymerizations are usually carried out at temperatures above 50 °C, the first reactions have been performed at a temperature of 60 °C. It was interesting to see if the SO1861-EMCH polymerization can be initiated spontaneously and if APS and TMEDA would have an influence on the polymerization degree. Thus, three reactions have been carried out, using the same SO1861-EMCH concentration, but differ in their APS / TMEDA composition. In the first reaction only the SO1861-EMCH was heated up to 60 °C for 3 h, while the second reaction contained SO1861-EMCH and APS, and the third reaction contained SO1861-EMCH, APS, and TMEDA. (For these experiments the same amount and concentration of starting materials have been used which are mentioned in the Materials and Methods section "Poly(SO1861) synthesis"). The batches have been analyzed via MALDI-TOF-MS as shown on Figure 80 A-C. Interestingly it has been shown that SO1861-EMCH started to form oligomers consisting of 2, 3, 4, 5, and 6 SO1861 molecules spontaneously when heated up to 60 °C (Figure 80 A). The addition of 11⁻³ equivalents APS at the same temperature had no influence on this trend (Figure 80 B). When using the initiator system of APS / TMEDA, however, SO1861 oligomers of up to 9 SO1861 molecules with a molecular weight of 18.2 kDa could be detected (Figure 80 C). In addition, the obtained peaks for the oligomers seemed much bigger in comparison with the peaks in Figure 80 A and 80 B, indicating a higher consumption of SO1861-EMCH for this reaction.

To further fine-tune the reaction conditions, other initiators such as azo-initiators like 2,2'-azobis[2-methyl-N-(2-hydroxyethyl)propionamide] and azobisisobutyronitrile will be tested, as well as other polymerization techniques such as controlled radical polymerization (atom-transfer radical-polymerization, reversible addition-fragmentation chain transfer, etc). Moreover, another hydrazide linker as a substitute for EMCH could be considered which obtains a functional group (such as an acryl or acrolyol residue) that is more suitable for radical polymerization than the maleimide group.

Another approach for the development of a SO1861 scaffold is the DNA approach. The idea of this approach is to utilize the concept of the so-called DNA-origami (Kolb et al, 2004; Bird et al, 1988). DNA-origami as the polymeric or assembled polymeric structure to conjugate saponins to it, can offer several inherent advantages including stability, scalability, and precise control of the final size and shape of the resulting DNA-saponin scaffold. Since these DNA nanocarriers are comprised of natural DNA, they are biocompatible and do not show toxicity to living cells, and can ease the release of cargo from internal cellular compartments. The multivalency of such a structure can further allow fine-tuning targeting capabilities and high capacity for a variety of payloads such as fluorophores and toxins. Thus, in this approach DNA strands are identified that offer chemical functional groups on the 3' and 5' endings respectively, and that are able to hybridize only in certain wanted areas of the sequence that allow a control over the final shape of the construct. The chemical groups should be utilized to couple saponins, for instance though a thiol-ene reaction between the already developed SO1861-EMCH and a thiol group on one of the 3' and 5' DNA strands. The complementary DNA strand can offer a click function group that can be used for coupling to a targeted toxin. The concept is illustrated in Figure 81.

A similar approach is imaginable by using a specific peptide sequence instead of DNA strands that is able to bind and release saponins and that can be polymerized forming a large poly(peptide)-like structure. In this approach, a peptide sequence has been identified and purchased that has a length fitting the calculated size of a SO1861-EMCH molecule, that offers a cysteine residue in the middle of the sequence, and that obtains an amine group at both the N-terminus and C-terminus. The cysteine residue can be utilized to conjugate SO1861-EMCH via a thiol-ene reaction of the maleimide group of SO1861-EMCH and the thiol group of the cysteine residue. The two amine groups can be utilized to polymerize the peptide-S01861 conjugate with a suitable crosslinker as shown on Figure 82.

Conjugation studies have shown that the conjugation of SO1861-EMCH to the customized peptide (sequence: SESDDAMFCDAMDESDSK) was successful. The peptide that bears a maleimide reactive cysteine in the middle of the sequence and its conjugation to SO1861-EMCH was analyzed via MALDI-TOF-MS (Figure 83). The MALDI-TOF-MS spectra shows the expected peak for the peptide-S01861 conjugate at *m*/*z* 4053 Da and an additional peak at *m*/*z* 3821 Da which is the peptide-Sd 861 conjugate of the corresponding saponin-EMCH of SO1730. As SO1861-EMCH has been used in slight excess (1.3 equivalents) and no SO1861-EMCH peak was detected after reaction, it can be assumed that the conjugation was quantitative. For starting first polymerization reactions of the peptide-S01861, disuccinimidyl tartrate will be utilized as the amine reactive cross-linker.

Since the endosomal escape enhancing properties of SO1861 are only exposed at low endosomal pH (< pH5), the scaffold or functionalized scaffold should not contain chemical groups that are able to interfere in acidification of the endosomes and thus block the activity of SO1861.

The amine containing polymeric structures of a G5 PAMAM (128 primary amines as well as approximately 126 tertiary amines) and G4-dendron (16 primary amines) were tested, in order to determine if these molecules inhibit the endosomal escape enhancing capacity of SO1861. Coadministration experiments of PAMAM (native or thiolated) or dendron (native) in combination with dianthin-EGF and various SO1861 concentrations on HeLa cells were performed. As control for the inhibition of endosomal acidification chloroquine was used.

HeLa cells show sufficient EGFR cell surface levels (Figure 88 A). It is observed that both 'native' PAMAM and chloroquine inhibit the SO1861-mediated endosomal escape of the targeted toxin and subsequent cell killing in Hela cells (Figure 88 B). PAMAM at 500 nM inhibits even to an equal extent as the endosomal acidification inhibitor chloroquine, while 667 nM dendron has no effect at all. To further address if the inhibitory activity of the 'native' PAMAM is due to the availability of amino groups in PAMAM, the primary amino groups of PAMAM were partially thiolated through reaction with 2-iminothiolane (Figure 89), resulting in 16 of128 (Figure 89 C), 65/128 (Figure 89 D), and 108/128 (Figure 89 E) blocked primary amines. It is observed that thiolation of 65 and 108 primary amines overcomes the inhibition of SO1861-mediated endosomal escape, whereas thiolation of up to 16 amines groups still shows the inhibitory effects of SO1861-mediated endosomal escape of the targeted toxin (Figure 88 C). The primary amino groups of PAMAM were also partially PEGylated through a reaction with mPEG2k-NHS (Figure 90), resulting in 3 of 128 (Figure 90 C), 8/128 (Figure 90 D), and 18/128 (Figure 90 E) blocked primary amines. Blocking only 3 primary amines by PEGylation is already sufficient to reverse the inhibition of SO1861-mediated endosomal escape (Figure 89 D). The shielding effect of PEGylation most likely extends beyond the small number of amines that are converted, as PEGylation is known to introduce a hydration layer that can shield off an entire molecule, if a sufficient level is reached.

These results demonstrate that the presence of a certain number of free amino groups on polymeric structures, such as PAMAM, can block endosomal acidification and thus inhibit the endosomal escape activity of SO1861 or other glycosides. When the number of amino groups is lower, as shown for the G4-dendron, or if the amino groups have been shielded, such as thiolation or PEGylation, the inhibitory effect is reversed.

### REFERENCES

Weng, A.; Thakur, M.; Beceren-Braun, F.; Bachran, D.; Bachran, C.; Riese, S.B.; Jenett-Siems, K.; Gilabert-Oriol, R.; Melzig, M.F.; Fuchs, H. The toxin component of targeted anti-tumor toxins determines their efficacy increase by saponins. Molecular oncology 2012, 6, 323-332. saponinum album in a synergistic way. Journal of immunotherapy 2009, 32, 713-725.
Sama, S.; Jerz, G.; Schmieder, P.; Joseph, J.F.; Melzig, M.F.; Weng, A. Plant derived triterpenes from Gypsophila elegans M.Bieb. enable non-toxic delivery of gene loaded nanoplexes. Journal of Biotechnology 2018, 284, 131-139
Kolb, H.C.; Finn, M.G.; Sharpless, K.B. Click Chemistry: Diverse Chemical Function from a Few Good Reactions. Angewandte Chemie 2001, 40, 2004-2021.
Bird, R.E.; Hardmann, K.D.; Jacobson, J.W.; Johnson, S.; Kaufman, B.M.; Lee, S.M.; Lee, T.; Pope, S.H.; Riordan, G.S.; Whitlow, M. Single-chain antigen-binding proteins. Science 1988, 242, 423-426.
Y Zhang, Z Qu, S Kim, V Shi, B Liao1, P Kraft, R Bandaru, Y Wu, LM Greenberger and ID Horak, Down-modulation of cancer targets using locked nucleic acid (LNA)-based antisense oligonucleotides without transfection, Gene Therapy (2011) 18, 326-333.

## Claims

1. A first proteinaceous molecule comprising a first binding site for binding to a first epitope of a first cell-surface molecule, covalently bound to at least one saponin via at least one linker and/or via an oligomeric or polymeric scaffold to an amino-acid residue of said first proteinaceous molecule, or covalently bound directly to an amino-acid residue of said first proteinaceous molecule, wherein
- the first binding site comprises or consists of an immunoglobulin, such as an antibody or an IgG, or comprises or consists of at least one ligand for binding to a cell-surface molecule such as EGF or a cytokine, and
- wherein the at least one saponin is selected from the group consisting of: SO1861, SA1641, GE1741, QS-21, QS-21A, QS21-B, Quil-A, SO1832, SO1904, and SO1862.

2. The first proteinaceous molecule of claim 1, wherein the first epitope of the first cell-surface molecule is a tumor-cell specific first epitope of a first tumor-cell surface molecule.

3. The first proteinaceous molecule of claim 1 or 2, wherein the at least one saponin is SO1861.

4. The first proteinaceous molecule of any one of the claims 1-3, wherein
- the at least one saponin is a bisdesmosidic triterpene saponin belonging to the type of a 12,13-dehydrooleanane with an aldehyde function in position C-23,
- the at least one saponin is covalently coupled to the amino-acid residue of the first proteinaceous molecule via an aldehyde function in the saponin, preferably said aldehyde function in position C-23, preferably via at least one linker, more preferably via at least one cleavable linker, wherein the amino-acid residue preferably is selected from cysteine and lysine.

5. The first proteinaceous molecule of any one of the claims 1-4, wherein
- the at least one saponin is a bisdesmosidic triterpene saponin belonging to the type of a 12,13-dehydrooleanane with an aldehyde function in position C-23 and comprising a glucuronic acid function in a carbohydrate substituent at the C-3beta-OH group of the saponin,
- the at least one saponin is covalently coupled to the amino-acid residue of the first proteinaceous molecule via the glucuronic acid function in the carbohydrate substituent at the C-3beta-OH group of the saponin, preferably via at least one linker, wherein the amino-acid residue preferably is selected from cysteine and lysine.

6. The first proteinaceous molecule of any one of the claims 4-5, wherein the aldehyde function in position C-23 of the at least one saponin is covalently coupled to linker N-ε-maleimidocaproic acid hydrazide, which linker is covalently coupled via a thio-ether bond to a sulfhydryl group in the first proteinaceous molecule, such as a sulfhydryl group of a cysteine.

7. The first proteinaceous molecule of any one of the claims 5-6, wherein the glucuronic acid function in the carbohydrate substituent at the C-3beta-OH group of the at least one saponin is covalently coupled to linker 1-[Bis(dimethylamino)methylene]-1H-1,2,3-triazolo[4,5-b]pyridinium 3-oxid hexafluorophosphate, which linker is covalently coupled via an amide bond to an amine group in the first proteinaceous molecule, such as an amine group of a lysine or an N-terminus of the first proteinaceous molecule.

8. The first proteinaceous molecule of any one of the claims 1-7, wherein the said first epitope is a tumor-cell specific first epitope of a tumor-cell specific receptor preferably selected from CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, more preferably selected from CD71, EGFR, HER2.

9. The first proteinaceous molecule of claim 8, wherein the said first binding site comprises or consists of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell receptor binding-fragment or domain thereof, preferably at least one tumor-cell specific receptor binding-fragment thereof and/or at least one tumor-cell specific receptor binding-domain thereof.

10. The first proteinaceous molecule of any one of the claims 1-9, wherein the first proteinaceous molecule comprises more than one saponin, preferably 2, 3, 4, 5, 6, 8, 10, 16, 32, 64 or 1-100 saponins, or any number of saponins therein between, such as 7, 9, 12 saponins, wherein the saponins are:
- covalently bound directly to an amino-acid residue of the first proteinaceous molecule, preferably to a cysteine and/or to a lysine, and/or
- covalently bound via at least one linker and/or via at least one cleavable linker and/or via at least one polymeric or oligomeric scaffold, preferably 1-8 of such scaffolds or 2-4 of such scaffolds, wherein the at least one scaffold is optionally based on a dendron, in which case 1-32 saponins such as 2, 3, 4, 5, 6, 8, 10, 16, 32 saponins, or any number of saponins therein between, such as 7, 9, 12 saponins, are covalently bound to the at least one scaffold.

11. The first proteinaceous molecule of any one of the claims 4-10, wherein the at least one linker is a non-cleavable linker or a cleavable linker, wherein the cleavable linker for example
- is subject to cleavage under acidic conditions, reductive conditions, enzymatic conditions or light-induced conditions, and preferably the cleavable linker comprises a hydrazone bond or a hydrazide bond subject to cleavage under acidic conditions when bound to saponin, and/or
- comprises a bond susceptible to proteolysis, for example proteolysis by Cathepsin B, and/or is a bond susceptible for cleavage under reductive conditions such as a disulphide bond, when bound to saponin.

12. The first proteinaceous molecule of any one of the claims 4-11, wherein the cleavable linker is subject to cleavage *in vivo* under acidic conditions as present in endosomes and/or lysosomes of mammalian cells, preferably human cells, preferably at pH 4.0 - 6.5, and more preferably at pH ≤ 5.5, when the cleavable linker is bound to a saponin.

13. The first proteinaceous molecule of any one of the claims 1-12, wherein the oligomeric or polymeric scaffold comprises a polymeric or oligomeric structure and comprises a chemical group, the said chemical group being for covalently coupling of the scaffold to the amino-acid residue of said first proteinaceous molecule.

14. The first proteinaceous molecule of any one of the claims 1-13, wherein the at least one saponin is covalently bound to the polymeric or oligomeric structure of the oligomeric or polymeric scaffold via at least one cleavable linker as defined in any one of the claims 10 or 11.

15. The first proteinaceous molecule of any one of the claims 1-14, wherein the chemical group of the oligomeric or polymeric scaffold, for covalently coupling of the oligomeric or polymeric scaffold to the amino-acid residue of said first proteinaceous molecule, is a click chemistry group, preferably selected from a tetrazine, an azide, an alkene or an alkyne, or a cyclic derivative of these groups, more preferably said chemical group is an azide.

16. The first proteinaceous molecule of any one of the claims 1-15, wherein the polymeric or oligomeric structure of the oligomeric or polymeric scaffold comprises a linear, branched and/or cyclic polymer, oligomer, dendrimer, dendron, dendronized polymer, dendronized oligomer, a DNA, a polypeptide, polylysine, a poly-ethylene glycol, or an assembly of these polymeric or oligomeric structures which assembly is preferably built up by covalent cross-linking.

17. A therapeutic combination, wherein the therapeutic combination comprises:
(a) a first pharmaceutical composition comprising the first proteinaceous molecule of any one of the claims 1-16 and optionally a pharmaceutically acceptable excipient; and
either
(b) a second pharmaceutical composition comprising a second proteinaceous molecule different from the first proteinaceous molecule and optionally further comprising a pharmaceutically acceptable excipient, the second proteinaceous molecule comprising
- a second binding site for binding to a second epitope of a second cell-surface molecule different from the first cell-surface molecule, and
- an effector moiety, such as a toxin, an enzyme or an oligonucleotide
wherein the second epitope is different from the first epitope,
or
(c) a third pharmaceutical composition comprising a third proteinaceous molecule and optionally further comprising a pharmaceutically acceptable excipient, the third proteinaceous molecule comprising
- a third binding site for binding to the first epitope on the cell-surface molecule of (a) and
- an effector moiety, such as a toxin, an enzyme or an oligonucleotide;
wherein the first binding site and the third binding site are the same, and wherein the first cell-surface molecule and the first epitope on the first cell-surface molecule, to which the first proteinaceous molecule can bind, and the first cell-surface molecule and the first epitope on the first cell-surface molecule, to which the third proteinaceous molecule can bind, are the same.

18. Therapeutic combination of claim 17, wherein:
(a) the therapeutic combination comprises the said first pharmaceutical composition and the said second pharmaceutical composition, wherein
- the first epitope on the first cell-surface molecule is a tumor-cell specific first epitope on a first tumor cell-specific surface molecule, preferably a tumor-cell specific first epitope on a first cell-surface receptor specifically present at a tumor cell; and
- the second cell-surface molecule is a second tumor cell-specific surface molecule different from the first tumor cell-specific surface molecule, preferably a second cell-surface receptor specifically present at a tumor cell different from the first cell-surface receptor specifically present at said tumor cell, and wherein the second epitope is a tumor-cell specific second epitope;
or
(b) the therapeutic combination comprises the said first pharmaceutical composition and the said third pharmaceutical composition,
wherein the first cell-surface molecule is expressed on a tumor cell surface, and preferably the first cell-surface molecule is a tumor cell-specific surface molecule, and wherein preferably the first epitope is a first tumor-cell specific epitope.

19. The therapeutic combination of claim 17 or 18, wherein the second binding site of the second proteinaceous molecule or the third binding site of the third proteinaceous molecule comprises or consists of an immunoglobulin, at least one binding domain of an immunoglobulin and/or at least one binding fragment of an immunoglobulin, such as an antibody, an IgG, a molecule comprising or consisting of a Vhh domain or Vh domain, a Fab, an scFv, an Fv, a dAb, an F(ab)2, Fcab fragment, or comprises or consists of at least one ligand for binding to a cell-surface molecule such as EGF or a cytokine.

20. The therapeutic combination of any one of the claims 17-19, wherein
- the said first binding site binds a first tumor-cell specific epitope on a first tumor-cell specific receptor, and
- the said second binding site binds a second tumor-cell specific epitope on a second tumor-cell specific receptor,
and wherein
- the said first and second tumor cell-specific receptors are different and present on the same tumor cell,
- the first and second binding site are different and
- the first and second tumor cell specific epitope are different.

21. The therapeutic combination of any one of the claims 17-20, wherein said first and third binding site, being the same, are a binding site for a first tumor-cell specific epitope on a first tumor-cell specific receptor.

22. The therapeutic combination of claim 20 or 21, wherein the first receptor and/or the second receptor are selected from CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably selected from CD71, EGFR and HER2; with the provisos that
- when the first and second proteinaceous molecules are present in the therapeutic combination, the first binding site of the first proteinaceous molecule is different from the second binding site of the second proteinaceous molecule,
- when the first and third proteinaceous molecules are present in the therapeutic combination, the first and third binding sites are the same.

23. The first proteinaceous molecule of any one of the claims 1-16 or therapeutic combination of any one of the claims 17-22,
wherein the first binding site and/or the second binding site is/are or comprise(s) a monoclonal antibody or at least one cell-surface molecule binding fragment and/or-domain thereof, and preferably comprise or consist of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, and an antibody of Table A4, preferably cetuximab or trastuzumab or OKT-9, or at least one cell-surface molecule binding fragment or -domain thereof, with the proviso that the first binding site of the first proteinaceous molecule is different from the second binding site of the second proteinaceous molecule.

24. The therapeutic combination of any one of the claims 17-23, wherein
(a) the said first binding site and the said third binding site comprise a monoclonal antibody, or a cell-surface molecule binding domain or -fragment thereof, and preferably comprise or consist of any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one cell-surface molecule binding fragment and/or -domain thereof, with the proviso that the first binding site of the first proteinaceous molecule is the same as the third binding site of the third proteinaceous molecule;
or
(b) the said second binding site or the said third binding site is or comprises a monoclonal antibody, or cell-surface molecule binding fragment or -domain thereof, and preferably comprises or consists of any one of Gemtuzumab ozogamicin, Brentuximab vedotin, Trastuzumab emtansine, Inotuzumab ozogamicin, Moxetumomab pasudotox, Polatuzumab vedotin or an antibody-drug conjugate of Table A2 or Table A3.

25. The therapeutic combination of any one of the claims 17-24, wherein the effector moiety that is comprised by the second proteinaceous molecule respectively by the third proteinaceous molecule comprises or consists of:
(i) any one or more of an oligonucleotide, a nucleic acid, a xeno nucleic acid, preferably selected from any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-0,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression;
or
(ii) at least one proteinaceous molecule, preferably selected from any one or more of a peptide, a protein, an enzyme such as urease and Cre-recombinase, a ribosome-inactivating protein, a proteinaceous toxin, more preferably selected from any one or more of a protein toxin selected from Table A5 and/or a viral toxin such as apoptin; a bacterial toxin such as Shiga toxin, Shiga-like toxin, Pseudomonas aeruginosa exotoxin (PE) or exotoxin A of PE, full-length or truncated diphtheria toxin (DT), cholera toxin; a fungal toxin such as alpha-sarcin; a plant toxin including ribosome-inactivating proteins and the A chain of type 2 ribosome-inactivating proteins such as dianthin e.g. dianthin-30 or dianthin-32, saporin e.g. saporin-S3 or saporin-S6, bouganin or de-immunized derivative debouganin of bouganin, shiga-like toxin A, pokeweed antiviral protein, ricin, ricin A chain, modeccin, modeccin A chain, abrin, abrin A chain, volkensin, volkensin A chain, viscumin, viscumin A chain; or an animal or human toxin such as frog RNase, or granzyme B or angiogenin from humans, or any fragment or derivative thereof; preferably the protein toxin is dianthin and/or saporin;
or
(iii) at least one payload, preferably selected from any one or more of a toxin targeting ribosomes, a toxin targeting elongation factors, a toxin targeting tubulin, a toxin targeting DNA and a toxin targeting RNA, more preferably any one or more of emtansine, pasudotox, maytansinoid derivative DM1, maytansinoid derivative DM4, monomethyl auristatin E (MMAE, vedotin), monomethyl auristatin F (MMAF, mafodotin), a Calicheamicin, N-Acetyl-y-calicheamicin, a pyrrolobenzodiazepine (PBD) dimer, a benzodiazepine, a CC-1065 analogue, a duocarmycin, Doxorubicin, paclitaxel, docetaxel, cisplatin, cyclophosphamide, etoposide, docetaxel, 5-fluorouracyl (5-FU), mitoxantrone, a tubulysin, an indolinobenzodiazepine, AZ13599185, a cryptophycin, rhizoxin, methotrexate, an anthracycline, a camptothecin analogue, SN-38, DX-8951f, exatecan mesylate, truncated form of *Pseudomonas aeruginosa* exotoxin (PE38), a Duocarmycin derivative, an amanitin, α-amanitin, a spliceostatin, a thailanstatin, ozogamicin, tesirine, Amberstatin269 and soravtansine, or a derivative thereof.

26. A composition comprising the first proteinaceous molecule of any one of the claims 1-16 and either
- the second proteinaceous molecule as defined in any one of the claims 17-25 and optionally a pharmaceutically acceptable excipient,
or
- the third proteinaceous molecule as defined in any one of the claims 17-25 and optionally a pharmaceutically acceptable excipient,and
- wherein optionally the effector moiety that is comprised by the second proteinaceous molecule or by the third proteinaceous molecule is any one of the effector moieties: an oligonucleotide, a nucleic acid, a xeno nucleic acid, preferably selected from any one or more of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression.

27. A composition comprising
- the first proteinaceous molecule of any one of the claims 1-16 or as defined in any one of the claims 17-23 and
- any one or more of an oligonucleotide, a nucleic acid and a xeno nucleic acid, preferably selected from at least one of a vector, a gene, a cell suicide inducing transgene, deoxyribonucleic acid (DNA), ribonucleic acid (RNA), anti-sense oligonucleotide (ASO, AON), short interfering RNA (siRNA), microRNA (miRNA), DNA aptamer, RNA aptamer, mRNA, mini-circle DNA, peptide nucleic acid (PNA), phosphoramidate morpholino oligomer (PMO), locked nucleic acid (LNA), bridged nucleic acid (BNA), 2'-deoxy-2'-fluoroarabino nucleic acid (FANA), 2'-O-methoxyethyl-RNA (MOE), 2'-O,4'-aminoethylene bridged nucleic acid, 3'-fluoro hexitol nucleic acid (FHNA), a plasmid, glycol nucleic acid (GNA) and threose nucleic acid (TNA), or a derivative thereof, more preferably a BNA, for example a BNA for silencing HSP27 protein expression.

28. An antibody-drug conjugate or a ligand-drug conjugate comprising the first proteinaceous molecule of any one of the claims 1-16 and an effector moiety, optionally wherein:
(i) the antibody can bind to any one of CD71, CA125, EpCAM(17-1A), CD52, CEA, CD44v6, FAP, EGF-IR, integrin, syndecan-1, vascular integrin alpha-V beta-3, HER2, EGFR, CD20, CD22, Folate receptor 1, CD146, CD56, CD19, CD138, CD27L receptor, PSMA, CanAg, integrin-alphaV, CA6, CD33, mesothelin, Cripto, CD3, CD30, CD239, CD70, CD123, CD352, DLL3, CD25, ephrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, preferably CD71, HER2, EGFR, and/or is or comprises any one of cetuximab, daratumumab, gemtuzumab, trastuzumab, panitumumab, brentuximab, inotuzumab, moxetumomab, polatuzumab, obinutuzumab, OKT-9 anti-CD71 monoclonal antibody of the IgG type, pertuzumab, rituximab, ofatumumab, Herceptin, alemtuzumab, pinatuzumab, OKT-10 anti-CD38 monoclonal antibody, an antibody of Table A2 or Table A3 or Table A4, preferably cetuximab or trastuzumab or OKT-9, or at least one tumor-cell receptor binding-fragment thereof and/or at least one tumor-cell receptor binding-domain thereof, and/or wherein the antibody-drug conjugate comprises any one of Gemtuzumab ozogamicin, Brentuximab vedotin, Trastuzumab emtansine, Inotuzumab ozogamicin, Moxetumomab pasudotox and Polatuzumab vedotin and an antibody-drug conjugate of Table A2 and Table A3;
and/or
(ii) the effector moiety is any one or more of the effector moieties defined in claim 26.

29. A pharmaceutical composition comprising the antibody-drug conjugate or the ligand-drug conjugate of claim 28, and optionally further comprising a pharmaceutically acceptable excipient.

30. The therapeutic combination of any one of the claims 17-25 or the composition of claim 26 or 27 or the antibody-drug conjugate or ligand-drug conjugate of claim 28 or the pharmaceutical composition of claim 29, for use as a medicament or for use in the treatment or prevention of a cancer or an autoimmune disease.

## Patentansprüche

1. Erstes proteinöses Molekül umfassend eine erste Bindungsstelle zum Binden an ein erstes Epitop eines ersten Zelloberflächenmoleküls, die kovalent gebunden ist an zumindest ein Saponin über zumindest einen Linker und/oder über ein oligomeres oder polymeres Gerüst an einen Aminosäurerest von besagtem ersten proteinösen Molekül, oder die direkt kovalent gebunden ist an einen Aminosäurerest von besagtem ersten proteinösen Molekül, wobei
- die erste Bindungsstelle umfasst oder besteht aus einem Immunglobulin, wie etwa einem Antikörper oder einem IgG, oder umfasst oder besteht aus zumindest einem Liganden zum Binden an ein Zelloberflächenmolekül, wie etwa EGF oder ein Zytokin, und
- wobei das zumindest eine Saponin ausgewählt ist aus der Gruppe bestehend aus: SO1861, SA1641, GE1741, QS-21, QS-21A, QS-21B, Quil-A, SO1832, SO1904, und SO1862.

2. Erstes proteinöses Molekül nach Anspruch 1, wobei das erste Epitop des ersten Zelloberflächenmoleküls ein tumorzellspezifisches erstes Epitop eines ersten Tumorzelloberflächenmoleküls ist.

3. Erstes proteinöses Molekül nach Anspruch 1 oder 2, wobei das zumindest eine Saponin SO1861 ist.

4. Erstes proteinöses Molekül nach einem der Ansprüche 1-3, wobei
- das zumindest eine Saponin ein bisdesmosidisches Triterpen-Saponin ist, das zur Art eines 12,13-Dehydrooleanan mit einer Aldehydfunktionalität in C-23-Position gehört,
- das zumindest eine Saponin kovalent gekoppelt ist an den Aminosäurerest des ersten proteinösen Moleküls über eine Aldehydfunktionalität im Saponin, bevorzugt besagte Aldehydfunktionalität in C-23-Position, bevorzugt über zumindest einen Linker, mehr bevorzugt über zumindest einen spaltbaren Linker, wobei der Aminosäurerest bevorzugt ausgewählt ist aus Cystein und Lysin.

5. Erstes proteinöses Molekül nach einem der Ansprüche 1-4, wobei
- das zumindest eine Saponin ein bisdesmosidisches Triterpen-Saponin ist, das zur Art eines 12,13-Dehydrooleanan mit einer Aldehydfunktionalität in C-23-Position gehört und eine Glucuronsäurefunktionalität in einem Kohlenwasserstoffsubstituenten an der C-3-Beta-OH-Gruppe des Saponins umfasst,
- das zumindest eine Saponin kovalent gekoppelt ist an den Aminosäurerest des ersten proteinösen Moleküls über die Glucuronsäurefunktionalität im Kohlenwasserstoffsubstituenten an der C-3-Beta-OH-Gruppe des Saponins, bevorzugt über zumindest einen Linker, wobei der Aminosäurerest bevorzugt ausgewählt ist aus Cystein und Lysin.

6. Erstes proteinöses Molekül nach einem der Ansprüche 4-5, wobei die Aldehydfunktionalität in C-23-Position des zumindest einen Saponins kovalent gekoppelt ist an Linker N-ε-Maleinimidcapronsäurehydrazid, wobei dieser Linker kovalent gekoppelt ist über eine Thioetherbindung an eine Sulfhydrylgruppe im ersten proteinösen Molekül, wie etwa eine Sulfhydrylgruppe eines Cysteins.

7. Erstes proteinöses Molekül nach einem der Ansprüche 5-6, wobei die Glucuronsäurefunktionalität im Kohlenwasserstoffsubstituenten an der C-3-Beta-OH-Gruppe des zumindest einen Saponins kovalent gekoppelt ist an Linker 1-[Bis(dimethylamino)methylen]-1H-1,2,3-triazol[4,5-b]pyridinium-3-oxid-hexafluorphosphat, wobei dieser Linker kovalent gekoppelt ist über eine Amidbindung an eine Aminogruppe im ersten proteinösen Molekül, wie etwa eine Aminogruppe eines Lysins oder ein N-Terminus des ersten proteinösen Moleküls.

8. Erstes proteinöses Molekül nach einem der Ansprüche 1-7, wobei das besagte erste Epitop ein tumorzellspezifisches erstes Epitop eines tumorzellspezifischen Rezeptors ist, bevorzugt ausgewählt aus CD71, CA125, EpCAM(17-1A), CD52, CEA, CD44v6, FAP, EGF-IR, Integrin, Syndecan-1, vaskulärem Integrin Alpha-V Beta-3, HER2, EGFR, CD20, CD22, Folatrezeptor 1, CD146, CD56, CD19, CD138, CD27L Rezeptor, PSMA, CanAg, Integrin-AlphaV, CA6, CD33, Mesothelin, Cripto, CD3, CD30, CD239, CD70, CD123, CD352, DLL3, CD25, EphrinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, mehr bevorzugt ausgewählt aus CD71, EGFR, HER2.

9. Erstes proteinöses Molekül nach Anspruch 8, wobei die besagte erste Bindungsstelle umfasst oder besteht aus einem aus Cetuximab, Daratumumab, Gemtuzumab, Trastuzumab, Panitumumab, Brentuximab, Inotuzumab, Moxetumomab, Polatuzumab, Obinutuzumab, OKT-9 Anti-CD71 monoklonalem Antikörper vom IgG-Typ, Pertuzumab, Rituximab, Ofatumumab, Herceptin, Alemtuzumab, Pinatuzumab, OKT-10 Anti-CD38 monoklonalem Antikörper, einem Antikörper aus Tabelle A2 oder Tabelle A3 oder Tabelle A4, bevorzugt Cetuximab oder Trastuzumab oder OKT-9, oder zumindest einem tumorzellrezeptorbindenden Fragment oder Domäne davon, bevorzugt zumindest einem tumorzellspezifischen rezeptorbindenden Fragment davon und/oder zumindest einer tumorzellspezifischen rezeptorbindenden Domäne davon.

10. Erstes proteinöses Molekül nach einem der Ansprüche 1-9, wobei das erste proteinöse Molekül mehr als ein Saponin umfasst, bevorzugt 2, 3, 4, 5, 6, 8, 10, 16, 32, 64 oder 1-100 Saponine, oder irgendeine Anzahl an Saponinen dazwischen, wie etwa 7, 9, 12 Saponine, wobei die Saponine sind:
- direkt kovalent gebunden an einen Aminosäurerest des ersten proteinösen Moleküls, bevorzugt an ein Cystein und/oder an ein Lysin, und/oder
- kovalent gebunden über zumindest einen Linker und/oder über zumindest einen spaltbaren Linker und/oder über zumindest ein polymeres oder oligomeres Gerüst, bevorzugt 1-8 solcher Gerüste oder 2-4 solcher Gerüste, wobei das zumindest eine Gerüst optional auf einem Dendron basiert, in welchem Fall 1-32 Saponine, wie etwa 2, 3, 4, 5, 6, 8, 10, 16, 32 Saponine oder irgendeine Anzahl an Saponinen dazwischen, wie etwa 7, 9, 12 Saponine, kovalent an das zumindest eine Gerüst gebunden sind.

11. Erstes proteinöses Molekül nach einem der Ansprüche 4-10, wobei der zumindest eine Linker ein nicht-spaltbarer Linker oder ein spaltbarer Linker ist, wobei der spaltbare Linker beispielsweise
- zur Spaltung unter sauren Bedingungen, reduktiven Bedingungen, enzymatischen Bedingungen oder lichtinduzierten Bedingungen unterliegt, und bevorzugt der spaltbare Linker eine Hydrazonbindung oder eine Hydrazidbindung umfasst, die zur Spaltung unter sauren Bedingungen unterliegt, wenn an Saponin gebunden, und/oder
- eine für Proteolyse anfällige Bindung umfasst, beispielsweise Proteolyse durch Cathepsin B, und/oder eine für Spaltung unter reduktiven Bedingungen anfällige Bindung ist, wie etwa eine Disulfidbindung, wenn an Saponin gebunden.

12. Erstes proteinöses Molekül nach einem der Ansprüche 4-11, wobei der spaltbare Linker zur Spaltung *in vivo* unter sauren Bedingungen unterliegt, wie vorliegend in Endosomen und/oder Lysosomen von Säugetierzellen, bevorzugt Menschenzellen, bevorzugt bei pH-Wert 4,0 - 6,5, und mehr bevorzugt bei pH-Wert ≤ 5,5, wenn der spaltbare Linker an ein Saponin gebunden ist.

13. Erstes proteinöses Molekül nach einem der Ansprüche 1-12, wobei das oligomere oder polymere Gerüst eine polymere oder oligomere Struktur umfasst und eine chemische Gruppe umfasst, wobei die besagte chemische Gruppe zum kovalenten Koppeln des Gerüsts an den Aminosäurerest von besagtem ersten proteinösen Molekül ist.

14. Erstes proteinöses Molekül nach einem der Ansprüche 1-13, wobei das zumindest eine Saponin kovalent gebunden ist an die polymere oder oligomere Struktur des oligomeren oder polymeren Gerüsts über zumindest einen spaltbaren Linker wie in einem der Ansprüche 10 oder 11 definiert.

15. Erstes proteinöses Molekül nach einem der Ansprüche 1-14, wobei die chemische Gruppe des oligomeren oder polymeren Gerüsts zum kovalenten Koppeln des oligomeren oder polymeren Gerüsts an den Aminosäurerest von besagtem ersten proteinösen Molekül eine Click-Chemie-Gruppe ist, bevorzugt ausgewählt aus einem Tetrazin, einem Azid, einem Alken oder einem Alkin, oder ein zyklisches Derivat dieser Gruppen, mehr bevorzugt ist besagte chemische Gruppe ein Azid.

16. Erstes proteinöses Molekül nach einem der Ansprüche 1-15, wobei die polymere oder oligomere Struktur des oligmeren oder polymeren Gerüsts ein lineares, verzweigtes und/oder zyklisches Polymer, Oligomer, Dendrimer, Dendron, dendronisiertes Polymer, dendronisiertes Oligomer, eine DNA, ein Polypeptid, Polylysin, ein Polyethylenglykol, oder eine Anordnung dieser polymeren oder oligomeren Strukturen umfasst, wobei diese Anordnung bevorzugt durch kovalentes Crosslinking ausgebildet ist.

17. Therapeutische Kombination, wobei die therapeutische Kombination umfasst:
(a) eine erste pharmazeutische Zusammensetzung, die das erste proteinöse Molekül nach einem der Ansprüche 1-16 und optional einen pharmazeutisch annehmbaren Arzneimittelträger umfasst;
und
entweder
(b) eine zweite pharmazeutische Zusammensetzung, die ein zweites proteinöses Molekül verschieden vom ersten proteinösen Molekül umfasst und optional ferner einen pharmazeutisch annehmbaren Arzneimittelträger umfasst, wobei das zweite proteinöse Molekül umfasst
- eine zweite Bindungsstelle zum Binden an ein zweites Epitop eines zweiten Zelloberflächenmoleküls verschieden vom ersten Zelloberflächenmolekül, und
- einen Effektorteil, wie etwa ein Toxin, ein Enzym oder ein Oligonukleotid
wobei das zweite Epitop verschieden vom ersten Epitop ist,
oder
(c) eine dritte pharmazeutische Zusammensetzung, die ein drittes proteinöses Molekül umfasst und optional ferner einen pharmazeutisch annehmbaren Arzneimittelträger umfasst, wobei das dritte proteinöse Molekül umfasst
- eine dritte Bindungstelle zum Binden an das erste Epitop am Zelloberflächenmolekül von (a)
und
- einen Effektorteil, wie etwa ein Toxin, ein Enzym oder ein Oligonukleotid;
wobei die erste Bindungsstelle und die dritte Bindungsstelle die gleiche sind, und wobei das erste Zelloberflächenmolekül und das erste Epitop am ersten Zelloberflächenmolekül, an das das erste proteinöse Molekül binden kann, und das erste Zelloberflächenmolekül und das erste Epitop am ersten Zelloberflächenmolekül, an das das dritte proteinöse Molekül binden kann, die gleichen sind.

18. Therapeutische Kombination nach Anspruch 17, wobei:
(a) die therapeutische Kombination die besagte erste pharmazeutische Zusammensetzung und die besagte zweite pharmazeutische Zusammensetzung umfasst, wobei
- das erste Epitop am ersten Zelloberflächenmolekül ein tumorzellspezifisches erstes Epitop an einem tumorzellspezifischen Oberflächenmolekül ist, bevorzugt ein tumorzellspezifisches erstes Epitop an einem ersten Zelloberflächenrezeptor, der spezifisch bei einer Tumorzelle vorliegt; und
- das zweite Zelloberflächenmolekül ein zweites tumorzellspezifisches Oberflächenmolekül verschieden vom ersten tumorzellspezifischen Oberflächenmolekül ist, bevorzugt ein zweiter Zelloberflächenrezeptor, der spezifisch bei einer Tumorzelle vorliegt, verschieden vom ersten Zelloberflächenrezeptor, der spezifisch bei besagter Tumorzelle vorliegt, und wobei das zweite Epitop ein tumorzellspezifisches zweites Epitop ist;
oder
(b) die therapeutische Kombination die besagte erste pharmazeutische Zusammensetzung und die besagte dritte pharmazeutische Zusammensetzung umfasst,
wobei das erste Zelloberflächenmolekül an einer Tumorzelloberfläche exprimiert ist, und bevorzugt das erste Zelloberflächenmolekül ein tumorzellspezifisches Oberflächenmolekül ist, und wobei bevorzugt das erste Epitop ein erstes tumorzellspezifisches Epitop ist.

19. Therapeutische Kombination nach Anspruch 17 oder 18, wobei die zweite Bindungsstelle des zweiten proteinösen Moleküls oder die dritte Bindungsstelle des dritten proteinösen Moleküls umfasst oder besteht aus einem Immunglobulin, zumindest einer Bindungsdomäne eines Immunglobulins und/oder zumindest einem Bindungsfragment eines Immunglobulins, wie etwa einem Antikörper, einem IgG, einem Molekül umfassend oder bestehend aus einer Vhh-Domäne oder Vh-Domäne, einem Fab, einem scFv, einem Fv, einem dAb, einem F(ab)2, Fcab-Fragment, oder umfasst oder besteht aus zumindest einem Ligand zum Binden an ein Zelloberflächenmolekül, wie etwa EGF oder ein Zytokin.

20. Therapeutische Kombination nach einem der Ansprüche 17-19, wobei
- die besagte erste Bindungsstelle ein erstes tumorzellspezifisches Epitop an einem ersten tumorzellspezifischen Rezeptor bindet, und
- die besagte zweite Bindungsstelle ein zweites tumorzellspezifisches Epitop an einem zweiten tumorzellspezifischen Rezeptor bindet,
und wobei
- die besagten ersten und zweiten tumorzellspezifischen Rezeptoren verschieden sind und an der gleichen Tumorzelle vorliegen,
- die erste und zweite Bindungsstelle verschieden sind und
- das erste und zweite tumorzellspezifische Epitop verschieden sind.

21. Therapeutische Kombination nach einem der Ansprüche 17-20, wobei besagte erste und dritte Bindungsstelle, die die gleiche sind, eine Bindungstelle für ein erstes tumorzellspezifisches Epitop an einem ersten tumorzellspezifischen Rezeptor sind.

22. Therapeutische Kombination nach Anspruch 20 oder 21, wobei der erste Rezeptor und/oder der zweite Rezeptor ausgewählt sind aus CD71, CA125, EpCAM(17-lA), CD52, CEA, CD44v6, FAP, EGF-IR, Integrin, Syndecan-1, vaskulärem Integrin Alpha-V Beta-3, HER2, EGFR, CD20, CD22, Folatrezeptor 1, CD146, CD56, CD19, CD138, CD27L Rezeptor, PSMA, CanAg, Integrin-AlphaV, CA6, CD33, Mesothelin, Cripto, CD3, CD30, CD239, CD70, CD123, CD352, DLL3, CD25, EphrinA4, MUC1, Trop2, CEACAM5, CEACAM6, HER3, CD74, PTK7, Notch3, FGF2, C4.4A, FTL3, CD38, FGFR3, CD7, PD-L1, CTLA4, CD52, PDGFRA, VEGFR1, VEGFR2, bevorzugt ausgewählt aus CD71, EGFR und HER2; unter der Voraussetzung, dass
- wenn das erste und zweite proteinöse Molekül in der therapeutischen Kombination vorliegen, die erste Bindungsstelle des ersten proteinösen Moleküls verschieden von der zweiten Bindungsstelle des zweiten proteinösen Molekül ist,
- wenn das erste und dritte proteinöse Molekül in der therapeutischen Kombination vorliegen, die erste und dritte Bindungsstelle die gleiche sind.

23. Erstes proteinöses Molekül nach einem der Ansprüche 1-16 oder therapeutische Kombination nach einem der Ansprüche 17-22,
wobei die erste Bindungstelle und/oder die zweite Bindungsstelle ist/sind oder umfasst/umfassen einen monoklonalen Antikörper oder zumindest ein Zelloberflächenmolekül bindendes Fragment und/oder -domäne davon, und bevorzugt umfassen oder bestehen aus einem aus Cetuximab, Daratumumab, Gemtuzumab, Trastuzumab, Panitumumab, Brentuximab, Inotuzumab, Moxetumomab, Polatuzumab, Obinutuzumab, OKT-9 Anti-CD71 monoklonalem Antikörper vom IgG-Typ, Pertuzumab, Rituximab, Ofatumumab, Herceptin, Alemtuzumab, Pinatuzumab, OKT-10 Anti-CD38 monoklonalem Antikörper, und einem Antikörper aus Tabelle A4, bevorzugt Cetuximab oder Trastuzumab oder OKT-9, oder zumindest einem Zelloberflächenmolekül bindenden Fragment oder -domäne davon, unter der Voraussetzung, dass die erste Bindungsstelle des ersten proteinösen Moleküls verschieden von der zweiten Bindungsstelle des zweiten proteinösen Moleküls ist.

24. Therapeutische Kombination nach einem der Ansprüche 17-23, wobei
(a) die besagte erste Bindungsstelle und die besagte dritte Bindungsstelle umfassen einen monoklonalen Antikörper, oder eine Zelloberflächenmolekül bindende Domäne oder -fragment davon, und bevorzugt umfassen oder bestehen aus einem aus Cetuximab, Daratumumab, Gemtuzumab, Trastuzumab, Panitumumab, Brentuximab, Inotuzumab, Moxetumomab, Polatuzumab, Obinutuzumab, OKT-9 Anti-CD71 monoklonalem Antikörper vom IgG-Typ, Pertuzumab, Rituximab, Ofatumumab, Herceptin, Alemtuzumab, Pinatuzumab, OKT-10 Anti-CD38 monoklonalem Antikörper, einem Antikörper aus Tabelle A2 oder Tabelle A3 oder Tabelle A4, bevorzugt Cetuximab oder Trastuzumab oder OKT-9, oder zumindest einem Zelloberflächenmolekül bindenden Fragment und/oder -domäne davon, unter der Voraussetzung, dass die erste Bindungsstelle des ersten proteinösen Moleküls die gleiche ist wie die dritte Bindungsstelle des dritten proteinösen Moleküls;
oder
(b) die besagte zweite Bindungsstelle oder die besagte dritte Bindungsstelle ist oder umfasst einen monoklonalen Antikörper, oder ein Zelloberflächenmolekül bindendes Fragment oder -domäne davon, und bevorzugt umfasst oder besteht aus einem aus Gemtuzumab Ozogamicin, Brentuximab Vedotin, Trastuzumab Emtansin, Inotuzumab Ozogamicin, Moxetumomab Pasudotox, Polatuzumab Vedotin oder einem Antikörper-Arzneimittelkonjugat aus Tabelle A2 oder Tabelle A3.

25. Therapeutische Kombination nach einem der Ansprüche 17-24, wobei der Effektorteil, der durch das zweite proteinöse Molekül beziehungsweise durch das dritte proteinöse Molekül umfasst ist, umfasst oder besteht aus:
(i) einem oder mehr aus einem Oligonuktleotid, einer Nukleinsäure, einer Xeno-Nukleinsäure, bevorzugt ausgewählt aus einem oder mehr aus einem Vektor, einem Gen, einem Zellsuizid induzierenden Transgen, Desoxyribonukleinsäure (DNA), Ribonukleinsäure (RNA), Antisense Oligonukleotid (ASO, AON), Shortinterfering RNA (siRNA), mikroRNA (miRNA), DNA Aptamer, RNA Aptamer, mRNA, mini-Circle DNA, Peptidnukleinsäure (PNA), Phosphoramidat-Morpholino Oligomer (PMO), locked Nukleinsäure (LNA), überbrückte Nukleinsäure (BNA), 2'-Desoxy-2'-fluorarabinonukleinsäure (FANA), 2'-O-Methoxyethyl-RNA (MOE), 2'-O,4'-Aminoethylen überbrückte Nukleinsäure, 3'-Fluorhexitolnukleinsäure (FHNA), einem Plasmid, Glykolnukleinsäure (GNA) und Threosenukleinsäure (TNA), oder einem Derivat davon, mehr bevorzugt einem BNA, beispielsweise einem BNA zum Eindämmen der HSP27 Proteinexpression;
oder
(ii) zumindest einem proteinösen Molekül, bevorzugt ausgewählt aus einem oder mehr aus einem Peptid, einem Protein, einem Enzym, wie etwa Urease und Cre-Rekombinase, einem Ribosom inaktivierenden Protein, einem proteinösen Toxin, mehr bevorzugt ausgewählt aus einem oder mehr aus einem Proteintoxin ausgewählt aus Tabelle A5 und/oder einem viralen Toxin, wie etwa Apoptin; einem bakteriellen Toxin, wie etwa Shigatoxin, einem Shiga-ähnlichen Toxin, Pseudomonas Aeruginosa Exotoxin (PE) oder Exotoxin A von PE, einem voll-langen oder trunkiertem Diphterietoxin (DT), Choleratoxin; einem Pilztoxin, wie etwa Alpha-Sarcin; einem Pflanzentoxin beinhaltend Ribosom inaktivierende Proteine und die A-Kette des Typ 2 Ribosom inaktivierenden Proteins, wie etwa Dianthin, e.g. Dianthin-30 oder Dianthin-32, Saporin, e.g. Saporin-S3 oder Saporin-S6, Bouganin oder deimmunisiertes Derivat Debouganin von Bouganin, Shiga-ähnliches Toxin A, Kermesbeeren-Antivirus-Protein, Ricin, Ricin-A-Kette, Modeccin, Modeccin-A-Kette, Abrin, Abrin-A-Kette, Volkensin, Volkensin-A-Kette, Viscumin, Viscumin-A-Kette; oder einem tierischen oder menschlichen Toxin, wie etwa Frosch RNase, oder Granzym B oder Angiogenin aus Menschen, oder irgendeinem Fragment oder Derivat davon; bevorzugt ist das Protein Dianthin und/oder Saporin;
oder
(iii) zumindest einer Ladung, bevorzugt ausgewählt aus einem oder mehr aus einem Toxin adressierende Ribosomen, einem Toxin adressierende Elongationsfaktoren, einem Toxin adressierenden Tubulin, einer Toxin adressierender DNA und einer Toxin adressierender RNA, mehr bevorzugt einem oder mehr aus Emtansin, Pasudotox, Maytansinoidderivat DM1, Maytansinoidderivat DM4, Monomethylauristatin E (MMAE, Vedotin), Monomethylauristatin F (MMAF, Mafodotin), einem Calicheamicin, N-Acetyl-γ-Cahcheamicin, einem Pyrrolbenzodiazepin (PBD)-Dimer, einem Benzodiazepin, einem CC-1065-Analogon, einem Duocarmycin, Doxorubicin, Paclitaxel, Docetaxel, Cisplatin, Cyclophosphamid, Etoposid, Docetaxel, 5-Fluoruracil (5-FU), Mitoxantron, einem Tubulysin, einem Indolino-Benzodiazepin, AZ13599185, einem Cryptophycin, Rhizoxin, Methotrexat, einem Anthracyclin, einem Camptothecin-Analogon, SN-38, DX-8951f, Exatecanmesylat, trunkierte Form von *Pseudomonas Aeruginosa* Exotoxin (PE38), einem Duocarmycinderivat, einem Amanitin, α-Amanitin, einem Spliceostatin, einem Thailanstatin, Ozogamicin, Tesirin, Amberstatin269 und Soravtansin, oder einem Derivat davon.

26. Zusammensetzung umfassend das erste proteinöse Molekül nach einem der Ansprüche 1-16 und
entweder
- das zweite proteinöse Molekül wie in einem der Ansprüche 17-25 definiert und optional einen pharmazeutisch annehmbaren Arzneimittelträger,
oder
- das dritte proteinöse Molekül wie in einem der Ansprüche 17-25 definiert und optional einen pharmazeutisch annehmbaren Arzneimittelträger, und
- wobei optional der Effektorteil, der durch das zweite proteinöse Molekül oder durch das dritte proteinöse Molekül umfasst ist, einer der Effektorteile ist: ein Oligonuktleotid, eine Nukleinsäure, eine Xeno-Nukleinsäure, bevorzugt ausgewählt aus einem oder mehr aus einem Vektor, einem Gen, einem Zellsuizid induzierenden Transgen, Desoxyribonukleinsäure (DNA), Ribonukleinsäure (RNA), Antisense Oligonukleotid (ASO, AON), Shortinterfering RNA (siRNA), mikroRNA (miRNA), DNA Aptamer, RNA Aptamer, mRNA, mini-Circle DNA, Peptidnukleinsäure (PNA), Phosphoramidat-Morpholino Oligomer (PMO), locked Nukleinsäure (LNA), überbrückte Nukleinsäure (BNA), 2'-Desoxy-2'-fluorarabinonukleinsäure (FANA), 2'-O-Methoxyethyl-RNA (MOE), 2'-O,4'-Aminoethylen überbrückte Nukleinsäure, 3'-Fluorhexitolnukleinsäure (FHNA), einem Plasmid, Glykolnukleinsäure (GNA) und Threosenukleinsäure (TNA), oder einem Derivat davon, mehr bevorzugt einem BNA, beispielsweise einem BNA zum Eindämmen der HSP27 Proteinexpression.

27. Zusammensetzung umfassend
- das erste proteinöse Molekül nach einem der Ansprüche 1-16 oder wie definiert in einem der Ansprüche 17-23 und
- eines oder mehr aus einem Oligonuktleotid, einer Nukleinsäure, einer Xeno-Nukleinsäure, bevorzugt ausgewählt aus zumindest einem aus einem Vektor, einem Gen, einem Zellsuizid induzierenden Transgen, Desoxyribonukleinsäure (DNA), Ribonukleinsäure (RNA), Antisense Oligonukleotid (ASO, AON), Shortinterfering RNA (siRNA), mikroRNA (miRNA), DNA Aptamer, RNA Aptamer, mRNA, mini-Circle DNA, Peptidnukleinsäure (PNA), Phosphoramidat-Morpholino Oligomer (PMO), locked Nukleinsäure (LNA), überbrückte Nukleinsäure (BNA), 2'-Desoxy-2'-fluorarabinonukleinsäure (FANA), 2'-O-Methoxyethyl-RNA (MOE), 2'-O,4'-Aminoethylen überbrückte Nukleinsäure, 3'-Fluorhexitolnukleinsäure (FHNA), einem Plasmid, Glykolnukleinsäure (GNA) und Threosenukleinsäure (TNA), oder einem Derivat davon, mehr bevorzugt einem BNA, beispielsweise einem BNA zum Eindämmen der HSP27 Proteinexpression.

28. Antikörper-Arzneimittelkonjugat oder ein Ligand-Arzneimittelkonjugat umfassend das erste proteinöse Molekül nach einem der Ansprüche 1-16 und ein Effektorteil, wobei optional:
(i) der Antikörper binden kann an eines aus CD71, CA125, EpCAM(17-1A), CD52, CEA, CD44v6, FAP, EGF-IR, Integrin, Syndecan-1, vaskuläres Integrin Alpha-V Beta-3, HER2, EGFR, CD20, CD22, Folatrezeptor 1, CD146, CD56, CD19, CD138, CD27L Rezeptor, PSMA, CanAg, Integrin-AlphaV, CA6, CD33, Mesothelin, Cripto, CD3, CD30, CD239, CD70, CD123, CD352, DLL3, CD25, EprinA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, bevorzugt CD71, HER2, EGFR, und/oder ist oder umfasst eines aus Cetuximab, Daratumumab, Gemtuzumab, Trastuzumab, Panitumumab, Brentuximab, Inotuzumab, Moxetumomab, Polatuzumab, Obinutuzumab, OKT-9 Anti-CD71 monoklonalem Antikörper vom IgG-Typ, Pertuzumab, Rituximab, Ofatumumab, Herceptin, Alemtuzumab, Pinatuzumab, OKT-10 Anti-CD38 monoklonalem Antikörper, einem Antikörper aus Tabelle A2 oder Tabelle A3 oder Tabelle A4, bevorzugt Cetuximab oder Trastuzumab oder OKT-9, oder zumindest einem Tumorzellrezeptor bindenden Fragment davon und/oder zumindest einer Tumorzellrezeptor bindenden Domäne davon, und/oder wobei das Antikörper-Arzneimittelkonjugat umfasst eines aus Gemtuzumab Ozogamicin, Brentuximab Vedotin, Trastuzumab Emtansin, Inotuzumab Ozogamicin, Moxetumomab Pasudotox und Polatuzumab Vedotin und einem Antikörper-Arzneimittelkonjugat aus Tabelle A2 und Tabelle A3;
und/oder
(ii) der Effektorteil eines oder mehr aus den Effektorteilen definiert in Anspruch 26 ist.

29. Pharmazeutische Zusammensetzung umfassend das Antikörper-Arzneimittelkonjugat oder das Ligand-Arzneimittelkonjugat nach Anspruch 28, und optional ferner umfassend einen pharmazeutisch annehmbaren Arzneimittelträger.

30. Therapeutische Kombination nach einem der Ansprüche 17-25 oder die Zusammensetzung nach Anspruch 26 oder 27 oder das Antikörper-Arzneimittelkonjugat oder das Ligand-Arzneimittelkonjugat nach Anspruch 28 oder die pharmazeutische Zusammensetzung nach Anspruch 29, zur Anwendung als ein Medikament oder zur Anwendung in der Behandlung oder Prävention von Krebs oder einer Autoimmunkrankheit.

## Revendications

1. Première molécule protéinique comprenant un premier site de liaison pour liaison à un premier épitope d'une première molécule de surface cellulaire, liée par liaison covalente à au moins une saponine par l'intermédiaire d'un lieur et/ou par l'intermédiaire d'un échafaudage oligomère ou polymère à un résidu d'acide aminé de ladite première molécule protéinique, ou liée par liaison covalente directement à un résidu d'acide aminé de ladite première molécule protéinique, dans laquelle
- le premier site de liaison comprend, ou est constitué de, une immunoglobuline, telle qu'un anticorps ou un IgG, ou comprend, ou est constitué de, au moins un ligand pour liaison à une molécule de surface cellulaire telle que l'EGF ou une cytokine, et
- dans laquelle l'au moins une saponine est choisie dans le groupe constitué par : SO1861, SA1641, GE1741, QS-21, QS-21A, QS21-B, Quil-A, SO1832, SO1904 et SO1862.

2. Première molécule protéinique selon la revendication 1, dans laquelle le premier épitope de la première molécule de surface cellulaire est un premier épitope spécifique de cellule tumorale d'une première molécule de surface de cellule tumorale.

3. Première molécule protéinique selon la revendication 1 ou 2, dans laquelle l'au moins une saponine est SO1861.

4. Première molécule protéinique selon l'une quelconque des revendications 1 à 3, dans laquelle
- l'au moins une saponine est une saponine triterpène bisdesmosidique appartenant au type d'un 12,13-déhydrooléanane ayant une fonction aldéhyde en position C-23,
- l'au moins une saponine est couplée par liaison covalente au résidu d'acide aminé de la première molécule protéinique par l'intermédiaire d'une fonction aldéhyde de la saponine, de préférence ladite fonction aldéhyde en position C-23, de préférence par l'intermédiaire d'au moins un lieur, plus préférablement par l'intermédiaire d'au moins un lieur clivable, dans lequel le résidu d'acide aminé est de préférence choisi parmi la cystéine et la lysine.

5. Première molécule protéinique selon l'une quelconque des revendications 1 à 4, dans laquelle
- l'au moins une saponine est une saponine triterpène bisdesmosidique appartenant au type d'un 12,13-déhydrooléanane ayant une fonction aldéhyde en position C-23 et comprenant une fonction acide glucuronique dans un substituant glucidique au niveau du groupe C-3bêta-OH de la saponine,
- l'au moins une saponine est couplée par liaison covalente au résidu d'acide aminé de la première molécule protéinique par l'intermédiaire d'une fonction acide glucuronique dans le substituant glucidique au niveau du groupe C-3bêta-OH de la saponine, de préférence par l'intermédiaire d'au moins un lieur, dans lequel le résidu d'acide aminé est de préférence choisi parmi la cystéine et la lysine.

6. Première molécule protéinique selon l'une quelconque des revendications 4-5, dans laquelle la fonction aldéhyde en position C-23 de l'au moins une saponine est couplée par liaison covalente au lieur hydrazide de l'acide N-ε-maléimidocaproïque, lequel lieur est couplé par liaison covalente par l'intermédiaire d'une liaison thio-éther à un groupe sulfhydryle de la première molécule protéinique, tel qu'un groupe sulfhydryle d'une cystéine.

7. Première molécule protéinique selon l'une quelconque des revendications 5-6, dans laquelle la fonction acide glucuronique du substituant glucidique au niveau du groupe C-3bêta-OH de l'au moins une saponine est couplée par liaison covalente au lieur hexafluorophosphate du 3-oxyde de 1-[bis (diméthylamino)méthylène]-1H-1,2,3-triazolo [4,5-b]pyridinium, lequel lieur est couplé par liaison covalente par l'intermédiaire d'une liaison amide à un groupe amine de la première molécule protéinique, tel qu'un groupe amine d'une lysine ou d'une extrémité N-terminale de la première molécule protéinique.

8. Première molécule protéinique selon l'une quelconque des revendications 1 à 7, dans laquelle ledit premier épitope est un premier épitope spécifique de cellule tumorale d'un récepteur spécifique des cellules tumorales choisi de préférence parmi CD71, CA125, EpCAM (17-1A), CD52, CEA, CD44v6, FAP, EGF-IR, l'intégrine, le syndécane-1, l'intégrine vasculaire alpha-V bêta-3, HER2, EGFR, CD20, CD22, le récepteur des folates 1, CD146, CD56, CD19, CD138, le récepteur CD27L, PSMA, CanAg, l'intégrine-alphaV, CA6, CD33, la mésothéline, Cripto, CD3, CD30, CD239, CD70, CD123, CD352, DLL3, CD25, l'éphrineA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, plus préférablement choisi parmi CD71, EGFR, HER2.

9. Première molécule protéinique selon la revendication 8, dans laquelle le premier site de liaison comprend, ou est constitué de, l'un quelconque parmi le cétuximab, le daratumumab, le gemtuzumab, le trastuzumab, le panitumumab, le brentuximab, l'inotuzumab, le moxétumomab, le polatuzumab, l'obinutuzumab, l'anticorps monoclonal anti-CD71 OKT-9 du type IgG, le pertuzumab, le rituximab, l'ofatumumab, l'herceptine, l'alemtuzumab, le pinatuzumab, l'anticorps monoclonal anti-CD38 OKT-10, un anticorps du Tableau A2 ou du Tableau A3 ou du Tableau A4, de préférence le cétuximab ou le trastuzumab ou l'OKT-9, ou au moins un fragment ou domaine de liaison au récepteur des cellules tumorales de ceux-ci, de préférence au moins un fragment de liaison au récepteur spécifique des cellules tumorales de ceux-ci et/ou au moins un domaine de liaison au récepteur spécifique des cellules tumorales de ceux-ci.

10. Première molécule protéinique selon l'une quelconque des revendications 1 à 9, dans laquelle la première molécule protéinique comprend plus d'une saponine, de préférence 2, 3, 4, 5, 6, 8, 10, 16, 32, 64 ou 1 à 100 saponines, ou un nombre quelconque de saponines entre ces nombres notamment 7, 9, 12 saponines, dans laquelle les saponines sont :
- liées par liaison covalente directement à un résidu d'acide aminé de la première molécule protéinique, de préférence à une cystéine et/ou à une lysine, et/ou
- liées par liaison covalente par l'intermédiaire d'au moins un lieur et/ou par l'intermédiaire d'au moins un lieur clivable et/ou par l'intermédiaire d'au moins un échafaudage polymère ou oligomère, de préférence de 1 à 8 de ces échafaudages ou de 2 à 4 de ces échafaudages, dans laquelle l'au moins un échafaudage se fonde éventuellement sur un dendron, auquel cas 1 à 32 saponines, notamment 2, 3, 4, 5, 6, 8, 10, 16, 32 saponines, ou un nombre quelconque de saponines entre ces derniers nombres, notamment 7, 9, 12 saponines, sont liées par liaison covalente à l'au moins un échafaudage.

11. Première molécule protéinique selon l'une quelconque des revendications 4 à 10, dans laquelle l'au moins un lieur est un lieur non clivable ou un lieur clivable, dans laquelle le lieur clivable, par exemple,
- est soumis à un clivage dans des conditions acides, des conditions réductrices, des conditions enzymatiques ou des conditions photo-induites, et de préférence le lieur clivable comprend une liaison hydrazone ou une liaison hydrazide soumise à clivage dans des conditions acides quand il est lié à la saponine, et/ou
- comprend une liaison sensible à la protéolyse, par exemple à une protéolyse par la cathepsine B, et/ou est une liaison sensible au clivage dans des conditions réductrices, telle qu'une liaison disulfure, quand il est lié à la saponine.

12. Première molécule protéinique selon l'une quelconque des revendications 4 à 11, dans laquelle le lieur clivable est soumis à un clivage *in vivo* dans des conditions acides telles celles qui sont présentes dans les endosomes et/ou les lysosomes des cellules de mammifère, de préférence des cellules humaines, de préférence à pH 4,0 - 6,5, et plus préférablement à pH ≤5,5, quand le lieur clivable est lié à une saponine.

13. Première molécule protéinique selon l'une quelconque des revendications 1 à 12, dans laquelle le échafaudage oligomère ou polymère comprend une structure polymère ou oligomère et comprend un groupe chimique, ledit groupe chimique étant destiné à un couplage par liaison covalente du échafaudage au résidu d'acide aminé de ladite première molécule protéinique.

14. Première molécule protéinique selon l'une quelconque des revendications 1 à 13, dans laquelle l'au moins une saponine est liée par liaison covalente à la structure polymère ou oligomère du échafaudage oligomère ou polymère par l'intermédiaire d'au moins un lieur clivable tel que défini dans l'une quelconque des revendications 10 ou 11.

15. Première molécule protéinique selon l'une quelconque des revendications 1 à 14, dans laquelle le groupe chimique du échafaudage oligomère ou polymère, pour couplage par liaison covalente du échafaudage oligomère ou polymère au résidu d'acide aminé de ladite première molécule protéinique, est un groupe de la chimie click, de préférence choisi parmi une tétrazine, un azide, un alcène ou un alcyne, ou un dérivé cyclique de ces groupes, plus préférablement ledit groupe chimique est un azide.

16. Première molécule protéinique selon l'une quelconque des revendications 1 à 15, dans laquelle la structure polymère ou oligomère du échafaudage oligomère ou polymère comprend un polymère linéaire, ramifié et/ou cyclique, un oligomère, un dendrimère, un dendron, un polymère dendronisé, un oligomère dendronisé, un ADN, un polypeptide, une polylysine, un polyéthylèneglycol, ou un ensemble de ces structures polymères ou oligomères, lequel ensemble est de préférence construit par des réticulations covalentes.

17. Association thérapeutique, dans laquelle l'association thérapeutique comprend :
(a) une première composition pharmaceutique comprenant la première molécule protéinique selon l'une quelconque des revendications 1 à 16 et éventuellement un excipient pharmaceutiquement acceptable ;
et
soit
(b) une deuxième composition pharmaceutique comprenant une deuxième molécule protéinique différente de la première molécule protéinique et éventuellement comprenant en outre un excipient pharmaceutiquement acceptable, la deuxième molécule protéinique comprenant
- un deuxième site de liaison pour liaison à un deuxième épitope d'une deuxième molécule de surface cellulaire différente de la première molécule de surface cellulaire, et
- une fraction effectrice, telle qu'une toxine, une enzyme ou un oligonucléotide,
dans laquelle le deuxième épitope est différent du premier épitope,
ou
(c) une troisième composition pharmaceutique comprenant une troisième molécule protéinique et éventuellement en outre comprenant un excipient pharmaceutiquement acceptable, la troisième molécule protéinique comprenant
- un troisième site de liaison pour liaison au premier épitope sur la molécule de surface cellulaire de (a) et
- une fraction effectrice, telle qu'une toxine, une enzyme ou un oligonucléotide ;
dans laquelle le premier site de liaison et le troisième site de liaison sont les mêmes, et dans laquelle la première molécule de surface cellulaire et le premier épitope sur la première molécule de surface cellulaire, auquel la première molécule protéinique peut se lier, et la première molécule de surface cellulaire et le premier épitope sur la première molécule de surface cellulaire, auquel la troisième molécule protéïque peut se lier, sont les mêmes.

18. Association thérapeutique selon la revendication 17, dans laquelle :
(a) l'association thérapeutique comprend ladite première composition pharmaceutique et ladite deuxième composition pharmaceutique, dans laquelle
- le premier épitope sur la première molécule de surface cellulaire est un premier épitope spécifique de cellule tumorale de la première molécule de surface spécifique de cellule tumorale, de préférence un premier épitope spécifique de cellule tumorale d'un premier récepteur de surface cellulaire spécifiquement présent au niveau d'une cellule tumorale ; et
- la deuxième molécule de surface cellulaire est une deuxième molécule de surface spécifique de cellule tumorale différente de la première molécule de surface spécifique de cellule tumorale, de préférence un deuxième récepteur de surface cellulaire spécifiquement présent au niveau d'une cellule tumorale différent du premier récepteur de surface cellulaire spécifiquement présent au niveau de ladite cellule tumorale, et dans laquelle le deuxième épitope est un deuxième épitope spécifique de cellule tumorale ;
ou
(b) l'association thérapeutique comprend ladite première composition pharmaceutique et ladite troisième composition pharmaceutique,
dans laquelle la première molécule de surface cellulaire est exprimée sur une surface de cellule tumorale, et de préférence la première molécule de surface cellulaire est une molécule de surface spécifique de cellule tumorale, et dans laquelle de préférence le premier épitope est un premier épitope spécifique de cellule tumorale.

19. Association thérapeutique selon la revendication 17 ou 18, dans laquelle le deuxième site de liaison de la deuxième molécule protéinique ou le troisième site de liaison de la troisième molécule protéinique comprend, ou est constitué de, une immunoglobuline, au moins un domaine de liaison d'une immunoglobuline et/ou au moins un fragment de liaison d'une immunoglobuline, tel qu'un anticorps, un IgG, une molécule comprenant, ou étant constituée de, un domaine Vhh ou un domaine Vh, un Fab, un scFv, un Fv, un dAb, un F (ab) 2, un fragment Fcab, ou comprend, ou est constitué de, au moins un ligand pour liaison à une molécule de surface cellulaire tel que l'EGF ou une cytokine.

20. Association thérapeutique selon l'une quelconque des revendications 17 à 19, dans laquelle
- ledit premier site de liaison se lie à un premier épitope spécifique de cellule tumorale sur un premier récepteur spécifique de cellule tumorale, et
- ledit deuxième site de liaison se lie à un deuxième épitope spécifique de cellule tumorale sur un deuxième récepteur spécifique de cellule tumorale,
et dans laquelle
- ledit premier et ledit deuxième récepteur spécifiques de cellule tumorale sont différents et sont présents sur la même cellule tumorale,
- le premier et le deuxième site de liaison sont différents et
- le premier et le deuxième épitopes spécifiques de cellule tumorale sont différents.

21. Association thérapeutique selon l'une quelconque des revendications 17 à 20, dans laquelle ledit premier et ledit troisième site de liaison, qui sont les mêmes, sont un site de liaison pour un premier épitope spécifique de cellule tumorale sur un premier récepteur spécifique de cellule tumorale.

22. Association thérapeutique selon la revendication 20 ou 21, dans laquelle le premier récepteur et/ou le deuxième récepteur sont choisis parmi CD71, CA125, EpCAM (17-1A), CD52, CEA, CD44v6, FAP, EGF-IR, l'intégrine, le syndécane-1, l'intégrine vasculaire alpha-V bêta-3, HER2, EGFR, CD20, CD22, le récepteur des folates 1, CD146, CD56, CD19, CD138, le récepteur CD27L, PSMA, CanAg, l'intégrine-alphaV, CA6, CD33, la mésothéline, Cripto, CD3, CD30, CD239, CD70, CD123, CD352, DLL3, CD25, l'éphrineA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, plus préférablement choisi parmi CD71, EGFR et HER2 ; aux conditions que
- quand la première et la deuxième molécules protéiniques sont présentes dans l'association thérapeutique, le premier site de liaison de la première molécule protéinique soit différent du deuxième site de liaison de la deuxième molécule protéinique,
- lorsque la première et la troisième molécules protéiniques sont présentes dans l'association thérapeutique, le premier et le troisième sites de liaison soient les mêmes.

23. Première molécule protéinique selon l'une quelconque des revendications 1 à 16 ou association thérapeutique selon l'une quelconque des revendications 17 à 22,
dans laquelle le premier site de liaison et/ou le deuxième site de liaison sont ou comprennent un anticorps monoclonal ou au moins un fragment et/ou un domaine de liaison à la molécule de surface cellulaire de celui-ci, et de préférence comprennent, ou sont constitués de, l'un quelconque parmi le cétuximab, le daratumumab, le gemtuzumab, le trastuzumab, le panitumumab, le brentuximab, l'inotuzumab, le moxétumomab, le polatuzumab, l'obinutuzumab, l'anticorps monoclonal anti-CD71 OKT-9 du type IgG, le pertuzumab, le rituximab, l'ofatumumab, l'herceptine, l'alemtuzumab, le pinatuzumab, l'anticorps monoclonal anti-CD38 OKT-10, et un anticorps du Tableau A4, de préférence le cétuximab ou le trastuzumab ou l'OKT-9, ou au moins un fragment ou un domaine de liaison à la molécule de surface cellulaire de ceux-ci, à la condition que le premier site de liaison de la première molécule protéinique soit différent du deuxième site de liaison de la deuxième molécule protéinique.

24. Association thérapeutique selon l'une quelconque des revendications 17 à 23, dans laquelle
(a) ledit premier site de liaison et ledit troisième site de liaison comprennent un anticorps monoclonal, ou un domaine ou un fragment de liaison à une molécule de surface cellulaire de celui-ci, et de préférence comprennent, ou sont constitués de, l'un quelconque parmi le cétuximab, le daratumumab, le gemtuzumab, le trastuzumab, le panitumumab, le brentuximab, l'inotuzumab, le moxétumomab, le polatuzumab, l'obinutuzumab, l'anticorps monoclonal anti-CD71 OKT-9 du type IgG, le pertuzumab, le rituximab, l'ofatumumab, l'herceptine, l'alemtuzumab, le pinatuzumab, l'anticorps monoclonal anti-CD38 OKT-10, un anticorps du Tableau A2 ou du Tableau A3 ou du Tableau A4, de préférence le cétuximab ou le trastuzumab ou l'OKT-9, ou au moins un fragment et/ou un domaine de liaison à une molécule de surface cellulaire de ceux-ci, à la condition que le premier site de liaison de la première molécule protéinique soit le même que le troisième site de liaison de la troisième molécule protéinique ;
ou
(b) ledit deuxième site de liaison ou ledit troisième site de liaison est ou comprend un anticorps monoclonal, ou un fragment ou un domaine de liaison à une molécule de surface cellulaire de celui-ci, et de préférence comprend, ou est constitué de, l'un quelconque parmi le gemtuzumab ozogamicine, le brentuximab védotine, le trastuzumab emtansine, l'inotuzumab ozogamicine, le moxétumomab pasudotox, le polatuzumab védotine ou un conjugué anticorps-médicament du Tableau A2 ou du Tableau A3.

25. Association thérapeutique selon l'une quelconque des revendications 17 à 24, dans laquelle la fraction effectrice qui est constituée respectivement de la deuxième molécule protéinique et de la troisième molécule protéinique comprend, ou est constituée de :
(i) un ou plusieurs quelconques parmi un oligonucléotide, un acide nucléique, un acide xéno nucléique, de préférence choisi parmi un ou plusieurs quelconques parmi un vecteur, un gène, un transgène induisant un suicide cellulaire, l'acide désoxyribonucléique (ADN), l'acide ribonucléique (ARN), un oligonucléotide antisens (ASO, AON), un petit ARN interférent (pARNi), un micro ARN (miARN), un aptamère d'ADN, un aptamère d'ARN, l'ARNm, l'ADN minicercle, un acide nucléique peptidique (PNA), un oligomère morpholino phosphoramidate (PMO), un acide nucléique verrouillé (LNA), un acide nucléique ponté (BNA), un acide 2'-désoxy-2'-fluoroarabino nucléique (FANA), un 2'-O-méthoxyéthyl-ARN (MOE), un acide nucléique ponté par un 2'-O,4'-aminoéthylène, un acide 3'-fluorohexitol nucléique (FHNA), un plasmide, un acide glycol-nucléique (GNA) et un acide thréose-nucléique (TNA), ou un dérivé de ceux-ci, plus préférablement un BNA, par exemple un BNA pour inactiver l'expression de la protéine HSP27 ;
ou
(ii) au moins une molécule protéinique, de préférence choisie parmi un ou plusieurs d'un peptide, d'une protéine, d'une enzyme telle qu'une uréase et une Cre-recombinase, d'une protéine d'inactivation des ribosomes, d'une toxine protéinique, plus préférablement choisie parmi un ou plusieurs quelconques d'une toxine protéinique choisie dans le Tableau A5 et/ou d'une toxine virale telle que l'apoptine, d'une toxine bactérienne telle que la toxine Shiga, la toxine Shiga-like, l'exotoxine de Pseudomonas aeruginosa (PE) ou l'exotoxine A de PE, de la toxine diphtérique (DT) pleine longueur ou tronquée, de la toxine cholérique ; d'une toxine fongique telle que l'alpha-sarcine ; d'une toxine végétale comprenant les protéines d'inactivation des ribosomes et la chaîne A des protéines d'inactivation des ribosomes de type 2 telles qu'une dianthine, par exemple la dianthine-30 ou la dianthine-32, d'une saporine, par exemple la saporine-S3 ou la saporine-S6, d'une bouganine ou d'une débouganine de bouganine dérivée désimmunisée, de la toxine A shiga-like, de la protéine antivirale du raisin d'Amérique, de la ricine, de la chaîne A de la ricine, de la modeccine, de la chaîne A de la modeccine, de l'abrine, de la chaîne A de l'abrine, de la volkensine, de la chaîne A de la volkensine, de la viscumine, de la chaîne A de la viscumine ; ou d'une toxine animale ou humaine telle que l'ARNase de grenouille ou la granzyme B ou l'angiogénine d'origine humaine, ou tout fragment ou dérivé de celles-ci ; de préférence, la toxine protéinique est la dianthine et/ou la saporine ;
ou
(iii) au moins une charge utile, de préférence choisie parmi un ou plusieurs quelconques de ribosomes ciblant une toxine, de facteurs d'allongement ciblant une toxine, d'une tubuline ciblant une toxine, d'un ADN ciblant une toxine et d'un ARN ciblant une toxine, plus préférablement parmi un ou plusieurs quelconques de l'emtansine, du pasudotox, du dérivé de maytansinoïde DM1, du dérivé de maytansinoïde DM4, de la monométhylauristatine E (MMAE, védotine), de la monométhylauristatine F (MMAF, mafodotine), d'une calicheamicine, de la N-acétyl-γ-calicheamicine, d'une pyrrolobenzodiazépine (PBD) dimère, d'une benzoidiazépine, d'un analogue de CC-1065, d'une duocarmycine, de doxorubicine, de paclitaxel, de docétaxel, de cisplatine, de cyclophosphamide, d'étoposide, de docétaxel, de 5-fluorouracile (5-FU), de mitoxantrone, d'une tubulysine, d'une indolinobenzodiazépine, de 1' AZ13599185, d'une cryptophycine, de rhizoxine, de méthotrexate, d'une anthracycline, d'un analogue de camptothécine, de SN-38, de DX-8951f, de mésylate d'exatécan, d'une forme tronquée de l'exotoxine de *Pseudomonas aeruginosa* (PE38), d'un dérivé de duocarmycine, d'une amanitine, de l'α-amanitine, d'une spliceostatine, d'une thailanstatine, de l'ozogamicine, de la tésirine, de l'amberstatine269 et de soravtansine ou d'un dérivé de ceux-ci.

26. Composition comprenant la première molécule protéinique selon l'une quelconque des revendications 1 à 16 et soit
- la deuxième molécule protéinique telle que définie dans l'une quelconque des revendications 17 à 25 et éventuellement un excipient pharmaceutiquement acceptable,
ou
- la troisième molécule protéinique telle que définie dans l'une quelconque des revendications 17 à 25 et éventuellement un excipient pharmaceutiquement acceptable, et
- dans laquelle éventuellement la fraction effectrice qui est constituée de la deuxième molécule protéinique ou de la troisième molécule protéinique est un ou plusieurs quelconques des fractions effectrices : un oligonucléotide, un acide nucléique, un acide xéno nucléique, de préférence choisi parmi un ou plusieurs quelconques parmi un vecteur, un gène, un transgène induisant un suicide cellulaire, l'acide désoxyribonucléique (ADN), l'acide ribonucléique (ARN), un oligonucléotide antisens (ASO, AON), un petit ARN interférent (pARNi), un micro ARN (miARN), un aptamère d'ADN, un aptamère d'ARN, l'ARNm, l'ADN minicercle, un acide nucléique peptidique (PNA), un oligomère morpholino phosphoramidate (PMO), un acide nucléique verrouillé (LNA), un acide nucléique ponté (BNA), un acide 2'-désoxy-2'-fluoroarabino nucléique (FANA), un 2'-O-méthoxyéthyl-ARN (MOE), un acide nucléique ponté par un 2'-O,4'-aminoéthylène, un acide 3'-fluorohexitol nucléique (FHNA), un plasmide, un acide glycol-nucléique (GNA) et un acide thréose-nucléique (TNA), ou un dérivé de ceux-ci, plus préférablement un BNA, par exemple un BNA pour inactiver l'expression de la protéine HSP27.

27. Composition comprenant
- la première molécule protéinique selon l'une quelconque des revendications 1 à 16 ou telle que définie dans l'une quelconque des revendications 17 à 23 et
- un ou plusieurs quelconques parmi un oligonucléotide, un acide nucléique, un acide xéno nucléique, de préférence choisi parmi un ou plusieurs quelconques parmi un vecteur, un gène, un transgène induisant un suicide cellulaire, l'acide désoxyribonucléique (ADN), l'acide ribonucléique (ARN), un oligonucléotide antisens (ASO, AON), un petit ARN interférent (pARNi), un micro ARN (miARN), un aptamère d'ADN, un aptamère d'ARN, l'ARNm, l'ADN minicercle, un acide nucléique peptidique (PNA), un oligomère morpholino phosphoramidate (PMO), un acide nucléique verrouillé (LNA), un acide nucléique ponté (BNA), un acide 2'-désoxy-2'-fluoroarabino nucléique (FANA), un 2'-O-méthoxyéthyl-ARN (MOE), un acide nucléique ponté par un 2'-O,4'-aminoéthylène, un acide 3'-fluorohexitol nucléique (FHNA), un plasmide, un acide glycol-nucléique (GNA) et un acide thréose-nucléique (TNA), ou un dérivé de ceux-ci, plus préférablement un BNA, par exemple un BNA pour inactiver l'expression de la protéine HSP27.

28. Conjugué anticorps-médicament ou conjugué ligand-médicament comprenant la première molécule protéinique selon l'une quelconque des revendications 1 à 16 et une fraction effectrice, éventuellement dans lequel :
(i) l'anticorps peut se lier à l'un quelconque de CD71, CA125, EpCAM (17-1A), CD52, CEA, CD44v6, FAP, EGF-IR, l'intégrine, le syndécane-1, l'intégrine vasculaire alpha-V bêta-3, HER2, EGFR, CD20, CD22, le récepteur des folates 1, CD146, CD56, CD19, CD138, le récepteur CD27L, PSMA, CanAg, l'intégrine-alphaV, CA6, CD33, la mésothéline, Cripto, CD3, CD30, CD239, CD70, CD123, CD352, DLL3, CD25, l'éphrineA4, MUC1, Trop2, CEACAM5, CEACAM6, HER3, CD74, PTK7, Notch3, FGF2, C4.4A, FLT3, CD38, FGFR3, CD7, PD-L1, CTLA4, CD52, PDGFRA, VEGFR1, VEGFR2, plus préférablement choisi parmi CD71, HER2 et EGFR, et/ou est ou comprend l'un quelconque parmi le cétuximab, le daratumumab, le gemtuzumab, le trastuzumab, le panitumumab, le brentuximab, l'inotuzumab, le moxétumomab, le polatuzumab, l'obinutuzumab, l'anticorps monoclonal anti-CD71 OKT-9 du type IgG, le pertuzumab, le rituximab, l'ofatumumab, l'herceptine, l'alemtuzumab, le pinatuzumab, l'anticorps monoclonal anti-CD38 OKT-10, un anticorps du Tableau A2 ou du Tableau A3 ou du Tableau A4, de préférence le cétuximab ou le trastuzumab ou l'OKT-9, ou au moins un fragment de liaison au récepteur des cellules tumorales de ceux-ci et/ou au moins un domaine de liaison au récepteur des cellules tumorales de ceux-ci, et/ou dans lequel le conjugué anticorps-médicament comprend l'un quelconque parmi le gemtuzumab ozogamicine, le brentuximab védotine, le trastuzumab emtansine, l'inotuzumab ozogamicine, le moxétumomab pasudotox et le polatuzumab védotine, et un conjugué anticorps-médicament du Tableau A2 et du Tableau A3 ;
et/ou
(ii) la fraction effectrice est une ou plusieurs quelconques parmi les fractions effectrices définies dans la revendication 26.

29. Composition pharmaceutique comprenant le conjugué anticorps-médicament ou le conjugué ligand-médicament selon la revendication 28, et éventuellement comprenant en outre un excipient pharmaceutiquement acceptable.

30. Association thérapeutique selon l'une quelconque des revendications 17 à 25 ou composition selon la revendication 26 ou 27 ou conjugué anticorps-médicament ou conjugué ligand-médicament selon la revendication 28 ou composition pharmaceutique selon la revendication 29, pour une utilisation comme médicament ou pour une utilisation dans le traitement ou la prévention d'un cancer ou d'une maladie auto-immune.
